(19) Europäisches Patentamt
European Patent Office
Office européen des brevets

(11) EP 2 216 339 A1

(12) EUROPEAN PATENT APPLICATION

(43) Date of publication:
**11.08.2010 Bulletin 2010/32**

(51) Int Cl.:
***C07H 21/02*** *(2006.01)*

(21) Application number: **10150046.0**

(22) Date of filing: **16.01.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **16.01.2006 IL 17317306**
**21.03.2006 IL 17446506**
**18.10.2006 US 852391 P**

(62) Document number(s) of the earlier application(s) in accordance with Art. 76 EPC:
**07700747.4 / 1 973 945**

(27) Previously filed application:
**16.01.2007 PCT/IL2007/000056**

(71) Applicant: **Compugen Ltd.**
**69512 Tel Aviv (IL)**

(72) Inventors:
- **Sella-Tavor, Osnat**
**19330 Kfar-Kish (IL)**
- **Pollock, Sarah**
**63506 Tel-Aviv (IL)**
- **Bazak, Lily**
**53461 Givatayim (IL)**
- **Tsypkin, Elena**
**64046 Tel-Aviv (IL)**
- **Sztybel, Dan**
**64376 Tel-Aviv (IL)**
- **Sameah-Greenwald, Shirley**
**44419 Kfar Saba (IL)**

(74) Representative: **Becker Kurig Straus**
**Patentanwälte**
**Bavariastrasse 7**
**80336 München (DE)**

Remarks:
This application was filed on 04-01-2010 as a divisional application to the application mentioned under INID code 62.

# (54) Novel nucleotide and amino acid sequences, and methods of use thereof for diagnosis

(57) The present invention relates to diagnostic markers comprising novel splice variants of known proteins and polynucleotides encoding same, useful in the qualitative and/or quantitative detection of various diseases and/or pathological conditions in a subject, and to the use of known proteins and polynucleotides encoding same for diagnosis. Particularly, the invention relates to the diagnosis of a disease in a sample of body fluid or secretion obtained from the subject, and to the diagnosis of cancer.

EP 2 216 339 A1

**Description**

FIELD OF THE INVENTION

The present invention is related to novel nucleotide and protein sequences, and assays and methods of use thereof.

BACKGROUND OF THE INVENTION

Diagnostic markers are important for early diagnosis of many diseases, as well as for predicting a response to treatment, monitoring treatment progress and determining prognosis of the disease.

Serum markers are examples of diagnostic markers, and are used for diagnosis of many different diseases. Typically, serum markers encompass secreted proteins and/or peptides; however, some serum markers may be released to the blood upon tissue lysis, for example from myocardial infarction (Troponin-I being a specific example). Serum markers can also be used as indicative risk factors of a disease (for example base-line levels of CRP, as a predictor of cardiovascular disease); to monitor disease activity and progression (for example, determination of CRP levels to monitor acute phase inflammatory response); and to predict and monitor drug response (for example, as shedded fragments of the protein Erb-B2).

Immunohistochemistry (IHC) is the study of the distribution of an antigen of choice in a sample based on specific antibody-antigen binding, typically performed on tissue slices. The antibody features a label which can be detected, for example as a stain which is detectable under a microscope. Preparation of the tissue slices for the assay involves fixation; IHC is therefore particularly suitable for antibody-antigen reactions that are not disturbed or destroyed by the process of fixing the tissue slices.

IHC permits determining the localization of the bound antibody-antigen, and hence mapping the presence of the antigen within the tissue and even within different compartments in the cell. Such mapping can provide useful diagnostic information, including:

1) The histological type of the tissue sample
2) The presence of specific cell types within the sample
3) Information regarding the physiological and/or pathological state of cells (e.g. which phase of the cell-cycle they are in)
4) The presence of disease related changes within the sample
5) Differentiation between specific disease subtypes where it is already known that the tissue is diseased (for example, the differentiation between different tumor types when it is already known the sample was taken from cancerous tissue).

IHC information is valuable for more than diagnosis. It can also be used to determine prognosis and progression of a therapy treatment (for example, as in the case of HER-2 in breast cancer) as well as to monitor the disease state.

IHC protein markers could be from any cellular location. Most often these markers are membrane proteins but secreted proteins or intracellular proteins (including intranuclear) can also be used as an IHC marker.

Although widely used as diagnostic tool, the IHC technique has at least two major disadvantages. It is performed on tissue samples and therefore a tissue sample has to be collected from the patient, which most often requires invasive procedures like biopsy associated with pain, discomfort, hospitalization and risk of infection. In addition, the interpretation of the result is observer dependent and therefore subjective. There is no measured value but rather only an estimation (on a scale of 1-4) of how prevalent the antigen is on the target.

Thus, there is a recognized need for, and it would be highly advantageous to have, an alternative diagnostic tool for diagnosing and monitoring diseases.

SUMMARY OF THE INVENTION

The present invention provides novel nucleic acid and amino acid sequences, which can be used as diagnostic markers.

According to one aspect, the present invention provides a number of novel variants of known proteins which are found in serum and can be used as diagnostic markers. The present invention overcomes the many deficiencies of the background art with regard to the need to obtain tissue samples and subjective interpretations of results. In certain embodiments of the present invention, tissue specific markers are identifiable in serum or plasma. Thus, according to the teachings of the present invention, a simple blood test can provide qualitative and/or quantitative indication of various diseases and/or pathological conditions, according to the expression of certain marker(s).

According to another aspect, the present invention discloses the novel use of known proteins as diagnostic markers. In some embodiments, the markers disclosed can also be used for in-vivo imaging applications.

It is disclosed in the present invention for the first time that the protein variants of the invention are useful as diagnostic markers for various diseases and/or pathological conditions as described in greater detail below. The variants themselves are described by "cluster" or by gene, as these variants are splice variants of known proteins. Therefore, as used in the present invention, the term "marker-detectable disease" refers to a disease that may be detected by a particular marker, with regard to the description of the disease provided herein below. The markers of the present invention, alone or in combination, show a high degree of differential diagnosis between disease and non-disease states.

The present invention further relates to diagnostic assays for detecting a disease, particularly in a sample taken from a subject (patient), preferably a blood sample or a body secretion sample. According to certain embodiments, the diagnostic assays disclosed in the present invention are NAT (nucleic acid amplification technology)-based assays, including, for example, PCR or variations thereof, e.g. real-time PCR. According to other embodiments, the assays encompass nucleic acid hybridization assays. The diagnostic assays can be qualitative or quantitative.

According to certain embodiments, the present invention provides a diagnostic marker comprising a novel splice variant of a known protein or a polynucleotide encoding same, wherein the protein is selected from the group consisting of Delta-like Protein 3 Precursor (DLL3), Complement Factor B Precursor, Serine/Threonine-Protein Kinase TNNI3K, Cardiomyopathy Associated 4 (CMYA4) and Myosin Regulatory Light Chain 2, Atrial Isoform. According to certain embodiments, the diagnostic marker is found in a body fluid or secretion.

According to one embodiment, the novel splice variant is an isolated polynucleotide comprising a nucleic acid having a nucleic acid sequence as set forth in any one of SEQ. ID NOs:37-39, 78-88, 156-160, 162-164, 167-170, 240-241, 276-281, or a sequence homologous thereto. According to one embodiment, the isolated polynucleotide is at least 85% homologous to any one of SEQ. ID NOs: 37-39, 78-88, 156-160, 162-164, 167-170, 240-241, 276-281.

According to another embodiment, the novel splice variant is an isolated polynucleotide comprising a nucleic acid having a nucleic acid sequence as set forth in any one of SEQ. ID NOs: 40-53, 89-130, 171-208, 242-266, 282-301, or a sequence homologous thereto. According to one embodiment, the isolated polynucleotide is at least 85% homologous to any one of SEQ. ID NOs: 40-53, 89-130, 171-208, 242-266, 282-301.

According to certain embodiments, the present invention also encompasses isolated polynucleotides having a sequence complementary to any one of the nucleic acid sequences listed herein. According to other embodiments, this invention provides an oligonucleotide of at least about 12 nucleotides, specifically hybridizable with the polynucleotides of this invention. The present invention further provides vectors, cells, liposomes and compositions comprising the isolated polynucleotides of this invention.

According to yet another embodiment, the novel splice variant is an isolated protein or polypeptide having an amino acid sequence as set forth in any one of SEQ. ID NOs: 57-58, 135, 137, 138, 140-142, 212-220, 222-229, 268, 269, 303-308, or a sequence homologous thereto. According to one embodiment, the isolated protein or polypeptide is at least 85% homologous to any one of SEQ. ID NOs: 57-58, 135, 137, 138, 140-142, 212-220, 222-229, 268, 269, 303-308.

According to some embodiments, the sample taken from a subject (patient) to perform the diagnostic assay according to the present invention is selected from the group consisting of a body fluid or secretion including but not limited to blood, serum, urine, plasma, prostatic fluid, seminal fluid, semen, the external secretions of the skin, respiratory, intestinal, and genitourinary tracts, tears, cerebrospinal fluid, sputum, saliva, milk, peritoneal fluid, pleural fluid, cyst fluid, secretions of the breast ductal system (and/or lavage thereof), broncho alveolar lavage, lavage of the reproductive system and lavage of any other part of the body or system in the body; samples of any organ including isolated cell(s) or tissue(s), wherein the cell or tissue can be obtained from an organ selected from, but not limited to lung, colon, ovarian and/or breast tissue; stool or a tissue sample, or any combination thereof. In some embodiments, the term encompasses samples of in vivo cell culture constituents. Prior to be subjected to the diagnostic assay, the sample can optionally be diluted with a suitable eluant.

The term "homology", as used herein, refers to a degree of sequence similarity in terms of shared amino acid or nucleotide sequences. There may be partial homology or complete homology (i.e., identity). For amino acid sequence homology amino acid similarity matrices may be used as are known in different bioinformatics programs (e.g. BLAST, Smith Waterman). Different results may be obtained when performing a particular search with a different matrix. Homologous peptide or polypeptides are characterized by one or more amino acid substitutions, insertions or deletions, such as, but not limited to, conservative substitutions, provided that these changes do not affect the biological activity of the peptide or polypeptide as described herein.

Degrees of homology for nucleotide sequences are based upon identity matches with penalties made for gaps or insertions required to optimize the alignment, as is well known in the art (e.g. Altschul S. F. et al., 1990, J Mol Biol 215(3):403-10; Altschul S.F. et al., 1997, Nucleic Acids Res. 25:3389-3402). The degree of sequence homology is presented in terms of percentage, e.g. "70% homology". As used herein, the term "at least" with regard to a certain degree of homology encompasses any degree of homology from the specified percentage up to 100%.

The terms "correspond" or "corresponding to" or "correspondence with" are used herein to indicate identity between two corresponding amino acid or nucleic acid sequences.

In some embodiments, the proteins or polypeptides of this invention comprise chimeric protein or polypeptides.

As used herein, the terms "chimeric protein or polypeptide", or "chimeric polynucleotide" or "chimera" refers to an assembly or a string of amino acids in a particular sequence, or nucleotides encoding the same, respectively, which does not correspond in their entirety to the sequence of the known (wild type) polypeptide or protein, or the nucleic acid encoding same.

In some embodiments, the variants of this invention are derived from two exons, or an exon and an intron of a known protein, or fragments thereof, or segments having sequences with the indicated homology.

According to certain embodiments, the present invention now discloses a novel cluster designated herein N43992, comprising novel amino acid and nucleic acid sequences that are variants of the known Delta-like protein 3 precursor (DLL3, SEQ. ID NO: 54, SwissProt accession identifier DLL3_HUMAN), known also according to the synonym Drosophila Delta homolog 3. The novel variant polynucleotides and polypeptides described by the present invention are useful as diagnostic markers, preferably as serum markers.

Surprisingly, the present invention now shows that the wild type DLL3 as well as its variants are overexpressed in cancerous tissues, particularly in cancerous lung tissues, and thus can be used for the diagnosis, prognosis, treatment selection, and treatment monitoring and/or assessment of cancers, particularly lung cancer, as is described in a greater detail below. According to certain embodiments of the present invention, the wild type DLL3 polynucleotides and polypeptides are useful as diagnostic markers, preferably as IHC markers and for in-vivo imaging.

According to one embodiment, the present invention provides an isolated polypeptide comprising an edge portion of N43992_P13 (SEQ. ID NO: 57), wherein the edge portion comprises an amino acid sequence being at least 70%, optionally at least about 80%, preferably at least about 85%, more preferably at least about 90% and most preferably at least about 95% homologous to the amino acid sequence APLPPLLQSLPEAWALRGGRRVPVRPGRGAECARTGLHRAARSARA (SEQ. ID NO: 326), corresponding to amino acids 44 - 89 of N43992_P13 (SEQ. ID NO: 57).

According to one embodiment, the isolated polypeptide is a chimeric polypeptides comprising a first amino acid sequence being at least about 90% homologous to the amino acid sequence MVSPRMSGLLSQTVILALIFLPQTRPAGVFELQIHSFGPGPGP, corresponding to amino acids 1 - 43 of DLL3_HUMAN (SEQ. ID NO: 54), also corresponding to amino acids 1 - 43 of N43992_P13 (SEQ. ID NO: 57), and a second amino acid sequence being at least about 70%, optionally at least about 80%, preferably at least about 85%, more preferably at least about 90% and most preferably at least about 95% homologous to the amino acid sequence APLPPLLQSLPEAWALRGGRRVPVRPGRGAECARTGLHRAARSARA (SEQ. ID NO: 326), corresponding to amino acids 44 - 89 of N43992_P13 (SEQ. ID NO: 57), wherein the first amino acid sequence and the second amino acid sequence are contiguous and in a sequential order.

According to another embodiment, the isolated polypeptide comprises an amino acid sequence as set forth in SEQ. ID NO:57 (N43992_P13).

According to another embodiment, the present invention provides an isolated polypeptide comprising an edge portion of N43992_P14 (SEQ. ID NO: 58), wherein the edge portion comprises an amino acid sequence being at least 70%, optionally at least about 80%, preferably at least about 85%, more preferably at least about 90% and most preferably at least about 95% homologous to the amino acid sequence VRARHGPLASSSCRSTLSGRVQALGPRGPPAAPGSPAASSSESA (SEQ. ID NO: 327), corresponding to amino acids 24-67 of N43992_P14 (SEQ. ID NO: 58).

According to one embodiment, the isolated polypeptides is a chimeric polypeptide comprising a first amino acid sequence being at least about 90% homologous to the amino acid sequence MVSPRMSGLLSQTVILALIFLPQ corresponding to amino acids 1 - 23 of DLL3_HUMAN (SEQ. ID NO: 54), also corresponding to amino acids 1 - 23 of N43992_P14 (SEQ. ID NO: 58), and a second amino acid sequence being at least about 70%, optionally at least about 80%, preferably at least about 85%, more preferably at least about 90% and most preferably at least about 95% homologous to a polypeptide having the sequence VRARHGPLASSSCRSTLSGRVQALGPRGPPAAPGSPAASSSESA (SEQ. ID NO: 327) corresponding to amino acids 24 - 67 of N43992_P14 (SEQ. ID NO: 58), wherein the first amino acid sequence and the second amino acid sequence are contiguous and in a sequential order.

According to another embodiment, the isolated polypeptide comprises an amino acid sequence as set forth in SEQ. ID NO:58 (N43992_P14).

According to a further embodiment the present invention provides an isolated polypeptide comprising an edge portion of N43992_P16 (SEQ. ID NO: 59), wherein the edge portion comprises an amino acid sequence being at least 70%, optionally at least about 80%, preferably at least about 85%, more preferably at least about 90% and most preferably at least about 95% homologous to the amino acid sequence EAWRPERRGMGWGSWMAQTVQGWNPGFDSSNPRAWGPDLPPASL (SEQ. ID NO: 328), corresponding to amino acids 366 - 409 of N43992_P16 (SEQ. ID NO: 59).

According to one embodiment, the isolated polypeptide is a chimeric polypeptides comprising a first amino acid sequence being at least about 90% homologous to the amino acid sequence MVSPRMSGLLSQTVILALIFLPQTRPAGVFELQIHSFGPGPGPGAPRSPCSARLPCRLFFRVCLK
PGLSEEAAESPCALGAALSARGPVYTEQPGAPAPDLPLPDGLLQVPFRDAWPGTFSFIIETWRE ELGDQIGGPAWSLLARVAGRRRLAAGGPWARDIQRAGAWELRFSYRARCEPPAVGTACTRL CRPRSAPSRCGPGLRPCAPLEDECEAPLVCRAGCSPEHGFCEQPGECRCLEGWTGPLCTVPVS TSSCLSPRGPSSATTGCLVPGPGPCDGNPCANGGSCSETPRSFECTCPRGFYGLRCEVSGVTCA DGPCFNGGLCVGGADPDSAYICHCPPGFQGSNCEKRVDRCSLQPCRNG corresponding to amino acids 1 - 365 of DLL3_HUMAN (SEQ. ID NO: 54), also corresponding to amino acids 1 - 365 of N43992_P16 (SEQ. ID NO: 59), and a second amino acid sequence being at least about 70%, optionally at least about 80%, preferably at least about 85%, more preferably at least about 90% and most preferably at least about 95% homologous to the amino acid sequence EAWRPERRGMGWGSWMAQTVQGWNPGFDSSNPRAWGPDLPPASL (SEQ. ID NO: 328) corresponding to amino acids 366 - 409 of N43992_P16 (SEQ. ID NO: 59), wherein said first amino acid sequence and second amino acid sequence are contiguous and in a sequential order.

According to another embodiment, the isolated chimeric polypeptides comprises a first amino acid sequence being at least about 90% homologous to the amino acid sequence MVSPRMSGLLSQTVILALIFLPQTRPAGVFELQIHS-FGPGPGPGAPRSPCSARLPCRLFFRVCLK PGLSEEAAESPCALGAALSARGPVYTEQPGAPAPDLPLPDGLLQVP-FRDAWPGTFSFIIETWRE ELGDQIGGPAWSLLARVAGRRRLAAGGPWARDIQRAGAWELR corresponding to amino acids 1 - 171 of Q8NBS4_HUMAN (SEQ. ID NO: 55), also corresponding to amino acids 1 - 171 of N43992_P16 (SEQ. ID NO: 59), a first bridging amino acid F corresponding to amino acid 172 of N43992_P16 (SEQ. ID NO: 59), a second amino acid sequence being at least about 90% homologous to the amino acid sequence SYRARCEPPAVGTACTRLCRPRSAPSRCGPGLRPCAPLEDECEAP corresponding to amino acids 173 - 217 of Q8NBS4_HUMAN (SEQ. ID NO: 55), also corresponding to amino acids 173 - 217 of N43992_P16 (SEQ. ID NO: 59), a second bridging amino acid L corresponding to amino acid 218 of N43992_P16 (SEQ. ID NO: 59), a third amino acid sequence being at least 90% homologous to the amino acid sequence VCRAGCSPEHGFCEQPGECRCLEGWTGPLCTVPVSTSSCLSPRGPSSATTGCLVPGPGPCDGN PCANGGSCSETPRSFECTCPRGFYGLRCEVSGVTCA corresponding to amino acids 219 - 317 of Q8NBS4_HUMAN (SEQ. ID NO: 55), also corresponding to amino acids 219 - 317 of N43992_P16 (SEQ. ID NO: 59), a third bridging amino acid D corresponding to amino acid 318 of N43992_P16 (SEQ. ID NO: 59), a fourth amino acid sequence being at least 90% homologous to the amino acid sequence GPCFNGGLCVGGADPDSAYICHCPPGFQGSNCEKRVDRCSLQPCRNG corresponding to amino acids 319 - 365 of Q8NBS4_HUMAN (SEQ. ID NO: 55), also corresponding to amino acids 319 - 365 of N43992_P16 (SEQ. ID NO: 59), and a fifth amino acid sequence being at least about 70%, optionally at least about 80%, preferably at least about 85%, more preferably at least about 90% and most preferably at least about 95% homologous to the amino acid sequence EAWRPERRGMGWGSWMAQTVQGWNPGFDSSNPRAWGPDLPPASL (SEQ. ID NO: 328) corresponding to amino acids 366 - 409 of N43992_P16 (SEQ. ID NO: 59), wherein the first amino acid sequence, first bridging amino acid, second amino acid sequence, second bridging amino acid, third amino acid sequence, third bridging amino acid, fourth amino acid sequence and fifth amino acid sequence are contiguous and in a sequential order.

According to yet other embodiments, the present invention now discloses a novel cluster designated herein D12115, comprising novel amino acid and nucleic acid sequences that are variants of the known Complement factor B precursor (CFAB_HUMAN (SEQ. ID NO: 395)). The novel polynucleotides and polypeptides described by the present invention are useful as diagnostic markers, preferably as serum markers.

Surprisingly, the present invention now shows that the D12115 variants are overexpressed in cancerous tissues, particularly in cancerous lung, cancerous breast and cancerous ovarian tissues, and thus can be used for the diagnosis, prognosis, treatment selection and treatment monitoring and/or assessment of cancers, particularly lung cancer, breast cancer and ovarian cancer, as described in a greater detail below.

According to one embodiment, the present invention provides an isolated polypeptide comprising an edge portion of D12115_P3 (SEQ. ID NO:134), comprising an amino acid sequence being at least about 70%, optionally at least about 80%, preferably at least about 85%, more preferably at least about 90% and most preferably at least about 95% homologous to the sequence AALAEGETPR (SEQ. ID NO: 329).

According to one embodiment, the isolated polypeptide is a chimeric polypeptides comprising a first amino acid sequence being at least about 90% homologous to amino acids 1 - 730 of CFAB_HUMAN (SEQ. ID NO: 395), also corresponding to amino acids 1 - 730 of D12115_P3 (SEQ. ID NO:134), and a second amino acid sequence being at least about 70%, optionally at least about 80%, preferably at least about 85%, more preferably at least about 90% and most preferably at least about 95% homologous to a polypeptide having the sequence AALAEGETPR (SEQ. ID NO: 329) corresponding to amino acids 731 - 740 of D12115_P3 (SEQ. ID NO:134), wherein said first amino acid sequence and second amino acid sequence are contiguous and in a sequential order.

According to one embodiment, the isolated polypeptide is a chimeric polypeptides comprising a first amino acid sequence being at least about 90% homologous to amino acids 1 - 31 of NP_001701 (SEQ. ID NO:133), also corresponding to amino acids 1 - 31 of D12115_P3 (SEQ. ID NO:134), a bridging amino acid R corresponding to amino acid 32 of D12115_P3 (SEQ. ID NO:134), a second amino acid sequence being at least about 90% homologous to amino acids 33 - 730 of NP_001701 (SEQ. ID NO:133), also corresponding to amino acids 33 - 730 of D12115_P3 (SEQ. ID NO:134), and a third amino acid sequence being at least about 70%, optionally at least about 80%, preferably at least about 85%, more preferably at least about 90% and most preferably at least about 95% homologous to a polypeptide having the sequence AALAEGETPR (SEQ. ID NO: 329) corresponding to amino acids 731 - 740 of D12115_P3 (SEQ. ID NO:134), wherein said first amino acid sequence, bridging amino acid, second amino acid sequence and third amino acid sequence are contiguous and in a sequential order.

According to one embodiment, the present invention provides an isolated polypeptide comprising an edge portion of D12115_P3 (SEQ. ID NO:134), comprising an amino acid sequence being at least about 70%, optionally at least about 80%, preferably at least about 85%, more preferably at least about 90% and most preferably at least about 95% homologous to the sequence - GEKRDLEIEVVLFHPNYNINGKKEAGIPEFYDYDVALIKLKNKLKYGQTIRPI-CLPCTEGTTRA LRLPPTTTCQQQKEELLPAQDIKALFVSEEEKKLTRKEVYIKNGDKKGSCERDAQYAPGYDK VKDIS-EVVTPRFLCTGGVSPYADPNTCRGDSGGPLIVHKRSRFIQVGVISWGVVDVCKNQKRA ALAEGETPR (SEQ. ID NO: 330).

According to one embodiment, the isolated polypeptide is a chimeric polypeptides comprising a first amino acid sequence being at least about 90% homologous to amino acids 1 - 542 of P00751-2 (SEQ. ID NO:132), also corresponding to amino acids 1 - 542 of D12115_P3 (SEQ. ID NO:134), and a second amino acid sequence being at least about 70%, optionally at least about 80%, preferably at least about 85%, more preferably at least about 90% and most preferably at least about 95% homologous to a polypeptide having the sequence GEKRDLEIEVVLFHPNYNINGKKEAGIPEFYDY-DVALIKLKNKLKYGQTIRPICLPCTEGTTRA LRLPPTTTCQQQKEELLPAQDIKALFVSEEEKKLTRKEVYIKNGDKKG-SCERDAQYAPGYDK VKDISEVVTPRFLCTGGVSPYADPNTCRGDSGGPLIVHKRSRFIQVGVISWGVVDVCKNQKRA ALAEGETPR (SEQ. ID NO: 330) corresponding to amino acids 543 - 740 of D12115_P3 (SEQ. ID NO:134), wherein said first amino acid sequence and second amino acid sequence are contiguous and in a sequential order.

In some embodiments, the isolated chimeric proteins or polypeptides of the invention may comprise an amino acid sequence corresponding to or homologous to D12115_P5 (SEQ. ID NO:135).

In some embodiments, such isolated chimeric proteins or polypeptides comprise a first amino acid sequence being at least about 90% homologous to amino acids 1 - 390 of CFAB_HUMAN (SEQ. ID NO: 395), which also corresponds to amino acids 1 - 390 of D12115_P5 (SEQ. ID NO:135), and a second amino acid sequence being at least about 70%, optionally at least about 80%, preferably at least about 85%, more preferably at least about 90% and most preferably at least about 95% homologous to a polypeptide having the sequence QKGPLSCPSLPTFSDQHVALKSTCNTIPMVGALNVTHSWLFISPVTLHKEFFLSPVINYL (SEQ. ID NO:331) corresponding to amino acids 391 - 450 of D12115_P5 (SEQ. ID NO:135), wherein said first amino acid sequence and second amino acid sequence are contiguous and in a sequential order.

In some embodiments, this invention provides an isolated polypeptide comprising an edge portion of D12115_P5 (SEQ. ID NO:135), comprising an amino acid sequence being at least about 70%, optionally at least about 80%, preferably at least about 85%, more preferably at least about 90% and most preferably at least about 95% homologous to the sequence -

QKGPLSCPSLPTFSDQHVALKSTCNTIPMVGALNVTHSWLFISPVTLHKEFFLSPVINYL (SEQ. ID NO: 331) of D12115_P5 (SEQ. ID NO:135).

In some embodiments, such isolated chimeric proteins or polypeptides comprise a first amino acid sequence being at least about 90% homologous to amino acids 1 - 390 of P00751-2 (SEQ. ID NO:132), which also corresponds to amino acids 1 - 390 of D12115_P5 (SEQ. ID NO:135), and a second amino acid sequence being at least about 70%, optionally at least about 80%, preferably at least about 85%, more preferably at least about 90% and most preferably at least about 95% homologous to a polypeptide having the sequence QKGPLSCPSLPTFSDQHVALKSTCNTIPMVGALNVTHSWLFISPVTLHKEFFLSPVINYL (SEQ. ID NO: 331) corresponding to amino acids 391 - 450 of D12115_P5 (SEQ. ID NO:135), wherein said first amino acid sequence and second amino acid sequence are contiguous and in a sequential order.

In some embodiments, this invention provides an isolated polypeptide comprising an edge portion of D12115_P5 (SEQ. ID NO:135), comprising an amino acid sequence being at least about 70%, optionally at least about 80%, preferably at least about 85%, more preferably at least about 90% and most preferably at least about 95% homologous to the sequence -

QKGPLSCPSLPTFSDQHVALKSTCNTIPMVGALNVTHSWLFISPVTLHKEFFLSPVINYL (SEQ. ID NO: 331) of D12115_P5 (SEQ. ID NO:135).

In some embodiments, such isolated chimeric proteins or polypeptides comprise a first amino acid sequence being at least about 90% homologous to MGSNLSPQLCLMPFILGLLSGGVTTTPWSLA corresponding to amino acids 1 - 31 of NP_001701 (SEQ. ID NO:133), which also corresponds to amino acids 1 - 31 of D12115_P5 (SEQ. ID NO: 135), a bridging amino acid R corresponding to amino acid 32 of D12115_P5 (SEQ. ID NO:135), a second amino acid sequence being at least about 90% homologous to amino acids 33 - 390 of NP_001701 (SEQ. ID NO:133), which also corresponds to amino acids 33 - 390 of D12115_P5 (SEQ. ID NO:135), and a third amino acid sequence being at least about 70%, optionally at least about 80%, preferably at least about 85%, more preferably at least about 90% and most preferably at least about 95% homologous to a polypeptide having the sequence QKGPLSCPSLPTFSDQHVALKSTCNTIPMVGALNVTHSWLFISPVTLHKEFFLSPVINYL (SEQ. ID NO:331) corresponding to amino acids 391 - 450 of D12115_P5 (SEQ. ID NO:135), wherein said first amino acid sequence, bridging amino acid, second amino acid sequence and third amino acid sequence are contiguous and in a sequential order.

In some embodiments, this invention provides an isolated polypeptide comprising an edge portion of D12115_P5 (SEQ. ID NO:135), comprising an amino acid sequence being at least about 70%, optionally at least about 80%, preferably at least about 85%, more preferably at least about 90% and most preferably at least about 95% homologous to the sequence -

QKGPLSCPSLPTFSDQHVALKSTCNTIPMVGALNVTHSWLFISPVTLHKEFFLSPVINYL (SEQ. ID NO: 331) of D12115_P5 (SEQ. ID NO:135).

In some embodiments, the isolated chimeric proteins or polypeptides of the invention comprise an amino acid sequence corresponding to or homologous to D12115_P12 (SEQ. ID NO:136). In some embodiments, such isolated chimeric proteins or polypeptides comprise a first amino acid sequence being at least about 90% homologous to amino acids 1 - 714 of CFAB_HUMAN (SEQ. ID NO: 395), which also corresponds to amino acids 1 - 714 of D12115_P12 (SEQ. ID NO:136), and a second amino acid sequence being at least about 70%, optionally at least about 80%, preferably at least about 85%, more preferably at least about 90% and most preferably at least about 95% homologous to a polypeptide having the sequence SPPFPIWGDAKWSAWAPKQESSMHVASNSR (SEQ. ID NO: 332) corresponding to amino acids 715 - 744 of D12115_P12 (SEQ. ID NO:136), wherein said first amino acid sequence and second amino acid sequence are contiguous and in a sequential order.

In some embodiments, this invention provides an isolated polypeptide an edge portion of D12115_P12 (SEQ. ID NO:136), comprising an amino acid sequence being at least about 70%, optionally at least about 80%, preferably at least about 85%, more preferably at least about 90% and most preferably at least about 95% homologous to the sequence SPPFPIWGDAKWSAWAPKQESSMHVASNSR (SEQ. ID NO: 332) of D12115_P12 (SEQ. ID NO:136).

In some embodiments, such isolated chimeric proteins or polypeptides comprise a first amino acid sequence being at least about 90% homologous to MGSNLSPQLCLMPFILGLLSGGVTTTPWSLA corresponding to amino acids 1 - 31 of NP_001701 (SEQ. ID NO:133), which also corresponds to amino acids 1 - 31 of D12115_P12 (SEQ. ID NO: 136), a bridging amino acid R corresponding to amino acid 32 of D12115_P12 (SEQ. ID NO:136), a second amino acid sequence being at least about 90% homologous to amino acids 33 - 714 of NP_001701 (SEQ. ID NO:133), which also corresponds to amino acids 33 - 714 of D12115_P12 (SEQ. ID NO:136), and a third amino acid sequence being at least about 70%, optionally at least about 80%, preferably at least about 85%, more preferably at least about 90% and most preferably at least about 95% homologous to a polypeptide having the sequence SPPFPIWGDAKWSAWAPKQESSMHVASNSR (SEQ. ID NO: 332) corresponding to amino acids 715 - 744 of D12115_P12 (SEQ. ID NO:136), wherein said first amino acid sequence, bridging amino acid, second amino acid sequence and third amino acid sequence are contiguous and in a sequential order.

In some embodiments, this invention provides an isolated polypeptide an edge portion of D12115_P12 (SEQ. ID NO:136), comprising an amino acid sequence being at least about 70%, optionally at least about 80%, preferably at least about 85%, more preferably at least about 90% and most preferably at least about 95% homologous to the sequence SPPFPIWGDAKWSAWAPKQESSMHVASNSR (SEQ. ID NO: 332) of D12115_P12 (SEQ. ID NO:136).

In some embodiments, such isolated chimeric proteins or polypeptides comprise comprising a first amino acid sequence being at least about 90% homologous to amino acids 1 - 542 of P00751-2 (SEQ. ID NO:132), which also corresponds to amino acids 1 - 542 of D12115_P12 (SEQ. ID NO:136), and a second amino acid sequence being at least about 70%, optionally at least about 80%, preferably at least about 85%, more preferably at least about 90% and most preferably at least about 95% homologous to a polypeptide having the sequence GEKRDLEIEVVLFHPNYNINGKKEAGIPEFYDYDVALIKLKNKLKYGQTIRPICLPCTEGTTRA LRLPPTTTCQQQKEELLPAQDIKALFVSEEEKKLTRKEVYIKNGDKKGSCERDAQYAPGYDK VKDISEVVTPRFLCTGGVSPYADPNTCRGDSGGPLIVHKRSRFIQVSPPFPIWGDAKWSAWAP KQESSMHVASNSR (SEQ. ID NO: 333) corresponding to amino acids 543 - 744 of D12115_P12 (SEQ. ID NO:136), wherein said first amino acid sequence and second amino acid sequence are contiguous and in a sequential order.

In some embodiments, this invention provides an isolated polypeptide comprising an edge portion of D12115_P12 (SEQ. ID NO:136), comprising an amino acid sequence being at least about 70%, optionally at least about 80%, preferably at least about 85%, more preferably at least about 90% and most preferably at least about 95% homologous to the sequence -

GEKRDLEIEVVLFHPNYNINGKKEAGIPEFYDYDVALIKLKNKLKYGQTIRPICLPCTEGTTRA LRLPPTTTCQQQKEELLPAQDIKALFVSEEEKKLTRKEVYIKNGDKKGSCERDAQYAPGYDK VKDISEVVTPRFLCTGGVSPYADPNTCRGDSGGPLIVHKRSRFIQVSPPFPIWGDAKWSAWAP KQESSMHVASNSR (SEQ. ID NO: 333) of D12115_P12 (SEQ. ID NO:136).

In some embodiments, the isolated chimeric proteins or polypeptides of the invention comprise an amino acid sequence corresponding to or homologous to D12115_P13 (SEQ. ID NO:137).

In some embodiments, such isolated chimeric proteins or polypeptides comprise a first amino acid sequence being at least about 90% homologous to amino acids 1 - 618 of CFAB_HUMAN (SEQ. ID NO: 395), which also corresponds to amino acids 1 - 618 of D12115_P13 (SEQ. ID NO:137), and a second amino acid sequence being at least about 70%, optionally at least about 80%, preferably at least about 85%, more preferably at least about 90% and most preferably at least about 95% homologous to a polypeptide having the sequence RRAAPCTGYQSSVCV (SEQ. ID NO: 334) corresponding to amino acids 619 - 633 of D12115_P13 (SEQ. ID NO:137), wherein said first amino acid sequence and second amino acid sequence are contiguous and in a sequential order.

In some embodiments, this invention provides an isolated polypeptide comprising an edge portion of D12115_P13 (SEQ. ID NO:137), comprising an amino acid sequence being at least about 70%, optionally at least about 80%, preferably at least about 85%, more preferably at least about 90% and most preferably at least about 95% homologous to the sequence RRAAPCTGYQSSVCV (SEQ. ID NO: 334) of D12115_P13 (SEQ. ID NO:137).

In some embodiments, such isolated chimeric proteins or polypeptides comprise a first amino acid sequence being at least about 90% homologous to amino acids 1 - 31 of NP_001701 (SEQ. ID NO:133), which also corresponds to amino acids 1 - 31 of D12115_P13 (SEQ. ID NO:137), a bridging amino acid R corresponding to amino acid 32 of D12115_P13 (SEQ. ID NO:137), a second amino acid sequence being at least about 90% homologous to amino acids 33 - 618 of NP_001701 (SEQ. ID NO:133), which also corresponds to amino acids 33 - 618 of D12115_P13 (SEQ. ID NO:137), and a third amino acid sequence being at least about 70%, optionally at least about 80%, preferably at least about 85%, more preferably at least about 90% and most preferably at least about 95% homologous to a polypeptide having the sequence RRAAPCTGYQSSVCV (SEQ. ID NO: 334) corresponding to amino acids 619 - 633 of D12115_P13 (SEQ. ID NO:137), wherein said first amino acid sequence, bridging amino acid, second amino acid sequence and third amino acid sequence are contiguous and in a sequential order.

In some embodiments, this invention provides an isolated polypeptide comprising an edge portion of D12115_P13 (SEQ. ID NO:137), comprising an amino acid sequence being at least about 70%, optionally at least about 80%, preferably at least about 85%, more preferably at least about 90% and most preferably at least about 95% homologous to the sequence RRAAPCTGYQSSVCV (SEQ. ID NO: 334) of D12115_P13 (SEQ. ID NO:137).

In some embodiments, such isolated chimeric proteins or polypeptides comprise a first amino acid sequence being at least about 90% homologous to amino acids 1 - 542 of P00751-2 (SEQ. ID NO:132), which also corresponds to amino acids 1 - 542 of D12115_P13 (SEQ. ID NO:137), and a second amino acid sequence being at least about 70%, optionally at least about 80%, preferably at least about 85%, more preferably at least about 90% and most preferably at least about 95% homologous to a polypeptide having the sequence GEKRDLEIEVVLFHPNYNINGKKEAGIPEFYDYDVALIKLKNKLKYGQTIRPICLPCTEGTTRA LRLPPTTTCQQQRRAAPCTGYQSSVCV (SEQ. ID NO: 335) corresponding to amino acids 543 - 633 of D12115_P13 (SEQ. ID NO:137), wherein said first amino acid sequence and second amino acid sequence are contiguous and in a sequential order.

In some embodiments, this invention provides an isolated polypeptide comprising an edge portion of D12115_P13 (SEQ. ID NO:137), comprising an amino acid sequence being at least about 70%, optionally at least about 80%, preferably at least about 85%, more preferably at least about 90% and most preferably at least about 95% homologous to the sequence -

GEKRDLEIEVVLFHPNYNINGKKEAGIPEFYDYDVALIKLKNKLKYGQTIRPICLPCTEGTTRA LRLPPTTTCQQQRRAAPCTGYQSSVCV (SEQ. ID NO: 335) of D12115_P13 (SEQ. ID NO:137).

In some embodiments, the isolated chimeric proteins or polypeptides of the invention comprise an amino acid sequence corresponding to or homologous to D12115_P15 (SEQ. ID NO:138).

In some embodiments, such isolated chimeric proteins or polypeptides comprise a first amino acid sequence being at least about 90% homologous to amino acids 1 - 593 of CFAB_HUMAN (SEQ. ID NO: 395), which also corresponds to amino acids 1 - 593 of D12115_P15 (SEQ. ID NO:138), and a second amino acid sequence being at least about 70%, optionally at least about 80%, preferably at least about 85%, more preferably at least about 90% and most preferably at least about 95% homologous to a polypeptide having the sequence GRAAPCTGYQSSVCV (SEQ. ID NO: 336) corresponding to amino acids 594 - 608 of D12115_P15 (SEQ. ID NO:138), wherein said first amino acid sequence and second amino acid sequence are contiguous and in a sequential order.

In some embodiments, this invention provides an isolated polypeptide comprising an edge portion of D12115_P15 (SEQ. ID NO:138), comprising an amino acid sequence being at least about 70%, optionally at least about 80%, preferably at least about 85%, more preferably at least about 90% and most preferably at least about 95% homologous to the sequence GRAAPCTGYQSSVCV (SEQ. ID NO: 336) of D12115_P15 (SEQ. ID NO:138).

In some embodiments, such isolated chimeric proteins or polypeptides comprise a first amino acid sequence being at least about 90% homologous to amino acids 1 - 31 of NP_001701 (SEQ. ID NO:133), which also corresponds to amino acids 1 - 31 of D12115_P15 (SEQ. ID NO:138), a bridging amino acid R corresponding to amino acid 32 of D12115_P15 (SEQ. ID NO:138), a second amino acid sequence being at least about 90% homologous to amino acids 33 - 593 of NP_001701 (SEQ. ID NO:133), which also corresponds to amino acids 33 - 593 of D12115_P15 (SEQ. ID NO:138), and a third amino acid sequence being at least about 70%, optionally at least about 80%, preferably at least about 85%, more preferably at least about 90% and most preferably at least about 95% homologous to a polypeptide having the sequence GRAAPCTGYQSSVCV (SEQ. ID NO: 336) corresponding to amino acids 594 - 608 of D12115_P15 (SEQ. ID NO:138), wherein said first amino acid sequence, bridging amino acid, second amino acid sequence and third amino acid sequence are contiguous and in a sequential order.

In some embodiments, this invention provides an isolated polypeptide an edge portion of D12115_P15 (SEQ. ID NO:138), comprising an amino acid sequence being at least about 70%, optionally at least about 80%, preferably at least about 85%, more preferably at least about 90% and most preferably at least about 95% homologous to the sequence GRAAPCTGYQSSVCV (SEQ. ID NO: 336) of D12115_P15 (SEQ. ID NO:138).

In some embodiments, such isolated chimeric proteins or polypeptides comprise a first amino acid sequence being at least about 90% homologous to amino acids 1 - 542 of P00751-2 (SEQ. ID NO:132), which also corresponds to amino acids 1 - 542 of D12115_P15 (SEQ. ID NO:138), and a second amino acid sequence being at least about 70%, optionally at least about 80%, preferably at least about 85%, more preferably at least about 90% and most preferably at least about 95% homologous to a polypeptide having the sequence GEKRDLEIEVVLFHPNYNINGKKEAGIPEFYDYDVALIKLKNKLKYGQTIRGRAAPCTGYQSS VCV (SEQ. ID NO: 337) corresponding to amino acids 543 - 608 of D12115_P15 (SEQ. ID NO:138), wherein said first amino acid sequence and second amino acid sequence are contiguous and in a sequential order.

In some embodiments, this invention provides an isolated polypeptide comprising an edge portion of D12115_P15 (SEQ. ID NO:138), comprising an amino acid sequence being at least about 70%, optionally at least about 80%, preferably at least about 85%, more preferably at least about 90% and most preferably at least about 95% homologous to the sequence -

GEKRDLEIEVVLFHPNYNINGKKEAGIPEFYDYDVALIKLKNKLKYGQTIRGRAAPCTGYQSS VCV (SEQ. ID NO: 337) of D12115_P15 (SEQ. ID NO:138).

In some embodiments, the isolated chimeric proteins or polypeptides of the invention comprise an amino acid sequence corresponding to or homologous to D12115_P16 (SEQ. ID NO:139). In some embodiments, such isolated chimeric proteins or polypeptides comprise a first amino acid sequence being at least about 90% homologous to amino acids 1 - 652 of CFAB_HUMAN (SEQ. ID NO: 395), which also corresponds to amino acids 1 - 652 of D12115_P16 (SEQ. ID NO:139), and a second amino acid sequence being at least about 70%, optionally at least about 80%, preferably at least about 85%, more preferably at least about 90% and most preferably at least about 95% homologous to a polypeptide having the sequence VRNGHPKEAL (SEQ. ID NO: 338) corresponding to amino acids 653 - 662 of D12115_P16 (SEQ. ID NO:139), wherein said first amino acid sequence and second amino acid sequence are contiguous and in a sequential order.

In some embodiments, this invention provides an isolated polypeptide comprising an edge portion of D12115_P16 (SEQ. ID NO:139), comprising an amino acid sequence being at least about 70%, optionally at least about 80%, preferably at least about 85%, more preferably at least about 90% and most preferably at least about 95% homologous to the sequence VRNGHPKEAL (SEQ. ID NO: 338) of D12115_P16 (SEQ. ID NO:139).

In some embodiments, such isolated chimeric proteins or polypeptides comprise a first amino acid sequence being at least about 90% homologous to amino acids 1 - 31 of NP_001701 (SEQ. ID NO:133), which also corresponds to amino acids 1 - 31 of D12115_P16 (SEQ. ID NO:139), a bridging amino acid R corresponding to amino acid 32 of D12115_P16 (SEQ. ID NO:139), a second amino acid sequence being at least about 90% homologous to amino acids 33 - 652 of NP_001701 (SEQ. ID NO:133), which also corresponds to amino acids 33 - 652 of D12115_P16 (SEQ. ID NO:139), and a third amino acid sequence being at least about 70%, optionally at least about 80%, preferably at least about 85%, more preferably at least about 90% and most preferably at least about 95% homologous to a polypeptide having the sequence VRNGHPKEAL (SEQ. ID NO: 338) corresponding to amino acids 653 - 662 of D12115_P16 (SEQ. ID NO:139), wherein said first amino acid sequence, bridging amino acid, second amino acid sequence and third amino acid sequence are contiguous and in a sequential order.

In some embodiments, this invention provides an isolated polypeptide comprising an edge portion of D12115_P16 (SEQ. ID NO:139), comprising an amino acid sequence being at least about 70%, optionally at least about 80%, preferably at least about 85%, more preferably at least about 90% and most preferably at least about 95% homologous to the sequence VRNGHPKEAL (SEQ. ID NO: 338) of D12115_P16 (SEQ. ID NO:139).

In some embodiments, such isolated chimeric proteins or polypeptides comprise a first amino acid sequence being at least about 90% homologous to amino acids 1 - 542 of P00751-2 (SEQ. ID NO:132), which also corresponds to amino acids 1 - 542 of D12115_P16 (SEQ. ID NO:139), and a second amino acid sequence being at least about 70%, optionally at least about 80%, preferably at least about 85%, more preferably at least about 90% and most preferably at least about 95% homologous to a polypeptide having the sequence GEKRDLEIEVVLFHPNYNINGKKEAGIPEFYDYDVALIKLKNKLKYGQTIRPICLPCTEGTTRA LRLPPTTTCQQQKEELLPAQDIKALFVSEEEKKLTRKEVYIKNGDKVRNGHPKEAL (SEQ. ID NO: 339) corresponding to amino acids 543 - 662 of D12115_P16 (SEQ. ID NO:139), wherein said first amino acid sequence and second amino acid sequence are contiguous and in a sequential order.

In some embodiments, this invention provides an isolated polypeptide comprising an edge portion of D12115_P16 (SEQ. ID NO:139), comprising an amino acid sequence being at least about 70%, optionally at least about 80%, preferably at least about 85%, more preferably at least about 90% and most preferably at least about 95% homologous to the sequence -

GEKRDLEIEVVLFHPNYNINGKKEAGIPEFYDYDVALIKLKNKLKYGQTIRPICLPCTEGTTRA LRLPPTTTCQQQKEELLPAQDIKALFVSEEEKKLTRKEVYIKNGDKVRNGHPKEAL (SEQ. ID NO: 339) of D12115_P16 (SEQ. ID NO:139).

In some embodiments, the isolated chimeric proteins or polypeptides of the invention comprise an amino acid sequence corresponding to or homologous to D12115_P20 (SEQ. ID NO:140).

In some embodiments, such isolated chimeric proteins or polypeptides comprise a first amino acid sequence being at least about 90% homologous to amino acids 1 - 541 of CFAB_HUMAN (SEQ. ID NO: 395), which also corresponds to amino acids 1 - 541 of D12115_P20 (SEQ. ID NO:140), a second bridging amino acid sequence comprising of E, and a third amino acid sequence being at least about 90% homologous to amino acids 620 - 764 of CFAB_HUMAN (SEQ. ID NO: 395), which also corresponds to amino acids 543 - 687 of D12115_P20 (SEQ. ID NO:140), wherein said first amino acid sequence, second amino acid sequence and third amino acid sequence are contiguous and in a sequential order.

In some embodiments, this invention provides an isolated polypeptide comprising an edge portion of D12115_P20 (SEQ. ID NO:140), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least 3 amino acids comprise VEE having a structure as follows (numbering according to D12115_P20 (SEQ. ID NO:140)): a sequence starting from any of amino acid numbers 541-x to 541; and ending at any of amino acid numbers 543 + ((n-3) - x), in which x varies from 0 to n-3.

In some embodiments, such isolated chimeric proteins or polypeptides comprise a first amino acid sequence being at least about 90% homologous to amino acids 1 - 31 of NP_001701 (SEQ. ID NO:133), which also corresponds to amino acids 1 - 31 of D12115_P20 (SEQ. ID NO:140), a bridging amino acid R corresponding to amino acid 32 of D12115_P20 (SEQ. ID NO:140), a second amino acid sequence being at least about 90% homologous to amino acids 33 - 541 of NP_001701 (SEQ. ID NO:133), which also corresponds to amino acids 33 - 541 of D12115_P20 (SEQ. ID NO:140), a third bridging amino acid sequence comprising of E, and a fourth amino acid sequence being at least about 90% homologous to amino acids 620 - 764 of NP_001701 (SEQ. ID NO:133), which also corresponds to amino acids 543 - 687 of D12115_P20 (SEQ. ID NO:140), wherein said first amino acid sequence, bridging amino acid, second amino acid sequence, third amino acid sequence and fourth amino acid sequence are contiguous and in a sequential order.

In some embodiments, such isolated chimeric proteins or polypeptides comprise a first amino acid sequence being at least about 90% homologous to amino acids 1 - 541 of P00751-2 (SEQ. ID NO:132), which also corresponds to amino acids 1 - 541 of D12115_P20 (SEQ. ID NO:140), and a second amino acid sequence being at least about 70%, optionally at least about 80%, preferably at least about 85%, more preferably at least about 90% and most preferably at least about 95% homologous to a polypeptide having the sequence EEELLPAQDIKALFVSEEEKKLTRKEVYIKNGD-KKGSCERDAQYAPGYDKVKDISEVVTPRFL CTGGVSPYADPNTCRGDSGGPLIVHKRSRFIQVGVISWGVVDVCKN-QKRQKQVPAHARDFHI NLFQVLPWLKEKLQDEDLGFL (SEQ. ID NO: 340) corresponding to amino acids 542 - 687 of D12115_P20 (SEQ. ID NO:140), wherein said first amino acid sequence and second amino acid sequence are contiguous and in a sequential order.

In some embodiments, this invention provides an isolated polypeptide comprising an edge portion of D12115_P20 (SEQ. ID NO:140), comprising an amino acid sequence being at least about 70%, optionally at least about 80%, preferably at least about 85%, more preferably at least about 90% and most preferably at least about 95% homologous to the sequence -

EEELLPAQDIKALFVSEEEKKLTRKEVYIKNGDKKGSCERDAQYAPGYDKVKDISEVVTPRFL
CTGGVSPYADPNTCRGDSGGPLIVHKRSRFIQVGVISWGVVDVCKNQKRQKQVPAHARDFHI
NLFQVLPWLKEKLQDEDLGFL (SEQ. ID NO: 340) of D12115_P20 (SEQ. ID NO:140).

In some embodiments, the isolated chimeric proteins or polypeptides of the invention comprise an amino acid sequence corresponding to or homologous to D12115_P32 (SEQ. ID NO:141). In some embodiments, such isolated chimeric proteins or polypeptides comprise a first amino acid sequence being at least about 90% homologous to amino acids 1 - 469 of CFAB_HUMAN (SEQ. ID NO: 395), which also corresponds to amino acids 1 - 469 of D12115_P32 (SEQ. ID NO:141), and a second amino acid sequence being at least about 70%, optionally at least about 80%, preferably at least about 85%, more preferably at least about 90% and most preferably at least about 95% homologous to a polypeptide having the sequence GREIQGNKEHNS (SEQ. ID NO: 341) corresponding to amino acids 470 - 481 of D12115_P32 (SEQ. ID NO:141), wherein said first amino acid sequence and second amino acid sequence are contiguous and in a sequential order.

In some embodiments, this invention provides an isolated polypeptide comprising an edge portion of D12115_P32 (SEQ. ID NO:141), comprising an amino acid sequence being at least about 70%, optionally at least about 80%, preferably at least about 85%, more preferably at least about 90% and most preferably at least about 95% homologous to the sequence GREIQGNKEHNS (SEQ. ID NO: 341) of D12115_P32 (SEQ. ID NO:141).

In some embodiments, such isolated chimeric proteins or polypeptides comprise a first amino acid sequence being at least about 90% homologous to amino acids 1 - 469 of P00751-2 (SEQ. ID NO:132), which also corresponds to amino acids 1 - 469 of D12115_P32 (SEQ. ID NO:141), and a second amino acid sequence being at least about 70%, optionally at least about 80%, preferably at least about 85%, more preferably at least about 90% and most preferably at least about 95% homologous to a polypeptide having the sequence GREIQGNKEHNS (SEQ. ID NO: 341) corresponding to amino acids 470 - 481 of D12115_P32 (SEQ. ID NO:141), wherein said first amino acid sequence and second amino acid sequence are contiguous and in a sequential order.

In some embodiments, this invention provides an isolated polypeptide comprising an edge portion of D12115_P32 (SEQ. ID NO:141), comprising an amino acid sequence being at least about 70%, optionally at least about 80%, preferably at least about 85%, more preferably at least about 90% and most preferably at least about 95% homologous to the sequence GREIQGNKEHNS (SEQ. ID NO: 341) of D12115_P32 (SEQ. ID NO:141).

In some embodiments, such isolated chimeric proteins or polypeptides comprise a first amino acid sequence being at least about 90% homologous to amino acids 1 - 31 of NP_001701 (SEQ. ID NO:133), which also corresponds to amino acids 1 - 31 of D12115_P32 (SEQ. ID NO:141), a bridging amino acid R corresponding to amino acid 32 of D12115_P32 (SEQ. ID NO:141), a second amino acid sequence being at least about 90% homologous to amino acids 33 - 469 of NP_001701 (SEQ. ID NO:133), which also corresponds to amino acids 33 - 469 of D12115_P32 (SEQ. ID NO:141), and a third amino acid sequence being at least about 70%, optionally at least about 80%, preferably at least about 85%, more preferably at least about 90% and most preferably at least about 95% homologous to a polypeptide having the sequence GREIQGNKEHNS (SEQ. ID NO: 341) corresponding to amino acids 470 - 481 of D12115_P32 (SEQ. ID NO:141), wherein said first amino acid sequence, bridging amino acid, second amino acid sequence and third amino acid sequence are contiguous and in a sequential order.

In some embodiments, this invention provides an isolated polypeptide comprising an edge portion of D12115_P32 (SEQ. ID NO:141), comprising an amino acid sequence being at least about 70%, optionally at least about 80%, preferably at least about 85%, more preferably at least about 90% and most preferably at least about 95% homologous to the sequence GREIQGNKEHNS (SEQ. ID NO: 341) of D12115_P32 (SEQ. ID NO:141).

According to a further embodiment, the present invention now discloses a novel cluster designated herein C03950, comprising novel amino acid and nucleic acid sequences that are variants of the known protein Serine/threonine-protein kinase TNNI3K (SwissProt accession identifier TNI3K_HUMAN (SEQ. ID NO: 396).

The novel polynucleotides and polypeptides described by the present invention are useful as diagnostic markers, preferably as serum markers.

Surprisingly, the present invention now shows that the C03950 variants are expressed specifically in heart tissue, and thus can indicate the onset, severity or prognosis of cardiovascular disease in a subject, and can be used for the selection of treatment, treatment monitoring, diagnosis or prognosis assessment of any cardiovascular disease, including, inter alia, myocardial infarct, acute coronary syndrome, coronary artery disease, angina pectoris (stable and unstable), cardiomyopathy, myocarditis, congestive heart failure or any type of heart failure, reinfarction, assessment of thrombolytic therapy, assessment of myocardial infarct size, differential diagnosis between heart-related versus lung-related conditions (such as pulmonary embolism), the differential diagnosis of Dyspnea, cardiac valves related conditions, vascular disease, or any combination thereof, as is described in a greater detail below.

In some embodiments, the isolated chimeric proteins or polypeptides of the invention comprise an amino acid sequence corresponding to or homologous to C03950_3_P5 (SEQ. ID NO:212).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence AVRRGLREGGA (SEQ. ID NO: 342) corresponding to amino acids 1 - 11 of C03950_3_P5 (SEQ. ID NO:212), a second amino acid sequence being at least about 90% homologous to amino acids 1 - 691 of TNI3K_HUMAN (SEQ. ID NO: 396), which also corresponds to amino acids 12 - 702 of C03950_3_P5 (SEQ. ID NO:212), a third amino acid sequence being at least about 70%, optionally at least about 80%, preferably at least about 85%, more preferably at least about 90% and most preferably at least about 95%, homologous to a polypeptide having the sequence RSAITSRIWITHSICIWRGAHYFNREECNFRCMLTSAILK corresponding to amino acids 703 - 742 of C03950_3_P5 (SEQ. ID NO:212), and a fourth amino acid sequence being at least about 90% homologous to amino acids 710 - 936 of TNI3K_HUMAN (SEQ. ID NO: 396), which also corresponds to amino acids 743 - 969 of C03950_3_P5 (SEQ. ID NO:212), wherein said first amino acid sequence, second amino acid sequence, third amino acid sequence and fourth amino acid sequence are contiguous and in a sequential order.

In some embodiments, this invention provides an isolated polypeptide comprising a head of C03950_3_P5 (SEQ. ID NO:212), comprising a polypeptide being at least about 70%, optionally at least about 80%, preferably at least about 85%, more preferably at least about 90% and most preferably at least about 95% homologous to the sequence AVRRGLREGGA (SEQ. ID NO: 342) of C03950_3_P5 (SEQ. ID NO:212).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence - AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P5 (SEQ. ID NO:212), a second amino acid sequence being at least about 90% homologous to amino acids 14 - 590 of NP_057062 (SEQ. ID NO:210), which also corresponds to amino acids 126 - 702 of C03950_3_P5 (SEQ. ID NO:212), a third amino acid sequence being at least about 70%, optionally at least about 80%, preferably at least about 85%, more preferably at least about 90% and most preferably at least about 95%, homologous to a polypeptide having the sequence RSAITSRIWITHSICIWRGAHYFNREECNFRCMLTSAILK corresponding to amino acids 703 - 742 of C03950_3_P5 (SEQ. ID NO:212), and a fourth amino acid sequence being at least about 90% homologous to amino acids 609 - 835 of NP_057062 (SEQ. ID NO:210), which also corresponds to amino acids 743 - 969 of C03950_3_P5 (SEQ. ID NO:212), wherein said first amino acid sequence, second amino acid sequence, third amino acid sequence and fourth amino acid sequence are contiguous and in a sequential order.

In some embodiments, this invention provides an isolated polypeptide comprising a head of C03950_3_P5 (SEQ. ID NO:212), comprising a polypeptide being at least about 70%, optionally at least about 80%, preferably at least about 85%, more preferably at least about 90% and most preferably at least about 95% homologous to the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) of C03950_3_P5 (SEQ. ID NO:212).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence - AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P5 (SEQ. ID NO:212), a second amino acid sequence being at least about 90% homologous to amino acids 14 - 590 of Q9Y2V6_HUMAN (SEQ. ID NO:210), which also corresponds to amino acids 126 - 702 of C03950_3_P5 (SEQ. ID NO:212), a third amino acid sequence being at least about 70%, optionally at least about 80%, preferably at least about 85%, more preferably at least about 90% and most preferably at least about 95%, homologous to a polypeptide having the sequence RSAITSRIWITHSICIWRGAHYFNREECNFRCMLTSAILK corresponding to amino acids 703 - 742 of C03950_3_P5 (SEQ. ID NO:212), and a fourth amino acid sequence being at least about 90% homologous to amino acids 609 - 835 of Q9Y2V6_HUMAN (SEQ. ID NO:210), which also corresponds to amino acids 743 - 969 of C03950_3_P5 (SEQ. ID NO:212), wherein said first amino acid sequence, second amino acid sequence, third amino acid sequence and fourth amino acid sequence are contiguous and in a sequential order.

In some embodiments, this invention provides an isolated polypeptide comprising a head of C03950_3_P5 (SEQ. ID NO:212), comprising a polypeptide being at least about 70%, optionally at least about 80%, preferably at least about 85%, more preferably at least about 90% and most preferably at least about 95% homologous to the sequence AVRRGLREGGAMAAARDPPEVSLREATQRKLRRFSELRGKLVARGEFWDIVAITAADEKQEL
AYNQQLSEKLKRKELPLGVQYHVFVDPAGAKIGNGGSTLCALQCLEKLYGDKWNSFTILLIH
S (SEQ. ID NO: 343) of C03950_3_P5 (SEQ. ID NO:212).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence AVRRGLR (SEQ. ID NO: 344) corresponding to amino acids 1 - 7 of C03950_3_P5 (SEQ. ID NO:212), a second amino acid sequence being at least about 90% homologous to amino acids 14 - 367 of Q6MZS9_HUMAN (SEQ. ID NO:211), which also corresponds to amino acids 8 - 361 of C03950_3_P5 (SEQ. ID NO:212), a bridging amino acid I corresponding to amino acid 362 of C03950_3_P5 (SEQ. ID NO:212), a third amino acid sequence being at least about 90% homologous to amino acids 369 - 484 of Q6MZS9_HUMAN (SEQ. ID NO:211), which also corresponds to amino acids 363 - 478 of C03950_3_P5 (SEQ. ID NO:212), a bridging amino acid N corresponding to amino acid 479 of C03950_3_P5 (SEQ. ID NO:212), a fourth amino acid sequence being at least about 90% homologous to amino acids 486 - 709 of Q6MZS9_HUMAN (SEQ. ID NO:211), which also corresponds to amino acids 480 - 703 of C03950_3_P5 (SEQ. ID NO:212), and a fifth amino acid sequence being at least about 70%, optionally at least about 80%, preferably at least about 85%, more preferably at least about 90% and most preferably at least about 95% homologous to a polypeptide having the sequence SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEWMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) corresponding to amino acids 704 - 969 of C03950_3_P5 (SEQ. ID NO:212), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising a head of C03950_3_P5 (SEQ. ID NO:212), comprising a polypeptide being at least about 70%, optionally at least about 80%, preferably at least about 85%, more preferably at least about 90% and most preferably at least about 95% homologous to the sequence AVRRGLR (SEQ. ID NO: 344) of C03950_3_P5 (SEQ. ID NO:212).

In some embodiments, this invention provides an isolated polypeptide comprising an edge portion of C03950_3_P5 (SEQ. ID NO:212), comprising an amino acid sequence being at least about 70%, optionally at least about 80%, preferably at least about 85%, more preferably at least about 90% and most preferably at least about 95% homologous to the sequence -

SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEVVMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) of C03950_3_P5 (SEQ. ID NO:212).

In some embodiments, the isolated chimeric proteins or polypeptides of the invention comprise an amino acid sequence corresponding to or homologous to C03950_3_P7 (SEQ. ID NO:213).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P7 (SEQ. ID NO:213), a second amino acid sequence being at least about 90% homologous to amino acids 115 - 691 of TNI3K_HUMAN (SEQ. ID NO: 396), which also corresponds to amino acids 14 - 590 of C03950_3_P7 (SEQ. ID NO:213), a third amino acid sequence being at least about 70%, optionally at least about 80%, preferably at least about 85%, more preferably at least about 90% and most preferably at least about 95%, homologous to a polypeptide having the sequence RSAITSRIWITHSICIWRGAHYFNREECNFRCMLTSAILK corresponding to amino acids 591 - 630 of C03950_3_P7 (SEQ. ID NO:213), and a fourth amino acid sequence being at least about 90% homologous to amino acids 710 - 936 of TNI3K_HUMAN (SEQ. ID NO: 396), which also corresponds to amino acids 631 - 857 of C03950_3_P7 (SEQ. ID NO:213), wherein said first amino acid sequence, second amino acid sequence, third amino acid sequence and fourth amino acid sequence are contiguous and in a sequential order.

In some embodiments, this invention provides an isolated polypeptide comprising a head of C03950_3_P7 (SEQ. ID NO:213), comprising a polypeptide being at least about 70%, optionally at least about 80%, preferably at least about 85%, more preferably at least about 90% and most preferably at least about 95% homologous to the sequence MGNYKSRPTQTCT (SEQ. ID NO: 346) of C03950_3_P7 (SEQ. ID NO:213).

In some embodiments, such isolated chimeric proteins or polypeptides comprise a first amino acid sequence being at least about 90% homologous to amino acids 1 - 590 of NP_057062 (SEQ. ID NO:210), which also corresponds to amino acids 1 - 590 of C03950_3_P7 (SEQ. ID NO:213), a second amino acid sequence being at least about 70%, optionally at least about 80%, preferably at least about 85%, more preferably at least about 90% and most preferably at least about 95%, homologous to a polypeptide having the sequence RSAITSRIWITHSICIWRGAHYFNREECNFRCMLTSAILK corresponding to amino acids 591 - 630 of C03950_3_P7 (SEQ. ID NO:213), and a third amino acid sequence being at least about 90% homologous to amino acids 609 - 835 of NP_057062 (SEQ. ID NO:210), which also corresponds to amino acids 631 - 857 of C03950_3_P7 (SEQ. ID NO:213), wherein said first amino acid sequence, second amino acid sequence and third amino acid sequence are contiguous and in a sequential order.

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P7 (SEQ. ID NO:213), a second amino acid sequence being at least about 90% homologous to amino acids 132 - 367 of Q6MZS9_HUMAN (SEQ. ID NO:211), which also corresponds to amino acids 14 - 249 of C03950_3_P7 (SEQ. ID NO:213), a bridging amino acid I corresponding to amino acid 250 of C03950_3_P7 (SEQ. ID NO:213), a third amino acid sequence being at least about 90% homologous to amino acids 369 - 484 of Q6MZS9_HUMAN (SEQ. ID NO:211), which also corresponds to amino acids 251 - 366 of C03950_3_P7 (SEQ. ID NO:213), a bridging amino acid N corresponding to amino acid 367 of C03950_3_P7 (SEQ. ID NO:213), a fourth amino acid sequence being at least about 90% homologous to amino acids 486 - 709 of Q6MZS9_HUMAN (SEQ. ID NO:211), which also corresponds to amino acids 368 - 591 of C03950_3_P7 (SEQ. ID NO: 213), and a fifth amino acid sequence being at least about 70%, optionally at least about 80%, preferably at least about 85%, more preferably at least about 90% and most preferably at least about 95% homologous to a polypeptide having the sequence SAITSRIWITHSICIWRGAHYFNREECNFRCMLTSAILKESRFLQSLDEDNMTKQPGNLRWMA PEVFT QCTRYTIKADVFSYALCLWEILTGEIPFAHLKPAAAAADMAYHHIRPPIGYSIPKPISSL LIRGWNACPEGRPEFSEVVMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) corresponding to amino acids 592 - 857 of C03950_3_P7 (SEQ. ID NO:213), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising a head of C03950_3_P7 (SEQ. ID NO:213), comprising a polypeptide being at least about 70%, optionally at least about 80%, preferably at least about 85%, more preferably at least about 90% and most preferably at least about 95% homologous to the sequence MGNYKSRPTQTCT (SEQ. ID NO: 346) of C03950_3_P7 (SEQ. ID NO:213).

In some embodiments, this invention provides an isolated polypeptide comprising an edge portion of C03950_3_P7 (SEQ. ID NO:213), comprising an amino acid sequence being at least about 70%, optionally at least about 80%, preferably at least about 85%, more preferably at least about 90% and most preferably at least about 95% homologous to the sequence -

SAITSRIWITHSICIWRGAHYFNREECNFRCMLTSAILKESRFLQSLDEDNMTKQPGNLRWMA
PEVFTQCTRYTIKADVFSYALCLWEILTGEIPFAHLKPAAAAADMAYHHIRPPIGYSIPKPISSL
LIRGWNACPEGRPEFSEVVMKLEECLCNIELMSPASSNSSGSLSPSSSSDCLVNRGGPGRSHVA
ALRSRFELEYALNARSYAALSQSAGQYSSQGLSLEEMKRSLQYTPIDKYGYVSDPMSSMHFH
SCRNSSSFEDSS (SEQ. ID NO: 345) of C03950_3_P7 (SEQ. ID NO:213).

In some embodiments, the isolated chimeric proteins or polypeptides of the invention comprise an amino acid sequence corresponding to or homologous to C03950_3_P9 (SEQ. ID NO:214).In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P9 (SEQ. ID NO:214), a second amino acid sequence being at least about 90% homologous to amino acids 115 - 911 of TNI3K_HUMAN (SEQ. ID NO: 396), which also corresponds to amino acids 14 - 810 of C03950_3_P9 (SEQ. ID NO:214), and a third amino acid sequence being at least about 70%, optionally at least about 80%, preferably at least about 85%, more preferably at least about 90% and most preferably at least about 95% homologous to a polypeptide having the sequence DVTS (SEQ. ID NO: 349) corresponding to amino acids 811 - 814 of C03950_3_P9 (SEQ. ID NO:214), wherein said first amino acid sequence, second amino acid sequence and third amino acid sequence are contiguous and in a sequential order.

In some embodiments, this invention provides an isolated polypeptide comprising a head of C03950_3_P9 (SEQ. ID NO:214), comprising a polypeptide being at least about 70%, optionally at least about 80%, preferably at least about 85%, more preferably at least about 90% and most preferably at least about 95% homologous to the sequence MGNYKSRPTQTCT (SEQ. ID NO: 346) of C03950_3_P9 (SEQ. ID NO:214).

In some embodiments, this invention provides an isolated polypeptide comprising an edge portion of C03950_3_P9 (SEQ. ID NO:214), comprising an amino acid sequence being at least about 70%, optionally at least about 80%, preferably at least about 85%, more preferably at least about 90% and most preferably at least about 95% homologous to the sequence DVTS (SEQ. ID NO: 349) of C03950_3_P9 (SEQ. ID NO:214).

In some embodiments, such isolated chimeric proteins or polypeptides comprise a first amino acid sequence being at least about 90% homologous to amino acids 1 - 810 of NP_057062 (SEQ. ID NO:210), which also corresponds to amino acids 1 - 810 of C03950_3_P9 (SEQ. ID NO:214), and a second amino acid sequence being at least about 70%, optionally at least about 80%, preferably at least about 85%, more preferably at least about 90% and most preferably at least about 95% homologous to a polypeptide having the sequence DVTS (SEQ. ID NO: 349) corresponding to amino acids 811 - 814 of C03950_3_P9 (SEQ. ID NO:214), wherein said first amino acid sequence and second amino acid sequence are contiguous and in a sequential order.

In some embodiments, such isolated chimeric proteins or polypeptides comprise a first amino acid sequence being at least about 90% homologous to amino acids 1 - 810 of Q9Y2V6_HUMAN (SEQ. ID NO:210), which also corresponds to amino acids 1 - 810 of C03950_3_P9 (SEQ. ID NO:214), and a second amino acid sequence being at least about 70%, optionally at least about 80%, preferably at least about 85%, more preferably at least about 90% and most preferably at least about 95% homologous to a polypeptide having the sequence DVTS (SEQ. ID NO: 349) corresponding to amino acids 811 - 814 of C03950_3_P9 (SEQ. ID NO:214), wherein said first amino acid sequence and second amino acid sequence are contiguous and in a sequential order.

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P9 (SEQ. ID NO:214), a second amino acid sequence being at least about 90% homologous to amino acids 132 - 367 of Q6MZS9_HUMAN (SEQ. ID NO:211), which also corresponds to amino acids 14 - 249 of C03950_3_P9 (SEQ. ID NO:214), a bridging amino acid I corresponding to amino acid 250 of C03950_3_P9 (SEQ. ID NO:214), a third amino acid sequence being at least about 90% homologous to amino acids 369 - 484 of Q6MZS9_HUMAN (SEQ. ID NO:211), which also corresponds to amino acids 251 - 366 of C03950_3_P9 (SEQ. ID NO:214), a bridging amino acid N corresponding to amino acid 367 of C03950_3_P9 (SEQ. ID NO:214), a fourth amino acid sequence being at least about 90% homologous to amino acids 486 - 708 of Q6MZS9_HUMAN (SEQ. ID NO:211), which also corresponds to amino acids 368 - 590 of C03950_3_P9 (SEQ. ID NO: 214), and a fifth amino acid sequence being at least about 70%, optionally at least about 80%, preferably at least about 85%, more preferably at least about 90% and most preferably at least about 95% homologous to a polypeptide having the sequence SHNILLYEDGHAVVADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEGRPEFSEVVM KLEECLCNIELMSPASSNSSGSLSPSSSSDCLVNRGGPGRSHVAALRSRFELEYALNARSYAAL SQSAGQYSSQGLSLEEMKRSLQYTPIDKYDVTS (SEQ. ID NO: 350) corresponding to amino acids 591 - 814 of C03950_3_P9 (SEQ. ID NO:214), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising a head of C03950_3_P9 (SEQ. ID NO:214), comprising a polypeptide being at least about 70%, optionally at least about 80%, preferably at least about 85%, more preferably at least about 90% and most preferably at least about 95% homologous to the sequence MGNYKSRPTQTCT (SEQ. ID NO: 346) of C03950_3_P9 (SEQ. ID NO:214).

In some embodiments, this invention provides an isolated polypeptide comprising an edge portion of C03950_3_P9 (SEQ. ID NO:214), comprising an amino acid sequence being at least about 70%, optionally at least about 80%, preferably at least about 85%, more preferably at least about 90% and most preferably at least about 95% homologous to the sequence SHNILLYEDGHAVVADFGESRFLQSLDEDNMTKQPGNLRWMAPEVFTQCTRYTIKAD-VFSYA LCLWEILTGEIPFAHLKPAAAAADMAYHHIRPPIGYSIPKPISSLLIRGWNACPEGRPEFSEVVM KLEECLCNI-ELMSPASSNSSGSLSPSSSSDCLVNRGGPGRSHVAALRSRFELEYALNARSYAAL SQSAGQYSSQGLSLEEMKRSL-QYTPIDKYDVTS (SEQ. ID NO: 350) of C03950_3_P9 (SEQ. ID NO:214).

In some embodiments, the isolated chimeric proteins or polypeptides of the invention comprise an amino acid sequence corresponding to or homologous to C03950_3_P10 (SEQ. ID NO:215).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence PSPCGLHFLIPWLTQ (SEQ. ID NO: 351) corresponding to amino acids 1 - 15 of C03950_3_P10 (SEQ. ID NO:215), and a second amino acid sequence being at least about 90% homologous to amino acids 213 - 936 of TNI3K_HUMAN (SEQ. ID NO: 396), which also corresponds to amino acids 16 - 739 of C03950_3-P10 (SEQ. ID NO:215), wherein said first amino acid sequence and second amino acid sequence are contiguous and in a sequential order.

In some embodiments, this invention provides an isolated polypeptide comprising a head of C03950_3_P10 (SEQ. ID NO:215), comprising a polypeptide being at least about 70%, optionally at least about 80%, preferably at least about 85%, more preferably at least about 90% and most preferably at least about 95% homologous to the sequence PSPCGLHFLIPWLTQ (SEQ. ID NO: 351) of C03950_3_P10 (SEQ. ID NO:215).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence PSPCGLHFLIPWLTQ (SEQ. ID NO: 351) corresponding to amino acids 1 - 15 of C03950_3_PIO (SEQ. ID NO:215), a second amino acid sequence being at least about 90% homologous to amino acids 230 - 367 of Q6MZS9_HUMAN (SEQ. ID NO:211), which also corresponds to amino acids 16 - 153 of C03950_3_P10 (SEQ. ID NO:215), a bridging amino acid I corresponding to amino acid 154 of C03950_3_P10 (SEQ. ID NO:215), a third amino acid sequence being at least about 90% homologous to amino acids 369 - 484 of Q6MZS9_HUMAN (SEQ. ID NO:211), which also corresponds to amino acids 155 - 270 of C03950_3_P10 (SEQ. ID NO:215), a bridging amino acid N corresponding to amino acid 271 of C03950_3_P10 (SEQ. ID NO:215), a fourth amino acid sequence being at least about 90% homologous to amino acids 486 - 708 of Q6MZS9_HUMAN (SEQ. ID NO:211), which also corresponds to amino acids 272 - 494 of C03950_3_P10 (SEQ. ID NO:215), and a fifth amino acid sequence being at least about 70%, optionally at least about 80%, preferably at least about 85%, more preferably at least about 90% and most preferably at least about 95% homologous to a polypeptide having the sequence SHNILLYEDGHAVVADFGESRFLQSLDEDNMTKQPGNLRWMAPEVFTQCTRY-TIKADVFSYA LCLWEILTGEIPFAHLKPAAAAADMAYHHIRPPIGYSIPKPISSLLIRGWNACPEGRPEFSEVVM KLEE-CLCNIELMSPASSNSSGSLSPSSSSDCLVNRGGPGRSHVAALRSRFELEYALNARSYAAL SQSAGQYSSQGLSLEEMKRSLQYTPIDKYGYVSDPMSSMHFHSCRNSS(SEQ. ID NO: 352) corresponding to amino acids 495 - 739 of C03950_3_P10 (SEQ. ID NO:215), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising an edge portion of C03950_3_P10 (SEQ. ID NO:215), comprising an amino acid sequence being at least about 70%, optionally at least about 80%, preferably at least about 85%, more preferably at least about 90% and most preferably at least about 95% homologous to the sequence -

SHNILLYEDGHAVVADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEGRPEFSEVVM KLEECLCNIELMSPASSNSSGSLSPSSSSDCLVNRGGPGRSHVAALRSRFELEYALNARSYAAL SQSAGQYSSQGLSLEEMKRSLQYTPIDKYGYVSDPMSSMHFHSCRNSS(SEQ. ID NO: 352) of C03950_3_P10 (SEQ. ID NO:215).

In some embodiments, the isolated chimeric proteins or polypeptides of the invention comprise an amino acid sequence corresponding to or homologous to C03950_3_P11 (SEQ. ID NO:216).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence PSPCGLHFLIPWLTQ (SEQ. ID NO: 351) corresponding to amino acids 1 - 15 of C03950_3 _P11 (SEQ. ID NO:216), a second amino acid sequence being at least about 90% homologous to amino acids 213 - 691 of TNI3K_HUMAN (SEQ. ID NO: 396), which also corresponds to amino acids 16 - 494 of C03950_3_P11 (SEQ. ID NO:216), a third amino acid sequence being at least about 70%, optionally at least about 80%, preferably at least about 85%, more preferably at least about 90% and most preferably at least about 95%, homologous to a polypeptide having the sequence RSAITSRIWITHSICIWRGAHYFNREECNFRCMLTSAILK corresponding to amino acids 495 - 534 of C03950_3_P1 (SEQ. ID NO:216), and a fourth amino acid sequence being at least about 90% homologous to amino acids 710 - 936 of TNI3K_HUMAN (SEQ. ID NO: 396), which also corresponds to amino acids 535 - 761 of C03950_3_P11 (SEQ. ID NO:216), wherein said first amino acid sequence, second amino acid sequence, third amino acid sequence and fourth amino acid sequence are contiguous and in a sequential order.

In some embodiments, this invention provides an isolated polypeptide comprising a head of C03950_3_P11 (SEQ. ID NO:216), comprising a polypeptide being at least about 70%, optionally at least about 80%, preferably at least about 85%, more preferably at least about 90% and most preferably at least about 95% homologous to the sequence PSPCGLHFLIPWLTQ (SEQ. ID NO: 351) of C03950_3_P11 (SEQ. ID NO:216).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence PSPCGLHFLIPWLTQ (SEQ. ID NO: 351) corresponding to amino acids 1 - 15 of C03950_3_P11 (SEQ. ID NO:216), a second amino acid sequence being at least about 90% homologous to amino acids 230 - 367 of Q6MZS9_HUMAN (SEQ. ID NO:211), which also corresponds to amino acids 16 - 153 of C03950_3_P11 (SEQ. ID NO:216), a bridging amino acid I corresponding to amino acid 154 of C03950_3_P11 (SEQ. ID NO:216), a third amino acid sequence being at least about 90% homologous to amino acids 369 - 484 of Q6MZS9_HUMAN (SEQ. ID NO:211), which also corresponds to amino acids 155 - 270 of C03950_3_P11 (SEQ. ID NO:216), a bridging amino acid N corresponding to amino acid 271 of C03950_3_P11 (SEQ. ID NO:216), a fourth amino acid sequence being at least about 90% homologous to amino acids 486 - 709 of Q6MZS9_HUMAN (SEQ. ID NO:211), which also corresponds to amino acids 272 - 495 of C03950_3_P11 (SEQ. ID NO:216), and a fifth amino acid sequence being at least about 70%, optionally at least about 80%, preferably at least about 85%, more preferably at least about 90% and most preferably at least about 95% homologous to a polypeptide having the sequence SAITSRIWITHSICIWRGAHYFNREECNFRCMLTSAILKESRFLQSLDEDN-MTKQPGNLRWMA PEVFTQCTRYTIKADVFSYALCLWEILTGEIPFAHLKPAAAAADMAYHHIRPPIGYSIPKPISSL LIR-GWNACPEGRPEFSEWMKLEECLCNIELMSPASSNSSGSLSPSSSSDCLVNRGGPGRSHVA ALRSRFELEYALNAR-SYAALSQSAGQYSSQGLSLEEMKRSLQYTPIDKYGYVSDPMSSMHFH SCRNSSSFEDSS (SEQ. ID NO: 345) corresponding to amino acids 496 - 761 of C03950_3_P11 (SEQ. ID NO:216), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising a head of C03950_3_P11 (SEQ. ID NO:216), comprising a polypeptide being at least about 70%, optionally at least about 80%, preferably at least about 85%, more preferably at least about 90% and most preferably at least about 95% homologous to the sequence PSPCGLHFLIPWLTQ (SEQ. ID NO: 351) of C03950_3_P11 (SEQ. ID NO:216).

In some embodiments, this invention provides an isolated polypeptide comprising an edge portion of C03950_3_P11 (SEQ. ID NO:216), comprising an amino acid sequence being at least about 70%, optionally at least about 80%, preferably at least about 85%, more preferably at least about 90% and most preferably at least about 95% homologous to the sequence -

SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEVVMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) of C03950_3_P11 (SEQ. ID NO:216).

In some embodiments, the isolated chimeric proteins or polypeptides of the invention comprise an amino acid sequence corresponding to or homologous to C03950_3_P12 (SEQ. ID NO:217). In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence AVRRGLREGGA (SEQ. ID NO: 342) corresponding to amino acids 1 - I 1 of C03950_3_P12 (SEQ. ID NO:217), a second amino acid sequence being at least about 90% homologous to amino acids 1 - 808 of TNI3K_HUMAN (SEQ. ID NO: 396), which also corresponds to amino acids 12 - 819 of C03950_3_P12 (SEQ. ID NO:217), and a third amino acid sequence being at least about 70%, optionally at least about 80%, preferably at least about 85%, more preferably at least about 90% and most preferably at least about 95% homologous to a polypeptide having the sequence AKSRPSHYPVSSVYTETLKKKNEDRFGMWIEYLRR (SEQ. ID NO: 356) corresponding to amino acids 820 - 854 of C03950_3_P12 (SEQ. ID NO:217), wherein said first amino acid sequence, second amino acid sequence and third amino acid sequence are contiguous and in a sequential order.

In some embodiments, this invention provides an isolated polypeptide comprising a head of C03950_3_P12 (SEQ. ID NO:217), comprising a polypeptide being at least about 70%, optionally at least about 80%, preferably at least about 85%, more preferably at least about 90% and most preferably at least about 95% homologous to the sequence AVRRGLREGGA (SEQ. ID NO: 342) of C03950_3_P12 (SEQ. ID NO:217).

In some embodiments, this invention provides an isolated polypeptide comprising an edge portion of C03950_3_P12 (SEQ. ID NO:217), comprising an amino acid sequence being at least about 70%, optionally at least about 80%, preferably at least about 85%, more preferably at least about 90% and most preferably at least about 95% homologous to the sequence AKSRPSHYPVSSVYTETLKKKNEDRTGMWIEYLRR (SEQ. ID NO: 356) of C03950_3_P12 (SEQ. ID NO:217).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence AVRRGLR (SEQ. ID NO: 344) corresponding to amino acids 1 - 7 of C03950_3_P12 (SEQ. ID NO:217), a second amino acid sequence being at least about 90% homologous to amino acids 14 - 367 of Q6MZS9_HUMAN (SEQ. ID NO:211), which also corresponds to amino acids 8 - 361 of C03950_3_P12 (SEQ. ID NO:217), a bridging amino acid I corresponding to amino acid 362 of C03950_3_P12 (SEQ. ID NO:217), a third amino acid sequence being at least about 90% homologous to amino acids 369 - 484 of Q6MZS9_HUMAN (SEQ. ID NO:211), which also corresponds to amino acids 363 - 478 of C03950_3_P12 (SEQ. ID NO:217), a bridging amino acid N corresponding to amino acid 479 of C03950_3_P12 (SEQ. ID NO:217), a fourth amino acid sequence being at least about 90% homologous to amino acids 486 - 708 of Q6MZS9_HUMAN (SEQ. ID NO:211), which also corresponds to amino acids 480 - 702 of C03950_3_P12 (SEQ. ID NO:217), and a fifth amino acid sequence being at least about 70%, optionally at least about 80%, preferably at least about 85%, more preferably at least about 90% and most preferably at least about 95% homologous to a polypeptide having the sequence SHNILLYEDGHAVVADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEAKSRPSHYPV SSVYTETLKKKNEDRFGMWIEYLRR (SEQ. ID NO: 358) corresponding to amino acids 703 - 854 of C03950_3_P12 (SEQ. ID NO:217), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising a head of C03950_3_P12 (SEQ. ID NO:217), comprising a polypeptide being at least about 70%, optionally at least about 80%, preferably at least about 85%, more preferably at least about 90% and most preferably at least about 95% homologous to the sequence AVRRGLR (SEQ. ID NO: 344) of C03950_3_P12 (SEQ. ID NO:217).

In some embodiments, this invention provides an isolated polypeptide comprising an edge portion of C03950_3_P12 (SEQ. ID NO:217), comprising an amino acid sequence being at least about 70%, optionally at least about 80%, preferably at least about 85%, more preferably at least about 90% and most preferably at least about 95% homologous to the sequence -

SHNILLYEDGHAVVADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEAKSRPSHYPV SSVYTETLKKKNEDRFGMWIEYLRR (SEQ. ID NO: 358) of C03950_3_P12 (SEQ. ID NO:217).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence - AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P12 (SEQ. ID NO:217), a second amino acid sequence being at least about 90% homologous to amino acids 14 - 707 of NP_057062 (SEQ. ID NO:210), which also corresponds to amino acids 126 - 819 of C03950_3_P12 (SEQ. ID NO:217), and a third amino acid sequence being at least about 70%, optionally at least about 80%, preferably at least about 85%, more preferably at least about 90% and most preferably at least about 95% homologous to a polypeptide having the sequence AKSRPSHYPVSSVYTETLKKKNEDRFGMWIEYLRR (SEQ. ID NO: 356) corresponding to amino acids 820 - 854 of C03950_3_P12 (SEQ. ID NO:217), wherein said first amino acid sequence, second amino acid sequence and third amino acid sequence are contiguous and in a sequential order.

In some embodiments, this invention provides an isolated polypeptide comprising a head of C03950_3_P12 (SEQ. ID NO:217), comprising a polypeptide being at least about 70%, optionally at least about 80%, preferably at least about 85%, more preferably at least about 90% and most preferably at least about 95% homologous to the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) of C03950_3_P12 (SEQ. ID NO:217).

In some embodiments, the isolated chimeric proteins or polypeptides of the invention comprise an amino acid sequence corresponding to or homologous to In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P13 (SEQ. ID NO:218), a second amino acid sequence being at least about 90% homologous to amino acids 115 - 808 of TNI3K_ HUMAN (SEQ. ID NO: 396), which also corresponds to amino acids 14 - 707 of C03950_3_P13 (SEQ. ID NO:218), and a third amino acid sequence being at least about 70%, optionally at least about 80%, preferably at least about 85%, more preferably at least about 90% and most preferably at least about 95% homologous to a polypeptide having the sequence AKSRPSHYPVSSVYTETLKKKNEDRFGMWIEYLRR (SEQ. ID NO: 356) corresponding to amino acids 708 - 742 of C03950_3_P13 (SEQ. ID NO:218), wherein said first amino acid sequence, second amino acid sequence and third amino acid sequence are contiguous and in a sequential order.

In some embodiments, this invention provides an isolated polypeptide comprising a head of C03950_3_P13 (SEQ. ID NO:218), comprising a polypeptide being at least about 70%, optionally at least about 80%, preferably at least about 85%, more preferably at least about 90% and most preferably at least about 95% homologous to the sequence MGNYKSRPTQTCT (SEQ. ID NO: 346) of C03950_3_P13 (SEQ. ID NO:218).

In some embodiments, this invention provides an isolated polypeptide comprising an edge portion of C03950_3_P13 (SEQ. ID NO:218), comprising an amino acid sequence being at least about 70%, optionally at least about 80%, preferably at least about 85%, more preferably at least about 90% and most preferably at least about 95% homologous to the sequence AKSRPSHYPVSSVYTETLKKKNEDRFGMWIEYLRR (SEQ. ID NO: 356) of C03950_3_P13 (SEQ. ID NO:218).

In some embodiments, such isolated chimeric proteins or polypeptides comprise a first amino acid sequence being at least about 90% homologous to amino acids 1 - 707 of NP_057062 (SEQ. ID NO:210), which also corresponds to amino acids 1 - 707 of C03950_3_P13 (SEQ. ID NO:218), and a second amino acid sequence being at least about 70%, optionally at least about 80%, preferably at least about 85%, more preferably at least about 90% and most preferably at least about 95% homologous to a polypeptide having the sequence AKSRPSHYPVSSVYTETLKKKNEDRFGMWIEYLRR (SEQ. ID NO: 356) corresponding to amino acids 708 - 742 of C03950_3_P13 (SEQ. ID NO:218), wherein said first amino acid sequence and second amino acid sequence are contiguous and in a sequential order.

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P13 (SEQ. ID NO:218), a second amino acid sequence being at least about 90% homologous to amino acids 132 - 367 of Q6MZS9_HUMAN (SEQ. ID NO:211), which also corresponds to amino acids 14 - 249 of C03950_3_P13 (SEQ. ID NO:218), a bridging amino acid I corresponding to amino acid 250 of C03950_3_P13 (SEQ. ID NO:218), a third amino acid sequence being at least about 90% homologous to amino acids 369 - 484 of Q6MZS9_HUMAN (SEQ. ID NO:211), which also corresponds to amino acids 251 - 366 of C03950_3_P13 (SEQ. ID NO:218), a bridging amino acid N corresponding to amino acid 367 of C03950_3_P13 (SEQ. ID NO:218), a fourth amino acid sequence being at least about 90% homologous to amino acids 486 - 708 of Q6MZS9_HUMAN (SEQ. ID NO:211), which also corresponds to amino acids 368 - 590 of C03950_3_P13 (SEQ. ID NO:218), and a fifth amino acid sequence being at least about 70%, optionally at least about 80%, preferably at least about 85%, more preferably at least about 90% and most preferably at least about 95% homologous to a polypeptide having the sequence SHNILLYEDGHAVVADFGESRFLQSLDEDNMTKQPGNLRWMAPEVFTQCTRY-TIKADVFSYA LCLWEILTGEIPFAHLKPAAAAADMAYHHIRPPIGYSIPKPISSLLIRGWNACPEAKSRPSHYPV SS-VYTETLKKKNEDRFGMWIEYLRR (SEQ. ID NO: 358) corresponding to amino acids 591 - 742 of C03950_3_P13 (SEQ. ID NO:218), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising an edge portion of C03950_3_P13 (SEQ. ID NO:218), comprising an amino acid sequence being at least about 70%, optionally at least about 80%, preferably at least about 85%, more preferably at least about 90% and most preferably at least about 95% homologous to the sequence -

SHNILLYEDGHAVVADFGESRFLQSLDEDNMTKQPGNLRWMAPEVFTQCTRYTIKADVFSYA
LCLWEILTGEIPFAHLKPAAAAADMAYHHIRPPIGYSIPKPISSLLIRGWNACPEAKSRPSHYPV
SSVYTETLKKKNEDRFGMWIEYLRR (SEQ. ID NO: 358) of C03950_3_P13 (SEQ. ID NO:218).

In some embodiments, the isolated chimeric proteins or polypeptides of the invention comprise an amino acid sequence corresponding to or homologous to C03950_3_P15 (SEQ. ID NO:219).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence AVRRGLREGGA (SEQ. ID NO: 342) corresponding to amino acids 1 - 11 of C03950_3_P15 (SEQ. ID NO:219), a second amino acid sequence being at least about 90% homologous to amino acids 1 - 691 of TNI3K_HUMAN (SEQ. ID NO: 396), which also corresponds to amino acids 12 - 702 of C03950_3_P15 (SEQ. ID NO:219), and a third amino acid sequence being at least about 70%, optionally at least about 80%, preferably at least about 85%, more preferably at least about 90% and most preferably at least about 95% homologous to a polypeptide having the sequence RYFFPK (SEQ. ID NO: 364) corresponding to amino acids 703 - 708 of C03950_3_P15 (SEQ. ID NO:219), wherein said first amino acid sequence, second amino acid sequence and third amino acid sequence are contiguous and in a sequential order.

In some embodiments, this invention provides an isolated polypeptide comprising a head of C03950_3_P15 (SEQ. ID NO:219), comprising a polypeptide being at least about 70%, optionally at least about 80%, preferably at least about 85%, more preferably at least about 90% and most preferably at least about 95% homologous to the sequence AVRRGLREGGA (SEQ. ID NO: 342) of C03950_3_P15 (SEQ. ID NO:219).

In some embodiments, this invention provides an isolated polypeptide comprising an edge portion of C03950_3_P15 (SEQ. ID NO:219), comprising an amino acid sequence being at least about 70%, optionally at least about 80%, preferably at least about 85%, more preferably at least about 90% and most preferably at least about 95% homologous to the sequence RYFFPK (SEQ. ID NO: 364) of C03950_3_P15 (SEQ. ID NO:219).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence AVRRGLR (SEQ. ID NO: 344) corresponding to amino acids 1 - 7 of C03950_3_P15 (SEQ. ID NO:219), a second amino acid sequence being at least about 90% homologous to amino acids 14 - 367 of Q6MZS9_HUMAN (SEQ. ID NO:211), which also corresponds to amino acids 8 - 361 of C03950_3_P15 (SEQ. ID NO:219), a bridging amino acid I corresponding to amino acid 362 of C03950_3_P15 (SEQ. ID NO:219), a third amino acid sequence being at least about 90% homologous to amino acids 369 - 484 of Q6MZS9_HUMAN (SEQ. ID NO:211), which also corresponds to amino acids 363 - 478 of C03950_3_P15 (SEQ. ID NO:219), a bridging amino acid N corresponding to amino acid 479 of C03950_3_P15 (SEQ. ID NO:219), and a fourth amino acid sequence being at least about 90% homologous to amino acids 486 - 714 of Q6MZS9_HUMAN (SEQ. ID NO:211), which also corresponds to amino acids 480 - 708 of C03950_3_P15 (SEQ. ID NO:219), wherein said first amino acid sequence, second amino acid sequence, bridging amino acid, third amino acid sequence, bridging amino acid and fourth amino acid sequence are contiguous and in a sequential order.

In some embodiments, this invention provides an isolated polypeptide comprising a head of C03950_3_P15 (SEQ. ID NO:219), comprising a polypeptide being at least about 70%, optionally at least about 80%, preferably at least about 85%, more preferably at least about 90% and most preferably at least about 95% homologous to the sequence AVRRGLR (SEQ. ID NO: 344) of C03950_3_P15 (SEQ. ID NO:219).

A. An isolated chimeric polypeptide encoding for C03950_3_P15 (SEQ. ID NO:219), comprising a first amino acid sequence being at least about 70%, optionally at least about 80%, preferably at least about 85%, more preferably at least about 90% and most preferably at least about 95%, homologous to a polypeptide having the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P15 (SEQ. ID NO:219), a second amino acid sequence being at least about 90% homologous to amino acids 14 - 590 of NP_057062 (SEQ. ID NO:210), which also corresponds to amino acids 126 - 702 of C03950_3_P15 (SEQ. ID NO:219), and a third amino acid sequence being at least about 70%, optionally at least about 80%, preferably at least about 85%, more preferably at least about 90% and most preferably at least about 95% homologous to a polypeptide having the sequence RYFFPK (SEQ. ID NO: 364) corresponding to amino acids 703 - 708 of C03950_3_P15 (SEQ. ID NO:219), wherein said first amino acid sequence, second amino acid sequence and third amino acid sequence are contiguous and in a sequential order.

In some embodiments, this invention provides an isolated polypeptide comprising a head of C03950_3_P15 (SEQ. ID NO:219), comprising a polypeptide being at least about 70%, optionally at least about 80%, preferably at least about 85%, more preferably at least about 90% and most preferably at least about 95% homologous to the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) of C03950_3_P15 (SEQ. ID NO:219).

In some embodiments, the isolated chimeric proteins or polypeptides of the invention comprise an amino acid sequence corresponding to or homologous to C03950_3_P17 (SEQ. ID NO:220).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P17 (SEQ. ID NO:220), a second amino acid sequence being at least about 90% homologous to amino acids 115 - 691 of TN13K_HUMAN (SEQ. ID NO: 396), which also corresponds to amino acids 14 - 590 of C03950_3_P17 (SEQ. ID NO:220), and a third amino acid sequence being at least about 70%, optionally at least about 80%, preferably at least about 85%, more preferably at least about 90% and most preferably at least about 95% homologous to a polypeptide having the sequence RCCTGWLSCYHPD (SEQ. ID NO: 368) corresponding to amino acids 591 - 603 of C03950_3_P17 (SEQ. ID NO:220), wherein said first amino acid sequence, second amino acid sequence and third amino acid sequence are contiguous and in a sequential order.

In some embodiments, this invention provides an isolated polypeptide comprising a head of C03950_3_P17 (SEQ. ID NO:220), comprising a polypeptide being at least about 70%, optionally at least about 80%, preferably at least about 85%, more preferably at least about 90% and most preferably at least about 95% homologous to the sequence MGNYKSRPTQTCT (SEQ. ID NO: 346) of C03950_3_P17 (SEQ. ID NO:220).

In some embodiments, this invention provides an isolated polypeptide comprising an edge portion of C03950_3_P17 (SEQ. ID NO:220), comprising an amino acid sequence being at least about 70%, optionally at least about 80%, preferably at least about 85%, more preferably at least about 90% and most preferably at least about 95% homologous to the sequence RCCTGWLSCYHPD (SEQ. ID NO: 368) of C03950_3_P17 (SEQ. ID NO:220).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P17 (SEQ. ID NO:220), a second amino acid sequence being at least about 90% homologous to amino acids 132 - 367 of Q6MZS9_HUMAN (SEQ. ID NO:211), which also corresponds to amino acids 14 - 249 of C03950_3_P17 (SEQ. ID NO:220), a bridging amino acid I corresponding to amino acid 250 of C03950_3_P17 (SEQ. ID NO:220), a third amino acid sequence being at least about 90% homologous to amino acids 369 - 484 of Q6MZS9_HUMAN (SEQ. ID NO:211), which also corresponds to amino acids 251 - 366 of C03950_3_P17 (SEQ. ID NO:220), a bridging amino acid N corresponding to amino acid 367 of C03950_3_P17 (SEQ. ID NO:220), a fourth amino acid sequence being at least about 90% homologous to amino acids 486 - 709 of Q6MZS9_HUMAN (SEQ. ID NO:211), which also corresponds to amino acids 368 - 591 of C03950_3_P17 (SEQ. ID NO:220), and a fifth amino acid sequence being at least about 70%, optionally at least about 80%, preferably at least about 85%, more preferably at least about 90% and most preferably at least about 95% homologous to a polypeptide having the sequence CCTGWLSCYHPD (SEQ. ID NO: 369) corresponding to amino acids 592 - 603 of C03950_3_P17 (SEQ. ID NO:220), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising an edge portion of C03950_3_P17 (SEQ. ID NO:220), comprising an amino acid sequence being at least about 70%, optionally at least about 80%, preferably at least about 85%, more preferably at least about 90% and most preferably at least about 95% homologous to the sequence CCTGWLSCYHPD (SEQ. ID NO: 369) of C03950_3_P17 (SEQ. ID NO:220).

In some embodiments, such isolated chimeric proteins or polypeptides comprise a first amino acid sequence being at least about 90% homologous to amino acids 1 - 590 of NP_057062 (SEQ. ID NO:210), which also corresponds to amino acids 1 - 590 of C03950_3_P17 (SEQ. ID NO:220), and a second amino acid sequence being at least about 70%, optionally at least about 80%, preferably at least about 85%, more preferably at least about 90% and most preferably at least about 95% homologous to a polypeptide having the sequence RCCTGWLSCYHPD (SEQ. ID NO: 368) corresponding to amino acids 591 - 603 of C03950_3_P17 (SEQ. ID NO:220), wherein said first amino acid sequence and second amino acid sequence are contiguous and in a sequential order.

In some embodiments, the isolated chimeric proteins or polypeptides of the invention comprise an amino acid sequence corresponding to or homologous to C03950_3-P19 (SEQ. ID NO:221).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P19 (SEQ. ID NO:221), a second amino acid sequence being at least about 90% homologous to amino acids 115 - 691 of TNI3K_HUMAN (SEQ. ID NO: 396), which also corresponds to amino acids 14 - 590 of C03950_3_P19 (SEQ. ID NO:221), and a third amino acid sequence being at least about 70%, optionally at least about 80%, preferably at least about 85%, more preferably at least about 90% and most preferably at least about 95% homologous to a polypeptide having the sequence RAS (SEQ. ID NO: 370) corresponding to amino acids 591 - 593 of C03950_3_P19 (SEQ. ID NO:221), wherein said first amino acid sequence, second amino acid sequence and third amino acid sequence are contiguous and in a sequential order.

In some embodiments, this invention provides an isolated polypeptide comprising an edge portion of C03950_3_P19 (SEQ. ID NO:221), comprising an amino acid sequence being at least about 70%, optionally at least about 80%, preferably at least about 85%, more preferably at least about 90% and most preferably at least about 95% homologous to the sequence RAS (SEQ. ID NO: 370) of C03950_3_P19 (SEQ. ID NO:221).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P19 (SEQ. ID NO:221), a second amino acid sequence being at least about 90% homologous to amino acids 132 - 367 of Q6MZS9_HUMAN (SEQ. ID NO:211), which also corresponds to amino acids 14 - 249 of C03950_3_P19 (SEQ. ID NO:221), a bridging amino acid I corresponding to amino acid 250 of C03950_3_P19 (SEQ. ID NO:221), a third amino acid sequence being at least about 90% homologous to amino acids 369 - 484 of Q6MZS9_HUMAN (SEQ. ID NO:211), which also corresponds to amino acids 251 - 366 of C03950_3_P19 (SEQ. ID NO:221), a bridging amino acid N corresponding to amino acid 367 of C03950_3_P19 (SEQ. ID NO:221), a fourth amino acid sequence being at least about 90% homologous to amino acids 486 - 709 of Q6MZS9_HUMAN (SEQ. ID NO:211), which also corresponds to amino acids 368 - 591 of C03950_3_P19 (SEQ. ID NO:221), and a fifth amino acid sequence being at least about 70%, optionally at least about 80%, preferably at least about 85%, more preferably at least about 90% and most preferably at least about 95% homologous to a polypeptide having the sequence AS corresponding to amino acids 592 - 593 of C03950_3_P19 (SEQ. ID NO:221), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, such isolated chimeric proteins or polypeptides comprise a first amino acid sequence being at least about 90% homologous to amino acids 1 - 590 of NP_057062 (SEQ. ID NO:210), which also corresponds to amino acids 1 - 590 of C03950_3_P19 (SEQ. ID NO:221), and a second amino acid sequence being at least about 70%, optionally at least about 80%, preferably at least about 85%, more preferably at least about 90% and most preferably at least about 95% homologous to a polypeptide having the sequence RAS (SEQ. ID NO: 370) corresponding to amino acids 591 - 593 of C03950_3_P19 (SEQ. ID NO:221), wherein said first amino acid sequence and second amino acid sequence are contiguous and in a sequential order.

In some embodiments, the isolated chimeric proteins or polypeptides of the invention comprise an amino acid sequence corresponding to or homologous to C03950_3_P20 (SEQ. ID NO:222).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence AVRRGLREGGA (SEQ.

ID NO: 342) corresponding to amino acids 1 - 11 of C03950_3_P20 (SEQ. ID NO:222), a second amino acid sequence being at least about 90% homologous to amino acids 1 - 657 of TNI3K_HUMAN (SEQ. ID NO: 396), which also corresponds to amino acids 12 - 668 of C03950_3_P20 (SEQ. ID NO:222), and a third amino acid sequence being at least about 70%, optionally at least about 80%, preferably at least about 85%, more preferably at least about 90% and most preferably at least about 95% homologous to a polypeptide having the sequence YGSFVLIYPWTFRRNYSCNTSEGFPLDEPSPFEI (SEQ. ID NO: 372) corresponding to amino acids 669 - 702 of C03950_3_P20 (SEQ. ID NO:222), wherein said first amino acid sequence, second amino acid sequence and third amino acid sequence are contiguous and in a sequential order.

In some embodiments, this invention provides an isolated polypeptide comprising a head of C03950_3_P20 (SEQ. ID NO:222), comprising a polypeptide being at least about 70%, optionally at least about 80%, preferably at least about 85%, more preferably at least about 90% and most preferably at least about 95% homologous to the sequence AVRRGLREGGA (SEQ. ID NO: 342) of C03950_3_P20 (SEQ. ID NO:222).

In some embodiments, this invention provides an isolated polypeptide comprising an edge portion of C03950_3_P20 (SEQ. ID NO:222), comprising an amino acid sequence being at least about 70%, optionally at least about 80%, preferably at least about 85%, more preferably at least about 90% and most preferably at least about 95% homologous to the sequence YGSFVLIYPWTFRRNYSCNTSEGFPLDEPSPFEI (SEQ. ID NO: 372) of C03950_3_P20 (SEQ. ID NO:222).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence AVRRGLR (SEQ. ID NO: 344) corresponding to amino acids 1 - 7 of C03950_3_P20 (SEQ. ID NO:222), a second amino acid sequence being at least about 90% homologous to amino acids 14 - 367 of Q6MZS9_HUMAN (SEQ. ID NO:211), which also corresponds to amino acids 8 - 361 of C03950_3_P20 (SEQ. ID NO:222), a bridging amino acid I corresponding to amino acid 362 of C03950_3_P20 (SEQ. ID NO:222), a third amino acid sequence being at least about 90% homologous to amino acids 369 - 484 of Q6MZS9_HUMAN (SEQ. ID NO:211), which also corresponds to amino acids 363 - 478 of C03950_3_P20 (SEQ. ID NO:222), a bridging amino acid N corresponding to amino acid 479 of C03950_3_P20 (SEQ. ID NO:222), a fourth amino acid sequence being at least about 90% homologous to amino acids 486 - 674 of Q6MZS9_HUMAN (SEQ. ID NO:211), which also corresponds to amino acids 480 - 668 of C03950_3_P20 (SEQ. ID NO:222), and a fifth amino acid sequence being at least about 70%, optionally at least about 80%, preferably at least about 85%, more preferably at least about 90% and most preferably at least about 95% homologous to a polypeptide having the sequence YGSFVLIYPWTFRRNYSCNTSEGFPLDEPSPFEI (SEQ. ID NO: 372) corresponding to amino acids 669 - 702 of C03950_3_P20 (SEQ. ID NO:222), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising a head of C03950_3_P20 (SEQ. ID NO:222), comprising a polypeptide being at least about 70%, optionally at least about 80%, preferably at least about 85%, more preferably at least about 90% and most preferably at least about 95% homologous to the sequence AVRRGLR (SEQ. ID NO: 344) of C03950_3_P20 (SEQ. ID NO:222).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence -

AVRRGLREGGAMAAARDPPEVSLREATQRKLRRFSELRGKLVARGEFWDIVAITAADEKQEL
AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P20 (SEQ. ID NO:222), a second amino acid sequence being at least about 90% homologous to amino acids 14 - 556 of NP_057062 (SEQ. ID NO:210), which also corresponds to amino acids 126 - 668 of C03950_3_P20 (SEQ. ID NO:222), and a third amino acid sequence being at least about 70%, optionally at least about 80%, preferably at least about 85%, more preferably at least about 90% and most preferably at least about 95% homologous to a polypeptide having the sequence YGSFVLIYPWTFRRNYSCNTSEGFPLDEPSPFEI (SEQ. ID NO: 372) corresponding to amino acids 669 - 702 of C03950_3_P20 (SEQ. ID NO:222), wherein said first amino acid sequence, second amino acid sequence and third amino acid sequence are contiguous and in a sequential order.

In some embodiments, this invention provides an isolated polypeptide comprising a head of C03950_3_P20 (SEQ. ID NO:222), comprising a polypeptide being at least about 70%, optionally at least about 80%, preferably at least about 85%, more preferably at least about 90% and most preferably at least about 95% homologous to the sequence -

AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) of C03950_3_P20 (SEQ. ID NO:222).

In some embodiments, the isolated chimeric proteins or polypeptides of the invention comprise an amino acid sequence corresponding to or homologous to C03950_3_P21 (SEQ. ID NO:223).In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P21 (SEQ. ID NO:223), a second amino acid sequence being at least about 90% homologous to amino acids 115 - 657 of TNI3K_HUMAN (SEQ. ID NO: 396), which also corresponds to amino acids 14 - 556 of C03950_3_P21 (SEQ. ID NO:223), and a third amino acid sequence being at least about 70%, optionally at least about 80%, preferably at least about 85%, more preferably at least about 90% and most preferably at least about 95% homologous to a polypeptide having the sequence YGSFVLIYPWTFRRNYSCNTSEGFPLDEPSPFEI (SEQ. ID NO: 372) corresponding to amino acids 557 - 590 of C03950_3_P21 (SEQ. ID NO:223), wherein said first amino acid sequence, second amino acid sequence and third amino acid sequence are contiguous and in a sequential order.

In some embodiments, this invention provides an isolated polypeptide comprising a head of C03950_3_P21 (SEQ. ID NO:223), comprising a polypeptide being at least about 70%, optionally at least about 80%, preferably at least about 85%, more preferably at least about 90% and most preferably at least about 95% homologous to the sequence MGNYKSRPTQTCT (SEQ. ID NO: 346) of C03950_3_P21 (SEQ. ID NO:223).

In some embodiments, this invention provides an isolated polypeptide comprising an edge portion of C03950_3_P21 (SEQ. ID NO:223), comprising an amino acid sequence being at least about 70%, optionally at least about 80%, preferably at least about 85%, more preferably at least about 90% and most preferably at least about 95% homologous to the sequence YGSFVLIYPWTFRRNYSCNTSEGFPLDEPSPFEI (SEQ. ID NO: 372) of C03950_3_P21 (SEQ. ID NO:223).

I n some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P21 (SEQ. ID NO:223), a second amino acid sequence being at least about 90% homologous to amino acids 132 - 367 of Q6MZS9_HUMAN (SEQ. ID NO:211), which also corresponds to amino acids 14 - 249 of C03950_3_P21 (SEQ. ID NO:223), a bridging amino acid I corresponding to amino acid 250 of C03950_3_P21 (SEQ. ID NO:223), a third amino acid sequence being at least about 90% homologous to amino acids 369 - 484 of Q6MZS9_HUMAN (SEQ. ID NO:211), which also corresponds to amino acids 251 - 366 of C03950_3_P21 (SEQ. ID NO:223), a bridging amino acid N corresponding to amino acid 367 of C03950_3_P21 (SEQ. ID NO:223), a fourth amino acid sequence being at least about 90% homologous to amino acids 486 - 674 of Q6MZS9_HUMAN (SEQ. ID NO:211), which also corresponds to amino acids 368 - 556 of C03950_3_P21 (SEQ. ID NO:223), and a fifth amino acid sequence being at least about 70%, optionally at least about 80%, preferably at least about 85%, more preferably at least about 90% and most preferably at least about 95% homologous to a polypeptide having the sequence YGSFVLIYPWTFRRNYSCNTSEGFPLDEPSPFEI (SEQ. ID NO: 372) corresponding to amino acids 557 - 590 of C03950_3_P21 (SEQ. ID NO:223), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, such isolated chimeric proteins or polypeptides comprise a first amino acid sequence being at least about 90% homologous to amino acids 1 - 556 of NP_057062 (SEQ. ID NO:210), which also corresponds to amino acids 1 - 556 of C03950_3_P21 (SEQ. ID NO:223), and a second amino acid sequence being at least about 70%, optionally at least about 80%, preferably at least about 85%, more preferably at least about 90% and most preferably at least about 95% homologous to a polypeptide having the sequence YGSFVLIYPWTFRRNYSCNTSEGFPLDEPSPFEI (SEQ. ID NO: 372) corresponding to amino acids 557 - 590 of C03950_3_P21 (SEQ. ID NO:223), wherein said first amino acid sequence and second amino acid sequence are contiguous and in a sequential order.

In some embodiments, the isolated chimeric proteins or polypeptides of the invention comprise an amino acid sequence corresponding to or homologous to C03950_3_P23 (SEQ. ID NO:224).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MGNYKSRPTQTCT (SEQ. ID NO: 346) corresponding to amino acids 1 - 13 of C03950_3_P23 (SEQ. ID NO:224), a second amino acid sequence being at least about 90% homologous to amino acids 132 - 367 of Q6MZS9_HUMAN (SEQ. ID NO:211), which also corresponds to amino acids 14 - 249 of C03950_3_P23 (SEQ. ID NO:224), a bridging amino acid I corresponding to amino acid 250 of C03950_3_P23 (SEQ. ID NO:224), a third amino acid sequence being at least about 90% homologous to amino acids 369 - 484 of Q6MZS9_HUMAN (SEQ. ID NO:211), which also corresponds to amino acids 251 - 366 of C03950_3_P23 (SEQ. ID NO:224), a bridging amino acid N corresponding to amino acid 367 of C03950_3_P23 (SEQ. ID NO:224), a fourth amino acid sequence being at least about 90% homologous to amino acids 486 - 590 of Q6MZS9_HUMAN (SEQ. ID NO:211), which also corresponds to amino acids 368 - 472 of C03950_3_P23 (SEQ. ID NO:224), and a fifth amino acid sequence being at least about 70%, optionally at least about 80%, preferably at least about 85%, more preferably at least about 90% and most preferably at least about 95% homologous to a polypeptide having the sequence NLK (SEQ. ID NO: 378) corresponding to amino acids 473 - 475 of C03950_3_P23 (SEQ. ID NO:224), wherein said first amino acid sequence, second amino acid sequence, bridging amino acid, third amino acid sequence, bridging amino acid, fourth amino acid sequence and fifth amino acid sequence are contiguous and in a sequential order.

In some embodiments, this invention provides an isolated polypeptide comprising a head of C03950_3_P23 (SEQ. ID NO:224), comprising a polypeptide being at least about 70%, optionally at least about 80%, preferably at least about 85%, more preferably at least about 90% and most preferably at least about 95% homologous to the sequence MGNYKSRPTQTCT (SEQ. ID NO: 346) of C03950_3_P23 (SEQ. ID NO:224).

In some embodiments, this invention provides an isolated polypeptide comprising an edge portion of C03950_3_P23 (SEQ. ID NO:224), comprising an amino acid sequence being at least about 70%, optionally at least about 80%, preferably at least about 85%, more preferably at least about 90% and most preferably at least about 95% homologous to the sequence NLK (SEQ. ID NO: 378) of C03950_3_P23 (SEQ. ID NO:224).

In some embodiments, such isolated chimeric proteins or polypeptides comprise a first amino acid sequence being at least about 90% homologous to amino acids 1 - 472 of NP_057062 (SEQ. ID NO:210), which also corresponds to amino acids 1 - 472 of C03950_3_P23 (SEQ. ID NO:224), and a second amino acid sequence being at least about 70%, optionally at least about 80%, preferably at least about 85%, more preferably at least about 90% and most preferably at least about 95% homologous to a polypeptide having the sequence NLK (SEQ. ID NO: 378) corresponding to amino acids 473 - 475 of C03950_3_P23 (SEQ. ID NO:224), wherein said first amino acid sequence and second amino acid sequence are contiguous and in a sequential order.

In some embodiments, the isolated chimeric proteins or polypeptides of the invention comprise an amino acid sequence corresponding to or homologous to C03950_3_P28 (SEQ. ID NO:227).

In some embodiments, such isolated chimeric proteins or polypeptides comprise a first amino acid sequence being at least about 90% homologous to amino acids 1 - 691 of TNI3K_HUMAN (SEQ. ID NO: 396), which also corresponds to amino acids 1 - 691 of C03950_3_P28 (SEQ. ID NO:227), a second amino acid sequence being at least about 70%, optionally at least about 80%, preferably at least about 85%, more preferably at least about 90% and most preferably at least about 95%, homologous to a polypeptide having the sequence RSAITSRIWITHSICIWRGAHYFNREECNFRCMLTSAILK corresponding to amino acids 692 - 731 of C03950_3_P28 (SEQ. ID NO:227), and a third amino acid sequence being at least about 90% homologous to amino acids 710 - 936 of TNI3K_HUMAN (SEQ. ID NO: 396), which also corresponds to amino acids 732 - 958 of C03950_3_P28 (SEQ. ID NO:227), wherein said first amino acid sequence, second amino acid sequence and third amino acid sequence are contiguous and in a sequential order.

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence - MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) corresponding to amino acids 1 - 114 of C03950_3_P28 (SEQ. ID NO:227), a second amino acid sequence being at least about 90% homologous to amino acids 14 - 590 of NP_057062 (SEQ. ID NO:210), which also corresponds to amino acids 115 - 691 of C03950_3_P28 (SEQ. ID NO:227), a third amino acid sequence being at least about 70%, optionally at least about 80%, preferably at least about 85%, more preferably at least about 90% and most preferably at least about 95%, homologous to a polypeptide having the sequence RSAITSRIWITHSICIWRGAHYFNREECNFRCMLTSAILK corresponding to amino acids 692 - 731 of C03950_3_P28 (SEQ. ID NO:227), and a fourth amino acid sequence being at least about 90% homologous to amino acids 609 - 835 of NP_057062 (SEQ. ID NO:210), which also corresponds to amino acids 732 - 958 of C03950_3_P28 (SEQ. ID NO:227), wherein said first amino acid sequence, second amino acid sequence, third amino acid sequence and fourth amino acid sequence are contiguous and in a sequential order.

In some embodiments, this invention provides an isolated polypeptide comprising a head of C03950_3_P28 (SEQ. ID NO:227), comprising a polypeptide being at least about 70%, optionally at least about 80%, preferably at least about 85%, more preferably at least about 90% and most preferably at least about 95% homologous to the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) of C03950_3_P28 (SEQ. ID NO:227).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MAAARDPPEVSLREATQRKLRRFSELRGKLVARGEFWDNAITAADEKQELAYNQQLSEKLK RKELPLGVQYHVFVDPAGAKIGNGGSTLCALQCLEKLYGDKWNSFTILLIHS (SEQ. ID NO: 379) corresponding to amino acids 1 - 114 of C03950_3_P28 (SEQ. ID NO:227), a second amino acid sequence being at least about 90% homologous to amino acids 14 - 590 of Q9Y2V6_HUMAN (SEQ. ID NO:210), which also corresponds to amino acids 115 - 691 of C03950_3_P28 (SEQ. ID NO:227), a third amino acid sequence being at least about 70%, optionally at least about 80%, preferably at least about 85%, more preferably at least about 90% and most preferably at least about 95%, homologous to a polypeptide having the sequence RSAITSRIWITHSICIWRGAHYFNREECNFRCMLTSAILK corresponding to amino acids 692 - 731 of C03950_3_P28 (SEQ. ID NO:227), and a fourth amino acid sequence being at least about 90% homologous to amino acids 609 - 835 of Q9Y2V6_HUMAN (SEQ. ID NO:210), which also corresponds to amino acids 732 - 958 of C03950_3_P28 (SEQ. ID NO:227), wherein said first amino acid sequence, second amino acid sequence, third amino acid sequence and fourth amino acid sequence are contiguous and in a sequential order.

In some embodiments, such isolated chimeric proteins or polypeptides comprise a first amino acid sequence being at least about 90% homologous to amino acids 18 - 367 of Q6MZS9_HUMAN (SEQ. ID NO:211), which also corresponds to amino acids 1 - 350 of C03950_3_P28 (SEQ. ID NO:227), a bridging amino acid I corresponding to amino acid 351 of C03950_3_P28 (SEQ. ID NO:227), a second amino acid sequence being at least about 90% homologous to amino acids 369 - 484 of Q6MZS9_HUMAN (SEQ. ID NO:211), which also corresponds to amino acids 352 - 467 of C03950_3_P28 (SEQ. ID NO:227), a bridging amino acid N corresponding to amino acid 468 of C03950_3_P28 (SEQ. ID NO:227), a third amino acid sequence being at least about 90% homologous to amino acids 486 - 709 of Q6MZS9_HUMAN (SEQ. ID NO:211), which also corresponds to amino acids 469 - 692 of C03950_3_P28 (SEQ. ID NO:227), and a fourth amino acid sequence being at least about 70%, optionally at least about 80%, preferably at least about 85%, more preferably at least about 90% and most preferably at least about 95% homologous to a polypeptide having the sequence SAITSRIWITHSICIWRGAHYFNREECNFRCMLTSAELKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEVVMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) corresponding to amino acids 693 - 958 of C03950_3_P28 (SEQ. ID NO:227), wherein said , first amino acid sequence, bridging amino acid, second amino acid sequence, bridging amino acid, third amino acid sequence and fourth amino acid sequence are contiguous and in a sequential order.

In some embodiments, this invention provides an isolated polypeptide comprising an edge portion of C03950_3_P28 (SEQ. ID NO:227), comprising an amino acid sequence being at least about 70%, optionally at least about 80%, preferably at least about 85%, more preferably at least about 90% and most preferably at least about 95% homologous to the sequence -

SAITSRIWITHSICIWRGAHYFNREECNFRCMLTSAILKESRFLQSLDEDNMTKQPGNLRWMA
PEVFTQCTRYTIKADVFSYALCLWEILTGEIPFAHLKPAAAAADMAYHHIRPPIGYSIPKPISSL
LIRGWNACPEGRPEFSEVVMKLEECLCNIELMSPASSNSSGSLSPSSSSDCLVNRGGPGRSHVA
ALRSRFELEYALNARSYAALSQSAGQYSSQGLSLEEMKRSLQYTPIDKYGYVSDPMSSMHFH
SCRNSSSFEDSS (SEQ. ID NO: 345) of C03950_3_P28 (SEQ. ID NO:227).

In some embodiments, such isolated chimeric proteins or polypeptides comprise a first amino acid sequence being at least about 90% homologous to amino acids 1 - 808 of TNI3K_HUMAN (SEQ. ID NO: 396), which also corresponds to amino acids 1 - 808 of C03950_3_P31 (SEQ. ID NO:228), and a second amino acid sequence being at least about 70%, optionally at least about 80%, preferably at least about 85%, more preferably at least about 90% and most preferably at least about 95% homologous to a polypeptide having the sequence AKSRPSHYPVSSVYTETLKKKNEDRFGMWIEYLRR (SEQ. ID NO: 356) corresponding to amino acids 809 - 843 of C03950_3_P31 (SEQ. ID NO228), wherein said first amino acid sequence and second amino acid sequence are contiguous and in a sequential order.

In some embodiments, this invention provides an isolated polypeptide comprising an edge portion of C03950_3_P31 (SEQ. ID NO:228), comprising an amino acid sequence being at least about 70%, optionally at least about 80%, preferably at least about 85%, more preferably at least about 90% and most preferably at least about 95% homologous to the sequence AKSRPSHYPVSSVYTETLKKKNEDRFGMWIEYLRR (SEQ. ID NO: 356) of C03950_3_P31 (SEQ. ID NO:228).

In some embodiments, such isolated chimeric proteins or polypeptides comprise a first amino acid sequence being at least about 90% homologous to amino acids 18 - 367 of Q6MZS9_HUMAN (SEQ. ID NO:211), which also corresponds to amino acids 1 - 350 of C03950_3_P31 (SEQ. ID NO:228), a bridging amino acid I corresponding to amino acid 351 of C03950_3_P31 (SEQ. ID NO:228), a second amino acid sequence being at least about 90% homologous to amino acids 369 - 484 of Q6MZS9_HUMAN (SEQ. ID NO:211), which also corresponds to amino acids 352 - 467 of C03950_3_P31 (SEQ. ID NO:228), a bridging amino acid N corresponding to amino acid 468 of C03950_3_P31 (SEQ. ID NO:228), a third amino acid sequence being at least about 90% homologous to amino acids 486 - 708 of Q6MZS9_HUMAN (SEQ. ID NO:211), which also corresponds to amino acids 469 - 691 of C03950_3_P31 (SEQ. ID NO:228), and a fourth amino acid sequence being at least about 70%, optionally at least about 80%, preferably at least about 85%, more preferably at least about 90% and most preferably at least about 95% homologous to a polypeptide having the sequence SHNILLYEDGHAVVADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEAKSRPSHYPV SSVYTETLKKKNEDRFGMWIEYLRR (SEQ. ID NO: 358) corresponding to amino acids 692 - 843 of C03950_3_P31 (SEQ. ID NO:228), wherein said , first amino acid sequence, bridging amino acid, second amino acid sequence, bridging amino acid, third amino acid sequence and fourth amino acid sequence are contiguous and in a sequential order.

In some embodiments, this invention provides an isolated polypeptide comprising an edge portion of C03950_3_P31 (SEQ. ID NO:228), comprising an amino acid sequence being at least about 70%, optionally at least about 80%, preferably at least about 85%, more preferably at least about 90% and most preferably at least about 95% homologous to the sequence -

SHNILLYEDGHAVVADFGESRFLQSLDEDNMTKQPGNLRWMAPEVFTQCTRYTIKADVFSYA
LCLWEILTGEIPFAHLKPAAAAADMAYHHIRPPIGYSIPKPISSLLIRGWNACPEAKSRPSHYPV
SSVYTETLKKKNEDRFGMWIEYLRR (SEQ. ID NO: 358) of C03950_3_P31 (SEQ. ID NO:228).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence-

MAAARDPPEVSLREATQRKLRRFSELRGKLVARGEFWDIVAITAADEKQELAYNQQLSEKLK
RKELPLGVQYHVFVDPAGAKIGNGGSTLCALQCLEKLYGDKWNSFTILLIHS (SEQ. ID NO:
379) corresponding to amino acids 1 - 114 of C03950_3_P31 (SEQ. ID NO:228), a second amino acid sequence being at least about 90% homologous to amino acids 14 - 707 of NP_057062 (SEQ. ID NO:210), which also corresponds to amino acids 115 - 808 of C03950_3_P31 (SEQ. ID NO:228), and a third amino acid sequence being at least about 70%, optionally at least about 80%, preferably at least about 85%, more preferably at least about 90% and most preferably at least about 95% homologous to a polypeptide having the sequence AKSRPSHYPVSSVYTETLKKKNEDRFGMWIEYLRR (SEQ. ID NO: 356) corresponding to amino acids 809 - 843 of C03950_3_P31 (SEQ. ID NO:228), wherein said first amino acid sequence, second amino acid sequence and third amino acid sequence are contiguous and in a sequential order.

In some embodiments, this invention provides an isolated polypeptide comprising a head of C03950_3_P31 (SEQ. ID NO:228), comprising a polypeptide being at least about 70%, optionally at least about 80%, preferably at least about 85%, more preferably at least about 90% and most preferably at least about 95% homologous to the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) of C03950_3_P31 (SEQ. ID NO:228).

In some embodiments, this invention provides an isolated polypeptide comprising an edge portion of C03950_3_P31 (SEQ. ID NO:228), comprising an amino acid sequence being at least about 70%, optionally at least about 80%, preferably at least about 85%, more preferably at least about 90% and most preferably at least about 95% homologous to the sequence AKSRPSHYPVSSVYTETLKKKNEDRFGMWIEYLRR (SEQ. ID NO: 356) of C03950_3_P31 (SEQ. ID NO:228).

In some embodiments, the isolated chimeric proteins or polypeptides of the invention comprise an amino acid sequence corresponding to or homologous to C03950_3_P33 (SEQ. ID NO:229).

In some embodiments, such isolated chimeric proteins or polypeptides comprise a first amino acid sequence being at least about 90% homologous to amino acids 1 - 691 of TNI3K_HUMAN (SEQ. ID NO: 396), which also corresponds to amino acids 1 - 691 of C03950_3_P33 (SEQ. ID NO:229), and a second amino acid sequence being at least about 70%, optionally at least about 80%, preferably at least about 85%, more preferably at least about 90% and most preferably at least about 95% homologous to a polypeptide having the sequence RYFFPK (SEQ. ID NO: 364) corresponding to amino acids 692 - 697 of C03950_3_P33 (SEQ. ID NO:229), wherein said first amino acid sequence and second amino acid sequence are contiguous and in a sequential order.

In some embodiments, this invention provides an isolated polypeptide comprising an edge portion of C03950_3_P33 (SEQ. ID NO:229), comprising an amino acid sequence being at least about 70%, optionally at least about 80%, preferably at least about 85%, more preferably at least about 90% and most preferably at least about 95% homologous to the sequence RYFFPK (SEQ. ID NO: 364) of C03950_3_P33 (SEQ. ID NO:229).

In some embodiments, such isolated chimeric proteins or polypeptides comprise a first amino acid sequence being at least about 90% homologous to amino acids 18 - 367 of Q6MZS9_HUMAN (SEQ. ID NO:211), which also corresponds to amino acids 1 - 350 of C03950_3_P33 (SEQ. ID NO:229), a bridging amino acid I corresponding to amino acid 351 of C03950_3_P33 (SEQ. ID NO:229), a second amino acid sequence being at least about 90% homologous to amino acids 369 - 484 of Q6MZS9_HUMAN (SEQ. ID NO:211), which also corresponds to amino acids 352 - 467 of C03950_3_P33 (SEQ. ID NO:229), a bridging amino acid N corresponding to amino acid 468 of C03950_3_P33 (SEQ. ID NO:229), and a third amino acid sequence being at least about 90% homologous to amino acids 486 - 714 of Q6MZS9_HUMAN (SEQ. ID NO:211), which also corresponds to amino acids 469 - 697 of C03950_3_P33 (SEQ. ID NO:229), wherein said , first amino acid sequence, bridging amino acid, second amino acid sequence, bridging amino acid and third amino acid sequence are contiguous and in a sequential order.

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) corresponding to amino acids 1 - 114 of C03950_3_P33 (SEQ. ID NO:229), a second amino acid sequence being at least about 90% homologous to amino acids 14 - 590 of NP_057062 (SEQ. ID NO:210), which also corresponds to amino acids 115 - 691 of C03950_3_P33 (SEQ. ID NO:229), and a third amino acid sequence being at least about 70%, optionally at least about 80%, preferably at least about 85%, more preferably at least about 90% and most preferably at least about 95% homologous to a polypeptide having the sequence RYFFPK (SEQ. ID NO: 364) corresponding to amino acids 692 - 697 of C03950_3_P33 (SEQ. ID NO:229), wherein said first amino acid sequence, second amino acid sequence and third amino acid sequence are contiguous and in a sequential order.

In some embodiments, this invention provides an isolated polypeptide comprising a head of C03950_3_P33 (SEQ. ID NO:229), comprising a polypeptide being at least about 70%, optionally at least about 80%, preferably at least about 85%, more preferably at least about 90% and most preferably at least about 95% homologous to the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) of C03950_3_P33 (SEQ. ID NO:229).

In some embodiments, this invention provides an isolated polypeptide comprising an edge portion of C03950_3_P33 (SEQ. ID NO:229), comprising an amino acid sequence being at least about 70%, optionally at least about 80%, preferably at least about 85%, more preferably at least about 90% and most preferably at least about 95% homologous to the sequence RYFFPK (SEQ. ID NO: 364) of C03950_3_P33 (SEQ. ID NO:229).

In some embodiments, the isolated chimeric proteins or polypeptides of the invention comprise an amino acid sequence corresponding to or homologous to C03950_3_P35 (SEQ. ID NO:230).

In some embodiments, such isolated chimeric proteins or polypeptides comprise a first amino acid sequence being at least about 90% homologous to amino acids 1 - 657 of TNI3K_HUMAN (SEQ. ID NO: 396), which also corresponds to amino acids 1 - 657 of C03950_3_P35 (SEQ. ID NO:230), and a second amino acid sequence being at least about 70%, optionally at least about 80%, preferably at least about 85%, more preferably at least about 90% and most preferably at least about 95% homologous to a polypeptide having the sequence YGSFVLIYPWTFRRNYSCNTSEGFPLDEPSPFEI (SEQ. ID NO: 372) corresponding to amino acids 658 - 691 of C03950_3_P35 (SEQ. ID NO:230), wherein said first amino acid sequence and second amino acid sequence are contiguous and in a sequential order.

In some embodiments, this invention provides an isolated polypeptide comprising an edge portion of C03950_3_P35 (SEQ. ID NO:230), comprising an amino acid sequence being at least about 70%, optionally at least about 80%, preferably at least about 85%, more preferably at least about 90% and most preferably at least about 95% homologous to the sequence YGSFVLIYPWTFRRNYSCNTSEGFPLDEPSPFEI (SEQ. ID NO: 372) of C03950_3_P35 (SEQ. ID NO:230).

In some embodiments, such isolated chimeric proteins or polypeptides comprise a first amino acid sequence being at least about 90% homologous to amino acids 18 - 367 of Q6MZS9_HUMAN (SEQ. ID NO:211), which also corresponds to amino acids 1 - 350 of C03950_3_P35 (SEQ. ID NO:230), a bridging amino acid I corresponding to amino acid 351 of C03950_3_P35 (SEQ. ID NO:230), a second amino acid sequence being at least about 90% homologous to amino acids 369 - 484 of Q6MZS9_HUMAN (SEQ. ID NO:211), which also corresponds to amino acids 352 - 467 of C03950_3_P35 (SEQ. ID NO:230), a bridging amino acid N corresponding to amino acid 468 of C03950_3_P35 (SEQ. ID NO:230), a third amino acid sequence being at least about 90% homologous to amino acids 486 - 674 of Q6MZS9_HUMAN (SEQ. ID NO:211), which also corresponds to amino acids 469 - 657 of C03950_3_P35 (SEQ. ID NO:230), and a fourth amino acid sequence being at least about 70%, optionally at least about 80%, preferably at least about 85%, more preferably at least about 90% and most preferably at least about 95% homologous to a polypeptide having the sequence YGSFVLIYPWTFRRNYSCNTSEGFPLDEPSPFEI (SEQ. ID NO: 372) corresponding to amino acids 658 - 691 of C03950_3_P35 (SEQ. ID NO:230), wherein said , first amino acid sequence, bridging amino acid, second amino acid sequence, bridging amino acid, third amino acid sequence and fourth amino acid sequence are contiguous and in a sequential order.

In some embodiments, this invention provides an isolated polypeptide comprising an edge portion of C03950_3_P35 (SEQ. ID NO:230), comprising an amino acid sequence being at least about 70%, optionally at least about 80%, preferably at least about 85%, more preferably at least about 90% and most preferably at least about 95% homologous to the sequence YGSFVLIYPWTFRRNYSCNTSEGFPLDEPSPFEI (SEQ. ID NO: 372) of C03950_3_P35 (SEQ. ID NO:230).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence - MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) corresponding to amino acids 1 - 114 of C03950_3_P35 (SEQ. ID NO:230), a second amino acid sequence being at least about 90% homologous to amino acids 14 - 556 of NP_057062 (SEQ. ID NO:210), which also corresponds to amino acids 115 - 657 of C03950_3_P35 (SEQ. ID NO:230), and a third amino acid sequence being at least about 70%, optionally at least about 80%, preferably at least about 85%, more preferably at least about 90% and most preferably at least about 95% homologous to a polypeptide having the sequence YGSFVLIYPWTFRRNYSCNTSEGFPLDEPSPFEI (SEQ. ID NO: 372) corresponding to amino acids 658 - 691 of C03950_3_P35 (SEQ. ID NO:230), wherein said first amino acid sequence, second amino acid sequence and third amino acid sequence are contiguous and in a sequential order.

In some embodiments, this invention provides an isolated polypeptide comprising a head of C03950_3_P35 (SEQ. ID NO:230), comprising a polypeptide being at least about 70%, optionally at least about 80%, preferably at least about 85%, more preferably at least about 90% and most preferably at least about 95% homologous to the sequence -

MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) of C03950_3_P35 (SEQ. ID NO:230).

According to yet a further embodiment, the present invention now discloses a novel cluster designated herein R15601, comprising novel amino acid and nucleic acid sequences that are variants of the known protein cardiomyopathy associated 4 (SEQ. ID NO:267).

The novel polynucleotides and polypeptides described by the present invention are useful as diagnostic markers, preferably as serum markers.

Surprisingly, the present invention now shows that the R15601 variants are expressed specifically in heart tissues, and thus can indicate the onset, severity or prognosis of cardiovascular disease in a subject, and can be used for the selection of treatment, treatment monitoring, diagnosis or prognosis assessment of any cardiovascular disease, including, inter alia, myocardial infarct, acute coronary syndrome, coronary artery disease, angina pectoris (stable and unstable), cardiomyopathy, myocarditis, congestive heart failure or any type of heart failure, reinfarction, assessment of thrombolytic therapy, assessment of myocardial infarct size, differential diagnosis between heart-related versus lung-related conditions (such as pulmonary embolism), the differential diagnosis of Dyspnea, cardiac valves related conditions, vascular disease, or any combination thereof, as is described in a greater detail below.

In some embodiments, the isolated chimeric proteins or polypeptides of the invention comprise an amino acid sequence corresponding to or homologous to R15601_P2 (SEQ. ID NO:268).

In some embodiments, such isolated chimeric proteins or polypeptides comprise a first amino acid sequence being at least about 70%, optionally at least about 80%, preferably at least about 85%, more preferably at least about 90% and most preferably at least about 95%, homologous to a polypeptide having the sequence MPRKDRNSSRAESAQCQVLSCVIHGILLMAREIAVVVLPLSQ (SEQ. ID NO: 388) corresponding to amino acids 1 - 42 of R15601_P2 (SEQ. ID NO:268), and a second amino acid sequence being at least about 90% homologous to amino acids 57 - 931 of NP_775259 (SEQ. ID NO: 397), which also corresponds to amino acids 43 - 917 of R15601_P2 (SEQ ID NO:268), wherein said first amino acid sequence and second amino acid sequence are contiguous and in a sequential order.

In some embodiments, this invention provides an isolated polypeptide comprising a head of R15601_P2 (SEQ. ID NO:268), comprising a polypeptide being at least about 70%, optionally at least about 80%, preferably at least about 85%, more preferably at least about 90% and most preferably at least about 95% homologous to the sequence MPRKDRNSSRAESAQCQVLSCVIHGILLMAREIAVVVLPLSQ (SEQ. ID NO: 388) of R15601_P2 (SEQ. ID NO:268).

In some embodiments, the isolated chimeric proteins or polypeptides of the invention comprise an amino acid sequence corresponding to or homologous to R15601_P3 (SEQ. ID NO:269).

In some embodiments, such isolated chimeric proteins or polypeptides comprise a first amino acid sequence being at least about 90% homologous to amino acids 1 - 565 of NP_775259 (SEQ. ID NO: 397), which also corresponds to amino acids 1 - 565 of R15601_P3 (SEQ. ID NO:269), and a second amino acid sequence being at least about 70%, optionally at least about 80%, preferably at least about 85%, more preferably at least about 90% and most preferably at least about 95% homologous to a polypeptide having the sequence VGESGPTTNLRKGLLGPDPQGMDPSLPPGSTPYPCINMIGYFPLSGPHFT (SEQ. ID NO: 389) corresponding to amino acids 566 - 615 of R15601_P3 (SEQ. ID NO: 269), wherein said first amino acid sequence and second amino acid sequence are contiguous and in a sequential order.

In some embodiments, this invention provides an isolated polypeptide comprising an edge portion of R15601_P3 (SEQ. ID NO:269), comprising an amino acid sequence being at least about 70%, optionally at least about 80%, preferably at least about 85%, more preferably at least about 90% and most preferably at least about 95% homologous to the sequence VGESGPTTNLRKGLLGPDPQGMDPSLPPGSTPYPCINMIGYFPLSGPHFT (SEQ. ID NO: 389) of R15601_P3 (SEQ. ID NO:269).

According to an additional embodiment, the present invention now discloses a novel cluster designated herein T11811, comprising novel amino acid and nucleic acid sequences that are variants of the known protein Myosin regulatory light chain 2, atrial isoform (SwissProt accession identifier MLRA_HUMAN (SEQ. ID NO: 398).

The novel polynucleotides and polypeptides described by the present invention are useful as diagnostic markers, preferably as serum markers.

Surprisingly, the present invention now shows that the T11811 variants are expressed specifically in heart tissue, and thus can indicate the onset, severity or prognosis of cardiovascular disease in a subject, and can be used for the selection of treatment, treatment monitoring, diagnosis or prognosis assessment of any cardiovascular disease, including, inter alia, myocardial infarct, acute coronary syndrome, coronary artery disease, angina pectoris (stable and unstable), cardiomyopathy, myocarditis, congestive heart failure or any type of heart failure, reinfarction, assessment of thrombolytic therapy, assessment of myocardial infarct size, differential diagnosis between heart-related versus lung-related conditions (such as pulmonary embolism), the differential diagnosis of Dyspnea, cardiac valves related conditions, vascular disease, or any combination thereof, as is described in a greater detail below.

In some embodiments, the isolated chimeric proteins or polypeptides of the invention comprise an amino acid sequence corresponding to or homologous to T11811_P2 (SEQ. ID NO:303).

In some embodiments, such isolated chimeric proteins or polypeptides comprise a first amino acid sequence being at least about 90% homologous to amino acids 1 - 142 of MLRA_HUMAN (SEQ. ID NO: 398), which also corresponds to amino acids 1 - 142 of T11811_P2 (SEQ. ID NO:303), a second amino acid sequence being at least about 70%, optionally at least about 80%, preferably at least about 85%, more preferably at least about 90% and most preferably at least about 95% homologous to a polypeptide having the sequence VRLPSPFNTHPQHLLWAFTHDPEPSTSEAVAGR (SEQ. ID NO: 390) corresponding to amino acids 143 - 175 of T11811_P2 (SEQ. ID NO:303), and a third amino acid sequence being at least about 90% homologous to amino acids 143 - 175 of MLRA_HUMAN (SEQ. ID NO: 398), which also corresponds to amino acids 176 - 208 of T11811_P2 (SEQ. ID NO:303), wherein said first amino acid sequence, second amino acid sequence and third amino acid sequence are contiguous and in a sequential order.

In some embodiments, this invention provides an isolated polypeptide comprising an edge portion of T11811_P2 (SEQ. ID NO:303), comprising an amino acid sequence being at least about 70%, optionally at least about 80%, preferably at least about 85%, more preferably at least about 90% and most preferably at least about 95% homologous to the sequence VRLPSPFNTHPQHLLWAFTHDPEPSTSEAVAGR (SEQ. ID NO: 390) of T11811_P2 (SEQ. ID NO:303).

In some embodiments, the isolated chimeric proteins or polypeptides of the invention comprise an amino acid sequence corresponding to or homologous to T11811_P4 (SEQ. ID NO:304):

In some embodiments, such isolated chimeric proteins or polypeptides comprise a first amino acid sequence being at least about 90% homologous to amino acids 1 - 125 of MLRA_HUMAN (SEQ. ID NO: 398), which also corresponds to amino acids 1 - 125 of T11811_P4 (SEQ. ID NO:304), and a second amino acid sequence being at least about 70%, optionally at least about 80%, preferably at least about 85%, more preferably at least about 90% and most preferably at least about 95% homologous to a polypeptide having the sequence DQPFPAPWEPPYPPSLCSHSPAVSCSDPPHPPGSSSFS (SEQ. ID NO: 391) corresponding to amino acids 126 - 163 of T11811_P4 (SEQ. ID NO:304), wherein said first amino acid sequence and second amino acid sequence are contiguous and in a sequential order.

In some embodiments, this invention provides an isolated polypeptide comprising an edge portion of T11811_P4 (SEQ. ID NO:304), comprising an amino acid sequence being at least about 70%, optionally at least about 80%, preferably at least about 85%, more preferably at least about 90% and most preferably at least about 95% homologous to the sequence DQPFPAPWEPPYPPSLCSHSPAVSCSDPPHPPGSSSFS (SEQ. ID NO: 391) of T11811_P4 (SEQ. ID NO: 304).

In some embodiments, the isolated chimeric proteins or polypeptides of the invention comprise an amino acid sequence corresponding to or homologous to T11811_P7 (SEQ. ID NO:305).

In some embodiments, such isolated chimeric proteins or polypeptides comprise a first amino acid sequence being at least about 90% homologous to amino acids 1 - 39 of MLRA_HUMAN (SEQ. ID NO: 398), which also corresponds to amino acids 1 - 39 of T11811_P7 (SEQ. ID NO:305), a second amino acid sequence being at least about 70%, optionally at least about 80%, preferably at least about 85%, more preferably at least about 90% and most preferably at least about 95% homologous to a polypeptide having the sequence VSPPPPTFPRAGGCSHLKAPIPQ (SEQ. ID NO: 392) corresponding to amino acids 40 - 62 of T11811_P7 (SEQ. ID NO:305), and a third amino acid sequence being at least about 90% homologous to amino acids 40 - 175 of MLRA_HUMAN (SEQ. ID NO: 398), which also corresponds to amino acids 63 - 198 of T11811_P7 (SEQ. ID NO:305), wherein said first amino acid sequence, second amino acid sequence and third amino acid sequence are contiguous and in a sequential order.

In some embodiments, this invention provides an isolated polypeptide comprising an edge portion of T11811_P7 (SEQ. ID NO:305), comprising an amino acid sequence being at least about 70%, optionally at least about 80%, preferably at least about 85%, more preferably at least about 90% and most preferably at least about 95% homologous to the sequence VSPPPPTFPRAGGCSHLKAPIPQ (SEQ. ID NO: 392) of T11811_P7 (SEQ. ID NO:305).

In some embodiments, the isolated chimeric proteins or polypeptides of the invention comprise an amino acid sequence corresponding to or homologous to T11811_P8 (SEQ. ID NO:306).

In some embodiments, such isolated chimeric proteins or polypeptides comprise a first amino acid sequence being at least about 90% homologous to amino acids 1 - 126 of MLRA_HUMAN (SEQ. ID NO: 398), which also corresponds to amino acids 1 - 126 of T11811_P8 (SEQ. ID NO:306), and a second amino acid sequence being at least about 70%, optionally at least about 80%, preferably at least about 85%, more preferably at least about 90% and most preferably at least about 95% homologous to a polypeptide having the sequence WSRCSP (SEQ. ID NO: 393) corresponding to amino acids 127 - 132 of T11811_P8 (SEQ. ID NO:306), wherein said first amino acid sequence and second amino acid sequence are contiguous and in a sequential order.

In some embodiments, this invention provides an isolated polypeptide comprising an edge portion of T11811_P8 (SEQ. ID NO:306), comprising an amino acid sequence being at least about 70%, optionally at least about 80%, preferably at least about 85%, more preferably at least about 90% and most preferably at least about 95% homologous to the sequence WSRCSP (SEQ. ID NO: 393) of T11811_P8 (SEQ. ID NO:306).

According to certain embodiments, the polypeptides of this invention comprise variants of known proteins, and in other embodiments the polypeptides of this invention comprise splice variants of native proteins expressed in a given subject. In some embodiments, the polypeptides may be obtained through known protein evolution techniques available in the art. In other embodiments, the polypeptides of this invention may be obtained via rational design, based on a particular native polypeptide sequence.

According to another aspect the present invention provides antibodies or antibody fragments specifically interacting with or recognizing a polypeptide of this invention.

According to certain embodiments, the antibody recognizes one or more epitopes (antigen determinants) contained within the polypeptides of this invention, wherein such that binding of the antibody to an epitope distinguish between the splice variants of the present invention and a known polypeptide or protein. Reference to the antibody property of "specific interaction" or "recognition" is to be understood as including covalent and non-covalent associations with a variance of affinity over several orders of magnitude. These terms are to be understood as relative with respect to an index molecule, for which the antibody is thought to have little to no specific interaction or recognition. In one embodiment, the antibodies specifically interact or recognize a particular antigen determinant.

In certain embodiments, the antibodies or antibody fragments of this invention recognize or interact with a polypeptide or protein of the invention, while not substantially recognize or interact with other molecules, even when present in the same sample, for example a biological sample. According to one embodiment, the antibodies of this invention have a specificity such that the specific interaction with or binding to the antigen is at least about 2, or in another embodiment, at least about 5, or in still further embodiment, at least about 10-fold greater than interaction or binding observed under the same reaction conditions with a molecule that does not include the antigenic determinant.

According to certain embodiments, the antibodies are useful in detecting qualitative and/or quantitative changes in the expression of the polypeptides or polynucleotides of this invention. In some embodiments, changes in expression are associated with a particular disease or disorder, such that detection of the changes comprises a diagnostic method of the present invention.

According to other embodiments, the present invention provides an antibody capable of specifically binding to at least one epitope of a polypeptide comprising an amino acid sequence as set forth in any one of SEQ. ID NOs: 57-59, 63, 76, 77, 134-143, 212-230, 268-269, 303-308, 326-394.

According to additional aspect the present invention provides a diagnostic kit for detecting a disease, comprising markers and reagents for detecting qualitative and/or quantitative changes in the expression of a polypeptide or a polynucleotide of this invention.

According to on embodiment, the kit comprises markers and reagents for detecting the changes by employing a NAT-based technology. In one embodiment, the NAT-based assay is selected from the group consisting of a PCR, Real-Time PCR, LCR, Self-Sustained Synthetic Reaction, Q-Beta Replicase, Cycling Probe Reaction, Branched DNA, RFLP analysis, DGGE/TGGE, Single-Strand Conformation Polymorphism, Dideoxy Fingerprinting, Microarrays, Fluorescence In Situ Hybridization or Comparative Genomic Hybridization.

According to certain currently preferred embodiments, the kit comprises at least one nucleotide probe or primer. In one embodiment, the kit comprises at least one primer pair capable of selectively hybridizing to a nucleic acid sequence according to the teaching of the present invention. In another embodiment, the kit comprises at least one oligonucleotide capable of selectively hybridizing to a nucleic acid sequence according to the teaching of the present invention.

According to other currently preferred embodiments, the kit comprises an antibody capable of recognizing or interacting with a polypeptide or protein of the present invention. According to certain embodiments, the kit further comprises at least one reagent for performing an ELISA, an RIA, a slot blot, an immunohistochemical assay, FACS, in-vivo imaging, a radio-imaging assay, or a Western blot.

The present invention further provides diagnostic methods for screening for a disease, disorder or conditions, comprising the detection of a polypeptide or polynucleotide of this invention, whereby expression, or relative changes in expression of the polypeptide or polynucleotide herald the onset, severity, or prognosis of an individual with regard to a particular disease, disorder or condition. The detection may comprise detection of the expression of a specific splice variant, or other polypeptide or polynucleotide of this invention, via any means known in the art, and as described herein.

As used herein, the term "screening for a disease" encompasses diagnosing the presence of a disease, its prognosis and/or severity, as well as selecting a treatment and monitoring the treatment of the disease. According to certain currently preferred embodiments, the disease is a marker-detectable disease, wherein the marker is a polynucleotide, polypeptide or protein according to the present invention.

Thus, according to certain aspects, the present invention provides methods for screening for a marker detectable disease, comprising detecting in a subject or in a sample obtained from the subject at least one transcript and/or protein or polypeptide being a member of a cluster selected from the group consisting of cluster N43992, cluster D12115, cluster C03950, cluster R15601, cluster T11811, or any combination thereof. According to certain currently preferred embodiments, the method comprises detecting the expression of a splice variant transcript or a product thereof.

According to one aspect, the present invention provide a method for screening for a marker detectable disease in a subject, comprising (a) obtaining a sample from the subject and (b) detecting in the sample at least one polynucleotide and/or polypeptide being a member of a cluster selected from the group consisting of cluster C03950, cluster R15601, cluster T11811, or any combination thereof. According to one embodiment, the presence of the polynucleotide or polypeptide in the sample is indicative of the presence of the disease and/or its severity and/or its progress. According to another embodiment, a change in the level of the polynucleotide or polypeptide in the sample compared to its level in a sample obtained from a healthy subject is indicative of the presence of the disease and/or its severity. According to another embodiment, a change in the level of the polynucleotide or polypeptide in the sample compared to its level in a sample previously obtained from said subject is indicative of the presence of the disease, its severity and/or the progress of the disease.

According to one embodiment, the present invention provides a method for screening for a cardiovascular disease in a subject, comprising (a) obtaining a sample from the subject and (b) detecting in the sample at least one polypeptide being a member of a cluster selected from the group consisting of cluster C03950, cluster R15601, cluster T11811, or any combination thereof. According to one embodiment, the presence of the polypeptide in the sample is indicative of the presence of the disease and/or its severity and/or its progress. According to another embodiment, a change in the level of the polypeptide in the sample compared to its level in a sample obtained from a healthy subject is indicative of the presence of the disease and/or its severity. According to another embodiment, a change in the level of the polypeptide in the sample compared to its level in a sample previously obtained from said subject is indicative of the presence of the disease, its severity and/or the progress of the disease. According to currently preferred embodiments, the sample is a serum sample.

According to other embodiments, the cardiovascular disease include inter alia, myocardial infarct, acute coronary syndrome, coronary artery disease, angina pectoris (stable and unstable), cardiomyopathy, myocarditis, congestive heart failure or any type of heart failure and reinfarction. According to other embodiments, the method is useful for the assessment of thrombolytic therapy, assessment of myocardial infarct size, differential diagnosis between heart-related versus lung-related conditions (such as pulmonary embolism), the differential diagnosis of Dyspnea, cardiac valves related conditions, vascular disease, or any combination thereof. In further embodiments, the polypeptides of any one of the clusters C03950, R15601, T11811, or a combination thereof, are useful in the diagnosis, treatment or assessment of the prognosis of a subject with congestive heart failure (CHF). According to still other embodiments, they are useful in the diagnosis, treatment or assessment of the prognosis of a subject with sudden cardiac death, from arrhythmia or any other heart related reason; rejection of a transplanted heart; conditions that lead to heart failure including but not limited to myocardial infarction, angina, arrhythmias, valvular diseases, atrial and/or ventricular septal defects; conditions that cause atrial and or ventricular wall volume overload, including but not limited to systemic arterial hypertension, pulmonary hypertension and pulmonary embolism; conditions which have similar clinical symptoms as heart failure and as states that cause atrial and or ventricular pressure-overload, where the differential diagnosis between these conditions to the latter is of clinical importance including but not limited to breathing difficulty and/or hypoxia due to pulmonary disease, anemia or anxiety.

Each polypeptide of the C03950 variants, R15601 variants, and/or T11811 variants described herein as a marker for cardiovascular conditions, can be used alone or in combination with one or more other variant markers described herein, and/or in combination with known markers for cardiovascular conditions, including but not limited to Heart-type fatty acid binding protein (H-FABP), Angiotensin, C-reactive protein (CRP), myeloperoxidase (MPO), and/or in combination with the known protein(s) for the variant marker as described herein.

The present invention further discloses that surprisingly, detecting in a subject at least one polypeptide or polynucleotide of cluster N43992, including the known N43992 polypeptide and/or polynucleotide sequences as well as variants thereof as disclosed in the present invention, are indicative of cancer, particularly lung cancer. Detecting the presence of the polynucleotide or polypeptide in the subject or detecting a relative change in their expression and/or level compared to a healthy subject or compared to their expression and/or level in said subject at an earlier stage is indicative of the presence, onset, severity or prognosis, and/or staging, and/or progression, of lung cancer in said subject. These polynucleotides and polypeptides of cluster N43992 are also useful for treatment selection and treatment monitoring of lung cancer, which may be an invasive lung cancer and/or metastatic lung cancer.

Thus, according to another aspect, the present invention provides a method for screening for a cancer in a subject, comprising detecting in the subject at least one polynucleotide and/or polypeptide being a member of cluster N43992.

According to one embodiment, the polypeptide or polynucleotide is at least 85% homologous to the wild type protein DLL3 or a polynucleotide encoding same, respectively, or a fragment thereof. As the wild type DLL3 protein is a type I membrane protein it is used as a diagnostic marker preferably with in vivo imaging technologies, including but not limited to magnetic resonance imaging, computed tomography scanning, PET, SPECT and the like. Optionally, according to the present invention, the wild type DLL3 protein diagnostic marker is used as IHC marker.

According to another embodiment, the polypeptide or polynucleotide is at least 85% homologous to a secreted splice variant of protein DLL3 or a polynucleotide encoding same, respectively, or a fragment thereof. According to this embodiment, the method for screening for a cancer is performed in vitro with a sample obtained from the subject.

According to another aspect, the present invention provides a method for screening for cancer in a subject, comprising detecting in the subject a polypeptide comprising an amino acid sequence at least 85% homologous to the amino acid sequence set forth in any one of SEQ. ID NOs: 54-59, 63, 76 and 77. According to one embodiment, the method comprises detecting a polypeptide comprising an amino acid sequence as set forth in any one of SEQ. ID NOs: 54-59, 63, 76 and 77.

According to yet another aspect, the present invention provides a method for screening for cancer in a subject, comprising detecting in the subject a polynucleotide comprising a nucleic acid sequence at least 85% homologous to the nucleic acid sequence set forth in any one of SEQ. ID NOs: 37-39, 40-53, 74-75. According to one embodiment, the method comprises detecting a polynucleotide comprising a nucleic acid sequence as set forth in any one of SEQ. ID NOs: 37-39, 40-53, 74-75.

According to one embodiment, the cancer is a lung cancer, wherein the lung cancer can be invasive or metastatic.

In other aspects, the present invention discloses that detection of polypeptides or polynucleotides of cluster D12115 variants, or relative changes in expression and/or level of these variants and their products is indicative of the presence, onset, severity or prognosis, and/or staging, and/or progression of cancer, including but not limited to ovarian cancer, lung cancer or breast cancer, in a subject. In some embodiments, the polypeptides, polynucleotides and/or methods of this invention may be useful in the treatment selection and monitoring, diagnosis or prognosis assessment of cancer, including but not limited to ovarian cancer, lung cancer or breast cancer, or ovarian, breast or lung cancer invasion and metastasis.

With regard to lung cancer, the disease is selected from the group consisting of invasive or metastatic lung cancer; squamous cell lung carcinoma, lung adenocarcinoma, carcinoid, small cell lung cancer or non-small cell lung cancer; detection of overexpression in lung metastasis (vs. primary tumor); detection of overexpression in lung cancer, for example non small cell lung cancer, for example adenocarcinoma, squamous cell cancer or carcinoid, or large cell carcinoma; identification of a metastasis of unknown origin which originated from a primary lung cancer; assessment of a malignant tissue residing in the lung that is from a non-lung origin, including but not limited to: osteogenic and soft tissue sarcomas; colorectal, uterine, cervix and corpus tumors; head and neck, breast, testis and salivary gland cancers; melanoma; and bladder and kidney tumors; distinguishing between different types of lung cancer, therefore potentially affecting treatment choice (e.g. small cell vs. non small cell tumors); analysis of unexplained dyspnea and/or chronic cough and/or hemoptysis; differential diagnosis of the origin of a pleural effusion; diagnosis of conditions which have similar symptoms, signs and complications as lung cancer and where the differential diagnosis between them and lung cancer is of clinical importance including but not limited to: non-malignant causes of lung symptoms and signs, including but not limited to: lung lesions and infiltrates, wheeze, stridor, tracheal obstruction, esophageal compression, dysphagia, recurrent laryngeal nerve paralysis, hoarseness, phrenic nerve paralysis with elevation of the hemidiaphragm and Horner syndrome; or detecting a cause of any condition suggestive of a malignant tumor including but not limited to anorexia, cachexia, weight loss, fever, hypercalcemia, hypophosphatemia, hyponatremia, syndrome of inappropriate secretion of antidiuretic hormone, elevated ANP, elevated ACTH, hypokalemia, clubbing, neurologic-myopathic syndromes and thrombophlebitis.

The polypeptides and/or polynucleotides of cluster D12115 and/or N43992 used as markers for lung cancer can be used alone or in combination with one or more alternative polynucleotides or polypeptides described herein, and/or in combination with known markers for lung cancer, including but not limited to CEA, CA15-3, Beta-2-microglobulin, CA19-9, TPA, and/or in combination with the known protein(s) for the variant marker as described herein.

With regard to ovarian cancer, the polypeptides and/or polynucleotide of cluster D12115 of the present invention can be used in the diagnosis, treatment or prognostic assessment of invasive or metastatic ovarian cancer; correlating stage and malignant potential; identification of a metastasis of unknown origin which originated from a primary ovarian cancer; differential diagnosis between benign and malignant ovarian cysts; diagnosing a cause of infertility, for example differential diagnosis of various causes thereof; detecting of one or more non-ovarian cancer conditions that may elevate serum levels of ovary related markers, including but not limited to: cancers of the endometrium, cervix, fallopian tubes, pancreas, breast, lung and colon; nonmalignant conditions such as pregnancy, endometriosis, pelvic inflammatory disease and uterine fibroids; diagnosing conditions which have similar symptoms, signs and complications as ovarian cancer and where the differential diagnosis between them and ovarian cancer is of clinical importance including but not limited to: non-malignant causes of pelvic mass, including, but not limited to: benign (functional) ovarian cyst, uterine fibroids, endometriosis, benign ovarian neoplasms and inflammatory bowel lesions; determining a cause of any condition suggestive of a malignant tumor including but not limited to anorexia, cachexia, weight loss, fever, hypercalcemia, skeletal or abdominal pain, paraneoplastic syndrome, or ascites.

The polypeptides and/or polynucleotides of cluster D12115 used in the diagnosis, treatment or prognostic assessment of ovarian cancer can be used alone or in combination with one or more polypeptides and/or polynucleotides of this invention, and/or in combination with known markers for ovarian cancer, including but not limited to CEA, CA125 (Mucin 16), CA72-4TAG, CA-50, CA 54-61, CA-195 and CA 19-9 in combination with CA-125, and/or in combination with the known protein(s) associated with the indicated polypeptide or polynucleotide, as described herein.

With regard to breast cancer, the polypeptides and/or polynucleotides of cluster D12115 are useful in determining a probable outcome in breast cancer; identification of a metastasis of unknown origin which originated from a primary breast cancer tumor; assessing lymphadenopathy, and in particular axillary lymphadenopathy; distinguishing between different types of breast cancer, therefore potentially affect treatment choice (e.g. as HER-2); differentially diagnosing between a benign and malignant breast mass; as a tool in the assessment of conditions affecting breast skin (e.g. Paget's disease) and their differentiation from breast cancer; differential diagnosis of breast pain or discomfort resulting from either breast cancer or other possible conditions (e.g. mastitis, Mondors syndrome); non-breast cancer conditions which have similar symptoms, signs and complications as breast cancer and where the differential diagnosis between them and breast cancer is of clinical importance including but not limited to: abnormal mammogram and/or nipple retraction and/or nipple discharge due to causes other than breast cancer, including but not limited to benign breast masses, melanoma, trauma and technical and/or anatomical variations; determining a cause of any condition suggestive of a malignant tumor including but not limited to anorexia, cachexia, weight loss, fever, hypercalcemia, paraneoplastic syndrome; or determining a cause of lymphadenopathy, weight loss and other signs and symptoms associated with breast cancer but originate from diseases different from breast cancer including but not limited to other malignancies, infections and autoimmune diseases.

Each variant marker of the present invention described herein as potential marker for breast cancer can be used alone or in combination with one or more other variant breast cancer described herein, and/or in combination with known markers for breast cancer, including but not limited to Calcitonin, CA15-3 (Mucin1), CA27-29, TPA, a combination of CA 15-3 and CEA, CA 27.29 (monoclonal antibody directed against MUC1), Estrogen 2 (beta), HER-2 (c-erbB2), and/or in combination with the known protein(s) for the variant marker as described herein.

According to certain embodiments, a combination of anyone of the polynucleotides or polypeptides markers of the present invention with another marker can be used for determining a ratio between a quantitative or semi-quantitative measurement of any marker described herein to any other marker described herein, and/or any other known marker, and/or any other marker. With regard to such a ratio between any marker described herein (or a combination thereof) and a known marker, the known marker preferably comprises the "known protein" as described in greater detail below with regard to each cluster or gene.

It is to be understood that any polynucleotide or polypeptide of this invention may be useful as a marker for a disease, disorder or condition, and such use is to be considered a part of this invention.

According to certain embodiments, detecting the expression of a polynucleotide or polypeptide according to the teaching of the present invention is performed by employing a NAT-based technology (optionally by employing at least one nucleotide probe or primer), or by employing an immunoassay (optionally by employing an antibody according to any of the embodiments described herein), respectively.

In some embodiments, this invention provides a method for screening for a disease in a subject, comprising detecting in the subject or in a sample obtained from said subject at least one polypeptide or polynucleotide selected from the group consisting of:

a. a polypeptide having an amino acid sequence as set forth in any one of SEQ. ID NOs: 54-59, 134-143, 212-230, 268-269, 303-308, or a homologue or a fragment thereof;

b. a polypeptide comprising a bridge, edge portion, tail, or head portion, wherein the polypeptide has an amino acid sequence as set forth in any one of SEQ. ID NOs: 326-394, or a homologue or a fragment thereof;

c. a polynucleotide having a nucleic acid sequence as set forth in any one of SEQ. ID NOs: 37-39, 74-75, 78-88, 156-170, 240-241, 276-281, or a homologue or a fragment thereof;

d. a polynucleotide comprising a node having a nucleic acid sequence as set forth in any one of SEQ. ID NOs: 40-53, 89-130, 171-208, 242-266, 282-301;

e. an oligonucleotide having a nucleic acid sequence as set forth in SEQ. ID NOs: 62, 64, 67, 68, 70, 71, 73, 146, 149, 152, 155, 233, 236, 239, 272, 275, 311, 314, 317, 320, 323-325.

According to one embodiment, detecting the presence of the polypeptide or polynucleotide is indicative of the presence of the disease and/or its severity and/or its progress. According to another embodiment, a change in the expression and/or the level of the polynucleotide or polypeptide compared to its expression and/or level in a healthy subject or a sample obtained therefrom is indicative of the presence of the disease and/or its severity and/or its progress. According to a further embodiment, a change in the expression and/or level of the polynucleotide or polypeptide compared to its level and/or expression in said subject or in a sample obtained therefrom at earlier stage is indicative of the progress of the disease. According to still further embodiment, detecting the presence and/or relative change in the expression and/or level of the polynucleotide or polypeptide is useful for selecting a treatment and/or monitoring a treatment of the disease.

According to one embodiment, detecting a polynucleotide of the invention comprises employing a primer pair, comprising a pair of isolated oligonucleotides capable of specifically hybridizing to at least a portion of a polynucleotide having a nucleic acid sequence as set forth in SEQ. ID NOs: 62, 67, 70, 73, 146, 149, 152, 155, 233, 236, 239, 272, 275, 311, 314, 317, 320, 323 or polynucleotides homologous thereto.

According to another embodiment, detecting a polynucleotide of the invention comprises employing a primer pair, comprising a pair of isolated oligonucleotides as set forth in SEQ. ID NOs:60-61, 65-66, 69, 72, 144-145, 147-148, 150-151, 153-154, 231-232, 234-235, 237-238, 270-271, 273-274, 309-310, 312-313, 315-316, 318-319, 321-322.

According to further embodiment, detecting a polypeptide of the invention comprises employing an antibody capable of specifically binding to at least one epitope of a polypeptide comprising an amino acid sequence as set forth in any one of SEQ. ID NOs: 57-59, 63, 76, 77, 134-143, 212-230, 268-269, 303-308, 326-394.

In some embodiments, a method of this invention may make use of a polynucleotide, polypeptide, vector, antibody, biomarker, or combination thereof, as described herein, including any embodiments thereof.

In some embodiments, the methods of this invention are conducted on a whole body. According to other embodiments, the methods of the present invention are conducted with a sample isolated from a subject having, predisposed to, or suspected of having the disease, disorder or condition. According to certain embodiments, the sample is a cell or tissue or a body fluid sample. In some embodiments, the methods are directed to the monitoring of disease progression and/or treatment efficacy and/or relapse of the indicated disease, disorder or condition.

In another embodiment, this invention provides methods for the selection of a particular therapy, or optimization of a given therapy for a disease, disorder or condition, the method comprising quantitatively and/or qualitatively determining or assessing expression of the polypeptides and/or polynucleotides, whereby differences in expression from an index sample, or a sample taken from a subject prior to the initiation of the therapy, or during the course of therapy, is indicative of the efficacy, or optimal activity of the therapy.

According to still further aspect, the present invention provides a method for detecting a splice variant nucleic acid sequence in a biological sample, comprising: hybridizing the isolated splice variant nucleic acid molecules or oligonucleotide fragments thereof of at least about 12 nucleotides to a nucleic acid material of the biological sample and detecting a hybridization complex; wherein the presence of the hybridization complex correlates with the presence of said splice variant nucleic acid sequence in the biological sample.

The nucleic acid sequences and/or amino acid sequences shown herein as embodiments of the present invention relate, in some embodiments, to their isolated form, as isolated polynucleotides (including for all transcripts), oligonucleotides (including for all segments, amplicons and primers), peptides (including for all tails, bridges, insertions or heads, optionally including other antibody epitopes as described herein) and/or polypeptides (including for all proteins). It should be noted that the terms "oligonucleotide" and "polynucleotide" and "nucleic acid molecule", or "peptide" and "polypeptide" and "protein", may optionally be used interchangeably.

All technical and scientific terms used herein should be understood to have the meaning commonly understood by a person skilled in the art to which this invention belongs, as well as any other specified description. The following references provide one of skill in the art with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). All of these are hereby incorporated by reference as if fully set forth herein.

## BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

Figure 1 shows a schematic description of the cancer biomarker selection engine.

Figure 2 shows a schematic illustration, depicting grouping of transcripts of a given cluster based on presence or absence of unique sequence regions.

Figure 3 shows a schematic presentation of the oligonucleotide based microarray fabrication.

Figure 4 shows a schematic summary of the oligonucleotide based microarray experimental flow.

Figure 5 shows a schematic summary of quantitative real-time PCR analysis.

Figure 6 shows a graph of cancer and cell-line vs. normal tissue expression for N43992.

Figures 7A-B is a histogram showing over expression of the Homo sapiens delta-like 3 (Drosophila) (DLL3) N43992 transcripts which are detectable by Taqman probes as depicted in sequence names N43992-T4 (SEQ. ID NO: 64) and N43992-T4II (SEQ. ID NO: 68) in normal and cancerous Lung tissues. Figure 7A is a histogram showing the results using N43992-T4 (SEQ. ID NO: 64) probe. Figure 7B is a histogram showing the results using N43992T4II (SEQ. ID NO: 68) probe.

Figure 8A is a histogram showing over expression of the Homo sapiens delta-like 3 (Drosophila) (DLL3) N43992 transcripts which are detectable by amplicon as depicted in sequence name N43992-seg12WTF2R2 (SEQ. ID NO: 62) in normal and cancerous Lung tissues.

Figure 8B is a histogram showing Expression of Homo sapiens delta-like 3 (Drosophila) (DLL3) N43992 transcripts which are detectable by Taqman probe as depicted in sequence names N43992T3 (SEQ. ID NO: 71) in normal and cancerous Lung tissues.

Figure 9 is a histogram showing expression of Homo sapiens delta-like 3 (Drosophila) (DLL3) N43992 transcripts which are detectable by Taqman probe as depicted in sequence names N43992-T4 (SEQ. ID NO: 64) in different normal tissues.

Figure 10A is a histogram showing over expression of the Homo sapiens delta-like 3 (Drosophila) (DLL3) N43992 transcripts which are detectable by amplicon as depicted in sequence name N43992_seg12WTF2R2 (SEQ. ID NO: 62) in different normal tissues.

Figure 10B is a histogram showing expression of Homo sapiens delta-like 3 (Drosophila) (DLL3) N43992 transcripts which are detectable by Taqman probe as depicted in sequence names N43992T3 (SEQ. ID NO: 71) in different normal tissues.

Figure 11 shows a graph of cancer and cell-line vs. normal tissue expression for D12115.

Figure 12 is a histogram showing expression of Homo sapiens B-factor, properdin (BF) D12115 transcripts which are detectable by junction 0-2 and segment 6 in normal and cancerous ovary (Figure 12A) and breast (Figure 12B) tissues.

Figure 13 is a histogram showing over expression of the Homo sapiens B-factor, properdin (BF) D12115 transcripts which are detectable by amplicon as depicted in sequence name D12115_seg4 (SEQ. ID NO: 146) in cancerous Breast samples relative to the normal samples.

Figure 14 is a histogram showing expression of the Homo sapiens B-factor, properdin (BF) D12115 transcripts which are detectable by amplicon as depicted in sequence name D12115_seg4 (SEQ. ID NO: 146) in different normal samples.

Figure 15 is a histogram showing over expression of the Homo sapiens B-factor, properdin (BF) D12115 transcripts which are detectable by amplicon as depicted in sequence name D12115_seg4 (SEQ. ID NO: 146) in cancerous ovarian samples relative to the normal samples.

Figure 16 is a histogram showing over expression of the Homo sapiens B-factor, properdin (BF) D12115 transcripts which are detectable by amplicon as depicted in sequence name D12115_seg6 (SEQ. ID NO: 149) in cancerous Breast samples relative to the normal samples.

Figure 17 is a histogram showing over expression of the Homo sapiens B-factor, properdin (BF) D12115 transcripts which are detectable by amplicon as depicted in sequence name D12115_seg6 (SEQ. ID NO: 149) in cancerous lung samples relative to the normal samples.

Figure 18 is a histogram showing expression of the Homo sapiens B-factor, properdin (BF) D12115 transcripts which are detectable by amplicon as depicted in sequence name D12115_seg6 (SEQ. ID NO: 149) in different normal samples.

Figure 19 is a histogram showing over expression of the Homo sapiens B-factor, properdin (BF) D12115 transcripts which are detectable by amplicon as depicted in sequence name D12115_seg6 (SEQ. ID NO: 149) in cancerous ovarian samples relative to the normal samples.

Figure 20 is a histogram showing expression of the Homo sapiens B-factor, properdin (BF) D12115 transcripts which are detectable by amplicon as depicted in sequence name D12115 seg40WT (SEQ. ID NO: 152) in different normal samples.

Figure 21 is a histogram showing expression of the Homo sapiens B-factor, properdin (BF) D12115 transcripts which are detectable by amplicon as depicted in sequence name D12115_ seg46-47 (SEQ. ID NO: 155) in different normal samples.

Figure 22 is a histogram showing over expression of the Homo sapiens B-factor, properdin (BF) D12115 transcripts which are detectable by amplicon as depicted in sequence name D12115_ seg46-47 (SEQ. ID NO: 155) in cancerous ovarian samples relative to the normal samples.

Figure 23 shows a graph of cancer and cell-line vs. normal tissue expression for C03950.

Figure 24 is a histogram showing relative expression of the above-indicated Homo sapiens TNNI3 interacting kinase (TNNI3K) transcripts which are detectable by amplicon as depicted in sequence name C03950_seg44WT (SEQ. ID NO: 233) in heart tissue samples as opposed to other tissues.

Figure 25 is a histogram showing relative expression of the above-indicated Homo sapiens TNIVI3 interacting kinase (TNNI3K) transcripts which are detectable by amplicon as depicted in sequence name C03950_seg51 (SEQ. ID NO: 236) in heart tissue samples as opposed to other tissues.

Figure 26 is a histogram showing relative expression of the above-indicated Homo sapiens TNNI3 interacting kinase (TNNI3K) transcripts which are detectable by amplicon as depicted in sequence name C03950_ seg67F2R2 (SEQ. ID NO: 239) in heart tissue samples as opposed to other tissues.

Figure 27 shows a graph of cancer and cell-line vs. normal tissue expression for R15601.

Figure 28 is a histogram showing relative expression of the above-indicated Homo sapiens cardiomyopathy associated 4 (CMYA4) transcripts, which are detectable by amplicon as depicted in sequence name R15601_seg28 (SEQ. ID NO: 272) in heart tissue samples as opposed to other tissues.

Figure 29 is a histogram showing relative expression of the above-indicated Homo sapiens cardiomyopathy associated 4 (CMYA4) transcripts, which are detectable by amplicon as depicted in sequence name R15601_seg30WT (SEQ. ID NO: 275) in heart tissue samples as opposed to other tissues.

Figure 30 is a histogram showing relative expression of the above-indicated Homo sapiens myosin, light polypeptide 7, regulatory (MYL7) transcripts, transcripts which are detectable by amplicon as depicted in sequence name T1 1811_seg14WT (SEQ. ID NO: 311) in heart tissue samples as opposed to other tissues.

Figure 31 is a histogram showing relative expression of the above-indicated Homo sapiens myosin, light polypeptide 7, regulatory (MYL7) transcripts, transcripts which are detectable by amplicon as depicted in sequence name T11811_seg7-8-9 (SEQ. ID NO: 314) in heart tissue samples as opposed to other tissues.

Figure 32 is a histogram showing relative expression of the above-indicated Homo sapiens myosin, light polypeptide 7, regulatory (MYL7) transcripts, transcripts which are detectable by amplicon as depicted in sequence name T11811_seg23 (SEQ. ID NO: 317) in heart tissue samples as opposed to other tissues.

## DESCRIPTION OF EMBODIMENTS

The present invention provides polynucleotides, polypeptides, particularly variants of known proteins, and uses thereof, particularly as diagnostic markers.

In some embodiments, the polypeptides and polynucleotides of the present invention are useful as diagnostic markers for certain diseases, and as such the term "marker-detectable" or "variant-detectable" with regard to a disease is to be understood as encompassing use of the described polynucleotides and/or polypeptides for diagnosis.

In some embodiments, certain diseases are associated with differential expression, qualitatively or quantitatively, of the polynucleotides and polypeptides of this invention. Assessment of such expression, in turn, can therefore serve as a marker for a particular disease state, susceptibility to a disease, pathogenesis, etc., including any desired disease-specific event, whose analysis is useful, as will be appreciated by one skilled in the art. In one embodiment, such use as a marker is also referred to herein as the polynucleotides and polypeptides being "variant disease markers".

The markers of the present invention, alone or in combination, can be used for prognosis, prediction, screening, early diagnosis, staging, therapy selection and treatment monitoring of a marker-detectable disease. For example, optionally and preferably, these markers may be used for staging the disease in patient (for example if the disease features cancer) and/or monitoring the progression of the disease. Furthermore, the markers of the present invention, alone or in combination, can be used for detection of the source of metastasis found in anatomical places other than the originating tissue, again in the example of cancer. Also, one or more of the markers may optionally be used in combination with one or more other disease markers (other than those described herein).

Biomolecular sequences (amino acid and/or nucleic acid sequences) uncovered using the methodology of the present invention and described herein can be efficiently utilized as tissue or pathological markers and/or as drugs or drug targets for treating or preventing a disease.

In some embodiments, these markers are specifically released to the bloodstream under conditions of a particular disease, and/or are otherwise expressed at a much higher level and/or specifically expressed in tissue or cells afflicted with or demonstrating the disease. The measurement of these markers, alone or in combination, in patient samples provides information that the diagnostician can correlate with a probable diagnosis of a particular disease and/or a condition that is indicative of a higher risk for a particular disease.

The present invention provides, in some embodiments, diagnostic assays for a marker-detectable disease and/or an indicative condition, and methods of use of such markers for detection of marker-detectable disease and/or an indicative condition, for example in a sample taken from a subject (patient), which in some embodiments, is a blood sample.

Some embodiments of this invention have been exemplified herein wherein cellular localization was determined according to four different software programs: (i) tmhmm (from Center for Biological Sequence Analysis, Technical University of Denmark DTU, http://www.cbs.dtu.dk/services/TMHMM/TMHMM2.0b.guide.php) or (ii) tmpred (from EMBnet, maintained by the ISREC Bionformatics group and the LICR Information Technology Office, Ludwig Institute for Cancer Research, Swiss Institute of Bioinformatics, http://www.ch.embnet.org/sofrware/TMPRED_form.html) for transmembrane region prediction; (iii) signalp_hmm or (iv) signalp_nn (both from Center for Biological Sequence Analysis, Technical University of Denmark DTU, http://www.cbs.dtu.dk/services/SignalP/background/prediction.php) for signal peptide prediction. The terms "signalp_hmm" and "signalp_nn" refer to two modes of operation for the program SignalP: hmm refers to Hidden Markov Model, while nn refers to neural networks. Localization was also determined through manual inspection of known protein localization and/or gene structure, and the use of heuristics by the individual inventor. In some cases for the manual inspection of cellular localization prediction inventors used the ProLoc computational platform [Einat Hazkani-Covo, Erez Levanon, Galit Rotman, Dan Graur and Amit Novik; (2004) "Evolution of multicellularity in metazoa: comparative analysis of the subcellular localization of proteins in Saccharomyces, Drosophila and Caenorhabditis." Cell Biology International 2004;28(3):171-8.], which predicts protein localization based on various parameters including, protein domains (e.g., prediction of trans-membranous regions and localization thereof within the protein), pl, protein length, amino acid composition, homology to pre-annotated proteins, recognition of sequence patterns which direct the protein to a certain organelle (such as, nuclear localization signal, NLS, mitochondria localization signal), signal peptide and anchor modeling and using unique domains from Pfam that are specific to a single compartment.

Information is given in the text with regard to SNPs (single nucleotide polymorphisms). A description of the abbreviations is as follows. "T - > C", for example, means that the SNP results in a change at the position given in the table from T to C. Similarly, "M - > Q", for example, means that the SNP has caused a change in the corresponding amino acid sequence, from methionine (M) to glutamine (Q). If, in place of a letter at the right hand side for the nucleotide sequence SNP, there is a space, it indicates that a frame shift has occurred. A frame shift may also be indicated with a hyphen (-). A stop codon is indicated with an asterisk at the right hand side (*). As part of the description of an SNP, a comment may be found in parentheses after the above description of the SNP itself. This comment may include an FTId, which is an identifier to a SwissProt entry that was created with the indicated SNP. An FTId is a unique and stable feature identifier, which allows construction of links directly from position-specific annotation in the feature table to specialized protein-related databases. The FTId is always the last component of a feature in the description field, as follows: FTId=XXX_number, in which XXX is the 3-letter code for the specific feature key, separated by an underscore from a 6-digit number. In the table of the amino acid mutations of the wild type proteins of the selected splice variants of the invention, the header of the first column is "SNP position(s) on amino acid sequence", representing a position of a known mutation on amino acid sequence. SNPs may optionally be used as diagnostic markers according to the present invention, alone or in combination with one or more other SNPs and/or any other diagnostic marker. Preferred embodiments of the present invention comprise such SNPs, including but not limited to novel SNPs on the known (WT or wild type) protein sequences given below, as well as novel nucleic acid and/or amino acid sequences formed through such SNPs, and/or any SNP on a variant amino acid and/or nucleic acid sequence described herein.

Information given in the text with regard to the Homology to the known proteins was determined by Smith-Waterman version 5.1.2 using special (non default) parameters as follows:

- model=sw.model
- GAPEXT=0
- GAPOP=100.0
    - MATRIX=blosum100

Information is given with regard to overexpression of a cluster in cancer based on ESTs. A key to the p values with regard to the analysis of such overexpression is as follows:

- Library-based statistics: P-value without including the level of expression in cell-lines (P1)
- Library based statistics: P-value including the level of expression in cell-lines (P2)
- EST clone statistics: P-value without including the level of expression in cell-lines (SP1)
- EST clone statistics: predicted overexpression ratio without including the level of expression in cell-lines (R3)
- EST clone statistics: P-value including the level of expression in cell-lines (SP2)
- EST clone statistics: predicted overexpression ratio including the level of expression in cell-lines (R4)

Library-based statistics refer to statistics over an entire library, while EST clone statistics refer to expression only for ESTs from a particular tissue or cancer.

Some embodiments of this invention have been exemplified herein wherein overexpression of a cluster in cancer was a determination based on microarray use. As a microarray reference, in the specific segment paragraphs, the unabbreviated tissue name was used as the reference to the type of chip for which expression was measured. There are two types of microarray results: those from microarrays prepared according to a design by the present inventors, for which the microarray fabrication procedure is described in detail in Materials and Experimental Procedures section herein; and those results from microarrays using Affymetrix technology. As a microarray reference, in the specific segment paragraphs, the unabbreviated tissue name was used as the reference to the type of chip for which expression was measured. For microarrays prepared according to a design by the present inventors, the probe name begins with the name of the cluster (gene), followed by an identifying number.

Oligonucleotide microarray results taken from Affymetrix data were from chips available from Affymetrix Inc, Santa Clara, CA, USA (see for example data regarding the Human Genome U133 (HG-U133) Set at www.affymetrix.com/products/arrays/specific/hgu133.affx; GeneChip Human Genome U133A 2.0 Array at www.affymetrix.com/products/arrays/specific/hgu133av2.affx; and Human Genome U133 Plus 2.0 Array at www.affymetrix.com/products/arrays/specific/hgu133plus.affx). The probe names follow the Affymetrix naming convention. The data is available from NCBI Gene Expression Omnibus (see www.ncbi.nlm.nih.gov/projects/geo/ and Edgar et al, Nucleic Acids Research, 2002, Vol. 30, No. 1 207-210). The dataset (including results) is available from www.ncbi.nlm.nih.gov/geo/query/acc.cgi?acc=GSE1133 for the Series GSE1133 database (published on March 2004); a reference to these results is as follows: Su et al (Proc Natl Acad Sci U S A. 2004 Apr 20;101(16):6062-7. Epub 2004 Apr 09).

Oligonucleotide microarray results taken from Affymetrix data were from chips available from Affymetrix Inc, Santa Clara, CA, USA (see for example data regarding the Human Genome U133 (HG-U133) Set at www.affymetrix.com/products/arrays/specific/hgu133.affx; GeneChip Human Genome U133A 2.0 Array at www.affymetrix.com/products/arrays/specific/hgu133av2.affx; and Human Genome U133 Plus 2.0 Array at www.affymetrix.com/products/arrays/specific/hgul33plus.affx). The probe names follow the Affymetrix naming convention. The data is available from NCBI Gene Expression Omnibus (see www.ncbi.nlm.nih.gov/projects/geo/ and Edgar et al, Nucleic Acids Research, 2002, Vol. 30, No. 1 207-210). The dataset (including results) is available from www.ncbi.nlm.nih.gov/geo/query/acc.cgi?acc=GSE1133 for the Series GSE1133 database (published on March 2004); a reference to these results is as follows: Su et al (Proc Natl Acad Sci U S A. 2004 Apr 20;101(16):6062-7. Epub 2004 Apr 09).

The following list of abbreviations for tissues was used in the TAA histograms. The term "TAA" stands for "Tumor Associated Antigen", and the TAA histograms, given in the text, represent the cancerous tissue expression pattern as predicted by the biomarkers selection engine, as described in detail in examples 1-5 below (the first word is the abbreviation while the second word is the full name):

("BONE", "bone");
("COL", "colon");
("EPI", "epithelial");
("GEN", "general");
("LIVER", "liver");
("LUN", "lung");
("LYMPH", "lymph nodes");
("MARROW", "bone marrow");
("OVA", "ovary");
("PANCREAS", "pancreas");
("PRO", "prostate");
("STOMACH", "stomach");
("TCELL", "T cells");
("THYROID", "Thyroid");
("MAM", "breast");
("BRAIN", "brain");
("UTERUS", "uterus");
("SKIN", "skin");
("KIDNEY", "kidney");
("MUSCLE", "muscle");
("ADREN", "adrenal");
("HEAD", "head and neck");
("BLADDER", "bladder");

It should be noted that the terms "segment", "seg" and "node" (abbreviated as "N" in the names of nodes) are used interchangeably in reference to nucleic acid sequences of the present invention, they refer to portions of nucleic acid sequences that were shown to have one or more properties as described herein. They are also the building blocks that were used to construct complete nucleic acid sequences as described in greater detail elsewhere herein. Optionally and preferably, they are examples of oligonucleotides which are embodiments of the present invention, for example as amplicons, hybridization units and/or from which primers and/or complementary oligonucleotides may optionally be derived, and/or for any other use.

In some embodiments, the phrase "disease" refers to its commonly understood meaning, and includes, *inter alia,* any type of pathology and/or damage, including both chronic and acute damage, as well as a progress from acute to chronic damage.

In some embodiments, the phrase "marker" in the context of the present invention refers to a nucleic acid fragment, a peptide, or a polypeptide, which is differentially present in a sample taken from patients (subjects) having one of the herein-described diseases or conditions, as compared to a comparable sample taken from subjects who do not have one the above-described diseases or conditions.

In some embodiments, the term "polypeptide" is to be understood to refer to a molecule comprising from at least 2 to several thousand or more amino acids. The term "polypeptide" is to be understood to include, *inter alia,* native peptides (either degradation products, synthetically synthesized peptides or recombinant peptides), peptidomimetics, such as peptoids and semipeptoids or peptide analogs, which may comprise, for example, any desirable modification, including, *inter alia,* modifications rendering the peptides more stable while in a body or more capable of penetrating into cells, or others as will be appreciated by one skilled in the art. Such modifications include, but are not limited to N terminus modification, C terminus modification, peptide bond modification, backbone modifications, residue modification, or others. Inclusion of such peptides within the polypeptides of this invention may produce a polypeptide sharing identity with the polypeptides described herein, for example, those provided in the sequence listing.

In some embodiments, the phrase "differentially present" refers to differences in the quantity or quality of a marker present in a sample taken from patients having one of the herein-described diseases or conditions as compared to a comparable sample taken from patients who do not have one of the herein-described diseases or conditions. For example, a nucleic acid fragment may optionally be differentially present between the two samples if the amount of the nucleic acid fragment in one sample is significantly different from the amount of the nucleic acid fragment in the other sample, for example as measured by hybridization and/or NAT-based assays. A polypeptide is differentially present between the two samples if the amount of the polypeptide in one sample is significantly different from the amount of the polypeptide in the other sample. It should be noted that if the marker is detectable in one sample and not detectable in the other, then such a marker can be considered to be differentially present. Optionally, a relatively low amount of up-regulation may serve as the marker, as described herein. One of ordinary skill in the art could easily determine such relative levels of the markers; further guidance is provided in the description of each individual marker below.

In some embodiments, the phrase "diagnostic" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

In some embodiments, the phrase "qualitative" when in reference to differences in expression levels of a polynucleotide, polypeptide or cluster as described herein, refers to the presence versus absence of expression, or in some embodiments, the temporal regulation of expression, or in some embodiments, the timing of expression, or in some embodiments, the variant expressed, or in some embodiments, any post-translational modifications to the expressed molecule, and others, as will be appreciated by one skilled in the art. In some embodiments, the phrase "quantitative" when in reference to differences in expression levels of a polynucleotide, polypeptide or cluster as described herein, refers to absolute differences in quantity of expression, as determined by any means, known in the art, or in other embodiments, relative differences, which may be statistically significant, or in some embodiments, when viewed as a whole or over a prolonged period of time, etc., indicate a trend in terms of differences in expression.

In some embodiments, the term "diagnosing" refers to classifying a disease or a symptom, determining a severity of the disease, monitoring disease progression, forecasting an outcome of a disease and/or prospects of recovery. The term "detecting" may also optionally encompass any of the above.

Diagnosis of a disease according to the present invention can, in some embodiments, be affected by determining a level of a polynucleotide or a polypeptide of the present invention in a biological sample obtained from the subject, wherein the level determined can be correlated with predisposition to, or presence or absence of the disease. It should be noted that a "biological sample obtained from the subject" may also optionally comprise a sample that has not been physically removed from the subject, as described in greater detail below.

In some embodiments, the term "level" refers to expression levels of RNA and/or protein or to DNA copy number of a marker of the present invention.

Typically the level of the marker in a biological sample obtained from the subject is different (i.e., increased or decreased) from the level of the same variant in a similar sample obtained from a healthy individual (examples of biological samples are described herein).

Numerous well known tissue or fluid collection methods can be utilized to collect the biological sample from the subject in order to determine the level of DNA, RNA and/or polypeptide of the variant of interest in the subject.

Examples include, but are not limited to, fine needle biopsy, needle biopsy, core needle biopsy and surgical biopsy (e.g., brain biopsy), and lavage. Regardless of the procedure employed, once a biopsy/sample is obtained the level of the variant can be determined and a diagnosis can thus be made.

Determining the level of the same variant in normal tissues of the same origin is preferably effected along-side to detect an elevated expression and/or amplification and/or a decreased expression, of the variant as opposed to the normal tissues.

In some embodiments, the term "test amount" of a marker refers to an amount of a marker in a subject's sample that is consistent with a diagnosis of a particular disease or condition. A test amount can be either in absolute amount (e.g., microgram/ml) or a relative amount (e.g., relative intensity of signals).

In some embodiments, the term "control amount" of a marker can be any amount or a range of amounts to be compared against a test amount of a marker. For example, a control amount of a marker can be the amount of a marker in a patient with a particular disease or condition or a person without such a disease or condition. A control amount can be either in absolute amount (e.g., microgram/ml) or a relative amount (e.g., relative intensity of signals).

In some embodiments, the term "detect" refers to identifying the presence, absence or amount of the object to be detected.

In some embodiments, the term "label" includes any moiety or item detectable by spectroscopic, photo chemical, biochemical, immunochemical, or chemical means. For example, useful labels include $^{32}P$, $^{35}S$, fluorescent dyes, electron-dense reagents, enzymes (e.g., as commonly used in an ELISA), biotin-streptavadin, dioxigenin, haptens and proteins for which antisera or monoclonal antibodies are available, or nucleic acid molecules with a sequence complementary to a target. The label often generates a measurable signal, such as a radioactive, chromogenic, or fluorescent signal, that can be used to quantify the amount of bound label in a sample. The label can be incorporated in or attached to a primer or probe either covalently, or through ionic, van der Waals or hydrogen bonds, e.g., incorporation of radioactive nucleotides, or biotinylated nucleotides that are recognized by streptavadin. The label may be directly or indirectly detectable. Indirect detection can involve the binding of a second label to the first label, directly or indirectly. For example, the label can be the ligand of a binding partner, such as biotin, which is a binding partner for streptavadin, or a nucleotide sequence, which is the binding partner for a complementary sequence, to which it can specifically hybridize. The binding partner may itself be directly detectable, for example, an antibody may be itself labeled with a fluorescent molecule. The binding partner also may be indirectly detectable, for example, a nucleic acid having a complementary nucleotide sequence can be a part of a branched DNA molecule that is in turn detectable through hybridization with other labeled nucleic acid molecules (see, e.g., P. D. Fahrlander and A. Klausner, Bio/Technology 6:1165 (1988)). Quantitation of the signal is achieved by, e.g., scintillation counting, densitometry, or flow cytometry.

Exemplary detectable labels, optionally and preferably for use with immunoassays, include but are not limited to magnetic beads, fluorescent dyes, radiolabels, enzymes (e.g., horse radish peroxide, alkaline phosphatase and others commonly used in an ELISA), and calorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

"Immunoassay" is an assay that uses an antibody to specifically bind an antigen. The immunoassay is characterized by the use of specific binding properties of a particular antibody to isolate, target, and/or quantify the antigen.

The phrase "specifically (or selectively) binds" to an antibody or "specifically (or selectively) immunoreactive with," or "specifically interacts or binds" when referring to a protein or peptide (or other epitope), refers, in some embodiments, to a binding reaction that is determinative of the presence of the protein in a heterogeneous population of proteins and other biologics. Thus, under designated immunoassay conditions, the specified antibodies bind to a particular protein at least two times greater than the background (non-specific signal) and do not substantially bind in a significant amount to other proteins present in the sample. Specific binding to an antibody under such conditions may require an antibody that is selected for its specificity for a particular protein. For example, polyclonal antibodies raised to seminal basic protein from specific species such as rat, mouse, or human can be selected to obtain only those polyclonal antibodies that are specifically immunoreactive with seminal basic protein and not with other proteins, except for polymorphic variants and alleles of seminal basic protein. This selection may be achieved by subtracting out antibodies that cross-react with seminal basic protein molecules from other species. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity). Typically a specific or selective reaction will be at least twice background signal or noise and more typically more than 10 to 100 times background.

In another embodiment, the present invention relates to bridges, tails, heads and/or insertions, and/or analogs, homologs and derivatives of such peptides. Such bridges, tails, heads and/or insertions are described in greater detail below with regard to the Examples.

In some embodiments, the term "tail" refers to a peptide sequence at the end of an amino acid sequence that is unique to a splice variant according to the present invention. Therefore, a splice variant having such a tail may optionally be considered as a chimera, in that at least a first portion of the splice variant is typically highly homologous (often 100% identical) to a portion of the corresponding known protein, while at least a second portion of the variant comprises the tail.

In some embodiments, the term "head" refers to a peptide sequence at the beginning of an amino acid sequence that is unique to a splice variant according to the present invention. Therefore, a splice variant having such a head may optionally be considered as a chimera, in that at least a first portion of the splice variant comprises the head, while at least a second portion is typically highly homologous (often 100% identical) to a portion of the corresponding known protein.

In some embodiments, the term "an edge portion" refers to a connection between two portions of a splice variant according to the present invention that were not joined in the wild type or known protein. An edge may optionally arise due to a join between the above "known protein" portion of a variant and the tail, for example, and/or may occur if an internal portion of the wild type sequence is no longer present, such that two portions of the sequence are now contiguous in the splice variant that were not contiguous in the known protein. A "bridge" may optionally be an edge portion as described above, but may also include a join between a head and a "known protein" portion of a variant, or a join between a tail and a "known protein" portion of a variant, or a join between an insertion and a "known protein" portion of a variant.

In some embodiments, a bridge between a tail or a head or a unique insertion, and a "known protein" portion of a variant, comprises at least about 10 amino acids, or in some embodiments at least about 20 amino acids, or in some embodiments at least about 30 amino acids, or in some embodiments at least about 40 amino acids, in which at least one amino acid is from the tail/head/insertion and at least one amino acid is from the "known protein" portion of a variant. In some embodiments, the bridge may comprise any number of amino acids from about 10 to about 40 amino acids (for example, 10, 11, 12, 13...37, 38, 39, 40 amino acids in length, or any number in between).

It should be noted that a bridge cannot be extended beyond the length of the sequence in either direction, and it should be assumed that every bridge description is to be read in such manner that the bridge length does not extend beyond the sequence itself.

Furthermore, bridges are described with regard to a sliding window in certain contexts below. For example, certain descriptions of the bridges feature the following format: a bridge between two edges (in which a portion of the known protein is not present in the variant) may optionally be described as follows: a bridge portion of CONTIG-NAME_P1 (representing the name of the protein), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise XX (2 amino acids in the center of the bridge, one from each end of the edge), having a structure as follows (numbering according to the sequence of CONTIG-NAME_P1): a sequence starting from any of amino acid numbers 49-x to 49 (for example); and ending at any of amino acid numbers 50 + ((n-2) - x) (for example), in which x varies from 0 to n-2. In this example, it should also be read as including bridges in which n is any number of amino acids between 10-50 amino acids in length. Furthermore, the bridge polypeptide cannot extend beyond the sequence, so it should be read such that 49-x (for example) is not less than 1, nor 50 + ((n-2) - x) (for example) greater than the total sequence length.

In another embodiment, this invention provides isolated nucleic acid molecules, which in some embodiments encode for splice variants, having a nucleotide sequence as set forth in any one of the sequences listed herein, being homologous to such sequences, at a percent as described herein, or a sequence complementary thereto. In another embodiment, this invention provides an oligonucleotide of at least about 12 nucleotides, which specifically hybridizes with the nucleic acid molecules of this invention. In another embodiment, this invention provides vectors, cells, liposomes and compositions comprising the isolated nucleic acids or polypeptides of this invention, as appropriate.

In another embodiment, this invention provides a method for detecting the polypeptides of this invention in a biological sample, comprising: contacting a biological sample with an antibody specifically recognizing a splice variant according to the present invention under conditions whereby the antibody specifically interacts with the splice variant in the biological sample but do not recognize known corresponding proteins (wherein the known protein is discussed with regard to its splice variant(s) in the Examples below), and detecting said interaction; wherein the presence of an interaction correlates with the presence of a splice variant in the biological sample.

In another embodiment, this invention provides a method for detecting a polynucleotide of this invention in a biological sample, comprising: hybridizing the isolated nucleic acid molecules or oligonucleotide fragments of at least about a minimum length to a nucleic acid material of a biological sample and detecting a hybridization complex; wherein the presence of a hybridization complex correlates with the presence of a the polynucleotide in the biological sample.

In some embodiments of the present invention, the polypeptides/polynucleotides described herein are non-limiting examples of markers for diagnosing marker-detectable disease and/or an indicative condition. Each polypeptide/polynucleotide marker of the present invention can be used alone or in combination, for various uses, including but not limited to, prognosis, prediction, screening, early diagnosis, determination of progression, therapy selection and treatment monitoring of marker-detectable disease and/or an indicative condition, including a transition from an indicative condition to marker-detectable disease.

According to some embodiments of the present invention, any marker according to the present invention may optionally be used alone or combination. Such a combination may optionally comprise a plurality of markers described herein, optionally including any subcombination of markers, and/or a combination featuring at least one other marker, for example a known marker. Furthermore, such a combination may optionally and preferably be used as described above with regard to determining a ratio between a quantitative or semi-quantitative measurement of any marker described herein to any other marker described herein, and/or any other known marker, and/or any other marker. With regard to such a ratio between any marker described herein (or a combination thereof) and a known marker, more preferably the known marker comprises the "known protein" as described in greater detail below with regard to each cluster or gene.

In some embodiments of the present invention, there are provided of methods, uses, devices and assays for the diagnosis of a disease or condition. Optionally a plurality of biomarkers (or markers) may be used with the present invention. The plurality of markers may optionally include a plurality of markers described herein, and/or one or more known markers. The plurality of markers is preferably then correlated with the disease or condition. For example, such correlating may optionally comprise determining the concentration of each of the plurality of markers, and individually comparing each marker concentration to a threshold level. Optionally, if the marker concentration is above or below the threshold level (depending upon the marker and/or the diagnostic test being performed), the marker concentration correlates with the disease or condition. Optionally and preferably, a plurality of marker concentrations correlates with the disease or condition.

Alternatively, such correlating may optionally comprise determining the concentration of each of the plurality of markers, calculating a single index value based on the concentration of each of the plurality of markers, and comparing the index value to a threshold level.

Also alternatively, such correlating may optionally comprise determining a temporal change in at least one of the markers, and wherein the temporal change is used in the correlating step.

Also alternatively, such correlating may optionally comprise determining whether at least "X" number of the plurality of markers has a concentration outside of a predetermined range and/or above or below a threshold (as described above). The value of "X" may optionally be one marker, a plurality of markers or all of the markers; alternatively or additionally, rather than including any marker in the count for "X", one or more specific markers of the plurality of markers may optionally be required to correlate with the disease or condition (according to a range and/or threshold).

Also alternatively, such correlating may optionally comprise determining whether a ratio of marker concentrations for two markers is outside a range and/or above or below a threshold. Optionally, if the ratio is above or below the threshold level and/or outside a range, the ratio correlates with the disease or condition.

Optionally, a combination of two or more these correlations may be used with a single panel and/or for correlating between a plurality of panels.

Optionally, the method distinguishes a disease or condition with a sensitivity of at least 70% at a specificity of at least 85% when compared to normal subjects. As used herein, sensitivity relates to the number of positive (diseased) samples detected out of the total number of positive samples present; specificity relates to the number of true negative (non-diseased) samples detected out of the total number of negative samples present. Preferably, the method distinguishes a disease or condition with a sensitivity of at least 80% at a specificity of at least 90% when compared to normal subjects. More preferably, the method distinguishes a disease or condition with a sensitivity of at least 90% at a specificity of at least 90% when compared to normal subjects. Also more preferably, the method distinguishes a disease or condition with a sensitivity of at least 70% at a specificity of at least 85% when compared to subjects exhibiting symptoms that mimic disease or condition symptoms.

A marker panel may be analyzed in a number of fashions well known to those of skill in the art. For example, each member of a panel may be compared to a "normal" value, or a value indicating a particular outcome. A particular diagnosis/prognosis may depend upon the comparison of each marker to this value; alternatively, if only a subset of markers is outside of a normal range, this subset may be indicative of a particular diagnosis/prognosis. The skilled artisan will also understand that diagnostic markers, differential diagnostic markers, prognostic markers, time of onset markers, disease or condition differentiating markers, etc., may be combined in a single assay or device. Markers may also be commonly used for multiple purposes by, for example, applying a different threshold or a different weighting factor to the marker for the different purpose(s).

In one embodiment, the panels comprise markers for the following purposes: diagnosis of a disease; diagnosis of disease and indication if the disease is in an acute phase and/or if an acute attack of the disease has occurred; diagnosis of disease and indication if the disease is in a non-acute phase and/or if a non-acute attack of the disease has occurred; indication whether a combination of acute and non-acute phases or attacks has occurred; diagnosis of a disease and prognosis of a subsequent adverse outcome; diagnosis of a disease and prognosis of a subsequent acute or non-acute phase or attack; disease progression (for example for cancer, such progression may include for example occurrence or recurrence of metastasis).

The above diagnoses may also optionally include differential diagnosis of the disease to distinguish it from other diseases, including those diseases that may feature one or more similar or identical symptoms.

In certain embodiments, one or more diagnostic or prognostic indicators are correlated to a condition or disease by merely the presence or absence of the indicator(s). In other embodiments, threshold level(s) of a diagnostic or prognostic indicator(s) can be established, and the level of the indicator(s) in a patient sample can simply be compared to the threshold level(s). The sensitivity and specificity of a diagnostic and/or prognostic test depends on more than just the analytical "quality" of the test--they also depend on the definition of what constitutes an abnormal result. In practice, Receiver Operating Characteristic curves, or "ROC" curves, are typically calculated by plotting the value of a variable versus its relative frequency in "normal" and "disease" populations, and/or by comparison of results from a subject before, during and/or after treatment. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition.

The horizontal axis of the ROC curve represents (1-specificity), which increases with the rate of false positives. The vertical axis of the curve represents sensitivity, which increases with the rate of true positives. Thus, for a particular cutoff selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

ROC curves can be used even when test results don't necessarily give an accurate number. As long as one can rank results, one can create an ROC curve. For example, results of a test on "disease" samples might be ranked according to degree (say 1=low, 2=normal, and 3=high). This ranking can be correlated to results in the "normal" population, and a ROC curve created. These methods are well known in the art (see for example Hanley et al., Radiology 143: 29-36 (1982), incorporated by reference as if fully set forth herein).

One or more markers may lack diagnostic or prognostic value when considered alone, but when used as part of a panel, such markers may be of great value in determining a particular diagnosis/prognosis. In some embodiments, particular thresholds for one or more markers in a panel are not relied upon to determine if a profile of marker levels obtained from a subject are indicative of a particular diagnosis/prognosis. Rather, the present invention may utilize an evaluation of the entire marker profile by plotting ROC curves for the sensitivity of a particular panel of markers versus 1-(specificity) for the panel at various cutoffs. In these methods, a profile of marker measurements from a subject is considered together to provide a global probability (expressed either as a numeric score or as a percentage risk) that an individual has had a disease, is at risk for developing such a disease, optionally the type of disease which the individual has had or is at risk for, and so forth etc. In such embodiments, an increase in a certain subset of markers may be sufficient to indicate a particular diagnosis/prognosis in one patient, while an increase in a different subset of markers may be sufficient to indicate the same or a different diagnosis/prognosis in another patient. Weighting factors may also be applied to one or more markers in a panel, for example, when a marker is of particularly high utility in identifying a particular diagnosis/prognosis, it may be weighted so that at a given level it alone is sufficient to signal a positive result. Likewise, a weighting factor may provide that no given level of a particular marker is sufficient to signal a positive result, but only signals a result when another marker also contributes to the analysis.

In some embodiments, markers and/or marker panels are selected to exhibit at least 70% sensitivity, more preferably at least 80% sensitivity, even more preferably at least 85% sensitivity, still more preferably at least 90% sensitivity, and most preferably at least 95% sensitivity, combined with at least 70% specificity, more preferably at least 80% specificity, even more preferably at least 85% specificity, still more preferably at least 90% specificity, and most preferably at least 95% specificity. In some embodiments, both the sensitivity and specificity are at least 75%, more preferably at least 80%, even more preferably at least 85%, still more preferably at least 90%, and most preferably at least 95%. Sensitivity and/or specificity may optionally be determined as described above, with regard to the construction of ROC graphs and so forth, for example.

According to some embodiments of the present invention, individual markers and/or combinations (panels) of markers may optionally be used for diagnosis of time of onset of a disease or condition. Such diagnosis may optionally be useful for a wide variety of conditions, preferably including those conditions with an abrupt onset.

The phrase "determining the prognosis" as used herein refers to methods by which the skilled artisan can predict the course or outcome of a condition in a patient. The term "prognosis" does not refer to the ability to predict the course or outcome of a condition with 100% accuracy, or even that a given course or outcome is more likely to occur than not. Instead, the skilled artisan will understand that the term "prognosis" refers to an increased probability that a certain course or outcome will occur; that is, that a course or outcome is more likely to occur in a patient exhibiting a given condition, when compared to those individuals not exhibiting the condition. For example, in individuals not exhibiting the condition, the chance of a given outcome may be about 3%. In some embodiments, a prognosis is about a 5% chance of a given outcome, about a 7% chance, about a 10% chance, about a 12% chance, about a 15% chance, about a 20% chance, about a 25% chance, about a 30% chance, about a 40% chance, about a 50% chance, about a 60% chance, about a 75% chance, about a 90% chance, and about a 95% chance. The term "about" in this context refers to +/-1%.

The skilled artisan will understand that associating a prognostic indicator with a predisposition to an adverse outcome is a statistical analysis. For example, a marker level of greater than 80 pg/mL may signal that a patient is more likely to suffer from an adverse outcome than patients with a level less than or equal to 80 pg/mL, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, e.g., Dowdy and Wearden, Statistics for Research, John Wiley & Sons, New York, 1983. In one embodiment the confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001. Exemplary statistical tests for associating a prognostic indicator with a predisposition to an adverse outcome are described hereinafter.

In other embodiments, a threshold degree of change in the level of a prognostic or diagnostic indicator can be established, and the degree of change in the level of the indicator in a patient sample can simply be compared to the threshold degree of change in the level. A preferred threshold change in the level for markers of the invention is about 5%, about 10%, about 15%, about 20%, about 25%, about 30%, about 50%, about 75%, about 100%, and about 150%. The term "about" in this context refers to +/-10%. In yet other embodiments, a "nomogram" can be established, by which a level of a prognostic or diagnostic indicator can be directly related to an associated disposition towards a given outcome. The skilled artisan is acquainted with the use of such nomograms to relate two numeric values with the understanding that the uncertainty in this measurement is the same as the uncertainty in the marker concentration because individual sample measurements are referenced, not population averages.

Exemplary, non-limiting methods and systems for identification of suitable biomarkers for marker panels are now described. Methods and systems for the identification of one or more markers for the diagnosis, and in particular for the differential diagnosis, of disease have been described previously. Suitable methods for identifying markers useful for the diagnosis of disease states are described in detail in U.S. patent application no. 2004-0126767, entitled METHOD AND SYSTEM FOR DISEASE DETECTION USING MARKER COMBINATIONS, filed Dec. 27, 2002, hereby incorporated by reference in its entirety as if fully set forth herein. One skilled in the art will also recognize that univariate analysis of markers can be performed and the data from the univariate analyses of multiple markers can be combined to form panels of markers to differentiate different disease conditions.

In developing a panel of markers useful in diagnosis, data for a number of potential markers may be obtained from a group of subjects by testing for the presence or level of certain markers. The group of subjects is divided into two sets, and preferably the first set and the second set each have an approximately equal number of subjects. The first set includes subjects who have been confirmed as having a disease or, more generally, being in a first condition state. For example, this first set of patients may be those that have recently had a disease and/or a particular type of the disease. The confirmation of this condition state may be made through more rigorous and/or expensive testing, preferably according to a previously defined diagnostic standard. Hereinafter, subjects in this first set will be referred to as "diseased".

The second set of subjects is simply those who do not fall within the first set. Subjects in this second set may be "non-diseased;" that is, normal subjects. Alternatively, subjects in this second set may be selected to exhibit one symptom or a constellation of symptoms that mimic those symptoms exhibited by the "diseased" subjects.

The data obtained from subjects in these sets includes levels of a plurality of markers. Preferably, data for the same set of markers is available for each patient. This set of markers may include all candidate markers which may be suspected as being relevant to the detection of a particular disease or condition. Actual known relevance is not required. Embodiments of the methods and systems described herein may be used to determine which of the candidate markers are most relevant to the diagnosis of the disease or condition. The levels of each marker in the two sets of subjects may be distributed across a broad range, e.g., as a Gaussian distribution. However, no distribution fit is required.

As noted above, a marker often is incapable of definitively identifying a patient as either diseased or non-diseased. For example, if a patient is measured as having a marker level that falls within the overlapping region, the results of the test will be useless in diagnosing the patient. An artificial cutoff may be used to distinguish between a positive and a negative test result for the detection of the disease or condition. Regardless of where the cutoff is selected, the effectiveness of the single marker as a diagnosis tool is unaffected. Changing the cutoff merely trades off between the number of false positives and the number of false negatives resulting from the use of the single marker. The effectiveness of a test having such an overlap is often expressed using a ROC (Receiver Operating Characteristic) curve as described above.

As discussed above, the measurement of the level of a single marker may have limited usefulness. The measurement of additional markers provides additional information, but the difficulty lies in properly combining the levels of two potentially unrelated measurements. In the methods and systems according to embodiments of the present invention, data relating to levels of various markers for the sets of diseased and non-diseased patients may be used to develop a panel of markers to provide a useful panel response. The data may be provided in a database such as Microsoft Access, Oracle, other SQL databases or simply in a data file. The database or data file may contain, for example, a patient identifier such as a name or number, the levels of the various markers present, and whether the patient is diseased or non-diseased.

Next, an artificial cutoff region may be initially selected for each marker. The location of the cutoff region may initially be selected at any point, but the selection may affect the optimization process described below. In this regard, selection near a suspected optimal location may facilitate faster convergence of the optimizer. In an embodiment method, the cutoff region is initially centered about the center of the overlap region of the two sets of patients. In one embodiment, the cutoff region may simply be a cutoff point. In other embodiments, the cutoff region may have a length of greater than zero. In this regard, the cutoff region may be defined by a center value and a magnitude of length. In practice, the initial selection of the limits of the cutoff region may be determined according to a pre-selected percentile of each set of subjects. For example, a point above which a pre-selected percentile of diseased patients is measured may be used as the right (upper) end of the cutoff range.

Each marker value for each patient may then be mapped to an indicator. The indicator is assigned one value below the cutoff region and another value above the cutoff region. For example, if a marker generally has a lower value for non-diseased patients and a higher value for diseased patients, a zero indicator will be assigned to a low value for a particular marker, indicating a potentially low likelihood of a positive diagnosis. In other embodiments, the indicator may be calculated based on a polynomial. The coefficients of the polynomial may be determined based on the distributions of the marker values among the diseased and non-diseased subjects.

The relative importance of the various markers may be indicated by a weighting factor. The weighting factor may initially be assigned as a coefficient for each marker. As with the cutoff region, the initial selection of the weighting factor may be selected at any acceptable value, but the selection may affect the optimization process. In this regard, selection near a suspected optimal location may facilitate faster convergence of the optimizer. In an embodiment method, acceptable weighting coefficients may range between zero and one, and an initial weighting coefficient for each marker may be assigned as 0.5. In one embodiment, the initial weighting coefficient for each marker may be associated with the effectiveness of that marker by itself. For example, a ROC curve may be generated for the single marker, and the area under the ROC curve may be used as the initial weighting coefficient for that marker.

Next, a panel response may be calculated for each subject in each of the two sets. The panel response is a function of the indicators to which each marker level is mapped and the weighting coefficients for each marker. One advantage of using an indicator value rather than the marker value is that an extraordinarily high or low marker levels do not change the probability of a diagnosis of diseased or non-diseased for that particular marker. Typically, a marker value above a certain level generally indicates a certain condition state. Marker values above that level indicate the condition state with the same certainty. Thus, an extraordinarily high marker value may not indicate an extraordinarily high probability of that condition state. The use of an indicator which is constant on one side of the cutoff region eliminates this concern.

The panel response may also be a general function of several parameters including the marker levels and other factors including, for example, race and gender of the patient. Other factors contributing to the panel response may include the slope of the value of a particular marker over time. For example, a patient may be measured when first arriving at the hospital for a particular marker. The same marker may be measured again an hour later, and the level of change may be reflected in the panel response. Further, additional markers may be derived from other markers and may contribute to the value of the panel response. For example, the ratio of values of two markers may be a factor in calculating the panel response.

Having obtained panel responses for each subject in each set of subjects, the distribution of the panel responses for each set may now be analyzed. An objective function may be defined to facilitate the selection of an effective panel. The objective function should generally be indicative of the effectiveness of the panel, as may be expressed by, for example, overlap of the panel responses of the diseased set of subjects and the panel responses of the non-diseased set of subjects. In this manner, the objective function may be optimized to maximize the effectiveness of the panel by, for example, minimizing the overlap.

In some embodiments, the ROC curve representing the panel responses of the two sets of subjects may be used to define the objective function. For example, the objective function may reflect the area under the ROC curve. By maximizing the area under the curve, one may maximize the effectiveness of the panel of markers. In other embodiments, other features of the ROC curve may be used to define the objective function. For example, the point at which the slope of the ROC curve is equal to one may be a useful feature. In other embodiments, the point at which the product of sensitivity and specificity is a maximum, sometimes referred to as the "knee," may be used. In an embodiment, the sensitivity at the knee may be maximized. In further embodiments, the sensitivity at a predetermined specificity level may be used to define the objective function. Other embodiments may use the specificity at a predetermined sensitivity level may be used. In still other embodiments, combinations of two or more of these ROC-curve features may be used.

It is possible that one of the markers in the panel is specific to the disease or condition being diagnosed. When such markers are present at above or below a certain threshold, the panel response may be set to return a "positive" test result. When the threshold is not satisfied, however, the levels of the marker may nevertheless be used as possible contributors to the objective function.

An optimization algorithm may be used to maximize or minimize the objective function. Optimization algorithms are well-known to those skilled in the art and include several commonly available minimizing or maximizing functions including the Simplex method and other constrained optimization techniques. It is understood by those skilled in the art that some minimization functions are better than others at searching for global minimums, rather than local minimums. In the optimization process, the location and size of the cutoff region for each marker may be allowed to vary to provide at least two degrees of freedom per marker. Such variable parameters are referred to herein as independent variables. In one embodiment, the weighting coefficient for each marker is also allowed to vary across iterations of the optimization algorithm. In various embodiments, any permutation of these parameters may be used as independent variables.

In addition to the above-described parameters, the sense of each marker may also be used as an independent variable. For example, in many cases, it may not be known whether a higher level for a certain marker is generally indicative of a diseased state or a non-diseased state. In such a case, it may be useful to allow the optimization process to search on both sides. In practice, this may be implemented in several ways. For example, in one embodiment, the sense may be a truly separate independent variable which may be flipped between positive and negative by the optimization process. Alternatively, the sense may be implemented by allowing the weighting coefficient to be negative.

The optimization algorithm may be provided with certain constraints as well. For example, the resulting ROC curve may be constrained to provide an area-under-curve of greater than a particular value. ROC curves having an area under the curve of 0.5 indicate complete randomness, while an area under the curve of 1.0 reflects perfect separation of the two sets. Thus, a minimum acceptable value, such as 0.75, may be used as a constraint, particularly if the objective function does not incorporate the area under the curve. Other constraints may include limitations on the weighting coefficients of particular markers. Additional constraints may limit the sum of all the weighting coefficients to a particular value, such as 1.0.

The iterations of the optimization algorithm generally vary the independent parameters to satisfy the constraints while minimizing or maximizing the objective function. The number of iterations may be limited in the optimization process. Further, the optimization process may be terminated when the difference in the objective function between two consecutive iterations is below a predetermined threshold, thereby indicating that the optimization algorithm has reached a region of a local minimum or a maximum.

Thus, the optimization process may provide a panel of markers including weighting coefficients for each marker and cutoff regions for the mapping of marker values to indicators. In order to develop lower-cost panels which require the measurement of fewer marker levels, certain markers may be eliminated from the panel. In this regard, the effective contribution of each marker in the panel may be determined to identify the relative importance of the markers. In one embodiment, the weighting coefficients resulting from the optimization process may be used to determine the relative importance of each marker. The markers with the lowest coefficients may be eliminated.

Individual panel response values may also be used as markers in the methods described herein. For example, a panel may be constructed from a plurality of markers, and each marker of the panel may be described by a function and a weighting factor to be applied to that marker (as determined by the methods described above). Each individual marker level is determined for a sample to be tested, and that level is applied to the predetermined function and weighting factor for that particular marker to arrive at a sample value for that marker. The sample values for each marker are added together to arrive at the panel response for that particular sample to be tested. For a "diseased" and "non-diseased" group of patients, the resulting panel responses may be treated as if they were just levels of another disease marker.

Measures of test accuracy may be obtained as described in Fischer et al., Intensive Care Med. 29: 1043-51, 2003 (hereby incorporated by reference as if fully set forth herein), and used to determine the effectiveness of a given marker or panel of markers. These measures include sensitivity and specificity, predictive values, likelihood ratios, diagnostic odds ratios, and ROC curve areas. As discussed above, suitable tests may exhibit one or more of the following results on these various measures: at least 75% sensitivity, combined with at least 75% specificity; ROC curve area of at least 0.7, more preferably at least 0.8, even more preferably at least 0.9, and most preferably at least 0.95; and/or a positive likelihood ratio (calculated as sensitivity/(1-specificity)) of at least 5, more preferably at least 10, and most preferably at least 20, and a negative likelihood ratio (calculated as (1-sensitivity)/specificity) of less than or equal to 0.3, more preferably less than or equal to 0.2, and most preferably less than or equal to 0.1.

According to embodiments of the present invention, a splice variant protein or a fragment thereof, or a splice variant nucleic acid sequence or a fragment thereof, may be featured as a biomarker for detecting marker-detectable disease and/or an indicative condition, such that a biomarker may optionally comprise any of the above.

According to still other embodiments, the present invention optionally and preferably encompasses any amino acid sequence or fragment thereof encoded by a nucleic acid sequence corresponding to a splice variant protein as described herein. Any oligopeptide or peptide relating to such an amino acid sequence or fragment thereof may optionally also (additionally or alternatively) be used as a biomarker, including but not limited to the unique amino acid sequences of these proteins that are depicted as tails, heads, insertions, edges or bridges. The present invention also optionally encompasses antibodies capable of recognizing, and/or being elicited by, such oligopeptides or peptides.

The present invention also optionally and preferably encompasses any nucleic acid sequence or fragment thereof, or amino acid sequence or fragment thereof, corresponding to a splice variant of the present invention as described above, optionally for any application.

Non-limiting examples of methods or assays are described below.

The present invention also relates to kits based upon such diagnostic methods or assays.

<u>Nucleic acid sequences and Oligonucleotides</u>

Various embodiments of the present invention encompass nucleic acid sequences described hereinabove; fragments thereof, sequences hybridizable therewith, sequences homologous thereto, sequences encoding similar polypeptides with different codon usage, altered sequences characterized by mutations, such as deletion, insertion or substitution of one or more nucleotides, either naturally occurring or artificially induced, either randomly or in a targeted fashion.

The present invention encompasses nucleic acid sequences described herein; fragments thereof, sequences hybridizable therewith, sequences homologous thereto [e.g., at least 50 %, at least 55 %, at least 60%, at least 65 %, at least 70 %, at least 75 %, at least 80 %, at least 85 %, at least 95 % or more say 100 % identical to the nucleic acid sequences set forth below], sequences encoding similar polypeptides with different codon usage, altered sequences characterized by mutations, such as deletion, insertion or substitution of one or more nucleotides, either naturally occurring or man induced, either randomly or in a targeted fashion. The present invention also encompasses homologous nucleic acid sequences (i.e., which form a part of a polynucleotide sequence of the present invention) which include sequence regions unique to the polynucleotides of the present invention.

In cases where the polynucleotide sequences of the present invention encode previously unidentified polypeptides, the present invention also encompasses novel polypeptides or portions thereof, which are encoded by the isolated polynucleotide and respective nucleic acid fragments thereof described hereinabove.

A "nucleic acid fragment" or an "oligonucleotide" or a "polynucleotide" are used herein interchangeably to refer to a polymer of nucleic acids. A polynucleotide sequence of the present invention refers to a single or double stranded nucleic acid sequences which is isolated and provided in the form of an RNA sequence, a complementary polynucleotide sequence (cDNA), a genomic polynucleotide sequence and/or a composite polynucleotide sequences (e.g., a combination of the above).

As used herein the phrase "complementary polynucleotide sequence" refers to a sequence, which results from reverse transcription of messenger RNA using a reverse transcriptase or any other RNA dependent DNA polymerase. Such a sequence can be subsequently amplified *in vivo* or *in vitro* using a DNA dependent DNA polymerase.

As used herein the phrase "genomic polynucleotide sequence" refers to a sequence derived (isolated) from a chromosome and thus it represents a contiguous portion of a chromosome.

As used herein the phrase "composite polynucleotide sequence" refers to a sequence, which is composed of genomic and cDNA sequences. A composite sequence can include some exonal sequences required to encode the polypeptide of the present invention, as well as some intronic sequences interposing therebetween. The intronic sequences can be of any source, including of other genes, and typically will include conserved splicing signal sequences. Such intronic sequences may further include cis acting expression regulatory elements.

Preferred embodiments of the present invention encompass oligonucleotide probes.

An example of an oligonucleotide probe which can be utilized by the present invention is a single stranded polynucleotide which includes a sequence complementary to the unique sequence region of any variant according to the present invention, including but not limited to a nucleotide sequence coding for an amino sequence of a bridge, tail, head and/or insertion according to the present invention, and/or the equivalent portions of any nucleotide sequence given herein (including but not limited to a nucleotide sequence of a node, segment or amplicon described herein).

Alternatively, an oligonucleotide probe of the present invention can be designed to hybridize with a nucleic acid sequence encompassed by any of the above nucleic acid sequences, particularly the portions specified above, including but not limited to a nucleotide sequence coding for an amino sequence of a bridge, tail, head and/or insertion according to the present invention, and/or the equivalent portions of any nucleotide sequence given herein (including but not limited to a nucleotide sequence of a node, segment or amplicon described herein).

Oligonucleotides designed according to the teachings of the present invention can be generated according to any oligonucleotide synthesis method known in the art such as enzymatic synthesis or solid phase synthesis. Equipment and reagents for executing solid-phase synthesis are commercially available from, for example, Applied Biosystems. Any other means for such synthesis may also be employed; the actual synthesis of the oligonucleotides is well within the capabilities of one skilled in the art and can be accomplished via established methodologies as detailed in, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988) and "Oligonucleotide Synthesis" Gait, M. J., ed. (1984) utilizing solid phase chemistry, e.g. cyanoethyl phosphoramidite followed by deprotection, desalting and purification by for example, an automated trityl-on method or HPLC.

Oligonucleotides used according to this aspect of the present invention are those having a length selected from a range of about 10 to about 200 bases preferably about 15 to about 150 bases, more preferably about 20 to about 100 bases, most preferably about 20 to about 50 bases. Preferably, the oligonucleotide of the present invention features at least 17, at least 18, at least 19, at least 20, at least 22, at least 25, at least 30 or at least 40, bases specifically hybridizable with the biomarkers of the present invention.

The oligonucleotides of the present invention may comprise heterocylic nucleosides consisting of purines and the pyrimidines bases, bonded in a 3' to 5' phosphodiester linkage.

Preferably used oligonucleotides are those modified at one or more of the backbone, internucleoside linkages or bases, as is broadly described hereinunder.

Specific examples of preferred oligonucleotides useful according to this aspect of the present invention include oligonucleotides containing modified backbones or non-natural internucleoside linkages. Oligonucleotides having modified backbones include those that retain a phosphorus atom in the backbone, as disclosed in U.S. Pat. NOs: 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466, 677; 5,476,925; 5,519,126; 5,536,821; 5,541,306; 5,550,111; 5,563,253; 5,571,799; 5,587,361; and 5,625,050.

Preferred modified oligonucleotide backbones include, for example, phosphorothioates, chiral phosphorothioates, phosphorodithioates, phosphotriesters, aminoalkyl phosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms can also be used.

Alternatively, modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts, as disclosed in U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623, 070; 5,663,312; 5,633,360; 5,677,437; and 5,677,439.

Other oligonucleotides which can be used according to the present invention, are those modified in both sugar and the internucleoside linkage, i.e., the backbone, of the nucleotide units are replaced with novel groups. The base units are maintained for complementation with the appropriate polynucleotide target. An example for such an oligonucleotide mimetic includes peptide nucleic acid (PNA). United States patents that teach the preparation of PNA compounds include, but are not limited to, U.S. Pat. Nos. 5,539,082; 5,714,331; and 5,719,262, each of which is herein incorporated by reference. Other backbone modifications, which can be used in the present invention, are disclosed in U.S. Pat. No: 6,303,374.

Oligonucleotides of the present invention may also include base modifications or substitutions. As used herein, "unmodified" or "natural" bases include the purine bases adenine (A) and guanine (G), and the pyrimidine bases thymine (T), cytosine (C) and uracil (U). Modified bases include but are not limited to other synthetic and natural bases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further bases particularly useful for increasing the binding affinity of the oligomeric compounds of the invention include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2 °C and are presently preferred base substitutions, even more particularly when combined with 2'-O-methoxyethyl sugar modifications.

Another modification of the oligonucleotides of the invention involves chemically linking to the oligonucleotide one or more moieties or conjugates, which enhance the activity, cellular distribution or cellular uptake of the oligonucleotide. Such moieties include but are not limited to lipid moieties such as a cholesterol moiety, cholic acid, a thioether, e.g., hexyl-S-tritylthiol, a thiocholesterol, an aliphatic chain, e.g., dodecandiol or undecyl residues, a phospholipid, e.g., di-hexadecyl-rac-glycerol or triethylammonium 1,2-di-0-hexadecyl-rac-glycero-3-H-phosphonate, a polyamine or a polyethylene glycol chain, or adamantane acetic acid, a palmityl moiety, or an octadecylamine or hexylamino-carbonyl-oxycholesterol moiety, as disclosed in U.S. Pat. No: 6,303,374.

It is not necessary for all positions in a given oligonucleotide molecule to be uniformly modified, and in fact more than one of the aforementioned modifications may be incorporated in a single compound or even at a single nucleoside within an oligonucleotide.

It will be appreciated that oligonucleotides of the present invention may include further modifications for more efficient use as diagnostic agents and/or to increase bioavailability, therapeutic efficacy and reduce cytotoxicity.

To enable cellular expression of the polynucleotides of the present invention, a nucleic acid construct according to the present invention may be used, which includes at least a coding region of one of the above nucleic acid sequences, and further includes at least one cis acting regulatory element. As used herein, the phrase "cis acting regulatory element" refers to a polynucleotide sequence, preferably a promoter, which binds a trans acting regulator and regulates the transcription of a coding sequence located downstream thereto.

Any suitable promoter sequence can be used by the nucleic acid construct of the present invention.

Preferably, the promoter utilized by the nucleic acid construct of the present invention is active in the specific cell population transformed. Examples of cell type-specific and/or tissue-specific promoters include promoters such as albumin that is liver specific, lymphoid specific promoters [Calame et al., (1988) Adv. Immunol. 43:235-275]; in particular promoters of T-cell receptors [Winoto et al., (1989) EMBO J. 8:729-733] and immunoglobulins; [Banerji et al. (1983) Cell 33729-740], neuron-specific promoters such as the neurofilament promoter [Byrne et al. (1989) Proc. Natl. Acad. Sci. USA 86:5473-5477], pancreas-specific promoters [Edlunch et al. (1985) Science 230:912-916] or mammary gland-specific promoters such as the milk whey promoter (U.S. Pat. No. 4,873,316 and European Application Publication No. 264,166). The nucleic acid construct of the present invention can further include an enhancer, which can be adjacent or distant to the promoter sequence and can function in up regulating the transcription therefrom.

The nucleic acid construct of the present invention preferably further includes an appropriate selectable marker and/or an origin of replication. Preferably, the nucleic acid construct utilized is a shuttle vector, which can propagate both in *E. coli* (wherein the construct comprises an appropriate selectable marker and origin of replication) and be compatible for propagation in cells, or integration in a gene and a tissue of choice. The construct according to the present invention can be, for example, a plasmid, a bacmid, a phagemid, a cosmid, a phage, a virus or an artificial chromosome.

Examples of suitable constructs include, but are not limited to, pcDNA3, pcDNA3.1 (+/-), pGL3, PzeoSV2 (+/-), pDisplay, pEF/myc/cyto, pCMV/myc/cyto each of which is commercially available from Invitrogen Co. (www.invitrogen.com). Examples of retroviral vector and packaging systems are those sold by Clontech, San Diego, Calif., includingRetro-X vectors pLNCX and pLXSN, which permit cloning into multiple cloning sites and the transgene is transcribed from CMV promoter. Vectors derived from Mo-MuLV are also included such as pBabe, where the transgene will be transcribed from the 5'LTR promoter.

Currently preferred in vivo nucleic acid transfer techniques include transfection with viral or non-viral constructs, such as adenovirus, lentivirus, Herpes simplex I virus, or adeno-associated virus (AAV) and lipid-based systems. Useful lipids for lipid-mediated transfer of the gene are, for example, DOTMA, DOPE, and DC-Chol [Tonkinson et al., Cancer Investigation, 14(1): 54-65 (1996)]. The most preferred constructs for use in gene therapy are viruses, most preferably adenoviruses, AAV, lentiviruses, or retroviruses. A viral construct such as a retroviral construct includes at least one transcriptional promoter/enhancer or locus-defining element(s), or other elements that control gene expression by other means such as alternate splicing, nuclear RNA export, or post-translational modification of messenger. Such vector constructs also include a packaging signal, long terminal repeats (LTRs) or portions thereof, and positive and negative strand primer binding sites appropriate to the virus used, unless it is already present in the viral construct. In addition, such a construct typically includes a signal sequence for secretion of the peptide from a host cell in which it is placed. Preferably the signal sequence for this purpose is a mammalian signal sequence or the signal sequence of the polypeptide variants of the present invention. Optionally, the construct may also include a signal that directs polyadenylation, as well as one or more restriction sites and a translation termination sequence. By way of example, such constructs will typically include a 5'LTR, a tRNA binding site, a packaging signal, an origin of second-strand DNA synthesis, and a 3' LTR or a portion thereof. Other vectors can be used that are non-viral, such as cationic lipids, polylysine, and dendrimers.

Variant Recombinant Expression Vectors and Host Cells

Another aspect of the invention pertains to vectors, preferably expression vectors, containing a nucleic acid encoding a variant protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (e.g., in an in vitro transcription/translation system or in a host cell when the vector is introduced into the host cell).

The term "regulatory sequence" is intended to include promoters, enhancers and other expression control elements (e.g., polyadenylation signals). Such regulatory sequences are described, for example, in Goeddel, Gene Expression Technology: Methods in Enzymology 185, Academic Press, San Diego, Calif. (1990). Regulatory sequences include those that direct constitutive expression of a nucleotide sequence in many types of host cell and those that direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (e.g., variant proteins, mutant forms of variant proteins, fusion proteins, etc.).

The recombinant expression vectors of the invention can be designed for production of variant proteins in prokaryotic or eukaryotic cells. For example, variant proteins can be expressed in bacterial cells such as Escherichia coli, insect cells (using baculovirus expression vectors) yeast cells or mammalian cells. Suitable host cells are discussed further in Goeddel, Gene Expression Technology: Methods in Enzymology 185, Academic Press, San Diego, Calif. (1990). Alternatively, the recombinant expression vector can be transcribed and translated in vitro, for example using T7 promoter regulatory sequences and T7 polymerase.

Expression of proteins in prokaryotes is most often carried out in Escherichia coli with vectors containing constitutive or inducible promoters directing the expression of either fusion or non-fusion proteins. Fusion vectors add a number of amino acids to a protein encoded therein, to the amino or carboxyl terminus of the recombinant protein. Such fusion vectors typically serve three purposes: (i) to increase expression of recombinant protein; (ii) to increase the solubility of the recombinant protein; and (iii) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin, PreScission, TEV and enterokinase. Typical fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) and pTrcHis (Invitrogen Life Technologies) that fuse glutathione S-transferase (GST), maltose E binding protein, protein A or 6xHis, respectively, to the target recombinant protein.

Examples of suitable inducible non-fusion E. coli expression vectors include pTrc (Amrann et al., (1988) Gene 69:301-315).

One strategy to maximize recombinant protein expression in E. coli is to express the protein in host bacteria with an impaired capacity to proteolytically cleave the recombinant protein. See, e.g., Gottesman, Gene Expression Technology: Methods in Enzymology 185, Academic Press, San Diego, Calif. (1990) 119-128. Another strategy is to alter the nucleic acid sequence of the nucleic acid to be inserted into an expression vector so that the individual codons for each amino acid are those preferentially utilized in E. coli (see, e.g., Wada, et al., 1992. Nucl. Acids Res. 20: 2111-2118). Such alteration of nucleic acid sequences of the invention can be carried out by standard DNA synthesis techniques. Another optional strategy to solve codon bias is by using BL21-codon plus bacterial strains (Invitrogen) or Rosetta bacterial strain (Novagen), as these strains contain extra copies of rare E. coli tRNA genes.

In another embodiment, the expression vector encoding for the variant protein is a yeast expression vector. Examples of vectors for expression in yeast Saccharomyces cerivisae include pYepSec1 (Baldari, et al., 1987. EMBO J. 6: 229-234), pMFa (Kurjan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

Alternatively, variant protein can be produced in insect cells using baculovirus expression vectors. Baculovirus vectors available for expression of proteins in cultured insect cells (e.g., SF9 cells) include the pAc series (Smith, et al., 1983. Mol. Cell. Biol. 3: 2156-2165) and the pVL series (Lucklow and Summers, 1989. Virology 170: 31-39).

In yet another embodiment, a nucleic acid of the invention is expressed in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195), pIRESpuro (Clontech), pUB6 (Invitrogen), pCEP4 (Invitrogen) pREP4 (Invitrogen), pcDNA3 (Invitrogen). When used in mammalian cells, the expression vector's control functions are often provided by viral regulatory elements. For example, commonly used promoters are derived from polyoma, adenovirus 2, cytomegalovirus, Rous Sarcoma Virus, and simian virus 40. For other suitable expression systems for both prokaryotic and eukaryotic cells see, e.g., Chapters 16 and 17 of Sambrook, et al., Molecular Cloning: A Laboratory Manual. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Banerji, et al., 1983. Cell 33: 729-740; Queen and Baltimore, 1983. Cell 33: 741-748), neuron-specific promoters (e.g., the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (e.g., milk whey promoter; U.S. Pat. No. 4,873,316 and European Application Publication No. 264,166). Developmentally-regulated promoters are also encompassed, e.g., the murine hox promoters (Kessel and Gruss, 1990. Science 249: 374-379) and the alpha-fetoprotein promoter (Campes and Tilghman, 1989. Genes Dev. 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to mRNA encoding for variant protein. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see, e.g., Weintraub, et al., "Antisense RNA as a molecular tool for genetic analysis," Reviews-Trends in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, variant protein can be produced in bacterial cells such as E. coli, insect cells, yeast or mammalian cells (such as Chinese hamster ovary cells (CHO) or COS or 293 cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (Molecular Cloning: A Laboratory Manual. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Various selectable markers include those that confer resistance to drugs, such as G418, hygromycin, puromycin, blasticidin and methotrexate. Nucleic acids encoding a selectable marker can be introduced into a host cell on the same vector as that encoding variant protein or can be introduced on a separate vector. Cells stably transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce (i.e., express) variant protein. Accordingly, the invention further provides methods for producing variant protein using the host cells of the invention. In one embodiment, the method comprises culturing the host cell of the present invention (into which a recombinant expression vector encoding variant protein has been introduced) in a suitable medium such that variant protein is produced. In another embodiment, the method further comprises isolating variant protein from the medium or the host cell.

For efficient production of the protein, it is preferable to place the nucleotide sequences encoding the variant protein under the control of expression control sequences optimized for expression in a desired host. For example, the sequences may include optimized transcriptional and/or translational regulatory sequences (such as altered Kozak sequences).

Hybridization assays

Detection of a nucleic acid of interest in a biological sample may optionally be effected by hybridization-based assays using an oligonucleotide probe (non-limiting examples of probes according to the present invention were previously described).

Traditional hybridization assays include PCR, RT-PCR, Real-time PCR, RNase protection, insitu hybridization, primer extension, Southern blots (DNA detection), dot or slot blots (DNA, RNA), and Northern blots (RNA detection) (NAT type assays are described in greater detail below). More recently, PNAs have been described (Nielsen et al. 1999, Current Opin. Biotechnol. 10:71-75). Other detection methods include kits containing probes on a dipstick setup and the like.

Hybridization based assays which allow the detection of a variant of interest (i.e., DNA or RNA) in a biological sample rely on the use of oligonucleotides which can be 10, 15, 20, or 30 to 100 nucleotides long preferably from 10 to 50, more preferably from 40 to 50 nucleotides long.

Thus, the isolated polynucleotides (oligonucleotides) of the present invention are preferably hybridizable with any of the herein described nucleic acid sequences under moderate to stringent hybridization conditions.

Moderate to stringent hybridization conditions are characterized by a hybridization solution such as containing 10 % dextrane sulfate, 1 M NaCl, 1 % SDS and 5 x $10^6$ cpm $^{32}P$ labeled probe, at 65 °C, with a final wash solution of 0.2 x SSC and 0.1 % SDS and final wash at 65°C and whereas moderate hybridization is effected using a hybridization solution containing 10 % dextrane sulfate, 1 M NaCl, 1 % SDS and 5 x $10^6$ cpm $^{32}P$ labeled probe, at 65 °C, with a final wash solution of 1 x SSC and 0.1 % SDS and final wash at 50 °C.

More generally, hybridization of short nucleic acids (below 200 bp in length, e.g. 17-40 bp in length) can be effected using the following exemplary hybridization protocols which can be modified according to the desired stringency; *(i)* hybridization solution of 6 x SSC and 1 % SDS or 3 M TMACl, 0.01 M sodium phosphate (pH 6.8), 1 mM EDTA (pH 7.6), 0.5 % SDS, 100 μg/ml denatured salmon sperm DNA and 0.1 % nonfat dried milk, hybridization temperature of 1 - 1.5 °C below the $T_m$, final wash solution of 3 M TMACl, 0.01 M sodium phosphate (pH 6.8), 1 mM EDTA (pH 7.6), 0.5 % SDS at 1 - 1.5 °C below the $T_m$; *(ii)* hybridization solution of 6 x SSC and 0.1 % SDS or 3 M TMACl, 0.01 M sodium phosphate (pH 6.8), 1 mM EDTA (pH 7.6), 0.5 % SDS, 100 μg/ml denatured salmon sperm DNA and 0.1 % nonfat dried milk, hybridization temperature of 2 - 2.5 °C below the $T_m$, final wash solution of 3 M TMACl, 0.01 M sodium phosphate (pH 6.8), 1 mM EDTA (pH 7.6), 0.5 % SDS at 1 - 1.5 °C below the $T_m$, final wash solution of 6 x SSC, and final wash at 22 °C; (iii) hybridization solution of 6 x SSC and 1 % SDS or 3 M TMACl, 0.01 M sodium phosphate (pH 6.8), 1 mM EDTA (pH 7.6), 0.5 % SDS, 100 μg/ml denatured salmon sperm DNA and 0.1 % nonfat dried milk, hybridization temperature.

The detection of hybrid duplexes can be carried out by a number of methods. Typically, hybridization duplexes are separated from unhybridized nucleic acids and the labels bound to the duplexes are then detected. Such labels refer to radioactive, fluorescent, biological or enzymatic tags or labels of standard use in the art. A label can be conjugated to either the oligonucleotide probes or the nucleic acids derived from the biological sample.

Probes can be labeled according to numerous well known methods. Non-limiting examples of radioactive labels include 3H, 14C, 32P, and 35S. Non-limiting examples of detectable markers include ligands, fluorophores, chemiluminescent agents, enzymes, and antibodies. Other detectable markers for use with probes, which can enable an increase in sensitivity of the method of the invention, include biotin and radio-nucleotides. It will become evident to the person of ordinary skill that the choice of a particular label dictates the manner in which it is bound to the probe.

For example, oligonucleotides of the present invention can be labeled subsequent to synthesis, by incorporating biotinylated dNTPs or rNTP, or some similar means (e.g., photo-cross-linking a psoralen derivative of biotin to RNAs), followed by addition of labeled streptavidin (e.g., phycoerythrin-conjugated streptavidin) or the equivalent. Alternatively, when fluorescently-labeled oligonucleotide probes are used, fluorescein, lissamine, phycoerythrin, rhodamine (Perkin Elmer Cetus), Cy2, Cy3, Cy3.5, Cy5, Cy5.5, Cy7, FluorX (Amersham) and others [e.g., Kricka et al. (1992), Academic Press San Diego, Calif] can be attached to the oligonucleotides.

Those skilled in the art will appreciate that wash steps may be employed to wash away excess target DNA or probe as well as unbound conjugate. Further, standard heterogeneous assay formats are suitable for detecting the hybrids using the labels present on the oligonucleotide primers and probes.

It will be appreciated that a variety of controls may be usefully employed to improve accuracy of hybridization assays. For instance, samples may be hybridized to an irrelevant probe and treated with RNAse A prior to hybridization, to assess false hybridization.

Although the present invention is not specifically dependent on the use of a label for the detection of a particular nucleic acid sequence, such a label might be beneficial, by increasing the sensitivity of the detection. Furthermore, it enables automation. Probes can be labeled according to numerous well known methods.

As commonly known, radioactive nucleotides can be incorporated into probes of the invention by several methods. Non-limiting examples of radioactive labels include $^3H$, $^{14}C$, $^{32}P$, and $^{35}S$.

Those skilled in the art will appreciate that wash steps may be employed to wash away excess target DNA or probe as well as unbound conjugate. Further, standard heterogeneous assay formats are suitable for detecting the hybrids using the labels present on the oligonucleotide primers and probes.
It will be appreciated that a variety of controls may be usefully employed to improve accuracy of hybridization assays.
Probes of the invention can be utilized with naturally occurring sugar-phosphate backbones as well as modified backbones including phosphorothioates, dithionates, alkyl phosphonates and a-nucleotides and the like. Probes of the invention can be constructed of either ribonucleic acid (RNA) or deoxyribonucleic acid (DNA), and preferably of DNA.

NAT Assays

Detection of a nucleic acid of interest in a biological sample may also optionally be effected by NAT-based assays, which involve nucleic acid amplification technology, such as PCR for example (or variations thereof such as real-time PCR for example).
As used herein, a "primer" defines an oligonucleotide which is capable of annealing to (hybridizing with) a target sequence, thereby creating a double stranded region which can serve as an initiation point for DNA synthesis under suitable conditions.
Amplification of a selected, or target, nucleic acid sequence may be carried out by a number of suitable methods. See generally Kwoh et al., 1990, Am. Biotechnol. Lab. 8:14 Numerous amplification techniques have been described and can be readily adapted to suit particular needs of a person of ordinary skill. Non-limiting examples of amplification techniques include polymerase chain reaction (PCR), ligase chain reaction (LCR), strand displacement amplification (SDA), transcription-based amplification, the q3 replicase system and NASBA (Kwoh et al., 1989, Proc. Natl. Acad. Sci. USA 86, 1173-1177; Lizardi et al., 1988, BioTechnology 6:1197-1202; Malek et al., 1994, Methods Mol. Biol., 28:253-260; and Sambrook et al., 1989, supra).
The terminology "amplification pair" (or "primer pair") refers herein to a pair of oligonucleotides (oligos) of the present invention, which are selected to be used together in amplifying a selected nucleic acid sequence by one of a number of types of amplification processes, preferably a polymerase chain reaction. Other types of amplification processes include ligase chain reaction, strand displacement amplification, or nucleic acid sequence-based amplification, as explained in greater detail below. As commonly known in the art, the oligos are designed to bind to a complementary sequence under selected conditions.
In one particular embodiment, amplification of a nucleic acid sample from a patient is amplified under conditions which favor the amplification of the most abundant differentially expressed nucleic acid. In one preferred embodiment, RT-PCR is carried out on an mRNA sample from a patient under conditions which favor the amplification of the most abundant mRNA. In another preferred embodiment, the amplification of the differentially expressed nucleic acids is carried out simultaneously. It will be realized by a person skilled in the art that such methods could be adapted for the detection of differentially expressed proteins instead of differentially expressed nucleic acid sequences.
The nucleic acid (i.e. DNA or RNA) for practicing the present invention may be obtained according to well known methods.
Oligonucleotide primers of the present invention may be of any suitable length, depending on the particular assay format and the particular needs and targeted genomes employed. Optionally, the oligonucleotide primers are at least 12 nucleotides in length, preferably between 15 and 24 molecules, and they may be adapted to be especially suited to a chosen nucleic acid amplification system. As commonly known in the art, the oligonucleotide primers can be designed by taking into consideration the melting point of hybridization thereof with its targeted sequence (Sambrook et al., 1989, Molecular Cloning -A Laboratory Manual, 2nd Edition, CSH Laboratories; Ausubel et al., 1989, in Current Protocols in Molecular Biology, John Wiley & Sons Inc., N.Y.).
It will be appreciated that antisense oligonucleotides may be employed to quantify expression of a splice isoform of interest. Such detection is effected at the pre-mRNA level. Essentially the ability to quantitate transcription from a splice site of interest can be effected based on splice site accessibility. Oligonucleotides may compete with splicing factors for the splice site sequences. Thus, low activity of the antisense oligonucleotide is indicative of splicing activity.
The polymerase chain reaction and other nucleic acid amplification reactions are well known in the art (various non-limiting examples of these reactions are described in greater detail below). The pair of oligonucleotides according to this aspect of the present invention are preferably selected to have compatible melting temperatures (Tm), e.g., melting temperatures which differ by less than that 7 °C, preferably less than 5 °C, more preferably less than 4 °C, most preferably less than 3 °C, ideally between 3 °C and 0 °C.
***Polymerase Chain Reaction (PCR):*** The polymerase chain reaction (PCR), as described in U.S. Pat. Nos. 4,683,195 and 4,683,202 to Mullis and Mullis *et al.,* is a method of increasing the concentration of a segment of target sequence in a mixture of genomic DNA without cloning or purification. This technology provides one approach to the problems of low target sequence concentration. PCR can be used to directly increase the concentration of the target to an easily detectable level. This process for amplifying the target sequence involves the introduction of a molar excess of two oligonucleotide primers which are complementary to their respective strands of the double-stranded target sequence to the DNA mixture containing the desired target sequence. The mixture is denatured and then allowed to hybridize. Following hybridization, the primers are extended with polymerase so as to form complementary strands. The steps of denaturation, hybridization (annealing), and polymerase extension (elongation) can be repeated as often as needed, in order to obtain relatively high concentrations of a segment of the desired target sequence.

The length of the segment of the desired target sequence is determined by the relative positions of the primers with respect to each other, and, therefore, this length is a controllable parameter. Because the desired segments of the target sequence become the dominant sequences (in terms of concentration) in the mixture, they are said to be "PCR-amplified."

***Ligase Chain Reaction (LCR or LAR):*** The ligase chain reaction [LCR; sometimes referred to as "Ligase Amplification Reaction" (LAR)] has developed into a well-recognized alternative method of amplifying nucleic acids. In LCR, four oligonucleotides, two adjacent oligonucleotides which uniquely hybridize to one strand of target DNA, and a complementary set of adjacent oligonucleotides, which hybridize to the opposite strand are mixed and DNA ligase is added to the mixture. Provided that there is complete complementarity at the junction, ligase will covalently link each set of hybridized molecules. Importantly, in LCR, two probes are ligated together only when they base-pair with sequences in the target sample, without gaps or mismatches. Repeated cycles of denaturation and ligation amplify a short segment of DNA. LCR has also been used in combination with PCR to achieve enhanced detection of single-base changes: see for example Segev, PCT Publication No. W09001069 A1 (1990). However, because the four oligonucleotides used in this assay can pair to form two short ligatable fragments, there is the potential for the generation of target-independent background signal. The use of LCR for mutant screening is limited to the examination of specific nucleic acid positions.

***Self-Sustained Synthetic Reaction (3SR/NASBA):*** The self-sustained sequence replication reaction (3SR) is a transcription-based in vitro amplification system that can exponentially amplify RNA sequences at a uniform temperature. The amplified RNA can then be utilized for mutation detection. In this method, an oligonucleotide primer is used to add a phage RNA polymerase promoter to the 5' end of the sequence of interest. In a cocktail of enzymes and substrates that includes a second primer, reverse transcriptase, RNase H, RNA polymerase and ribo-and deoxyribonucleoside triphosphates, the target sequence undergoes repeated rounds of transcription, cDNA synthesis and second-strand synthesis to amplify the area of interest. The use of 3SR to detect mutations is kinetically limited to screening small segments of DNA (e.g., 200-300 base pairs).

***Q-Beta* (Qβ) *Replicase:*** In this method, a probe which recognizes the sequence of interest is attached to the replicatable RNA template for Qβ replicase. A previously identified major problem with false positives resulting from the replication of unhybridized probes has been addressed through use of a sequence-specific ligation step. However, available thermostable DNA ligases are not effective on this RNA substrate, so the ligation must be performed by T4 DNA ligase at low temperatures (37 degrees C.). This prevents the use of high temperature as a means of achieving specificity as in the LCR, the ligation event can be used to detect a mutation at the junction site, but not elsewhere.

A successful diagnostic method must be very specific. A straight-forward method of controlling the specificity of nucleic acid hybridization is by controlling the temperature of the reaction. While the 3SR/NASBA, and Qβ systems are all able to generate a large quantity of signal, one or more of the enzymes involved in each cannot be used at high temperature (i.e., > 55 degrees C). Therefore the reaction temperatures cannot be raised to prevent non-specific hybridization of the probes. If probes are shortened in order to make them melt more easily at low temperatures, the likelihood of having more than one perfect match in a complex genome increases. For these reasons, PCR and LCR currently dominate the research field in detection technologies.

The basis of the amplification procedure in the PCR and LCR is the fact that the products of one cycle become usable templates in all subsequent cycles, consequently doubling the population with each cycle. The final yield of any such doubling system can be expressed as: $(1+X)^n = y$, where "X" is the mean efficiency (percent copied in each cycle), "n" is the number of cycles, and "y" is the overall efficiency, or yield of the reaction. If every copy of a target DNA is utilized as a template in every cycle of a polymerase chain reaction, then the mean efficiency is 100 %. If 20 cycles of PCR are performed, then the yield will be $2^{20}$, or 1,048,576 copies of the starting material. If the reaction conditions reduce the mean efficiency to 85 %, then the yield in those 20 cycles will be only $1.85^{20}$, or 220,513 copies of the starting material. In other words, a PCR running at 85 % efficiency will yield only 21 % as much final product, compared to a reaction running at 100 % efficiency. A reaction that is reduced to 50 % mean efficiency will yield less than I % of the possible product.

In practice, routine polymerase chain reactions rarely achieve the theoretical maximum yield, and PCRs are usually run for more than 20 cycles to compensate for the lower yield. At 50 % mean efficiency, it would take 34 cycles to achieve the million-fold amplification theoretically possible in 20, and at lower efficiencies, the number of cycles required becomes prohibitive. In addition, any background products that amplify with a better mean efficiency than the intended target will become the dominant products.

Also, many variables can influence the mean efficiency of PCR, including target DNA length and secondary structure, primer length and design, primer and dNTP concentrations, and buffer composition, to name but a few. Contamination of the reaction with exogenous DNA (e.g., DNA spilled onto lab surfaces) or cross-contamination is also a major consideration. Reaction conditions must be carefully optimized for each different primer pair and target sequence, and the process can take days, even for an experienced investigator. The laboriousness of this process, including numerous technical considerations and other factors, presents a significant drawback to using PCR in the clinical setting. Indeed, PCR has yet to penetrate the clinical market in a significant way. The same concerns arise with LCR, as LCR must also be optimized to use different oligonucleotide sequences for each target sequence. In addition, both methods require expensive equipment, capable of precise temperature cycling.

Additional NAT tests are Fluorescence In Situ Hybridization (FISH) and Comparative Genomic Hybridization (CGH). Fluorescence In Situ Hybridization (FISH) - The test uses fluorescent single-stranded DNA probes which are complementary to the DNA sequences that are under examination (genes or chromosomes). These probes hybridize with the complementary DNA and allow the identification of the chromosomal location of genomic sequences of DNA.

Comparative Genomic Hybridization (CGH) - allows a comprehensive analysis of multiple DNA gains and losses in entire genomes. Genomic DNA from the tissue to be investigated and a reference DNA are differentially labeled and simultaneously hybridized in situ to normal metaphase chromosomes. Variations in signal intensities are indicative of differences in the genomic content of the tissue under investigation.

Many applications of nucleic acid detection technologies, such as in studies of allelic variation, involve not only detection of a specific sequence in a complex background, but also the discrimination between sequences with few, or single, nucleotide differences. One method of the detection of allele-specific variants by PCR is based upon the fact that it is difficult for Taq polymerase to synthesize a DNA strand when there is a mismatch between the template strand and the 3' end of the primer. An allele-specific variant may be detected by the use of a primer that is perfectly matched with only one of the possible alleles; the mismatch to the other allele acts to prevent the extension of the primer, thereby preventing the amplification of that sequence. This method has a substantial limitation in that the base composition of the mismatch influences the ability to prevent extension across the mismatch, and certain mismatches do not prevent extension or have only a minimal effect.

A similar 3'-mismatch strategy is used with greater effect to prevent ligation in the LCR. Any mismatch effectively blocks the action of the thermostable ligase, but LCR still has the drawback of target-independent background ligation products initiating the amplification. Moreover, the combination of PCR with subsequent LCR to identify the nucleotides at individual positions is also a clearly cumbersome proposition for the clinical laboratory.

The direct detection method according to various preferred embodiments of the present invention may be, for example a cycling probe reaction (CPR) or a branched DNA analysis.

When a sufficient amount of a nucleic acid to be detected is available, there are advantages to detecting that sequence directly, instead of making more copies of that target, (e.g., as in PCR and LCR). Most notably, a method that does not amplify the signal exponentially is more amenable to quantitative analysis. Even if the signal is enhanced by attaching multiple dyes to a single oligonucleotide, the correlation between the final signal intensity and amount of target is direct. Such a system has an additional advantage that the products of the reaction will not themselves promote further reaction, so contamination of lab surfaces by the products is not as much of a concern. Recently devised techniques have sought to eliminate the use of radioactivity and/or improve the sensitivity in automatable formats. Two examples are the "Cycling Probe Reaction" (CPR), and "Branched DNA" (bDNA).

***Cycling probe reaction (CPR):*** The cycling probe reaction (CPR), uses a long chimeric oligonucleotide in which a central portion is made of RNA while the two termini are made of DNA. Hybridization of the probe to a target DNA and exposure to a thermostable RNase H causes the RNA portion to be digested. This destabilizes the remaining DNA portions of the duplex, releasing the remainder of the probe from the target DNA and allowing another probe molecule to repeat the process. The signal, in the form of cleaved probe molecules, accumulates at a linear rate. While the repeating process increases the signal, the RNA portion of the oligonucleotide is vulnerable to RNases that may carried through sample preparation.

***Branched DNA:*** Branched DNA (bDNA), involves oligonucleotides with branched structures that allow each individual oligonucleotide to carry 35 to 40 labels (e.g., alkaline phosphatase enzymes). While this enhances the signal from a hybridization event, signal from non-specific binding is similarly increased.

The detection of at least one sequence change according to various preferred embodiments of the present invention may be accomplished by, for example restriction fragment length polymorphism (RFLP analysis), allele specific oligonucleotide (ASO) analysis, Denaturing/Temperature Gradient Gel Electrophoresis (DGGE/TGGE), Single-Strand Conformation Polymorphism (SSCP) analysis or Dideoxy fingerprinting (ddF).

The demand for tests which allow the detection of specific nucleic acid sequences and sequence changes is growing rapidly in clinical diagnostics. As nucleic acid sequence data for genes from humans and pathogenic organisms accumulates, the demand for fast, cost-effective, and easy-to-use tests for as yet mutations within specific sequences is rapidly increasing.

A handful of methods have been devised to scan nucleic acid segments for mutations. One option is to determine the entire gene sequence of each test sample (e.g., a bacterial isolate). For sequences under approximately 600 nucleotides, this may be accomplished using amplified material (e.g., PCR reaction products). This avoids the time and expense associated with cloning the segment of interest. However, specialized equipment and highly trained personnel are required, and the method is too labor-intense and expensive to be practical and effective in the clinical setting.

In view of the difficulties associated with sequencing, a given segment of nucleic acid may be characterized on several other levels. At the lowest resolution, the size of the molecule can be determined by electrophoresis by comparison to a known standard run on the same gel. A more detailed picture of the molecule may be achieved by cleavage with combinations of restriction enzymes prior to electrophoresis, to allow construction of an ordered map. The presence of specific sequences within the fragment can be detected by hybridization of a labeled probe, or the precise nucleotide sequence can be determined by partial chemical degradation or by primer extension in the presence of chain-terminating nucleotide analogs.

***Restriction fragment length polymorphism (RFLP):*** For detection of single-base differences between like sequences, the requirements of the analysis are often at the highest level of resolution. For cases in which the position of the nucleotide in question is known in advance, several methods have been developed for examining single base changes without direct sequencing. For example, if a mutation of interest happens to fall within a restriction recognition sequence, a change in the pattern of digestion can be used as a diagnostic tool (e.g., restriction fragment length polymorphism [RFLP] analysis).

Single point mutations have been also detected by the creation or destruction of RFLPs. Mutations are detected and localized by the presence and size of the RNA fragments generated by cleavage at the mismatches. Single nucleotide mismatches in DNA heteroduplexes are also recognized and cleaved by some chemicals, providing an alternative strategy to detect single base substitutions, generically named the "Mismatch Chemical Cleavage" (MCC). However, this method requires the use of osmium tetroxide and piperidine, two highly noxious chemicals which are not suited for use in a clinical laboratory.

RFLP analysis suffers from low sensitivity and requires a large amount of sample. When RFLP analysis is used for the detection of point mutations, it is, by its nature, limited to the detection of only those single base changes which fall within a restriction sequence of a known restriction endonuclease. Moreover, the majority of the available enzymes has 4 to 6 base-pair recognition sequences, and cleaves too frequently for many large-scale DNA manipulations. Thus, it is applicable only in a small fraction of cases, as most mutations do not fall within such sites.

A handful of rare-cutting restriction enzymes with 8 base-pair specificities have been isolated and these are widely used in genetic mapping, but these enzymes are few in number, are limited to the recognition of G+C-rich sequences, and cleave at sites that tend to be highly clustered. Recently, endonucleases encoded by group I introns have been discovered that might have greater than 12 base-pair specificity, but again, these are few in number.

***Allele specific oligonucleotide (ASO):*** If the change is not in a recognition sequence, then allele-specific oligonucleotides (ASOs), can be designed to hybridize in proximity to the mutated nucleotide, such that a primer extension or ligation event can bused as the indicator of a match or a mis-match. Hybridization with radioactively labeled allelic specific oligonucleotides (ASO) also has been applied to the detection of specific point mutations. The method is based on the differences in the melting temperature of short DNA fragments differing by a single nucleotide. Stringent hybridization and washing conditions can differentiate between mutant and wild-type alleles. The ASO approach applied to PCR products also has been extensively utilized by various researchers to detect and characterize point mutations in ras genes and gsp/gip oncogenes. Because of the presence of various nucleotide changes in multiple positions, the ASO method requires the use of many oligonucleotides to cover all possible oncogenic mutations.

With either of the techniques described above (i.e., RFLP and ASO), the precise location of the suspected mutation must be known in advance of the test. That is to say, they are inapplicable when one needs to detect the presence of a mutation within a gene or sequence of interest.

***Denaturing/Temperature Gradient Gel Electrophoresis (DGGE/TGGE):*** Two other methods rely on detecting changes in electrophoretic mobility in response to minor sequence changes. One of these methods, termed "Denaturing Gradient Gel Electrophoresis" (DGGE) is based on the observation that slightly different sequences will display different patterns of local melting when electrophoretically resolved on a gradient gel. In this manner, variants can be distinguished, as differences in melting properties of homoduplexes versus heteroduplexes differing in a single nucleotide can detect the presence of mutations in the target sequences because of the corresponding changes in their electrophoretic mobilities. The fragments to be analyzed, usually PCR products, are "clamped" at one end by a long stretch of G-C base pairs (30-80) to allow complete denaturation of the sequence of interest without complete dissociation of the strands. The attachment of a GC "clamp" to the DNA fragments increases the fraction of mutations that can be recognized by DGGE. Attaching a GC clamp to one primer is critical to ensure that the amplified sequence has a low dissociation temperature. Modifications of the technique have been developed, using temperature gradients, and the method can be also applied to RNA:RNA duplexes.

Limitations on the utility of DGGE include the requirement that the denaturing conditions must be optimized for each type of DNA to be tested. Furthermore, the method requires specialized equipment to prepare the gels and maintain the needed high temperatures during electrophoresis. The expense associated with the synthesis of the clamping tail on one oligonucleotide for each sequence to be tested is also a major consideration. In addition, long running times are required for DGGE. The long running time of DGGE was shortened in a modification of DGGE called constant denaturant gel electrophoresis (CDGE). CDGE requires that gels be performed under different denaturant conditions in order to reach high efficiency for the detection of mutations.

A technique analogous to DGGE, termed temperature gradient gel electrophoresis (TGGE), uses a thermal gradient rather than a chemical denaturant gradient. TGGE requires the use of specialized equipment which can generate a temperature gradient perpendicularly oriented relative to the electrical field. TGGE can detect mutations in relatively small fragments of DNA therefore scanning of large gene segments requires the use of multiple PCR products prior to running the gel.

***Single Strand Conformation Polymorphism (SSCP):*** Another common method, called "Single-Strand Conformation Polymorphism" (SSCP) was developed by Hayashi, Sekya and colleagues and is based on the observation that single strands of nucleic acid can take on characteristic conformations in non-denaturing conditions, and these conformations influence electrophoretic mobility. The complementary strands assume sufficiently different structures that one strand may be resolved from the other. Changes in sequences within the fragment will also change the conformation, consequently altering the mobility and allowing this to be used as an assay for sequence variations.

The SSCP process involves denaturing a DNA segment (e.g., a PCR product) that is labeled on both strands, followed by slow electrophoretic separation on a non-denaturing polyacrylamide gel, so that intra-molecular interactions can form and not be disturbed during the run. This technique is extremely sensitive to variations in gel composition and temperature. A serious limitation of this method is the relative difficulty encountered in comparing data generated in different laboratories, under apparently similar conditions.

***Dideoxy fingerprinting (ddF):*** The dideoxy fingerprinting (ddF) is another technique developed to scan genes for the presence of mutations. The ddF technique combines components of Sanger dideoxy sequencing with SSCP. A dideoxy sequencing reaction is performed using one dideoxy terminator and then the reaction products are electrophoresed on nondenaturing polyacrylamide gels to detect alterations in mobility of the termination segments as in SSCP analysis. While ddF is an improvement over SSCP in terms of increased sensitivity, ddF requires the use of expensive dideoxynucleotides and this technique is still limited to the analysis of fragments of the size suitable for SSCP (i.e., fragments of 200-300 bases for optimal detection of mutations).

In addition to the above limitations, all of these methods are limited as to the size of the nucleic acid fragment that can be analyzed. For the direct sequencing approach, sequences of greater than 600 base pairs require cloning, with the consequent delays and expense of either deletion sub-cloning or primer walking, in order to cover the entire fragment. SSCP and DGGE have even more severe size limitations. Because of reduced sensitivity to sequence changes, these methods are not considered suitable for larger fragments. Although SSCP is reportedly able to detect 90 % of single-base substitutions within a 200 base-pair fragment, the detection drops to less than 50 % for 400 base pair fragments. Similarly, the sensitivity of DGGE decreases as the length of the fragment reaches 500 base-pairs. The ddF technique, as a combination of direct sequencing and SSCP, is also limited by the relatively small size of the DNA that can be screened.

According to a presently preferred embodiment of the present invention the step of searching for any of the nucleic acid sequences described here, in tumor cells or in cells derived from a cancer patient is effected by any suitable technique, including, but not limited to, nucleic acid sequencing, polymerase chain reaction, ligase chain reaction, self-sustained synthetic reaction, Qβ-Replicase, cycling probe reaction, branched DNA, restriction fragment length polymorphism analysis, mismatch chemical cleavage, heteroduplex analysis, allele-specific oligonucleotides, denaturing gradient gel electrophoresis, constant denaturant gel electrophoresis, temperature gradient gel electrophoresis and dideoxy fingerprinting.

Detection may also optionally be performed with a chip or other such device. The nucleic acid sample which includes the candidate region to be analyzed is preferably isolated, amplified and labeled with a reporter group. This reporter group can be a fluorescent group such as phycoerythrin. The labeled nucleic acid is then incubated with the probes immobilized on the chip using a fluidics station.

Once the reaction is completed, the chip is inserted into a scanner and patterns of hybridization are detected. The hybridization data is collected, as a signal emitted from the reporter groups already incorporated into the nucleic acid, which is now bound to the probes attached to the chip. Since the sequence and position of each probe immobilized on the chip is known, the identity of the nucleic acid hybridized to a given probe can be determined.

It will be appreciated that when utilized along with automated equipment, the above described detection methods can be used to screen multiple samples for a disease and/or pathological condition both rapidly and easily.

<u>Amino acid sequences and peptides</u>

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide" and "protein" include glycoproteins, as well as non-glycoproteins.

Polypeptide products can be biochemically synthesized such as by employing standard solid phase techniques. Such methods include but are not limited to exclusive solid phase synthesis, partial solid phase synthesis methods, fragment condensation, classical solution synthesis. These methods are preferably used when the peptide is relatively short (i.e., 10 kDa) and/or when it cannot be produced by recombinant techniques (i.e., not encoded by a nucleic acid sequence) and therefore involves different chemistry.

Solid phase polypeptide synthesis procedures are well known in the art and further described by John Morrow Stewart and Janis Dillaha Young, Solid Phase Peptide Syntheses (2nd Ed., Pierce Chemical Company, 1984).

Synthetic polypeptides can optionally be purified by preparative high performance liquid chromatography [Creighton T. (1983) Proteins, structures and molecular principles. WH Freeman and Co. N.Y.], after which their composition can be confirmed via amino acid sequencing.

In cases where large amounts of a polypeptide are desired, it can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

The present invention also encompasses polypeptides encoded by the polynucleotide sequences of the present invention, as well as polypeptides according to the amino acid sequences described herein. The present invention also encompasses homologues of these polypeptides, such homologues can be at least 50 %, at least 55 %, at least 60%, at least 65 %, at least 70 %, at least 75 %, at least 80 %, at least 85 %, at least 95 % or more say 100 % homologous to the amino acid sequences set forth below, as can be determined using BlastP software of the National Center of Biotechnology Information (NCBI) using default parameters, optionally and preferably including the following: filtering on (this option filters repetitive or low-complexity sequences from the query using the Seg (protein) program), scoring matrix is BLOSUM62 for proteins, word size is 3, E value is 10, gap costs are 11, 1 (initialization and extension), and number of alignments shown is 50. Preferably, nucleic acid sequence homology/identity is determined by using BlastN software of the National Center of Biotechnology Information (NCBI) using default parameters, which preferably include using the DUST filter program, and also preferably include having an E value of 10, filtering low complexity sequences and a word size of 11. Finally, the present invention also encompasses fragments of the above described polypeptides and polypeptides having mutations, such as deletions, insertions or substitutions of one or more amino acids, either naturally occurring or artificially induced, either randomly or in a targeted fashion.

It will be appreciated that peptides identified according the present invention may be degradation products, synthetic peptides or recombinant peptides as well as peptidomimetics, typically, synthetic peptides and peptoids and semipeptoids which are peptide analogs, which may have, for example, modifications rendering the peptides more stable while in a body or more capable of penetrating into cells. Such modifications include, but are not limited to N terminus modification, C terminus modification, peptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified. Further details in this respect are provided hereinunder.

Peptide bonds (-CO-NH-) within the peptide may be substituted, for example, by N-methylated bonds (-N (CH3)-CO-), ester bonds (-C(R)H-C-O-O-C(R)-N-), ketomethylen bonds (-CO-CH2-), $\alpha$-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefmic double bonds (-CH=CH-), retro amide bonds (-NH-CO-), peptide derivatives (-N(R)-CH2-CO-), wherein R is the "normal" side chain, naturally presented on the carbon atom.

These modifications can occur at any of the bonds along the peptide chain and even at several (2-3) at the same time.

Natural aromatic amino acids, Trp, Tyr and Phe, may be substituted for synthetic non-natural acid such as Phenylglycine, TIC, naphthylelanine (Nol), ring-methylated derivatives of Phe, halogenated derivatives of Phe or o-methyl-Tyr.

In addition to the above, the peptides of the present invention may also include one or more modified amino acids or one or more non-amino acid monomers (e.g. fatty acids, complex carbohydrates etc).

As used herein in the specification and in the claims section below the term "amino acid" or "amino acids" is understood to include the 20 naturally occurring amino acids; those amino acids often modified post-translationally *in vivo,* including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids. Non-conventional or modified amino acids can be incorporated in the polypeptides of this invention as well, as will be known to one skilled in the art.

Since the peptides of the present invention are preferably utilized in diagnostics which require the peptides to be in soluble form, the peptides of the present invention preferably include one or more non-natural or natural polar amino acids, including but not limited to serine and threonine which are capable of increasing peptide solubility due to their hydroxyl-containing side chain.

The peptides of the present invention are preferably utilized in a linear form, although it will be appreciated that in cases where cyclization does not severely interfere with peptide characteristics, cyclic forms of the peptide can also be utilized.

The peptides of present invention can be biochemically synthesized such as by using standard solid phase techniques. These methods include exclusive solid phase synthesis well known in the art, partial solid phase synthesis methods, fragment condensation, classical solution synthesis. These methods are preferably used when the peptide is relatively short (i.e., 10 kDa) and/or when it cannot be produced by recombinant techniques (i.e., not encoded by a nucleic acid sequence) and therefore involves different chemistry.

Synthetic peptides can be purified by preparative high performance liquid chromatography and the composition of which can be confirmed via amino acid sequencing.

In cases where large amounts of the peptides of the present invention are desired, the peptides of the present invention can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224: 838-843, Gurley et al. (1986) Mol. Cell. Biol. 6:559-565 and Weissbach & Weissbach, 1988, Methods for Plant Molecular Biology, Academic Press, NY, Section VIII, pp 421-463 and also as described above.

Antibodies:

"Antibody" refers to a polypeptide ligand that is preferably substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad-immunoglobulin variable region genes. Antibodies exist, e.g., as intact immunoglobulins or as a number of well characterized fragments produced by digestion with various peptidases. This includes, e.g., Fab' and $F(ab)'_2$ fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region.

The functional fragments of antibodies, such as Fab, F(ab')2, and Fv that are capable of binding to macrophages, are described as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; and (5) Single chain antibody ("SCA"), a genetically engineered molecule containing the variable region of the light chain and the variable region of the heavy chain, linked by a suitable polypeptide linker as a genetically fused single chain molecule.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988, incorporated herein by reference).

Monoclonal antibody development may optionally be performed according to any method that is known in the art. The method described below is provided for the purposes of description only and is not meant to be limiting in any way.

Antibody Engineering in Phage Display Libraries:

Antibodies of this invention may be prepared through the use of phage display libraries, as is known in the art, for example, as described in PCT Application No. WO 94/18219, US Patent No. 6096551, both of which are hereby fully incorporated by reference, The method involves inducing mutagenesis in a complementarity determining region (CDR) of an immunoglobulin light chain gene for the purpose of producing light chain gene libraries for use in combination with heavy chain genes and gene libraries to produce antibody libraries of diverse and novel immuno-specificities. The method comprises amplifying a CDR portion of an immunoglobulin light chain gene by polymerase chain reaction (PCR) using a PCR primer oligonucleotide. The resultant gene portions are inserted into phagemids for production of a phage display library, wherein the engineered light chains are displayed by the phages, for example for testing their binding specificity.

Antibody fragments according to the present invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using Papain produces two monovalent Fab' fragments and an Fc fragment directly. These methods are described, for example, by Goldenberg, U.S. Pat. Nos. 4,036,945 and 4,331,647, and references contained therein, which patents are hereby incorporated by reference in their entirety. See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'1 Acad. Sci. USA 69:2659-62 (1972)]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. A scFv antibody fragment is an engineered antibody derivative that includes heavy- and light chain variable regions joined by a peptide linker. The minimal size of antibody molecules are those that still comprise the complete antigen binding site. ScFv antibody fragments are potentially more effective than unmodified IgG antibodies. The reduced size of 27-30 kDa permits them to penetrate tissues and solid tumors more readily. Methods for producing sFvs are described, for example, by [Whitlow and Filpula, Methods 2: 97-105 (1991); Bird et al., Science 242:423-426 (1988); Pack et al., Bio/Technology 11:1271-77 (1993); and U.S. Pat. No. 4,946,778, which is hereby incorporated by reference in its entirety.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)]. Optionally, there may be 1, 2 or 3 CDRs of different chains, but preferably there are 3 CDRs of 1 chain. The chain could be the heavy or the light chain.

Humanized forms of non-human (e.g., murine) antibodies, are chimeric molecules of immunoglobulins, immunoglobulin chains or fragments thereof (such as Fv, Fab, Fab', F(ab') or other antigen-binding subsequences of antibodies) which contain minimal sequence derived from non-human immunoglobulin, or fragments thereof may comprise the antibodies of this invention. Humanized antibodies are well known in the art. Methods for humanizing non-human antibodies are well known in the art, for example, as described in Winter and co-workers [Jones et al., Nature, 321:522-525 (1986); Riechmann et al., Nature 332:323-327 (1988); Verhoeyen et al., Science, 239:1534-1536 (1988)], U.S. Pat. No. 4,816,567, Hoogenboom and Winter, J. Mol. Biol., 227:381 (1991); Marks et al., J. Mol. Biol., 222:581 (1991), Cole et al., Monoclonal Antibodies and Cancer Therapy, Alan R. Liss, p. 77 (1985), Boerner et al., J. Immunol., 147(1):86-95 (1991), U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995), all of which are incorporated herein by reference.

Preferably, the antibody of this aspect of the present invention specifically binds at least one epitope of the polypeptide variants of the present invention. As used herein, the term "epitope" refers to any antigenic determinant on an antigen to which the paratope of an antibody binds.

Epitopic determinants usually consist of chemically active surface groupings of molecules such as amino acids or carbohydrate side chains and usually have specific three dimensional structural characteristics, as well as specific charge characteristics.

Optionally, a unique epitope may be created in a variant due to a change in one or more post-translational modifications, including but not limited to glycosylation and/or phosphorylation, as described below. Such a change may also cause a new epitope to be created, for example through removal of glycosylation at a particular site.

An epitope according to the present invention may also optionally comprise part or all of a unique sequence portion of a variant according to the present invention in combination with at least one other portion of the variant which is not contiguous to the unique sequence portion in the linear polypeptide itself, yet which are able to form an epitope in combination. One or more unique sequence portions may optionally combine with one or more other non-contiguous portions of the variant (including a portion which may have high homology to a portion of the known protein) to form an epitope.

Immunoassays

In another embodiment of the present invention, an immunoassay can be used to qualitatively or quantitatively detect and analyze markers in a sample. This method comprises: providing an antibody that specifically binds to a marker; contacting a sample with the antibody; and detecting the presence of a complex of the antibody bound to the marker in the sample.

To prepare an antibody that specifically binds to a marker, purified protein markers can be used. Antibodies that specifically bind to a protein marker can be prepared using any suitable methods known in the art.

After the antibody is provided, a marker can be detected and/or quantified using any of a number of well recognized immunological binding assays. Useful assays include, for example, an enzyme immune assay (EIA) such as enzyme-linked immunosorbent assay (ELISA), a radioimmune assay (RIA), a Western blot assay, or a slot blot assay see, e.g., U.S. Pat. Nos. 4,366,241; 4,376,110; 4,517,288; and 4,837,168). Generally, a sample obtained from a subject can be contacted with the antibody that specifically binds the marker.

Optionally, the antibody can be fixed to a solid support to facilitate washing and subsequent isolation of the complex, prior to contacting the antibody with a sample. Examples of solid supports include but are not limited to glass or plastic in the form of, e.g., a microtiter plate, a stick, a bead, or a microbead. Antibodies can also be attached to a solid support.

After incubating the sample with antibodies, the mixture is washed and the antibody-marker complex formed can be detected. This can be accomplished by incubating the washed mixture with a detection reagent. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume of solution, concentrations and the like. Usually the assays will be carried out at ambient temperature, although they can be conducted over a range of temperatures, such as 10 °C to 40 °C.

The immunoassay can be used to determine a test amount of a marker in a sample from a subject. First, a test amount of a marker in a sample can be detected using the immunoassay methods described above. If a marker is present in the sample, it will form an antibody-marker complex with an antibody that specifically binds the marker under suitable incubation conditions described above. The amount of an antibody-marker complex can optionally be determined by comparing to a standard. As noted above, the test amount of marker need not be measured in absolute units, as long as the unit of measurement can be compared to a control amount and/or signal.

Preferably used are antibodies which specifically interact with the polypeptides of the present invention and not with wild type proteins or other isoforms thereof, for example. Such antibodies are directed, for example, to the unique sequence portions of the polypeptide variants of the present invention, including but not limited to bridges, heads, tails and insertions described in greater detail below. Preferred embodiments of antibodies according to the present invention are described in greater detail with regard to the section entitled "Antibodies".

*Radio-immunoassay (RIA):* In one version, this method involves precipitation of the desired substrate and in the methods detailed hereinbelow, with a specific antibody and radiolabelled antibody binding protein (e.g., protein A labeled with $I^{125}$) immobilized on a precipitable carrier such as agarose beads. The number of counts in the precipitated pellet is proportional to the amount of substrate.

In an alternate version of the RIA, a labeled substrate and an unlabelled antibody binding protein are employed. A sample containing an unknown amount of substrate is added in varying amounts. The decrease in precipitated counts from the labeled substrate is proportional to the amount of substrate in the added sample.

*Enzyme linked immunosorbent assay (ELISA):* This method involves fixation of a sample (e.g., fixed cells or a proteinaceous solution) containing a protein substrate to a surface such as a well of a microtiter plate. A substrate specific antibody coupled to an enzyme is applied and allowed to bind to the substrate. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

*Western blot:* This method involves separation of a substrate from other protein by means of an acrylamide gel followed by transfer of the substrate to a membrane (e.g., nylon or PVDF). Presence of the substrate is then detected by antibodies specific to the substrate, which are in turn detected by antibody binding reagents. Antibody binding reagents may be, for example, protein A, or other antibodies. Antibody binding reagents may be radiolabelled or enzyme linked as described hereinabove. Detection may be by autoradiography, colorimetric reaction or chemiluminescence. This method allows both quantitation of an amount of substrate and determination of its identity by a relative position on the membrane which is indicative of a migration distance in the acrylamide gel during electrophoresis.

*Immunohistochemical analysis:* This method involves detection of a substrate *in situ* in fixed cells by substrate specific antibodies. The substrate specific antibodies may be enzyme linked or linked to fluorophores. Detection is by microscopy and subjective evaluation. If enzyme linked antibodies are employed, a colorimetric reaction may be required.

*Fluorescence activated cell sorting (FACS):* This method involves detection of a substrate *in situ* in cells by substrate specific antibodies. The substrate specific antibodies are linked to fluorophores. Detection is by means of a cell sorting machine which reads the wavelength of light emitted from each cell as it passes through a light beam. This method may employ two or more antibodies simultaneously.

## Radio-imaging Methods

These methods include but are not limited to, positron emission tomography (PET) single photon emission computed tomography (SPECT). Both of these techniques are non-invasive, and can be used to detect and/or measure a wide variety of tissue events and/or functions, such as detecting cancerous cells for example. Unlike PET, SPECT can optionally be used with two labels simultaneously. SPECT has some other advantages as well, for example with regard to cost and the types of labels that can be used. For example, US Patent No. 6,696,686 describes the use of SPECT for detection of breast cancer, and is hereby incorporated by reference as if fully set forth herein.

## Display Libraries

According to still another aspect of the present invention there is provided a display library comprising a plurality of display vehicles (such as phages, viruses or bacteria) each displaying at least 6, at least 7, at least 8, at least 9, at least 10, 10-15, 12-17, 15-20, 15-30 or 20-50 consecutive amino acids derived from the polypeptide sequences of the present invention.

Methods of constructing such display libraries are well known in the art. Such methods are described in, for example, Young AC, et al., "The three-dimensional structures of a polysaccharide binding antibody to Cryptococcus neoformans and its complex with a peptide from a phage display library: implications for the identification of peptide mimotopes" J Mol Biol 1997 Dec 12;274(4):622-34; Giebel LB et al. "Screening of cyclic peptide phage libraries identifies ligands that bind streptavidin with high affinities" Biochemistry 1995 Nov 28;34(47):15430-5; Davies EL et al., "Selection of specific phage-display antibodies using libraries derived from chicken immunoglobulin genes" J Immunol Methods 1995 Oct 12;186(1):125-35; Jones C RT al. "Current trends in molecular recognition and bioseparation" J Chromatogr A 1995 Jul 14;707(1):3-22; Deng SJ et al. "Basis for selection of improved carbohydrate-binding single-chain antibodies from synthetic gene libraries" Proc Natl Acad Sci U S A 1995 May 23;92(11):4992-6; and Deng SJ et al. "Selection of antibody single-chain variable fragments with improved carbohydrate binding by phage display" J Biol Chem 1994 Apr 1;269(13):9533-8, which are incorporated herein by reference.

## Theranostics:

The term theranostics describes the use of diagnostic testing to diagnose the disease, choose the correct treatment regime according to the results of diagnostic testing and/or monitor the patient response to therapy according to the results of diagnostic testing. Theranostic tests can be used to select patients for treatments that are particularly likely to benefit them and unlikely to produce side-effects. They can also provide an early and objective indication of treatment efficacy in individual patients, so that (if necessary) the treatment can be altered with a minimum of delay. For example: DAKO and Genentech together created HercepTest and Herceptin (trastuzumab) for the treatment of breast cancer, the first theranostic test approved simultaneously with a new therapeutic drug. In addition to HercepTest (which is an immunohistochemical test), other theranostic tests are in development which use traditional clinical chemistry, immunoassay, cell-based technologies and nucleic acid tests. PPGx's recently launched TPMT (thiopurine S-methyltransferase) test, which is enabling doctors to identify patients at risk for potentially fatal adverse reactions to 6-mercaptopurine, an agent used in the treatment of leukemia. Also, Nova Molecular pioneered SNP genotyping of the apolipoprotein E gene to predict Alzheimer's disease patients' responses to cholinomimetic therapies and it is now widely used in clinical trials of new drugs for this indication. Thus, the field of theranostics represents the intersection of diagnostic testing information that predicts the response of a patient to a treatment with the selection of the appropriate treatment for that particular patient.

Surrogate markers:

A surrogate marker is a marker, that is detectable in a laboratory and/or according to a physical sign or symptom on the patient, and that is used in therapeutic trials as a substitute for a clinically meaningful endpoint. The surrogate marker is a direct measure of how a patient feels, functions, or survives which is expected to predict the effect of the therapy. The need for surrogate markers mainly arises when such markers can be measured earlier, more conveniently, or more frequently than the endpoints of interest in terms of the effect of a treatment on a patient, which are referred to as the clinical endpoints. Ideally, a surrogate marker should be biologically plausible, predictive of disease progression and measurable by standardized assays (including but not limited to traditional clinical chemistry, immunoassay, cell-based technologies, nucleic acid tests and imaging modalities).

Surrogate endpoints were used first mainly in the cardiovascular area. For example, antihypertensive drugs have been approved based on their effectiveness in lowering blood pressure. Similarly, in the past, cholesterol-lowering agents have been approved based on their ability to decrease serum cholesterol, not on the direct evidence that they decrease mortality from atherosclerotic heart disease. The measurement of cholesterol levels is now an accepted surrogate marker of atherosclerosis. In addition, currently two commonly used surrogate markers in HIV studies are CD4+ T cell counts and quantitative plasma HIV RNA (viral load). In some embodiments of this invention, the polypeptide/polynucleotide expression pattern may serve as a surrogate marker for a particular disease, as will be appreciated by one skilled in the art.

Monoclonal Antibody Therapy:

In some embodiments, monoclonal antibodies are useful for the identification of cancer cells. In some embodiments, monoclonal antibody therapy is a form of passive immunotherapy useful in cancer treatment. Such antibodies may comprise naked monoclonal antibodies or conjugated monoclonal antibodies - joined to a chemotherapy drug, radioactive particle, or a toxin (a substance that poisons cells). In some embodiments, the former is directly cytotoxic to the target (cancer) cell, or in another embodiment, stimulates or otherwise participates in an immune response ultimately resulting in the lysis of the target cell.

In some embodiments, the conjugated monoclonal antibodies are joined to drugs, toxins, or radioactive atoms. They are used as delivery vehicles to take those substances directly to the cancer cells. The MAb acts as a homing device, circulating in the body until it finds a cancer cell with a matching antigen. It delivers the toxic substance to where it is needed most, minimizing damage to normal cells in other parts of the body. Conjugated MAbs are also sometimes referred to as "tagged," "labeled," or "loaded" antibodies. MAbs with chemotherapy drugs attached are generally referred to as chemolabeled. MAbs with radioactive particles attached are referred to as radiolabeled, and this type of therapy is known as radioimmunotherapy (RIT). MAbs attached to toxins are called immunotoxins.

An illustrative, non-limiting example is provided herein of a method of treatment of a patient with an antibody to a variant as described herein, such that the variant is a target of the antibody. A patient with breast cancer is treated with a radiolabeled humanized antibody against an appropriate breast cancer target as described herein. The patient is optionally treated with a dosage of labeled antibody ranging from 10 to 30 mCi. Of course any type of therapeutic label may optionally be used.

The following sections relate to Candidate Marker Examples. It should be noted that Table numbering is restarted within each Example, which starts with the words "Description for Cluster".

CANDIDATE MARKER EXAMPLES SECTION

This Section relates to Examples of sequences according to the present invention, including illustrative methods of selection thereof with regard to cancer; other markers were selected as described below for the individual markers.

Description of the methodology undertaken to uncover the biomolecular sequences of the present invention

Human ESTs and cDNAs were obtained from GenBank versions 136 (June 15, 2003 ftp.ncbi.nih.gov/genbank/release.notes/gb136.release.notes); NCBI genome assembly of April 2003; RefSeq sequences from June 2003; Genbank version 139 (December 2003); Human Genome from NCBI (Build 34) (from Oct 2003); and RefSeq sequences from December 2003. With regard to GenBank sequences, the human EST sequences from the EST (GBEST) section and the human mRNA sequences from the primate (GBPRI) section were used; also the human nucleotide RefSeq mRNA sequences were used (see for example www.ncbi.nlm.nih.gov/Genbank/GenbankOverview.html and for a reference to the EST section, see www.ncbi.nlm.nih.gov/dbEST/; a general reference to dbEST, the EST database in GenBank, may be found in Boguski et al, Nat Genet. 1993 Aug;4(4):332-3; all of which are hereby incorporated by reference as if fully set forth herein).

Novel splice variants were predicted using the LEADS clustering and assembly system as described in Sorek, R., Ast, G. & Graur, D. Alu-containing exons are alternatively spliced. Genome Res 12, 1060-7 (2002); US patent No: 6,625,545; and U.S. Pat. Appl. No. 10/426,002, published as US20040101876 on May 27 2004; all of which are hereby incorporated by reference as if fully set forth herein. Briefly, the software cleans the expressed sequences from repeats, vectors and immunoglobulins. It then aligns the expressed sequences to the genome taking alternatively splicing into account and clusters overlapping expressed sequences into "clusters" that represent genes or partial genes.

These were annotated using the GeneCarta (Compugen, Tel-Aviv, Israel) platform. The GeneCarta platform includes a rich pool of annotations, sequence information (particularly of spliced sequences), chromosomal information, alignments, and additional information such as SNPs, gene ontology terms, expression profiles, functional analyses, detailed domain structures, known and predicted proteins and detailed homology reports.

A brief explanation is provided with regard to the method of selecting the candidates. However, it should be noted that this explanation is provided for descriptive purposes only, and is not intended to be limiting in any way. The potential markers were identified by a computational process that was designed to find genes and/or their splice variants that are differentially expressed in cancer tissues as opposed to non-cancerous. Various parameters related to the information in the EST libraries, determined according to classification by library annotation, were used to assist in locating genes and/or splice variants thereof that are specifically and/or differentially expressed in heart tissues. The detailed description of the selection method and of these parameters is presented in Example 1 below.

SELECTING CANDIDATES WITH REGARD TO CANCER

A brief explanation is provided with regard to a non-limiting method of selecting the candidates for cancer diagnostics. However, it should noted that this explanation is provided for descriptive purposes only, and is not intended to be limiting in any way. The potential markers were identified by a computational process that was designed to find genes and/or their splice variants that are over-expressed in tumor tissues, by using databases of expressed sequences. Various parameters related to the information in the EST libraries, determined according to a manual classification process, were used to assist in locating genes and/or splice variants thereof that are over-expressed in cancerous tissues. The detailed description of the selection method is presented in Example 1 below. The cancer biomarkers selection engine and the following wet validation stages are schematically summarized in Figure 1.

## EXAMPLE 1

### *Identification of differentially expressed gene products-Algorithm*

In order to distinguish between differentially expressed gene products and constitutively expressed genes (i.e., house keeping genes) an algorithm based on an analysis of frequencies was configured. A specific algorithm for identification of transcripts over expressed in cancer is described hereinbelow.

Dry analysis

**Library annotation** - EST libraries are manually classified according to:

(i) Tissue origin
(ii) Biological source - Examples of frequently used biological sources for construction of EST libraries include cancer cell-lines; normal tissues; cancer tissues; fetal tissues; and others such as normal cell lines and pools of normal cell-lines, cancer cell-lines and combinations thereof. A specific description of abbreviations used below with regard to these tissues/cell lines etc is given above.
(iii) Protocol of library construction - various methods are known in the art for library construction including normalized library construction; non-normalized library construction; subtracted libraries; ORESTES and others. It will be appreciated that at times the protocol of library construction is not indicated.

The following rules are followed:

EST libraries originating from identical biological samples are considered as a single library.
EST libraries which include above-average levels of DNA contamination are eliminated.

**Dry computation** - development of engines which are capable of identifying genes and splice variants that are temporally and spacially expressed.

Clusters (genes) having at least five sequences including at least two sequences from the tissue of interest are analyzed.

## EXAMPLE 2

***Identification of genes over expressed in cancer.***

Two different scoring algorithms were developed.

Libraries score -candidate sequences which are supported by a number of cancer libraries, are more likely to serve as specific and effective diagnostic markers.

**The basic algorithm** - for each cluster the number of cancer and normal libraries contributing sequences to the cluster was counted. Fisher exact test was used to check if cancer libraries are significantly over-represented in the cluster as compared to the total number of cancer and normal libraries.

**Library counting**: Small libraries (e.g., less than 1000 sequences) were excluded from consideration unless they participate in the cluster. For this reason, the total number of libraries is actually adjusted for each cluster.

**Clones no. score** - Generally, when the number of ESTs is much higher in the cancer libraries relative to the normal libraries it might indicate actual over-expression.

**The algorithm -**

Clone counting: For counting EST clones each library protocol class was given a weight based on our belief of how much the protocol reflects actual expression levels:

(i) non-normalized : 1
(ii) normalized : 0.2
(iii) all other classes : 0.1

Clones number score - The total weighted number of EST clones from cancer libraries was compared to the EST clones from normal libraries. To avoid cases where one library contributes to the majority of the score, the contribution of the library that gives most clones for a given cluster was limited to 2 clones.

The score was computed as

$$\frac{\frac{c+1}{C}}{\frac{n+1}{N}}$$

where:

c - weighted number of "cancer" clones in the cluster.
C- weighted number of clones in all "cancer" libraries.
n - weighted number of "normal" clones in the cluster.
N- weighted number of clones in all "normal" libraries.

Clones number score significance - Fisher exact test was used to check if EST clones from cancer libraries are significantly over-represented in the cluster as compared to the total number of EST clones from cancer and normal libraries.

Two search approaches were used to find either general cancer-specific candidates or tumor specific candidates.

- Libraries/sequences originating from tumor tissues are counted as well as libraries originating from cancer cell-lines ("normal" cell-lines were ignored).
- Only libraries/sequences originating from tumor tissues are counted

## EXAMPLE 3

### *Identification of tissue specific genes*

For detection of tissue specific clusters, tissue libraries/sequences were compared to the total number of libraries/sequences in cluster. Similar statistical tools to those described in above were employed to identify tissue specific genes. Tissue abbreviations are the same as for cancerous tissues, but are indicated with the header "normal tissue".

The algorithm - for each tested tissue T and for each tested cluster the following were examined:

1. Each cluster includes at least 2 libraries from the tissue T. At least 3 clones (weighed - as described above) from tissue T in the cluster; and
2. Clones from the tissue T are at least 40 % from all the clones participating in the tested cluster

Fisher exact test P-values were computed both for library and weighted clone counts to check that the counts are statistically significant.

## EXAMPLE 4

### *Oligonucleotide-based micro-array experiment protocol*

Microarray fabrication

Microarrays (chips) were printed by pin deposition using the MicroGrid II MGII 600 robot from BioRobotics Limited (Cambridge, UK). 50-mer oligonucleotides target sequences were designed by Compugen Ltd (Tel-Aviv, IL) as described by A. Shoshan et al, "Optical technologies and informatics", Proceedings of SPIE. Vol 4266, pp. 86-95 (2001). The designed oligonucleotides were synthesized and purified by desalting with the Sigma-Genosys system (The Woodlands, TX, US) and all of the oligonucleotides were joined to a C6 amino-modified linker at the 5' end, or being attached directly to CodeLink slides (Cat #25-6700-01. Amersham Bioscience, Piscataway, NJ, US). The 50-mer oligonucleotides, forming the target sequences, were first suspended in Ultra-pure DDW (Cat # 01-866-1A Kibbutz Beit-Haemek, Israel) to a concentration of 50μM. Before printing the slides, the oligonucleotides were resuspended in 300mM sodium phosphate (pH 8.5) to final concentration of 150mM and printed at 35-40% relative humidity at 21°C.

Each slide contained a total of 9792 features in 32 subarrays. Of these features, 4224 features were sequences of interest according to the present invention and negative controls that were printed in duplicate. An additional 288 features (96 target sequences printed in triplicate) contained housekeeping genes from Human Evaluation Library2, Compugen Ltd, Israel. Another 384 features are E. coli spikes 1-6, which are oligos to E-Coli genes which are commercially available in the Array Control product (Array control- sense oligo spots, Ambion Inc. Austin, TX. Cat #1781, Lot #112K06).

Post-coupling processing of printed slides

After the spotting of the oligonucleotides to the glass (CodeLink) slides, the slides were incubated for 24 hours in a sealed saturated NaCl humidification chamber (relative humidity 70-75%).

Slides were treated for blocking of the residual reactive groups by incubating them in blocking solution at 50°C for 15 minutes (10ml/slide of buffer containing 0.1M Tris, 50mM ethanolamine, 0.1% SDS). The slides were then rinsed twice with Ultra-pure DDW (double distilled water). The slides were then washed with wash solution (10ml/slide. 4X SSC, 0.1% SDS)) at 50°C for 30 minutes on the shaker. The slides were then rinsed twice with Ultra-pure DDW, followed by drying by centrifugation for 3 minutes at 800 rpm.

Next, in order to assist in automatic operation of the hybridization protocol, the slides were treated with Ventana Discovery hybridization station barcode adhesives. The printed slides were loaded on a Bio-Optica (Milan, Italy) hematology staining device and were incubated for 10 minutes in 50ml of 3-Aminopropyl Triethoxysilane (Sigma A3648 lot #122K589). Excess fluid was dried and slides were then incubated for three hours in 20 mm/Hg in a dark vacuum desiccator (Pelco 2251, Ted Pella, Inc. Redding CA).

The following protocol was then followed with the Genisphere 900-RP (random primer), with mini elute columns on the Ventana Discovery HybStation™, to perform the microarray experiments. Briefly, the protocol was performed as described with regard to the instructions and information provided with the device itself. The protocol included cDNA synthesis and labeling. cDNA concentration was measured with the TBS-380 (Turner Biosystems. Sunnyvale, CA.) PicoFlour, which is used with the OliGreen ssDNA Quantitation reagent and kit.

Hybridization was performed with the Ventana Hybridization device, according to the provided protocols (Discovery Hybridization Station Tuscon AZ).

The slides were then scanned with GenePix 4000B dual laser scanner from Axon Instruments Inc, and analyzed by GenePix Pro 5.0 software.

Schematic summary of the oligonucleotide based microarray fabrication and the experimental flow is presented in Figures 3 and 4.

Briefly, as shown in Figure 3, DNA oligonucleotides at 25uM were deposited (printed) onto Amersham 'CodeLink' glass slides generating a well defined 'spot'. These slides are covered with a long-chain, hydrophilic polymer chemistry that creates an active 3-D surface that covalently binds the DNA oligonucleotides 5'-end via the C6-amine modification. This binding ensures that the full length of the DNA oligonucleotides is available for hybridization to the cDNA and also allows lower background, high sensitivity and reproducibility.

Figure 4 shows a schematic method for performing the microarray experiments. It should be noted that stages on the left-hand or right-hand side may optionally be performed in any order, including in parallel, until stage 4 (hybridization). Briefly, on the left-hand side, the target oligonucleotides are being spotted on a glass microscope slide (although optionally other materials could be used) to form a spotted slide (stage 1). On the right hand side, control sample RNA and cancer sample RNA are Cy3 and Cy5 labeled, respectively (stage 2), to form labeled probes. It should be noted that the control and cancer samples come from corresponding tissues (for example, normal prostate tissue and cancerous prostate tissue). Furthermore, the tissue from which the RNA was taken is indicated below in the specific examples of data for particular clusters, with regard to overexpression of an oligonucleotide from a "chip" (microarray), as for example "prostate" for chips in which prostate cancerous tissue and normal tissue were tested as described above. In stage 3, the probes are mixed. In stage 4, hybridization is performed to form a processed slide. In stage 5, the slide is washed and scanned to form an image file, followed by data analysis in stage 6.

## EXAMPLE 5

### *Diseases and conditions that may be diagnosed with one or more variants according to the present invention*

Cardiovascular and cerebrovascular conditions

Various examples are listed below for conditions that affect the vascular system, including various cardiovascular and cerebrovascular conditions, for which one or more variants according to the present invention may have a diagnostic utility. Based on these diseases mechanisms and the correlation between the known proteins and the cardiovascular and cerebrovascular conditions, such correlation was predicted also for one or more variants according to the present invention, as described below. Each variant marker of the present invention described herein as potential marker for cardiovascular conditions, might optionally be used alone or in combination with one or more other variant markers described herein, and or in combination with known markers for cardiovascular conditions, including but not limited to Heart-type fatty acid binding protein (H-FABP), Angiotensin, C-reactive protein (CRP), myeloperoxidase (MPO), and/or in combination with the known protein(s) for the variant marker as described herein. Each variant marker of the present invention described herein as potential marker for cerebrovascular conditions, might optionally be used alone or in combination with one or more other variant markers described herein, and or in combination with known markers for cerebrovascular conditions, including but not limited to CRP, S100b, BNGF, CD40, MCP1, N-Acetyl-Aspartate (NAA), N-methyl-d-aspartate (NMDA) receptor antibodies (NR2Ab), and/or in combination with the known protein(s) for the variant marker as described herein.

Myocardial infarction

C03950 variants, R15601 variants and/or T11811 variants are potential markers for myocardial infarction. Other conditions that may be diagnosed by these markers or variants of them include but are not limited to the presence, risk and/or extent of the following:

1. Myocarditis - in myocarditis cardiac muscle cells can go through cell lysis and leakage with the release of intracellular content to the extracellular space and blood, a similar process as happens in myocardial infarction (see also extended description below).
2. Angina - stable or unstable, as the reduction of oxygen delivery to part of the heart often leads to local ischemic conditions that facilitate leakage of intracellular content.
3. Traumatic injury to myocardial tissue - blunt or penetrating, may also result in myocardial cell leakage.
4. Opening an occluded coronary artery following thrombolytic therapy - If such treatment is successful, proteins and other products of the local tissue are washed into the blood and can be detected there.
5. Cardiomyopathy - which is characterized by slow degeneration of the heart muscle (see also extended description below).

6. Myocardial injury after rejection of heart transplant.

7. Congestive heart failure where heart myocytes slowly degenerate (as had been shown for Troponin-I; see also extended description below).

8. Future cardiovascular disease (as a risk factor).

9. Conditions which have similar clinical symptoms as myocardial infarction and where the differential diagnosis between them and myocardial infarction is of clinical importance including but not limited to:

a. Clinical symptoms resulting from lung related tissue (e.g. Pleuritis, pulmonary embolism)

b. Musculoskeletal origin of pain

c. Clinical symptoms resulting from heart related tissue which are not due to myocardial infarction, e.g. acute pericarditis

d. Upper abdominal pain from abdominal organs including but nor limited to esophagitis, gastro-esophageal reflux, gastritis, gastric ulcer, duodenitis, duodenal ulcer, enteritis, gastroenteritis, cholecystitis, cholelithiasis, cholangiolithiasis, pancreatitis, splenic infarction, splenic trauma, Aortic dissection.

One or more of these markers (variants according to the present invention) may optionally be used a tool to decide on treatment options e.g. anti platelet inhibitors (as has been shown for Troponin-I); as a tool in the assessment of pericardial effusion; and/or as a tool in the assessment of endocarditis and/or rheumatic fever, where progressive damage to the heart muscle may occur.

Cardiomyopathy and myocarditis

Cardiomyopathy may be treated with the polynucleotides/polypeptides and/or methods of this invention. Cardiomyopathy is a general diagnostic term designating primary myocardial disease which may progress to heart failure. The disease comprises inflammatory cardiomyopathies, cardiomyopathies resulting from a metabolic disorder such as a nutritional deficiency or by altered endocrine function, exposure to toxic substances, for example from alcohol or exposure to cobalt or lead, infiltration and deposition of abnormal. In some embodiments, the marker(s) for diagnosis of cardiomyopathy and myocarditis, and related conditions as described herein, may optionally be selected from the group consisting of C03950 variants, R15601 variants and/or T11811 variants

Congestive Heart Failure (CHF)

C03950 variants, R15601 variants and/or T11811 variants are potential markers for, and may be used to treat, etc., CHF.

The invention provides a means for the identification/prognostication, etc., of a number of conditions including the assessment of the presence, risk and/or extent of the following:

1. A risk factor for sudden cardiac death, from arrhythmia or any other heart related reason.
2. Rejection of a transplanted heart.
3. Conditions that lead to heart failure including but not limited to myocardial infarction, angina, arrhythmias, valvular diseases, atrial and/or ventricular septal defects.
4. Conditions that cause atrial and or ventricular wall volume overload. Wall stretch results in enhanced secretion of cardiac extracellular regulators. Such conditions include but are not limited to systemic arterial hypertension, pulmonary hypertension and pulmonary embolism.
5. Conditions which have similar clinical symptoms as heart failure and as states that cause atrial and or ventricular pressure-overload, where the differential diagnosis between these conditions to the latter is of clinical importance including but not limited to breathing difficulty and/or hypoxia due to pulmonary disease, anemia or anxiety.

Cancerous conditions

Various non-limiting examples are given below of cancerous conditions for which one or more variants according to the present invention may have a diagnostic, or therapeutic utility.

Ovarian cancer

Ovarian cancer causes more deaths than any other cancer of the female reproductive system, however, only 25% of ovarian cancers are detected in stage I. No single marker has been shown to be sufficiently sensitive or specific to contribute to the diagnosis of ovarian cancer.

In one embodiment, the markers of this invention are utilized alone, or in combination with other markers, for the diagnosis, treatment or assessment of prognosis of ovarian cancer. Such other markers may comprise CA-125 or mucin 16, CA-50, CA 54-61, CA-195 and CA 19-9, STN and TAG-72, kallikreins, cathepsin L, urine gonadotropin, inhibins, cytokeratins, such as TPA and TPS, members of the Transforming Growth Factors (TGF) beta superfamily, Epidermal Growth Factor, p53 and HER-2 or any combination thereof.

Immunohistochemistry may be used to assess the origin of the tumor and staging as part of the methods of this invention, and as protected uses for the polypeptides of this invention.

In some embodiments, this invention provides polypeptides/polynucleotides which serves as markers for ovarian cancer. In some embodiments, the marker is any polypeptide/polynucleotide as described herein. In some embodiments, the marker is D12115, or variants as described herein or markers related thereto. Each variant marker of the present invention described herein may be used alone or in combination with one or more other variant ovarian cancer described herein, and/or in combination with known markers for ovarian cancer, as described herein. Diagnosis of ovarian cancer and/or of other conditions that may be diagnosed by these markers or variants of them, include but are not limited to the presence, risk and/or extent of the following:

1. The identification of a metastasis of unknown origin which originated from a primary ovarian cancer.
2. As a marker to distinguish between different types of ovarian cancer, therefore potentially affect treatment choice (e.g. discrimination between epithelial tumors and germ cell tumors).
3. As a tool in the assessment of abdominal mass and in particular in the differential diagnosis between a benign and malignant ovarian cysts.
4. As a tool for the assessment of infertility.
5. Other conditions that may elevate serum levels of ovary related markers. These include but are not limited to: cancers of the endometrium, cervix, fallopian tubes, pancreas, breast, lung and colon; nonmalignant conditions such as pregnancy, endometriosis, pelvic inflammatory disease and uterine fibroids.
6. Conditions which have similar symptoms, signs and complications as ovarian cancer and where the differential diagnosis between them and ovarian cancer is of clinical importance including but not limited to:
    a. Non-malignant causes of pelvic mass. Including, but not limited to: benign (functional) ovarian cyst, uterine fibroids, endometriosis, benign ovarian neoplasms and inflammatory bowel lesions
    b. Any condition suggestive of a malignant tumor including but not limited to anorexia, cachexia, weight loss, fever, hypercalcemia, skeletal or abdominal pain, paraneoplastic syndrome.
    c. Ascites.
7. Prediction of patient's drug response
8. As surrogate markers for clinical outcome of a treated cancer.
9. Screening for early detection of ovarian cancer.

Breast cancer

Breast cancer is the most commonly occurring cancer in women, comprising almost a third of all malignancies in females. In one embodiment, the polypeptides and/or polynucleotides of this invention are utilized alone, or in combination with other markers, for the diagnosis, treatment or assessment of prognosis of breast cancer. In one embodiment, the polypeptides and/or polynucleotides serve as markers of disease.

Such markers may be used alone, or in combination with other known markers for breast cancer, including, inter alia, Mucin1 (measured as CA 15-3), CEA (CarcinoEmbryonic Antigen), HER-2, CA125, CA 19-9, PCNA, Ki-67, E-Cadherin, Cathepsin D, TFF1, epidermal growth factor receptor (EGFR), cyclin E, p53, bcl-2, vascular endothelial growth factor, urokinase-type plasminogen activator-1, survivin, or any combination thereof, and includes use of any compound which detects or quantifies the same. ESR (Erythrocyte Sedimentation Rate) values may be obtained, and comprise the marker panel for breast cancer.

In some embodiments, the polypeptides/polynucleotides of this invention serve as prognosticators, in identifying, inter alia, patients at minimal risk of relapse, patients with a worse prognosis, or patients likely to benefit from specific treatments.

There are some non-cancerous pathological conditions which represent an increased risk factor for development breast cancer, and as such, patients with these conditions may be evaluated using the polypeptides/polynucleotides and according to the methods of this invention, for example, as part of the screening methods of this invention, Some of these conditions include, but are not limited to ductal hyperplasia without atypia, atypical hyperplasia, and others.

In some embodiments, the polypeptides/polynucleotides of this invention serve as markers for breast cancer, including, but not limited to: D12115 or homologues thereof. In some embodiments, the D12115 or polynucleotides encoding the same, can be used alone or in combination with any other desired marker, including, inter alia, Calcitonin, CA15-3 (Mucin1), CA27-29, TPA, a combination of CA 15-3 and CEA, CA 27.29 (monoclonal antibody directed against MUC1), Estrogen 2 (beta), HER-2 (c-erbB2), or any combinations thereof.

In some embodiments, the polypeptides/polynucleotides of this invention may be useful in, inter alia, assessing the presence, risk and/or extent of the following:

1. The identification of a metastasis of unknown origin which originated from a primary breast cancer tumor.
2. In the assessment of lymphadenopathy, and in particular axillary lymphadenopathy.
3. As a marker to distinguish between different types of breast cancer, therefore potentially affect treatment choice (e.g. as HER-2)
4. As a tool in the assessment of palpable breast mass and in particular in the differential diagnosis between a benign and malignant breast mass.
5. As a tool in the assessment of conditions affecting breast skin (e.g. Paget's disease) and their differentiation from breast cancer.
6. As a tool in the assessment of breast pain or discomfort resulting from either breast cancer or other possible conditions (e.g. Mastitis, Mondors syndrome).
7. Other conditions not mentioned above which have similar symptoms, signs and complications as breast cancer and where the differential diagnosis between them and breast cancer is of clinical importance including but not limited to:

    a. Abnormal mammogram and/or nipple retraction and/or nipple discharge due to causes other than breast cancer. Such causes include but are not limited to benign breast masses, melanoma, trauma and technical and/or anatomical variations.
    b. Any condition suggestive of a malignant tumor including but not limited to anorexia, cachexia, weight loss, fever, hypercalcemia, paraneoplastic syndrome.

Lymphadenopathy, weight loss and other signs and symptoms associated with breast cancer but originate from diseases different from breast cancer including but not limited to other malignancies, infections and autoimmune diseases.

8. Prediction of patient's drug response
9. As surrogate markers for clinical outcome of a treated cancer.
10. Screening for early detection of breast cancer.

Lung cancer

Lung cancer is the primary cause of cancer death among both men and women in the U. S. In one embodiment, the polypeptides and/or polynucleotides of this invention are utilized alone, or in combination with other markers, for the diagnosis, treatment or assessment of prognosis of lung cancer. In one embodiment, the term "lung cancer" is to be understood as encompassing small cell or non-small cell lung cancers, including adenocarcinomas, bronchoalveolar-alveolar, squamous cell and large cell carcinomas.

In some embodiments, the polypeptides/polynucleotides of this invention are utilized in conjunction with other screening procedures, as well as the use of other markers, for the diagnosis, or assessment of prognosis of lung cancer in a subject. In some embodiments, such screening procedures may comprise the use of chest x-rays, analysis of the type of cells contained in sputum, fiberoptic examination of the bronchial passages, or any combination thereof. Such evaluation in turn may impact the type of treatment regimen pursued, which in turn may reflect the type and stage of the cancer, and include surgery, radiation therapy and/or chemotherapy.

Current radiotherapeutic agents, chemotherapeutic agents and biological toxins are potent cytotoxins, yet do not discriminate between normal and malignant cells, producing adverse effects and dose-limiting toxicities. In some embodiments of this invention, the polypeptides/polynucleotides provide a means for more specific targeting to neoplastic versus normal cells.

In some embodiments, the polypeptides for use in the diagnosis, treatment and/or assessment of progression of lung cancer may comprise: D12115, N43992 or homologous thereof, or polynucleotides encoding the same. In some embodiments, these polypeptides/polynucleotides may be used alone or in combination with one or more other appropriate markers, including, inter alia, other polypeptides/polynucleotides of this invention. In some embodiments, such use may be in combination with other known markers for lung cancer, including but not limited to CEA, CA15-3, Beta-2-microglobulin, CA19-9, TPA, and/or in combination with native sequences associated with the polypeptides/polynucleotides of this invention, as herein described..

In some embodiments, the polypeptides/polynucleotides of this invention may be useful in, inter alia, assessing the presence, risk and/or extent of the following:

1. The identification of a metastasis of unknown origin which originated from a primary lung cancer.
2. The assessment of a malignant tissue residing in the lung that is from a non-lung origin, including but not limited to: osteogenic and soft tissue sarcomas; colorectal, uterine, cervix and corpus tumors; head and neck, breast, testis and salivary gland cancers; melanoma; and bladder and kidney tumors.
3. Distinguishing between different types of lung cancer, therefore potentially affect treatment choice (e.g. small cell vs. non small cell tumors).
4. Unexplained dyspnea and/or chronic cough and/or hemoptysis, and analysis thereof.
5. Differential diagnosis of the origin of a pleural effusion.
6. Conditions which have similar symptoms, signs and complications as lung cancer and where the differential diagnosis between them and lung cancer is of clinical importance including but not limited to:

    a. Non-malignant causes of lung symptoms and signs. Symptoms and signs include, but are not limited to: lung lesions and infiltrates, wheeze, stridor.
    b. Other symptoms, signs and complications suggestive of lung cancer, such as tracheal obstruction, esophageal compression, dysphagia, recurrent laryngeal nerve paralysis, hoarseness, phrenic nerve paralysis with elevation of the hemidiaphragm and Horner syndrome.
    c. Any condition suggestive of a malignant tumor including but not limited to anorexia, cachexia, weight loss, fever, hypercalcemia, hypophosphatemia, hyponatremia, syndrome of inappropriate secretion of antidiuretic hormone, elevated ANP, elevated ACTH, hypokalemia, clubbing, neurologic-myopathic syndromes and thrombophlebitis.

7. Prediction of patient's drug response
8. As surrogate markers for clinical outcome of a treated cancer.
9. Screening for early detection of lung cancer.

**CANDIDATE MARKER EXAMPLES SECTION**

This section relates to examples of sequences according to the present invention, including illustrative methods of selection thereof.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

The markers of the present invention were tested with regard to their expression in various cancerous and non-cancerous tissue samples. Unless otherwise noted, all experimental data relates to variants of the present invention, named according to the segment being tested (as expression was tested through RT-PCR as described). A description of the samples used in the ovarian cancer testing panel is provided in Table 1_1 below. A description of the samples used in the lung cancer testing panel is provided in Table 1_2 or Table 1_5 below. A description of the samples used in the breast cancer testing panel is provided in Table 1_3 below. A description of the samples used in the colon cancer testing panel is provided in Table 1_4 below. The key for the table 1_5is listed in tables 1_5_1 below. A description of the samples used in the normal tissue panel is provided in Table 1_6 and 1_7 below. A description of samples used for microarray analysis is provided in Table 1_8 for breast panel and 1_9 for ovary panel. Tests were then performed as described in the "Materials and Experimental Procedures" section below.

Table 1_1: Tissue samples in ovarian cancer testing panel

| Sample name | Lot number | Source | Pathology | Grade | age |
|---|---|---|---|---|---|
| 33-B-Pap Sero CystAde G1 | A503175 | BioChain | Serous papillary cystadenocarcinoma | 1 | 41 |
| 41-G-Mix Sero/Muc/Endo G2 | 98-03-G803 | GOG | Mixed epithelial cystadenocarcinoma with mucinous, endometrioid, squamous anc papillary serous (Stage2) | 2 | 38 |
| 35-G-Endo Adeno G2 | 94-08-7604 | GOG | Endometrioid adenocarcinoma | 2 | 39 |
| 14-B-Adeno G2 | A501111 | BioChain | Adenocarcinoma | 2 | 41 |
| 12-B-Adeno G3 | A406023 | Biochain | Adenocarcinoma | 3 | 45 |
| 40-G-Mix Sero/Endo G2 | 95-11-G006 | GOG | Papillary serous and endometrioid cystadenocarcinoma (Stage3C) | 2 | 49 |
| 4-A-Pap CystAdeno G2 | ILS-7286 | ABS | Papillary cystadenocarcinoma | 2 | 50 |
| 3-A-Pap Adeno G2 | ILS-1431 | ABS | Papillary adenocarcinoma | 2 | 52 |
| 2-A-Pap Adeno G2 | ILS-1408 | ABS | Papillary adenocarcinoma | 2 | 53 |
| 5-G-Adeno G3 | 99-12-G432 | GOG | Adenocarcinoma (Stage3C) | 3 | 46 |
| 11-B-Adeno G3 | A407068 | Biochain | Adenocarcinoma | 3 | 49 |
| 39--G-Mix Sero/Endo G3 | 2001-12-G037 | GOG | Mixed serous and endometrioid adenocarcinoma | 3 | 49 |
| 29-G-Sero Adeno G3 | 2001-12-G035 | GOG | Serous adenocarcinoma (Stage3A) | 3 | 50 |
| 70-G-Pap Sero Adeno G3 | 95-08-G069 | GOG | Papillary serous adenocarcinoma | 3 | 50 |
| 6-A-Adeno G3 | A0106 | ABS | adenocarcinoma | 3 | 51 |
| 31-B-Pap Sero CystAde G3 | A503176 | BioChain | Serous papillary cystadenocarcinoma | 3 | 52 |
| 25-A-Pap Sero Adeno G3 | N0021 | ABS | Papillary serous adenocarcinoma (StageT3CNlMX) | 3 | 55 |
| 37-G-Mix Sero/Endo G3 | 2002-05-G513 | GOG | Mixed serous and endometrioid adenocarcinoma | 3 | 56 |
| 7-A-Adeno G3 | IND-00375 | ABS | adenocarcinoma | 3 | 59 |
| 8-B-Adeno G3 | A501113 | BioChain | adenocarcinoma | 3 | 60 |
| 10-B-Adeno G3 | A407069 | Biochain | Adenocarcinoma | 3 | 60 |
| 38-G-Mix Sero/Endo G3 | 2002-05-G509 | GOG | Mixed serous and endometrioid adenocarcinoma of mullerian (Stage3C) | 3 | 64 |
| 13-G-Adeno G3 | 94-05-7603 | GOG | Poorly differentiated adenocarcinoma from primary peritoneal | 3 | 67 |
| 24-G- Pap Sero Adeno G3 | 2001-07-G801 | GOG | Papillary serous adenocarcinoma | 3 | 68 |
| 34-G-Pap Endo Adeno G3 | 95-04-2002 | GOG | Papillary endometrioid adenocarcinoma (Stage3C) | 3 | 68 |
| 30-G-Pap Sero Adeno G3 | 2001-08-G011 | GOG | Papillary serous carcinoma (Stage1C) | 3 | 72 |
| 1-A-Pap Adeno G3 | ILS-1406 | ABS | Papillary adenocarcinoma | 3 | 73 |
| 9-G-Adeno G3 | 99-06-G901 | GOG | Adenocarcinoma (maybe serous) | 3 | 84 |
| 32-G-Pap Sero CystAde G3 | 93-09-4901 | GOG | Serous papillary cystadenocarcinoma | 3 | 67 |
| 66-G-Pap Sero Adeno G3 SIV | 2000-01-G413 | GOG | Papillary serous carcinoma (metastasis of primary peritoneum) (Stage4) | 3 | 67 |
| 19- B-Muc Adeno G3 | A504085 | BioChain | Mucinous adenocarcinoma | 3 | 34 |

(continued)

| Sample name | Lot number | Source | Pathology | Grade | age |
|---|---|---|---|---|---|
| 21-G- Muc CystAde G2-3 | 95-10-G020 | GOG | Mucinous cystadenocarcinoma (Stage2) | 2-3 | 44 |
| 18-B-Muc Adeno G3 | A504083 | BioChain | Mucinous adenocarcinoma | 3 | 45 |
| 20- A-Pap Muc CystAde | USA-00273 | ABS | Papillary mucinous cystadenocarcinoma | | 46 |
| 17-B-Muc Adeno G3 | A504084 | BioChain | Mucinous adenocarcinoma | 3 | 51 |
| 22-A-Muc CystAde G2 | A0139 | ABS | Mucinous cystadenocarcinoma (Stage1C) | 2 | 72 |
| 43-G-Clear cell Adeno G3 | 2001-10-G002 | GOG | Clear cell adenocarcinoma | 3 | 74 |
| 44-G-Clear cell Adeno | 2001-07-G084 | GOG | Clear cell adenocarcinoma (Stage3A) | | 73 |
| 15-B-Adeno G3 | A407065 | BioChain | Carcinoma | 3 | 27 |
| 16-Ct-Adeno | 1090387 | Clontech | Carcinoma NOS | NA | 58 |
| 23-A-Muc CystAde G3 | VNM-00187 | ABS | Mucinous cystadenocarcinoma with low malignant | 3 | 45 |
| 42-G-Adeno borderline | 98-08-G001 | GOG | Epithelial adenocarcinoma of borderline malignancy | | 46 |
| 63-G-Sero CysAdenoFibroma | 2000-10-G620 | GOG | Serous CysAdenoFibroma of borderline malignancy | | 71 |
| 62-G-Ben Muc CysAdenoma | 99-10-G442 | GOG | Benbin mucinus cysadenoma | | 32 |
| 60-G- Muc CysAdenoma | 99-01-G043 | GOG | Mucinous Cysadenoma | | 40 |
| 56-G-Ben Muc CysAdeno | 99-01-G407 | GOG | Bengin mucinus cysadenoma | | 46 |
| 64-G-Ben Sero CysAdenoma | 99-06-G039 | GOG | Serous Bengin CysAdenoma | | 57 |
| 61-G- Muc CysAdenoma | 99-07-G011 | GOG | Mucinous Cysadenoma | | 63 |
| 59-G-Sero CysAdenoFibroma | 98-12-G401 | GOG | Serous CysAdenoFibroma | | 77 |
| 51-G-N M41 | 98-03-G803N | GOG | Normal (matched tumor 98-03-G803) | | 38 |
| 75-G-N M60 | 99-01-G043N | GOG | Normal (matched tumor 99-01-G043) | | 40 |
| 49-B-N M14 | A501112 | BioChain | Normal (matched tumor A501111) | | 41 |
| 52-G-N M42 | 98-08-G001N | GOG | Normal (matched tumor 98-08-G001) | | 46 |
| 68-G-N M56 | 99-01-G407N | GOG | Normal (matched bengin 99-01-G407) | | 46 |
| 50-B-N M8 | A501114 | BioChain | Normal (matched tumor A501113) | | 60 |
| 67-G-N M38 | 2002-05-509N | GOG | Normal (matched tumor 2002-05-G509) | | 64 |
| 69-G-N M24 | 2001-07-G801N | GOG | Normal (matched tumor 2001-07-G801) | | 68 |
| 73-G-N M59 | 98-12-G401 N | GOG | Normal (matched tumor 98-12-G401) | | 77 |
| 72-G-N M66 | 2000-01-G413N | GOG | Normal (matched tumor 2000-01-G413) | | |
| 45-B-N | A503274 | BioChain | Normal PM | | 41 |
| 46-B-N | A504086 | BioChain | Normal PM | | 41 |
| 71-CG-N | CG-188-7 | Ichilov | Normal PM | | 49 |
| 48-B-N | A504087 | BioChain | Normal PM | | 51 |

Table 1 2: Tissue samples in lung cancer testing panel

| sample name | Lot No. | source | pathology | Grade | gender/age |
|---|---|---|---|---|---|
| 1-B-Adeno G1 | A504117 | Biochain | Adenocarcinoma | 1 | F/29 |
| 2-B-Adeno G1 | A504118 | Biochain | Adenocarcinoma | 1 | M/64 |
| 95-B-Adeno G1 | A610063 | Biochain | Adenocarcinoma | 1 | F/54 |
| 12-B-Adeno G2 | A504119 | Biochain | Adenocarcinoma | 2 | F/74 |
| 75-B-Adeno G2 | A609217 | Biochain | Adenocarcinoma | 2 | M/65 |
| 77-B-Adeno G2 | A608301 | Biochain | Adenocarcinoma | 2 | M/44 |
| 13-B-Adeno G2-3 | A504116 | Biochain | Adenocarcinoma | 2-3 | M/64 |
| 89-B-Adeno G2-3 | A609077 | Biochain | Adenocarcinoma | 2-3 | M/62 |
| 76-B-Adeno G3 | A609218 | Biochain | Adenocarcinoma | 3 | M/57 |
| 94-B-Adeno G3 | A610118 | Biochain | Adenocarcinoma | 3 | M/68 |
| 3-CG-Adeno | CG-200 | Ichilov | Adenocarcinoma | | NA |
| 14-CG- Adeno | CG-111 | Ichilov | Adenocarcinoma | | M/68 |
| 15-CG-Bronch adeno | CG-244 | Ichilov | Bronchioloalveolar adenocarcinoma | | M/74 |
| 45-B-Alvelous Adeno | A501221 | Biochain | Alveolus carcinoma | | F/50 |
| 44-B-Alvelous Adeno G2 | A501123 | Biochain | Alveolus carcinoma | 2 | F/61 |
| 19-B-Squamous G1 | A408175 | Biochain | Squamous carcinoma | 1 | M/78 |
| 16-B-Squamous G2 | A409091 | Biochain | Squamous carcinoma | 2 | F/68 |
| 17-B-Squamous G2 | A503183 | Biochain | Squamous carcinoma | 2 | M/57 |
| 21-B-Squamous G2 | A503187 | Biochain | Squamous carcinoma | 2 | M/52 |
| 78-B-Squamous G2 | A607125 | Biochain | Squamous Cell Carcinoma | 2 | M/62 |
| 80-B-Squamous G2 | A609163 | Biochain | Squamous Cell Carcinoma | 2 | M/74 |
| 18-B-Squamous G2-3 | A503387 | Biochain | Squamous Cell Carcinoma | 2-3 | M/63 |
| 81-B-Squamous G3 | A609076 | Biochain | Squamous Carcinoma | 3 | m/53 |
| 79-B-Squamous G3 | A609018 | Biochain | Squamous Cell Carcinoma | 3 | M/67 |
| 20-B-Squamous | A501121 | Biochain | Squamous Carcinoma | | M/64 |
| 22-B-Squamous | A503386 | Biochain | Squamous Carcinoma | | M/48 |
| 88-B-Squamous | A609219 | Biochain | Squamous Cell Carcinoma | | M/64 |
| 100-B-Squamous | A409017 | Biochain | Squamous Carcinoma | | M/64 |
| 23-CG-Squamous | CG-109 (1) | Ichilov | Squamous Carcinoma | | M/65 |
| 24-CG-Squamous | CG-123 | Ichilov | Squamous Carcinoma | | M/76 |
| 25-CG-Squamous | CG-204 | Ichilov | Squamous Carcinoma | | M/72 |
| 87-B-Large cell G3 | A609165 | Biochain | Large Cell Carcinoma | 3 | F/47 |
| 38-B-Large cell | A504113 | Biochain | Large cell | | M/58 |
| 39-B-Large cell | A504114 | Biochain | Large cell | | F/3 5 |
| 82-B-Large cell | A609170 | Biochain | Large Cell Neuroendocrine Carcinoma | | M/68 |
| 30-B-Small cell carci G3 | A501389 | Biochain | small cell | 3 | M/34 |

(continued)

| sample name | Lot No. | source | pathology | Grade | gender/age |
|---|---|---|---|---|---|
| 31-B-Small cell carci G3 | A501390 | Biochain | small cell | 3 | F/59 |
| 32-B-Small cell carci G3 | A501391 | Biochain | small cell | 3 | M/30 |
| 33-B-Small cell carci G3 | A504115 | Biochain | small cell | 3 | M |
| 86-B-Small cell carci G3 | A608032 | Biochain | Small Cell Carcinoma | 3 | F/52 |
| 83-B-Small cell carci | A609162 | Biochain | Small Cell Carcinoma | | F/47 |
| 84-B-Small cell carci | A609167 | Biochain | Small Cell Carcinoma | | F/59 |
| 85-B-Small cell carci | A609169 | Biochain | Small Cell Carcinoma | | M/66 |
| 46-B-N M44 | A501124 | Biochain | Normal M44 | | F/61 |
| 47-B-N | A503205 | Biochain | Normal PM | | M/26 |
| 48-B-N | A503206 | Biochain | Normal PM | | M/44 |
| 49-B-N | A503384 | Biochain | Normal PM | | M/27 |
| 50-B-N | A503385 | Biochain | Normal PM | | M/28 |
| 90-B-N | A608152 | Biochain | Normal (Pool 2) PM | | pool 2 |
| 91-B-N | A607257 | Biochain | Normal (Pool 2) PM | | pool 2 |
| 92-B-N | A503204 | Biochain | Normal PM | | m/28 |
| 93-Am-N | 111P0103A | Ambion | Normal PM | | F/61 |
| 96-Am-N | 36853 | Ambion | Normal PM | | F/43 |
| 97-Am-N | 36854 | Ambion | Normal PM | | M/46 |
| 98-Am-N | 36855 | Ambion | Normal PM | | F/72 |
| 99-Am-N | 36856 | Ambion | Normal PM | | M/31 |

Table 1 3: Tissue samples in breast cancer testing panel

| sample name | Lot no | source | pathology | grade | age | TNM | stage |
|---|---|---|---|---|---|---|---|
| 14-A-IDC G2 | A0135T | ABS | IDC | 2 | 37 | T2N2Mx | |
| 43-B-IDC G2 | A609183 | Biochain | IDC | 2 | 40 | | |
| 54-B-IDC G2 | A605353 | Biochain | IDC | 2 | 41 | | |
| 55-B-IDC G2 | A609179 | Biochain | IDC | 2 | 42 | | |
| 47-B-IDC G2 | A609221 | Biochain | IDC | 2 | 42 | | |
| 17-A-IDC G2 | 4904020036T | ABS | IDC | 2-3 | 42 | T3N1Mx | |
| 42-A-IDC G3 | 6005020031T | ABS | IDC | 3 | 42 | T1cN0Mx | |
| 7-A-IDC G2 | 7263T | ABS | IDC | 2 | 43 | T1N0M0 | stage 1 |
| 48-B-IDC G2 | A609222 | Biochain | IDC | 2 | 44 | | |
| 53-B-IDC G2 | A605151 | Biochain | IDC | 2 | 44 | | |
| 12-A-IDC G2 | 1432T | ABS | IDC | 2 | 46 | T2N0M0 | stage 2A |
| 61-B-IDC G2 | A610029 | Biochain | IDC | 2 | 46 | | |
| 46-B-Carci G2 | A609177 | Biochain | Carcinoma | 2 | 48 | | |
| 16-A-IDC G2 | 4904020032T | ABS | IDC | 2 | 49 | T3N1Mx | |
| 62-B-IDC G2 | A609194 | Biochain | IDC | 2 | 51 | | |

(continued)

| sample name | Lot no | source | pathology | grade | age | TNM | stage |
|---|---|---|---|---|---|---|---|
| 49-B-IDC G2 | A609223 | Biochain | IDC | 2 | 54 | | |
| 32-A-Muc Carci | 7116T | ABS | Mucinous carcinoma | | 54 | T2N0M0 | stage 2A |
| 45-B-IDC G2 | A609181 | Biochain | IDC | 2 | 58 | | |
| 15-A-IDC G2 | 7259T | ABS | IDC | 2 | 59 | T3N1M0 | stage 3A |
| 52-B-ILC G1 | A605360 | Biochain | Invasive Lobular Carcinoma | 1 | 60 | | |
| 6-A-IDC G1 | 7238T | ABS | IDC | 1 | 60 | T2N0M0 | stage 2A |
| 26-A-IDC G3 | 7249T | ABS | IDC | 3 | 60 | T2N0M0 | stage 2A |
| 13-A-IDC G2 | A0133T | ABS | IDC | 2 | 63 | T2N1aMx | |
| 50-B-IDC G2 | A609224 | Biochain | IDC | 2 | 69 | | |
| 44-B-IDC G2 | A609198 | Biochain | IDC | 2 | 77 | | |
| 51-B-IDC G1 | A605361 | Biochain | IDC | 1 | 79 | | |
| 31-CG-IDC | CG-154 | Ichilov | IDC | | 83 | | |
| 27-A-IDC G3 | 4907020072T | ABS | IDC | 3 | 91 | T2N0Mx | |
| 36-A-N M7 | 7263N | ABS | Normal matched to 7T | | 43 | | |
| 40-A-N M 12 | 1432N | ABS | Normal matched to 12T | | 46 | | |
| 39-A-N M15 | 7259N | ABS | Normal matched to 15T | | 59 | | |
| 35-A-N M6 | 7238N | ABS | Normal matched to 6T | | 60 | | |
| 41-A-N M26 | 7249N | ABS | Normal matched to 26T | | 60 | | |
| 57-B-N | A609233 | Biochain | Normal PM | | 34 | | |
| 59-B-N | A607155 | Biochain | Normal PM | | 35 | | |
| 60-B-N | A609234 | Biochain | Normal PM | | 36 | | |
| 63-Am-N | 26486 | Ambion | Normal PS | | 43 | | |
| 66-Am-N | 36678 | Ambion | Normal PM | | 45 | | |
| 64-Am-N | 23036 | Ambion | Normal PM | | 57 | | |
| 56-B-N | A609235 | Biochain | Normal PM | | 59 | | |
| 65-Am-N | 31410 | Ambion | Normal PM | | 63 | | |
| 67-Am-N | 073P010602086A | Ambion | Normal PM | | 64 | | |
| 58-B-N | A609232 | Biochain | Normal PM | | 65 | | |

Table 1 4: Tissue samples in colon cancer testing panel

| sample name | Lot No. | tissue | source | pathology | Grade | gender/age |
|---|---|---|---|---|---|---|
| 58-B-Adeno G1 | A609152 | Colon | biochain | Adenocarcinoma | 1 | M/73 |

(continued)

| sample name | Lot No. | tissue | source | pathology | Grade | gender/age |
|---|---|---|---|---|---|---|
| 59-B-Adeno G1 | A609059 | Colon | biochain | Adenocarcinoma, Ulcer | 1 | M/58 |
| 14-CG-Polypoid Adeno G1 D-C | CG-222 (2) | Rectum | Ichilov | Well polypoid adeocarcinoma Duke's C | | F/49 |
| 17-CG-Adeno G1-2 | CG-163 | Rectum | Ichilov | Adenocarcinoma | 2 | M/73 |
| 10-CG-Adeno G1-2 D-B2 | CG-311 | Sigmod colon | Ichilov | Adenocarcinoma Astler-Coller B2. | 1-2 | M/88 |
| 11-CG-Adeno G1-2 D-C2 | CG-337 | Colon | Ichilov | Adenocarcinoma Astler-Coller C2. | 1-2 | NA |
| 6-CG-Adeno G1-2 D-C2 | CG-303 (3) | Colon | Ichilov | Adenocarcinoma Astler-Coller C2. | 1-2 | F/77 |
| 5-CG-Adeno G2 | CG-308 | Colon Sigma | Ichilov | Adenocarcinoma. | 2 | F/80 |
| 16-CG-Adeno G2 | CG-278C | colon | Ichilov | Adenocarcinoma | 2 | F/60 |
| 56-B-Adeno G2 | A609148 | Colon | biochain | Adenocarcinoma | 2 | F48 |
| 61-B-Adeno G2 | A606258 | Colon | biochain | Adenocarcinoma, Ulcer | 2 | M/41 |
| 60-B-Adeno G2 | A609058 | Colon | biochain | Adenocarcinoma, Ulcer | 2 | M/67 |
| 22-CG-Adeno G2 D-B | CG-229C | Colon | Ichilov | Adenocarcinoma Duke's B | 2 | F/55 |
| 1-CG-Adeno G2 D-B2 | CG-335 | Cecum | Ichilov | Adenocarcinoma Dukes B2. | 2 | F/66 |
| 12-CG-Adeno G2 D-B2 | CG-340 | Colon Sigma | Ichilov | Adenocarcinoma Astler-Coller B2. | 2 | M/66 |
| 28-CG-Adeno G2 D-B2 | CG-284 | sigma | Ichilov | Adenocarcinoma Duke's B2 | 2 | F/72 |
| 2-CG-Adeno G2 D-C2 | CG-307 X2 | Cecum | Ichilov | Adenocarcinoma Astler-Coller C2. | 2 | F/89 |
| 9-CG-Adeno G2 D-D | CG-297 X2 | Rectum | Ichilov | Adenocarcinoma Dukes D. | 2 | M/62 |
| 13-CG-Adeno G2 D -D | CG-290 X2 | Rectosigmoidal colon | Ichilov | Adenocarcinoma Dukes D. | 2 | M/47 |
| 26-CG-Adeno G2 D-D | CG-283 | sigma | Ichilov | Colonic adenocarcinoma Duke's D | 2 | F/63 |
| 4-CG-Adeno G3 | CG-276 | Colon | Ichilov | Carcinoma. | 3 | M/64 |
| 53-B-Adeno G3 | A609161 | Colon | biochain | Adenocarcinoma | 3 | F/53 |
| 54-B-Adeno G3 | A609142 | Colon | biochain | Adenocarcinoma | 3 | M/53 |
| 55-B-Adeno G3 | A609144 | Colon | biochain | Adenocarcinoma | 3 | M/68 |
| 57-B-Adeno G3 | A609150 | Colon | biochain | Adenocarcinoma | 3 | F/45 |

(continued)

| sample name | Lot No. | tissue | source | pathology | Grade | gender/age |
|---|---|---|---|---|---|---|
| 72-CG-Adeno G3 | CG-309 | colon | Ichilov | Adenocarcinoma | 3 | F/88 |
| 20-CG-Adeno G3 D-B2 | CG-249 | Colon | Ichilov | Ulcerated adenocarcinoma Duke's B2 | 3 | M/36 |
| 7-CG-Adeno D-A | CG-235 | Rectum | Ichilov | Adenocarcinoma intramucosal Duke's A. | | F/66 |
| 23-CG-Adeno D-C | CG-282 | sigma | Ichilov | Mucinus adenocarcinoma Astler Coller C | | M/51 |
| 3-CG-Muc adeno D-D | CG-224 | Colon | Ichilov | Mucinois adenocarcinoma Duke's D | | M/48 |
| 18-CG-Adeno | CG-22C | Colon | Ichilov | Adenocarcinoma | | NA |
| 19-CG-Adeno | CG-19C (1) | Colon | Ichilov | Adenocarcinoma | | NA |
| 21-CG-Adeno | CG-18C | Colon | Ichilov | Adenocarcinoma | | NA |
| 24-CG-Adeno | CG-12 (2) | Colon | Ichilov | Adenocarcinoma | | NA |
| 25-CG-Adeno | CG-2 | Colon | Ichilov | Adenocarcinoma | | NA |
| 27-CG-Adeno | CG-4 | Colon | Ichilov | Adenocarcinoma | | NA |
| 8-CG-diverticolosis, diverticulitis | CG-291 | Wall of sigma | Ichilov | Diverticolosis and diverticulitis of the Colon | | F/65 |
| 46-CG-Crohn's disease | CG-338C | Cecum | Ichilov | Crohn's disease | | M/22 |
| 47-CG-Crohn's disease | CG-338AC | Colon | Ichilov | Crohn's disease. | | M/22 |
| 42-CG-N M20 | CG-249N | Colon | Ichilov | Normal | | M/36 |
| 43-CG-N M8 | CG-291N | Wall of sigma | Ichilov | Normal | | F/65 |
| 44-CG-N M21 | CG-18N | Colon | Ichilov | Normal | | NA |
| 45-CG-N M11 | CG-337N | Colon | Ichilov | Normal | | M/75 |
| 49-CG-N M14 | CG-222N | Rectum | Ichilov | Normal | | F/49 |
| 50-CG-N M5 | CG-308N | Sigma | Ichilov | Within normal limits | | F/80 |
| 51-CG-N M26 | CG-283N | Sigma | Ichilov | Normal | | F/63 |
| 41-B-N | A501156 | Colon | biochain | Normal PM | | M/78 |
| 52-CG-N | CG-309TR | Colon | Ichilov | Within normal limits | | F/88 |
| 62-B-N | A608273 | Colon | biochain | Normal PM | | M/66 |
| 63-B-N | A609260 | Colon | biochain | Normal PM | | M/61 |
| 64-B-N | A609261 | Colon | biochain | Normal PM | | F/68 |
| 65-B-N | A607115 | Colon | biochain | Normal PM | | M/24 |
| 66-B-N | A609262 | Colon | biochain | Normal PM | | M/58 |

(continued)

| sample name | Lot No. | tissue | source | pathology | Grade | gender/age |
|---|---|---|---|---|---|---|
| 67-B-N | A406029 | Colon | biochain | Normal PM (Pool of 10) | | |
| 69-B-N | A411078 | Colon | biochain | Normal PM (Pool of 10) | | F&M |
| 70-Cl-N | 1110101 | Colon | clontech | Normal PM (Pool of 3) | | |
| 71-Am-N | 071P10B | Colon | Ambion | Normal (IC BLEED) | | F/34 |

Table 1_5: Tissue samples in lung cancer testing panel

| Tis-sue | Source /Deliv-ery | sample name | sample id (GCI) /case id (Aster-and)/lot no. (old samples) | TISSUE ID (GCI)/ spec-imen ID (Asterand) | RNA ID (GCI)/ Sample ID (Asterand) | Diag | Diag rem-arks | Spec-imen locat-ion | Gr | TNM | CS | Tum % | Gen | age | Eth-nic B | Smo k-ing Status | # Cig. Per day | # of Y. Use of Toba-cco | # Y. off Toba c-co | Sm P Y? | Sm ppl | Dr Al | # Dr | HT (CM) | BMI | Rec-overy Type | Cau-se of Dea-th | Exc. Y. |
|---|---|---|---|---|---|---|---|---|---|---|---|---|---|---|---|---|---|---|---|---|---|---|---|---|---|---|---|---|
| LC | GCI | 1-GC-BAC-SIA | 7Z9V4 | 7Z9V4AYM | | Aden | BC | | | | IA | 80 | F | 63 | WCAU | Prev U. | 20 | 15 | 27 | N | . | Y | 0 | 165 | 25.3 | Surg | | 2001 |
| LC | GCI | 2-GC-BAC-SIB | ZW2AQ | ZW2AQARP | | Aden | BC | | | | IB | 70 | F | 56 | WCAU | Prev U. | 15 | 28 | 10 | Y | 1 | Y | 6 | 165 | 23 | Surg | | 2002 |
| LC | Bioch | 72-(44)-Bc-BAC | A501123 | | | AC | | | 2 | UN | | | F | 61 | | | | | | | | | | | | | | |
| LC | Bioch | 73-(45)-Bc-BAC | A501221 | | | AC | | | UN | UN | | | F | 50 | | | | | | | | | | | | | | |
| LC | GCI | 4-GC-Adeno-SIA | 3MOPL | 3MOPLA79 | | Aden | | | | | IA | 60 | M | 68 | WCAU | Nev U. | . | . | . | N | . | N | . | 175 | 27.3 | Surg | | 2001 |
| LC | GCI | 5-GC-Adeno-SIA | KOJXD | KOJXDAV4 | | Aden | | | | | IA | 90 | F | 64 | WCAU | Prev U. | 15 | 40 | 7 | Y | 1 | N | 0 | 157 | 19.6 | Surg | | 2003 |
| LC | GCI | 6-GC-Adeno-SIA | X2Q44 | X2Q44A79 | | Aden | | | | | IA | 85 | M | 58 | WCAU | Prev U. | 10 | 47 | 0 | Y | 2 | N | . | 170 | 24.6 | Surg | | 2004 |
| LC | GCI | 7-GC-Adeno-SIA | 6BACZ | 6BACZAP5 | | Aden | | | | | IA | 60 | F | 65 | WCAU | Curr U. | 6 | 30 | . | Y | 1 | N | . | 168 | 21 | Surg | | 2004 |
| LC | GCI | 8-GC-Adeno-SIA | BS9AF | BS9AFA3E | | Aden | | | | | IA | 55 | F | 59 | WCAU | Curr U. | 20 | 40 | . | N | . | N | . | 160 | 23.9 | Surg | | 2004 |
| LC | GCI | 9-GC-Adeno-SIA | UCLOA | UCLOAA9L | | Aden | | | | | IA | 80 | F | 69 | WCAU | Curr U. | 30 | 52 | . | Y | 4 | N | . | 157 | 34.8 | Surg | | 2005 |
| LC | GCI | 10-GC-Adeno-SIA | BVYK3 | BVYK3A7Z | | Aden | | | | | IA | 60 | F | 60 | WCAU | Curr U. | 40 | 40 | . | N | . | N | . | 163 | 31.8 | Surg | | 2002 |

| | | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| LC | GCI | 11-GC-Adeno-SIB | U4DM4 | U4DM4AFZ | | Aden | | | | | IB | 65 | F | 68 | WCAU | Prev U. | 5 | 4 | 43 | N | . | N | . | 165 | 22.3 | Surg | | 2003 |
| LC | GCI | 12-GC-Adeno-SIB | OWX5Y | OWX5YA3S | | Aden | | | | | IB | 90 | M | 69 | WCAU | Curr U. | 10 | . | . | | . | N | . | 183 | 30.5 | Surg | | 2002 |
| LC | GCI | 13-GC-Adeno-SIIA | XYY96 | XYY96A6B | | Aden | | | | | IIA | 70 | F | 62 | WCAU | Prev U. | 6 | 40 | 6 | N | . | Y | 0 | 160 | 27 | Surg | | 2004 |
| LC | GCI | 14-GC-Adeno-SIIA | SO7B1 | SO7B1AIJ | | Aden | | | | | IIA | 70 | M | 56 | WCAU | Curr U. | 30 | 25 | . | Y | 1 | N | . | 180 | 36.4 | Surg | | 2001 |
| LC | GCI | 15-GC-Adeno-SIIIA | QANSY | QANSYACD | | Aden | | | | | IIIA | 65 | F | 61 | WCAU | Curr U. | 30 | 36 | . | Y | 1 | N | . | 163 | 25.1 | Surg | | 2004 |
| LC | Bioch | 16-(95)-BC-Adeno | A610063 | | | Aden | | | 1 | UN | | | F | 54 | | | | | | | | | | | | | | |
| LC | Bioch | 17-(89)-Bc-Adeno | A609077 | | | Aden | | | 2-3 | UN | | | M | 62 | | | | | | | | | | | | | | |
| LC | Bioch | 18-(76)-Bc-Adeno | A609218 | | | Aden | | | 3 | UN | | | M | 57 | | | | | | | | | | | | | | |
| LC | Bioch | 74-(2)-Bc-Adeno | A504118 | | | Aden | | | 1 | UN | | | M | 64 | | | | | | | | | | | | | | |
| LC | Bioch | 76-(75)-Bc-Adeno | A609217 | | | Aden | | | 2 | UN | | | M | 65 | | | | | | | | | | | | | | |
| LC | Bioch | 77-(12)-Bc-Adeno | A504119 | | | Aden | | | 2 | UN | | | F | 74 | | | | | | | | | | | | | | |
| LC | Bioch | 78-(13)-Bc-Adeno | A504116 | | | Aden | | | 2-3 | UN | | | M | 64 | | | | | | | | | | | | | | |
| LC | Bioch | 79-(94)-Bc-Adeno | A610118 | | | Aden | | | 3 | UN | | | M | 68 | | | | | | | | | | | | | | |

| | | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| LC | Ichilov | 80-(3)-Ic-Adeno | CG-200 | | | Aden | | | UN | UN | | | F | 56 | | | | | | | | | | | | | | |
| LC | Ichilov | 81-(14)-Ic- Adeno | CG-111 | | | Aden | | | UN | UN | | | M | 68 | | | | | | | | | | | | | | |
| LC | Aster | 19-As-Sq-S0 | 9220 | 9418 | 9418A1 | SCC | | | 1 | TXN0 M0 | Oc cult | 80 | M | 67 | CAU | Curr U. | 11-20 | 31-40 | | | | O | | 163 | 28.6 | Surg | | 2003 |
| LC | GCI | 20-GC-Sq-SIA | U2QHS | U2QHSA2N | | SCC | | | | | IA | 55 | F | 68 | WCAU | Prev U. | 10 | 20 | 0 | N | . | N | . | 157 | 22.9 | Surg | | 2004 |
| LC | GCI | 21-GC-Sq-SIB | TRQR7 | TRQR7ACD | | SCC | | | | | IB | 75 | M | 62 | WCAU | Prev U. | 20 | 50 | 0 | Y | 5 | N | . | 175 | 25.5 | Surg | | 2005 |
| LC | Aster | 22-As-Sq-SIB | 17581 | 32603 | 32603B1 | SCC | | | 3 | T2N0 M0 | IB | 90 | M | 73 | CAU | Prev U. | | | | | | O | | 170 | 22.1 | Surg | | 2004 |
| LC | Aster | 23-As-Sq-SIB | 18309 | 41454 | 41454B1 | SCC | | | 2 | T2N0 MX | IB | 100 | M | 66 | CAU | Prev U. | 11-20 | 45 | | | | P | | 178 | 33.8 | Surg | | 2005 |
| LC | Aster | 24-As-Sq-SIB | 9217 | 9415 | 9415B1 | SCC | | | 2 | T2N0 M0 | IB | 90 | M | 65 | CAU | Curr U. | 6-10 | 41-50 | | | | O | | 176 | 22 | Surg | | 2002 |
| LC | GCI | 25-GC-Sq-SIIB | RXQ1P | RXQ1PAEA | | SCC | | | | | IIB | 55 | F | 44 | WCAU | Prev U. | 20 | 20 | 0 | Y | 2 | N | . | 155 | 22.7 | Surg | | 2004 |
| LC | GCI | 26-GC-Sq-SIIB | KB5KH | KB5KHA6X | | SCC | | | | | IIB | 65 | M | 68 | WCAU | Prev U. | 40 | 40 | 0 | Y | 2 | N | . | 170 | 23.2 | Surg | | 2004 |
| LC | GCI | 27-GC-Sq-SIIIA | LAYMB | LAYMBALF | | SCC | | | | | IIIA | 65 | F | 58 | WCAU | Prev U. | 50 | 40 | 1 | Y | 2 | N | . | 173 | 27.4 | Surg | | 2004 |
| LC | Ichilov | 28-(23)-Ic-Sq | CG-109 (1) | | | SCC | | | UN | UN | | | M | 65 | | | | | | | | | | | | | | |
| LC | Ichilov | 29-(25)-Ic-Sq | CG-204 | | | SCC | | | UN | UN | | | M | 72 | | | | | | | | | | | | | | |
| LC | Bioch | 30-(19)-Bc-Sq | A408175 | | | SCC | | | 1 | UN | | | M | 78 | | | | | | | | | | | | | | |
| LC | Bioch | 31-(78)-Bc-Sq | A607125 | | | SCC | | | 2 | UN | | | M | 62 | | | | | | | | | | | | | | |
| LC | Bioch | 32-(16)-Bc-Sq | A409091 | | | SCC | | | 2 | UN | | | F | 68 | | | | | | | | | | | | | | |
| LC | Bioch | 33-(80)-Bc-Sq | A609163 | | | SCC | | | 2 | UN | | | M | 74 | | | | | | | | | | | | | | |
| LC | Bioch | 34-(18)-Bc-Sq | A503387 | | | SCC | | | 2-3 | UN | | | M | 63 | | | | | | | | | | | | | | |

| | | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| LC | Bioch | 92-(38)-Bc-LCC | A504113 | | | LCC | | | UN | UN | | | M | 58 | | | | | | | | | | | | | | |
| LC | Bioch | 93-(82)-Bc-LCC | A609170 | | | LCNC | | | UN | UN | | | M | 68 | | | | | | | | | | | | | | |
| LC | GCI | 42-GC-SCC-SIB | QPJQL | QPJQLAF 6 | | SMCC | NC | | 3 | | IB | 65 | F | 62 | WCAU | Prev U. | 20 | 35 | 0.15 | Y | 2 | N | . | 165 | 19.8 | Surg | | 2003 |
| LC | Bioch | 43-(32)-Bc-SCC | A501391 | | | SMCC | | | | UN | | | M | 30 | | | | | | | | | | | | | | |
| LC | Bioch | 44-(30)-Bc-SCC | A501389 | | | SMCC | | | 3 | UN | | | M | 34 | | | | | | | | | | | | | | |
| LC | Bioch | 45-(83)-Bc-SCC | A609162 | | | SMCC | | | UN | UN | | | F | 47 | | | | | | | | | | | | | | |
| LC | Bioch | 46-(86)-Bc-SCC | A608032 | | | SMCC | | | 3 | UN | | | F | 52 | | | | | | | | | | | | | | |
| LC | Bioch | 47-(31)-Bc-SCC | A501390 | | | SMCC | | | | UN | | | F | 59 | | | | | | | | | | | | | | |
| LC | Bioch | 48-(84)-Bc-SCC | A609167 | | | SMCC | | | UN | UN | | | F | 59 | | | | | | | | | | | | | | |
| LC | Bioch | 49-(85)-Bc-SCC | A609169 | | | SMCC | | | UN | UN | | | M | 66 | | | | | | | | | | | | | | |
| LC | Bioch | 50-(33)-Bc-SCC | A504115 | | | SMCC | | | | UN | | | M | | | | | | | | | | | | | | | |
| LN | Aster | 51-As-N-PS | 9078 | 9275 | 9275B1 | Norm-L | PS | | | | | | M | 22 | CAU | Nev U. | | | | | | NU | | 0 | 0 | Surg | | 2003 |
| LN | Aster | 52-As-N-PM | 8757 | 8100 | 8100B1 | Norm-L | PM | (Right), Lobe Infer-ior | | | | | F | 26 | CAU | Nev U. | | | | | | O | | 170 | 22.1 | Aut | CA | 2003 |
| LN | Aster | 53-As-N-PM | 6692 | 6161 | 6161A1 | Norm-L | PM | | | | | | M | 37 | CAU | Nev U. | | | | | | C | | 183 | 20.9 | Aut | MCE | 2002 |
| LN | Aster | 54-As-N-PM | 7900 | 7180 | 7180F1 | Norm-L | PM | | | | | | F | 76 | CAU | Prev U. | | | | | | | | 165 | 26.8 | Aut | CPulA | 2002 |
| LN | Aster | 55-As-N-PM | 8771 | 8163 | 8163A1 | Norm-L | PM | (Left), Lobe Super-ior | | | | | M | 81 | CAU | Prev U. | 41 or more | 31-40 | | | | O | | 183 | 30.5 | Aut | CA | 2003 |

| | | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| LC | Bioch | 35-(81)-Bc-Sq | A609076 | | | SCC | | | 3 | UN | | | M | 53 | | | | | | | | | | | | | | |
| LC | Bioch | 82-(21)-Bc-Sq | A503187 | | | SCC | | | 2 | UN | | | M | 52 | | | | | | | | | | | | | | |
| LC | Bioch | 83-(17)-Bc-Sq | A503183 | | | SCC | | | 2 | UN | | | M | 57 | | | | | | | | | | | | | | |
| LC | Bioch | 84-(79)-Bc-Sq | A609018 | | | SCC | | | 3 | UN | | | M | 67 | | | | | | | | | | | | | | |
| LC | Bioch | 85-(22)-Bc-Sq | A503386 | | | SCC | | | UN | UN | | | M | 48 | | | | | | | | | | | | | | |
| LC | Bioch | 86-(20)-Bc-Sq | A501121 | | | SCC | | | UN | UN | | | M | 64 | | | | | | | | | | | | | | |
| LC | Bioch | 87-(88)-Bc-Sq | A609219 | | | SCC | | | UN | UN | | | M | 64 | | | | | | | | | | | | | | |
| LC | Bioch | 88-(100)-Bc-Sq | A409017 | | | SCC | | | UN | UN | | | M | 64 | | | | | | | | | | | | | | |
| LC | Ichilov | 89-(24)-Ic-Sq | CG-123 | | | SCC | | | UN | UN | | | M | 76 | | | | | | | | | | | | | | |
| LC | GCI | 36-GC-LCC-SIA | AF8AL | AF8ALAAL | | LCC | | | | | IA | 85 | M | 45 | WCAU | Prev U. | 45 | 33 | 0 | Y | 2 | Y | 28 | 178 | 31.9 | Surg | | 2004 |
| LC | GCI | 37-GC-LCC-SIB | O62XU | O62XUA1X | | LCC | | | | | IB | 75 | F | 60 | WCAU | Prev U. | 30 | 45 | 0 | Y | 3 | N | . | 160 | 16.8 | Surg | | 2004 |
| LC | GCI | 38-GC-LCC-SIB | OLOIM | OLOIMAS1 | | LCC | | | | | IB | 70 | M | 68 | WCAU | Prev U. | . | 55 | . | Y | . | N | . | 173 | 22.8 | Surg | | 2001 |
| LC | GCI | 39-GC-LCC-SIIB | 1ZWSV | 1ZWSVAB9 | | LCC | | | | | IIB | 50 | M | 51 | WCAU | Prev U. | 20 | 12 | 22 | Y | 1 | N | . | 183 | 26.6 | Surg | | 2004 |
| LC | GCI | 40-GC-LCC-SIIB | 2YHOD | 2YHODA1H | | LCC | NSCC... | | | | IIB | 95 | M | 62 | WCAU | Prev U. | 40 | 40 | 0 | Y | 2 | Y | 12 | 185 | 23.1 | Surg | | 2004 |
| LC | GCI | 41-GC-LCC-SIIB | 38B4D | 38B4DAQK | | LCC | | | | | IIB | 90 | F | 70 | WCAU | Prev U. | 30 | 50 | . | Y | 2 | Y | 13 | 168 | 20.7 | Surg | | 2002 |
| LC | Bioch | 90-(39)-Bc-LCC | A504114 | | | LCC | | | UN | UN | | | F | 35 | | | | | | | | | | | | | | |
| LC | Bioch | 91-(87)-Bc-LCC | A609165 | | | LCC | | | 3 | UN | | | F | 47 | | | | | | | | | | | | | | |

| | | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| LN | Aster | 56-As-N-PM | 13094 | 19763 | 19763A1 | Norm-L | PM | | | | | | M | 0 | CAU | Prev U. | 21-40 | 41-50 | | | | P | | 175 | 25.1 | Aut | IC | |
| LN | Aster | 57-As-N-PM | 19174 | 40654 | 40654A2 | Norm-L | PM | | | | | | F | 69 | CAU | Curr U. | 21-40 | 31-40 | | | | P | | 165 | 22.4 | Aut | CPul A | 2005 |
| LN | Aster | 58-As-N-PM | 13128 | 19642 | 19642A1 | Norm-L | PM | | | | | | F | 75 | CAU | | | | | | | | | 160 | 21.5 | Aut | CPul A | 2004 |
| LN | Aster | 59-As-N-PM | 14374 | 20548 | 20548C1 | Norm-L | PM | (Right), Lobe Super-ior | | | | | F | 75 | CAU | | | | | | | | | 175 | 32.7 | Aut | Cer A | 2004 |
| LN | Amb | 60-(99)-Am-N PM | 36856 | | | N-PM | PM | | | | | | M | 31 | | | | | | | | | | | | | | |
| LN | Amb | 61-(96)-Am-N PM | 36853 | | | N-PM | PM | | | | | | F | 43 | | | | | | | | | | | | | | |
| LN | Amb | 62-(97)-Am-N PM | 36854 | | | N-PM | PM | | | | | | M | 46 | | | | | | | | | | | | | | |
| LN | Amb | 63-(93)-Am-N PM | 111P0103 A | | | N-PM | PM-ICH | | | | | | F | 61 | | | | | | | | | | | | | | |
| LN | Amb | 64-(98)-Am-N PM | 36855 | | | N-PM | PM | | | | | | F | 72 | | | | | | | | | | | | | | |
| LN | Bioch | 67-(50)-Bc-N PM | A503385 | | | N-PM | PM | | | | | | M | 28 | | | | | | | | | | | | | | |
| LN | Bioch | 68-(92)-Bc-N PM | A503204 | | | N-PM | PM | | | | | | M | 28 | | | | | | | | | | | | | | |
| LN | Bioch | 69-(91)-Bc-N PM | A607257 | | | N-P2-PM | PM | | | | | | P2 | 24,29 | | | | | | | | | | | | | | |
| LN | Bioch | 70-(90)-Bc-N PM | A608152 | | | N-P2 PM | PM | | | | | | P2 | 27,28 | | | | | | | | | | | | | | |

| | | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| LN | Bioch | 71-(48)-Bc-N PM | A503206 | | | N-PM | PM | | | | | | M | 44 | | | | | | | | | | | | | | |

Table 1_5_1

| Key | Full Name |
|---|---|
| # Cig. Per day | Number of Cigarettes per day |
| # Dr | Number of Drinks |
| # of Y. Use of Tobacco | Number of Years Using Tobacco |
| # Y. off Tobacco | Number of Years Off Tobacco |
| AC | Alveolus carcinoma |
| Aden | ADENOCARCINOMA |
| Amb | Ambion |
| Aster | Asterand |
| Aut | Autopsy |
| BC | BRONCHIOLOALVEOLAR CARCINOMA |
| Bioch | Biochain |
| C | Current Use |
| CA | Cardiac arrest |
| CAU | Caucasian |
| Cer A | Cerebrovascular accident |
| CPul A | Cardiopulmonary arrest |
| CS | Cancer Stage |
| Curr U. | Current Use |
| Diag | Diagnosis |
| Dr Al | Drink Alcohol? |
| Exc Y. | Excision Year |
| Gen | Gender |
| Gr | Grade |
| Height | HT |
| IC | Ischemic cardiomyopathy |
| LC | Lung Cancer |
| LCC | LARGE CELL CARCINOMA |
| LCNC | Large Cell Neuroendocrine Carcinoma |
| LN | Lung Normal |
| MCE | Massive cerebral edema |
| N | No |
| NC | NEUROENDOCRINE CARCINOMA |
| Nev. U. | Never Used |
| Norm-L | Normal Lung |

(continued)

| Key | Full Name |
|---|---|
| N-P2-PM | Normal (Pool 2)- PM |
| N-PM | Normal-PM |
| NSCC... | NON-SMALL CELL CARCINOMA WITH SARCOMUTOUS TRANSFORMTAIO |
| NU | Never used |
| O | Occasional Use |
| P | Previous Use |
| P2 | Pool 2 |
| Prev U. | Previous Use |
| SCC | Squamous Cell Carcinoma |
| Sm P Y? | Have people at home smoked in past 15 yr |
| Sm ppl | If yes, how many? |
| SMCC | SMALL CELL CARCINOMA |
| SMOKE_GROWING_UP | Did people smoke at home while growing up |
| Surg | Surgical |
| Tum % | Tumor Percentage |
| WCAU | White Caucasian |
| Y | Yes |

Table 1_6: Tissue samples in normal panel:

| | Lot no. | Source | Tissue | Pathology | Sex/Age |
|---|---|---|---|---|---|
| 1-Am-Colon (C71) | 071P10B | Ambion | Colon | PM IC bleed | F/43 |
| 2-B-Colon (C69) | A411078 | Biochain | Colon | PM-Pool of 10 M (26-78)&F(53-77) | |
| 3-Cl-Colon (C70) | 1110101 | Clontech | Colon | PM-Pool of 3 sudden death | M&F (20-50) |
| 4-Am-Small Intestine | 091P0201A | Ambion | Small Intestine | PM ICH | M/85 |
| 5-B-Small Intestine | A501158 | Biochain | Small Intestine | PM | M/63 |
| 6-B-Rectum | A605138 | Biochain | Rectum | PM | M/25 |
| 7-B-Rectum | A610297 | Biochain | Rectum | PM | M/24 |
| 8-B-Rectum | A610298 | Biochain | Rectum | PM | M/27 |
| 9-Am-Stomach | 110P04A | Ambion | Stomach | PM GSW | M/16 |
| 10-B-Stomach | A501159 | Biochain | Stomach | PM | M/24 |
| 11-B-Esophagus | A603814 | Biochain | Esophagus | PM | M/26 |
| 12-B-Esophagus | A603813 | Biochain | Esophagus | PM | M/41 |
| 13-Am-Pancreas | 071P25C | Ambion | Pancreas | PM MVA | F/25 |
| 14-CG-Pancreas | CG-255-2 | Ichilov | Pancreas | PM | M/75 |
| 15-B-Lung | A409363 | Biochain | Lung | PM-Pool of 5 | M(24-28)&F62 |
| 16-Am-Lung (L93) | 111P0103A | Ambion | Lung | PM ICH | F/61 |

(continued)

| | Lot no. | Source | Tissue | Pathology | Sex/Age |
|---|---|---|---|---|---|
| 17-B-Lung (L92) | A503204 | Biochain | Lung | PM | M/28 |
| 19-B-Ovary (O48) | A504087 | Biochain | Ovary | PM | F/51 |
| 20-B-Ovary (O46) | A504086 | Biochain | Ovary | PM | F/41 |
| 75-G-Ovary | L629FRV1 | GCI | Ovary | PS DIGESTIVE HEMORRHAGE (ALCOHOLISM) | F/47 |
| 76-G-Ovary | DWHTZRQX | GCI | Ovary | PS LEIOMYOMAS | F/42 |
| 77-G-Ovary | FDPL9NJ6 | GCI | Ovary | PS VAGINAL BLEEDING | F/56 |
| 78-G-Ovary | GWXUZN5M | GCI | Ovary | PS ABNORMAL PAP SMEARS | F/53 |
| 21-Am-Cervix | 101P0101A | Ambion | Cervix | PM Surgery | F/40 |
| 23-B-Cervix | A504089 | Biochain | Cervix | PM-Pool of 5 | F (36-55) |
| 24-B-Uterus | A411074 | Biochain | Uterus | PM-Pool of 10 | F (32-53) |
| 25-B-Uterus | A409248 | Biochain | Uterus | PM | F/35 |
| 26-B-Uterus | A504090 | Biochain | Uterus | PM-Pool of 5 | F(40-53) |
| 28-Am-Bladder | 071P02C | Ambion | Bladder | PM GSW | M/28 |
| 29-B-Bladder | A504088 | Biochain | Bladder | PM-Pool of 5 | M(26-44)&F30 |
| 30-Am-Placenta | 021P33A | Ambion | Placenta | PB | F/33 |
| 31-B-Placenta | A410165 | Biochain | Placenta | PB | F/26 |
| 32-B-Placenta | A411073 | Biochain | Placenta | PB-Pool of 5 | F(24-30) |
| 33-B-Breast (B59) | A607155 | Biochain | Breast | PM | F/36 |
| 34-Am-Breast (B63) | 26486 | Ambion | Breast | PS bilateral breast reduction | F/43 |
| 35-Am-Breast (B64) | 23036 | Ambion | Breast | PM lung cancer | F/57 |
| 36-Cl-Prostate (P53) | 1070317 | Clontech | Prostate | PM-Pool of 47 sudden death | M (14-57) |
| 37-Am-Prostate | 061P04A | Ambion | Prostate | PM IC bleed | M/47 |
| (P42) | | | | | |
| 38-Am-Prostate | 25955 | Ambion | Prostate | PM head trauma | M/62 |
| (P59) | | | | | |
| 39-Am-Testis | 111P0104A | Ambion | Testis | PM GSW | M/25 |
| 40-B-Testis | A411147 | Biochain | Testis | PM | M/74 |
| 41-C1-Testis | 1110320 | Clontech | Testis | PM-Pool of 45 sudden death | M (14-64) |
| 42-CG-Adrenal | CG-184-10 | Ichilov | Adrenal | PM | F/81 |
| 43-B-Adrenal | A610374 | Biochain | Adrenal | PM | F/83 |
| 44-B-Heart | A411077 | Biochain | Heart | PM-Pool of 5 | M(23-70) |
| 45-CG-Heart | CG-255-9 | Ichilov | Heart focal fibrosis | PM | M/75 |
| 46-CG-Heart | CG-227-1 | Ichilov | Heart | PM | F/36 |

(continued)

| | Lot no. | Source | Tissue | Pathology | Sex/Age |
|---|---|---|---|---|---|
| 47-Am-Liver | 081P0101A | Ambion | Liver | PM ICH | M/64 |
| 48-CG-Liver | CG-93-3 | Ichilov | Liver | PM | F/19 |
| 49-CG-Liver | CG-124-4 | Ichilov | Liver of fetus | PM | fetus |
| 50-Cl-BM | 1110932 | Clontech | Bone Marrow | PM-Pool of 8 sudden death | M&F (22-65) |
| 51-CGEN-Blood | WBC#5 | CGEN | Blood | - | M |
| 52-CGEN-Blood | WBC#4 | CGEN | Blood | - | M |
| 53-CGEN-Blood | WBC#3 | CGEN | Blood | - | M |
| 54-CG-Spleen | CG-267 | Ichilov | Spleen | PM | F/25 |
| 55-CG-Spleen | 111P0106B | Ambion | Spleen | PM GSW | M/25 |
| 56-CG-Spleen | A409246 | Biochain | Spleen | PM | F/12 |
| 57-CG-Thymus | CG-98-7 | Ichilov | Thymus | PM | F/28 |
| 58-Am-Thymus | 101P0101A | Ambion | Thymus | PM head injury | M/14 |
| 59-B-Thymus | A409278 | Biochain | Thymus | PM | M/28 |
| 60-B-Thyroid | A610287 | Biochain | Thyroid | PM | M/27 |
| 61-B-Thyroid | A610286 | Biochain | Thyroid | PM | M/24 |
| 62-CG-Thyroid | CG-119-2 | Ichilov | Thyroid | PM | F/66 |
| 63-Cl-Salivary Gland | 1070319 | Clontech | Salivary Gland | PM-Pool of 24 sudden death | M&F 15-60 |
| 64-Am-Kidney | 111P0101B | Ambion | Kidney | PM ICH | M 60 |
| 65-Cl-Kidney | 1110970 | Clontech | Kidney | PM-Pool of 14 sudden death | M&F 18-59 |
| 66-B-Kidney | A411080 | Biochain | Kidney | PM-Pool of 5 | M24-46 |
| 67-CG-Cerebellum | CG-183-5 | Ichilov | Cerebellum | PM | M/74 |
| 68-CG-Cerebellum | CG-212-5 | Ichilov | Cerebellum | PM | M/54 |
| 69-B-Brain | A411322 | Biochain | Brain | PM | M/28 |
| 70-Cl-Brain | 1120022 | Clontech | Brain | PM | - |
| 71-B-Brain | A411079 | Biochain | Brain | PM-Pool of 2 | M27-28 |
| 72-CG-Brain | CG-151-1 | Ichilov | Brain | PM | F/86 |
| 73-Am-Skeletal Muscle | 101P013A | Ambion | Skeletal Muscle | PM head injury | F/28 |
| 74-Cl-Skeletal Muscle | 1061038 | Clontech | Skeletal Muscle | PM-Pool of 2 sudden death | M&F 43-46 |

Table 1_7

| sample name | Source | Sample id(GCI)/case id (Asterand) Lot no. | Tissue id (GCI)/ Specimen id (Asternd) | Sample id (Asterand) /RNA id (GCI) |
|---|---|---|---|---|
| 1-(7)-Bc-Rectum | Biochain | A610297 | | |

(continued)

| sample name | Source | Sample id(GCI)/case id (Asterand) Lot no. | Tissue id (GCI)/ Specimen id (Asternd) | Sample id (Asterand) /RNA id (GCI) |
|---|---|---|---|---|
| 2-(8)-Bc-Rectum | Biochain | A610298 | | |
| 3-GC-Colon | GCI | CDSUV | CDSUVNR3 | |
| 4-As-Colon | Asterand | 16364 | 31802 | 31802B1 |
| 5-As-Colon | Asterand | 22900 | 74446 | 74446B1 |
| 6-GC-Small bowl | GCI | V9L7D | V9L7DN6Z | |
| 7-GC-Small bowl | GCI | M3GVT | M3GVTN5R | |
| 8-GC-Small bowl | GCI | 196S2 | 196S2AJN | |
| 9-(9)-Am-Stomach | Ambion | 110P04A | | |
| 10-(10)-Bc-Stomach | Biochain | A501159 | | |
| 11-(11)-Bc-Esoph | Biochain | A603814 | | |
| 12-(12)-Bc-Esoph | Biochain | A603813 | | |
| 13-As-Panc | Asterand | 8918 | 9442 | 9442C1 |
| 14-As-Panc | Asterand | 10082 | 11134 | 11134B1 |
| 15-(48)-Ic-Liver | Ichilov | CG-93-3 | | |
| 16-As-Liver | Asterand | 7916 | 7203 | 7203B1 |
| 17-(28)-Am-Bladder | Ambion | 071P02C | | |
| 18-(29)-Bc-Bladder | Biochain | A504088 | | |
| 19-(64)-Am-Kidney | Ambion | 111P0101B | | |
| 20-(65)-Cl-Kidney | Clontech | 1110970 | | |
| 21-(66)-Bc-Kidney | Biochain | A411080 | | |
| 22-GC-Kidney | GCI | N1EVZ | N1EVZN91 | |
| 23-GC-Kidney | GCI | BMI6W | BMI6WN9F | |
| 24-(42)-Ic-Adrenal | Ichilov | CG-184-10 | | |
| 25-(43)-Bc-Adrenal | Biochain | A610374 | | |
| 26-(16)-Am-Lung | Ambion | 111P0103A | | |
| 27-(17)-Bc-Lung | Biochain | A503204 | | |
| 28-As-Lung | Asterand | 9078 | 9275 | 9275B1 |
| 29-As-Lung | Asterand | 6692 | 6161 | 6161A1 |
| 30-As-Lung | Asterand | 7900 | 7180 | 7180F1 |
| 31-(75)-GC-Ovary | GCI | L629FRV1 | | |
| 32-(76)-GC-Ovary | GCI | DWHTZRQX | | |
| 33-(77)-GC-Ovary | GCI | FDPL9NJ6 | | |
| 34-(78)-GC-Ovary | GCI | GWXUZN5M | | |
| 35-(21)-Am-Cerix | Ambion | 101P0101A | | |
| 36-GC-cervix | GCI | E2P2N | E2P2NAP4 | |
| 37-(24)-Bc-Uterus | Biochain | A411074 | | |
| 38-(26)-Bc-Uterus | Biochain | A504090 | | |

(continued)

| sample name | Source | Sample id(GCI)/case id (Asterand) Lot no. | Tissue id (GCI)/ Specimen id (Asternd) | Sample id (Asterand) /RNA id (GCI) |
|---|---|---|---|---|
| 39-(30)-Am-Placen | Ambion | 021P33A | | |
| 40-(32)-Bc-Placen | Biochain | A411073 | | |
| 41-GC-Breast | GCI | DHLR1 | | |
| 42-GC-Breast | GCI | TG6J6 | | |
| 43-GC-Breast | GCI | E6UDD | E6UDDNCF | |
| 44-(38)-Am-Prostate | Ambion | 25955 | | |
| 45-Bc-Prostate | Biochain | A609258 | | |
| 46-As-Testis | Asterand | 13071 | 19567 | 19567B1 |
| 47-As-Testis | Asterand | 19671 | 42120 | 42120A1 |
| 48-GC-Artery | GCI | 7FUUP | 7FUUPAMP | |
| 49-GC-Artery | GCI | YGTVY | YGTVYAIN | |
| 50-Th-Blood-PBMC | Tel-Hashomer | 52497 | | |
| 51-Th-Blood-PBMC | Tel-Hashomer | 31055 | | |
| 52-Th-Blood-PBMC | Tel-Hashomer | 31058 | | |
| 53-(54)-Ic-Spleen | Ichilov | CG-267 | | |
| 54-(55)-Ic-Spleen | Ichilov | 111P0106B | | |
| 55-(57)-Ic-Thymus | Ichilov | CG-98-7 | | |
| 56-(58)-Am-Thymus | Ambion | 101P0101A | | |
| 57-(60)-Bc-Thyroid | Biochain | A610287 | | |
| 58-(62)-Ic-Thyroid | Ichilov | CG-119-2 | | |
| 59-Gc-Sali gland | GCI | NNSMV | NNSMVNJC | |
| 60-(67)-Ic-Cerebellum | Ichilov | CG-183-5 | | |
| 61-(68)-Ic-Cerebellum | Ichilov | CG-212-5 | | |
| 62-(69)-Bc-Brain | Biochain | A411322 | | |
| 63-(71)-Bc-Brain | Biochain | A411079 | | |
| 64-(72)-Ic-Brain | Ichilov | CG-151-1 | | |
| 65-(44)-Bc-Heart | Biochain | A411077 | | |
| 66-(46)-Ic-Heart | Ichilov | CG-227-1 | | |
| 67-(45)-Ic-Heart | | | | |
| (Fibrotic) | Ichilov | CG-255-9 | | |
| 68-GC-Skel Mus | GCI | T8YZS | T8YZSN7O | |
| 69-GC-Skel Mus | GCI | Q3WKA | Q3WKANCJ | |
| 70-As-Skel Mus | Asterand | 8774 | 8235 | 8235G1 |
| 71-As-Skel Mus | Asterand | 8775 | 8244 | 8244A1 |
| 72-As-Skel Mus | Asterand | 10937 | 12648 | 12648C1 |
| 73-As-Skel Mus | Asterand | 6692 | 6166 | 6166A1 |

Table 1_8: Breast panel for MA analysis

| Sample RT # | MA-TAA | sample rename | Lot no | source | pathology |
|---|---|---|---|---|---|
| | | Cancer | | | |
| RT-1 | BreCa-1 | 14-A-IDC G2 | A0135T | ABS | IDC |
| RT-2 | BreCa-2 | 43-B-IDC G2 | A609183 | Biochain | IDC |
| RT-3 | BreCa-3 | 54-B-IDC G2 | A605353 | Biochain | IDC |
| RT-4 | BreCa-4 | 55-B-IDC G2 | A609179 | Biochain | IDC |
| RT-5 | BreCa-5 | 17-A-IDC G2 | 4904020036T | ABS | IDC |
| RT-6 | BreCa-6 | 42-A-IDC G3 | 6005020031T | ABS | IDC |
| RT-7 | BreCa-7 | 7-A-IDC G2 | 7263T | ABS | IDC |
| RT-8 | BreCa-8 | 48-B-IDC G2 | A609222 | Biochain | IDC |
| RT-9 | BreCa-9 | 12-A-IDC G2 | 1432T | ABS | IDC |
| RT-10 | BreCa-10 | 46-B-Carci G2 | A609177 | Biochain | Carcinoma |
| RT-11 | BreCa-11 | 16-A-IDC G2 | 4904020032T | ABS | IDC |
| RT-12 | BreCa-12 | 49-B-IDC G2 | A609223 | Biochain | IDC |
| RT-13 | BreCa-13 | 32-A-Muc Carci | 7116T | ABS | Mucinous |
| RT-14 | BreCa-14 | 45-B-IDC G2 | A609181 | Biochain | IDC |
| RT-15 | BreCa-15 | 15-A-IDC G2 | 7259T | ABS | IDC |
| RT-16 | BreCa-16 | 6-A-IDC G1 | 7238T | ABS | IDC |
| RT-17 | BreCa-17 | 26-A-IDC G3 | 7249T | ABS | IDC |
| RT-18 | BreCa-18 | 13-A-IDC G2 | A0133T | ABS | IDC |
| RT-19 | BreCa-19 | 50-B-IDC G2 | A609224 | Biochain | IDC |
| RT-20 | BreCa-20 | 44-B-IDC G2 | A609198 | Biochain | IDC |
| RT-21 | BreCa-21 | 51-B-IDC G1 | A605361 | Biochain | IDC |
| RT-22 | BreCa-22 | 27-A-IDC G3 | 4907020072T | ABS | IDC |
| RT-23 | BreCa-23 | 3Z5Z4ANH | 3Z5Z4RVE | GCI | IDC |
| RT-24 | BreCa-24 | 4W2NYAC1 | 4W2NYR9S | GCI | IDC |
| RT-25 | BreCa-25 | 54NTAAKT | 54NTAR75 | GCI | IDC |
| RT-26 | BreCa-26 | I2YLEACP | I2YLERVY | GCI | IDC |
| RT-27 | BreCa-27 | J5MPNA9Q | J5MPNRQI | GCI | IDC |
| RT-28 | BreCa-28 | KIOE7AI9 | KIOE7RWK | GCI | IDC |
| RT-29 | BreCa-29 | OLKL4AO6 | OLKL4RZ9 | GCI | IDC |
| RT-30 | BreCa-30 | RD3F9AFQ | RD3F9RY9 | GCI | IDC |
| RT-31 | BreCa-31 | SE5BKAEQ | SE5BKRHY | GCI | IDC |
| RT-32 | BreCa-32 | VK1EJAQE | VK1EJRKH | GCI | IDC |
| RT-33 | BreCa-33 | YOLOFARG | YOLOFRE7 | GCI | IDC |
| RT-34 | BreCa-34 | YQ1WWAUV | YQ1WWROR | GCI | IDC |
| RT-35 | BreCa-35 | YSZ67A48 | YSZ67ROA | GCI | IDC |
| RT-36 | BreCa-36 | POPHPAZ4 | POPHPRDM | GCI | IDC |

(continued)

| Sample RT # | MA-TAA | sample rename | Lot no | source | pathology |
|---|---|---|---|---|---|
| RT-37 | BreCa-37 | 5IRTKAXT | 5IRTKRTG | GCI | IDC |
| RT-38 | BreCa-38 | DSI52AH3 | DSI52RVW | GCI | IDC |
| RT-39 | BreCa-39 | GETCVAY2 | GETCVRIT | GCI | IDC |
| RT-40 | BreCa-40 | S2GBYAGC | S2GBYRR1 | GCI | IDC |
| RT-41 | BreCa-41 | UT3SEAQY | UT3SERM8 | GCI | IDC |
| RT-42 | BreCa-42 | PVSYXA72 | PVSYXR66 | GCI | IDC |
| RT-43 | BreCa-43 | 17138 | 30697A1 | Asterand | IDC |
| RT-44 | BreCa-44 | 17959 | 31225A1 | Asterand | IDC |
| RT-45 | BreCa-45 | 52-B-ILC G1 | A605360 | Biochain | ILC |
| RT-46 | BreCa-46 | IS84YAAY | IS84YR6E | GCI | ILC |
| RT-47 | BreCa-47 | I35USA9G | I35USR7K | GCI | ILC |
| RT-48 | BreCa-48 | 17090 | 30738A1 | Asterand | ILC |
| RT-49 | BreCa-49 | 42509 | 42509A1 | Asterand | Ductal Carcinoma In Situ(DCIS) |
| | | Benign | | | |
| RT-50 | BreBe-1 | NNP3QA4V | NNP3QRCW | GCI | FIBROADENOMA OF THE BREAST |
| RT-51 | BreBe-2 | QK8IYALU | QK8IYRW1 | GCI | FIBROADENOMA OF THE BREAST |
| RT-52 | BreBe-3 | ZT15MAMR | ZT15MR2Y | GCI | FIBROADENOMA OF THE BREAST |
| RT-53 | BreBe-4 | 11975 | 15478B1 | Asterand | Fibroadenoma |
| | | Normal | | | |
| RT-54 | BreNo-1 | 57-B-N | A609233 | Biochain | Normal post mortem |
| RT-55 | BreNo-2 | 59-B-N | A607155 | Biochain | Normal post mortem |
| RT-56 | BreNo-3 | 60-B-N | A609234 | Biochain | Normal post mortem |
| RT-57 | BreNo-4 | 63-Am-N | 26486 | Ambion | Normal post surgery |
| RT-58 | BreNo-5 | 66-Am-N | 36678 | Ambion | Normal post mortem |
| RT-59 | BreNo-6 | 64-Am-N | 23036 | Ambion | Normal post mortem |
| RT-60 | BreNo-7 | 56-B-N | A609235 | Biochain | Normal post mortem |
| RT-61 | BreNo-8 | 65-Am-N | 31410 | Ambion | Normal post mortem |
| RT-62 | BreNo-9 | 67-Am-N | 073P010602086A | Ambion | Normal post mortem |
| RT-63 | BreNo-10 | 58-B-N | A609232 | Biochain | Normal post mortem |
| RT-64 | BreNo-11 | DHLR1NIQ | DHLR1R8J | GCI | Normal post surgery |
| RT-65 | BreNo-12 | 14398 | 20021D1 | Asterand | Normal post surgery |

Table 1_9: Ovary panel for MA analysis

| | | OvSr = SEROUS ADENOCARCINOMA | | |
|---|---|---|---|---|
| Sample # | TAA2_MA ID | Tissue ID | RNA ID | Source |
| 1 | OvSr1 | 2O37OAI3 | 2O37ORTX | GCI |
| 2 | OvSr2 | 3NTISA77 | 3NTISRY4 | GCI |
| 3 | OvSr3 | 4WAABA68 | 4WAABR62 | GCI |
| 4 | OvSr4 | 79Z67AL4 | 79Z67RFA | GCI |
| 5 | OvSr5 | 7B3DPA5S | 7B3DPR3Y | GCI |
| 6 | OvSr6 | 7RMHZAMG | 7RMHZRQ9 | GCI |
| 7 | OvSr7 | CEJUSAVO | CEJUSRZG | GCI |
| 8 | OvSr8 | DDSNLAWD | DDSNLR79 | GCI |
| 9 | OvSr9 | DH8PHAMR | DH8PHRPE | GCI |
| 10 | OvSr10 | 5NCLKA15 | 5NCLKR2O | GCI |
| 11 | OvSr11 | 1HI5HAHH | 1HI5HRE2 | GCI |
| 12 | OvSr12 | 33-B-Pap Sero CystAde G1 | | BioChain |
| 13 | OvSr13 | 31-B-Pap Sero CystAde G3 | | BioChain |
| 14 | OvSr14 | 29-G-Sero Adeno G3 | G035 | GOG |
| 15 | OvSr15 | 9-G-Adeno G3 | 99-06-G901 | GOG |
| 66 | OvSr16 | 18701 | 40773C1 | Asterand |
| 67 | OvSr17 | 13268 | 19832A1 | Asterand |
| | | OvPp = Papillary adenocarcinoma | | |
| Sample # | TAA2_MA ID | Tissue ID | RNA ID | Source |
| 16 | OvPp1 | 4-A-Pap CystAdeno G2 | ILS-7286 | ABS |
| 17 | OvPp2 | 3-A-Pap Adeno G2 | ILS-1431 | ABS |
| 18 | OvPp3 | 2-A-Pap Adeno G2 | ILS-1408 | ABS |
| 19 | OvPp4 | 25-A-Pap Sero Adeno G3 | N0021 | ABS |
| 20 | OvPp5 | 1-A-Pap Adeno G3 | ILS-1406 | ABS |
| 21 | OvPp6 | 66-G-Pap Sero Adeno G3 SIV | 2000-01-G413 | GOG |
| | | OvEm = ENDOMETROID ADENOCARINOMA | | |
| Sample # | TAA2_MA ID | Tissue ID | RNA ID | Source |
| 22 | OvEm1 | 1U52XAHJ | 1U52XRPE | GCI |
| 23 | OvEm2 | 533DXAHE | 533DXRKV | GCI |
| 24 | OvEm3 | 5895CAXD | 5895CR56 | GCI |
| 25 | OvEm4 | A17WSACA | A17WSR7Y | GCI |
| 26 | OvEm5 | E2WKFA4F | E2WKFRPT | GCI |
| 27 | OvEm6 | HZ2EYAU6 | HZ2EYRC6 | GCI |
| 28 | OvEm7 | PZQXHALS | PZQXHRGN | GCI |

(continued)

| | | | | |
|---|---|---|---|---|
| | | OvEm = ENDOMETROID ADENOCARINOMA | | |
| Sample # | TAA2_MA ID | Tissue ID | RNA ID | Source |
| 29 | OvEm8 | RWOIVALL | RWOIVRI1 | GCI |
| 30 | OvEm9 | 1VT3IAZ6 | 1VT3IRT1 | GCI |
| 31 | OvEm10 | 18VHZALI | I8VHZRR4 | GCI |
| 32 | OvEm11 | 34-G-Pap Endo Adeno G3 | 95-04-2002 | GOG |
| | | OvMu = Mucinous adenocarcinoma | | |
| Sample # | TAA2_MA ID | Tissue ID | RNA ID | Source |
| 33 | OvMu1 | 22-A-Muc CystAde G2 | A0139 | ABS |
| 34 | OvMu4 | 23-A-Muc CystAde G3 | VNM-00187 | ABS |
| 35 | OvMu6 | 19- B-Muc Adeno G3 | A504085 | BioChain |
| 36 | OvMu3 | 17-B-Muc Adeno G3 | A504084 | BioChain |
| 37 | OvMu5 | IMDA1ANG | IMDA1RQG | GCI |
| 38 | OvMu2 | 21-G- Muc CystAde G2-3 | 95-10-G020 | GOG |
| 68 | OvMu7 | 12742 | 18920A1 | Asterand |
| 69 | OvMu8 | NJM4UAC4 | NJM4URI5 | GCI |
| 70 | OvMu9 BL | 3D5FOA9R | 3D5FORJ9 | GCI |
| 71 | OvMu10_BL | 7JP3FAIH | 7JP3FRCY | GCI |
| 72 | OvMu11_BL | SC656AKT | SC656RN6 | GCI |
| | | OvBe = Benign samples | | |
| Sample # | TAA2_MA ID | Tissue ID | RNA ID | Source |
| 39 | OvBe1 | 62-G-Ben Muc CysAdenoma | 99-10-G442 | GOG |
| 40 | OvBe2 | 60-G- Muc CysAdenoma | 99-01-G043 | GOG |
| 41 | OvBe3 | 56-G-Ben Muc CysAdeno | 99-01-G407 | GOG |
| 42 | OvBe4 | 64-G-Ben Sero CysAdenoma | 99-06-G039 | GOG |
| 43 | OvBe5 | 59-G-Sero CysAdenoFibroma | 98-12-G401 | GOG |
| 44 | OvBe6 | QLIKYAKS | QLIKYRNG | GCI |
| 45 | OvBe7 | 943ECATN | 943ECRVO | GCI |
| 46 | OvBe8 | 943ECAW7 | 943ECRYK | GCI |
| 47 | OvBe9 | JO8W7AKQ | JO8W7RTI | GCI |
| 48 | OvBe10 | DQQ2FAMC | DQQ2FRAC | GCI |
| | | NOv = Normal Samples | | |
| Sample # | TAA2_MA ID | Tissue ID | RNA ID | Source |
| 49 | NOv1 | 45-B-N | A503274 | BioChain |
| 50 | NOv2 | 46-B-N | A504086 | BioChain |
| 51 | NOv3 | 48-B-N | A504087 | BioChain |

(continued)

| | | NOv = Normal Samples | | |
|---|---|---|---|---|
| Sample # | TAA2_MA ID | Tissue ID | RNA ID | Source |
| 52 | NOv4 | GWXUZN5M | GWXUZRI3 | GCI |
| 53 | NOv5 | IDUVYN9I | IDUVYROT | GCI |
| 54 | NOv6 | L629FN58 | L629FRV1 | GCI |
| 55 | NOv7 | SJ2R2NPS | SJ2R2RFN | GCI |
| 56 | NOv8 | TW9PMN69 | TW9PMR25 | GCI |
| 57 | NOv9 | XLB23NA4 | XLB23RKV | GCI |
| 58 | NOv10 | DWHTZNBF | DWHTZRQX | GCI |
| 59 | NOv11 | FDPL9NJ6 | FDPL9RVC | GCI |
| 60 | NOv12 | TOAE5N2M | TOAE5R37 | GCI |
| 61 | NOv13 | DD73BNIO | DD73BR3V | GCI |
| | | OvExtr = Clear cell & other samples | | |
| Sample # | TAA2_MA ID | Tissue ID | RNA ID | Source |
| 62 | OvExtr1 | 41-G-Mix Sero/Muc/Endo G2 | 98-03-G803 | GOG |
| 63 | OvExtr2 | 43-G-Clear cell Adeno G3 | 2001-10-G002 | GOG |
| 64 | OvExtr3 | 44-G-Clear cell Adeno | 2001-07-G084 | GOG |
| 65 | OvExtr4 | 42-G-Adeno borderline | 98-08-G001 | GOG |

**Materials and Experimental Procedures**

RNA preparation - RNA was obtained from ABS (Wilmington, DE 19801, USA, absbioreagents.com), BioChain Inst. Inc. (Hayward, CA 94545 USA biochain.com), GOG for ovary samples- Pediatric Cooperative Human Tissue Network, Gynecologic Oncology Group Tissue Bank, Children Hospital of Columbus (Columbus OH 43205 USA), Clontech (Franklin Lakes, NJ USA 07417, clontech.com), Ambion (Austin, TX 78744 USA, ambion.com), Asternad (Detroit, MI 48202-3420, USA, asterand.com), and from Genomics Collaborative Inc., a Division of Seracare (Cambridge, MA 02139, USA, .genomicsinc.com). Alternatively, RNA was generated from tissue samples using TRI-Reagent (Molecular Research Center), according to Manufacturer's instructions. Tissue and RNA samples were obtained from patients or from postmortem. Total RNA samples were treated with DNaseI (Ambion).

RT PCR - Purified RNA (1 μg) was mixed with 150 ng Random Hexamer primers (Invitrogen) and 500 μM dNTP in a total volume of 15.6 μl. The mixture was incubated for 5 min at 65 °C and then quickly chilled on ice. Thereafter, 5 μl of 5X SuperscriptII first strand buffer (Invitrogen), 2.4μl 0.1M DTT and 40 units RNasin (Promega) were added, and the mixture was incubated for 10 min at 25 °C, followed by further incubation at 42 °C for 2 min. Then, 1 μl (200units) of SuperscriptII (Invitrogen) was added and the reaction (final volume of 25μl) was incubated for 50 min at 42 °C and then inactivated at 70 °C for 15min. The resulting cDNA was diluted 1:20 in TE buffer (10 mM Tris pH=8, 1 mM EDTA pH=8).

Real-Time RT-PCR analysis- cDNA (5μl), prepared as described above, was used as a template in Real-Time PCR reactions using the SYBR Green I assay (PE Applied Biosystem) with specific primers and UNG Enzyme (Eurogentech or ABI or Roche). The amplification was effected as follows: 50 °C for 2 min, 95 °C for 10 min, and then 40 cycles of 95 °C for 15sec, followed by 60 °C for 1 min. Detection was performed by using the PE Applied Biosystem SDS 7000. The cycle in which the reactions achieved a threshold level (Ct) of fluorescence was registered and was used to calculate the relative transcript quantity in the RT reactions. Non-detected samples were assigned Ct value of 41 and were calculated accordingly. The relative quantity was calculated using the equation Q=efficiency$^{\wedge-Ct}$. The efficiency of the PCR reaction was calculated from a standard curve, created by using serial dilutions of several reverse transcription (RT) reactions. To minimize inherent differences in the RT reaction, the resulting relative quantities were normalized to normalization factor calculated in one of the following methods as indicated in the text:

Method 1- the geometric mean of the relative quantities of the selected housekeeping (HSKP) genes was used as normalization factor.

Method 2 - The expression of several housekeeping (HSKP) genes was checked on every panel. The relative quantity (Q) of each housekeeping gene in each sample, calculted as described above, was diveded by the median quantity of this gene in all panel samples to obtain the "relative Q rel to MED". Then, for each sample the median of the "relative Q rel to MED" of the selected housekeeping genes was calculted and served as normalization factor of this sample for further calculations. Schematic summary of quantitative real-time PCR analysis is presented in Figure 5. As shown, the x-axis shows the cycle number.The $C_T$ = Threshold Cycle point, which is the cycle that the amplification curve crosses the fluorescence threshold that was set in the experiment. This point is a calculated cycle number in which PCR products signal is above the background level (passive dye ROX) and still in the Geometric/Exponential phase (as shown, once the level of fluorescence crosses the measurement threshold, it has a geometrically increasing phase, during which measurements are most accurate, followed by a linear phase and a plateau phase; for quantitative measurements, the latter two phases do not provide accurate measurements). The y-axis shows the normalized reporter fluorescence. It should be noted that this type of analysis provides relative quantification.

Real-Time RT-PCR analysis using TaqMan® probes - cDNA (5μl), prepared as described above, was used as a template in Real-Time PCR reactions using the TaqMan Universal PCR Master mix (PE Applied Biosystem) with specific primers and specific TaqMan® MGB probes. The primers were used at a concentration of 500 nM and the probes at a concentration of 200 nM. The amplification was effected as follows: 50 °C for 2 min, 95 °C for 10 min, and then 40 cycles of 95 °C for 15sec, followed by 60 °C for 1 min. Detection was performed by using the PE Applied Biosystem SDS 7000. The cycle in which the reactions achieved a threshold level (Ct) of fluorescence was registered and was used to calculate the relative transcript quantity in the RT reactions. The relative quantity was calculated using the equation Q=2^-Ct. To minimize inherent differences in the RT reaction, the resulting relative quantities were normalized using normalization factor calculated as follows: The expression of several housekeeping (HSKP) genes was checked on the RT panel by qRT-PCR using SYBR Green detection. The relative quantity (Q) of each housekeeping gene in each sample, calculted as described above, was diveded by the median quantity of this gene in all panel samples to obtain the "relative Q rel to MED". Then, for each sample the median of the "relative Q rel to MED" of the selected housekeeping genes was calculted and served as normalization factor of this sample for further calculations. Schematic summary of quantitative real-time PCR analysis is presented in Figure 1. As shown, the x-axis shows the cycle number. The CT = Threshold Cycle point, which is the cycle that the amplification curve crosses the fluorescence threshold that was set in the experiment. This point is a calculated cycle number in which PCR products signal is above the background level (passive dye ROX) and still in the Geometric/Exponential phase (as shown, once the level of fluorescence crosses the measurement threshold, it has a geometrically increasing phase, during which measurements are most accurate, followed by a linear phase and a plateau phase; for quantitative measurements, the latter two phases do not provide accurate measurements). The y-axis shows the normalized reporter fluorescence. It should be noted that this type of analysis provides relative quantification.

The sequences of the housekeeping genes measured in all the examples on ovarian cancer panel were as follows:

SDHA (GenBank Accession No. NM_004168 (SEQ. ID NO: 33);
SDHA Forward primer (SEQ. ID NO: 34): TGGGAACAAGAGGGCATCTG
SDHA Reverse primer (SEQ. ID NO: 35): CCACCACTGCATCAAATTCATG
SDHA-amplicon (SEQ. ID NO:36):

TGGGAACAAGAGGGCATCTGCTAAAGTTTCAGATTCCATTTCTGCTCAGTATCCAGTAGT
GGATCATGAATTTGATGCAGTGGTGG

PBGD (GenBank Accession No. BC019323 (SEQ. ID NO: 1)),
PBGD Forward primer (SEQ. ID NO: 2): TGAGAGTGATTCGCGTGGG
PBGD Reverse primer (SEQ. ID NO: 3): CCAGGGTACGAGGCTTTCAAT
PBGD-amplicon (SEQ. ID NO: 4):

TGAGAGTGATTCGCGTGGGTACCCGCAAGAGCCAGCTTGCTCGCATACAGACGGACAGT
GTGGTGGCAACATTGAAAGCCTCGTACCCTGG

HPRT1 (GenBank Accession No. NM_000194 (SEQ. ID NO: 5)),
HPRT1 Forward primer (SEQ. ID NO:6): TGACACTGGCAAAACAATGCA
HPRT1 Reverse primer (SEQ. ID NO: 7): GGTCCTTTTCACCAGCAAGCT
HPRT1-amplicon (SEQ. ID NO: 8):

TGACACTGGCAAAACAATGCAGACTTTGCTTTCCTTGGTCAGGCAGTATAATCCAAAGAT
GGTCAAGGTCGCAAGCTTGCTGGTGAAAAGGACC

GAPDH (GenBank Accession No. BC026907 (SEQ. ID NO: 9))
GAPDH Forward primer (SEQ. ID NO:10): TGCACCACCAACTGCTTAGC
GAPDH Reverse primer (SEQ. ID NO:11): CCATCACGCCACAGTTTCC
GAPDH-amplicon (SEQ. ID NO:12):

TGCACCACCAACTGCTTAGCACCCCTGGCCAAGGTCATCCATGACAACTTTGGTATCGTG
GAAGGACTCATGACCACAGTCCATGCCATCACTGCCACCCAGAAGACTGTGGATGG

The sequences of the housekeeping genes measured in all the examples on colon cancer tissue testing panel were as follows:

PBGD (GenBank Accession No. BC019323 (SEQ. ID NO: 1)),
PBGD Forward primer (SEQ. ID NO: 2): TGAGAGTGATTCGCGTGGG
PBGD Reverse primer (SEQ. ID NO: 3): CCAGGGTACGAGGCTTTCAAT
PBGD-amplicon (SEQ. ID NO: 4):

TGAGAGTGATTCGCGTGGGTACCCGCAAGAGCCAGCTTGCTCGCATACAGACGGACAGT
GTGGTGGCAACATTGAAAGCCTCGTACCCTGG

HPRT1 (GenBank Accession No. NM_000194 (SEQ. ID NO: 5)),
HPRT1 Forward primer (SEQ. ID NO:6): TGACACTGGCAAAACAATGCA
HPRT1 Reverse primer (SEQ. ID NO: 7): GGTCCTTTTCACCAGCAAGCT
HPRT1-amplicon (SEQ. ID NO: 8):

TGACACTGGCAAAACAATGCAGACTTTGCTTTCCTTGGTCAGGCAGTATAATCCAAAGAT
GGTCAAGGTCGCAAGCTTGCTGGTGAAAAGGACC

G6PD (GenBank Accession No. NM_000402 (SEQ. ID NO: 13))
G6PD Forward primer (SEQ. ID NO:14): gaggccgtcaccaagaacat
G6PD Reverse primer (SEQ. ID NO:15): ggacagccggtcagagctc
G6PD-amplicon (SEQ. ID NO:16):

gaggccgtcaccaagaacattcacgagtcctgcatgagccagataggctggaaccgcatcatcgtggagaagcccttcgggagggacctgcaga
gctctgaccggctgtcc

RPS27A (GenBank Accession No. NM_002954 (SEQ. ID NO: 17))
RPS27A Forward primer (SEQ. ID NO:18): CTGGCAAGCAGCTGGAAGAT
RPS27A Reverse primer (SEQ. ID NO:19): TTTCTTAGCACCACCACGAAGTC
RPS27A-amplicon (SEQ. ID NO:20):

CTGGCAAGCAGCTGGAAGATGGACGTACTTTGTCTGACTACAATATTCAAAAGGAGTCTA
CTCTTCATCTTGTGTTGAGACTTCGTGGTGGTGCTAAGAAA

The sequences of the housekeeping genes measured in all the examples in the lung panel were as follows:

Ubiquitin (GenBank Accession No. BC000449 (SEQ. ID NO: 29))
Ubiquitin Forward primer (SEQ. ID NO: 30): ATTTGGGTCGCGGTTCTTG
Ubiquitin Reverse primer (SEQ. ID NO: 31): TGCCTTGACATTCTCGATGGT
Ubiquitin-amplicon (SEQ. ID NO: 32)

ATTTGGGTCGCGGTTCTTGTTTGTGGATCGCTGTGATCGTCACTTGACAATGCAGATCTTC
GTGAAGACTCTGACTGGTAAGACCATCACCCTCGAGG
TTGAGCCCAGTGACACCATCGAGAATGTCAAGGCA

SDHA (GenBank Accession No. NM_004168 (SEQ. ID NO: 33))
SDHA Forward primer (SEQ. ID NO: 34): TGGGAACAAGAGGGCATCTG
SDHA Reverse primer (SEQ. ID NO: 35): CCACCACTGCATCAAATTCATG
SDHA-amplicon (SEQ. ID NO:36):

TGGGAACAAGAGGGCATCTGCTAAAGTTTCAGATTCCATTTCTGCTCAGTATCCAGTAGT
GGATCATGAATTTGATGCAGTGGTGG

PBGD (GenBank Accession No. BC019323 (SEQ. ID NO: 1)),
PBGD Forward primer (SEQ. ID NO: 2): TGAGAGTGATTCGCGTGGG
PBGD Reverse primer (SEQ. ID NO: 3): CCAGGGTACGAGGCTTTCAAT
PBGD-amplicon (SEQ. ID NO: 4):

TGAGAGTGATTCGCGTGGGTACCCGCAAGAGCCAGCTTGCTCGCATACAGACGGACAGT
GTGGTGGCAACATTGAAAGCCTCGTACCCTGG

HPRT1 (GenBank Accession No. NM_000194 (SEQ. ID NO: 5)),
HPRT1 Forward primer (SEQ. ID NO:6): TGACACTGGCAAAACAATGCA
HPRT1 Reverse primer (SEQ. ID NO: 7): GGTCCTTTTCACCAGCAAGCT
HPRT1-amplicon (SEQ. ID NO: 8):

TGACACTGGCAAAACAATGCAGACTTTGCTTTCCTTGGTCAGGCAGTATAATCCAAAGAT
GGTCAAGGTCGCAAGCTTGCTGGTGAAAAGGACC

The sequences of the housekeeping genes measured in all the examples on breast cancer panel were as follows:

G6PD (GenBank Accession No. NM_000402 (SEQ. ID NO: 13))
G6PD Forward primer (SEQ. ID NO:14): gaggccgtcaccaagaacat
G6PD Reverse primer (SEQ. ID NO:15): ggacagccggtcagagctc
G6PD-amplicon (SEQ. ID NO:16):

gaggccgtcaccaagaacattcacgagtcctgcatgagccagataggctggaaccgcatcatcgtggagaagcccttcgggagggacctgcaga gctctgaccggctgtcc

SDHA (GenBank Accession No. NM_004168 (SEQ. ID NO: 33))
SDHA Forward primer (SEQ. ID NO: 34): TGGGAACAAGAGGGCATCTG
SDHA Reverse primer (SEQ. ID NO: 35): CCACCACTGCATCAAATTCATG
SDHA-amplicon (SEQ. ID NO:36):

TGGGAACAAGAGGGCATCTGCTAAAGTTTCAGATTCCATTTCTGCTCAGTATCCAGTAGT GGATCATGAATTTGATGCAGTGGTGG

PBGD (GenBank Accession No. BC019323 (SEQ. ID NO: 1)),
PBGD Forward primer (SEQ. ID NO: 2): TGAGAGTGATTCGCGTGGG
PBGD Reverse primer (SEQ. ID NO: 3): CCAGGGTACGAGGCTTTCAAT
PBGD-amplicon (SEQ. ID NO: 4):

TGAGAGTGATTCGCGTGGGTACCCGCAAGAGCCAGCTTGCTCGCATACAGACGGACAGT GTGGTGGCAACATTGAAAGCCTCGTACCCTGG

HPRT1 (GenBank Accession No. NM_000194 (SEQ. ID NO: 5)),
HPRT1 Forward primer (SEQ. ID NO:6): TGACACTGGCAAAACAATGCA
HPRT1 Reverse primer (SEQ. ID NO: 7): GGTCCTTTTCACCAGCAAGCT
HPRT1-amplicon (SEQ. ID NO: 8):

TGACACTGGCAAAACAATGCAGACTTTGCTTTCCTTGGTCAGGCAGTATAATCCAAAGAT GGTCAAGGTCGCAAGCTTGCTGGTGAAAAGGACC

The sequences of the housekeeping genes measured in all the examples on normal tissue samples panel were as follows:

RPL19 (GenBank Accession No. NM_000981 (SEQ. ID NO: 21))
RPL19Forward primer (SEQ. ID NO:22): TGGCAAGAAGAAGGTCTGGTTAG
RPL19Reverse primer (SEQ. ID NO:23): TGATCAGCCCATCTTTGATGAG
RPL19-amplicon (SEQ. ID NO:24):

TGGCAAGAAGAAGGTCTGGTTAGACCCCAATGAGACCAATGAAATCGCCAATGCCAACT CCCGTCAGCAGATCCGGAAGCTCATCAAAGATGGGCTGATCA

TATA box (GenBank Accession No. NM_003194 (SEQ. ID NO: 25)),
TATA box Forward primer (SEQ. ID NO:26): CGGTTTGCTGCGGTAATCAT
TATA box Reverse primer (SEQ. ID NO:27): TTTCTTGCTGCCAGTCTGGAC
TATA box -amplicon (SEQ. ID NO: 28):

CGGTTTGCTGCGGTAATCATGAGGATAAGAGAGCCACGAACCACGGCACTGATTTTCAGT
TCTGGGAAAATGGTGTGCACAGGAGCCAAGAGTGAAGAACAGTCCAGACTGGCAGCAAG
AAA

Ubiquitin (GenBank Accession No. BC000449 (SEQ. ID NO: 29))
Ubiquitin Forward primer (SEQ. ID NO: 30): ATTTGGGTCGCGGTTCTTG
Ubiquitin Reverse primer (SEQ. ID NO: 31): TGCCTTGACATTCTCGATGGT
Ubiquitin-amplicon (SEQ. ID NO: 32)

ATTTGGGTCGCGGTTCTTGTTTGTGGATCGCTGTGATCGTCACTTGACAATGCAGATCTTC
GTGAAGACTCTGACTGGTAAGACCATCACCCTCGAGG
TTGAGCCCAGTGACACCATCGAGAATGTCAAGGCA

SDHA (GenBank Accession No. NM_004168 (SEQ. ID NO: 33))
SDHA Forward primer (SEQ. ID NO: 34): TGGGAACAAGAGGGCATCTG
SDHA Reverse primer (SEQ. ID NO: 35): CCACCACTGCATCAAATTCATG
SDHA-amplicon (SEQ. ID NO:36):

TGGGAACAAGAGGGCATCTGCTAAAGTTTCAGATTCCATTTCTGCTCAGTATCCAGTAGT
GGATCATGAATTTGATGCAGTGGTGG

**Actual Marker Examples**

The following examples relate to specific actual marker examples. It should be noted that Table numbering is restarted within each example related to a particular Cluster, as indicated by the titles below.

DESCRIPTION FOR CLUSTER N43992

Cluster N43992 features 3 transcript(s) and 14 segment(s) of interest, the names for which are given in Tables 2 and 3, respectively. The selected encoded protein variants are given in table 4.

*Table 2 - Transcripts of interest*

| Transcript Name |
|---|
| N43992_T1 (SEQ. ID NO:37) |
| (SEQ. ID NO:38) |
| N43992_T4 N43992_T9 (SEQ. ID NO:39) |

*Table 3 - Segments of interest*

| Segment Name |
|---|
| N43992_N0 (SEQ. ID NO: 40) |
| N43992_N5 (SEQ. ID NO: 41) |
| N43992_N9 (SEQ. ID NO: 42) |
| N43992_N12 (SEQ. ID NO: 43) |

(continued)

| Segment Name |
|---|
| N43992_N14 (SEQ. ID NO: 44) |
| N43992_N15 (SEQ. ID NO: 45) |
| N43992_N17 (SEQ. ID NO: 46) |
| N43992_N22 (SEQ. ID NO: 47) |
| N43992_N1 (SEQ. ID NO: 48) |
| N43992_N3 (SEQ. ID NO: 49) |
| N43992_N4 (SEQ. ID NO: 50) |
| N43992_N7 (SEQ. ID NO: 51) |
| N43992_N10 (SEQ. ID NO: 52) |
| N43992_N20 (SEQ. ID NO: 53) |

*Table 4 - Proteins of interest*

| Protein Name | Corresponding Transcript(s) |
|---|---|
| N43992_P13 (SEQ. ID NO: 57) | N43992_T1 (SEQ. ID NO:37) |
| N43992_P14 (SEQ. ID NO: 58) | N43992_T4 (SEQ. ID NO:38) |
| N43992_P16 (SEQ. ID NO: 59) | N43992_T9 (SEQ. ID NO:39) |

These sequences are variants of the known protein Delta-like protein 3 precursor (SEQ. ID NO: 54, SwissProt accession identifier DLL3_HUMAN); known also according to the synonym Drosophila Delta homolog 3, referred to herein as the previously known protein. The nucleic acid sequence of the known protein Delta-like protein 3 precursor is given in SEQ. ID NOs: 54 -56.

Protein Delta-like protein 3 precursor is known or believed to have the following function(s): inhibits primary neurogenesis; may be required to divert neurons along a specific differentiation pathway; play a role in the formation of somite boundaries during segmentation of the paraxial mesoderm (by similarity). Known polymorphisms for this sequence are as shown in Table 5.

*Table 5 - Amino acid mutations for Known Protein*

| SNP position(s) on amino acid sequence | Comment |
|---|---|
| 218 | L -> P (in dbSNP:1110627). /FTId=VAR_016776 |
| 385 | G -> D (in SCDO1). /FTId=VAR_009952 |

Protein Delta-like protein 3 precursor (SEQ. ID NO: 54) localization is believed to be Type I membrane protein.

The following GO Annotation(s) apply to the previously known protein. The following annotation(s) were found: cell fate determination; embryonic development (sensu Mammalia); neurogenesis; Notch signaling pathway; skeletal development, which are annotation(s) related to Biological Process; Notch binding, which are annotation(s) related to Molecular Function; and integral to membrane, which are annotation(s) related to Cellular Component.

The GO assignment relies on information from one or more of the SwissProt/TremB1 Protein knowledgebase, available from <http://www.expasy.ch/sprot/>; or Locuslink, available from <http://www.ncbi.nlm.nih.gov/projects/LocusLink/>.

The present invention provides a number of different novel amino acid and nucleic acid sequences of known DLL3 protein, which may optionally be used as diagnostic markers, preferably as serum markers.

The variant N43992_P16 (SEQ. ID NO: 59) was previously disclosed by the inventors in published PCT application no WO2005/071058, hereby incorporated by reference as if fully set forth herein, but have now been shown to have novel and surprising diagnostic uses as described herein for other variants of cluster N43992.

According to the present invention, the known (wild type) DLL3 protein is used as novel diagnostic marker.

According to the present invention, the wild type DLL3 protein diagnostic marker is optionally used with in vivo imaging technologies, including but not limited to magnetic resonance imaging, computed tomography scanning, PET, SPECT and the like. Optionally, according to the present invention, the wild type DLL3 protein diagnostic marker is used as IHC marker.

According to optional but preferred embodiments of the present invention, variants of this cluster according to the present invention (amino acid and/or nucleic acid sequences of N43992) may optionally have one or more of the utilities based on the finding that mutations in DLL3 cause axial skeletal defects in spondylocostal dysostosis (10742114). It should be noted that these utilities are optionally and preferably suitable for human and non-human animals as subjects, except where otherwise noted. The reasoning is described with regard to biological and/or physiological and/or other information about the known protein, but is given to demonstrate particular diagnostic utility for the variants according to the present invention.

Other non-limiting exemplary utilities for N43992 variants, according to the present invention are described in greater detail below and also with regard to the previous section on clinical utility.

Cluster N43992 can be used as a diagnostic marker according to overexpression of transcripts of this cluster in cancer. Expression of such transcripts in normal tissues is also given according to the previously described methods. The term "number" in the left hand column of the table and the numbers on the y-axis of figure 6 refer to weighted expression of ESTs in each category, as "parts per million" (ratio of the expression of ESTs for a particular cluster to the expression of all ESTs in that category, according to parts per million).

Overall, the following results were obtained as shown with regard to the histograms in Figure 6 and Table 6. This cluster is overexpressed (at least at a minimum level) in the following pathological conditions: brain malignant tumors, a mixture of malignant tumors from different tissues and epithelial malignant tumors.

*Table 6 - Normal tissue distribution*

| Name of Tissue | Number |
|---|---|
| pancreas | 0 |
| uterus | 0 |
| brain | 3 |
| lung | 0 |
| general | 1 |
| skin | 0 |
| epithelial | 0 |

*Table 7 - P values and ratios for expression in cancerous tissue*

| Name of Tissue | P1 | P2 | SP1 | R3 | SP2 | R4 |
|---|---|---|---|---|---|---|
| pancreas | 3.1e-01 | 1.6e-01 | 4.2e-01 | 2.4 | 1.1e-02 | 3.7 |
| uterus | N/A | 3.7e-01 | N/A | N/A | 8.0e-01 | 1.3 |
| brain | 1.5e-05 | 6.0e-06 | 1.0e-19 | 35.3 | 1.1e-21 | 30.3 |
| lung | 4.7e-01 | 3.7e-01 | 4.1e-01 | 3.7 | 2.3e-01 | 3.4 |
| general | 2.4e-05 | 2.9e-05 | 4.2e-17 | 16.6 | 2.2e-28 | 18.4 |
| skin | 3.3e-01 | 2.4e-01 | 1.5e-01 | 6.8 | 1.9e-07 | 2.7 |
| epithelial | 2.3e-01 | 1.0e-01 | 7.8e-02 | 3.7 | 3.8e-08 | 6.0 |

As noted above, cluster N43992 features 3 transcript(s), which were listed in Table 2 above. These transcript (s) encode for protein(s) which are variant(s) of protein Delta-like protein 3 precursor (SEQ. ID NO: 54). A description of each variant protein according to the present invention is now provided.

Variant protein N43992_P13 (SEQ. ID NO: 57) according to the present invention is encoded by transcript N43992_T1 (SEQ. ID NO:37).

1. Comparison report between N43992_P13 (SEQ. ID NO: 57) and DLL3_HUMAN (SEQ. ID NO: 54):

A. An isolated chimeric polypeptide encoding for N43992_P13 (SEQ. ID NO: 57), comprising a first amino acid sequence being at least 90% homologous to MVSPRMSGLLSQTVILALIFLPQTRPAGVFELQIHSFGPGPGP corresponding to amino acids 1 - 43 of DLL3_HUMAN (SEQ. ID NO: 54), which also corresponds to amino acids 1 - 43 of N43992_P13 (SEQ. ID NO: 57), and a second amino acid sequence being at least 70%, optionally at least 80%, preferably at least 85%, more preferably at least 90% and most preferably at least 95% homologous to a polypeptide having the sequence APLPPLLQSLPEAWALRGGRRVPVRPGRGAECARTGLHRAARSARA (SEQ. ID NO: 326) corresponding to amino acids 44 - 89 of N43992_P13 (SEQ. ID NO: 57), wherein said first amino acid sequence and second amino acid sequence are contiguous and in a sequential order.

B. An isolated polypeptide encoding for an edge portion of N43992_P13 (SEQ. ID NO: 57), comprising an amino acid sequence being at least 70%, optionally at least about 80%, preferably at least about 85%, more preferably at least about 90% and most preferably at least about 95% homologous to the sequence APLPPLLQSLPEAWALRGGRRVPVRPGRGAECARTGLHRAARSARA (SEQ. ID NO: 326) of N43992_P13 (SEQ. ID NO: 57).

The localization of the variant protein was determined according to results from a number of different software programs and analyses, including analyses from SignalP and other specialized programs. The variant protein is secreted.

Variant protein N43992_P13 (SEQ. ID NO: 57) also has the following non-silent SNPs (Single Nucleotide Polymorphisms) as listed in Table 8, (given according to their position(s) on the amino acid sequence, with the alternative amino acid(s) listed; the last column indicates whether the SNP is known or not; the presence of known SNPs in variant protein N43992_P13 (SEQ. ID NO: 57) sequence provides support for the deduced sequence of this variant protein according to the present invention).

*Table 8 - Amino acid mutations*

| SNP position(s) on amino acid sequence | Alternative amino acid(s) |
|---|---|
| 44 | A -> |

Variant protein N43992_P13 (SEQ. ID NO: 57) is encoded by the following transcript(s): N43992_T1 (SEQ. ID NO:37), for which the coding portion starts at position 71 and ends at position 337. The transcript also has the following SNPs as listed in Table 9 (given according to their position on the nucleotide sequence, with the alternative nucleic acid listed; the last column indicates whether the SNP is known or not; the presence of known SNPs in variant protein N43992_P13 (SEQ. ID NO: 57) sequence provides support for the deduced sequence of this variant protein according to the present invention).

*Table 9 - Nucleic acid SNPs*

| SNP position(s) on nucleotide sequence | Alternative nucleic acid(s) |
|---|---|
| 201 | C -> |
| 509 | C -> |
| 556 | T -> G |
| 587 | C -> G |
| 626 | C -> |
| 694 | T -> C |
| 953 | C -> T |
| 1070 | C -> T |
| 1588 | G -> T |
| 1626 | C -> T |
| 1844 | T -> |
| 1890 | G -> T |

1. Comparison report between N43992_P14 (SEQ. ID NO: 58) and DLL3_HUMAN (SEQ. ID NO: 54):

A. An isolated chimeric polypeptide encoding for N43992_P14 (SEQ. ID NO: 58), comprising a first amino acid sequence being at least 90% homologous to MVSPRMSGLLSQTVILALIFLPQ corresponding to amino acids 1 - 23 of DLL3_HUMAN (SEQ. ID NO: 54), which also corresponds to amino acids 1 - 23 of N43992_P14 (SEQ. ID NO: 58), and a second amino acid sequence being at least 70%, optionally at least 80%, preferably at least 85%, more preferably at least 90% and most preferably at least 95% homologous to a polypeptide having the sequence VRARHGPLASSSCRSTLSGRVQALGPRGPPAAPGSPAASSSESA (SEQ. ID NO: 327) corresponding to amino acids 24 - 67 of N43992_P14 (SEQ. ID NO: 58), wherein said first amino acid sequence and second amino acid sequence are contiguous and in a sequential order.

B. An isolated polypeptide encoding for an edge portion of N43992_P14 (SEQ. ID NO: 58), comprising an amino acid sequence being at least 70%, optionally at least about 80%, preferably at least about 85%, more preferably at least about 90% and most preferably at least about 95% homologous to the sequence VRARHGPLASSSCRSTLSGRVQALGPRGPPAAPGSPAASSSESA (SEQ. ID NO: 327) of N43992_P14 (SEQ. ID NO: 58).

The localization of the variant protein was determined according to results from a number of different software programs and analyses, including analyses from SignalP and other specialized programs. The variant protein is secreted.

Variant protein N43992_P14 (SEQ. ID NO: 58) also has the following non-silent SNPs (Single Nucleotide Polymorphisms) as listed in Table 10, (given according to their position(s) on the amino acid sequence, with the alternative amino acid(s) listed; the last column indicates whether the SNP is known or not; the presence of known SNPs in variant protein N43992_P14 (SEQ. ID NO: 58) sequence provides support for the deduced sequence of this variant protein according to the present invention).

*Table 10 - Amino acid mutations*

| SNP position(s) on amino acid sequence | Alternative amino acid(s) |
|---|---|
| 57 | G -> |

Variant protein N43992_P14 (SEQ. ID NO: 58) is encoded by the following transcript(s): N43992_T4 (SEQ. ID NO:38), for which the coding portion starts at position 71 and ends at position 271. The transcript also has the following SNPs as listed in Table 11 (given according to their position on the nucleotide sequence, with the alternative nucleic acid listed; the last column indicates whether the SNP is known or not; the presence of known SNPs in variant protein N43992_P14 (SEQ. ID NO: 58) sequence provides support for the deduced sequence of this variant protein according to the present invention).

*Table 11 - Nucleic acid SNPs*

| SNP position(s) on nucleotide sequence | Alternative nucleic acid(s) |
|---|---|
| 241 | C -> |
| 549 | C -> |
| 596 | T -> G |
| 627 | C -> G |
| 666 | C -> |
| 734 | T -> C |
| 993 | C -> T |
| 1110 | C -> T |
| 1628 | G -> T |
| 1666 | C -> T |
| 1884 | T -> |
| 1930 | G -> T |

Variant protein N43992_P16 (SEQ. ID NO: 59) according to the present invention is encoded by transcript(s) N43992_T9 (SEQ. ID NO:39). One or more alignments to one or more previously published Delta-like protein 3 precursor (SEQ. ID NO: 54) protein sequences are given in the alignment table on the attached CD-ROM. A brief description of the relationship of the variant protein according to the present invention to each such aligned protein is as follows:

1. Comparison report between N43992_P16 (SEQ. ID NO: 59) and DLL3_HUMAN (SEQ. ID NO: 54):

A. An isolated chimeric polypeptide encoding for N43992_P16 (SEQ. ID NO: 59), comprising a first amino acid sequence being at least 90% homologous to MVSPRMSGLLSQTVILALIFLPQTRPAGVFELQIHSFGPGPGP-GAPRSPCSARLPCRLFFRVCLK PGLSEEAAESPCALGAALSARGPVYTEQPGAPAPDLPLPDGLLQVPFRD-AWPGTFSFIIETWRE ELGDQIGGPAWSLLARVAGRRRLAAGGPWARDIQRAGAWELRFSYRARCEPPAVG-TACTRL CRPRSAPSRCGPGLRPCAPLEDECEAPLVCRAGCSPEHGFCEQPGECRCLEGWTGPLCTVPVS TSSCLSPRGPSSATTGCLVPGPGPCDGNPCANGGSCSETPRSFECTCPRGFYGLRCEVSGVTCA DGPCFNGGLCVGGADPDSAYICHCPPGFQGSNCEKRVDRCSLQPCRNG corresponding to amino acids 1 - 365 of DLL3_HUMAN (SEQ. ID NO: 54), which also corresponds to amino acids 1 - 365 of N43992_P16 (SEQ. ID NO: 59), and a second amino acid sequence being at least 70%, optionally at least 80%, preferably at least 85%, more preferably at least 90% and most preferably at least 95% homologous to a polypeptide having the sequence EAWRPERRGMGWGSWMAQTVQGWNPGFDSSNPRAWGPDLPPASL (SEQ. ID NO: 328) corresponding to amino acids 366 - 409 of N43992_P16 (SEQ. ID NO: 59), wherein said first amino acid sequence and second amino acid sequence are contiguous and in a sequential order.
B. An isolated polypeptide encoding for an edge portion of N43992_P16 (SEQ. ID NO: 59), comprising an amino acid sequence being at least 70%, optionally at least about 80%, preferably at least about 85%, more preferably at least about 90% and most preferably at least about 95% homologous to the sequence EAWRPERRGMGWGSWMAQTVQGWNPGFDSSNPRAWGPDLPPASL (SEQ. ID NO: 328) of N43992_P16 (SEQ. ID NO: 59).

3. Comparison report between N43992_P16 (SEQ. ID NO: 59) and Q8NBS4_HUMAN (SEQ. ID NO: 55)

A. An isolated chimeric polypeptide encoding for N43992_P16 (SEQ. ID NO: 59), comprising a first amino acid sequence being at least 90% homologous to MVSPRMSGLLSQTVILALIFLPQTRPAGVFELQWSFGPGPGP-GAPRSPCSARLPCRLFFRVCLK PGLSEEAAESPCALGAALSARGPVYTEQPGAPAPDLPLPDGLLQVPFRD-AWPGTFSFIIETWRE ELGDQIGGPAWSLLARVAGRRRLAAGGPWARDIQRAGAWELR corresponding to amino acids 1 - 171 of Q8NBS4_HUMAN (SEQ. ID NO: 55), which also corresponds to amino acids 1 - 171 of N43992_P16 (SEQ. ID NO: 59), a bridging amino acid F corresponding to amino acid 172 of N43992_P16 (SEQ. ID NO: 59), a second amino acid sequence being at least 90% homologous to SYRARCEPPAVGTACTRLCRPRSAPSRCGPGLRPCAPLEDECEAP corresponding to amino acids 173 - 217 of Q8NBS4_HUMAN (SEQ. ID NO: 55), which also corresponds to amino acids 173 - 217 of N43992_P16 (SEQ. ID NO: 59), a bridging amino acid L corresponding to amino acid 218 of N43992_P16 (SEQ. ID NO: 59), a third amino acid sequence being at least 90% homologous to VCRAGCSPEHGFCEQPGECRCLEGWTGPLCTVPVSTSSCLSPRGPSSATTGCLVPGPGPCDGN PCANGGSCSETPRSFECTCPRGFYGLRCEVSGVTCA corresponding to amino acids 219 - 317 of Q8NBS4_HUMAN (SEQ. ID NO: 55), which also corresponds to amino acids 219 - 317 of N43992_P16 (SEQ. ID NO: 59), a bridging amino acid D corresponding to amino acid 318 of N43992_P16 (SEQ. ID NO: 59), a fourth amino acid sequence being at least 90% homologous to GPCFNGGLCVGGADPDSAYICHCPPGFQGSNCEKRVDRCSLQPCRNG corresponding to amino acids 319 - 365 of Q8NBS4_HUMAN (SEQ. ID NO: 55), which also corresponds to amino acids 319 - 365 of N43992_P16 (SEQ. ID NO: 59), and a fifth amino acid sequence being at least 70%, optionally at least 80%, preferably at least 85%, more preferably at least 90% and most preferably at least 95% homologous to a polypeptide having the sequence EAWRPERRGMGWGSWMAQTVQGWNPGFDSSNPRAWGPDLPPASL (SEQ. ID NO: 328) corresponding to amino acids 366 - 409 of N43992_P16 (SEQ. ID NO: 59), wherein said first amino acid sequence, bridging amino acid, second amino acid sequence, bridging amino acid, third amino acid sequence, bridging amino acid, fourth amino acid sequence and fifth amino acid sequence are contiguous and in a sequential order.
B. An isolated polypeptide encoding for an edge portion of N43992_P16 (SEQ. ID NO: 59), comprising an amino acid sequence being at least 70%, optionally at least about 80%, preferably at least about 85%, more preferably at least about 90% and most preferably at least about 95% homologous to the sequence EAWRPERRGMGWGSWMAQTVQGWNPGFDSSNPRAWGPDLPPASL (SEQ. ID NO: 328) of N43992_P16 (SEQ. ID NO: 59).

The localization of the variant protein was determined according to results from a number of different software programs and analyses, including analyses from SignalP and other specialized programs. The variant protein is secreted.
Variant protein N43992_P16 (SEQ. ID NO: 59) also has the following non-silent SNPs (Single Nucleotide Polymorphisms) as listed in Table 12, (given according to their position(s) on the amino acid sequence, with the alternative amino acid(s) listed; the last column indicates whether the SNP is known or not; the presence of known SNPs in variant protein N43992_P16 (SEQ. ID NO: 59) sequence provides support for the deduced sequence of this variant protein according to the present invention).

*Table 12 - Amino acid mutations*

| SNP position(s) on amino acid sequence | Alternative amino acid(s) |
|---|---|
| 54 | L -> |
| 156 | G -> |
| 172 | F -> C |
| 195 | S -> |
| 218 | L -> P |

The variant protein has the following domains, as determined by using InterPro. The domains are described in Table 13:

*Table 13 - InterPro domain(s)*

| Domain description | Analysis type | Position(s) on protein |
|---|---|---|
| Type II EGF-like signature | FPrintScan | 274-285, 286-293, 335-345, 346-352 |
| EGF-like | HMMPfam | 278-309, 316-350 |
| EGF-like calcium-binding | HMMSmart | 279-310, 316-351 |
| Type I EGF | HMMSmart | 213-249, 277-310, 315-351 |
| EGF-like, subtype 2 | ProfileScan | 278-309, 316-350 |
| EGF-like | ScanRegExp | 237-248, 298-309, 339-350 |
| Myb, DNA-binding | ScanRegExp | 140-162 |
| EGF-like | ScanRegExp | 237-248, 298-309, 339-350 |

Variant protein N43992_P16 (SEQ. ID NO: 59) is encoded by the following transcript(s): N43992_T9 (SEQ. ID NO:39), for which the coding portion starts at position 71 and ends at position 1297. The transcript also has the following SNPs as listed in Table 14 (given according to their position on the nucleotide sequence, with the alternative nucleic acid listed; the last column indicates whether the SNP is known or not; the presence of known SNPs in variant protein N43992_P16 (SEQ. ID NO: 59) sequence provides support for the deduced sequence of this variant protein according to the present invention).

*Table 14 - Nucleic acid SNPs*

| SNP position(s) on nucleotide sequence | Alternative nucleic acid(s) |
|---|---|
| 230 | C -> |
| 538 | C -> |
| 585 | T -> G |
| 616 | C -> G |
| 655 | C -> |
| 723 | T -> C |
| 982 | C -> T |
| 1099 | C -> T |

As noted above, cluster N43992 features 14 segment(s), which were listed in Table 3 above and for which the sequence(s) are given. These segment(s) are portions of nucleic acid sequence(s) which are described herein separately because they are of particular interest. A description of several segments according to the present invention is now provided.

Segment cluster N43992_N0 (SEQ. ID NO: 40) according to the present invention is supported by 15 libraries. The number of libraries was determined as previously described. This segment can be found in the following transcript (s): N43992_T1 (SEQ. ID NO:37), N43992_T4 (SEQ. ID NO:38) and N43992_T9 (SEQ. ID NO:39). Table 15 below describes the starting and ending position of this segment on each transcript.

*Table 15 - Segment location on transcripts*

| Transcript name | Segment starting position | Segment ending position |
|---|---|---|
| N43992_T1 (SEQ. ID NO:37) | 1 | 139 |
| N43992_T4 (SEQ. ID NO:38) | 1 | 139 |
| N43992_T9 (SEQ. ID NO:39) | 1 | 139 |

Segment cluster N43992_N5 (SEQ. ID NO: 41) according to the present invention is supported by 20 libraries. The number of libraries was determined as previously described. This segment can be found in the following transcript (s): N43992_T1 (SEQ. ID NO:37), N43992_T4 (SEQ. ID NO:38) and N43992_T9 (SEQ. ID NO:39). Table 16 below describes the starting and ending position of this segment on each transcript.

*Table 16 - Segment location on transcripts*

| Transcript name | Segment starting position | Segment ending position |
|---|---|---|
| N43992_T1 (SEQ. ID NO:37) | 198 | 392 |
| N43992_T4 (SEQ. ID NO:38) | 238 | 432 |
| N43992_T9 (SEQ. ID NO:39) | 227 | 421 |

Segment cluster N43992_N12 (SEQ. ID NO: 43) according to the present invention is supported by 22 libraries. The number of libraries was determined as previously described. This segment can be found in the following transcript (s): N43992_T1 (SEQ. ID NO:37), N43992_T4 (SEQ. ID NO:38) and N43992_T9 (SEQ. ID NO:39). Table 17 below describes the starting and ending position of this segment on each transcript.

*Table 17 - Segment location on transcripts*

| Transcript name | Segment starting position | Segment ending position |
|---|---|---|
| N43992_T1 (SEQ. ID NO:37) | 694 | 911 |
| N43992_T4 (SEQ. ID NO:38) | 734 | 951 |
| N43992_T9 (SEQ. ID NO:39) | 723 | 940 |

Segment cluster N43992_N15 (SEQ. ID NO: 45) according to the present invention is supported by 2 libraries. The number of libraries was determined as previously described. This segment can be found in the following transcript (s): N43992_T9 (SEQ. ID NO:39). Table 18 below describes the starting and ending position of this segment on each transcript.

*Table 18 - Segment location on transcripts*

| Transcript name | Segment starting position | Segment ending position |
|---|---|---|
| N43992_T9 (SEQ. ID NO:39) | 1164 | 1866 |

According to an optional embodiment of the present invention, short segments related to the above cluster are also provided. These segments are up to about 120 bp in length, and so are included in a separate description.

Segment cluster N43992_N1 (SEQ. ID NO: 48) according to the present invention is supported by 1 libraries. The number of libraries was determined as previously described. This segment can be found in the following transcript (s): N43992_T4 (SEQ. ID NO:38). Table 19 below describes the starting and ending position of this segment on each transcript.

*Table 19 - Segment location on transcripts*

| Transcript name | Segment starting position | Segment ending position |
|---|---|---|
| N43992_T4 (SEQ. ID NO:38) | 140 | 150 |

Segment cluster N43992_N3 (SEQ. ID NO: 49) according to the present invention is supported by 18 libraries. The number of libraries was determined as previously described. This segment can be found in the following transcript (s): N43992_T1 (SEQ. ID NO:37), N43992_T4 (SEQ. ID NO:38) and N43992_T9 (SEQ. ID NO:39). Table 20 below describes the starting and ending position of this segment on each transcript.

*Table 20 - Segment location on transcripts*

| Transcript name | Segment starting position | Segment ending position |
|---|---|---|
| N43992_T1 (SEQ. ID NO:37) | 140 | 197 |
| N43992_T4 (SEQ. ID NO:38) | 151 | 208 |
| N43992_T9 (SEQ. ID NO:39) | 140 | 197 |

Expression of Homo sapiens delta-like 3 (Drosophila) (DLL3) N43992 transcripts which are detectable by Taqman probes as depicted in sequence names N43992-T4 (SEQ. ID NO: 64) and N43992-T4II (SEQ. ID NO: 68) in normal and cancerous Lung tissues

Expression of Homo sapiens delta-like 3 (Drosophila) (DLL3) transcripts detectable by or according to junction 1-3 was measured by real time PCR with MGB-Taqman probes and primers as follows:

1. Probe: N43992T4 (SEQ. ID NO: 64) and primers Fwd: N43992seg0-1F-TaqDan (SEQ. ID NO: 65) and Rev: N43992seg3R-taq (SEQ. ID NO: 66)

2. Probe: N43992T4II (SEQ. ID NO: 68) and primers Fwd: N43992seg0F-Taq (SEQ. ID NO: 69) and Rev: N43992seg3R-taq (SEQ. ID NO: 66)

In the experiment carried out with probe N43992T4 (SEQ. ID NO: 64) samples 1, 2, 4-20, 22-27, 29-33, 35, 37-41, 51-64, 69, 70, 72, 74-76, 78, 81-85, 89 and 90-92 were undetected. In the experiment carried out with probe N43992T4II (SEQ. ID NO: 68) samples 1, 4-27, 29-33, 35, 37, 38, 40, 41, 51-64, 69, 70, 72, 75, 76, 78, 81-85, 87, 89 and 90-92 were undetected. Undetected samples were assigned a value of 41 and calculated accordingly. In parallel the expression of four housekeeping genes - HPRT1 (GenBank Accession No. NM_000194 (SEQ. ID NO: 5); amplicon - HPRT1-amplicon (SEQ. ID NO: 8)), PBGD (GenBank Accession No. BC019323 (SEQ. ID NO: 1); amplicon - PBGD-amplicon (SEQ. ID NO: 4)), SDHA (GenBank Accession No. NM_004168 (SEQ. ID NO: 33); amplicon - SDHA-amplicon (SEQ. ID NO:36)) and Ubiquitin (GenBank Accession No. BC000449 (SEQ. ID NO: 29); amplicon - Ubiquitin-amplicon (SEQ. ID NO: 32)) was measured by real time PCR with SYBR green detection. For each RT sample, the expression of the above amplicon was normalized to the normalization factor calculted from the expression of these house keeping genes as described in "Real-Time RT-PCR analysis using TaqMan® probes" in the "materials and methods" section. The normalized quantity of each RT sample was then divided by the median of the quantities of the normal samples (sample numbers 51 - 64, 69, 70 Table 1_5 above), to obtain a value of fold up-regulation for each sample relative to median of the normal samples. In the experiment done with probe N43992T4 (SEQ. ID NO: 64) samples 1, 2, 4-20, 22-27, 29-33, 35, 37-41, 51-64, 69, 70, 72, 74-76, 78, 81-85, 89 and 90-92 were undetected.

Figures 7A and 7B are histograms showing over expression of the above-indicated Homo sapiens delta-like 3 (Drosophila) (DLL3) transcripts in cancerous Lung samples relative to the normal samples using the 2 different Taqman probes. Figure 7A - results using N43992T4 (SEQ. ID NO: 64) probe, Figure 7B - results using N43992T4II (SEQ. ID NO: 68) probe.

As is evident from Figures 7A and 7B the expression of Homo sapiens delta-like 3 (Drosophila) (DLL3) transcripts detectable by the above primers and probes in small cell carcinoma samples was significantly higher than in the non-cancerous samples (sample numbers 51 - 64, 69, 70 Table 1_5 above). Notably an over-expression of at least 5 fold was found in 8 out of 9 small cells carcinoma samples and 6 out of 57 non-small cell carcinoma samples (in 4 out of 23 squamous cell carcinoma samples and 2 out of 10 large cell carcinoma samples) when experiment was carried out with N43992T4 (SEQ. ID NO: 64) probe and over-expression of at least 5 fold was found in 9 out of 9 small cell carcinoma samples and 5 out of 57 non-small cell carcinoma samples (1 out of 24 adenocarcinoma samples, 2 out of 23 squamous cell carcinoma samples and 2 out of 10 large cell carcinoma samples) when experiment was carried out with N43992T4II (SEQ. ID NO: 68) probe.

Statistical analysis was applied to verify the significance of these results, as described below.

The P value for the difference in the expression levels of Homo sapiens delta-like 3 (Drosophila) (DLL3) transcripts detectable probes N43992T4 (SEQ. ID NO: 64) and N43992T4II (SEQ. ID NO: 68) in Lung small cell carcinoma samples versus the normal tissue samples was determined by T test as 1.76e-03 and 2.06e-03, respectively.

Threshold of 5 fold over expression of Homo sapiens delta-like 3 (Drosophila) (DLL3) transcripts detectable by probes N43992T4 (SEQ. ID NO: 64) and N43992T4II (SEQ. ID NO: 68) was found to differentiate between small cell carcinoma and normal samples with P value of 8.32e-06 and 4.89e-07, respectively, as checked by exact Fisher test.

The above values demonstrate statistical significance of the results.

Primer pairs and probes are also optionally and preferably encompassed within the present invention; for example, for the above experiment, the following primer pairs and probes were used as a non-limiting illustrative example only of a suitable primer pairs:

1. Probe: N43992T4 (SEQ. ID NO: 64) and primers Fwd: N43992seg0-1F-TaqDan (SEQ. ID NO: 65) and Rev: N43992seg3R-taq (SEQ. ID NO: 66)

2. Probe: N43992T4II (SEQ. ID NO: 68) and primers Fwd: N43992seg0F-Taq (SEQ. ID NO: 69) and Rev: N43992seg3R-taq (SEQ. ID NO: 66)

The present invention also preferably encompasses any amplicon obtained through the use of any suitable primer pair; for example, for the above experiment, the following amplicon was obtained as a non-limiting illustrative example only of a suitable amplicon: N43992_junc1-3 (SEQ. ID NO: 67), and N43_992junc1-3II (SEQ. ID NO: 70).

1. TaqMan¨ MGB probe: Name: N43992T4 (SEQ. ID NO: 64) - FAM-AGCCAGACACGGCC-BQ
Forward Primer: N43992seg0-1F-TaqDan (SEQ. ID NO: 65) - TCATTTTCCTCCCCCAGGTC
Reverse Primer: N43992seg3R-taq (SEQ. ID NO: 66) - GTGGATCTGCAGCTCGAAGAC
Amplicon N43992_junc1-3 (SEQ. ID NO: 67):

TCATTTTCCTCCCCCAGGTCAGAGCCAGACACGGCCCGCTGGCGTCTTCGAGCTGCAGAT
CCAC

2. TaqMan¨ MGB probe: Name: N43992T4II (SEQ. ID NO: 68) - FAM- TCAGAGCCAGACACGG-BQ
Forward Primer: N43992seg0F-Taq (SEQ. ID NO: 69) - TCCTCTCCCAGACTGTGATCCT
Reverse Primer: N43992seg3R-taq (SEQ. ID NO: 66) - GTGGATCTGCAGCTCGAAGAC
Amplicon N43992_junc1-3II (SEQ. ID NO: 70):

TCCTCTCCCAGACTGTGATCCTAGCGCTCATTTTCCTCCCCCAGGTCAGAGCCAGACACGG
CCCGCTGGCGTCTTCGAGCTGCAGATCCAC

Expression of Homo sapiens delta-like 3 (Drosophila) (DLL3) N43992 transcripts which are detectable by amplicon as depicted in sequence name N43992_seg12WTF2R2 (SEQ. ID NO: 62) in normal and cancerous Lung tissues

Expression of Homo sapiens delta-like 3 (Drosophila) (DLL3) transcripts detectable by or according to seg12WTF2R2 - N43992_seg12WTF2R2 (SEQ. ID NO: 62) amplicon and primers N43992_seg12WTF2 (SEQ. ID NO: 60) and N43992_seg12WTR2 (SEQ. ID NO: 61), including but not limited to the known DLL3 transcript SEQ. ID NOs: 74, 75, was measured by real time PCR. In parallel the expression of four housekeeping genes - HPRT1 (GenBank Accession No. NM_000194 (SEQ. ID NO: 5); amplicon - HPRT1-amplicon (SEQ. ID NO: 8), PBGD (GenBank Accession No. BC019323 (SEQ. ID NO: 1); amplicon - PBGD-amplicon (SEQ. ID NO: 4), SDHA (GenBank Accession No. NM_ 004168 (SEQ. ID NO: 33); amplicon - SDHA-amplicon (SEQ. ID NO:36) and Ubiquitin (GenBank Accession No. BC000449 (SEQ. ID NO: 29); amplicon - Ubiquitin-amplicon (SEQ. ID NO: 32) was measured similarly. For each RT sample, the expression of the above amplicon was normalized to the geometric mean of the quantities of the housekeeping genes. The normalized quantity of each RT sample was then divided by the median of the quantities of the normal post-mortem (PM) samples (sample numbers 47, 48, 49, 50, 90, 91, 92, 93, 96, 97 and 98, Table 1_2 above), to obtain a value of fold up-regulation for each sample relative to median of the normal PM samples.

Figure 8A is a histogram showing over expression of the above-indicated Homo sapiens delta-like 3 (Drosophila) (DLL3) transcripts in cancerous Lung samples relative to the normal samples.

As is evident from Figure 8A, the expression of Homo sapiens delta-like 3 (Drosophila) (DLL3) transcripts detectable by the above amplicon in small cell carcinoma samples was significantly higher than in the non-cancerous samples (sample numbers 47, 48, 49, 50, 90, 91, 92, 93, 96, 97 and 98, Table 1_2 above) and was higher in a few non-small cell carcinoma samples than in the non-cancerous samples. Notably an over-expression of at least 5 fold was found in 8 out of 8 small cell carcinoma samples, and in 8 out of 27 non-small cell carcinoma samples, specifically in 5 out of 13 squamous cell carcinoma samples.

Statistical analysis was applied to verify the significance of these results, as described below.

The P value for the difference in the expression levels of Homo sapiens delta-like 3 (Drosophila) (DLL3) transcripts detectable by the above amplicon in Lung small cell carcinoma samples versus the normal tissue samples was determined by T test as 1.21 e-03. The P value for the difference in the expression levels of Homo sapiens delta-like 3 (Drosophila) (DLL3) transcripts detectable by the above amplicon in Lung non-small cell carcinoma samples versus the normal tissue samples was determined by T test as 1.76e-02. The P value for the difference in the expression levels of Homo sapiens delta-like 3 (Drosophila) (DLL3) transcripts detectable by the above amplicon in Lung squamous cell carcinoma samples versus the normal tissue samples was determined by T test as 4.58e-02.

Threshold of 5 fold over expression was found to differentiate between small cell carcinoma and normal samples with P value of 5.95e-04 as checked by exact Fisher test.

The above values demonstrate statistical significance of the results.

Primer pairs are also optionally and preferably encompassed within the present invention; for example, for the above experiment, the following primer pair was used as a non-limiting illustrative example only of a suitable primer pair: N43992_seg12WTF2 (SEQ. ID NO: 60) forward primer; and N43992_seg12WTR2 (SEQ. ID NO: 61) reverse primer.

The present invention also preferably encompasses any amplicon obtained through the use of any suitable primer pair; for example, for the above experiment, the following amplicon was obtained as a non-limiting illustrative example only of a suitable amplicon: N43992_seg12WTF2R2 (SEQ. ID NO:62).

Forward Primer (N43992_seg12WTF2 (SEQ. ID NO: 60)) -TGTGAACAGCCCGGTGAA

Reverse Primer (N43992_seg12WTR2 (SEQ. ID NO: 61)) -GACAAGGCATCCGGTGGTAG

Amplicon (N43992_seg12WTF2R2 (SEQ. ID NO: 62))

TGTGAACAGCCCGGTGAATGCCGATGCCTAGAGGGCTGGACTGGACCCCTCTGCAC
GGTCCCTGTCTCCACCAGCAGCTGCCTCAGCCCCAGGGGCCCGTCCTCTGCTACCACCGG
ATGCCTTGTC

Expression of Homo sapiens delta-like 3 (Drosophila) (DLL3) N43992 transcripts which are detectable by Taqman probe as depicted in sequence names N43992T3 (SEQ. ID NO: 71) in normal and cancerous Lung tissues

Expression of Homo sapiens delta-like 3 (Drosophila) (DLL3) transcripts detectable by or according to junction0-3 was measured by real time PCR with MGB-Taqman probe N43992T3 (SEQ. ID NO: 71) and primers N43992seg0F-Taq (SEQ. ID NO: 69) and N43992seg3WT_R-Taq (SEQ. ID NO: 72). Samples 4, 6, 14, 16-18, 24, 51-57, 60-62, 64, 69, 70, 72, 75, 78, 81-83 and 89 were undetected. These samples were assigned a value of 41 and calculated accordingly. In parallel the expression of four housekeeping genes - HPRT1 (GenBank Accession No. NM_000194 (SEQ. ID NO: 5); amplicon - HPRT1-amplicon (SEQ. ID NO: 8)), PBGD (GenBank Accession No. BC019323 (SEQ. ID NO: 1); amplicon - PBGD-amplicon (SEQ. ID NO: 4)), SDHA (GenBank Accession No. NM_004168 (SEQ. ID NO: 33); amplicon - SDHA-amplicon (SEQ. ID NO:36)) and Ubiquitin (GenBank Accession No. BC000449 (SEQ. ID NO: 29); amplicon - Ubiquitin-amplicon (SEQ. ID NO: 32)) was measured by real time PCR with SYBR green detection. For each RT sample, the expression of the above amplicon was normalized to the normalization factor calculted from the expression of these house keeping genes as described in "Real-Time RT-PCR analysis using TaqMan® probes" in the "materials and methods" section. The normalized quantity of each RT sample was then divided by the median of the quantities of the normal samples (sample numbers 51 - 64, 69, 70 Table 1_5 above), to obtain a value of fold up-regulation for each sample relative to median of the normal samples.

Figure 8B is a histogram showing over expression of the above-indicated Homo sapiens delta-like 3 (Drosophila) (DLL3) transcripts in cancerous Lung samples relative to the normal samples.

As is evident from Figure 8B, the expression of Homo sapiens delta-like 3 (Drosophila) (DLL3) transcripts detectable by the above primers and probe in small cell carcinoma samples was significantly higher than in the non-cancerous samples (sample numbers 51 - 64, 69, 70 Table 1_5 above). Notably an over-expression of at least 250 fold was found in 9 out of 9 small cell carcinoma samples and in 8 out 57 non-small cell carcinoma samples (2 out of 24 adenocarcinoma samples, 4 out of 23 squamous cell carcinoma samples and 2 out of 10 large cell carcinoma samples).

Statistical analysis was applied to verify the significance of these results, as described below.

The P value for the difference in the expression levels of Homo sapiens delta-like 3 (Drosophila) (DLL3) transcripts detectable probe N43992T3 (SEQ. ID NO: 71) in Lung small cell carcinoma samples and non-small cell carcinoma versus the normal tissue samples was determined by T test as 3.41e-04 and 9.52e-03, respectively.

Threshold of 250 fold over expression of Homo sapiens delta-like 3 (Drosophila) (DLL3) transcripts detectable by probe N43992T3 (SEQ. ID NO: 71) was found to differentiate between small cell carcinoma and normal samples with P value of 4.89e-07, as checked by exact Fisher test.

The above values demonstrate statistical significance of the results.

Primer pairs and probes are also optionally and preferably encompassed within the present invention; for example, for the above experiment, the following primer pairs and probes were used as a non-limiting illustrative example only of a suitable primer pairs: Probe: N43992T3 (SEQ. ID NO: 71) and primers Fwd: N43992seg0F-Taq (SEQ. ID NO: 69) and Rev: N43992seg3WT_R-Taq (SEQ. ID NO: 72)

The present invention also preferably encompasses any amplicon obtained through the use of any suitable primer pair; for example, for the above experiment, the following amplicon was obtained as a non-limiting illustrative example only of a suitable amplicon: N43992_junc0-3 (SEQ. ID NO: 73)

TaqMan¨ MGB probe: Name: N43992T3 (SEQ. ID NO: 71)- VIC- CCTCCCCCAGACACG-BQ
Forward Primer: N43992seg0F-Taq (SEQ. ID NO: 69) - TCCTCTCCCAGACTGTGATCCT
Reverse Primer: N43992seg3WT_R-Taq (SEQ. ID NO: 72) - ACCCGGCCCGAAAGAGT
Amplicon N43992_junc0-3 (SEQ. ID NO: 73):

TCCTCTCCCAGACTGTGATCCTAGCGCTCATTTTCCTCCCCCAGACACGGCCCGCT
GGCGTCTTCGAGCTGCAGATCCACTCTTTCGGGCCGGGT

Expression of Homo sapiens delta-like 3 (Drosophila) (DLL3) N43992 transcripts which are detectable by Taqman probe as depicted in sequence names N43992-T4 (SEQ. ID NO: 64) in different normal tissues

Expression of Homo sapiens delta-like 3 (Drosophila) (DLL3) transcripts detectable by or according to junction1-3 was measured by real time PCR with MGB-Taqman probe N43992T4 (SEQ. ID NO: 64) and primers: Fwd: N43992seg0-1F-TaqDan (SEQ. ID NO: 65) and Rev: N43992seg3R-taq (SEQ. ID NO: 66). Samples 1-14, 16-23, 25-27, 28-34, 36, 38-45, 49-54, 56-59 and 65-73 were undetected. These samples were assigned a value of 41 and calculated accordingly. In parallel the expression of three housekeeping genes - SDHA (GenBank Accession No. NM_004168 (SEQ. ID NO: 33); amplicon - SDHA-amplicon (SEQ. ID NO:36)), Ubiquitin (GenBank Accession No. BC000449 (SEQ. ID NO: 29); amplicon - Ubiquitin-amplicon (SEQ. ID NO: 32)), and TATA box (GenBank Accession No. NM_003194 (SEQ. ID NO: 25); TATA amplicon (SEQ. ID NO: 28)) was measured by real time PCR with SYBR green detection. For each RT sample, the expression of the above amplicon was normalized to the normalization factor calculted from the expression of these house keeping genes as described in "Real-Time RT-PCR analysis using TaqMan® probes" in the "materials and methods" section. The normalized quantity of each RT sample was then divided by the median of the quantities of the lung samples (sample numbers 28, 29 and 30, Table 1_7 above), to obtain a value of relative expression of each sample relative to median of the Lung samples.
TaqMan¨ MGB probe: Name: N43992T4II (SEQ. ID NO: 68): FAM- TCAGAGCCAGACACGG-BQ
Forward Primer: N43992seg0F-Taq (SEQ. ID NO: 69) TCCTCTCCCAGACTGTGATCCT
Reverse Primer: N43992seg3R-taq (SEQ. ID NO: 66) GTGGATCTGCAGCTCGAAGAC
Amplicon (SEQ. ID NO: 70):

TCCTCTCCCAGACTGTGATCCTAGCGCTCATTTTCCTCCCCCAGGTCAGAGCCAGACACGG
CCCGCTGGCGTCTTCGAGCTGCAGATCCAC

Figure 9 is a histogram showing expression of Homo sapiens delta-like 3 (Drosophila) (DLL3) N43992 transcripts which are detectable by Taqman probe as depicted in sequence names N43992-T4 (SEQ. ID NO: 64) in different normal tissues.

Expression of Homo sapiens delta-like 3 (Drosophila) (DLL3) N43992 transcripts which are detectable by amplicon as depicted in sequence name N43992_seg12WTF2R2 (SEQ. ID NO: 62) in different normal tissues

Expression of Homo sapiens delta-like 3 (Drosophila) (DLL3) transcripts detectable by or according to seg12WTF2R2 - N43992_seg12WTF2R2 (SEQ. ID NO: 62) amplicon and primers N43992_seg12WTF2 (SEQ. ID NO:

60) and N43992_seg12WTR2 (SEQ. ID NO: 61), including but not limited to the known DLL3 transcript SEQ. ID NOs: 74, 75, was measured by real time PCR. In parallel the expression of four housekeeping genes - SDHA (GenBank Accession No. NM_004168 (SEQ. ID NO: 33); amplicon - SDHA-amplicon (SEQ. ID NO:36), Ubiquitin (GenBank Accession No. BC000449 (SEQ. ID NO: 29); amplicon - Ubiquitin-amplicon (SEQ. ID NO: 32), RPL19 (GenBank Accession No. NM_000981 (SEQ. ID NO: 21); RPL19 amplicon (SEQ. ID NO: 24) and TATA box (GenBank Accession No. NM_ 003194 (SEQ. ID NO: 25); TATA amplicon (SEQ. ID NO: 28) was measured similarly. For each RT sample, the expression of the above amplicon was normalized to the geometric mean of the quantities of the housekeeping genes. The normalized quantity of each RT sample was then divided by the median of the quantities of the lung samples (sample numbers 15 and 17, Table 1_6 above), to obtain a value of relative expression of each sample relative to median of the lung samples.
Forward Primer (N43992_seg12WTF2 (SEQ. ID NO: 60):TGTGAACAGCCCGGTGAA
Reverse Primer (N43992_seg12WTR2 (SEQ. ID NO: 61):GACAAGGCATCCGGTGGTAG
Amplicon (N43992_seg12WTF2R2 (SEQ. ID NO: 62):

TGTGAACAGCCCGGTGAATGCCGATGCCTAGAGGGCTGGACTGGACCCCTCTGCAC
GGTCCCTGTCTCCACCAGCAGCTGCCTCAGCCCCAGGGGCCCGTCCTCTGCTACCACCGG
ATGCCTTGTC

Figure 10A is a histogram showing over expression of the Homo sapiens delta-like 3 (Drosophila) (DLL3) N43992 transcripts which are detectable by amplicon as depicted in sequence name N43992_seg12WTF2R2 (SEQ. ID NO: 62) in different normal tissues.

Expression of Homo sapiens delta-like 3 (Drosophila) (DLL3) N43992 transcripts which are detectable by Taqman probe as depicted in sequence names N43992T3 (SEQ. ID NO: 71) in different normal tissues.

Expression of Homo sapiens delta-like 3 (Drosophila) (DLL3) transcripts detectable by or according to junction0-3 was measured by real time PCR with MGB-Taqman probe N43992T3 (SEQ. ID NO: 71) and primers: N43992seg0F-Taq (SEQ. ID NO: 69) and N43992seg3WT_R-Taq (SEQ. ID NO: 72). Samples 9-14, 16-21, 23, 25, 28, 30-34, 36, 38-43, 45, 49, 51-54, 59, 66-69, 72 and 73 were undetected. These samples were assigned a value of 41 and calculated accordingly. In parallel the expression of three housekeeping genes - SDHA (GenBank Accession No. NM_004168 (SEQ. ID NO: 33); amplicon - SDHA-amplicon (SEQ. ID NO:36)), Ubiquitin (GenBank Accession No. BC000449 (SEQ. ID NO: 29); amplicon - Ubiquitin-amplicon (SEQ. ID NO: 32)), and TATA box (GenBank Accession No. NM_003194 (SEQ. ID NO: 25); TATA amplicon (SEQ. ID NO: 28)) was measured by real time PCR with SYBR green detection. For each RT sample, the expression of the above amplicon was normalized to the normalization factor calculted from the expression of these house keeping genes as described in "Real-Time RT-PCR analysis using TaqMan® probes" in the "materials and methods" section. The normalized quantity of each RT sample was then divided by the median of the quantities of the lung samples (sample numbers 28, 29 and 30, Table 1_7 above), to obtain a value of relative expression of each sample relative to median of the Lung samples. TaqMan¨ MGB probe: Name: N43992T3 (SEQ. ID NO: 71) VIC- CCTCCCCCAGACACG-BQ Forward Primer: N43992seg0F-Taq (SEQ. ID NO: 69) - TCCTCTCCCAGACTGTGATCCT Reverse Primer: N43992seg3WT_R-Taq (SEQ. ID NO: 72) - ACCCGGCCCGAAAGAGT Amplicon (SEQ. ID NO: 73):

TCCTCTCCCAGACTGTGATCCTAGCGCTCATTTTCCTCCCCCAGACACGGCCCGCTGGCGT
CTTCGAGCTGCAGATCCACTCTTTCGGGCCGGGT

Figure 10B is a histogram showing expression of Homo sapiens delta-like 3 (Drosophila) (DLL3) N43992 transcripts which are detectable by Taqman probe as depicted in sequence names N43992T3 (SEQ. ID NO: 71) in different normal tissues.

DESCRIPTION FOR CLUSTER D12115

Cluster D12115 features 11 transcript(s) and 42 segment(s) of interest, the names for which are given in Tables 21 and 22, respectively. The selected protein variants are given in table 23.

*Table 21 - Transcripts of interest*

| Transcript Name |
|---|
| D12115_T3 (SEQ. ID NO:78) |
| D12115_T5 (SEQ. ID NO:79) |
| D12115_T9 (SEQ.ID NO:80) |
| D12115_T12 (SEQ. ID NO:81) |
| D12115_T13 (SEQ. ID NO:82) |
| D12115_T14 (SEQ. ID NO:83) |
| D12115_T19 (SEQ. ID NO:84) |
| D12115_T22 (SEQ. ID NO:85) |
| D12115_T27 (SEQ. ID NO:86) |
| D12115_T33 (SEQ. ID NO:87) |
| D12115_T36 (SEQ. ID NO:88) |

*Table 22 - Segments of interest*

| Segment Name |
|---|
| D12115_N0 (SEQ. ID NO:89) |
| D12115_N2 (SEQ. ID NO:90) |
| D12115_N4 (SEQ. ID NO:91) |
| D12115_N5 (SEQ. ID NO:92) |
| D12115_N6 (SEQ. ID NO:93) |
| D12115_N7 (SEQ. ID NO:94) |
| D12115_N26 (SEQ. ID NO:95) |
| D12115_N27 (SEQ. ID NO:96) |
| D12115_N34 (SEQ. ID NO:97) |
| D12115_N41 (SEQ. ID NO:98) |
| D12115_N53 (SEQ.ID NO:99) |
| D12115_N3 (SEQ. ID NO:100) |
| D12115_N11 (SEQ. ID NO:101) |
| D12115_N12 (SEQ. ID NO:102) |
| D12115_N14 (SEQ. ID NO:103) |
| D12115_N15 (SEQ. ID NO:104) |
| D12115_N16 (SEQ. ID NO:105) |
| D12115_N17 (SEQ. ID NO:106) |
| D12115_N20 (SEQ. ID NO:107) |
| D12115_N21 (SEQ.ID NO:108) |
| D12115_N22 (SEQ. ID NO:109) |
| D12115_N23 (SEQ. ID NO:110) |
| D12115_N24 (SEQ. ID NO:111) |
| D12115_N28 (SEQ. ID NO:112) |

(continued)

| Segment Name |
|---|
| D12115_N29 (SEQ. ID NO:113) |
| D12115_N31 (SEQ. ID NO:114) |
| D12115_N32 (SEQ. ID NO:115) |
| D12115_N33 (SEQ. ID NO:116) |
| D12115_N36 (SEQ. ID NO:117) |
| D12115_N38 (SEQ. ID NO:118) |
| D12115_N39 (SEQ. ID NO:119) |
| D12115_N43 (SEQ. ID NO:120) |
| D12115_N45 (SEQ. ID NO:121) |
| D12115_N46 (SEQ. ID NO:122) |
| D12115_N47 (SEQ. ID NO:123) |
| D12115_N48 (SEQ. ID NO:124) |
| D12115_N49 (SEQ. ID NO:125) |
| D12115_N50 (SEQ. ID NO:126) |
| D12115_N52 (SEQ. ID NO:127) |
| D12115_N54 (SEQ. ID NO:128) |
| D12115_N55 (SEQ. ID NO:129) |
| D12115_N56 (SEQ. ID NO:130) |

*Table 23 - Proteins of interest*

| Protein Name | Corresponding Transcript(s) |
|---|---|
| D12115_P3 (SEQ. ID NO:134) | D12115_T3 (SEQ. ID NO:78) |
| D12115_P5 (SEQ. ID NO:135) | D12115_T36 (SEQ. ID NO:88); D12115_T5 (SEQ. ID NO:79) |
| D12115_P12 (SEQ. ID NO:136) | D12115_T12 (SEQ. ID NO:81) |
| D12115_P13 (SEQ. ID NO:137) | D12115_T13 (SEQ. ID NO:82) |
| D12115_P15(SEQ. ID NO:138) | D12115_T19 (SEQ. ID NO:84) |
| D12115_P16 (SEQ. ID NO:139) | D12115_T22 (SEQ. ID NO:85) |
| D12115_P20 (SEQ. ID NO:140) | D12115_T27 (SEQ. ID NO:86) |
| D12115_P32 (SEQ. ID NO:141) | D12115_T33 (SEQ. ID NO:87) |
| D12115_P34 (SEQ. ID NO:142) | D12115_T14 (SEQ. ID NO:83) |
| D12115_P35 (SEQ. ID NO:143) | D12115_T9 (SEQ. ID NO:80) |

These sequences are variants of the known protein Complement factor B precursor (SEQ. ID NO:131) (Swiss-Prot accession identifier CFAB_HUMAN (SEQ. ID NO: 395); known also according to the synonyms EC 3.4.21.47; C3/C5 convertase; Properdin factor B; Glycine-rich beta glycoprotein; GBG; PBF2), referred to herein as the previously known protein.

The variant D12115_P3 (SEQ. ID NO:134) was previously disclosed by the inventors in published PCT application no WO2005/071058, and the variants D12115_P12 (SEQ. ID NO:136) and D12115_P16 (SEQ. ID NO:139) were previously disclosed by the inventors in published PCT applications no WO2005/071058 and WO2004/096979, hereby incorporated by reference as if fully set forth herein, but have now been shown to have novel and surprising diagnostic uses as described herein for other variants of cluster D12115.

Protein Complement factor B precursor (SEQ. ID NO:131) is known or believed to have the following function (s): Factor B which is part of the alternate pathway of the complement system is cleaved by factor D into 2 fragments: Ba and Bb. Bb, a serine protease, then combines with complement factor 3b to generate the C3 or C5 convertase. It has also been implicated in proliferation and differentiation of preactivated B lymphocytes, rapid spreading of peripheral blood monocytes, stimulation of lymphocyte blastogenesis and lysis of erythrocytes. Ba inhibits the proliferation of preactivated B lymphocytes. Known polymorphisms for this sequence are as shown in Table 24.

*Table 24 - Amino acid mutations for Known Protein*

| SNP position(s) on amino acid sequence | Comment |
|---|---|
| 9 | L -> H. /FTId=VAR_016274 |
| 28 | W -> R (in allele S). /FTId=VAR_006492 |
| 28 | W -> Q (in allele FA; requires 2 nucleotide substitutions). /FTId=VAR_006493 |
| 32 | R -> Q (in allele S). /FTId=VAR_006494 |
| 32 | R -> W. /FTId=VAR_016275 |
| 252 | G -> S. /FTId=VAR_016276 |
| 565 | K -> E. /FTId=VAR 016277 |
| 651 | D -> E. /FTId=VAR_016278 |
| 736 | A -> S (in allele FA). /FTId=VAR_006495 |
| 297 | I -> T |
| 300 | V -> L |
| 328 | D -> V |
| 356-357 | KK -> EE |
| 537 | I -> T |
| 764 | L -> H |

Protein Complement factor B precursor (SEQ. ID NO:131) localization is believed to be Secreted.

The previously known protein also has the following indication(s) and/or potential therapeutic use(s): Infection, general; Traumatic shock. It has been investigated for clinical/therapeutic use in humans, for example as a target for an antibody or small molecule, and/or as a direct therapeutic; available information related to these investigations is as follows. Potential pharmaceutically related or therapeutically related activity or activities of the previously known protein are as follows: Complement factor inhibitor. A therapeutic role for a protein represented by the cluster has been predicted. The cluster was assigned this field because there was information in the drug database or the public databases (e.g., described herein above) that this protein, or part thereof, is used or can be used for a potential therapeutic indication: Anti-inflammatory; Cardiovascular; Immunosuppressant; Neuroprotective; Recombinant, other; Septic shock treatment.

According to optional but preferred embodiments of the present invention, variants of this cluster according to the present invention (amino acid and/or nucleic acid sequences of D12115) may optionally have one or more of the following utilities, as described with regard to the Table below. It should be noted that these utilities are optionally and preferably suitable for human and non-human animals as subjects, except where otherwise noted. The reasoning is described with regard to biological and/or physiological and/or other information about the known protein, but is given to demonstrate particular diagnostic utility for the variants according to the present invention.

Table of Utilities for Variants of D12115, related to protein Complement factor B precursor (SEQ. ID NO:131):

Table 25

| Utility | Reason | Reference |
|---|---|---|
| Complement factor B allotypes in the susceptibility and severity of coeliac disease. | | 16164698 |
| diagnosis of Alzheimer’s disease (AD), Parkinson’s disease (PD), and multiple sclerosis (MS) in the CSF, by a verity of alternative splice isoforms. | | 15920296 |

(continued)

| Utility | Reason | Reference |
|---|---|---|
| diagnosis of ischemic acute tubular necrosis, in serum | | 15673300 |
| Complement factor B allotypes in the susceptibility of Chagas disease | | 12974797 |

According to other optional embodiments of the present invention, variants of this cluster according to the present invention (amino acid and/or nucleic acid sequences of D12115) may optionally have one or more of the following utilities, some of which are related to utilities described above. It should be noted that these utilities are optionally and preferably suitable for human and non-human animals as subjects, except where otherwise noted.

The Table below describes diagnostic utilities for the cluster D12115 that were found through microarrays, including the statistical significance thereof and a reference. One or more D12115 variants according to the present invention may optionally have one or more of these utilities.

Table 26

| | |
|---|---|
| Differential diagnosis of in situ prostates cancer vs. metastasis (lower expression in metastasis). | GSE3325 |

According to further optional but preferred embodiments of the present invention, variants of this cluster according to the present invention (amino acid and/or nucleic acid sequences of D12115) may optionally have one or more of the following utilities, as described in greater detail below, which may also optionally be related to one or more of the above utilities. It should be noted that these utilities are optionally and preferably suitable for human and non-human animals as subjects, except where otherwise noted. The reasoning is described with regard to biological and/or physiological and/or other information about the known protein, but is given to demonstrate particular diagnostic utility for the variants according to the present invention.

A non-limiting example of such a utility is the detection, diagnosis and/or determination of any condition that includes activation of the alternative complement pathway. For diagnostic utilities related to the activated form chain Bb, only variants T19 and T33,36 of the present invention are appropriate. The method comprises detecting a D12115 variant, for example a variant protein, protein fragment, peptide, polynucleotide, polynucleotide fragment and/or oligonucleotide as described herein, optionally and preferably in a serum sample. The expression levels of the D12115 variant as determined in a patient can be further compared to those in a normal individual.

For example, the known Protein Complement factor B has been shown to be useful for diagnosis of complement related immune disease, including but not limited to vasculitis, systemic lupus erythematosus, rheumatoid arthritis, myocardial infarction, ischemic/ reperfusion injury, cerebrovascular accident, Alzheimer's disease, transplantation rejection (xeno and allo), all antibody-mediated skin diseases, all antibody-mediated organ-specific diseases (including Type I and Type 11 diabetes mellitus, thyroiditis, idiopathic thrombocytopenic purpura and hemolytic anemia, and neuropathies), multiple sclerosis, cardiopulmonary bypass injury, membranoproliferative glomerulonephritis, polyarteritis nodosa, Henoch Schonlein purpura, serum sickness, Goodpasture's disease, systemic necrotizing vasculitis, post streptococcal glomerulonephritis, idiopathic pulmonary fibrosis (usual interstitial pneumonitis) and membranous glomerulonephritis; breast, ovarian and prostate cancer; ovarian malignant hyperplasia.

Antibodies recognizing the known Protein Complement factor B are described as being useful for recognizing proteins in the serum of breast cancer patients that are differentially present with regard to PCT Application No. WO 02/088750, hereby incorporated by reference as if fully set forth herein.

Differential expression of the known Protein Complement factor B in prostate cancer tissues is described with regard to PCT Application No. WO 00/055174, hereby incorporated by reference as if fully set forth herein.

Differential expression of the known Protein Complement factor B in ovarian cancer tissues is described with regard to US Patent Application No. US2005095592, hereby incorporated by reference as if fully set forth herein.

Differential expression of the known Protein Complement factor B in ovarian malignant hyperplasia tissues is described with regard to US Patent No. 6316213, hereby incorporated by reference as if fully set forth herein.

Yet another non-limiting example of a utility is described in PCT Application No. WO 98/32390, hereby incorporated by reference as if fully set forth herein, for distinguishing bacterial meningitis from viral meningitis according to levels of complement B in cerebro-spinal fluid samples.

Yet another non-limiting example of a utility is described in PCT Application No. WO 04/055519, hereby incorporated by reference as if fully set forth herein, in which complement factor B was shown to be upregulated in pancreatic cancer tissue samples.

Yet another non-limiting example of a utility is described in US Patent No. 6335170, hereby incorporated by reference as if fully set forth herein, in which expression of complement factor B was shown to be upregulated in bladder cancer tissue samples.

The following GO Annotation(s) apply to the previously known protein. The following annotation(s) were found: complement activation, alternative pathway, which are annotation(s) related to Biological Process; and complement binding, which are annotation(s) related to Molecular Function.

The GO assignment relies on information from one or more of the SwissProt/TremBl Protein knowledgebase, available from <http://www.expasy.ch/sprot/>; or Locuslink, available from <http://www.ncbi.nlm.nih.gov/projects/LocusLink/>.

Other non-limiting exemplary utilities for D12115 variants according to the present invention are described in greater detail below and also with regard to the previous section on clinical utility.

Cluster D12115 can be used as a diagnostic marker according to overexpression of transcripts of this cluster in cancer. Expression of such transcripts in normal tissues is also given according to the previously described methods. The term "number" in the left hand column of the table and the numbers on the y-axis of the figure below refer to weighted expression of ESTs in each category, as "parts per million" (ratio of the expression of ESTs for a particular cluster to the expression of all ESTs in that category, according to parts per million).

Overall, the following results were obtained as shown with regard to the histograms in Figure 11 and Table 27. This cluster is overexpressed (at least at a minimum level) in the following pathological conditions: ovarian carcinoma, prostate cancer, a mixture of malignant tumors from different tissues, uterine malignancies and epithelial malignant tumors.

*Table 27 - Normal tissue distribution*

| Name of Tissue | Number |
|---|---|
| brain | 31 |
| ovary | 0 |
| bladder | 123 |
| lung | 93 |
| pancreas | 42 |
| liver | 2675 |
| prostate | 1 |
| adrenal | 0 |
| general | 85 |
| Thyroid | 0 |
| uterus | 22 |
| colon | 141 |
| kidney | 65 |
| breast | 17 |
| stomach | 147 |
| epithelial | 159 |
| bone | 189 |

*Table 28 - P values and ratios for expression in cancerous tissue*

| Name of Tissue | P1 | P2 | SP1 | R3 | SP2 | R4 |
|---|---|---|---|---|---|---|
| brain | 6.3e-01 | 8.0e-01 | 9.4e-01 | 0.6 | 1.0e+00 | 0.3 |
| ovary | 3.7e-03 | 7.0e-03 | 3.5e-06 | 13.0 | 1.6e-04 | 8.9 |
| bladder | 7.0e-01 | 7.8e-01 | 3.0e-01 | 1.0 | 6.0e-01 | 0.7 |
| lung | 1.1e-01 | 4.6e-01 | 2.9e-01 | 1.2 | 8.5e-01 | 0.6 |

(continued)

| Name of Tissue | P1 | P2 | SP1 | R3 | SP2 | R4 |
|---|---|---|---|---|---|---|
| pancreas | 4.0e-01 | 3.8e-01 | 3.1e-07 | 2.9 | 3.3e-05 | 2.1 |
| liver | 4.9e-01 | 7.9e-01 | 9.2e-01 | 0.3 | 1.0e+00 | 0.1 |
| prostate | 1.1e-01 | 1.6e-01 | 4.9e-04 | 6.9 | 5.3e-04 | 7.1 |
| adrenal | 3.8e-01 | 4.3e-01 | 4.3e-02 | 3.4 | 8.0e-02 | 2.8 |
| general | 9.0e-06 | 8.7e-03 | 1.6e-25 | 2.2 | 7.8e-06 | 1.2 |
| Thyroid | 1.8e-01 | 1.8e-01 | 4.6e-01 | 2.0 | 4.6e-01 | 2.0 |
| uterus | 5.4e-04 | 5.1e-03 | 2.0e-12 | 6.0 | 4.3e-07 | 3.9 |
| colon | 5.9e-01 | 6.5e-01 | 9.4e-01 | 0.5 | 8.4e-01 | 0.5 |
| kidney | 2.3e-01 | 4.1e-01 | 2.4e-01 | 1.8 | 5.6e-01 | 1.2 |
| breast | 8.6e-02 | 8.9e-02 | 1.1e-02 | 4.0 | 4.3e-02 | 2.9 |
| stomach | 3.7e-01 | 8.4e-01 | 3.8e-01 | 0.8 | 9.6e-01 | 0.4 |
| epithelial | 2.8e-04 | 4.7e-02 | 2.4e-09 | 1.5 | 2.5e-01 | 0.9 |
| bone | 3.7e-01 | 3.2e-01 | 9.7e-01 | 0.4 | 9.9e-01 | 0.4 |

As noted above, cluster D12115 features 11 transcript(s), which were listed in Table 21 above. These transcript (s) encode for protein(s) which are variant(s) of protein Complement factor B precursor (SEQ. ID NO:131). A description of each variant protein according to the present invention is now provided.

Variant protein D12115_P3 (SEQ. ID NO:134) according to the present invention is encoded by transcript D12115_T3 (SEQ. ID NO:78). One or more alignments to one or more previously published Complement factor B precursor (SEQ. ID NO:131) protein sequences are given in the alignment table on the attached CD-ROM. A brief description of the relationship of the variant protein according to the present invention to each such aligned protein is as follows:

1. Comparison report between D12115_P3 (SEQ. ID NO:134) and CFAB_HUMAN (SEQ. ID NO: 395):

A. An isolated chimeric polypeptide encoding for D12115_P3 (SEQ. ID NO:134), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGI-PIGTRKVGSQYRLEDSVT YHCSRGLTLRGSQRRTCQEGGSWSGTEPSCQDSFMYDTPQEVAEAFLSSLTE-TIEGVDAEDGH GPGEQQKRKIVLDPSGSMNIYLVLDGSDSIGASNFTGAKKCLVNLIEKVASYGVKPRYGLV-TY ATYPKIWVKVSEADSSNADWVTKQLNEINYEDHKLKSGTNTKKALQAVYSMMSWPDDVPP EGWNRTRHVIILMTDGLHNMGGDPITVIDEIRDLLYIGKDRKNPREDYLDVYVFGVGPLVNQ VNINALASKKDNEQHVFKVKDMENLEDVFYQMIDESQSLSLCGMVWEHRKGTDYHKQPWQ AKISVIRPSKGHESCMGAVVSEYFVLTAAHCFTVDDKEHSIKVSVGGEKRDLEIEVVLFHPNY NINGKKEAGIPEFYDYDVALIKLKNKLKYGQTIRPICLPCTEGTTRALRLPPTTTCQQQKEELL PAQDIKALFVSEEEKKLTRKEVYIKNGDKKGSCERDAQYAPGYDKVKDISEVVTPRFLCTGG VSPYADPNTCRGDSGGPLIVHKRSRFIQVGVISWGVVDVCKNQKR corresponding to amino acids 1 - 730 of CFAB_HUMAN (SEQ. ID NO: 395), which also corresponds to amino acids 1 - 730 of D12115_P3 (SEQ. ID NO:134), and a second amino acid sequence being at least 70%, optionally at least 80%, preferably at least 85%, more preferably at least 90% and most preferably at least 95% homologous to a polypeptide having the sequence AALAEGETPR (SEQ. ID NO: 329) corresponding to amino acids 731 - 740 of D12115_P3 (SEQ. ID NO:134), wherein said first amino acid sequence and second amino acid sequence are contiguous and in a sequential order.

B. An isolated polypeptide encoding for an edge portion of D12115_P3 (SEQ. ID NO:134), comprising an amino acid sequence being at least 70%, optionally at least about 80%, preferably at least about 85%, more preferably at least about 90% and most preferably at least about 95% homologous to the sequence AALAEGETPR (SEQ. ID NO: 329) of D12115_P3 (SEQ. ID NO:134).

2. Comparison report between D12115_P3 (SEQ. ID NO:14) and NP_001701 (SEQ. ID NO:133):

A. An isolated chimeric polypeptide encoding for D12115_P3 (SEQ. ID NO:134), comprising a first amino acid sequence being at least 90% homologous to MGSNLSPQLCLMPFILGLLSGGVTTTPWSLA corresponding to amino acids 1 - 31 of NP_001701 (SEQ. ID NO:133), which also corresponds to amino acids 1 - 31 of D12115_P3 (SEQ. ID N0:134), a bridging amino acid R corresponding to amino acid 32 of D12115_P3 (SEQ. ID NO:134), a second amino acid sequence being at least 90% homologous to PQGSCSLEGVEIKG-GSFRLLQEGQALEYVCPSGFYPYPVQTRTCRSTGSWSTLKTQDQKTVRK AECRAIHCPRPHDFENGEY-WPRSPYYNVSDEISFHCYDGYTLRGSANRTCQVNGRWSGQTAI CDNGAGYCSNPGIPIGTRKVGSQYRL-EDSVTYHCSRGLTLRGSQRRTCQEGGSWSGTEPSCQ DSFMYDTPQEVAEAFLSSLTETIEGVDAEDGHG-PGEQQKRKIVLDPSGSMNIYLVLDGSDSIG ASNFTGAKKCLVNLIEKVASYGVKPRYGLVTYATYPKIWVKV-SEADSSNADWVTKQLNEIN YEDHKLKSGTNTKKALQAVYSMMSWPDDVPPEGWNRTRHVIILMTDGLHNM-GGDPITVIDE IRDLLYIGKDRKNPREDYLDVYVFGVGPLVNQVNINALASKKDNEQHVFKVKDMENLEDVF YQMIDESQSLSLCGMVWEHRKGTDYHKQPWQAKISVIRPSKGHESCMGAVVSEYFVLTAAH CFTVDDKEH-SIKVSVGGEKRDLEIEVVLFHPNYNINGKKEAGIPEFYDYDVALIKLKNKLKYG QTIRPICLPCTEGTTRALRL-PPTTTCQQQKEELLPAQDIKALFVSEEEKKLTRKEVYIKNGDKK GSCERDAQYAPGYDKVKDISEVVTPRFL-CTGGVSPYADPNTCRGDSGGPLIVHKRSRFIQVGV ISWGVVDVCKNQKR corresponding to amino acids 33 - 730 of NP_001701 (SEQ. ID NO:133), which also corresponds to amino acids 33 - 730 of D12115_P3 (SEQ. ID NO:134), and a third amino acid sequence being at least 70%, optionally at least 80%, preferably at least 85%, more preferably at least 90% and most preferably at least 95% homologous to a polypeptide having the sequence AALAEGETPR (SEQ. ID NO: 329) corresponding to amino acids 731 - 740 of D12115_P3 (SEQ. ID NO:134), wherein said first amino acid sequence, bridging amino acid, second amino acid sequence and third amino acid sequence are contiguous and in a sequential order.

3. Comparison report between D12115_P3 (SEQ. ID NO:134) and P00751-2 (SEQ. ID NO:132):

A. An isolated chimeric polypeptide encoding for D12115_P3 (SEQ. ID NO:134), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRK-VGSQYRLEDSVT YHCSRGLTLRGSQRRTCQEGGSWSGTEPSCQDSFMYDTPQEVAEAFLSSLTETIEGV-DAEDGH GPGEQQKRKIVLDPSGSMNIYLVLDGSDSIGASNFTGAKKCLVNLIEKVASYGVKPRYGLVTY ATY-PKIWVKVSEADSSNADWVTKQLNEINYEDHKLKSGTNTKKALQAVYSMMSWPDDVPP EGWNRTRHVI-ILMTD- GLHNMGGDPITVIDEIRDLLYIGKDRKNPREDYLDVYVFGVGPLVNQ VNINALASKKDNEQHV-FKVKDMENLEDVFYQMIDESQSLSLCGMVWEHRKGTDYHKQPWQ AKISVIRPSKGHESCMGAVVSEYFV-LTAAHCFTVDDKEHSIKVSVG corresponding to amino acids 1 - 542 of P00751-2 (SEQ. ID NO:132), which also corresponds to amino acids 1 - 542 of D12115_P3 (SEQ. ID NO:134), and a second amino acid sequence being at least 70%, optionally at least 80%, preferably at least 85%, more preferably at least 90% and most preferably at least 95% homologous to a polypeptide having the sequence GEKRDLEIEVVLFHPNYNINGKKEAGIPEFYDYDVALIKLKNKLKYGQTIRPICLPCTEGTTRA LRLPPTTTCQQQKEELLPAQDIKALFVSEEEKKLTRKEVYIKNGDKKGSCERDAQYAPGYDK VKDISEVVTPRFLCTGGVSPYADPNTCRGDSGGPLIVHKRSRFIQVGVISWGVVDVCKNQKRA ALAEGETPR (SEQ. ID NO:330) corresponding to amino acids 543 - 740 of D12115_P3 (SEQ. ID NO:134), wherein said first amino acid sequence and second amino acid sequence are contiguous and in a sequential order.

B. An isolated polypeptide encoding for an edge portion of D12115_P3 (SEQ. ID NO:134), comprising an amino acid sequence being at least 70%, optionally at least about 80%, preferably at least about 85%, more preferably at least about 90% and most preferably at least about 95% homologous to the sequence GEKRDLEIEVVLFHPNYNINGKKEAGIPEFYDYDVALIKLKNKLKYGQTIRPICLPCTEGTTRA LRLPPTTTCQQQKEELLPAQDIKALFVSEEEKKLTRKEVYIKNGDKKGSCERDAQYAPGYDK VKDISEVVTPRFLCTGGVSPYADPNTCRGDSGGPLIVHKRSRFIQVGVISWGVVDVCKNQKRA ALAEGETPR (SEQ. ID NO: 330) of D12115_P3 (SEQ. ID NO:134).

The localization of the variant protein was determined according to results from a number of different software programs and analyses, including analyses from SignalP and other specialized programs. The variant protein is believed to be secreted.

Variant protein D12115_P3 (SEQ. ID NO:134) also has the following non-silent SNPs (Single Nucleotide Polymorphisms) as listed in Table 29, (given according to their position(s) on the amino acid sequence, with the alternative amino acid(s) listed.

*Table 29 Amino acid mutations*

| SNP position(s) on amino acid sequence | Alternative amino acid(s) |
|---|---|
| 9 | L -> H |
| 32 | R -> Q |
| 32 | R -> W |
| 118 | S -> A |
| 142 | N -> I |
| 225 | D -> |
| 225 | D -> E |
| 252 | G -> S |
| 254 | G -> |
| 365 | M -> I |
| 428 | F -> |
| 556 | H -> Q |
| 565 | K -> E |
| 598 | P -> A |
| 598 | P -> S |
| 603 | T -> |
| 651 | D -> E |
| 677 | T -> |
| 693 | N -> |
| 729 | K -> R |
| 733 | L -> |

The glycosylation sites of variant protein D12115_P3 (SEQ. ID NO:134), as compared to the known protein Complement factor B precursor (SEQ. ID NO:131), are described in Table 30 (given according to their position(s) on the amino acid sequence in the first column; the second column indicates whether the glycosylation site is present in the variant protein; and the last column indicates whether the position is different on the variant protein).

*Table 30 - Glycosylation site(s)*

| Position(s) on known amino acid sequence | Present in variant protein? | Position(s) on variant protein |
|---|---|---|
| 122 | Yes | 122 |
| 142 | Yes | 142 |
| 285 | Yes | 285 |
| 291 | Yes | 291 |
| 378 | Yes | 378 |

The variant protein has the following domains, as determined by using InterPro. The domains are described in Table 31:

*Table 31 - InterPro domain(s)*

| Domain description | Analysis type | Position(s) on protein |
|---|---|---|
| von Willebrand factor, type A | FPrintScan | 269-286, 308-322, 383-391 |
| Peptidase S1A, chymotrypsin | FPrintScan | 512-527, 572-586, 692-704 |
| Sushi | HMMPfam | 37-86, 103-158, 165-218 |
| Peptidase S1, chymotrypsin | HMMPfam | 481-734 |
| von Willebrand factor, type A | HMMPfam | 270-468 |
| Peptidase S1, chymotrypsin | HMMSmart | 481-726 |
| Sushi | HMMSmart | 37-89, 103-158, 165-218 |
| von Willebrand factor, type A | HMMSmart | 268-473 |
| von Willebrand factor, type A | ProfileScan | 270-469 |
| Peptidase S1, chymotrypsin | ProfileScan | 477-740 |
| Peptidase S1, chymotrypsin | ScanRegExp | 522-527 |
| Peptidase S1, chymotrypsin | ScanRegExp | 693-704 |

Variant protein D12115_P3 (SEQ. ID NO:134) is encoded by the following transcript(s): D12115_T3 (SEQ. ID NO:78), for which the coding portion starts at position 514 and ends at position 2733. The transcript also has the following SNPs as listed in Table 32 (given according to their position on the nucleotide sequence, with the alternative nucleic acid listed.

*Table 32 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 865 | T -> G |
| 918 | C -> T |
| 938 | A -> T |
| 963 | G -> A |
| 1017 | G -> A |
| 1185 | C -> T |
| 1188 | C -> A |
| 1188 | C -> |
| 1267 | G -> A |
| 1273 | G -> |
| 1608 | G -> T |
| 1795 | T -> |
| 1878 | C -> T |
| 1894 | C -> T |
| 2181 | C -> A |
| 2184 | C -> T |

(continued)

| SNP position(s) on nucleotide sequence | Alternative nucleic acid(s) |
|---|---|
| 2206 | A -> G |
| 2305 | C -> G |
| 2305 | C -> T |
| 2320 | A -> |
| 2439 | G -> A |
| 2466 | T -> G |
| 2543 | C -> |
| 2591 | A -> |
| 2619 | C -> T |
| 2699 | A -> G |
| 2710 | C -> |
| 2729 | -> C |
| 2778 | C -> T |

Variant protein D12115_P5 (SEQ. ID NO:135) according to the present invention is encoded by transcripts D12115_T36 (SEQ. ID NO:88) and D12115_T5 (SEQ. ID NO:79). One or more alignments to one or more previously published Complement factor B precursor (SEQ. ID NO:131) protein sequences are given in the alignment table on the attached CD-ROM. A brief description of the relationship of the variant protein according to the present invention to each such aligned protein is as follows:

1. Comparison report between D12115_P5 (SEQ. ID NO:135) and CFAB_HUMAN (SEQ. ID NO: 395):

A. An isolated chimeric polypeptide encoding for D12115_P5 (SEQ. ID NO:135), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGV-DAEDGH GPGEQQKRKIVLDPSGSMNIYLVLDGSDSIGASNFTGAKKCLVNLIEKVASYGVKPRYGLVTY ATY-PKIWVKVSEADSSNADWVTKQLNEINYEDHKLKSGTNTKKALQAVYSMMSWPDDVPP EGWNRTRHVIILM-TDG corresponding to amino acids 1 - 390 of CFAB_HUMAN (SEQ. ID NO: 395), which also corresponds to amino acids 1 - 390 of D12115_P5 (SEQ. ID NO:135), and a second amino acid sequence being at least 70%, optionally at least 80%, preferably at least 85%, more preferably at least 90% and most preferably at least 95% homologous to a polypeptide having the sequence QKGPLSCPSLPTFSDQHVALKSTCNTIPMVGALNVTHSWLFISPVTLHKEFFLSPVINYL (SEQ. ID NO:331) corresponding to amino acids 391 - 450 of D12115_P5 (SEQ. ID NO:135), wherein said first amino acid sequence and second amino acid sequence are contiguous and in a sequential order.

B. An isolated polypeptide encoding for an edge portion of D12115_P5 (SEQ. ID NO:135), comprising an amino acid sequence being at least 70%, optionally at least about 80%, preferably at least about 85%, more preferably at least about 90% and most preferably at least about 95% homologous to the sequence QKGPLSCPSLPTFSDQHVALKSTCNTIPMVGALNVTHSWLFISPVTLHKEFFLSPVINYL (SEQ. ID NO: 331) of D12115_P5 (SEQ. ID NO:135).

2. Comparison report between D12115_P5 (SEQ. ID NO:135) and P00751-2 (SEQ. ID NO:132):

A. An isolated chimeric polypeptide encoding for D12115_P5 (SEQ. ID NO:135), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGV-

DAEDGH GPGEQQKRKIVLDPSGSMNIYLVLDGSDSIGASNFTGAKKCLVNLIEKVASYGVKPRYGLVTY ATYPKIWVKVSEADSSNADWVTKQLNEINYEDHKLKSGTNTKKALQAVYSMMSWPDDVPP EGWNRTRHVIILMTDG corresponding to amino acids 1 - 390 of P00751-2 (SEQ. ID NO:132), which also corresponds to amino acids 1 - 390 of D12115_P5 (SEQ. ID NO:135), and a second amino acid sequence being at least 70%, optionally at least 80%, preferably at least 85%, more preferably at least 90% and most preferably at least 95% homologous to a polypeptide having the sequence QKGPLSCPSLPTFSDQHVALKSTCNTIPMVGALNVTHSWLFISPVTLHKEFFLSPVINYL (SEQ. ID NO:331) corresponding to amino acids 391 - 450 of D12115_P5 (SEQ. ID NO: 135), wherein said first amino acid sequence and second amino acid sequence are contiguous and in a sequential order.

B. An isolated polypeptide encoding for an edge portion of D12115_P5 (SEQ. ID NO:135), comprising an amino acid sequence being at least 70%, optionally at least about 80%, preferably at least about 85%, more preferably at least about 90% and most preferably at least about 95% homologous to the sequence QKGPLSCPSLPTFSDQHVALKSTCNTIPMVGALNVTHSWLFISPVTLHKEFFLSPVINYL (SEQ. ID NO: 331) of D12115_PS (SEQ. ID NO:135).

3. Comparison report between D12115_P5 (SEQ. ID NO:135) andNP_001701 (SEQ. ID NO:133):

A. An isolated chimeric polypeptide encoding for D12115_P5 (SEQ. ID NO:135), comprising a first amino acid sequence being at least 90% homologous to MGSNLSPQLCLMPFILGLLSGGVTTTPWSLA corresponding to amino acids 1 - 31 of NP_001701 (SEQ. ID NO:133), which also corresponds to amino acids 1 - 31 of D12115_P5 (SEQ. ID NO:135), a bridging amino acid R corresponding to amino acid 32 of D12115-P5 (SEQ. ID NO:135), a second amino acid sequence being at least 90% homologous to PQGSCSLEGVEIKGGSFRLLQEGQALEYVCPSGFYPYPVQTRTCRSTGSWSTLKTQDQKTVRK AECRAIHCPRPHDFENGEYWPRSPYYNVSDEISFHCYDGYTLRGSANRTCQVNGRWSGQTAI CDNGAGYCSNPGIPIGTRKVGSQYRLEDSVTYHCSRGLTLRGSQRRTCQEGGSWSGTEPSCQ DSFMYDTPQEVAEAFLSSLTETIEGVDAEDGHGPGEQQKRKIVLDPSGSMNIYLVLDGSDSIG ASNFTGAKKCLVNLIEKVASYGVKPRYGLVTYATYPKIWVKVSEADSSNADWVTKQLNEIN YEDHKLKSGTNTKKALQAVYSMMSWPDDVPPEGWNRTRHVIILMTDG corresponding to amino acids 33 - 390 of NP_001701 (SEQ. ID NO:133), which also corresponds to amino acids 33 - 390 of D12115_P5 (SEQ. ID NO:135), and a third amino acid sequence being at least 70%, optionally at least 80%, preferably at least 85%, more preferably at least 90% and most preferably at least 95% homologous to a polypeptide having the sequence QKGPLSCPSLPTFSDQHVALKSTCNTIPMVGALNVTHSWLFISPVTLHKEFFLSPVINYL (SEQ. ID NO:331) corresponding to amino acids 391 - 450 of D12115_P5 (SEQ. ID NO:135), wherein said first amino acid sequence, bridging amino acid, second amino acid sequence and third amino acid sequence are contiguous and in a sequential order.

B. An isolated polypeptide encoding for an edge portion of D12115_P5 (SEQ. ID NO:135), comprising an amino acid sequence being at least 70%, optionally at least about 80%, preferably at least about 85%, more preferably at least about 90% and most preferably at least about 95% homologous to the sequence QKGPLSCPSLPTFSDQHVALKSTCNTIPMVGALNVTHSWLFISPVTLHKEFFLSPVINYL (SEQ. ID NO:331) of D12115_P5 (SEQ. ID NO:135).

The localization of the variant protein was determined according to results from a number of different software programs and analyses, including analyses from SignalP and other specialized programs. The variant protein is believed to be secreted.

Variant protein D12115_P5 (SEQ. ID NO:135) also has the following non-silent SNPs (Single Nucleotide Polymorphisms) as listed in Table 33, (given according to their position(s) on the amino acid sequence, with the alternative amino acid(s) listed.

*Table 33 - Amino acid mutations*

| SNP position) on amino acid sequence | Alternative amino acid(s) |
|---|---|
| 9 | L -> H |
| 32 | R -> Q |
| 32 | R -> W |
| 118 | S -> A |
| 142 | N -> I |

(continued)

| SNP position) on amino acid sequence | Alternative amino acid(s) |
|---|---|
| 225 | D -> |
| 225 | D -> E |
| 252 | G -> S |
| 254 | G -> |
| 365 | M -> I |
| 449 | Y -> C |

The glycosylation sites of variant protein D12115_P5 (SEQ. ID NO:135), as compared to the known protein Complement factor B precursor (SEQ. ID NO:131), are described in Table 34 (given according to their position(s) on the amino acid sequence in the first column; the second column indicates whether the glycosylation site is present in the variant protein; and the last column indicates whether the position is different on the variant protein).

*Table 34 - Glycosylation site(s)*

| Position(s) on known amino acid sequence | Present in variant protein? | Position(s) on variant protein |
|---|---|---|
| 122 | Yes | 122 |
| 142 | Yes | 142 |
| 285 | Yes | 285 |
| 291 | Yes | 291 |
| 378 | Yes | 378 |

The variant protein has the following domains, as determined by using InterPro. The domains are described in Table 35:

*Table 35 - InterPro domain(s)*

| Domain description | Analysis type | Position(s) on protein |
|---|---|---|
| von Willebrand factor, type A | FPrintScan | 269-286, 308-322, 383-391 |
| Sushi | HMMPfam | 37-86, 103-158, 165-218 |
| von Willebrand factor, type A | HMMPfam | 270-435 |
| Sushi | HMMSmart | 37-89, 103-158, 165-218 |
| von Willebrand factor, type A | HMMSmart | 268-436 |
| von Willebrand factor, type A | ProfileScan | 270-391 |

Variant protein D12115_P5 (SEQ. ID NO:135) is encoded by the following transcript(s): D12115_T36 (SEQ. ID NO:88) and D12115_T5 (SEQ. ID NO:79), for which the coding portion starts at position 514 and ends at position 1863. The transcript also has the following SNPs as listed in Table 36 (given according to their position on the nucleotide sequence, with the alternative nucleic acid listed.

*Table 36 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |

(continued)

| SNP position(s) on nucleotide sequence | Alternative nucleic acid(s) |
|---|---|
| 865 | T -> G |
| 918 | C -> T |
| 938 | A -> T |
| 963 | G -> A |
| 1017 | G -> A |
| 1185 | C -> T |
| 1188 | C -> A |
| 1188 | C -> |
| 1267 | G -> A |
| 1273 | G -> |
| 1608 | G -> T |
| 1859 | A -> G |
| 1894 | T -> C |
| 1928 | T -> A |
| 2076 | T -> |
| 2159 | C -> T |
| 2175 | C -> T |
| 2209 | A -> C |

The coding portion of transcript D12115_T5 (SEQ. ID NO:79) starts at position 514 and ends at position 1863. The transcript also has the following SNPs as listed in Table 37 (given according to their position on the nucleotide sequence, with the alternative nucleic acid listed.

*Table 37 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 865 | T -> G |
| 918 | C -> T |
| 938 | A -> T |
| 963 | G -> A |
| 1017 | G -> A |
| 1185 | C -> T |
| 1188 | C -> A |
| 1188 | C -> |
| 1267 | G -> A |
| 1273 | G -> |

(continued)

| SNP position(s) on nucleotide sequence | Alternative nucleic acid(s) |
|---|---|
| 1608 | G -> T |
| 1859 | A -> G |
| 1894 | T -> C |
| 1928 | T -> A |
| 2076 | T -> |
| 2159 | C -> T |
| 2175 | C -> T |
| 2462 | C -> A |
| 2465 | C -> T |
| 2487 | A -> G |
| 2586 | C -> G |
| 2586 | C -> T |
| 2601 | A -> |
| 2720 | G -> A |
| 2747 | T -> G |
| 2824 | C -> |
| 2872 | A -> |
| 2900 | C -> T |
| 2980 | A -> G |
| 3044 | C -> |
| 3063 | -> C |
| 3112 | C -> T |

Variant protein D12115_P12 (SEQ. ID NO:136) according to the present invention is encoded by transcript DI2115_TI2 (SEQ. ID NO:81). One or more alignments to one or more previously published Complement factor B precursor (SEQ. ID NO:131) protein sequences are given in the alignment table on the attached CD-ROM. A brief description of the relationship of the variant protein according to the present invention to each such aligned proteins is as follows:

1. Comparison report between D12115_P12 (SEQ. ID NO:136) and CFAB_HUMAN (SEQ. ID NO: 395):

A. An isolated chimeric polypeptide encoding for D12115_P12 (SEQ. ID NO:136), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVDA-EDGH GPGEQQKRKIVLDPSGSMNIYLVLDGSDSIGASNFTGAKKCLVNLIEKVASYGVKPRYGLVTY ATYP-KIWVKVSEADSSNADWVTKQLNEINYEDHKLKSGTNTKKALQAVYSMMSWPDDVPP EGWNRTRHVIILM-TDGLHNMGGDPITVIDEIRDLLYIGKDRKNPREDYLDVYVFGVGPLVNQ VNINALASKKDNEQHVFKVKDM-ENLEDVFYQMIDESQSLSLCGMVWEHRKGTDYHKQPWQ AKISVIRPSKGHESCMGAVVSEYFVLTAAHCF-TVDDKEHSIKVSVGGEKRDLEIEVVLFHPNY NINGKKEAGIPEFYDYDVALIKLKNKLKYGQTIRPICLPCTEG-TTRALRLPPTTTCQQQKEELL PAQDIKALFVSEEEKKLTRKEVYIKNGDKKGSCERDAQYAPGYDKVKDISE-VVTPRFLCTGG VSPYADPNTC- RGDSGGPLIVHKRSRFIQV corresponding to amino acids 1 - 714 of CFAB_HUMAN (SEQ. ID NO: 395), which also corresponds to amino acids 1 - 714 of D12115_P12 (SEQ. ID NO:136), and a second amino acid sequence being at least 70%, optionally at least 80%, preferably at least 85%, more preferably at least 90% and most preferably at least 95% homologous to a polypeptide having the sequence SPPFPIWGDAKWSAWAPKQESSMHVASNSR (SEQ. ID NO: 332) corresponding to amino acids 715 - 744 of D12115_P12 (SEQ. ID NO:136), wherein said first amino acid sequence and second amino acid sequence are contiguous and in a sequential order.

B. An isolated polypeptide encoding for an edge portion of D12115_P12 (SEQ. ID NO:136), comprising an amino acid sequence being at least 70%, optionally at least about 80%, preferably at least about 85%, more preferably at least about 90% and most preferably at least about 95% homologous to the sequence SPPFPIWGDAKWSAWAPKQESSMHVASNSR (SEQ. ID NO: 332) of D12115_P12 (SEQ. ID NO:136).

2. Comparison report between D12115_P12 (SEQ. ID NO:136) and NP_001701 (SEQ. ID NO:133):

A. An isolated chimeric polypeptide encoding for D12115_P12 (SEQ. ID NO:136), comprising a first amino acid sequence being at least 90% homologous to MGSNLSPQLCLMPFILGLLSGGVTTTPWSLA corresponding to amino acids 1 - 31 of NP_001701 (SEQ. ID NO:133), which also corresponds to amino acids 1 - 31 of D12115_P12 (SEQ. ID NO:136), a bridging amino acid R corresponding to amino acid 32 of D12115_P12 (SEQ. ID No:136), a second amino acid sequence being at least 90% homologous to PQGSCSLEGVEIKGGSFRLL-QEGQALEYVCPSGFYPYPVQTRTCRSTGSWSTLKTQDQKTVRK AECRAIHCPRPHDFENGEYWPRSPYY-NVSDEISFHCYDGYTLRGSANRTCQVNGRWSGQTAI CDNGAGYCSNPGIPIGTRKVGSQYRLEDSVTYHC-SRGLTLRGSQRRTCQEGGSWSGTEPSCQ DSFMYDTPQEVAEAFLSSLTETIEGVDAEDGHGPGEQQKR-KIVLDPSGSMNIYLVLDGSDSIG ASNFTGAKKCLVNLIEKVASYGVKPRYGLVTYATYPKIWVKVSEADSSNA-DWVTKQLNEIN YEDHKLKSGTNTKKALQAVYSMMSWPDDVPPEGWNRTRHVIILMTDGLHNMGGDPIT-VIDE IRDLLYIGKDRKNPREDYLDVYVFGVGPLVNQVNINALASKKDNEQHVFKVKDMENLEDVF YQMIDES-QSLSLCGMVWEHRKGTDYHKQPWQAKISVIRPSKGHESCMGAVVSEYFVLTAAH CFTVDDKEHSIKVSVG-GEKRDLEIEVVLFHPNYNINGKKEAGIPEFYDYDVALIKLKNKLKYG QTIRPICLPCTEGTTRALRLPPTTTCQ-QQKEELLPAQDIKALFVSEEEKKLTRKEVYIKNGDKK GSCERDAQYAPGYDKVKDISEVVTPRFLCTGGVSP-YADPNTCRGDSGGPLIVHKRSRFIQV corresponding to amino acids 33 - 714 of NP_001701 (SEQ. ID NO: 133), which also corresponds to amino acids 33 - 714 of D12115_P12 (SEQ. ID NO:136), and a third amino acid sequence being at least 70%, optionally at least 80%, preferably at least 85%, more preferably at least 90% and most preferably at least 95% homologous to a polypeptide having the sequence SPPFPIWGDAKWSAWAPKQESSMHVASNSR (SEQ. ID NO: 332) corresponding to amino acids 715 - 744 of D12115_P12 (SEQ. ID NO:136), wherein said first amino acid sequence, bridging amino acid, second amino acid sequence and third amino acid sequence are contiguous and in a sequential order.

B. An isolated polypeptide encoding for an edge portion of D12115_P12 (SEQ. ID NO:136), comprising an amino acid sequence being at least 70%, optionally at least about 80%, preferably at least about 85%, more preferably at least about 90% and most preferably at least about 95% homologous to the sequence SPPFPIWGDAKWSAWAPKQESSMHVASNSR (SEQ. ID NO: 332) of D12115_P12 (SEQ. ID NO:136).

3. Comparison report between D12115_P12 (SEQ. ID NO:136) and P00751-2 (SEQ. ID NO:132):

A. An isolated chimeric polypeptide encoding for D12115_P12 (SEQ. ID NO:136), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVD-AEDGH GPGEQQKRKIVLDPSGSMNIYLVLDGSDSIGASNFTGAKKCLVNLIEKVASYGVKPRYGLVTY ATYP-KIWVKVSEADSSNADWVTKQLNEINYEDHKLKSGTNTKKALQAVYSMMSWPDDVPP EGWNRTRHVIILMTDGLHNMGGDPITVIDEIRDLLYIGKDRKNPREDYLDVYVFGVGPLVNQ VNINALASKKDNEQHVFKVKDMENLEDVFYQMIDESQSLSLCGMVWEHRKGTDYHKQPWQ AKISVIRPSKGHESCMGAVVSEYFVLTAAHCFTVDDKEHSIKVSVG corresponding to amino acids 1 - 542 of P00751-2 (SEQ. ID NO:132), which also corresponds to amino acids 1 - 542 of D12115_P12 (SEQ. ID NO: 136), and a second amino acid sequence being at least 70%, optionally at least 80%, preferably at least 85%, more preferably at least 90% and most preferably at least 95% homologous to a polypeptide having the sequence GEKRDLEIEVVLFHPNYNINGKKEAGIPEFYDYDVALIKLKNKLKYGQTIRPICLPCTEGTTRA LRLPPTTTCQQQKEELLPAQDIKALFVSEEEKKLTRKEVYIKNGDKKGSCERDAQYAPGYDK VKDISEVVTPRFLCTGGVSPYADPNTCRGDSGGPLIVHKRSRFIQVSPPFPIWGDAKWSAWAP KQESSMHVASNSR (SEQ. ID NO: 333) corresponding to amino acids 543 - 744 of D12115_P12 (SEQ. ID NO:136), wherein said first amino acid sequence and second amino acid sequence are contiguous and in a sequential order.

B. An isolated polypeptide encoding for an edge portion of D12115_P12 (SEQ. ID NO:136), comprising an amino acid sequence being at least 70%, optionally at least about 80%, preferably at least about 85%, more preferably at least about 90% and most preferably at least about 95% homologous to the sequence GEKRDL-EIEVVLFHPNYNINGKKEAGIPEFYDYDVALIKLKNKLKYGQTIRPICLPCTEGTTRA LRLPPTTTCQQQKEELLPA-QDIKALFVSEEEKKLTRKEVYIKNGDKKGSCERDAQYAPGYDK VKDISEVVTPRFLCTGGVSPYADPNTCRGDS-GGPLIVHKRSRFIQVSPPFPIWGDAKWSAWAP KQESSMHVASNSR (SEQ. ID NO: 333) of D12115_P12 (SEQ. ID NO:136).

The localization of the variant protein was determined according to results from a number of different software programs and analyses, including analyses from SignalP and other specialized programs. The variant protein is believed to be secreted.

Variant protein D12115_P12 (SEQ. ID NO:136) also has the following non-silent SNPs (Single Nucleotide Polymorphisms) as listed in Table 38, (given according to their position(s) on the amino acid sequence, with the alternative amino acid(s) listed.

*Table 38 - Amino acid mutations*

| SNP positaon(s) on amino acid sequence | Alternative amino acid(s) |
|---|---|
| 9 | L -> H |
| 32 | R -> Q |
| 32 | R -> W |
| 118 | S -> A |
| 142 | N -> I |
| 225 | D -> |
| 225 | D -> E |
| 252 | G -> S |
| 254 | G -> |
| 365 | M -> I |
| 428 | F -> |
| 556 | H -> Q |
| 565 | K -> E |
| 598 | P -> A |
| 598 | P -> S |
| 603 | T -> |
| 651 | D -> E |
| 677 | T -> |
| 693 | N -> |

The glycosylation sites of variant protein D12115_P12 (SEQ. ID NO:136), as compared to the known protein Complement factor B precursor (SEQ. ID NO:131), are described in Table 39 (given according to their position(s) on the amino acid sequence in the first column; the second column indicates whether the glycosylation site is present in the variant protein; and the last column indicates whether the position is different on the variant protein).

*Table 39 - Glycosylation site(s)*

| Position(s) on known amino acid sequence | Present in variant protein? | Position(s) on variant protein |
|---|---|---|
| 122 | Yes | 122 |
| 142 | Yes | 142 |
| 285 | Yes | 285 |
| 291 | Yes | 291 |
| 378 | Yes | 378 |

The variant protein has the following domains, as determined by using InterPro. The domains are described in Table 40:

*Table 40 - InterPro domain(s)*

| Domain description | Analysis type | Position(s) on protein |
|---|---|---|
| von Willebrand factor, type A | FPrintScan | 269-286, 308-322, 383-391 |
| Peptidase S1A, chymotrypsin | FPrintScan | 512-527, 572-586, 692-704 |
| Sushi | HMMPfam | 37-86, 103-158, 165-218 |
| Peptidase S1, chymotrypsin | HMMPfam | 481-730 |
| von Willebrand factor, type A | HMMPfam | 270-468 |
| Peptidase S1, chymotrypsin | HMMSmart | 481-720 |
| Sushi | HMMSmart | 37-89, 103-158, 165-218 |
| von Willebrand factor, type A | HMMSmart | 268-473 |
| von Willebrand factor, type A | ProfileScan | 270-469 |
| Peptidase S1, chymotrypsin | ProfileScan | 477-744 |
| Peptidase S1, chymotrypsin | ScanRegExp | 522-527 |
| Peptidase S1, chymotrypsin | ScanRegExp | 693-704 |

Variant protein D12115_P12 (SEQ. ID NO:136) is encoded by the following transcript(s): D12115_T12 (SEQ. ID NO:81), for which the coding portion starts at position 514 and ends at position 2745. The transcript also has the following SNPs as listed in Table 41 (given according to their position on the nucleotide sequence, with the alternative nucleic acid listed.

*Table 41 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 865 | T -> G |
| 918 | C -> T |
| 938 | A -> T |
| 963 | G -> A |
| 1017 | G -> A |
| 1185 | C -> T |

(continued)

| SNP position(s) on nucleotide sequence | Alternative nucleic acid(s) |
|---|---|
| 1188 | C -> A |
| 1188 | C -> |
| 1267 | G -> A |
| 1273 | G -> |
| 1608 | G -> T |
| 1795 | T -> |
| 1878 | C -> T |
| 1894 | C -> T |
| 2181 | C -> A |
| 2184 | C -> T |
| 2206 | A -> G |
| 2305 | C -> G |
| 2305 | C -> T |
| 2320 | A -> |
| 2439 | G -> A |
| 2466 | T -> G |
| 2543 | C -> |
| 2591 | A -> |
| 2619 | C -> T |
| 2849 | G -> A |
| 2969 | A -> G |
| 3033 | C -> |
| 3052 | -> C |
| 3101 | C -> T |

Variant protein D12115_P13 (SEQ. ID NO:137) according to the present invention is encoded by transcript D12115_T13 (SEQ. ID NO:82). One or more alignments to one or more previously published Complement factor B precursor (SEQ. ID NO:131) protein sequences are given in the alignment table on the attached CD-ROM. A brief description of the relationship of the variant protein according to the present invention to each such aligned protein is as follows:

1. Comparison report between D12115_P13 (SEQ. ID NO:137) and CFAB_HUMAN (SEQ. ID NO: 395):

A. An isolated chimeric polypeptide encoding for D12115_P13 (SEQ. ID NO:137), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVDA-EDGH GPGEQQKRKIVLDPSGSMNIYLVLDGSDSIGASNFTGAKKCLVNLIEKVASYGVKPRYGLVTY ATYP-KIWVKVSEADSSNADWVTKQLNEINYEDHKLKSGTNTKKALQAVYSMMSWPDDVPP EGWNRTRHVIILMT-DGLHNMGGDPITVIDEIRDLLYIGKDRKNPREDYLDVYVFGVGPLVNQ VNINALASKKDNEQHVFKVKDMEN-LEDVFYQMIDESQSLSLCGMVWEHRKGTDYHKQPWQ AKISVIRPSKGHESCMGAVVSEYFVLTAAHCFTV-DDKEHSIKVSVGGEKRDLEIEVVLFHPNY NINGKKEAGIPEFYDYDVALIKLKNKLKYGQTIRPICLPCTEGTT-RALRLPPTTTCQQQ corresponding to amino acids 1 - 618 of CFAB_HUMAN (SEQ. ID NO: 395), which also corresponds to amino acids 1 - 618 of D12115_P13 (SEQ. ID NO:137), and a second amino acid sequence being at least 70%, optionally at least 80%, preferably at least 85%, more preferably at least 90% and most preferably at least 95% homologous to a polypeptide having the sequence RRAAPCTGYQSSVCV (SEQ. ID NO: 334) corresponding to amino acids 619 - 633 of D12115_P13 (SEQ. ID NO:137), wherein said first amino acid sequence and second amino acid sequence are contiguous and in a sequential order.

B. An isolated polypeptide encoding for an edge portion of D12115_P13 (SEQ. ID NO:137), comprising an amino acid sequence being at least 70%, optionally at least about 80%, preferably at least about 85%, more preferably at least about 90% and most preferably at least about 95% homologous to the sequence RRAAPCTGYQSSVCV (SEQ. ID NO: 334) of D12115_P13 (SEQ. ID NO:137).

2. Comparison report between D12115_P13 (SEQ. ID NO:137) and NP_001701 (SEQ. ID NO:133):

A. An isolated chimeric polypeptide encoding for D12115_P13 (SEQ. ID NO:137), comprising a first amino acid sequence being at least 90% homologous to MGSNLSPQLCLMPFILGLLSGGVTTTPWSLA corresponding to amino acids 1 - 31 of NP_001701 (SEQ. ID NO:133), which also corresponds to amino acids 1 - 31 of D12115_P13 (SEQ. ID NO:137), a bridging amino acid R corresponding to amino acid 32 of D12115_P13 (SEQ. ID NO:137), a second amino acid sequence being at least 90% homologous to PQGSCSLEGVEIKGGSFR-LLQEGQALEYVCPSGFYPYPVQTRTCRSTGSWSTLKTQDQKTVRK AECRAIHCPRPHDFENGEYWPRSP-YYNVSDEISFHCYDGYTLRGSANRTCQVNGRWSGQTAI CDNGAGYCSNPGIPIGTRKVGSQYRLEDSVTY-HCSRGLTLRGSQRRTCQEGGSWSGTEPSCQ DSFMYDTPQEVAEAFLSSLTETIEGVDAEDGHGPGEQQK-RKIVLDPSGSMNIYLVLDGSDSIG ASNFTGAKKCLVNLIEKVASYGVKPRYGLVTYATYPKIWVKVSEADSS-NADWVTKQLNEIN YEDHKLKSGTNTKKALQAVYSMMSWPDDVPPEGWNRTRHVIILMTDGLHNMGGDPIT-VIDE IRDLLYIGKDRKNPREDYLDVYVFGVGPLVNQVNINALASKKDNEQHVFKVKDMENLEDVF YQMIDE-SQSLSLCGMVWEHRKGTDYHKQPWQAKISVIRPSKGHESCMGAVVSEYFVLTAAH CFTVDDKEHSIKVSV-GGEKRDLEIEVVLFHPNYNINGKKEAGIPEFYDYDVALIKLKNKLKYG QTIRPICLPCTEGTTRALRLPPTTTC-QQQ corresponding to amino acids 33 - 618 of NP_001701 (SEQ. ID NO:133), which also corresponds to amino acids 33 - 618 of D12115_P13 (SEQ. ID NO:137), and a third amino acid sequence being at least 70%, optionally at least 80%, preferably at least 85%, more preferably at least 90% and most preferably at least 95% homologous to a polypeptide having the sequence RRAAPCTGYQSSVCV (SEQ. ID NO: 334) corresponding to amino acids 619 - 633 of D12115_P13 (SEQ. ID NO:137), wherein said first amino acid sequence, bridging amino acid, second amino acid sequence and third amino acid sequence are contiguous and in a sequential order.

B. An isolated polypeptide encoding for an edge portion of D12115_P13 (SEQ. ID NO:137), comprising an amino acid sequence being at least 70%, optionally at least about 80%, preferably at least about 85%, more preferably at least about 90% and most preferably at least about 95% homologous to the sequence RRAAPCTGYQSSVCV (SEQ. ID NO: 334) of D12115_P13 (SEQ. ID NO:137).

3. Comparison report between D12115_P13 (SEQ. ID NO:137) and P00751-2 (SEQ. ID NO:132):

A. An isolated chimeric polypeptide encoding for D12115_P13 (SEQ. ID NO:137), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVDA-EDGH GPGEQQKRKIVLDPSGSMNIYLVLDGSDSIGASNFTGAKKCLVNLIEKVASYGVKPRYGLVTY ATYP-KIWVKVSEADSSNADWVTKQLNEINYEDHKLKSGTNTKKALQAVYSMMSWPDDVPP EGWNRTRHVIILMT-DGLHNMGGDPITVIDEIRDLLYIGKDRKNPREDYLDVYVFGVGPLVNQ VNINALASKKDNEQHVFKVKDME-NLEDVFYQMIDESQSLSLCGMVWEHRKGTDYHKQPWQ AKISVIRPSKGHESCMGAVVSEYFVLTAAHCFT-VDDKEHSIKVSVG corresponding to amino acids 1 - 542 of P00751-2 (SEQ. ID NO:132), which also corresponds to amino acids 1 - 542 of D12115_P13 (SEQ. ID NO:137), and a second amino acid sequence being at least 70%, optionally at least 80%, preferably at least 85%, more preferably at least 90% and most preferably at least 95% homologous to a polypeptide having the sequence GEKRDLEIEVVLFHPNYNINGKKEAGIPEFYDYDVALIKLKNKLKYGQTIRPICLPCTEGTTRA LRLPPTTTCQQQRRAAPCTGYQSSVCV (SEQ. ID NO: 335) corresponding to amino acids 543 - 633 of D12115_P13 (SEQ. ID NO:137), wherein said first amino acid sequence and second amino acid sequence are contiguous and in a sequential order.

B. An isolated polypeptide encoding for an edge portion of D12115_P13 (SEQ. ID NO:137), comprising an amino acid sequence being at least 70%, optionally at least about 80%, preferably at least about 85%, more preferably at least about 90% and most preferably at least about 95% homologous to the sequence GEKRDLEIEVVLFHPNYNINGKKEAGIPEFYDYDVALIKLKNKLKYGQTIRPICLPCTEGTTRA LRLPPTTTCQQQRRAAPCTGYQSSVCV (SEQ. ID NO: 335) of D12115_P13 (SEQ. ID NO:137).

The localization of the variant protein was determined according to results from a number of different software programs and analyses, including analyses from SignalP and other specialized programs. The variant protein is believed to be secreted.

Variant protein D12115_P13 (SEQ. ID NO:137) also has the following non-silent SNPs (Single Nucleotide Polymorphisms) as listed in Table 42, (given according to their position(s) on the amino acid sequence, with the alternative amino acid(s) listed.

*Table 42 - Amino acid mutations*

| SNP position(s) on amino acid sequence | Alternative amino acid(s) |
|---|---|
| 9 | L -> H |
| 32 | R -> Q |
| 32 | R -> W |
| 118 | S -> A |
| 142 | N -> I |
| 225 | D -> |
| 225 | D -> E |
| 252 | G -> S |
| 254 | G -> |
| 365 | M -> I |
| 428 | F -> |
| 556 | H -> Q |
| 565 | K -> E |
| 598 | P -> A |
| 598 | P -> S |
| 603 | T -> |

The glycosylation sites of variant protein D12115_P13 (SEQ. ID NO:137), as compared to the known protein Complement factor B precursor (SEQ. ID NO:131), are described in Table 43 (given according to their position(s) on the amino acid sequence in the first column; the second column indicates whether the glycosylation site is present in the variant protein; and the last column indicates whether the position is different on the variant protein).

*Table 43 - Glycosylation site(s)*

| Position(s) on known amino acid sequence | Present in variant protein? | Position(s) on variant protein |
|---|---|---|
| 122 | Yes | 122 |
| 142 | Yes | 142 |
| 285 | Yes | 285 |
| 291 | Yes | 291 |
| 378 | Yes | 378 |

The variant protein has the following domains, as determined by using InterPro. The domains are described in Table 44:

*Table 44 - InterPro domain(s)*

| Domain description | Analysis type | Position(s) on protein |
|---|---|---|
| von Willebrand factor, type A | FPrintScan | 269-286, 308-322, 383-391 |
| Peptidase S1A, chymotrypsin | FPrintScan | 512-527, 572-586 |
| Sushi | HMMPfam | 37-86, 103-158, 165-218 |
| Peptidase S1, chymotrypsin | HMMPfam | 481-633 |
| von Willebrand factor, type A | HMMPfam | 270-468 |
| Peptidase S1, chymotrypsin | HMMSmart | 481-633 |
| Sushi | HMMSmart | 37-89, 103-158, 165-218 |
| von Willebrand factor, type A | HMMSmart | 268-473 |
| von Willebrand factor, type A | ProfileScan | 270-469 |
| Peptidase S1, chymotrypsin | ProfileScan | 477-633 |
| Peptidase S1, chymotrypsin | ScanRegExp | 522-527 |

Variant protein D12115_P13 (SEQ. ID NO:137) is encoded by the following transcript(s): D12115_T13 (SEQ. ID NO:82), for which the coding portion starts at position 514 and ends at position 2412. The transcript also has the following SNPs as listed in Table 45 (given according to their position on the nucleotide sequence, with the alternative nucleic acid listed.

*Table 45 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 865 | T -> G |
| 918 | C -> T |
| 938 | A -> T |
| 963 | G -> A |
| 1017 | G -> A |
| 1185 | C -> T |
| 1188 | C -> A |
| 1188 | C -> |
| 1267 | G -> A |
| 1273 | G -> |
| 1608 | G -> T |
| 1795 | T -> |
| 1878 | C -> T |
| 1894 | C -> T |
| 2181 | C -> A |

(continued)

| SNP position(s) on nucleotide sequence | Alternative nucleic acid(s) |
|---|---|
| 2184 | C -> T |
| 2206 | A -> G |
| 2305 | C -> G |
| 2305 | C -> T |
| 2320 | A -> |
| 2437 | G -> A |
| 2464 | T -> G |
| 2541 | C -> |
| 2589 | A -> |
| 2617 | C -> T |
| 2697 | A -> G |
| 2761 | C -> |
| 2780 | -> C |
| 2829 | C -> T |

Variant protein D12115_P15 (SEQ. ID NO:138) according to the present invention is encoded by transcript(s) D12115_T19 (SEQ. ID NO:84). One or more alignments to one or more previously published Complement factor B precursor (SEQ. ID NO:131) protein sequences are given in the alignment table on the attached CD-ROM. A brief description of the relationship of the variant protein according to the present invention to each such aligned protein is as follows:

1. Comparison report between D12115_P15 (SEQ. ID NO:138) and CFAB_HUMAN (SEQ. ID NO: 395):

A. An isolated chimeric polypeptide encoding for D12115_P15 (SEQ. ID NO:138), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVD-AEDGH GPGEQQKRKIVLDPSGSMNIYLVLDGSDSIGASNFTGAKKCLVNLIEKVASYGVKPRYGLVTY ATY-PKIWVKVSEADSSNADWVTKQLNEINYEDHKLKSGTNTKKALQAVYSMMSWPDDVPP EGWNRTRHVIIL-MTDGLHNMGGDPITVIDEIRDLLYIGKDRKNPREDYLDVYVFGVGPLVNQ VNINALASKKDNEQHVFKVKDM-ENLEDVFYQMIDESQSLSLCGMVWEHRKGTDYHKQPWQ AKISVIRPSKGHESCMGAVVSEYFVLTAAHCF-TVDDKEHSIKVSVGGEKRDLEIEVVLFHPNY NINGKKEAGIPEFYDYDVALIKLKNKLKYGQTIR corresponding to amino acids 1 - 593 of CFAB_HUMAN (SEQ. ID NO: 395), which also corresponds to amino acids 1 - 593 of D12115_P15 (SEQ. ID NO:138), and a second amino acid sequence being at least 70%, optionally at least 80%, preferably at least 85%, more preferably at least 90% and most preferably at least 95% homologous to a polypeptide having the sequence GRAAPCTGYQSSVCV (SEQ. ID NO: 336) corresponding to amino acids 594 - 608 of D12115_P15 (SEQ. ID NO:138), wherein said first amino acid sequence and second amino acid sequence are contiguous and in a sequential order.

B. An isolated polypeptide encoding for an edge portion of D12115_P15 (SEQ. ID NO:138), comprising an amino acid sequence being at least 70%, optionally at least about 80%, preferably at least about 85%, more preferably at least about 90% and most preferably at least about 95% homologous to the sequence GRAAPCTGYQSSVCV (SEQ. ID NO: 336) of D12115_P15 (SEQ. ID NO:138).

2. Comparison report between D12115_P15 (SEQ. ID NO:138) and NP_001701 (SEQ. ID NO:133):

A. An isolated chimeric polypeptide encoding for D12115_P15 (SEQ. ID NO:138), comprising a first amino acid sequence being at least 90% homologous to MGSNLSPQLCLMPFILGLLSGGVTTTPWSLA corresponding to amino acids 1 - 31 of NP_001701 (SEQ. ID NO:133), which also corresponds to amino acids 1 - 31 of D12115_P15 (SEQ. ID NO:138), a bridging amino acid R corresponding to amino acid 32 of D12115_P15 (SEQ. ID NO:138), a second amino acid sequence being at least 90% homologous to PQGSCSLEGVEIKGG-SFRLLQEGQALEYVCPSGFYPYPVQTRTCRSTGSWSTLKTQDQKTVRK AECRAIHCPRPHDFENGEYWP-RSPYYNVSDEISFHCYDGYTLRGSANRTCQVNGRWSGQTAI CDNGAGYCSNPGIPIGTRKVGSQYRLEDS-VTYHCSRGLTLRGSQRRTCQEGGSWSGTEPSCQ DSFMYDTPQEVAEAFLSSLTETIEGVDAEDGHGPGE-QQKRKIVLDPSGSMNIYLVLDGSDSIG ASNFTGAKKCLVNLIEKVASYGVKPRYGLVTYATYPKIWVKVSEAD-SSNADWVTKQLNEIN YEDHKLKSGTNTKKALQAVYSMMSWPDDVPPEGWNRTRHVIILMTDGLHNMGGD-PITVIDE IRDLLYIGKDRKNPREDYLDVYVFGVGPLVNQVNINALASKKDNEQHVFKVKDMENLEDVF YQMID-ESQSLSLCGMVWEHRKGTDYHKQPWQAKISVIRPSKGHESCMGAVVSEYFVLTAAH CFTVDDKEHSIKVS-VGGEKRDLEIEVVLFHPNYNINGKKEAGIPEFYDYDVALIKLKNKLKYG QTIR corresponding to amino acids 33 - 593 of NP_001701 (SEQ. ID NO:133), which also corresponds to amino acids 33 - 593 of D12115_P15 (SEQ. ID NO:138), and a third amino acid sequence being at least 70%, optionally at least 80%, preferably at least 85%, more preferably at least 90% and most preferably at least 95% homologous to a polypeptide having the sequence GRAAPCTGYQSSVCV (SEQ. ID NO: 336) corresponding to amino acids 594 - 608 of D12115_P15 (SEQ. ID NO:138), wherein said first amino acid sequence, bridging amino acid, second amino acid sequence and third amino acid sequence are contiguous and in a sequential order.

B. An isolated polypeptide encoding for an edge portion of D12115_-P15 (SEQ. ID NO:138), comprising an amino acid sequence being at least 70%, optionally at least about 80%, preferably at least about 85%, more preferably at least about 90% and most preferably at least about 95% homologous to the sequence GRAAPCTGYQSSVCV (SEQ. ID NO: 336) of D12115_P15 (SEQ. ID NO:138).

3. Comparison report between D12115_P15 (SEQ. ID NO:138) and P00751-2 (SEQ. ID NO:132):

A. An isolated chimeric polypeptide encoding for D12115_P15 (SEQ. ID NO:138), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRK-VGSQYRLEDSVT YHCSRGLTLRGSQRRTCQEGGSWSGTEPSCQDSFMYDTPQEVAEAFLSSLTETIEGVD-AEDGH GPGEQQKRKIVLDPSGSMNIYVLDGSDSIGASNFTGAKKCLVNLIEKVASYGVKPRYGLVTY ATYP-KIWVKVSEADSSNADWVTKQLNEINYEDHKLKSGTNTKKALQAVYSMMSWPDDVPP EGWNRTRHVIILMT-DGLHNMGGDPITVIDEIRDLLYIGKDRKNPREDYLDVYVFGVGPLVNQ VNINALASKKDNEQHVFKVKDMEN-LEDVFYQMIDESQSLSLCGMVWEHRKGTDYHKQPWQ AKISVIRPSKGHESCMGAVVSEYFVLTAAHCFTV-DDKEHSIKVSVG corresponding to amino acids 1 - 542 of P00751-2 (SEQ. ID NO:132), which also corresponds to amino acids 1 - 542 of D12115_P15 (SEQ. ID NO:138), and a second amino acid sequence being at least 70%, optionally at least 80%, preferably at least 85%, more preferably at least 90% and most preferably at least 95% homologous to a polypeptide having the sequence GEKRDLEIEVVLFHPNYNINGKKEAGIPEFYDYDVALIKLKNKLKYGQTIRGRAAPCTGYQSS VCV (SEQ. ID NO: 337) corresponding to amino acids 543 - 608 of D12115_P15 (SEQ. ID NO:138), wherein said first amino acid sequence and second amino acid sequence are contiguous and in a sequential order.

B. An isolated polypeptide encoding for an edge portion of D12115_P15 (SEQ. ID NO:138), comprising an amino acid sequence being at least 70%, optionally at least about 80%, preferably at least about 85%, more preferably at least about 90% and most preferably at least about 95% homologous to the sequence GEKRDLEIEVVLFHPNYNINGKKEAGIPEFYDYDVALIKLKNKLKYGQTIRGRAAPCTGYQSS VCV (SEQ. ID NO: 337) of D12115_P15 (SEQ. ID NO:138).

The localization of the variant protein was determined according to results from a number of different software programs and analyses, including analyses from SignalP and other specialized programs. The variant protein is believed to be secreted.

Variant protein D12115_P15 (SEQ. ID NO:138) also has the following non-silent SNPs (Single Nucleotide Polymorphisms) as listed in Table 46, (given according to their position(s) on the amino acid sequence, with the alternative amino acid(s) listed.

*Table 46 - Amino acid mutations*

| SNP position(s) on amino acid sequence | Alternative amino acid(s) |
|---|---|
| 9 | L -> H |
| 32 | R -> Q |
| 32 | R -> W |
| 118 | S -> A |
| 142 | N -> I |
| 225 | D -> |
| 225 | D -> E |
| 252 | G -> S |
| 254 | G -> |
| 365 | M -> I |
| 428 | F -> |
| 556 | H -> Q |
| 565 | K -> E |

The glycosylation sites of variant protein D12115_P15 (SEQ. ID NO:138), as compared to the known protein Complement factor B precursor (SEQ. ID NO:131), are described in Table 47 (given according to their position(s) on the amino acid sequence in the first column; the second column indicates whether the glycosylation site is present in the variant protein; and the last column indicates whether the position is different on the variant protein).

*Table 47 - Glycosylation site(s)*

| Position(s) on known amino acid sequence | Present in variant protein? | Position(s) on variant protein |
|---|---|---|
| 122 | Yes | 122 |
| 142 | Yes | 142 |
| 285 | Yes | 285 |
| 291 | Yes | 291 |
| 378 | Yes | 378 |

The variant protein has the following domains, as determined by using InterPro. The domains are described in Table 48:

*Table 48 - InterPro domain(s)*

| Domain description | Analysis type | Position(s) on protein |
|---|---|---|
| von Willebrand factor, type A | FPrintScan | 269-286, 308-322, 383-391 |
| Peptidase S1A, chymotrypsin | FPrintScan | 512-527, 572-586 |
| Sushi | HMMPfam | 37-86, 103-158, 165-218 |
| von Willebrand factor, type A | HMMPfam | 270-468 |
| Sushi | HMMSmart | 37-89, 103-158, 165-218 |
| von Willebrand factor, type A | HMMSmart | 268-473 |
| von Willebrand factor, type A | ProfileScan | 270-469 |
| Peptidase S1, chymotrypsin | ProfileScan | 477-602 |
| Peptidase S1, chymotrypsin | ScanRegExp | 522-527 |

Variant protein D12115_P15 (SEQ. ID NO:138) is encoded by the following transcript(s): D12115_T19 (SEQ. ID NO:84), for which the coding portion starts at position 514 and ends at position 2337. The transcript also has the following SNPs as listed in Table 49 (given according to their position on the nucleotide sequence, with the alternative nucleic acid listed.

*Table 49 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 865 | T -> G |
| 918 | C -> T |
| 938 | A -> T |
| 963 | G -> A |
| 1017 | G -> A |
| 1185 | C -> T |
| 1188 | C -> A |
| 1188 | C -> |
| 1267 | G -> A |
| 1273 | G -> |
| 1608 | G -> T |
| 1795 | T -> |
| 1878 | C -> T |
| 1894 | C -> T |
| 2181 | C -> A |
| 2184 | C -> T |
| 2206 | A -> G |
| 2362 | G -> A |
| 2389 | T -> G |
| 2466 | C -> |
| 2514 | A -> |
| 2542 | C -> T |
| 2622 | A -> G |
| 2686 | C -> |
| 2705 | -> C |
| 2754 | C -> T |

Variant protein D12115_P16 (SEQ. ID NO:139) according to the present invention is encoded by transcript D12115_T22 (SEQ. ID NO:85). One or more alignments to one or more previously published Complement factor B precursor (SEQ. ID NO:131) protein sequences are given in the alignment table on the attached CD-ROM. A brief description of the relationship of the variant protein according to the present invention to each such aligned protein is as follows:

1. Comparison report between D12115_P16 (SEQ. ID NO:139) and CFAB_HUMAN (SEQ. ID NO: 395):

A. An isolated chimeric polypeptide encoding for D12115_P16 (SEQ. ID NO:139), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVD-AEDGH GPGEQQKRKIVLDPSGSMNIYLVLDGSDSIGASNFTGAKKCLVNLIEKVASYGVKPRYGLVTY ATYP-KIWVKVSEADSSNADWVTKQLNEINYEDHKLKSGTNTKKALQAVYSMMSWPDDVPP EGWNRTRHVIILMT-DGLHNMGGDPITVIDEIRDLLYIGKDRKNPREDYLDVYVFGVGPLVNQ VNINALASKKDNEQHVFKVKDME-NLEDVFYQMIDESQSLSLCGMVWEHRKGTDYHKQPWQ AKISVIRPSKGHESCMGAVVSEYFVLTAAHCFT-VDDKEHSIKVSVGGEKRDLEIEVVLFHPNY NINGKKEAGIPEFYDYDVALIKLKNKLKYGQTIRPICLPCTEGT-TRALRLPPTTTCQQQKEELL PAQDIKALFVSEEEKKLTRKEVYIKNGDK corresponding to amino acids 1 - 652 of CFAB_HUMAN (SEQ. ID NO: 395), which also corresponds to amino acids 1 - 652 of D12115_P16 (SEQ. ID NO:139), and a second amino acid sequence being at least 70%, optionally at least 80%, preferably at least 85%, more preferably at least 90% and most preferably at least 95% homologous to a polypeptide having the sequence VRNGHPKEAL (SEQ. ID NO: 338) corresponding to amino acids 653 - 662 of D12115_P16 (SEQ. ID NO:139), wherein said first amino acid sequence and second amino acid sequence are contiguous and in a sequential order.

B. An isolated polypeptide encoding for an edge portion of D12115_P16 (SEQ. ID NO:139), comprising an amino acid sequence being at least 70%, optionally at least about 80%, preferably at least about 85%, more preferably at least about 90% and most preferably at least about 95% homologous to the sequence VRNGHPKEAL (SEQ. ID NO: 338) of D12115_P16 (SEQ. ID NO:139).

2. Comparison report between D12115_P16 (SEQ. ID NO:139) and NP_001701 (SEQ. ID NO:133):

A. An isolated chimeric polypeptide encoding for D12115_P16 (SEQ. ID NO:139), comprising a first amino acid sequence being at least 90% homologous to MGSNLSPQLCLMPFILGLLSGGVTTTPWSLA corresponding to amino acids 1 - 31 of NP_001701 (SEQ. ID NO:133), which also corresponds to amino acids 1 - 31 of D12115_P16 (SEQ. ID NO:139), a bridging amino acid R corresponding to amino acid 32 of D12115_P16 (SEQ. ID NO:139), a second amino acid sequence being at least 90% homologous to PQGSCSLEGVEIKGGSFRLL-QEGQALEYVCPSGFYPYPVQTRTCRSTGSWSTLKTQDQKTVRK AECRAIHCPRPHDFENGEYWPRSPYY-NVSDEISFHCYDGYTLRGSANRTCQVNGRWSGQTAI CDNGAGYCSNPGIPIGTRKVGSQYRLEDSVTYHC-SRGLTLRGSQRRTCQEGGSWSGTEPSCQ DSFMYDTPQEVAEAFLSSLTETIEGVDAEDGHGPGEQQKR-KIVLDPSGSMNIYLVLDGSDSIG ASNFTGAKKCLVNLIEKVASYGVKPRYGLVTYATYPKIWVKVSEADSSNA-DWVTKQLNEIN YEDHKLKSGTNTKKALQAVYSMMSWPDDVPPEGWNRTRHVIILMTDGLHNMGGDPIT-VIDE IRDLLYIGKDRKNPREDYLDVYVFGVGPLVNQVNINALASKKDNEQHVFKVKDMENLEDVF YQMIDE-SQSLSLCGMVWEHRKGTDYHKQPWQAKISVIRPSKGHESCMGAVVSEYFVLTAAH CFTVDDKEHSIKVSV-GGEKRDLEIEVVLFHPNYNINGKKEAGIPEFYDYDVALIKLKNKLKYG QTIRPICLPCTEGTTRALRLPPTTT-CQQQKEELLPAQDIKALFVSEEEKKLTRKEVYIKNGDK corresponding to amino acids 33 - 652 of NP_001701 (SEQ. ID NO:133), which also corresponds to amino acids 33 - 652 of D12115_P16 (SEQ. ID N0:139), and a third amino acid sequence being at least 70%, optionally at least 80%, preferably at least 85%, more preferably at least 90% and most preferably at least 95% homologous to a polypeptide having the sequence VRNGHPKEAL (SEQ. ID NO: 338) corresponding to amino acids 653 - 662 of D12115_P16 (SEQ. ID NO:139), wherein said first amino acid sequence, bridging amino acid, second amino acid sequence and third amino acid sequence are contiguous and in a sequential order.

B. An isolated polypeptide encoding for an edge portion of D12115_P16 (SEQ. ID NO:139), comprising an amino acid sequence being at least 70%, optionally at least about 80%, preferably at least about 85%, more preferably at least about 90% and most preferably at least about 95% homologous to the sequence VRNGHPKEAL (SEQ. ID NO: 338) of D12115_P16 (SEQ. ID NO:139).

3. Comparison report between D12115_P16 (SEQ. ID NO:139) and P00751-2 (SEQ. ID NO:132):

A. An isolated chimeric polypeptide encoding for D12115_P16 (SEQ. ID NO:139), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVD-

AEDGH GPGEQQKRKIVLDPSGSMNIYLVLDGSDSIGASNFTGAKKCLVNLIEKVASYGVKPRYGLVTY ATYPKIWVKVSEADSSNADWVTKQLNEINYEDHKLKSGTNTKKALQAVYSMMSWPDDVPP EGWNRTRHVIILMTDGLHNMGGDPITVIDEIRDLLYIGKDRKNPREDYLDVYVFGVGPLVNQ VNINALASKKDNEQHVFKVKDMENLEDVFYQMIDESQSLSLCGMVWEHRKGTDYHKQPWQ AKISVIRPSKGHESCMGAVVSEYFVLTAAHCFTVDDKEHSIKVSVG corresponding to amino acids 1 - 542 of P00751-2 (SEQ. ID NO:132), which also corresponds to amino acids 1 - 542 of D12115_P16 (SEQ. ID NO:139), and a second amino acid sequence being at least 70%, optionally at least 80%, preferably at least 85%, more preferably at least 90% and most preferably at least 95% homologous to a polypeptide having the sequence GEKRDLEIEVVLFHPNYNINGKKEAGIPEFYDYDVALIKLKNKLKYGQTIRPICLPCTEGTTRA LRLPPTTTCQQQKEELLPAQDIKALFVSEEEKKLTRKEVYIKNGDKVRNGHPKEAL (SEQ. ID NO: 339) corresponding to amino acids 543 - 662 of D12115_P16 (SEQ. ID NO:139), wherein said first amino acid sequence and second amino acid sequence are contiguous and in a sequential order.

B. An isolated polypeptide encoding for an edge portion of D12115_P16 (SEQ. ID NO:139), comprising an amino acid sequence being at least 70%, optionally at least about 80%, preferably at least about 85%, more preferably at least about 90% and most preferably at least about 95% homologous to the sequence GEKRDLEIEVVLFHPNYNINGKKEAGIPEFYDYDVALIKLKNKLKYGQTIRPICLPCTEGTTRA LRLPPTTTCQQQKEELLPAQDIKALFVSEEEKKLTRKEVYIKNGDKVRNGHPKEAL (SEQ. ID NO:339) of D12115_P16 (SEQ. ID NO:139).

The localization of the variant protein was determined according to results from a number of different software programs and analyses, including analyses from SignalP and other specialized programs. The variant protein is believed to be secreted.

Variant protein D12115_P16 (SEQ. ID NO:139) also has the following non-silent SNPs (Single Nucleotide Polymorphisms) as listed in Table 50, (given according to their position(s) on the amino acid sequence, with the alternative amino acid(s) listed.

*Table 50 - Amino acid mutations*

| SNP position(s) on amino acid sequence | Alternative amino acid(s) |
|---|---|
| 9 | L -> H |
| 32 | R -> Q |
| 32 | R -> W |
| 118 | S -> A |
| 142 | N -> I |
| 225 | D -> |
| 225 | D -> E |
| 252 | G -> S |
| 254 | G -> |
| 365 | M -> I |
| 428 | F -> |
| 556 | H -> Q |
| 565 | K -> E |
| 598 | P -> A |
| 598 | P -> S |
| 603 | T -> |
| 651 | D -> E |

The glycosylation sites of variant protein D12115_P16 (SEQ. ID NO:139), as compared to the known protein Complement factor B precursor (SEQ. ID NO:131), are described in Table 51 (given according to their position(s) on the amino acid sequence in the first column; the second column indicates whether the glycosylation site is present in the variant protein; and the last column indicates whether the position is different on the variant protein).

*Table 51 - Glycosylation site(s)*

| Position(s) on known amino acid sequence | Present in variant protein? | Position(s) on variant protein |
|---|---|---|
| 122 | Yes | 122 |
| 142 | Yes | 142 |
| 285 | Yes | 285 |
| 291 | Yes | 291 |
| 378 | Yes | 378 |

The variant protein has the following domains, as determined by using InterPro. The domains are described in Table 52:

*Table 52 - InterPro domain(s)*

| Domain description | Analysis type | Position(s) on protein |
|---|---|---|
| von Willebrand factor, type A | FPrintScan | 269-286, 308-322, 383-391 |
| Peptidase S1A, chymotrypsin | FPrintScan | 512-527, 572-586 |
| Sushi | HMMPfam | 37-86, 103-158, 165-218 |
| Peptidase S1, chymotrypsin | HMMPfam | 481-653 |
| von Willebrand factor, type A | HMMPfam | 270-468 |
| Peptidase S1, chymotrypsin | HMMSmart | 481-653 |
| Sushi | HMMSmart | 37-89, 103-158, 165-218 |
| von Willebrand factor, type A | HMMSmart | 268-473 |
| von Willebrand factor, type A | ProfileScan | 270-469 |
| Peptidase S1, chymotrypsin | ProfileScan | 477-615 |
| Peptidase S1, chymotrypsin | ScanRegExp | 522-527 |

Variant protein D12115_P16 (SEQ. ID NO:139) is encoded by the following transcript(s): D12115_T22 (SEQ. ID NO:85), for which the coding portion starts at position 514 and ends at position 2499. The transcript also has the following SNPs as listed in Table 53 (given according to their position on the nucleotide sequence, with the alternative nucleic acid listed.

*Table 53 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 865 | T -> G |
| 918 | C -> T |
| 938 | A -> T |
| 963 | G -> A |
| 1017 | G -> A |

(continued)

| SNP position(s) on nucleotide sequence | Alternative nucleic acid(s) |
|---|---|
| 1185 | C -> T |
| 1188 | C -> A |
| 1188 | C -> |
| 1267 | G -> A |
| 1273 | G -> |
| 1608 | G -> T |
| 1795 | T -> |
| 1878 | C -> T |
| 1894 | C -> T |
| 2181 | C -> A |
| 2184 | C ->T |
| 2206 | A -> G |
| 2305 | C -> G |
| 2305 | C -> T |
| 2320 | A -> |
| 2439 | G -> A |
| 2466 | T -> G |
| 2639 | C -> |
| 2687 | A -> |
| 2715 | C -> T |
| 2945 | G -> A |
| 3065 | A -> G |
| 3129 | C -> |
| 3148 | -> C |
| 3197 | C -> T |

Variant protein D12115_P20 (SEQ. ID NO:140) according to the present invention is encoded by transcript D12115_T27 (SEQ. ID NO:86). One or more alignments to one or more previously published Complement factor B precursor (SEQ. ID NO:131) protein sequences are given in the alignment table on the attached CD-ROM. A brief description of the relationship of the variant protein according to the present invention to each such aligned protein is as follows:

1. Comparison report between D12115_P20 (SEQ. ID NO:140) and CFAB_HUMAN (SEQ. ID NO: 395):

A. An isolated chimeric polypeptide encoding for D12115_P20 (SEQ. ID NO:140), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVD-AEDGH GPGEQQKRKIVLDPSGSMNIYLVLDGSDSIGASNFTGAKKCLVNLIEKVASYGVKPRYGLVTY ATYP-KIWVKVSEADSSNADWVTKQLNEINYEDHKLKSGTNTKKALQAVYSMMSWPDDVPP EGWNRTRHVIILMT-DGLHNMGGDPITVIDEIRDLLYIGKDRKNPREDYLDVYVFGVGPLVNQ VNINALASKKDNEQHVFKVKDMEN-LEDVFYQMIDESQSLSLCGMVWEHRKGTDYHKQPWQ AKISVIRPSKGHESCMGAVVSEYFVLTAAHCFTV-DDKEHSIKVSV corresponding to amino acids 1 - 541 of CFAB_HUMAN (SEQ. ID NO: 395), which also corresponds to amino acids 1 - 541 of D12115_ P20 (SEQ. ID NO:140), a second bridging amino acid sequence comprising of E, and a third amino acid sequence being at least 90% homologous to EELLPA-QDIKALFVSEEEKKLTRKEVYIKNGDKKGSCERDAQYAPGYDKVKDISEVVTPRFLC TGGVSPYADPNTCR-GDSGGPLIVHKRSRFIQVGVISWGVVDVCKNQKRQKQVPAHARDFHIN LFQVLPWLKEKLQDEDLGFL corresponding to amino acids 620 - 764 of CFAB_HUMAN (SEQ. ID NO: 395), which also corresponds to amino acids 543 - 687 of DI2115_P20 (SEQ. ID NO:140), wherein said first amino acid sequence, second amino acid sequence and third amino acid sequence are contiguous and in a sequential order.

B. An isolated polypeptide encoding for an edge portion of D12115_P20 (SEQ. ID NO:140), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least 3 amino acids comprise VEE having a structure as follows (numbering according to D12115_P20 (SEQ. ID NO:140)): a sequence starting from any of amino acid numbers 541-x to 541; and ending at any of amino acid numbers 543 + ((n-3) - x), in which x varies from 0 to n-3.

2. Comparison report between D12115_P20 (SEQ. ID NO:140) and NP_001701 (SEQ. ID NO:133):

A. An isolated chimeric polypeptide encoding for D12115_P20 (SEQ. ID NO:140), comprising a first amino acid sequence being at least 90% homologous to MGSNLSPQLCLMPFILGLLSGGVTTTPWSLA corresponding to amino acids 1 - 31 of NP_001701 (SEQ. ID NO:133), which also corresponds to amino acids 1 - 31 of D12115_P20 (SEQ. ID NO:140), a bridging amino acid R corresponding to amino acid 32 of D12115_P20 (SEQ. ID NO:140), a second amino acid sequence being at least 90% homologous to PQGSCSLEGVEIKG-GSFRLLQEGQALEYVCPSGFYPYPVQTRTCRSTGSWSTLKTQDQKTVRK AECRAIHCPRPHDFENGEYW-PRSPYYNVSDEISFHCYDGYTLRGSANRTCQVNGRWSGQTAI CDNGAGYCSNPGIPIGTRKVGSQYRLE-DSVTYHCSRGLTLRGSQRRTCQEGGSWSGTEPSCQ DSFMYDTPQEVAEAFLSSLTETIEGVDAEDGHG-PGEQQKRKIVLDPSGSMNIYLVLDGSDSIG ASNFTGAKKCLVNLIEKVASYGVKPRYGLVTYATYPKIWVKV-SEADSSNADWVTKQLNEIN YEDHKLKSGTNTKKALQAVYSMMSWPDDVPPEGWNRTRHVIILMTDGLHNM-GGDPITVIDE IRDLLYIGKDRKNPREDYLDVYVFGVGPLVNQVNINALASKKDNEQHVFKVKDMENLEDVF YQMIDESQSLSLCGMVWEHRKGTDYHKQPWQAKISVIRPSKGHESCMGAVVSEYFVLTAAH CFTVDDKEH-SIKVSV corresponding to amino acids 33 - 541 of NP_001701 (SEQ. ID NO:133), which also corresponds to amino acids 33 - 541 of D12115_P20 (SEQ. ID NO:140), a third bridging amino acid sequence comprising of E, and a fourth amino acid sequence being at least 90% homologous to EELLPAQDIKALF-VSEEEKKLTRKEVYIKNGDKKGSCERDAQYAPGYDKVKDISEVVTPRFLC TGGVSPYADPNTCRGDSGGP-LIVHKRSRFIQVGVISWGVVDVCKNQKRQKQVPAHARDFHIN LFQVLPWLKEKLQDEDLGFL corresponding to amino acids 620 - 764 of NP_001701 (SEQ. ID NO:133), which also corresponds to amino acids 543 - 687 of D12115_P20 (SEQ. ID NO:140), wherein said first amino acid sequence, bridging amino acid, second amino acid sequence, third amino acid sequence and fourth amino acid sequence are contiguous and in a sequential order.

3. Comparison report between D12115_P20 (SEQ. ID NO:140) and P00751-2 (SEQ. ID NO:132):

A. An isolated chimeric polypeptide encoding for D12115_P20 (SEQ. ID NO:140), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVDA-EDGH GPGEQQKRKIVLDPSGSMNIYLVLDGSDSIGASNFTGAKKCLVNLIEKVASYGVKPRYGLVTY ATYPK-IWVKVSEADSSNADWVTKQLNEINYEDHKLKSGTNTKKALQAVYSMMSWPDDVPP EGWNRTRHVIILMTD-GLHNMGGDPITVIDEIRDLLYIGKDRKNPREDYLDVYVFGVGPLVNQ VNINALASKKDNEQHVFKVKDMEN-LEDVFYQMIDESQSLSLCGMVWEHRKGTDYHKQPWQ AKISVIRPSKGHESCMGAVVSEYFVLTAAHCFTV-DDKEHSIKVSV corresponding to amino acids 1 - 541 of P00751-2 (SEQ. ID NO:132), which also corresponds to amino acids 1 - 541 of D12115_P20 (SEQ. ID NO:140), and a second amino acid sequence being at least 70%, optionally at least 80%, preferably at least 85%, more preferably at least 90% and most preferably at least 95% homologous to a polypeptide having the sequence EEELLPAQDIKALFVSEEEKKLTRKEVYIKNGDKKG-SCERDAQYAPGYDKVKDISEVVTPRFL CTGGVSPYADPNTCRGDSGGPLIVHKRSRFIQVGVISWGVVD-VCKNQKRQKQVPAHARDFHI NLFQVLPWLKEKLQDEDLGFL (SEQ. ID NO: 340) corresponding to amino acids 542 - 687 of D12115_P20 (SEQ. ID NO:140), wherein said first amino acid sequence and second amino acid sequence are contiguous and in a sequential order.

B. An isolated polypeptide encoding for an edge portion of D12115_P20 (SEQ. ID NO:140), comprising an amino acid sequence being at least 70%, optionally at least about 80%, preferably at least about 85%, more preferably at least about 90% and most preferably at least about 95% homologous to the sequence EEELLPAQDIKALFVSEEEKKLTRKEVYIKNGDKKGSCERDAQYAPGYDKVKDISEVVTPRFL CTGGVSPYADPNTCRGDSGGPLIVHKRSRFIQVGVISWGVVDVCKNQKRQKQVPAHARDFHI NLFQVLPWLKEKLQDEDLGFL (SEQ. ID NO: 340) of D12115_P20 (SEQ. ID NO:140).

The localization of the variant protein was determined according to results from a number of different software programs and analyses, including analyses from SignalP and other specialized programs. The variant protein is believed to be secreted.

Variant protein D12115_P20 (SEQ. ID NO:140) also has the following non-silent SNPs (Single Nucleotide Polymorphisms) as listed in Table 54, (given according to their position(s) on the amino acid sequence, with the alternative amino acid(s) listed.

*Table 54 - Amino acid mutations*

| SNP position(s) on amino acid sequence | Alternative amino acid(s) |
|---|---|
| 9 | L -> H |
| 32 | R -> Q |
| 32 | R -> W |
| 118 | S -> A |
| 142 | N -> I |
| 225 | D -> |
| 225 | D -> E |
| 252 | G -> S |
| 254 | G -> |
| 365 | M -> I |
| 428 | F -> |
| 574 | D -> E |
| 600 | T -> |
| 616 | N -> |
| 652 | K -> R |
| 673 | P -> |

The glycosylation sites of variant protein D12115_P20 (SEQ. ID NO:140), as compared to the known protein Complement factor B precursor (SEQ. ID NO:131), are described in Table 55 (given according to their position(s) on the amino acid sequence in the first column; the second column indicates whether the glycosylation site is present in the variant protein; and the last column indicates whether the position is different on the variant protein).

*Table 55 - Glycosylation site(s)*

| Position(s) on known amino acid sequence | Present in variant protein? | Position(s) on variant protein |
|---|---|---|
| 122 | Yes | 122 |
| 142 | Yes | 142 |
| 285 | Yes | 285 |
| 291 | Yes | 291 |

(continued)

| Position(s) on known amino acid sequence | Present in variant protein? | Position(s) on variant protein |
|---|---|---|
| 378 | Yes | 378 |

The variant protein has the following domains, as determined by using InterPro. The domains are described in Table 56:

*Table 56 - InterPro domain(s)*

| Domain description | Analysis type | Position(s) on protein |
|---|---|---|
| von Willebrand factor, type A | FPrintScan | 269-286, 308-322, 383-391 |
| Peptidase S1A, chymotrypsin | FPrintScan | 512-527, 615-627 |
| Sushi | HMMPfam | 37-86, 103-158, 165-218 |
| Peptidase S1, chymotrypsin | HMMPfam | 481-675 |
| von Willebrand factor, type A | HMMPfam | 270-468 |
| Peptidase S1, chymotrypsin | HMMSmart | 481-675 |
| Sushi | HMMSmart | 37-89, 103-158, 165-218 |
| von Willebrand factor, type A | HMMSmart | 268-473 |
| von Willebrand factor, type A | ProfileScan | 270-469 |
| Peptidase S1, chymotrypsin | ProfileScan | 477-680 |
| Peptidase S1, chymotrypsin | ScanRegExp | 522-527 |
| Peptidase S1, chymotrypsin | ScanRegExp | 616-627 |

Variant protein D12115_P20 (SEQ. ID NO:140) is encoded by the following transcript(s): D12115_T27 (SEQ. ID NO:86), for which coding portion starts at position 514 and ends at position 2574. The transcript also has the following SNPs as listed in Table 57 (given according to their position on the nucleotide sequence, with the alternative nucleic acid listed.

*Table 57 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 865 | T -> G |
| 918 | C -> T |
| 938 | A -> T |
| 963 | G -> A |
| 1017 | G -> A |
| 1185 | C -> T |
| 1188 | C -> A |
| 1188 | C -> |
| 1267 | G -> A |
| 1273 | G -> |

(continued)

| SNP position(s) on nucleotide sequence | Alternative nucleic acid(s) |
|---|---|
| 1608 | G -> T |
| 1795 | T -> |
| 1878 | C -> T |
| 1894 | C -> T |
| 2208 | G -> A |
| 2235 | T -> G |
| 2312 | C -> |
| 2360 | A -> |
| 2388 | C -> T |
| 2468 | A -> G |
| 2532 | C -> |
| 2551 | -> C |
| 2600 | C -> T |

Variant protein D12115_P32 (SEQ. ID NO:141) according to the present invention is encoded by transcript D12115_T33 (SEQ. ID NO:87). One or more alignments to one or more previously published Complement factor B precursor (SEQ. ID NO:131) protein sequences are given in the alignment table on the attached CD-ROM. A brief description of the relationship of the variant protein according to the present invention to each such aligned protein is as follows:

1. Comparison report between D12115_P32 (SEQ. ID NO:141) and CFAB_HUMAN (SEQ. ID NO: 395):

A. An isolated chimeric polypeptide encoding for D12115_P32 (SEQ. ID NO:141), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNV SDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVD-AEDGH GPGEQQKRKIVLDPSGSMNIYLVLDGSDSIGASNFTGAKKCLVNLIEKVASYGVKPRYGLVTY ATYP-KIWVKVSEADSSNADWVTKQLNEINYEDHKLKSGTNTKKALQAVYSMMSWPDDVPP EGWNRTRHVIILMT-DGLHNMGGDPITVIDEIRDLLYIGKDRKNPREDYLDVYVFGVGPLVNQ VNINALASKKDNEQHVFKVKDMEN-LEDVFYQMI corresponding to amino acids 1 - 469 of CFAB_HUMAN (SEQ. ID NO: 395), which also corresponds to amino acids 1 - 469 of D12115_P32 (SEQ. ID NO:141), and a second amino acid sequence being at least 70%, optionally at least 80%, preferably at least 85%, more preferably at least 90% and most preferably at least 95% homologous to a polypeptide having the sequence GREIQGNKEHNS (SEQ. ID NO: 341) corresponding to amino acids 470 - 481 of D12115-_P32 (SEQ. ID NO:141), wherein said first amino acid sequence and second amino acid sequence are contiguous and in a sequential order.
B. An isolated polypeptide encoding for an edge portion of D12115_P32 (SEQ. ID NO:141), comprising an amino acid sequence being at least 70%, optionally at least about 80%, preferably at least about 85%, more preferably at least about 90% and most preferably at least about 95% homologous to the sequence GREIQGNKEHNS (SEQ. ID NO: 341) of D12115_P32 (SEQ. ID NO:141).

2. Comparison report between D12115_P32 (SEQ. ID NO:141) and P00751-2 (SEQ. ID NO:132):

A. An isolated chimeric polypeptide encoding for D12115_P32 (SEQ. ID NO:141), comprising a first amino acid sequence being at least 90% homologous to MGSNLSPQLCLMPFILGLLSGGVTTTPWSLARPQGSC-SLEGVEIKGGSFRLLQEGQALEYVCPS GFYPYPVQTRTCRSTGSWSTLKTQDQKTVRKAECRAIHCPRPH-DFENGEYWPRSPYYNVSDE ISFHCYDGYTLRGSANRTCQVNGRWSGQTAICDNGAGYCSNPGIPIGTRKV-GSQYRLEDSVT YHCSRGLTLRGSQRRTCQEGGSWSGTEPSCQDSFMYDTPQEVAEAFLSSLTETIEGVD-AEDGH GPGEQQKRKIVLDPSGSMNIYLVLDGSDSIGASNFTGAKKCLVNLIEKVASYGVKPRYGLVTY ATY-

PKIWVKVSEADSSNADWVTKQLNEINYEDHKLKSGTNTKKALQAVYSMMSWPDDVPP EGWNRTRHVIILM-TDGLHNMGGDPITVIDEIRDLLYIGKDRKNPREDYLDVYVFGVGPLVNQ VNINALASKKDNEQHVFKVKDME-NLEDVFYQMI corresponding to amino acids 1 - 469 of P00751-2 (SEQ. ID NO:132), which also corresponds to amino acids 1 - 469 of D12115_P32 (SEQ. ID NO:141), and a second amino acid sequence being at least 70%, optionally at least 80%, preferably at least 85%, more preferably at least 90% and most preferably at least 95% homologous to a polypeptide having the sequence GREIQGNKEHNS (SEQ. ID NO: 341) corresponding to amino acids 470 - 481 of D12115_P32 (SEQ. ID NO:141), wherein said first amino acid sequence and second amino acid sequence are contiguous and in a sequential order.

B. An isolated polypeptide encoding for an edge portion of D12115_P32 (SEQ. ID NO:141), comprising an amino acid sequence being at least 70%, optionally at least about 80%, preferably at least about 85%, more preferably at least about 90% and most preferably at least about 95% homologous to the sequence GREIQGNKEHNS (SEQ. ID NO: 341) of D12115_P32 (SEQ. ID NO:141).

3. Comparison report between D12115_P32 (SEQ. ID NO:141) and NP_001701 (SEQ. ID NO:133):

A. An isolated chimeric polypeptide encoding for D12115_P32 (SEQ. ID NO:141), comprising a first amino acid sequence being at least 90% homologous to MGSNLSPQLCLMPFILGLLSGGVTTTPWSLA corresponding to amino acids 1-31 of NP_001701 (SEQ. ID NO:133), which also corresponds to amino acids 1-31 of D12115_P32 (SEQ. ID NO:141), a bridging amino acid R corresponding to amino acid 32 of D12115_P32 (SEQ. ID NO:141), a second amino acid sequence being at least 90% homologous to PQGSCSLEGVEIKGG-SFRLLQEGQALEYVCPSGFYPYPVQTRTCRSTGSWSTLKTQDQKTVRK AECRAIHCPRPHDFENGEYWP-RSPYYNVSDEISFHCYDGYTLRGSANRTCQVNGRWSGQTAI CDNGAGYCSNPGIPIGTRKVGSQYRLED-SVTYHCSRGLTLRGSQRRTCQEGGSWSGTEPSCQ DSFMYDTPQEVAEAFLSSLTETIEGVDAEDGHGP-GEQQKRKIVLDPSGSMNIYLVLDGSDSIG ASNFTGAKKCLVNLIEKVASYGVKPRYGLVTYATYPKIWVKV-SEADSSNADWVTKQLNEIN YEDHKLKSGTNTKKALQAVYSMMSWPDDVPPEGWNRTRHVIILMTDGLHNM-GGDPITVIDE IRDLLYIGKDRKNPREDYLDVYVFGVGPLVNQVNINALASKKDNEQHVFKVKDMENLEDVF Y-QMI corresponding to amino acids 33 - 469 of NP_001701 (SEQ. ID NO:133), which also corresponds to amino acids 33 - 469 of D12115_P32 (SEQ. ID NO:141), and a third amino acid sequence being at least 70%, optionally at least 80%, preferably at least 85%, more preferably at least 90% and most preferably at least 95% homologous to a polypeptide having the sequence GREIQGNKEHNS (SEQ. ID NO: 341) corresponding to amino acids 470 - 481 of D12115_P32 (SEQ. ID NO:141), wherein said first amino acid sequence, bridging amino acid, second amino acid sequence and third amino acid sequence are contiguous and in a sequential order.

B. An isolated polypeptide encoding for an edge portion of D12115_P32 (SEQ. ID NO:141), comprising an amino acid sequence being at least 70%, optionally at least about 80%, preferably at least about 85%, more preferably at least about 90% and most preferably at least about 95% homologous to the sequence GREIQGNKEHNS (SEQ. ID NO: 341) of D12115_P32 (SEQ. ID NO:141).

The localization of the variant protein was determined according to results from a number of different software programs and analyses, including analyses from SignalP and other specialized programs. The variant protein is believed to be secreted.

Variant protein D12115_P32 (SEQ. ID NO:141) also has the following non-silent SNPs (Single Nucleotide Polymorphisms) as listed in Table 58, (given according to their position(s) on the amino acid sequence, with the alternative amino acid(s) listed.

*Table 58 - Amino acid mutations*

| SNP position(s) on amino acid sequence | Alternative amino acid(s) |
|---|---|
| 9 | L -> H |
| 32 | R -> Q |
| 32 | R -> W |
| 118 | S -> A |
| 142 | N -> I |
| 225 | D -> |
| 225 | D -> E |

(continued)

| SNP position(s) on amino acid sequence | Alternative amino acid(s) |
|---|---|
| 252 | G -> S |
| 254 | G -> |
| 365 | M -> I |
| 428 | F-> |
| 472 | E->A |

The glycosylation sites of variant protein D12115_P32 (SEQ. ID NO:141), as compared to the known protein Complement factor B precursor (SEQ. ID NO:131), are described in Table 59 (given according to their position(s) on the amino acid sequence in the first column; the second column indicates whether the glycosylation site is present in the variant protein; and the last column indicates whether the position is different on the variant protein).

*Table 59 - Glycosylation site(s)*

| Position(s) on known amino acid sequence | Present in variant protein? | Position(s) on variant protein |
|---|---|---|
| 122 | Yes | 122 |
| 142 | Yes | 142 |
| 285 | Yes | 285 |
| 291 | Yes | 291 |
| 378 | Yes | 378 |

The variant protein has the following domains, as determined by using InterPro. The domains are described in Table 60:

*Table 60 - InterPro domain(s)*

| Domain description | Analysis type | Position(s) on protein |
|---|---|---|
| von Willebrand factor, type A | FPrintScan | 269-286, 308-322, 383-391 |
| Sushi | HMMPfam | 37-86, 103-158, 165-218 |
| von Willebrand factor, type A | HMMPfam | 270-468 |
| Sushi | HMMSmart | 37-89, 103-158, 165-218 |
| von Willebrand factor, type A | HMMSmart | 268-473 |
| von Willebrand factor, type A | ProfileScan | 270-469 |

Variant protein D12115_P32 (SEQ. ID NO:141) is encoded by the following transcript(s): D12115_T33 (SEQ. ID NO:87), for which the coding portion starts at position 514 and ends at position 1956. The transcript also has the following SNPs as listed in Table 61 (given according to their position on the nucleotide sequence, with the alternative nucleic acid listed.

*Table 61 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 865 | T -> G |

(continued)

| SNP position(s) on nucleotide sequence | Alternative nucleic acid(s) |
|---|---|
| 918 | C -> T |
| 938 | A -> T |
| 963 | G -> A |
| 1017 | G -> A |
| 1185 | C -> T |
| 1188 | C -> A |
| 1188 | C -> |
| 1267 | G -> A |
| 1273 | G -> |
| 1608 | G -> T |
| 1795 | T -> |
| 1878 | C -> T |
| 1894 | C -> T |
| 1928 | A -> C |

Variant protein D12115_P34 (SEQ. ID NO:142) according to the present invention is encoded by transcript D12115-T14 (SEQ. ID NO:83).

The localization of the variant protein was determined according to results from a number of different software programs and analyses, including analyses from SignalP and other specialized programs. The variant protein is believed to be secreted.

Variant protein D12115_P34 (SEQ. ID NO:142) also has the following non-silent SNPs (Single Nucleotide Polymorphisms) as listed in Table 62, (given according to their position(s) on the amino acid sequence, with the alternative amino acid(s) listed.

*Table 62 - Amino acid mutations*

| SNP position(s) on amino acid sequence | Alternative amino acid(s) |
|---|---|
| 9 | L -> H |
| 32 | R -> Q |
| 32 | R -> W |
| 118 | S -> A |
| 142 | N -> I |
| 220 | R -> Q |
| 276 | T -> M |
| 277 | T -> |
| 277 | T -> K |

The glycosylation sites of variant protein D12115_P34 (SEQ. ID NO:142), as compared to the known protein Complement factor B precursor (SEQ. ID NO:131), are described in Table 63 (given according to their position(s) on the amino acid sequence in the first column; the second column indicates whether the glycosylation site is present in the variant protein; and the last column indicates whether the position is different on the variant protein).

*Table 63 - Glycosylation sife(s)*

| Position(s) on known amino acid sequence | Present in variant protein? | Position(s) on variant protein |
|---|---|---|
| 122 | Yes | 122 |
| 142 | Yes | 142 |
| 285 | No | |
| 291 | No | |
| 378 | No | |

The variant protein has the following domains, as determined by using InterPro. The domains are described in Table 64:

*Table 64 - InterPro domain(s)*

| Domain description | Analysis type | Position(s) on protein |
|---|---|---|
| Sushi | HMMPfam | 37-86, 103-158 |
| Sushi | HMMSmart | 37-89, 103-158 |

Variant protein D12115 P34 (SEQ. ID NO:142) is encoded by the following transcript(s): D12115_T14 (SEQ. ID NO:83), for which the coding portion starts at position 514 and ends at position 1380. The transcript also has the following SNPs as listed in Table 65 (given according to their position on the nucleotide sequence, with the alternative nucleic acid listed.

*Table 65 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 865 | T -> G |
| 918 | C -> T |
| 938 | A -> T |
| 963 | G -> A |
| 1172 | G -> A |
| 1340 | C -> T |
| 1343 | C -> A |
| 1343 | C -> |
| 1422 | G -> A |
| 1428 | G -> |
| 1653 | C -> T |
| 1876 | G -> T |
| 2063 | T -> |
| 2146 | C -> T |
| 2162 | C -> T |
| 2449 | C -> A |

(continued)

| SNP position(s) on nucleotide sequence | Alternative nucleic acid(s) |
|---|---|
| 2452 | C -> T |
| 2474 | A -> G |
| 2573 | C -> G |
| 2573 | C -> T |
| 2588 | A -> |
| 2707 | G -> A |
| 2734 | T -> G |
| 2811 | C -> |
| 2859 | A -> |
| 2887 | C -> T |
| 2967 | A -> G |
| 3031 | C -> |
| 3050 | -> C |
| 3099 | C -> T |

Variant protein D12115_P35 (SEQ. ID NO:143) according to the present invention is encoded by transcript D12115_T9 (SEQ. ID NO:80).

The localization of the variant protein was determined according to results from a number of different software programs and analyses, including analyses from SignalP and other specialized programs. The variant protein is believed to be secreted.

Variant protein D12115_P35 (SEQ. ID NO:143) also has the following non-silent SNPs (Single Nucleotide Polymorphisms) as listed in Table 66, (given according to their position(s) on the amino acid sequence, with the alternative amino acid(s) listed.

*Table 66 - Amino acid mutations*

| SNP position(s) on amino acid sequence | Alternative amino acid(s) |
|---|---|
| 9 | L -> H |
| 32 | R -> Q |
| 32 | R -> W |

The glycosylation sites of variant protein D12115_P35 (SEQ. ID NO: 143), as compared to the known protein Complement factor B precursor (SEQ. ID NO:131), are described in Table 67 (given according to their position(s) on the amino acid sequence in the first column; the second column indicates whether the glycosylation site is present in the variant protein; and the last column indicates whether the position is different on the variant protein).

*Table 67 - Glycosylation site(s)*

| Position(s) on known amino acid sequence | Present in variant protein? | Position(s) on variant protein |
|---|---|---|
| 122 | No | |
| 142 | No | |
| 285 | No | |
| 291 | No | |
| 378 | No | |

The variant protein has the following domains, as determined by using InterPro. The domains are described in Table 68:

*Table 68 - InterPro domain(s)*

| Domain description | Analysis type | Position(s) on protein |
|---|---|---|
| Sushi | HMMPfam | 37-86 |

Variant protein D12115_P35 (SEQ. ID NO:143) is encoded by the following transcript(s): D12115_T9 (SEQ. ID NO:80), for which the coding portion starts at position 514 and ends at position 879. The transcript also has the following SNPs as listed in Table 69 (given according to their position on the nucleotide sequence, with the alternative nucleic acid listed.

*Table 69 - Nucleic acid SNPs*

| SNP position(s) on nucleotide sequence | Alternative nucleic acid(s) |
|---|---|
| 325 | G -> |
| 539 | T -> A |
| 607 | C -> T |
| 608 | G -> A |
| 1097 | T -> A |
| 1101 | C -> T |
| 1265 | T -> G |
| 1318 | C -> T |
| 1338 | A -> T |
| 1363 | G -> A |
| 1417 | G -> A |
| 1585 | C -> T |
| 1588 | C -> A |
| 1588 | C -> |
| 1667 | G -> A |
| 1673 | G -> |
| 2008 | G -> T |
| 2195 | T -> |
| 2278 | C -> T |
| 2294 | C -> T |
| 2581 | C -> A |
| 2584 | C -> T |
| 2606 | A -> G |
| 2705 | C -> G |
| 2705 | C -> T |
| 2720 | A -> |
| 2839 | G -> A |
| 2866 | T -> G |
| 2943 | C -> |

(continued)

| SNP position(s) on nucleotide sequence | Alternative nucleic acid(s) |
|---|---|
| 2991 | A -> |
| 3019 | C -> T |
| 3099 | A -> G |
| 3163 | C -> |
| 3182 | -> C |
| 3231 | C -> T |

As noted above, cluster D12115 features 42 segment(s), which were listed in Table 22 above and for which the sequence(s) are given. These segment(s) are portions of nucleic acid sequence(s) which are described herein separately because they are of particular interest. A description of several segments according to the present invention is now provided.

Segment cluster D12115_N4 (SEQ. ID NO:91) according to the present invention is supported by 11 libraries. The number of libraries was determined as previously described. This segment can be found in the following transcript (s): D12115_T9 (SEQ. ID NO:80). Table 70 below describes the starting and ending position of this segment on each transcript.

*Table 70 - Segment location on transcripts*

| Transcript name | Segment starting position | Segment ending position |
|---|---|---|
| D12115_T9 (SEQ.ID NO:80) | 812 | 1211 |

Segment cluster D12115_N6 (SEQ. ID NO:93) according to the present invention is supported by 4 libraries. The number of libraries was determined as previously described. This segment can be found in the following transcript (s): D12115_T14 (SEQ. ID NO:83). Table 71 below describes the starting and ending position of this segment on each transcript.

*Table 71 - Segment location on transcripts*

| Transcript name | Segment starting position | Segment ending position |
|---|---|---|
| D12115_T14 (SEQ. ID NO:83) | 998 | 1152 |

Segment cluster D12115_N27 (SEQ. ID NO:96) according to the present invention is supported by 7 libraries. The number of libraries was determined as previously described. This segment can be found in the following transcript (s): D12115_T36 (SEQ. ID NO:88) and D12115_T5 (SEQ. ID NO:79). Table 72 below describes the starting and ending position of this segment on each transcript.

*Table 72 - Segment location on transcripts*

| Transcript name | Segment starting position | Segment ending position |
|---|---|---|
| D12115_T36 (SEQ. ID NO:88) | 1682 | 1962 |
| D12115_T5 (SEQ. ID NO:79) | 1682 | 1962 |

Segment cluster D12115_N34 (SEQ. ID NO:97) according to the present invention is supported by 13 libraries. The number of libraries was determined as previously described. This segment can be found in the following transcript (s): D12115_T33 (SEQ. ID NO:87) and D12115_T36 (SEQ. ID NO:88). Table 73 below describes the starting and ending position of this segment on each transcript.

*Table 73 - Segment location on transcripts*

| Transcript name | Segment starting position | Segment ending position |
|---|---|---|
| D12115_T33 (SEQ. ID NO:87) | 1922 | 1957 |
| D12115_T36 (SEQ. ID NO:88) | 2203 | 2238 |

Segment cluster D12115_N41 (SEQ. ID NO:98) according to the present invention is supported by 182 libraries. The number of libraries was determined as previously described. This segment can be found in the following transcript (s): D12115_ T12 (SEQ. ID NO:81), D12115_T13 (SEQ. ID NO:82), D12115_T14 (SEQ. ID NO:83), D12115_T19 (SEQ. ID NO:84), D12115_T22 (SEQ. ID NO:85), D12115_T3 (SEQ. ID NO:78), D12115_T5 (SEQ. ID NO:79) and D12115_T9 (SEQ. ID NO:80). Table 74 below describes the starting and ending position of this segment on each transcript.

*Table 74 - Segment location on transcripts*

| Transcript name | Segment starting position | Segment ending position |
|---|---|---|
| D12115_T12 (SEQ. ID NO:81) | 2138 | 2291 |
| D12115_T13 (SEQ. ID NO:82) | 2138 | 2291 |
| D12115_T14 (SEQ. ID NO:83) | 2406 | 2559 |
| D12115_T19 (SEQ. ID NO:84) | 213 | 2291 |
| D12115 T22 (SEQ. ID NO:85) | 2138 | 2291 |
| D12115_T3 (SEQ. ID NO:78) | 2138 | 2291 |
| D12115_T5 (SEQ. ID NO:79) | 2419 | 2572 |
| D12115_T9 (SEQ.ID NO:80) | 2538 | 2691 |

Segment cluster D12115_N53 (SEQ. ID NO:99) according to the present invention is supported by 8 libraries. The number of libraries was determined as previously described. This segment can be found in the following transcript (s): D12115_T12 (SEQ. ID NO:81) and D12115_T22 (SEQ. ID NO:85). Table 75 below describes the starting and ending position of this segment on each transcript.

*Table 75 - Segment location on transcripts*

| Transcript name | Segment starting position | Segment ending position |
|---|---|---|
| D12115_T12 (SEQ. ID NO:81) | 2653 | 2922 |
| D12115_T22 (SEQ. ID NO:85) | 2749 | 3018 |

According to an optional embodiment of the present invention, short segments related to the above cluster are also provided. These segments are up to about 120 bp in length, and so are included in a separate description.

Segment cluster D12115_N39 (SEQ. ID NO:119) according to the present invention is supported by 152 libraries. The number of libraries was determined as previously described. This segment can be found in the following transcript (s): D121I5_T12 (SEQ. ID NO:81), D12115_T13 (SEQ. ID NO:82), D12115_T14 (SEQ. ID NO:83), D12115_T19 (SEQ. ID NO:84), D12115_T22 (SEQ. ID NO:85), D12115_T27 (SEQ. ID NO:86), D12115_T3 (SEQ. ID NO:78), D12115_T5 (SEQ. ID NO:79) and D12115_T9 (SEQ. ID NO:80). Table 76 below describes the starting and ending position of this segment on each transcript.

*Table 76 - Segment location on transcripts*

| Transcript name | Segment starting position | Segment ending position |
|---|---|---|
| D12115_T12 (SEQ. ID NO:81) | 2084 | 2137 |
| D12115_T13 (SEQ. ID NO:82) | 2084 | 2137 |
| D12115_T14 (SEQ. ID NO:83) | 2352 | 2405 |
| D12115_T19 (SEQ. ID NO:84) | 2084 | 2137 |

(continued)

| Transcript name | Segment starting position | Segment ending position |
|---|---|---|
| D12115_T22 (SEQ. ID NO:85) | 2084 | 2137 |
| D12115_T27 (SEQ. ID NO:86) | 2084 | 2137 |
| D12115_T3 (SEQ. ID NO:78) | 2084 | 2137 |
| D12115_T5 (SEQ. ID NO:79) | 2365 | 2418 |
| D12115_T9 (SEQ. ID NO:80) | 2484 | 2537 |

Segment cluster D12115_N43 (SEQ. ID NO:120) according to the present invention is supported by 178 libraries. The number of libraries was determined as previously described. This segment can be found in the following transcript (s): D12115_T12 (SEQ. ID NO:81), D12115_T13 (SEQ. ID NO:82), D12115_T14 (SEQ. ID NO:83), D12115_T22 (SEQ. ID NO:85), D12115_T3 (SEQ. ID NO:78), D12115_T5 (SEQ. ID NO:79) and D12115_T9 (SEQ. ID NO:80). Table 77 below describes the starting and ending position of this segment on each transcript.

*Table 77 - Segment location on transcripts*

| Transcript name | Segment starting position | Segment ending position |
|---|---|---|
| D12115_ T12 (SEQ. ID NO:81) | 2292 | 2368 |
| D12115_Tl3 (SEQ. ID NO:82) | 2292 | 2368 |
| D12115 T14 (SEQ. ID NO:83) | 2560 | 2636 |
| D12115_T22 (SEQ. ID NO:85) | 2292 | 2368 |
| D12115_T3 (SEQ. ID NO:78) | 2292 | 2368 |
| D12115_ T5 (SEQ. ID NO:79) | 2573 | 2649 |
| D12115_T9 (SEQ. ID NO:80) | 2692 | 2768 |

Segment cluster D12115_N45 (SEQ. ID NO:121) according to the present invention is supported by 171 libraries. The number of libraries was determined as previously described. This segment can be found in the following transcript (s): D12115_T12 (SEQ. ID NO:81), D12115_T14 (SEQ. ID NO:83), D12115_T19 (SEQ. ID NO:84), D12115_T22 (SEQ. ID NO:85), D12115_T27 (SEQ. ID NO:86), D12115_T3 (SEQ. ID NO:78), D12115_T5 (SEQ. ID NO:79) and D12115_T9 (SEQ. ID NO:80). Table 78 below describes the starting and ending position of this segment on each transcript.

*Table 78 - Segment location on transcripts*

| Transcript name | Segment starting position | Segment ending position |
|---|---|---|
| D12115_T12 (SEQ. ID NO:81) | 2369 | 2370 |
| D12115_T14 (SEQ. ID NO:83) | 2637 | 2638 |
| D12115_T19 (SEQ. ID NO:84) | 2292 | 2293 |
| D12115_T22 (SEQ. ID NO:85) | 2369 | 2370 |
| D12115_T27 (SEQ. ID NO:86) | 2138 | 2139 |
| D12115_T3 (SEQ. ID NO:78) | 2369 | 2370 |
| D12115_T5 (SEQ. ID NO:79) | 2650 | 2651 |
| D12115_T9 (SEQ. ID NO:80) | 2769 | 2770 |

Segment cluster D12115_N46 (SEQ. ID NO:122) according to the present invention is supported by 190 libraries. The number of libraries was determined as previously described. This segment can be found in the following transcript (s): D12115_T12 (SEQ. ID NO:81), D121I5_T13 (SEQ. ID NO:82), D12115_T14 (SEQ. ID NO:83), D12115_T19 (SEQ. ID NO:84), D12115_T22 (SEQ. ID NO:85), D12115_T27 (SEQ. ID NO:86), D12115_T3 (SEQ. ID NO:78), D12115_T5 (SEQ. ID NO:79) and D12115_T9 (SEQ. ID NO:80). Table 79 below describes the starting and ending position of this segment on each transcript.

*Table 79 - Segment location on transcripts*

| Transcript name | Segment starting position | Segment ending position |
|---|---|---|
| D12115_T12 (SEQ. ID NO:81) | 2371 | 2401 |
| D12115_T13 (SEQ. ID NO:82) | 2369 | 2399 |
| D12115_T14 (SEQ. ID NO:83) | 2639 | 2669 |
| D12115_T19 (SEQ. ID NO:84) | 2294 | 2324 |
| D12115_T22 (SEQ. ID NO:85) | 2371 | 2401 |
| D12115_T27 (SEQ. ID NO: 86) | 2140 | 2170 |
| D12115_T3 (SEQ. ID NO:78) | 2371 | 2401 |
| D12115_T5 (SEQ. ID NO:79) | 2652 | 2682 |
| D12115_T9 (SEQ. ID NO:80) | 2771 | 2801 |

Segment cluster D12115_N47 (SEQ. ID NO:123) according to the present invention is supported by 204 libraries. The number of libraries was determined as previously described. This segment can be found in the following transcript (s): D12115_T12 (SEQ. ID NO:81), D12115_T13 (SEQ. ID NO:82), D12115_T14 (SEQ. ID NO:83), D12115_T19 (SEQ. ID NO:84), D12115_T22 (SEQ. ID NO:85), DI2115_T27 (SEQ. ID NO:86), D12115_T3 (SEQ. ID NO:78), D12115_T5 (SEQ. ID NO:79) and D12115_T9 (SEQ. ID NO:80). Table 80 below describes the starting and ending position of this segment on each transcript.

*Table 80 - Segment location on transcripts*

| Transcript name | Segment starting position | Segment ending position |
|---|---|---|
| D12115_T12 (SEQ. ID NO:81) | 2402 | 2469 |
| D12115_T13 (SEQ. ID NO:82) | 2400 | 2467 |
| D1115_T14 (SEQ. ID NO:83) | 2670 | 2737 |
| D12115_T19 (SEQ. ID NO:84) | 2325 | 2392 |
| D12115_T22 (SEQ. ID NO:85) | 2402 | 2469 |
| D12115_T27 (SEQ. ID NO:86) | 2171 | 2238 |
| D12115_T3 (SEQ. ID NO:78) | 2402 | 2469 |
| D12115_T5 (SEQ. ID NO:79) | 2683 | 2750 |
| D12115_T9 (SEQ. ID NO:80) | 2802 | 2869 |

Segment cluster D12115_N48 (SEQ. ID NO:124) according to the present invention is supported by 3 libraries. The number of libraries was determined as previously described. This segment can be found in the following transcript (s): D12115_T22 (SEQ. ID NO:85). Table 81 below describes the starting and ending position of this segment on each transcript.

*Table 81 - Segment location on transcripts*

| Transcript name | Segment starting position | Segment ending position |
|---|---|---|
| D12115_22 (SEQ. ID NO:85) | 2470 | 2565 |

Segment cluster D12115_N54 (SEQ. ID NO:128) according to the present invention is supported by 182 libraries. The number of libraries was determined as previously described. This segment can be found in the following transcript (s): D12115_T12 (SEQ. ID NO:81), D121I5_T13 (SEQ. ID NO:82), D12115_T14 (SEQ. ID NO:83), D12I15_T19 (SEQ. ID NO:84), D12115_T22 (SEQ. ID NO:85), D12115_T27 (SEQ. ID NO:86), D12115_T3 (SEQ. ID NO:78), D12115_T5 (SEQ. ID NO:79) and D12115_T9 (SEQ. ID NO:80). Table 82 below describes the starting and ending position of this segment on each transcript.

*Table 82 - Segment location on transcripts*

| Transcript name | Segment starting position | Segment ending position |
|---|---|---|
| D12115_T12 (SEQ. ID NO:81) | 2923 | 2972 |
| D12115_T13 (SEQ. ID NO:82) | 2651 | 2700 |
| D12115_T14 (SEQ. ID NO:83) | 2921 | 2970 |
| D12115_T19 (SEQ. ID NO:84) | 2576 | 2625 |
| D12115_T22 (SEQ. ID NO:85) | 3019 | 3068 |
| D12115_T27 (SEQ. ID NO:86) | 2422 | 2471 |
| D12115_T3 (SEQ. ID NO:78) | 2653 | 2702 |
| D12115_T5 (SEQ. ID NO:79) | 2934 | 2983 |
| D12115 T9 (SEQ. ID NO:80) | 3053 | 3102 |

Segment cluster D12115_N55 (SEQ. ID NO:129) according to the present invention is supported by 172 libraries. The number of libraries was determined as previously described. This segment can be found in the following transcript (s): D12115_T12 (SEQ. ID NO:81), D1215_T13 (SEQ. ID NO:82), D12115_T14 (SEQ. ID NO:83), D12115_T19 (SEQ. ID NO:84), D12115_T22 (SEQ. ID NO:85), D12115_T27 (SEQ. ID NO:86), D12115_T5 (SEQ. ID NO:79) and D12115_T9 (SEQ. ID NO:80). Table 83 below describes the starting and ending position of this segment on each transcript.

*Table 83 - Segment location on transcripts*

| Transcript name | Segment starting position | Segment ending position |
|---|---|---|
| D12115_T12 (SEQ. ID NO:81) | 2973 | 3025 |
| D12115_T13 (SEQ. ID NO:82) | 2701 | 2753 |
| D12115_T14 (SEQ. ID NO:83) | 2971 | 3023 |
| D12115_T19 (SEQ. ID NO:84) | 2626 | 2678 |
| D12115 T22 (SEQ. ID NO:85) | 3069 | 3121 |
| D12115_T27 (SEQ. ID NO: 86) | 2472 | 2524 |
| D12115_T5 (SEQ. ID NO:79) | 2984 | 3036 |
| D12115_T9 (SEQ. ID NO:80) | 3103 | 3155 |

Segment cluster D12115_N56 (SEQ. ID NO:130) according to the present invention is supported by 154 libraries. The number of libraries was determined as previously described. This segment can be found in the following transcript (s): D12115_T12 (SEQ. ID NO:81), D12115_T13 (SEQ. ID NO:82), D12115_T14 (SEQ. ID NO:83), D12115_T19 (SEQ. ID NO:84), D12115_T22 (SEQ. ID NO:85), D12115_T27 (SEQ. ID NO:86), D12115_T3 (SEQ. ID NO:78), D12115_T5 (SEQ. ID NO:79) and D12113_T9 (SEQ. ID NO:80). Table 84 below describes the starting and ending position of this segment on each transcript.

*Table 84 - Segment location on transcripts*

| Transcript name | Segment starting position | Segment ending position |
|---|---|---|
| D12115_T12 (SEQ. ID NO:81) | 3026 | 3132 |
| D12115_T13 (SEQ. ID NO:82) | 2754 | 2860 |
| D12115_T14 (SEQ. ID NO:83) | 3024 | 3130 |
| D12115_T19 (SEQ. ID NO:84) | 2679 | 2785 |

(continued)

| Transcript name | Segment starting position | Segment ending position |
|---|---|---|
| D12115_T22 (SEQ. ID NO:85) | 3122 | 3228 |
| D12115_T27 (SEQ. ID NO:86) | 2525 | 2631 |
| D12115_T3 (SEQ. ID NO:78) | 2703 | 2809 |
| D12115_T5 (SEQ. ID NO:79) | 3037 | 3143 |
| D12115_T9 (SEQ. ID NO:80) | 3156 | 3262 |

Expression of Homo sapiens B-factor, properdin (BF) D12115 transcripts which are detectable by junction 0-2 and segment 6 in normal and cancerous ovary and breast tissues

Expression of Homo sapiens B-factor, properdin (BF) transcripts detectable by or according to junction 0-2 and segment 6 was measured with oligonucleotide-based micro-arrays. The results of image intensities for each feature were normalized according to the ninetieth percentile of the image intensities of all the features on the chip. Then, feature image intensities for replicates of the same oligonucleotide on the chip and replicates of the same sample were averaged. Outlying results were discarded.

For every oligonucleotide (D12115_0_24_19 (SEQ. ID NO: 324) and D12115_0_62_120 (SEQ. ID NO: 325)) the calculated intensities in the different tissue samples hybridized are presented in figures figure 12A for ovary samples and figure 12B for breast samples. As is evident from the histogram, the expression of Homo sapiens B-factor, properdin (BF) transcripts detectable with the above oligonucleotides in ovary and breast cancer samples was higher than in the normal samples.

D12115_0_24_19 (SEQ. ID NO: 324) AGTGGGCACTCGGCTCCGGACACTGTAACTCTTGCTCTCTACCTTGCTCA

DI2115_0_62_120 (SEQ. ID NO: 325) ATGCCCTTTATCTTGGGCCTCTTGTCTGGAGGTGTGACCACCACTCCATG

Expression of Homo sapiens B-factor, properdin (BF) D12115 transcripts, detectable by or according to D12115_seg4 (SEQ. ID NO: 146) amplicon and primers D12115_seg4F (SEQ. ID NO: 144) and D12115_seg4R (SEQ. ID NO: 145); or of Homo sapiens B-factor, properdin (BF) D12115 transcripts, detectable by or according to D12115_seg6 (SEQ. ID NO: 149) amplicon and primers D12115_seg6F (SEQ. ID NO: 147) and D12115_seg6R (SEQ. ID NO: 148); or Homo sapiens B-factor, properdin (BF) transcripts detectable by or according to seg40WT - D12115_seg40WT (SEQ. ID NO: 152) amplicon and primers D12115_seg40WTF (SEQ. ID NO: 150) and D12115_seg40WTR (SEQ. ID NO: 151); or Homo sapiens B-factor, properdin (BF) transcripts detectable by or according to seg46-47 - D12115_seg46-47 (SEQ. ID NO: 155) amplicon and primers D12115_seg46-47F (SEQ. ID NO: 153) and D12115_seg46-47R (SEQ. ID NO: 154); or Homo sapiens B-factor, properdin (BF) transcripts detectable by or according to seg27 and seg34 - *D12115seg27 (SEQ. ID NO: 320) and D12115seg34 (SEQ. ID NO: 323)* amplicons and primers *D12115seg27F (SEQ. ID NO: 318), D12115seg27R (SEQ. ID NO: 319), D12115seg34F (SEQ. ID NO: 321) and D12115seg34R (SEQ. ID NO: 322)* was measured by real time PCR.

The sequences of corresponding primers and amplicons are given below. Forward Primer D12115_seg4F (SEQ. ID NO: 144) (SEQ. ID NO:144):

GTTTGAGGGCAATGAGTGTG

Reverse Primer D12115_seg4R (SEQ. ID NO:145): AAACTGCTCCTACTCCCGGTC

Amplicon D12115_seg4 (SEQ. ID NO:146):

GTTTGAGGGCAATGAGTGTGGGCAGTGGCCTAAGGCAGAAACAGGGCAGGCGGCAGCAA
GGTCAGGACTAGGATGAGACTAGGCAGGGTGACAAGGTGGGCTGACCGGGAGTAGGAGC
AGTTT

Forward Primer D12115_seg6F (SEQ. ID NO:147):CTACATTGCTGTCTCCCTGACG

Reverse Primer D12115_seg6R (SEQ. ID NO:148): AGGTAAGCACTGAAGCCTGAGG

Amplicon D12115_seg6 (SEQ. ID NO:149):

CTACATTGCTGTCTCCCTGACGGCGCCCAGCCCGAGGAGTGGGCACTCGGCTCCGGACAC
TGTAACTCTTGCTCTCTACCTTGCTCACGGGGCCTCAGGCTTCAGTGCTTACCT

Forward Primer D12115_seg40WTF (SEQ. ID NO:150):AGGCAACACCTCCCACTTTCT
Reverse Primer D12115_seg40WTR (SEQ. ID NO: 51):TTCACGTCTTCCCCCATCC
Amplicon D12115_seg40WT (SEQ. ID NO:152):

AGGCAACACCTCCCACTTTCTACAGATCCTACACTCCACCCATCCTCAATGCAGCCCCATT
CCTTGCACCCCAGACCAGTCAGGGATGGGGGAAGACGTGA

Forward Primer D12115_seg46-47F (SEQ. ID NO:153):GAAGAGCTGCTCCCTGCA
Reverse Primer D12115_seg46-47R (SEQ. ID NO:154): CCCCATTCTTGATGTAGACCTC
Amplicon (D12115_seg46-47 (SEQ. ID NO: 155):

GAAGAGCTGCTCCCTGCACAGGATATCAAAGCTCTGTTTGTGTCTGAGGAGGAGAAAAA
GCTGACTCGGAAGGAGGTCTACATCAAGAATGGGG

Forward Primer D12115seg27F (SEQ. ID NO:318): TGTCCCAGCCTCCCCAC
Reverse Primer D12115seg27R (SEQ. ID NO:319): GAGTCACATTCAGGGCCCC
Amplicon D12115seg27 (SEQ. ID NO:320):

TGTCCCAGCCTCCCCACCTTCTCAGACCAGCATGTGGCCCTTAAGTCCACTTGTAACACTA
TACCCATGGTTGGGGCCCTGAATGTGACTC

Forward Primer D12115seg34F (SEQ. ID NO: 321): CAACTCTCCTCAGGTTCCCCT
Reverse Primer D12115seg34R (SEQ. ID NO:322): GAGAAGGAGGAATGAAGAAGGCTT
Amplicon D12115seg34 (SEQ. ID NO:323):

CAACTCTCCTCAGGTTCCCCTGAAGTAATTCATTCTTCCTCTACACCTGAAGCTCTAGTTG
CCTGGAAAGCCTTCTTCATTCCTCCTTCTC

Expression of Homo sapiens B-factor, properdin (BF) D12115 transcripts which are detectable by amplicon as depicted in sequence name D12115_seg4 (SEQ. ID NO: 146) in normal and cancerous Breast tissues

Expression of Homo sapiens B-factor, properdin (BF) transcripts detectable by or according to seg4 - D12115_seg4 (SEQ. ID NO: 146) amplicon and primers D12115_seg4F (SEQ. ID NO: 144) and D12115_seg4R (SEQ. ID NO: 145) was measured by real time PCR. In parallel the expression of four housekeeping genes - G6PD (GenBank Accession No. NM_000402 (SEQ. ID NO: 13); G6PD amplicon (SEQ. ID NO: 16)), HPRT1 (GenBank Accession No. NM_000194 (SEQ. ID NO: 5); amplicon - HPRT1-amplicon (SEQ. ID NO: 8)), PBGD (GenBank Accession No. BC019323 (SEQ. ID NO: 1); amplicon - PBGD-amplicon (SEQ. ID NO: 4)) and SDHA (GenBank Accession No. NM_004168 (SEQ. ID NO: 33); amplicon - SDHA-amplicon (SEQ. ID NO:36) ) was measured similarly. For each RT sample, the expression of the above amplicon was normalized to the geometric mean of the quantities of the housekeeping genes. The normalized quantity of each RT sample was then divided by the median of the quantities of the normal post-mortem (PM) samples (sample numbers 57, 59, 60, 63, 66, 64, 56, 65, 67 and 58, Table 1_3 above), to obtain a value of fold up-regulation for each sample relative to median of the normal PM samples.

Figure 13 is a histogram showing over expression of the above-indicated Homo sapiens B-factor, properdin (BF) transcripts in cancerous Breast samples relative to the normal samples.

As is evident from Figure 13, the expression of Homo sapiens B-factor, properdin (BF) transcripts detectable by the above amplicon in cancer samples was higher than in the non-cancerous samples (sample numbers 57, 59, 60, 63, 66, 64, 56, 65, 67 and 58, Table 1_3 above). Notably an over-expression of at least 5 fold was found in 10 out of 28 adenocarcinoma samples.

Primer pairs are also optionally and preferably encompassed within the present invention; for example, for the above experiment, the following primer pair was used as a non-limiting illustrative example only of a suitable primer pair:

D12115_seg4F (SEQ. ID NO: 144) forward primer; and D12115_seg4R (SEQ. ID NO: 145) reverse primer.

The present invention also preferably encompasses any amplicon obtained through the use of any suitable primer pair; for example, for the above experiment, the following amplicon was obtained as a non-limiting illustrative example only of a suitable amplicon: D12115_seg4 (SEQ. ID NO: 146).

Expression of Homo sapiens B-factor, properdin (BF) D12115 transcripts which are detectable by amplicon as depicted in sequence name D12115_seg4 (SEQ. ID NO: 146) in different normal tissues

Expression of Homo sapiens B-factor, properdin (BF) transcripts detectable by or according to seg4 - D12115_seg4 (SEQ. ID NO: 146) amplicon and primers D12115_seg4F (SEQ. ID NO: 144) and D12115_seg4R (SEQ. ID NO: 145) was measured by real time PCR. In parallel the expression of four housekeeping genes - SDHA (GenBank Accession No. NM_004168 (SEQ. ID NO: 33); amplicon - SDHA-amplicon (SEQ. ID NO:36) ), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24) ) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the median of the quantities of the ovary samples (sample numbers 19 and 20, Table 1_6 above), to obtain a value of relative expression of each sample relative to median of the ovary samples.

Figure 14 is a histogram showing expression of the Homo sapiens B-factor, properdin (BF) D12115 transcripts which are detectable by amplicon as depicted in sequence name D12115_seg4 (SEQ. ID NO: 146) in different normal samples.

Expression of Homo sapiens B-factor, properdin (BF) D12115 transcripts which are detectable by amplicon as depicted in sequence name D12115_seg4 (SEQ. ID NO: 146) in normal and cancerous Ovary tissues

Expression of Homo sapiens B-factor, properdin (BF) transcripts detectable by or according to seg4 - D12115_seg4 (SEQ. ID NO: 146) amplicon and primers D12115_seg4F (SEQ. ID NO: 144) and D12115_seg4R (SEQ. ID NO: 145) was measured by real time PCR. In parallel the expression of four housekeeping genes - SDHA (GenBank Accession No. NM_004168 (SEQ. ID NO: 33); amplicon - SDHA-amplicon (SEQ. ID NO:36) ), HPRT1 (GenBank Accession No. NM_000194 (SEQ. ID NO: 5); amplicon - HPRT1-amplicon (SEQ. ID NO: 8)), PBGD (GenBank Accession No. BC019323 (SEQ. ID NO: 1); amplicon - PBGD-amplicon (SEQ. ID NO: 4)) and GAPDH (GenBank Accession No. BC026907 (SEQ. ID NO: 9); GAPDH amplicon (SEQ. ID NO: 12)) was measured similarly. For each RT sample, the expression of the above amplicon was normalized to the geometric mean of the quantities of the housekeeping genes. The normalized quantity of each RT sample was then divided by the median of the quantities of the normal post-mortem (PM) samples (sample numbers 45, 46, 71 and 48, Table 1_1 above), to obtain a value of fold up-regulation for each sample relative to median of the normal PM samples.

Figure 15 is a histogram showing over expression of the above-indicated Homo sapiens B-factor, properdin (BF) transcripts in cancerous Ovary samples relative to the normal samples.

As is evident from Figure 15, the expression of Homo sapiens B-factor, properdin (BF) transcripts detectable by the above amplicon in serous carcinoma samples was higher than in the non-cancerous samples (sample numbers 45, 46, 71 and 48, Table 1_1 above). Notably an over-expression of at least 5 fold was found in 12 out of 43 adenocarcinoma samples, specifically in 11 out of 30 serous carcinoma samples.

Statistical analysis was applied to verify the significance of these results, as described below.

The P value for the difference in the expression levels of Homo sapiens B-factor, properdin (BF) transcripts detectable by the above amplicon in Ovary serous carcinoma samples versus the normal tissue samples was determined by T test as 2.19e-04. The P value for the difference in the expression levels of Homo sapiens B-factor, properdin (BF) transcripts detectable by the above amplicon in Ovary adenocarcinoma samples versus the normal tissue samples was determined by T test as 1.75e-04.

The above values demonstrate statistical significance of the results.

Primer pairs are also optionally and preferably encompassed within the present invention; for example, for the above experiment, the following primer pair was used as a non-limiting illustrative example only of a suitable primer pair: D12115_seg4F (SEQ. ID NO: 144) forward primer; and D12115_seg4R (SEQ. ID NO: 145) reverse primer.

The present invention also preferably encompasses any amplicon obtained through the use of any suitable primer pair; for example, for the above experiment, the following amplicon was obtained as a non-limiting illustrative example only of a suitable amplicon: D12115_seg4 (SEQ. ID NO: 146).

Expression of Homo sapiens B-factor, properdin (BF) D12115 transcripts which are detectable by amplicon as depicted in sequence name D12115_seg6 (SEQ. ID NO: 149) in normal and cancerous Breast tissues

Expression of Homo sapiens B-factor, properdin (BF) transcripts detectable by or according to seg6 - D12115_seg6 (SEQ. ID NO: 149) amplicon and primers D12115_seg6F (SEQ. ID NO: 147) and D12115_seg6R (SEQ. ID NO: 148) was measured by real time PCR. In parallel the expression of four housekeeping genes - G6PD (GenBank Accession No. NM_000402 (SEQ. ID NO: 13); G6PD amplicon (SEQ. ID NO: 16)), HPRT1 (GenBank Accession No.

NM_000194 (SEQ. ID NO: 5); amplicon - HPRT1-amplicon (SEQ. ID NO: 8)), PBGD (GenBank Accession No. BC019323 (SEQ. ID NO: 1); amplicon - PBGD-amplicon (SEQ. ID NO: 4)) and SDHA (GenBank Accession No. NM_004168 (SEQ. ID NO: 33); amplicon - SDHA-amplicon (SEQ. ID NO:36) ) was measured similarly. For each RT sample, the expression of the above amplicon was normalized to the geometric mean of the quantities of the housekeeping genes. The normalized quantity of each RT sample was then divided by the median of the quantities of the normal post-mortem (PM) samples (sample numbers 57, 59, 60, 63, 66, 64, 56, 65, 67 and 58, Table 1_3 above), to obtain a value of fold up-regulation for each sample relative to median of the normal PM samples.

Figure 16 is a histogram showing over expression of the above-indicated Homo sapiens B-factor, properdin (BF) transcripts in cancerous Breast samples relative to the normal samples.

As is evident from Figure 16, the expression of Homo sapiens B-factor, properdin (BF) transcripts detectable by the above amplicon in cancer samples was higher than in the non-cancerous samples (sample numbers 57, 59, 60, 63, 66, 64, 56, 65, 67 and 58, Table 1_3 above). Notably an over-expression of at least 5 fold was found in 14 out of 28 adenocarcinoma samples.

Primer pairs are also optionally and preferably encompassed within the present invention; for example, for the above experiment, the following primer pair was used as a non-limiting illustrative example only of a suitable primer pair: D12115_seg6F (SEQ. ID NO: 147) forward primer; and D12115_seg6R (SEQ. ID NO: 148) reverse primer.

The present invention also preferably encompasses any amplicon obtained through the use of any suitable primer pair; for example, for the above experiment, the following amplicon was obtained as a non-limiting illustrative example only of a suitable amplicon: D12115_seg6 (SEQ. ID NO: 149).

Expression of Homo sapiens B-factor, properdin (BF) D12115 transcripts which are detectable by amplicon as depicted in sequence name D12115_seg6 (SEQ. ID NO: 149) in normal and cancerous Lung tissues

Expression of Homo sapiens B-factor, properdin (BF) transcripts detectable by or according to seg6 - D12115_seg6 (SEQ. ID NO: 149) amplicon and primers D12115_seg6F (SEQ. ID NO: 147) and D12115_seg6R (SEQ. ID NO: 148) was measured by real time PCR. In parallel the expression of four housekeeping genes - HPRT1 (GenBank Accession No. NM_000194 (SEQ. ID NO: 5); amplicon - HPRT1-amplicon (SEQ. ID NO: 8)), PBGD (GenBank Accession No. BC019323 (SEQ. ID NO: 1); amplicon - PBGD-amplicon (SEQ. ID NO: 4)), SDHA (GenBank Accession No. NM_ 004168 (SEQ. ID NO: 33); amplicon - SDHA-amplicon (SEQ. ID NO:36) ) and Ubiquitin (GenBank Accession No. BC000449 (SEQ. ID NO: 29); amplicon - Ubiquitin-amplicon (SEQ. ID NO: 32)) was measured similarly. For each RT sample, the expression of the above amplicon was normalized to the geometric mean of the quantities of the housekeeping genes. The normalized quantity of each RT sample was then divided by the median of the quantities of the normal post-mortem (PM) samples (sample numbers 47, 48, 49, 50, 90, 91, 92, 93, 96, 97, 98 and 99, Table 1_2 above), to obtain a value of fold up-regulation for each sample relative to median of the normal PM samples.

Figure 17 is a histogram showing over expression of the above-indicated Homo sapiens B-factor, properdin (BF) transcripts in cancerous Lung samples relative to the normal samples.

As is evident from Figure 17, the expression of Homo sapiens B-factor, properdin (BF) transcripts detectable by the above amplicon in adenocarcinoma and squamous cell carcinoma was higher than in the non-cancerous samples (sample numbers 47, 48, 49, 50, 90, 91, 92, 93, 96, 97, 98 and 99, Table 1_2 above). Notably an over-expression of at least 5 fold was found in 8 out of 15 adenocarcinoma samples and in 3 out of 16 squamous cell carcinoma.

Statistical analysis was applied to verify the significance of these results, as described below.

The P value for the difference in the expression levels of Homo sapiens B-factor, properdin (BF) transcripts detectable by the above amplicon in Lung non-small cell carcinoma samples versus the normal tissue samples was determined by T test as 2.64e-02.

Threshold of 5 fold over expression was found to differentiate between adenocarcinoma and normal samples with P value of 2.90e-03 as checked by exact Fisher test. Threshold of 5 fold over expression was found to differentiate between non-small cell carcinoma and normal samples with P value of 2.40e-02 as checked by exact Fisher test.

The above values demonstrate statistical significance of the results.

Primer pairs are also optionally and preferably encompassed within the present invention; for example, for the above experiment, the following primer pair was used as a non-limiting illustrative example only of a suitable primer pair: D12115_seg6F (SEQ. ID NO: 147) forward primer; and D12115_seg6R (SEQ. ID NO: 148) reverse primer.

The present invention also preferably encompasses any amplicon obtained through the use of any suitable primer pair; for example, for the above experiment, the following amplicon was obtained as a non-limiting illustrative example only of a suitable amplicon: D12115_seg6 (SEQ. ID NO: 149).

***Expression of Homo sapiens B-factor, properdin (BF) D12115 transcripts which are detectable by amplicon as depicted in sequence name D12115_seg6 (SEQ. ID NO: 149) in different normal tissues***

Expression of Homo sapiens B-factor, properdin (BF) transcripts detectable by or according to seg6 - D12115_seg6 (SEQ. ID NO: 149) amplicon and primers D12115_seg6F (SEQ. ID NO: 147) and D12115_seg6R (SEQ.

ID NO: 148) was measured by real time PCR. In parallel the expression of four housekeeping genes - SDHA (GenBank Accession No. NM_004168 (SEQ. ID NO: 33); amplicon - SDHA-amplicon (SEQ. ID NO:36) ), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24) ) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the median of the quantities of the ovary samples (sample numbers 19 and 20, Table 1_6 above), to obtain a value of relative expression of each sample relative to median of the ovary samples.

Figure 18 is a histogram showing expression of the Homo sapiens B-factor, properdin (BF) D12115 transcripts which are detectable by amplicon as depicted in sequence name D12115_seg6 (SEQ. ID NO: 149) in different normal samples.

Expression of Homo sapiens B-factor, properdin (BF) D12115 transcripts which are detectable by amplicon as depicted in sequence name D12115_seg6 (SEQ. ID NO: 149) in normal and cancerous Ovary tissues

Expression of Homo sapiens B-factor, properdin (BF) transcripts detectable by or according to seg6 - D12115_seg6 (SEQ. ID NO: 149) amplicon and primers D12115_seg6F (SEQ. ID NO: 147) and D12115_seg6R (SEQ. ID NO: 148) was measured by real time PCR. In parallel the expression of four housekeeping genes - SDHA (GenBank Accession No. NM_004168 (SEQ. ID NO: 33); amplicon - SDHA-amplicon (SEQ. ID NO:36) ), HPRT1 (GenBank Accession No. NM_000194 (SEQ. ID NO: 5); amplicon - HPRTl-amplicon (SEQ. ID NO: 8)), PBGD (GenBank Accession No. BC019323 (SEQ. ID NO: 1); amplicon - PBGD-amplicon (SEQ. ID NO: 4)) and GAPDH (GenBank Accession No. BC026907 (SEQ. ID NO: 9); GAPDH amplicon (SEQ. ID NO: 12)) was measured similarly. For each RT sample, the expression of the above amplicon was normalized to the geometric mean of the quantities of the housekeeping genes. The normalized quantity of each RT sample was then divided by the median of the quantities of the normal post-mortem (PM) samples (sample numbers 45, 46, 71 and 48, Table 1_1 above), to obtain a value of fold up-regulation for each sample relative to median of the normal PM samples.

Figure 19 is a histogram showing over expression of the above-indicated Homo sapiens B-factor, properdin (BF) transcripts in cancerous Ovary samples relative to the normal samples. Values represent the average of duplicate experiments. Error bars indicate the minimal and maximal values obtained.

As is evident from Figure 19, the expression of Homo sapiens B-factor, properdin (BF) transcripts detectable by the above amplicon in serous carcinoma samples was significantly higher than in the non-cancerous samples (sample numbers 45, 46, 71 and 48, Table 1_1 above). Notably an over-expression of at least 5 fold was found in 20 out of 43 adenocarcinoma samples, specifically in 18 out of 30 serous carcinoma samples.

Statistical analysis was applied to verify the significance of these results, as described below.

The P value for the difference in the expression levels of Homo sapiens B-factor, properdin (BF) transcripts detectable by the above amplicon in Ovary serous carcinoma samples versus the normal tissue samples was determined by T test as 1.60e-04. The P value for the difference in the expression levels of Homo sapiens B-factor, properdin (BF) transcripts detectable by the above amplicon in Ovary adenocarcinoma samples versus the normal tissue samples was determined by T test as 1.28e-04.

Threshold of 5 fold over expression was found to differentiate between serous carcinoma and normal samples with P value of 3.92e-02 as checked by exact Fisher test.

The above values demonstrate statistical significance of the results.

Primer pairs are also optionally and preferably encompassed within the present invention; for example, for the above experiment, the following primer pair was used as a non-limiting illustrative example only of a suitable primer pair: D12115_seg6F (SEQ. ID NO: 147) forward primer; and D12115_seg6R (SEQ. ID NO: 148) reverse primer.

The present invention also preferably encompasses any amplicon obtained through the use of any suitable primer pair; for example, for the above experiment, the following amplicon was obtained as a non-limiting illustrative example only of a suitable amplicon: D12115_seg6 (SEQ. ID NO: 149).

Expression of Homo sapiens B-factor, properdin (BF) D12115 transcripts which are detectable by amplicon as depicted in sequence name D12115_seg40WT (SEQ. ID NO: 152) in different normal tissues

Expression of Homo sapiens B-factor, properdin (BF) transcripts detectable by or according to seg40WT - D12115_seg40WT (SEQ. ID NO: 152) amplicon and primers D12115_seg40WTF (SEQ. ID NO: 150) and D12115_seg40WTR (SEQ. ID NO: 151) was measured by real time PCR. In parallel the expression of four housekeeping genes - SDHA (GenBank Accession No. NM_004168 (SEQ. ID NO: 33); amplicon - SDHA-amplicon (SEQ. ID NO:36) ), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24) ) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the median of the quantities of the ovary samples (sample numbers 19 and 20, Table 1_6 above), to obtain a value of relative expression of each sample relative to median of the ovary samples.

Figure 20 is a histogram showing expression of the Homo sapiens B-factor, properdin (BF) D12115 transcripts which are detectable by amplicon as depicted in sequence name D12115 seg40WT (SEQ. ID NO: 152) in different normal samples.

Expression of Homo sapiens B-factor, properdin (BF) D12115 transcripts which are detectable by amplicon as depicted in sequence name D12115_seg46-47 (SEQ. ID NO: 155) in different normal tissues

Expression of Homo sapiens B-factor, properdin (BF) transcripts detectable by or according to seg46-47 - D12115_seg46-47 (SEQ. ID NO: 155) amplicon and primers D12115_seg46-47F (SEQ. ID NO: 153) and D12115_seg46-47R (SEQ. ID NO: 154) was measured by real time PCR. In parallel the expression of four housekeeping genes - SDHA (GenBank Accession No. NM_004168 (SEQ. ID NO: 33); amplicon - SDHA-amplicon (SEQ. ID NO:36), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24) ) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the median of the quantities of the ovary samples (sample numbers 19 and 20, Table 1_6 above), to obtain a value of relative expression of each sample relative to median of the ovary samples.

Figure 21 is a histogram showing expression of the Homo sapiens B-factor, properdin (BF) D12115 transcripts which are detectable by amplicon as depicted in sequence name D12115_ seg46-47 (SEQ. ID NO: 155) in different normal samples.

Expression of Homo sapiens B-factor, properdin (BF) D12115 transcripts which are detectable by amplicon as depicted in sequence name D12115_seg46-47 (SEQ. ID NO: 155) in normal and cancerous Ovary tissues

Expression of Homo sapiens B-factor, properdin (BF) transcripts detectable by or according to seg46-47 - D12115_seg46-47 (SEQ. ID NO: 155) amplicon and primers D12115_seg46-47F (SEQ. ID NO: 153) and D12115_seg46-47R (SEQ. ID NO: 154) was measured by real time PCR. In parallel the expression of four housekeeping genes - SDHA (GenBank Accession No. NM_004168 (SEQ. ID NO: 33); amplicon - SDHA-amplicon (SEQ. ID NO:36) ), HPRT1 (GenBank Accession No. NM_000194 (SEQ. ID NO: 5); amplicon - HPRT1-amplicon (SEQ. ID NO: 8)), PBGD (GenBank Accession No. BC019323 (SEQ. ID NO: 1); amplicon - PBGD-amplicon (SEQ. ID NO: 4)) and GAPDH (GenBank Accession No. BC026907 (SEQ. ID NO: 9); GAPDH amplicon (SEQ. ID NO: 12)) was measured similarly. For each RT sample, the expression of the above amplicon was normalized to the geometric mean of the quantities of the housekeeping genes. The normalized quantity of each RT sample was then divided by the median of the quantities of the normal post-mortem (PM) samples (sample numbers 45, 46, 71 and 48, Table 1_1 above), to obtain a value of fold up-regulation for each sample relative to median of the normal PM samples.

Figure 22 is a histogram showing over expression of the above-indicated Homo sapiens B-factor, properdin (BF) transcripts in cancerous Ovary samples relative to the normal samples.

As is evident from Figure 22, the expression of Homo sapiens B-factor, properdin (BF) transcripts detectable by the above amplicon in serous carcinoma samples was higher than in the non-cancerous samples (sample numbers 45, 46, 71 and 48, Table 1_1 above). Notably an over-expression of at least 5 fold was found in 11 out of 43 adenocarcinoma samples, specifically in 10 out of 30 serous carcinoma samples.

Statistical analysis was applied to verify the significance of these results, as described below.

The P value for the difference in the expression levels of Homo sapiens B-factor, properdin (BF) transcripts detectable by the above amplicon in Ovary serous carcinoma samples versus the normal tissue samples was determined by T test as 3.19e-04. The P value for the difference in the expression levels of Homo sapiens B-factor, properdin (BF) transcripts detectable by the above amplicon in Ovary adenocarcinoma samples versus the normal tissue samples was determined by T test as 1.11e-03.

The above values demonstrate statistical significance of the results.

Primer pairs are also optionally and preferably encompassed within the present invention; for example, for the above experiment, the following primer pair was used as a non-limiting illustrative example only of a suitable primer pair: D12115_seg46-47F (SEQ. ID NO: 153) forward primer; and D12115_seg46-47R (SEQ. ID NO: 154) reverse primer.

The present invention also preferably encompasses any amplicon obtained through the use of any suitable primer pair; for example, for the above experiment, the following amplicon was obtained as a non-limiting illustrative example only of a suitable amplicon: D12115_seg46-47 (SEQ. ID NO: 155).

Expression of Homo sapiens B-factor, properdin (BF) D12115 transcripts which are detectable by amplicons as depicted in sequence names D12115seg4 (SEQ. ID NO: 146) and D12115seg6 (SEQ. ID NO: 149) in normal and cancerous colon tissues

Expression of *Homo sapiens B-factor, properdin (BF)* transcripts detectable by or according to seg4 and seg6 - *D12115seg4 (SEQ. ID NO: 146) and D12115seg6 (SEQ. ID NO: 149)* amplicons and primers *D12115seg4F (SEQ.*

*ID NO: 144), D12115seg4R (SEQ. ID NO: 145), D12115seg6F (SEQ. ID NO: 147) and D12115seg6R (SEQ. ID NO:* 148) was measured by real time PCR. In parallel expression of several housekeeping genes as detailed in "Materials and Experimental Procedures" section herein, was measured similarly. For each RT sample, the expression of the above amplicons was normalized to the normalization factor calculted from the expression of the house keeping genes that were selected for colon tissue panel, as described in normalization method 2 in the "materials and methods" section. The normalized quantity of each RT sample was then divided by the median of the quantities of the normal samples of colon panel (Table 1_4 above), to obtain a value of fold differential expression for each sample relative to median of the normal samples.

In one experiment that was carried out with *D12115seg4 (SEQ. ID NO: 146) and D12115seg6 (SEQ. ID NO: 149)* no differential expression in the colon cancerous samples relative to the normal samples was observed.

Expression of Homo sapiens B-factor, properdin (BF) D12115 transcripts which are detectable by amplicons as depicted in sequence names D12115seg27 (SEQ. ID NO: 320) and D12115seg34 (SEQ. ID NO: 323) in normal and cancerous tissues

Expression of Homo sapiens B-factor, properdin (BF) transcripts detectable by or according to seg27 and *seg*34 *- D12115seg27 (SEQ. ID NO: 320) and D12115seg34 (SEQ. ID NO:* 323) amplicons and primers *D12115seg27F (SEQ. ID NO: 318), D12115seg27R (SEQ. ID NO: 319), D12115seg34F (SEQ. ID NO: 321) and D12115seg34R (SEQ. ID NO:* 322) was measured by real time PCR. In parallel expression of several housekeeping genes, as detailed in "Materials and Experimental Procedures" section above, was measured similarly. For each RT sample, the expression of the above amplicons was normalized to the normalization factor calculted from the expression of the house keeping genes that were selected for the relevant tissue panel, as described in normalization method 2 in the "materials and methods" section. The normalized quantity of each RT sample was then divided by the median of the quantities of the normal samples of the relevant panel (Tables 1_4, 1_3, 1_1 and 1_2 above), to obtain a value of fold differential expression for each sample relative to median of the normal samples.

In one experiment that was carried out with *D12115seg27 (SEQ. ID NO: 320) and D12115seg34 (SEQ. ID NO: 323)* no differential expression in the colon cancerous samples relative to the normal samples was observed.

In one experiment that was carried out with *D12115seg27 (SEQ. ID NO: 320) and D12115seg34 (SEQ. ID NO: 323)* no differential expression in the breast cancerous samples relative to the normal samples was observed.

In one experiment that was carried out with *D12115seg27 (SEQ. ID NO: 320) and D12115seg34 (SEQ. ID NO: 323)* no differential expression in the ovary cancerous samples relative to the normal samples was observed.

In one experiment that was carried out with *D12115seg27 (SEQ. ID NO: 320) and D12115seg34 (SEQ. ID NO: 323)* no differential expression in the lung cancerous samples relative to the normal samples was observed.

DESCRIPTION FOR CLUSTER C03950

Cluster C03950 features 15 transcript(s) and 38 segment(s) of interest, the names for which are given in Tables 85 and 86, respectively. The selected protein variants are given in table 87.

*Table 85 - Transcripts of interest*

| Transcript Name |
|---|
| C03950_3_T2 (SEQ. ID NO:156) |
| C03950_3_T4 (SEQ. ID NO:157) |
| C03950_3_T7 (SEQ. ID NO:158) |
| C03950_3_T8 (SEQ. ID NO:159) |
| C03950_3_T9 (SEQ. ID NO:160) |
| C03950_3_T10 (SEQ. ID NO:161) |
| C03950_3_T11 (SEQ. ID NO:162) |
| C03950_3_T13 (SEQ. ID NO:163) |
| C03950_3_T15 (SEQ. ID NO:164) |
| C03950_3_T17 (SEQ. ID NO:165) |
| C03950_3_T18 (SEQ. ID NO:166) |
| C03950_3_T19 (SEQ.IDNO:167) |

(continued)

| Transcript Name |
| --- |
| C03950_3_T21 (SEQ. ID NO:168) |
| C03950_3_T22 (SEQ. ID NO:169) |
| C03950_3_T23 (SEQ. ID NO:170) |

*Table 86 - Segments of interest*

| Segment Name |
| --- |
| C03950_3_N2 (SEQ. ID NO:171) |
| C03950_3_N6 (SEQ. ID NO:172) |
| C03950_3_N23 (SEQ. ID NO:173) |
| C03950_3_N27(SEQ. ID NO:174) |
| C03950_3_N33 (SEQ. ID NO:175) |
| C03950_3_N44 (SEQ. ID NO:176) |
| C03950_3_N45 (SEQ. ID NO:177) |
| C03950_3_N48 (SEQ. ID NO:178) |
| C03950_3_N49 (SEQ. ID NO:179) |
| C03950_3_N56 (SEQ. ID NO:180) |
| C03950_3_N62 (SEQ. ID NO:181) |
| C03950_3_N63 (SEQ. ID NO:182) |
| C03950_3_N67 (SEQ. ID NO:183) |
| C03950_3_N71 (SEQ. ID NO:184) |
| C03950_3_N77 (SEQ. ID NO:185) |
| C03950_3_N0 (SEQ. ID NO:186) |
| C03950_3_N4 (SEQ. ID NO:187) |
| C03950_3_N9 (SEQ. ID NO:188) |
| C03950_3_N13 (SEQ. ID NO:189) |
| C03950_3_N15 (SEQ. ID NO:190) |
| C03950_3_N17 (SEQ. ID NO:191) |
| C03950_3_N19 (SEQ. ID NO:192) |
| C03950_3_N21 (SEQ. ID NO:193) |
| C03950_3_N29 (SEQ. ID NO:194) |
| C03950_3_N31 (SEQ. ID NO:195) |
| C03950_3_N35 (SEQ. ID NO:196) |
| C03950_3_N37 (SEQ. ID NO:197) |
| C03950_3_N39 (SEQ. ID NO:198) |
| C03950_3_N40 (SEQ. ID NO:199) |
| C03950_3_N42 (SEQ. ID NO:200) |
| C03950_3_N47 (SEQ. ID NO:201) |
| C03950_3_N51 (SEQ. ID NO:202) |

(continued)

| Segment Name |
|---|
| C03950_3_N58 (SEQ. ID NO:203) |
| C03950_3_N60 (SEQ. ID NO:204) |
| C03950_3_N65 (SEQ. ID NO:205) |
| C03950_3_N69 (SEQ. ID NO:206) |
| C03950_3_N73 (SEQ. ID NO:207) |
| C03950_3_N75 (SEQ. ID NO:208) |

*Table 87 - Proteins of interest*

| Protein Name | Corresponding Transcript(s) |
|---|---|
| C03950_3_P5 (SEQ. ID NO:212) | C03950_3_T2 (SEQ. ID NO:156) |
| C03950_3_P7 (SEQ. ID NO:213) | C03950_3_T4 (SEQ.IDNO:157) |
| C03950_3_P9 (SEQ. ID NO:214) | C03950_3_T7 (SEQ. ID NO:158) |
| C03950_3_P10 (SEQ. ID NO:215) | C03950_3_T8 (SEQ. ID NO:159) |
| C03950_3_P11 (SEQ. ID NO:216) | C03950_3_T9 (SEQ. ID NO:160) |
| C03950_3_P12 (SEQ. ID NO:217) | C03950_3_T10 (SEQ. ID NO:161) |
| C03950_3_P13 (SEQ. ID NO:218) | C03950_3_T11 (SEQ. ID NO:162) |
| C03950_3_P15 (SEQ. ID NO:219) | C03950_3_T13 (SEQ. ID NO:163) |
| C03950_3_P17 (SEQ. ID NO:220) | C03950_3_T15 (SEQ. ID NO:164) |
| C03950_3_P19 (SEQ. ID NO:221) | C03950_3_T17 (SEQ. ID NO:165) |
| C03950_3_P20 (SEQ. ID NO:222) | C03950_3_T18 (SEQ. ID NO:166) |
| C03950_3_P21 (SEQ. ID NO:223) | C03950_3_T19 (SEQ. ID NO:167) |
| C03950_3_P23 (SEQ. ID NO:224) | C03950_3_T21 (SEQ. ID NO:168) |
| C03950_3_P24 (SEQ. ID NO:225) | C03950_3_T22 (SEQ. ID NO:169) |
| C03950_3_P25 (SEQ. ID NO:226) | C03950_3_T23 (SEQ. ID NO:170) |
| C03950_3_P28 (SEQ. ID NO:227) | C03950_3_T2 (SEQ. ID NO:156) |
| C03950_3_P31 (SEQ. ID NO:228) | C03950_3_T10 (SEQ. ID NO:161) |
| C03950_3_P33 (SEQ. ID NO:229) | C03950_3_T13 (SEQ. ID NO:163) |
| C03950_3_P35 (SEQ. ID NO:230) | C03950_3_T18 (SEQ. ID NO:166) |

These sequences are variants of the known protein Serine/threonine-protein kinase TNNI3K (SEQ. ID NO: 209) (SwissProt accession identifier TNI3K_HUMAN (SEQ. ID NO: 396); known also according to the synonyms EC 2.7.1.37; TNNI3- interacting kinase; Cardiac ankyrin repeat kinase), referred to herein as the previously known protein.

The variants C03950-T10 (SEQ. ID NO: 161) and C03950_T18 (SEQ. ID NO: 166) were previously disclosed by the inventors in published PCT application no WO0603527, and the variants C03950_T17 (SEQ. ID NO: 165), C03950_P19 (SEQ. ID NO: 221) and C03950_P35 (SEQ. ID NO: 230) were previously disclosed by the inventors in published PCT application no WO2005/071058, hereby incorporated by reference as if fully set forth herein, but have now been shown to have novel and surprising diagnostic uses as described herein for other variants of cluster C03950.

According to optional but preferred embodiments of the present invention, variants of this cluster according to the present invention (amino acid and/or nucleic acid sequences of C03950) may optionally have one or more of the following utilities. It should be noted that these utilities are optionally and preferably suitable for human and non-human animals as subjects, except where otherwise noted. The reasoning is described with regard to biological and/or physiological and/or other information about the known protein, but is given to demonstrate particular diagnostic utility for the variants according to the present invention.

A non-limiting example of such a utility is the detection, diagnosis and/or determination of risk of melanoma. The method comprises detecting a C03950 variant, for example a variant protein, protein fragment, peptide, polynucleotide, polynucleotide fragment and/or oligonucleotide as described herein, optionally and preferably in a serum sample. The expression levels of the C03950 variant as determined in a patient can be further compared to those in a normal individual.

Polymorphic variants of the known Serine/threonine-protein kinase TNNI3K (SEQ. ID NO:209) is described with regard to PCT Application No. WO 05/017176, hereby incorporated by reference as if fully set forth herein. These variants were shown to be related to risk of melanoma.

Another non-limiting example of such a utility is the detection, diagnosis and/or determination of lymphoma, optionally including prediction of survival. The method comprises detecting a C03950 variant, for example a variant protein, protein fragment, peptide, polynucleotide, polynucleotide fragment and/or oligonucleotide as described herein, optionally and preferably in a serum sample. The expression levels of the C03950 variant as determined in a patient can be further compared to those in a normal individual.

Expression level of the known Serine/threonine-protein kinase TNNI3K (SEQ. ID NO:209) is described with regard to PCT Application No. WO 05/024043, hereby incorporated by reference as if fully set forth herein. The expression level, measured by using microarrays, was shown to be related to risk of lymphoma, including but not limited to follicular lymphoma, diffuse large B cell lymphoma or mantle cell lymphoma.

Another non-limiting example of such a utility is the detection, diagnosis and/or determination of lung cancer, optionally including prediction of survival, including but not limited to small cell lung carcinoma (oat cell carcinoma), or non-small cell carcinomas (e.g., squamous cell carcinoma, adenocarcinoma, large cell lung carcinoma, carcinoid, granulomatous). The method comprises detecting a C03950 variant, for example a variant protein, protein fragment, peptide, polynucleotide, polynucleotide fragment and/or oligonucleotide as described herein, optionally and preferably in a serum sample. The expression levels of the C03950 variant as determined in a patient can be further compared to those in a normal individual.

Expression level of the known Serine/threonine-protein kinase TNNI3K (SEQ. ID NO:209) is described with regard to PCT Application No. WO 02/086443, hereby incorporated by reference as if fully set forth herein. The expression level, measured by using the Eos/Afiymetrix Hu03 Genechip array, was shown to be related to lung cancer, including but not limited to early detection of lung cancers, monitoring and early detection of relapse following treatment of lung cancers, monitoring response to therapy of lung cancers, determining prognosis of lung cancers, directing therapy of lung cancers, selecting patients for postoperative chemotherapy or radiation therapy, selecting therapy, determining tumor prognosis, treatment, or response to treatment, and early detection of precancerous lesions of the lung. Examples of benign or precancerous lesions include but are not limitd to atelectasis, emphysema, brochitis, chronic obstructive pulmonary disease, fibrosis, I hypersensitivity pneumonitis (HP), interstitial pulmonary fibrosis (IPF), asthma, and bronchiectasis.

Protein Serine/threonine-protein kinase TNNI3K (SEQ. ID NO:209) is known or believed to have a role in cardiac physiology. The sequence for protein Serine/threonine-protein kinase TNNI3K (SEQ. ID NO:209) is given. Known polymorphisms for this sequence are as shown in Table 88.

*Table 88 - Amino acid mutations for Known Protein*

| SNP position(s) on amino acid sequence | Comment |
|---|---|
| 228 | I -> V |
| 351 | I -> M |
| 468 | N -> S |
| 730 | R->L |

Protein Serine/threonine-protein kinase TNNI3K (SEQ. ID NO:209) localization is believed to be Nuclear Expressed at lower levels in the cytoplasm.

Other non-limiting exemplary utilities for C03950 variants according to the present invention are described in greater detail below and also with regard to the previous section on clinical utility.

The heart-selective diagnostic marker prediction engine provided the following results with regard to cluster C03950. Predictions were made for selective expression of transcripts of this contig in heart tissue, according to the previously described methods. The numbers on the y-axis of Figure 23 below refer to weighted expression of ESTs in each category, as "parts per million" (ratio of the expression of ESTs for a particular cluster to the expression of all ESTs in that category, according to parts per million).

Overall, the following results were obtained as shown with regard to the histogram in Figure 23, concerning the number of heart-specific clones in libraries/sequences.

This cluster was found to be selectively expressed in heart for the following reasons: in a comparison of the ratio of expression of the cluster in heart specific ESTs to the overall expression of the cluster in non-heart ESTs, which was found to be 9.5; the ratio of expression of the cluster in heart specific ESTs to the overall expression of the cluster in muscle-specific ESTs which was found to be 3.7; and fisher exact test P-values were computed both for library and weighted clone counts to check that the counts are statistically significant, and were found to be 1.40E-03.

One particularly important measure of specificity of expression of a cluster in heart tissue is the previously described comparison of the ratio of expression of the cluster in heart as opposed to muscle. This cluster was found to be specifically expressed in heart as opposed to non-heart ESTs as described above. However, many proteins have been shown to be generally expressed at a higher level in both heart and muscle, which is less desirable. For this cluster, as described above, the ratio of expression of the cluster in heart specific ESTs to the overall expression of the cluster in muscle-specific ESTs which was found to be 9.5, which clearly supports specific expression in heart tissue.

As noted above, cluster C03950 features 15 transcript(s), which were listed in Table 85 above. These transcript (s) encode for protein(s) which are variant(s) of protein Serine/threonine-protein kinase TNNI3K (SEQ. ID NO:209). A description of each variant protein according to the present invention is now provided.

Variant protein C03950_3_P5 (SEQ. ID NO:212) according to the present invention is encoded by transcript C03950_3_T2 (SEQ. ID N0:156). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P5 (SEQ. ID NO:212) and TNI3K_HUMAN (SEQ. ID NO: 396):

A. An isolated chimeric polypeptide encoding for C03950_3_P5 (SEQ. ID NO:212), comprising a first amino acid sequence being at least 70%, optionally at least 80%, preferably at least 85%, more preferably at least 90% and most preferably at least 95%, homologous to a polypeptide having the sequence AVRRGLREGGA (SEQ. ID NO: 342) corresponding to amino acids 1 - 11 of C03950_3_P5 (SEQ. ID NO:212), a second amino acid sequence being at least 90% homologous to MAAARDPPEVSLREATQRKLRRFSELRGKLVARGEFWDI-VAITAADEKQELAYNQQLSEKLK RKELPLGVQYHVFVDPAGAKIGNGGSTLCALQCLEKLYGDKWNSFTIL-LIHSDEWKKKVSES YVITIERLEDDLQIKEKELTELRNIFGSDEAFSKVNLNYRTENGLSLLHLCCICGGKKSHI-RTLM LKGLRPSRLTRNGFTALHLAVYKDNAELITSLLHSGADIQQVGYGGLTALHIATIAGHLEAAD VLLQH-GANVNIQDAVFFTPLHIAAYYGHEQVTRLLLKFGADVNVSGEVGDRPLHLASAKGFL NIAKLLMEEGSKADV-NAQDNEDHVPLHFCSRFGHHDIVKYLLQSDLEVQPHVVNIYGDTPLH LACYNGKFEVAKEIIQISGTESLTKE-NIFSETAFHSACTYGKSIDLVKFLLDQNVININHQGRDG HTGLHSACYHGHIRLVQFLLDNGADMNLVACDP-SRSSGEKDEQTCLMWAYEKGHDAIVTLL KHYKRPQDELPCNEYSQPGGDGSYVSVPSPLGKIKSMTKEKA-DILLLRAGLPSHFHLQLSEIEF HEIIGSGSFGKVYKGRCRNKIVAIKRYRANTYCSKSDVDMFCREVSILCQL-NHPCVIQFVGAC LNDPSQFAIVTQYISGGSLFSLLHEQKRILDLQSKLIIAVDVAKGMEYLHNLTQPIIHRDLN corresponding to amino acids 1 - 691 of TNI3K_HUMAN (SEQ. ID NO: 396), which also corresponds to amino acids 12 - 702 of C03950_3_P5 (SEQ. ID NO:212), a third amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 703 - 742 of C03950_3_P5 (SEQ. ID NO:212), and a fourth amino acid sequence being at least 90% homologous to ESRFLQSLDEDNMTKQPGNLRWMAPEVFTQCTRYTIKADVFSYALCLWEILTGEIP-FAHLKPA AAAADMAYHHIRPPIGYSIPKPISSLLIRGWNACPEGRPEFSEVVMKLEECLCNIELMSPASSNS S-GSLSPSSSSDCLVNRGGPGRSHVAALRSRFELEYALNARSYAALSQSAGQYSSQGLSLEEMK RSLQYTP IDKYGYVSDPMSSMHFHSCRNSSSFEDSS corresponding to amino acids 710 - 936 of TNI3K_HUMAN (SEQ. ID NO: 396), which also corresponds to amino acids 743 - 969 of C03950_3_P5 (SEQ. ID NO:212), wherein said first amino acid sequence, second amino acid sequence, third amino acid sequence and fourth amino acid sequence are contiguous and in a sequential order.

B. An isolated polypeptide encoding for a head of C03950_3_P5 (SEQ. ID NO:212), comprising a polypeptide being at least 70%, optionally at least about 80%, preferably at least about 85%, more preferably at least about 90% and most preferably at least about 95% homologous to the sequence AVRRGLREGGA (SEQ. ID NO: 342) of C03950_3_P5 (SEQ. ID NO:212).

2. Comparison report between C03950_3_P5 (SEQ. ID NO:212) and NP_057062 (SEQ. ID NO: 210):

A. An isolated chimeric polypeptide encoding for C03950_3_P5 (SEQ. ID NO:212), comprising a first amino acid sequence being at least 70%, optionally at least 80%, preferably at least 85%, more preferably at least 90% and most preferably at least 95%, homologous to a polypeptide having the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P5 (SEQ. ID NO:212), a second amino acid sequence being at least 90% homologous to DEWKKKVSESYVITIERLEDDLQIKEKELTELRNIFGSDEAFSKVNL-NYRTENGLSLLHLCCIC GGKKSHIRTLMLKGLRPSRLTRNGFTALHLAVYKDNAELITSLLHSGADIQQVGYG-GLTALHI ATIAGHLEAADVLLQHGANVNIQDAVFFTPLHIAAYYGHEQVTRLLLKFGADVNVSGEVGDR PLH-LASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLN corresponding to amino acids 14 - 590 ofNP_057062 (SEQ. ID NO:210), which also corresponds to amino acids 126 - 702 of C03950_3_P5 (SEQ. ID NO:212), a third amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 703 - 742 of C03950_3_P5 (SEQ. ID NO:212), and a fourth amino acid sequence being at least 90% homologous to ESRFLQSLDEDNMTKQPGNLRWMAPEVFTQCTRYTIKADVFSYALCLWEILTGEIP-FAHLKPA AAAADMAYHHIRPPIGYSIPKPISSLLIRGWNACPEGRPEFSEWMKLEECLCNIELMSPASSNS S-GSLSPSSSSDCLVNRGGPGRSHVAALRSRFELEYALNARSYAALSQSAGQYSSQGLSLEEMK RSLQYT-PIDKYGYVSDPMSSMHFHSCRNSSSFEDSS corresponding to amino acids 609 - 835 of NP_057062 (SEQ. ID NO:210), which also corresponds to amino acids 743 - 969 of C03950_3_P5 (SEQ. ID NO:212), wherein said first amino acid sequence, second amino acid sequence, third amino acid sequence and fourth amino acid sequence are contiguous and in a sequential order.

B. An isolated polypeptide encoding for a head of C03950_3_P5 (SEQ. ID NO:212), comprising a polypeptide being at least 70%, optionally at least about 80%, preferably at least about 85%, more preferably at least about 90% and most preferably at least about 95% homologous to the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) of C03950_3_P5 (SEQ. ID NO:212).

3. Comparison report between C03950_3_P5 (SEQ. ID NO:212) and Q9Y2V6_HUMAN (SEQ. ID NO:210):

A. An isolated chimeric polypeptide encoding for C03950_3_P5 (SEQ. ID NO:212), comprising a first amino acid sequence being at least 70%, optionally at least 80%, preferably at least 85%, more preferably at least 90% and most preferably at least 95%, homologous to a polypeptide having the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P5 (SEQ. ID N0:212), a second amino acid sequence being at least 90% homologous to DEWKKKVSESYVITIERLEDDLQIKEKELTELRNIFGSDEAFSKVNL-NYRTENGLSLLHLCCIC GGKKSHIRTLMLKGLRPSRLTRNGFTALHLAVYKDNAELITSLLHSGADIQQVGYG-GLTALHI ATIAGHLEAADVLLQHGANVNIQDAVFFTPLHIAAYYGHEQVTRLLLKFGADVNVSGEVGDR PLH-LASAKGFLNIAKLLMEEGSKADVNAQDNEDHVPLHFCSRFGHHDIVKYLLQSDLEVQPH VVNIYGDTPLHL-ACYNGKFEVAKEIIQISGTESLTKENIFSETAFHSACTYGKSIDLVKFLLDQN VININHQGRDGHTGLHSACY-HGHIRLVQFLLDNGADMNLVACDPSRSSGEKDEQTCLMWAY EKGHDAIVTLLKHYKRPQDELPCN-EYSQPGGDGSYVSVPSPLGKIKSMTKEKADILLLRAGLP SHFHLQLSEIEFHEIIGSGSFGKVYKGRCRNKI-VAIKRYRANTYCSKSDVDMFCREVSILCQLN HPCVIQFVGACLNDPSQFAIVTQYISGGSLFSLLHEQKRILD-LQSKLIIAVDVAKGMEYLHNLT QPIIHRDLN corresponding to amino acids 14 - 590 of Q9Y2V6_HUMAN (SEQ. ID NO:210), which also corresponds to amino acids 126 - 702 of C03950_3_P5 (SEQ. ID NO:212), a third amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 703 - 742 of C03950_3_P5 (SEQ. ID NO:212), and a fourth amino acid sequence being at least 90% homologous to ESRFLQSL DEDNMTKQPGNLRWMAPEVFTQCTRYTIKADVFSYALCLWEILTGEIPFAHLKPA AAAADMAYHHIRPPIGYSIPKPISSLLIRGWNACPEGRPEFSEWMKLEECLCNIELMSPASSNS SGSLSPSSSSDCLVNRGGPGRSHVAALRSRFELEYALNARSYAALSQSAGQYSSQGLSLEEMK RSLQYT-PIDKYGYVSDPMSSMHFHSCRNSSSFEDSS corresponding to amino acids 609 - 835 of Q9Y2V6_HUMAN (SEQ. ID NO:210), which also corresponds to amino acids 743 - 969 of C03950_3_P5 (SEQ. ID NO:212), wherein said first amino acid sequence, second amino acid sequence, third amino acid sequence and fourth amino acid sequence are contiguous and in a sequential order.

B. An isolated polypeptide encoding for a head of C03950_3_P5 (SEQ. ID NO:212), comprising a polypeptide being at least 70%, optionally at least about 80%, preferably at least about 85%, more preferably at least about 90% and most preferably at least about 95% homologous to the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) of C03950_3_P5 (SEQ. ID NO:212).

4. Comparison report between C03950_3_P5 (SEQ. ID NO:212) and Q6MZS9_HUMAN (SEQ. ID NO: 211):

A. An isolated chimeric polypeptide encoding for C03950_3_P5 (SEQ. ID NO:212), comprising a first amino acid sequence being at least 70%, optionally at least 80%, preferably at least 85%, more preferably at least 90% and most preferably at least 95%, homologous to a polypeptide having the sequence AVRRGLR (SEQ. ID NO: 344) corresponding to amino acids 1 - 7 of C03950_3_P5 (SEQ. ID NO:212), a second amino acid sequence being at least 90% homologous to EGGAMAAARDPPEVSLREATQRKLRRFSELRGKLVARGEFW-DIVAITAADEKQELAYNQQLS EKLKRKELPLGVQYHVFVDPAGAKIGNGGSTLCALQCLEKLYGDKWNSFTI-LLIHSDEWKKK VSESYVITIERLEDDLQIKEKELTELRNIFGSDEAFSKVNLNYRTENGLSLLHLCCICGGKK-SHI RTLMLKGLRPSRLTRNGFTALHLAVYKDNAELITSLLHSGADIQQVGYGGLTALHIATIAGHL EAADV-LLQHGANVNIQDAVFFTPLHIAAYYGHEQVTRLLLKFGADVNVSGEVGDRPLHLAS AKGFLNIAKLLMEEG-SKADVNAQDNEDHVPLHFCSRFGHHD corresponding to amino acids 14 - 367 of Q6MZS9_HUMAN (SEQ. ID NO:211), which also corresponds to amino acids 8 - 361 of C03950_3_P5 (SEQ. ID NO:212), a bridging amino acid I corresponding to amino acid 362 of C03950_3_P5 (SEQ. ID NO:212), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 363 - 478 ofC03950_3_P5 (SEQ. ID NO:212), a bridging amino acid N corresponding to amino acid 479 of C03950_3_P5 (SEQ. ID NO:212), a fourth amino acid sequence being at least 90% homologous to LVACDPSRSSGEKDEQTCLMWAYEKGHDAIVTLLKHYKRPQDELPCNEYSQPGGDGSYVSV PSPLGKI-KSMTKEKADILLLRAGLPSHFHLQLSEIEFHEIIGSGSFGKVYKGRCRNKIVAIKRYR ANTYCSKSDVDMF-CREVSILCQLNHPCVIQFVGACLNDPSQFAIVTQYISGGSLFSLLHEQKRI LDLQSKLIIAVDVAKGMEYL-HNLTQPIIHRDLNR corresponding to amino acids 486 - 709 of Q6MZS9_HUMAN (SEQ. ID NO:211), which also corresponds to amino acids 480 - 703 of C03950_3_P5 (SEQ. ID NO:212), and a fifth amino acid sequence being at least 70%, optionally at least 80%, preferably at least 85%, more preferably at least 90% and most preferably at least 95% homologous to a polypeptide having the sequence SAITSRIWITH-SICIWRGAHYFNREECNFRCMLTSAILKESRFLQSLDEDNMTKQPGNLRWMA PEVFTQCTRYTIKADVFS-YALCLWEILTGEIPFAHLKPAAAAADMAYHHIRPPIGYSIPKPISSL LIRGWNACPEGRPEFSEVVMKLEECLC-NIELMSPASSNSSGSLSPSSSSDCLVNRGGPGRSHVA ALRSRFELEYALNARSYAALSQSAGQYSSQGLSL-EEMKRSLQYTPIDKYGYVSDPMSSMHFH SCRNSSSFEDSS (SEQ. ID NO: 345)corresponding to amino acids 704 - 969 of C03950_3_P5 (SEQ. ID NO:212), wherein said first amino acid sequence, second amino acid sequence, bridging amino acid, third amino acid sequence, bridging amino acid, fourth amino acid sequence and fifth amino acid sequence are contiguous and in a sequential order.

B. An isolated polypeptide encoding for a head of C03950_3_P5 (SEQ. ID NO:212), comprising a polypeptide being at least 70%, optionally at least about 80%, preferably at least about 85%, more preferably at least about 90% and most preferably at least about 95% homologous to the sequence AVRRGLR (SEQ. ID NO: 344) ofC03950_3_P5 (SEQ. ID NO:212).

C. An isolated polypeptide encoding for an edge portion of C03950_3_P5 (SEQ. ID NO:212), comprising an amino acid sequence being at least 70%, optionally at least about 80%, preferably at least about 85%, more preferably at least about 90% and most preferably at least about 95% homologous to the sequence SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEVVMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) of C03950_3_P5 (SEQ. ID NO:212).

The localization of the variant protein was determined according to results from a number of different software programs and analyses, including analyses from SignalP and other specialized programs. The variant protein is believed to be located intracellularly.

Variant protein C03950_3_P5 (SEQ. ID NO:212) also has the following non-silent SNPs (Single Nucleotide Polymorphisms) as listed in Table 89, (given according to their position(s) on the amino acid sequence, with the alternative amino acid(s) listed.

*Table 89 - Amino acid mutations*

| SNP position(s) on amino acid sequence | Alternative amino acid(s) |
|---|---|
| 119 | T -> P |

The variant protein has the following domains, as determined by using InterPro. The domains are described in Table 90:

*Table 90 - InterPro domain(s)*

| Domain description | Analysis type | Position(s) on protein |
|---|---|---|
| Protein kinase | BlastProDom | 575-799 |
| Ankyrin | FPrintScan | 279-291, 464-476 |
| Ankyrin | HMMPfam | 178-211, 212-244, 245-277, 278-310, 311-345, 346-376, 381-414, 416-450, 451-483, 493-525 |
| Protein kinase | HMMPfam | 575-853 |
| Tyrosine protein kinase | HMMSmart | 575-853 |
| Serine | HMMSmart | 575-857 |
| Ankyrin | HMMSmart | 178-208, 212-241, 245-274, 278-307, 311-342, 346-377, 381-412, 416-447, 451-480, 493-522 |
| Protein kinase | ProfileScan | 575-857 |
| Ankyrin | ProfileScan | 212-244, 245-277, 281-310, 311-343, 381-413, 451-483 |
| Ankyrin | ProfileScan | 170-513 |
| Protein kinase | ScanRegExp | 581-602 |

Variant protein C03950_3_P5 (SEQ. ID NO:212) is encoded by the following transcript(s): C03950_3_T2 (SEQ. ID NO:156), for which the coding portion starts at position 3 and ends at position 2909. The transcript also has the following SNPs as listed in Table 91 (given according to their position on the nucleotide sequence, with the alternative nucleic acid listed.

*Table 91 - Nucleic acid SNPs*

| SNP position(s) on nucleotide sequence | Alternative nucleic acid(s) |
|---|---|
| 263 | A -> G |
| 357 | A -> C |

Variant protein C03950_3_P7 (SEQ. ID NO:213) according to the present invention is encoded by transcript C03950_3_T4 (SEQ. ID NO:157). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P7 (SEQID NO:213) and TNI3K_HUMAN (SEQID NO: 396):

A. An isolated chimeric polypeptide encoding for C03950_3_P7 (SEQID NO:213), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P7 (SEQ. ID NO:213), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHSGADI-QQVGYGGLTALHI ATIAGHLEAADVLLQHGANVNIQDAVFFTPLHIAAYYGHEQVTRLLLKFGADVNVSGEV-GDR PLHLASAKGFLNIAKLLMEEGSKADVNAQDNEDHVPLHFCSRFGHHDIVKYLLQSDLEVQPH VVNIY-GDTPLHLACYNGKFEVAKEIIQISGTESLTKENIFSETAFHSACTYGKSIDLVKFLLDQN VININHQGRDGH-TGLHSACYHGHIRLVQFLLDNGADMNLVACDPSRSSGEKDEQTCLMWAY EKGHDAIVTLLKHYKRPQDEL-PCNEYSQPGGDGSYVSVPSPLGKIKSMTKEKADILLLRAGLP SHFHLQLSEIEFHEIIGSGSFGKVYKGR-CRNKIVAIKRYRANTYCSKSDVDMFCREVSILCQLN HPCVIQFVGACLNDPSQFAIVTQYISGGSLFSLLHEQ-KRILDLQSKLIIAVDVAKGMEYLHNLT QPIIHRDLN corresponding to amino acids 115 - 691 of TNI3K_HUMAN (SEQ. ID NO: 396), which also corresponds to amino acids 14 - 590 of C03950_3_P7 (SEQ. ID NO:213), a third amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 591 - 630 of C03950_3_P7 (SEQ. ID NO:213), and a fourth amino acid sequence being at least 90% homologous to ESRFLQSLDEDNMTKQPGNLRWMAPEVFTQCTRYTIKADVFSYALCLWEILTGEIPFAHLKPA AAAADMAY-HHIRPPIGYSIPKPISSLLIRGWNACPEGRPEFSEVVMKLEECLCNIELMSPASSNS SGSLSPSSSSDCLVN-RGGPGRSHVAALRSRFELEYALNARSYAALSQSAGQYSSQGLSLEEMK RSLQYTPIDKYGYVSDPMSSMH-FHSCRNSSSFEDSS corresponding to amino acids 710 - 936 of TNI3K_HUMAN (SEQ. ID NO: 396), which also corresponds to amino acids 631 - 857 of C03950_3_P7 (SEQ. ID NO:213), wherein said first amino acid sequence, second amino acid sequence, third amino acid sequence and fourth amino acid sequence are contiguous and in a sequential order.

B. An isolated polypeptide encoding for a head of C03950_3_P7 (SEQ. ID NO:213), comprising a polypeptide being at least 70%, optionally at least about 80%, preferably at least about 85%, more preferably at least about 90% and most preferably at least about 95% homologous to the sequence MGNYKSRPTQTCT (SEQ. ID NO: 346) of C03950_3_P7 (SEQ ID NO:213).

2. Comparison report between C03950_3_P7 (SEQID NO:213) and NP_057062 (SEQID NO:210):

A. An isolated chimeric polypeptide encoding for C03950_3_P7 (SEQID NO:213), comprising a first amino acid sequence being at least 90% homologous to MGNYKSRPTQTCTDEWKKKVSESYVITIERLEDDL-QIKEKELTELRNIFGSDEAFSKVNLNYR TENGLSLLHLCCICGGKKSHIRTLMLKGLRPSRLTRNGFTALHLA-VYKDNAELITSLLHSGADI QQVGYGGLTALHIATIAGHLEAADVLLQHGANVNIQDAVFFTPLHIAAYYGHEQV-TRLLLKF GADVNVSGEVGDRPLHLASAKGFLNIAKLLMEEGSKADVNAQDNEDHVPLHFCSRFGHHDI VKY-LLQSDLEVQPHVVNIYGDTPLHLACYNGKFEVAKEIIQISGTESLTKENIFSETAFHSACT YGKSIDLVKFLLDQ-NVININHQGRDGHTGLHSACYHGHIRLVQFLLDNGADMNLVACDPSRS SGEKDEQTCLMWAYEKGHDAIV-TLLKHYKRPQDELPCNEYSQPGGDGSYVSVPSPLGKIKSM TKEKADILLLRAGLPSHFHLQLSEIEFHEII-GSGSFGKVYKGRCRNKIVAIKRYRANTYCSKSDV DMFCREVSILCQLNHPCVIQFVGACLNDPSQFAIVTQY-ISGGSLFSLLHEQKRILDLQSKLIIAV DVAKGMEYLHNLTQPIIHRDLN corresponding to amino acids 1 - 590 of NP_057062 (SEQ. ID NO:210), which also corresponds to amino acids 1 - 590 of C03950_3_P7 (SEQ. ID NO: 213), a second amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 591 - 630 of C03950_3_P7 (SEQ. ID NO:213), and a third amino acid sequence being at least 90% homologous to ESRFLQSLDEDNMTKQPGNLRWMAPEVFTQCTRYTIKADVFSYALCLWEILTGEIPFAHLKPA AAAADMAY-HHIRPPIGYSIPKPISSLLIRGWNACPEGRPEFSEVVMKLEECLCNIELMSPASSNS SGSLSPSSSSDCLVN-RGGPGRSHVAALRSRFELEYALNARSYAALSQSAGQYSSQGLSLEEMK RSLQYTPIDKYGYVSDPMSSMH-FHSCRNSSSFEDSS corresponding to amino acids 609 - 835 of NP_057062 (SEQ. ID NO:210), which also corresponds to amino acids 631 - 857 of C03950_3_P7 (SEQ. ID NO:213), wherein said first amino acid sequence, second amino acid sequence and third amino acid sequence are contiguous and in a sequential order.

3. Comparison report between C03950_3_P7 (SEQ ID NO:213) and Q9Y2V6_HUMAN (SEQ ID NO:210):

A. An isolated chimeric polypeptide encoding for C03950_3_P7 (SEQ. ID NO:213), comprising a first amino acid sequence being at least 90% homologous to MGNYKSRPTQTCTDEWKKKVSESYVITIERLEDDLQIKEKELTELRNIFGSDEAFSKVNLNYR TENGLSLLHLCCICGGKKSHIRTLMLKGLRPSRLTRNGFTALHLAVYKDNAELITSLLHSGADI QQVGYGGLTALHIATIAGHLEAADVLLQHGANVNIQDAVFFTPLHIAAYYGHEQVTRLLLKF GADVNVSGEVGDRPLHLASAKGFLNIAKLLMEEGSKADVNAQDNEDHVPLHFCSRFGHHDI VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADMNLVACDPSRS SGEKDEQTCLMWAYEKGHDAIVTLLKHYKRPQDELPCNEYSQPGGDGSYVSVPSPLGKIKSM TKEKADILLLRAGLPSHFHLQLSEIEFHEIIGSGSFGKVYKGRCRNKIVAIKRYRANTYCSKSDV DMFCREVSILCQLNHPCVIQFVGACLNDPSQFAIVTQYISGGSLFSLLHEQKRILDLQSKLIIAV DVAKGMEYLHNLTQPIIHRDLN corresponding to amino acids 1 - 590 of Q9Y2V6_HUMAN (SEQ. ID NO:210), which also corresponds to amino acids 1 - 590 of C03950_3_P7 (SEQ. ID NO:213), a second amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 591 - 630 of C03950_3_P7 (SEQ. ID NO:213), and a third amino acid sequence being at least 90% homologous to ESRFLQSLDEDNMTKQPGNLRWMAPEVFTQCTRYTIKADVFSYALCLWEILTGEIPFAHLKPA AAAADMAYHHIRPPIGYSIPKPISSLLIRGWNACPEGRPEFSEWMKLEECLCNIELMSPASSNS SGSLSPSSSSDCLVNRGGPGRSHVAALRSRFELEYALNARSYAALSQSAGQYSSQGLSLEEMK RSLQYTPIDKYGYVSDPMSSMHFHSCRNSSSFEDSS corresponding to amino acids 609 - 835 of Q9Y2V6_HUMAN (SEQ. ID NO:210), which also corresponds to amino acids 631 - 857 of C03950_3_P7 (SEQ. ID NO:213), wherein said first amino acid sequence, second amino acid sequence and third amino acid sequence are contiguous and in a sequential order.

4. Comparison report between C03950_3_P7 (SEQ. ID NO:213) and Q6MZS9_HUMAN (SEQ. ID NO:211):

A. An isolated chimeric polypeptide encoding for C03950_3_P7 (SEQ. ID NO:213), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P7 (SEQ. ID NO:213), a second amino acid sequence being at least 90% homologous to DEWKKKVSESYVITIERLEDDLQIKEKELTELRNIFGSDEAFSKVNLNYRTENGLSLLHLCCIC GGKKSHIRTLMLKGLRPSRLTRNGFTALHLAVYKDNAELITSLLHSGADIQQVGYGGLTALHI ATIAGHLEAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHD corresponding to amino acids 132 - 367 ofQ6MZS9_HUMAN (SEQ. ID NO:211), which also corresponds to amino acids 14 - 249 of C03950_3_P7 (SEQ. ID NO:213), a bridging amino acid I corresponding to amino acid 250 of C03950_3_P7 (SEQ. ID NO:213), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 251 - 366 of C03950_3_P7 (SEQ. ID NO:213), a bridging amino acid N corresponding to amino acid 367 of C03950_3_P7 (SEQ. ID NO:213), a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIAVDVAKGMEYLHNLTQPIIHRDLNR corresponding to amino acids 486 - 709 of Q6MZS9_HUMAN (SEQ ID NO:211), which also corresponds to amino acids 368 - 591 of C03950_3_P7 (SEQ. ID NO:213), and a fifth amino acid sequence being at least 70%, optionally at least 80%, preferably at least 85%, more preferably at least 90% and most preferably at least 95% homologous to a polypeptide having the sequence SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEVVMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) corresponding to amino acids 592 - 857 of C03950_3_P7 (SEQ. ID NO:213), wherein said first amino acid sequence, second amino acid sequence, bridging amino acid, third amino acid sequence, bridging amino acid, fourth amino acid sequence and fifth amino acid sequence are contiguous and in a sequential order.

B. An isolated polypeptide encoding for a head of C03950_3_P7 (SEQ. ID NO:213), comprising a polypeptide being at least 70%, optionally at least about 80%, preferably at least about 85%, more preferably at least about 90% and most preferably at least about 95% homologous to the sequence MGNYKSRPTQTCT (SEQ. ID NO: 346) of C03950_3_P7 (SEQ ID NO:213).

C. An isolated polypeptide encoding for an edge portion of C03950_3_P7 (SEQ. ID NO:213), comprising an amino acid sequence being at least 70%, optionally at least about 80%, preferably at least about 85%, more preferably at least about 90% and most preferably at least about 95% homologous to the sequence SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEVVMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) of C03950_3_P7 (SEQ. ID NO:213).

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 92:

*Table 92 - InterPro domain(s)*

| Domain description | Analysis type | Position(s) on protein |
|---|---|---|
| Protein kinase | BlastProDom | 463-687 |
| Ankyrin | FPrintScan | 167-179, 352-364 |
| Ankyrin | HMMPfam | 66-99, 100-132, 133-165, 166-198, 199-233, 234-264, 269-302, 304-338, 339-371, 381-413 |
| Protein kinase | HMMPfam | 463-741 |
| Tyrosine protein kinase | HMMSmart | 463-741 |
| Serine | HMMSmart | 463-745 |
| Ankyrin | HMMSmart | 66-96, 100-129, 133-162, 166-195, 199-230, 234-265, 269-300, 304-335, 339-368, 381-410 |
| Protein kinase | ProfileScan | 463-745 |
| Ankyrin | ProfileScan | 100-132, 133-165, 169-198, 199-231, 269-301, 339-371 |
| Ankyrin | ProfileScan | 58-401 |
| Protein kinase | ScanRegExp | 469-490 |

Variant protein C03950_3_P7 (SEQ. ID NO:213) is encoded by the following transcript(s): C03950_3_T4 (SEQ. ID NO:157), for which the coding portion starts at position 389 and ends at position 2959.

Variant protein C03950_3_P9 (SEQ. ID NO:214) according to the present invention is encoded by transcript C03950_3_T7 (SEQ. ID NO:158). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P9 (SEQ. ID NO:214) and TNI3K_HUMAN (SEQ. ID NO:396):

A. An isolated chimeric polypeptide encoding for C03950_3_P9 (SEQ. ID NO:214), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P9 (SEQ. ID NO:214), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHSGADI-QQVGYGGLTALHI ATIAGHLEAADVLLQHGANVNIQDAVFFTPLHIAAYYGHEQVTRLLLKFGADVNVSGEV-

GDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLNSHNILLYEDGHAWADFGESRFLQSLDEDNMTKQPGNLRWMAPEVFTQCTRYTI KADVFSYALCLWEILTGEIPFAHLKPAAAAADMAYHHIRPPIGYSIPKPISSLLIRGWNACPEG RPEFSEWMKLEECLCNIELMSPASSNSSGSLSPSSSSDCLVNRGGPGRSHVAALRSRFELEYA LNARSYAALSQSAGQYSSQGLSLEEMKRSLQYTPIDKY corresponding to amino acids 115-911 of TNI3K_HUMAN (SEQ. ID NO: 396), which also corresponds to amino acids 14 - 810 of C03950_3_P9 (SEQ. ID NO:214), and a third amino acid sequence being at least 70%, optionally at least 80%, preferably at least 85%, more preferably at least 90% and most preferably at least 95% homologous to a polypeptide having the sequence DVTS (SEQ. ID NO: 349) corresponding to amino acids 811 - 814 of C03950_3_P9 (SEQ. ID NO:214), wherein said first amino acid sequence, second amino acid sequence and third amino acid sequence are contiguous and in a sequential order.

B. An isolated polypeptide encoding for a head of C03950_3_P9 (SEQ. ID NO:214), comprising a polypeptide being at least 70%, optionally at least about 80%, preferably at least about 85%, more preferably at least about 90% and most preferably at least about 95% homologous to the sequence MGNYKSRPTQTCT (SEQ. ID NO: 346) of C03950_3_P9 (SEQ. ID NO:214).

C. An isolated polypeptide encoding for an edge portion of C03950_3_P9 (SEQ. ID NO:214), comprising an amino acid sequence being at least 70%, optionally at least about 80%, preferably at least about 85%, more preferably at least about 90% and most preferably at least about 95% homologous to the sequence DVTS (SEQ. ID NO: 349) of C03950_3_P9 (SEQ. ID NO:214).

2. Comparison report between C03950_3_P9 (SEQ. ID NO:214) and NP_057062 (SEQ. ID NO:210):

A. An isolated chimeric polypeptide encoding for C03950_3_P9 (SEQ. ID NO:214), comprising a first amino acid sequence being at least 90% homologous to MGNYKSRPTQTCTDEWKKICVSESYVITIERLEDDLQIKEKELTELRNIFGSDEAFSKVNLNYR TENGLSLLHLCCICGGKKSHIRTLMLKGLRPSRLTRNGFTALHLAVYKDNAELITSLLHSGADI QQVGYGGLTALHIATIAGHLEAADVLLQHGANVNIQDAVFFTPLHIAAYYGHEQVTRLLLKF GADVNVSGEVGDRPLHLASAKGFLNIAKLLMEEGSKADVNAQDNEDHVPLHFCSRFGHHDI VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADMNLVACDPSRS SGEKDEQTCLMWAYEKGHDAIVTLLKHYKRPQDELPCNEYSQPGGDGSYVSVPSPLGKIKSM TKEKADILLLRAGLPSHFHLQLSEIEFHEIIGSGSFGKVYKGRCRNKIVAIKRYRANTYCSKSDV DMFCREVSILCQLNHPCVIQFVGACLNDPSQFAIVTQYISGGSLFSLLHEQKRILDLQSKLIIAV DVAKGMEYLHNLTQPIIHRDLNSHNILLYEDGHAWADFGESRFLQSLDEDNMTKQPGNLR WMAPEVFTQCTRYTIKADVFSYALCLWEILTGEIPFAHLKPAAAAADMAYHHIRPPIGYSIPKP ISSLLIRGWNACPEGRPEFSEVVMKLEECLCNIELMSPASSNSSGSLSPSSSSDCLVNRGGPGRS HVAALRSRFELEYALNARSYAALSQSAGQYSSQGLSLEEMKRSLQYTPIDKY corresponding to amino acids 1-810 of NP_057062 (SEQ. ID NO:210), which also corresponds to amino acids 1-810 of C03950_3_P9 (SEQ. ID NO:214), and a second amino acid sequence being at least 70%, optionally at least 80%, preferably at least 85%, more preferably at least 90% and most preferably at least 95% homologous to a polypeptide having the sequence DVTS (SEQ. ID NO: 349) corresponding to amino acids 811 - 814 of C03950_3_P9 (SEQ. ID NO:214), wherein said first amino acid sequence and second amino acid sequence are contiguous and in a sequential order.

3. Comparison report between C03950_3_P9 (SEQ. ID NO:214) and Q9Y2V6_HUMAN (SEQ. ID NO:210):

A. An isolated chimeric polypeptide encoding for C03950_3_P9 (SEQ. ID NO:214), comprising a first amino acid sequence being at least 90% homologous to MGNYKSRPTQTCTDEWKKKVSESYVITIERLEDDLQIKEKELTELRNIFGSDEAFSKVNLNYR TENGLSLLHLCCICGGKKSHIRTLMLKGLRPSRLTRNGFTALHLAVYKDNAELITSLLHSGADI QQVGYGGLTALHIATIAGHLEAADVLLQHGANVNIQDAVFFTPLHIAAYYGHEQVTRLLLKF GADVNVSGEVGDRPLHLASAKGFLNIAKLLMEEGSKADVNAQDNEDHVPLHFCSRFGHHDI VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADMNLVACDPSRS SGEKDEQTCLMWAYEKGHDAIVTLLKHYKRPQDELPCNEYSQPGGDGSYVSVPSPLGKIKSM TKEKADILLLRAGLPSHFHLQLSEIEFHEIIGSGSFGKVYKGRCRNKIVAIKRYRANTYCSKSDV DMFCREVSILCQLNHPCVIQFVGACLNDPSQFAIVTQYISGGSLFSLLHEQKRILDLQSKLIIAV DVAKGMEYLHNLTQPIIHRDLNSHNILLYEDGHAWADFGESRFLQSLDEDNMTKQPGNLR WMAPEVFTQCTRYTIKADVFSYALCLWEILTGEIPFAHLKPAAAAADMAYHHIRPPIGYSIPKP ISSLLIRGWNACPEGRPEFSEVVMKLEECLCNIELMSPASSNSSGSLSPSSSSDCLVNRGGPGRS HVAALRSRFELEYALNARSYAALSQSAGQYSSQGLSLEEMKRSLQYTPIDKY corresponding to amino acids 1-810 of Q9Y2V6_HUMAN (SEQ. ID NO:210), which also corresponds to amino acids 1 - 810 of C03950_3_P9 (SEQ. ID NO:214), and a second amino acid sequence being at least 70%, optionally at least 80%, preferably at least 85%, more preferably at least 90% and most preferably at least 95% homologous to a polypeptide having the sequence DVTS (SEQ. ID NO: 349) corresponding to amino acids 811 - 814 of C03950_3_P9 (SEQ. ID NO:214), wherein said first amino acid sequence and second amino acid sequence are contiguous and in a sequential order.

B. An isolated polypeptide encoding for an edge portion of C03950_3_P9 (SEQ. ID NO:214), comprising an amino acid sequence being at least 70%, optionally at least about 80%, preferably at least about 85%, more preferably at least about 90% and most preferably at least about 95% homologous to the sequence DVTS (SEQ. ID NO: 349) of C03950 3 P9 (SEQ. ID NO:214).

4. Comparison report between C03950_3_P9 (SEQ. ID NO:214) and Q6MZS9_HUMAN (SEQ. ID NO:211):

A. An isolated chimeric polypeptide encoding for C03950_3_P9 (SEQ. ID NO:214), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P9 (SEQ. ID NO:214), a second amino acid sequence being at least 90% homologous to DEWKKICVSESYVITIERLEDDLQIKEKELTELRNIFGSDEAFSKVNLNYRTENGLSLLHLCCIC GGKKSHIRTLMLKGLRPSRLTRNGFTALHLAVYKDNAELITSLLHSGADIQQVGYGGLTALHI ATIAGHLEAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHD corresponding to amino acids 132 - 367 of Q6MZS9_HUMAN (SEQ. ID NO:211), which also corresponds to amino acids 14-249 of C03950_3_P9 (SEQ. ID NO:214), a bridging amino acid I corresponding to amino acid 250 of C03950_3_P9 (SEQ. ID NO:214), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 251 - 366 of C03950_3_P9 (SEQ. ID NO:214), a bridging amino acid N corresponding to amino acid 367 of C03950_3_P9 (SEQ. ID NO:214), a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIAVDVAKGMEYLHNLTQPIIHRDLN corresponding to amino acids 486 - 708 of Q6MZS9_HUMAN (SEQ. ID NO:211), which also corresponds to amino acids 368 - 590 of C03950_3_P9 (SEQ. ID NO:214), and a fifth amino acid sequence being at least 70%, optionally at least 80%, preferably at least 85%, more preferably at least 90% and most preferably at least 95% homologous to a polypeptide having the sequence SHNILLYEDGHAWADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEGRPEFSEWM KLEECLCNIELMSPASSNSSGSLSPSSSSDCLVNRGGPGRSHVAALRSRFELEYALNARSYAAL SQSAGQYSSQGLSLEEMKRSLQYTPIDKYDVTS (SEQ. ID NO: 350) corresponding to amino acids 591 - 814 of C03950_3_P9 (SEQ. ID NO:214), wherein said first amino acid sequence, second amino acid sequence, bridging amino acid, third amino acid sequence, bridging amino acid, fourth amino acid sequence and fifth amino acid sequence are contiguous and in a sequential order.

B. An isolated polypeptide encoding for a head of C03950_3_P9 (SEQ. ID NO:214), comprising a polypeptide being at least 70%, optionally at least about 80%, preferably at least about 85%, more preferably at least about 90% and most preferably at least about 95% homologous to the sequence MGNYKSRPTQTCT (SEQ. ID NO: 346) of C03950_3_P9 (SEQ. ID NO:214).

C. An isolated polypeptide encoding for an edge portion of C03950_3_P9 (SEQ. ID NO:214), comprising an amino acid sequence being at least 70%, optionally at least about 80%, preferably at least about 85%, more preferably at least about 90% and most preferably at least about 95% homologous to the sequence SHNILLYEDGHAWADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEGRPEFSEWM KLEECLCNIELMSPASSNSSGSLSPSSSSDCLVNRGGPGRSHVAALRSRFELEYALNARSYAAL SQSAGQYSSQGLSLEEMKRSLQYTPIDKYDVTS (SEQ. ID NO: 350) of C03950_3_P9 (SEQ. ID NO:214).

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 93:

*Table 93 - InterPro domain(s)*

| Domain description | Analysis type | Position(s) on protein |
|---|---|---|
| Protein kinase | BlastProDom | 463-665 |
| Tyrosine protein kinase | FPrintScan | 539-552, 578-596, 626-636, 690-712 |
| Ankyrin | FPrintScan | 167-179, 352-364 |
| Ankyrin | HMMPfam | 66-99, 100-132, 133-165, 166-198, 199-233, 234-264, 269-302, 304-338, 339-371, 381-413 |
| Protein kinase | HMMPfam | 463-719 |
| Tyrosine protein kinase | HMMSmart | 463-719 |
| Serine | HMMSmart | 463-723 |
| Ankyrin | HMMSmart | 66-96, 100-129, 133-162, 166-195, 199-230, 234-265, 269-300, 304-335, 339-368, 381-410 |
| Protein kinase | ProfileScan | 463-723 |
| Ankyrin | ProfileScan | 100-132, 133-165, 169-198, 199-231, 269-301, 339-371 |
| Ankyrin | ProfileScan | 58-401 |
| Protein kinase | ScanRegExp | 469-490 |

Variant protein C03950_3_P9 (SEQ. ID NO:214) is encoded by the following transcript(s): C03950_3_T7 (SEQ. ID NO:158), for which the coding portion starts at position 389 and ends at position 2830.

Variant protein C03950_3_P10 (SEQ. ID NO:215) according to the present invention is encoded by transcript C03950_3_T8 (SEQ. ID NO:159). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P10 (SEQ. ID NO:215) and TNI3K_HUMAN (SEQ. ID NO: 396):

A. An isolated chimeric polypeptide encoding for C03950_3_P10 (SEQ. ID NO:215), comprising a first amino acid sequence being at least 70%, optionally at least 80%, preferably at least 85%, more preferably at least 90% and most preferably at least 95%, homologous to a polypeptide having the sequence PSPCGLHFLIPWLTQ (SEQ. ID NO: 351) corresponding to amino acids 1-15 of C03950_3_P10 (SEQ. ID NO:215), and a second amino acid sequence being at least 90% homologous to DNAELITSLLHSGADIQQVGYGGLTALHIATIAGH-LEAADVLLQHGANVNIQDAVFFTPLHIA AYYGHEQVTRLLLKFGADVNVSGEVGDRPLHLASAKGFLNIAKLL-MEEGSKADVNAQDNED HVPLHFCSRFGHHDIVKYLLQSDLEVQPHVUNIYGDTPLHLACYNGKFEVAKEII-QISGTESLT KENIFSETAFHSACTYGKSIDLVKFLLDQNVININHQGRDGHTGLHSACYHGHIRLVQFLLDN GADMNLVACDPSRSSGEKDEQTCLMWAYEKGHDAIVTLLKHYKRPQDELPCNEYSQPGGD GSYVSVPSPLGKIKSMTKEKADILLLRAGLPSHFHLQLSEIEFHEIIGSGSFGKVYKGRCRNKIV AIKRYRANTYCSKSDVDMFCREVSILCQLNHPCVIQFVGACLNDPSQFAIVTQYISGGSLFSLL HEQKRILDLQSKLIIAVDVAKGMEYLHNLTQPIIHRDLNSHNILLYEDGHAWADFGESRFLQS LDEDNMTKQPGNLRWMAPEVFTQCTRYTIKADVFSYALCLWEILTGEIPFAHLKPAAAAAD MAYHHIRPPIGYSIPKPISSLLIRGWNACPEGRPEFSEVVMKLEECLCNIELMSPASSNSSGSLSP SSSSDCLVNRGGPGRSHVAALRSRFELEYALNARSYAALSQSAGQYSSQGLSLEEMKRSLQY TPIDKYGYVSDPMSSMHFHSCRNSSSFEDSS corresponding to amino acids 213 - 936 of TNI3K_HUMAN (SEQ. ID NO: 396), which also corresponds to amino acids 16 - 739 of C03950_3_PIO (SEQ. ID NO:215), wherein said first amino acid sequence and second amino acid sequence are contiguous and in a sequential order.

B. An isolated polypeptide encoding for a head of C03950_3_P10 (SEQ. ID NO:215), comprising a polypeptide being at least 70%, optionally at least about 80%, preferably at least about 85%, more preferably at least about 90% and most preferably at least about 95% homologous to the sequence PSPCGLHFLIPWLTQ (SEQ. ID NO: 351) of C03950_3_P10 (SEQ. ID NO:215).

2. Comparison report between C03950_3_P10 (SEQ. ID NO:215) and Q6MZS9_HUMAN (SEQ. ID NO:211):

A. An isolated chimeric polypeptide encoding for C03950_3_P10 (SEQ. ID NO:215), comprising a first amino acid sequence being at least 70%, optionally at least 80%, preferably at least 85%, more preferably at least 90% and most preferably at least 95%, homologous to a polypeptide having the sequence PSPCGLHFLIPWLTQ (SEQ. ID NO: 351) corresponding to amino acids 1-15 of C03950_3_PIO (SEQ. ID NO:215), a second amino acid sequence being at least 90% homologous to DNAELITSLLHSGADIQQVGYGGLTALHIATIAGHLEAADV-LLQHGANVNIQDAVFFTPLHIA AYYGHEQVTRLLLKFGADVNVSGEVGDRPLHLASAKGFLNIAKLLMEEG-SKADVNAQDNED HVPLHFCSRFGHHD corresponding to amino acids 230 - 367 of Q6MZS9_HUMAN (SEQ. ID NO:211), which also corresponds to amino acids 16 - 153 of C03950_3_P10 (SEQ. ID NO:215), a bridging amino acid I corresponding to amino acid 154 of C03950_3_P10 (SEQ. ID NO:215), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 155 - 270 of C03950_3_P10 (SEQ. ID NO:215), a bridging amino acid N corresponding to amino acid 271 of C03950_3_P10 (SEQ. ID NO:215), a fourth amino acid sequence being at least 90% homologous to LVACDPSRSSGEKDEQTCLMWAYEKGHDAIVTLLKHYKRPQDELPCNEYSQPGGDGSYVSV PSPLG-KIKSMTKEKADILLLRAGLPSHFHLQLSEIEFHEIIGSGSFGKVYKGRCRNKIVAIKRYR ANTYCSKSDVDMF-CREVSILCQLNHPCVIQFVGACLNDPSQFAIVTQYISGGSLFSLLHEQKRI LDLQSKLIIAVDVAKGMEYL-HNLTQPIIHRDLN corresponding to amino acids 486 - 708 of Q6MZS9_HUMAN (SEQ. ID NO:211), which also corresponds to amino acids 272 - 494 of C03950_3_P10 (SEQ. ID NO:215), and a fifth amino acid sequence being at least 70%, optionally at least 80%, preferably at least 85%, more preferably at least 90% and most preferably at least 95% homologous to a polypeptide having the sequence SHNILLYED-GHAWADFGESRFLQSLDEDNMTKQPGNLRWMAPEVFTQCTRYTIKADVFSYA LCLWEILTGEIPFAHLKP-AAAAADMAYHHIRPPIGYSIPKPISSLLIRGWNACPEGRPEFSEVVM KLEECLCNIELMSPASSNSSGSLSP-SSSSDCLVNRGGPGRSHVAALRSRFELEYALNARSYAAL SQSAGQYSSQGLSLEEMKRSLQYTPIDKYGY-VSDPMSSMHFHSCRNSS(SEQ. ID NO: 352) corresponding to amino acids 495 - 739 of C03950_3_P10 (SEQ. ID NO:215), wherein said first amino acid sequence, second amino acid sequence, bridging amino acid, third amino acid sequence, bridging amino acid, fourth amino acid sequence and fifth amino acid sequence are contiguous and in a sequential order.

C. An isolated polypeptide encoding for an edge portion of C03950_3_P10 (SEQ. ID NO:215), comprising an amino acid sequence being at least 70%, optionally at least about 80%, preferably at least about 85%, more preferably at least about 90% and most preferably at least about 95% homologous to the sequence SHNILLYEDGHAWADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEGRPEFSEWM KLEECLCNIELMSPASSNSSGSLSPSSSSDCLVNRGGPGRSHVAALRSRFELEYALNARSYAAL SQSAGQYSSQGLSLEEMKRSLQYTPIDKYGYVSDPMSSMHFHSCRNSS(SEQ. ID NO: 352) of C03950_3_PIO (SEQ. ID NO:215).

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 94:

*Table 94 - InterPro domain(s)*

| Domain description | Analysis | type Position(s) on protein |
|---|---|---|
| Protein kinase | BlastProDom | 367-569 |
| Tyrosine protein kinase | FPrintScan | 443-456, 482-500, 530-540, 594-616 |
| Ankyrin | FPrintScan | 71-83, 256-268 |
| Ankyrin | HMMPfam | 37-69, 70-102, 103-137, 138-168, 173-206, 208-242, 243-275, 285-317 |

(continued)

| Domain description | Analysis | type Position(s) on protein |
|---|---|---|
| Protein kinase | HMMPfam | 367-623 |
| Tyrosine protein kinase | HMMSmart | 367-623 |
| Serine | HMMSmart | 367-627 |
| Ankyrin | HMMSmart | 37-66, 70-99, 103-134, 138-169, 173-204, 208-239, 243-272, 285-314 |
| Protein kinase | ProfileScan | 367-627 |
| Ankyrin | ProfileScan | 37-69, 73-102, 103-135, 173-205, 243-275 |
| Ankyrin | ProfileScan | 17-305 |
| Protein kinase | ScanRegExp | 373-394 |

Variant protein C03950_3_P10 (SEQ. ID NO:215) is encoded by the following transcript(s): C03950_3_T8 (SEQ. ID NO:159), for which the coding portion starts at position 1 and ends at position 2217.

Variant protein C03950_3_PI (SEQ. ID NO:216) according to the present invention is encoded by transcript C03950_3_T9 (SEQ. ID NO: 160). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P11 (SEQ. ID NO:216) and TNI3K_HUMAN (SEQ. ID NO:396):

A. An isolated chimeric polypeptide encoding for C03950_3_P11 (SEQ. ID NO:216), comprising a first amino acid sequence being at least 70%, optionally at least 80%, preferably at least 85%, more preferably at least 90% and most preferably at least 95%, homologous to a polypeptide having the sequence PSPCGLHFLIPWLTQ (SEQ. ID NO: 351) corresponding to amino acids 1-15 of C03950_3_P11 (SEQ. ID NO:216), a second amino acid sequence being at least 90% homologous to DNAELITSLLHSGADIQQVGYGGLTALHIATIAGHLEAADV-LLQHGANVNIQDAVFFTPLHIA AYYGHEQVTRLLLKFGADVNVSGEVGDRPLHLASAKGFLNIAKLLMEEG-SKADVNAQDNED HVPLHFCSRFGHHDIVKYLLQSDLEVQPHVVNIYGDTPLHLACYNGKFEVAKEIIQISGTE-SLT KENIFSETAFHSACTYGKSIDLVKFLLDQNVININHQGRDGHTGLHSACYHGHIRLVQFLLDN GADMNL-VACDPSRSSGEKDEQTCLMWAYEKGHDAIVTLLKHYKRPQDELPCNEYSQPGGD GSYVSVPSPLGKIKSMTKEKADILLLRAGLPSHFHLQLSEIEFHEIIGSGSFGKVYKGRCRNKIV AIKRYRANTYCSKSDVDMFCREVSILCQLNHPCVIQFVGACLNDPSQFAIVTQYISGGSLFSLL HEQKRILD-LQSKLIIAVDVAKGMEYLHNLTQPIIHRDLN corresponding to amino acids 213-691 of TNI3K_HUMAN (SEQ. ID NO: 396), which also corresponds to amino acids 16 - 494 of C03950_3_P11 (SEQ. ID NO:216), a third amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 495 - 534 of C03950_3_PI (SEQ. ID NO:216), and a fourth amino acid sequence being at least 90% homologous to ESRFLQ-SLDEDNMTKQPGNLRWMAPEVFTQCTRYTIKADVFSYALCLWEILTGEIPFAHLKPA AAAADMAYHHIRPPIGYSIPKPISSLLIRGWNACPEGRPEFSEWMKLEECLCNIELMSPASSNS SGSLSPSSSSDCLVNRGGPGRSHVAALRSRFELEYALNARSYAALSQSAGQYSSQGLSLEEMK RSLQYT-PIDKYGYVSDPMSSMHFHSCRNSSSFEDSS corresponding to amino acids 710 - 936 of TNI3K HUMAN (SEQ. ID NO: 396), which also corresponds to amino acids 535 - 761 of C03950_3_P11 (SEQ. ID NO:216), wherein said first amino acid sequence, second amino acid sequence, third amino acid sequence and fourth amino acid sequence are contiguous and in a sequential order.

B. An isolated polypeptide encoding for a head of C03950_3_P11 (SEQ. ID NO:216), comprising a polypeptide being at least 70%, optionally at least about 80%, preferably at least about 85%, more preferably at least about 90% and most preferably at least about 95% homologous to the sequence PSPCGLHFLIPWLTQ (SEQ. ID NO: 351) of C03950_3_P11 (SEQ. ID NO:216).

2. Comparison report between C03950_3_P11 (SEQ. ID NO:216) and Q6MZS9_HUMAN (SEQ. ID NO:211):

A. An isolated chimeric polypeptide encoding for C03950_3_P11 (SEQ. ID NO:216), comprising a first amino acid sequence being at least 70%, optionally at least 80%, preferably at least 85%, more preferably at least 90% and most preferably at least 95%, homologous to a polypeptide having the sequence PSPCGLHFLIPWLTQ (SEQ. ID NO: 351) corresponding to amino acids 1 - 15 of C03950_3_P11 (SEQ. ID NO:216), a second amino acid sequence being at least 90% homologous to DNAELITSLLHSGADIQQVGYGGLTALHIATIAGHLEAADV-LLQHGANVNIQDAVFFTPLHIA AYYGHEQVTRLLLKFGADVNVSGEVGDRPLHLASAKGFLNIAKLLMEEG-SKADVNAQDNED HVPLHFCSRFGHHD corresponding to amino acids 230 - 367 of Q6MZS9_HUMAN (SEQ. ID NO:211), which also corresponds to amino acids 16 - 153 of C03950_3_PI1 (SEQ. ID NO:216), a bridging amino acid I corresponding to amino acid 154 of C03950_3_PlI (SEQ. ID NO:216), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9 HUMAN (SEQ. ID NO:211), which also corresponds to amino acids 155 - 270 of C03950_3_P11 (SEQ. ID NO:216), a bridging amino acid N corresponding to amino acid 271 of C03950_3_P11 (SEQ. ID NO:216), a fourth amino acid sequence being at least 90% homologous to LVACDPSRSSGEKDEQTCLMWAYEKGHDAIVTLLKHYKRPQDELPCNEYSQPGGDGSYVSV PSPLG-KIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIA-VDVAKGMEYLHNLTQPIIHRDLNR corresponding to amino acids 486 - 709 of Q6MZS9 HUMAN (SEQ. ID NO: 211), which also corresponds to amino acids 272 - 495 of C03950_3_P11 (SEQ. ID NO:216), and a fifth amino acid sequence being at least 70%, optionally at least 80%, preferably at least 85%, more preferably at least 90% and most preferably at least 95% homologous to a polypeptide having the sequence SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEWMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) corresponding to amino acids 496 - 761 of C03950_3_P11 (SEQ. ID NO:216), wherein said first amino acid sequence, second amino acid sequence, bridging amino acid, third amino acid sequence, bridging amino acid, fourth amino acid sequence and fifth amino acid sequence are contiguous and in a sequential order.

B. An isolated polypeptide encoding for a head of C03950_3_P11 (SEQ. ID NO:216), comprising a polypeptide being at least 70%, optionally at least about 80%, preferably at least about 85%, more preferably at least about 90% and most preferably at least about 95% homologous to the sequence PSPCGLHFLIPWLTQ (SEQ. ID NO: 351) of C03950_3_P11 (SEQ. ID NO:216).

C. An isolated polypeptide encoding for an edge portion of C03950_3_P11 (SEQ. ID NO:216), comprising an amino acid sequence being at least 70%, optionally at least about 80%, preferably at least about 85%, more preferably at least about 90% and most preferably at least about 95% homologous to the sequence SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEWMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) of C03950_3_P11 (SEQ. ID NO:216).

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 95:

*Table 95 - InterPro domain(s)*

| Domain description | Analysis type | Position(s) on protein |
|---|---|---|
| Protein kinase | BlastProDom | 367-591 |
| Ankyrin | FPrintScan | 71-83, 256-268 |
| Ankyrin | HMMPfam | 37-69, 70-102, 103-137, 138-168, 173-206, 208-242, 243-275, 285-317 |
| Protein kinase | HMMPfam | 367-645 |
| Tyrosine protein kinase | HMMSmart | 367-645 |
| Serine | HMMSmart | 367-649 |
| Ankyrin | HMMSmart | 37-66, 70-99, 103-134, 138-169, 173-204, 208-239, 243-272, 285-314 |
| Protein kinase | ProfileScan | 367-649 |
| Ankyrin | ProfileScan | 37-69, 73-102, 103-135, 173-205, 243-275 |
| Ankyrin | ProfileScan | 17-305 |
| Protein kinase | ScanRegExp | 373-394 |

Variant protein C03950_3_P11 (SEQ. ID NO:216) is encoded by the following transcript(s): C03950_3_T9 (SEQ. ID NO:160), for which the coding portion starts at position 1 and ends at position 2283.

Variant protein C03950_3_P12 (SEQ. ID NO:217) according to the present invention is encoded by transcript C03950_3_T10 (SEQ. ID NO:161). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P12 (SEQ. ID NO:217) and TNI3K_HUMAN (SEQ. ID NO: 396):

A. An isolated chimeric polypeptide encoding for C03950_3_P12 (SEQ. ID NO:217), comprising a first amino acid sequence being at least 70%, optionally at least 80%, preferably at least 85%, more preferably at least 90% and most preferably at least 95%, homologous to a polypeptide having the sequence AVRRGLREGGA (SEQ. ID NO: 342) corresponding to amino acids 1 - 11 of C03950_3_PI2 (SEQ. ID NO:217), a second amino acid sequence being at least 90% homologous to MAAARDPPEVSLREATQRKLRRFSELRGKLVARGEFWDI-VAITAADEKQELAYNQQLSEKLK RKELPLGVQYHVFVDPAGAKIGNGGSTLCALQCLEKLYGDKWNSFTILLI-HSDEWKKKVSES YVITIERLEDDLQIKEKELTELRNIFGSDEAFSKVNLNYRTENGLSLLHLCCICGGKK-SHIRTLM LKGLRPSRLTRNGFTALHLAVYKDNAELITSLLHSGADIQQVGYGGLTALHIATIAGHLEAAD VLL-QHGANVNIQDAVFFTPLHIAAYYGHEQVTRLLLKFGADVNVSGEVGDRPLHLASAKGFL NIAKLLMEEGSKADVNAQDNEDHVPLHFCSRFGHHDIVKYLLQSDLEVQPHUVNIYGDTPLH LACYNGKFEVAKEIIQISGTESLTKENIFSETAFHSACTYGKSIDLVKFLLDQNVININHQGRDG HTGLHSACYHGHIRLVQFLLDNGADMNLVACDPSRSSGEKDEQTCLMWAYEKGHDAIVTLL KHYKRPQDELPCNEYSQPGGDGSYVSVPSPLGKIKSMTKEKADILLLRAGLPSHFHLQLSEIEF HEIIGSGSFGKVYKGRCRNKIVAIKRYRANTYCSKSDVDMFCREVSILCQLNHPCVIQFVGAC LNDPSQFAIVTQYISGGSLFSLLHEQKRILDLQSKLIIAVDVAKGMEYLHNLTQPIIHRDLNSHN ILLYEDGHAWADFGESRFLQSLDEDNMTKQPGNLRWMAPEVFTQCTRYTIKADVFSYALCL WEILTGEIPFAHLKPAAAAADMAYHHIRPPIGYSIPKPISSLLIRGWNACPE corresponding to amino acids 1 - 808 of TNI3K_HUMAN (SEQ. ID NO: 396), which also corresponds to amino acids 12 - 819 of C03950_3_P12 (SEQ. ID NO:217), and a third amino acid sequence being at least 70%, optionally at least 80%, preferably at least 85%, more preferably at least 90% and most preferably at least 95% homologous to a polypeptide having the sequence AKSRPSHYPVSSVYTETLKKKNEDRFGMWIEYLRR (SEQ. ID NO: 356) corresponding to amino acids 820 - 854 of C03950_3_P12 (SEQ. ID NO:217), wherein said first amino acid sequence, second amino acid sequence and third amino acid sequence are contiguous and in a sequential order.

B. An isolated polypeptide encoding for a head of C03950_3_P12 (SEQ. ID NO:217), comprising a polypeptide being at least 70%, optionally at least about 80%, preferably at least about 85%, more preferably at least about 90% and most preferably at least about 95% homologous to the sequence AVRRGLREGGA (SEQ. ID NO: 342) of C03950_3_P12 (SEQ. ID NO:217).

C. An isolated polypeptide encoding for an edge portion ofC03950_3_P12 (SEQ. ID NO:217), comprising an amino acid sequence being at least 70%, optionally at least about 80%, preferably at least about 85%, more preferably at least about 90% and most preferably at least about 95% homologous to the sequence AKSRPSHYPVSSVYTETLKKKNEDRFGMWIEYLRR (SEQ. ID NO: 356) of C03950_3_P12 (SEQ. ID NO:217).

2. Comparison report between C03950_3_P12 (SEQ. ID NO:217) and Q6MZS9_HUMAN (SEQ. ID NO:211):

A. An isolated chimeric polypeptide encoding for C03950_3_P12 (SEQ. ID NO:217), comprising a first amino acid sequence being at least 70%, optionally at least 80%, preferably at least 85%, more preferably at least 90% and most preferably at least 95%, homologous to a polypeptide having the sequence AVRRGLR (SEQ. ID NO: 344) corresponding to amino acids 1 - 7 of C03950_3_P12 (SEQ. ID NO:217), a second amino acid sequence being at least 90% homologous to EGGAMAAARDPPEVSLREATQRKLRRFSELRGKLVARGEFWDIVAITAADEKQELAYNQQLS EKLKRKELPLGVQYHVFVDPAGAKIGNGGSTLCALQCLEKLYGDKWNSFTILLIHSDEWKKK VSESYVITIERLEDDLQIKEKELTELRNIFGSDEAFSKVNLNYRTENGLSLLHLCCICGGKKSHI RTLMLKGLRPSRLTRNGFTALHLAVYKDNAELITSLLHSGADIQQVGYGGLTALHIATIAGHL EAADVLLQHGANVNIQDAVFFTPLHIAAYYGHEQVTRLLLKFGADVNVSGEVGDRPLHLAS AKGFLNIAKLLMEEGSKADVNAQDNEDHVPLHFCSRFGHHD corresponding to amino acids 14 - 367 of Q6MZS9_HUMAN (SEQ. ID NO:211), which also corresponds to amino acids 8 - 361 of C03950_3_P12 (SEQ. ID NO:217), a bridging amino acid I corresponding to amino acid 362 of C03950_3_P12 (SEQ. ID NO:217), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 363 - 478 of C03950_3_P12 (SEQ. ID NO:217), a bridging amino acid N corresponding to amino acid 479 of C03950_3_P12 (SEQ. ID NO:217), a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIAVDVAKGMEYLHNLTQPIIHRDLN corresponding to amino acids 486 - 708 of Q6MZS9_HUMAN (SEQ. ID NO: 211), which also corresponds to amino acids 480 - 702 of C03950_3_P12 (SEQ. ID NO:217), and a fifth amino acid sequence being at least 70%, optionally at least 80%, preferably at least 85%, more preferably at least 90% and most preferably at least 95% homologous to a polypeptide having the sequence SHNILLYEDGHAWADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEAKSRPSHYPV SSVYTETLKKKNEDRFGMWIEYLRR (SEQ. ID NO: 358) corresponding to amino acids 703 - 854 of C03950_3_P12 (SEQ. ID NO:217), wherein said first amino acid sequence, second amino acid sequence, bridging amino acid, third amino acid sequence, bridging amino acid, fourth amino acid sequence and fifth amino acid sequence are contiguous and in a sequential order.

B. An isolated polypeptide encoding for a head of C03950_3_P12 (SEQ. ID NO:217), comprising a polypeptide being at least 70%, optionally at least about 80%, preferably at least about 85%, more preferably at least about 90% and most preferably at least about 95% homologous to the sequence AVRRGLR (SEQ. ID NO: 344) of C03950_3_P12 (SEQ. ID NO:217).

C. An isolated polypeptide encoding for an edge portion of C03950_3_P12 (SEQ. ID NO:217), comprising an amino acid sequence being at least 70%, optionally at least about 80%, preferably at least about 85%, more preferably at least about 90% and most preferably at least about 95% homologous to the sequence SHNILLYEDGHAVVADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEAKSRPSHYPV SSVYTETLKKKNEDRFGMWIEYLRR (SEQ. ID NO: 358) of C03950_3_P12 (SEQ. ID NO:217).

3. Comparison report between C03950_3_P12 (SEQ. ID NO:217) and NP_057062 (SEQ. ID NO:210):

A. An isolated chimeric polypeptide encoding for C03950_3_P12 (SEQ. ID NO:217), comprising a first amino acid sequence being at least 70%, optionally at least 80%, preferably at least 85%, more preferably at least 90% and most preferably at least 95%, homologous to a polypeptide having the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P12 (SEQ. ID NO:217), a second amino acid sequence being at least 90% homologous to DEWKKKVSESYVITIERLEDDLQIKEKELTELRNIFGSDEAFSKVNLNYRTENGLSLLHLCCIC GGKKSHIRTLMLKGLRPSRLTRNGFTALHLAVYKDNAELITSLLHSGADIQQVGYGGLTALHI ATIAGHLEAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLNSHNILLYEDGHAVVADFGESRFLQSLDEDNMTKQPGNLRWMAPEVFTQCTRYTI KADVFSYALCLWEILTGEIPFAHLKPAAAAADMAYHHIRPPIGYSIPKPISSLLIRGWNACPE corresponding to amino acids 14 - 707 of NP_057062 (SEQ. ID NO:210), which also corresponds to amino acids 126 - 819 of C03950_3_P12 (SEQ. ID NO:217), and a third amino acid sequence being at least 70%, optionally at least 80%, preferably at least 85%, more preferably at least 90% and most preferably at least 95% homologous to a polypeptide having the sequence AKSRPSHYPVSSVYTETLKKKNEDRFGMWIEYLRR (SEQ. ID NO: 356) corresponding to amino acids 820 - 854 of C03950_3_PI2 (SEQ. ID NO:217), wherein said first amino acid sequence, second amino acid sequence and third amino acid sequence are contiguous and in a sequential order.
B. An isolated polypeptide encoding for a head of C03950_3_P12 (SEQ. ID NO:217), comprising a polypeptide being at least 70%, optionally at least about 80%, preferably at least about 85%, more preferably at least about 90% and most preferably at least about 95% homologous to the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) of C03950_3_P12 (SEQ. ID NO:217).

The localization of the variant protein was determined according to results from a number of different software programs and analyses, including analyses from SignalP and other specialized programs. The variant protein is believed to be located intracellularly.

Variant protein C03950-3-Pl2 (SEQ. ID NO:217) also has the following non-silent SNPs (Single Nucleotide Polymorphisms) as listed in Table 96, (given according to their position(s) on the amino acid sequence, with the alternative amino acid(s) listed.

*Table 96 - Amino acid-mutations*

| SNP position(s) on amino acid sequence | Alternative amino acid(s) |
|---|---|
| 119 | T -> P |

The variant protein has the following domains, as determined by using InterPro. The domains are described in Table 97:

*Table 97 - InterPro domain(s)*

| Domain description | Analysis type | Position(s) on protein |
|---|---|---|
| Protein kinase | BlastProDom | 575-777 |
| Ankyrin | FPrintScan | 279-291, 464-476 |
| Ankyrin | HMMPfam | 178-211, 212-244, 245-277, 278-310, 311-345, 346-376, 381-414, 416-450, 451-483, 493-525 |
| Protein kinase | HMMPfam | 575-832 |
| Tyrosine protein kinase | HMMSmart | 575-833 |
| Serine | HMMSmart | 575-834 |
| Ankyrin | HMMSmart | 178-208, 212-241, 245-274, 278-307, 311-342, 346-377, 381-412, 416-447, 451-480, 493-522 |
| Protein kinase | ProfileScan | 575-837 |
| Ankyrin | ProfileScan | 212-244, 245-277, 281-310, 311-343, 381-413, 451-483 |
| Ankyrin | ProfileScan | 170-513 |
| Protein kinase | ScanRegExp | 581-602 |

Variant protein C03950_3_P12 (SEQ. ID NO:217) is encoded by the following transcript(s): C03950_3_T10 (SEQ. ID NO:161), for which the coding portion starts at position 3 and ends at position 2564. The transcript also has the following SNPs as listed in Table 98 (given according to their position on the nucleotide sequence, with the alternative nucleic acid listed.

*Table 98 - Nucleic acid SNPs*

| SNP position(s) on nucleotide sequence | Alternative nucleic acid(s) |
|---|---|
| 263 | A -> G |
| 357 | A -> C |
| 2677 | T -> C |

Variant protein C03950_3_P13 (SEQ. ID NO:218) according to the present invention is encoded by transcript C03950_3_T11 (SEQ. ID NO:162). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P13 (SEQ. ID NO:218) and TNI3K_HUMAN (SEQ. ID NO:396):

A. An isolated chimeric polypeptide encoding for C03950_3_P13 (SEQ. ID NO:218), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P13 (SEQ. ID NO:218), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHSGADI-QQVGYGGLTALHI ATIAGHLEAADVLLQHGANVNIQDAVFFTPLHIAAYYGHEQVTRLLLKFGADVNVSGEV-GDR PLHLASAKGFLNIAKLLMEEGSKADVNAQDNEDHVPLHFCSRFGHHDIVKYLLQSDLEVQPH VVNIY-GDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLNSHNILLYEDGHAWADFGESRFLQSLDEDNMTKQPGNLRWMAPEVFTQCTRYTI KADVFSYALCLWEILTGEIPFAHLKPAAAAADMAYHHIRPPIGYSIPKPISSLLIRGWNACPE corresponding to amino acids 115 - 808 of TNI3K_HUMAN (SEQ. ID NO: 396), which also corresponds to amino acids 14 - 707 of C03950_3_P13 (SEQ. ID NO:218), and a third amino acid sequence being at least 70%, optionally at least 80%, preferably at least 85%, more preferably at least 90% and most preferably at least 95% homologous to a polypeptide having the sequence AKSRPSHYPVSSVYTETLKKKNEDRFGMWIEYLRR (SEQ. ID NO: 356) corresponding to amino acids 708 - 742 of C03950_3_P13 (SEQ. ID NO:218), wherein said first amino acid sequence, second amino acid sequence and third amino acid sequence are contiguous and in a sequential order.

B. An isolated polypeptide encoding for a head of C03950_3_P13 (SEQ. ID NO:218), comprising a polypeptide being at least 70%, optionally at least about 80%, preferably at least about 85%, more preferably at least about 90% and most preferably at least about 95% homologous to the sequence MGNYKSRPTQTCT (SEQ. ID NO: 346) of C03950_3_P13 (SEQ. ID NO:218).

C. An isolated polypeptide encoding for an edge portion of C03950_3_P13 (SEQ. ID NO: 218), comprising an amino acid sequence being at least 70%, optionally at least about 80%, preferably at least about 85%, more preferably at least about 90% and most preferably at least about 95% homologous to the sequence AKSRPSHYPVSSVYTETLKKKNEDRFGMWIEYLRR (SEQ. ID NO: 356) of C03950_3_P13 (SEQ. ID NO:218).

2. Comparison report between C03950_3_P13 (SEQ. ID NO:218) and NP_057062 (SEQ. ID NO:210):

A. An isolated chimeric polypeptide encoding for C03950_3_P13 (SEQ. ID NO:218), comprising a first amino acid sequence being at least 90% homologous to MGNYKSRPTQTCTDEWKKKVSESYVITIERLEDDL-QIKEKELTELRNIFGSDEAFSKVNLNYR TENGLSLLHLCCICGGKKSHIRTLMLKGLRPSRLTRNGFTALH-LAVYKDNAELITSLLHSGADI QQVGYGGLTALHIATIAGHLEAADVLLQHGANVNIQDAVFFTPLHIAAYYGHE-

QVTRLLLKF GADVNVSGEVGDRPLHLASAKGFLNIAKLLMEEGSKADVNAQDNEDHVPLHFCSRFGHHDI VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADMNLVACDPSRS SGEKDEQTCLMWAYEKGHDAIVTLLKHYKRPQDELPCNEYSQPGGDGSYVSVPSPLGKIKSM TKEKADILLLRAGLPSHFHLQLSEIEFHEIIGSGSFGKVYKGRCRNKIVAIKRYRANTYCSKSDV DMFCREVSILCQLNHPCVIQFVGACLNDPSQFAIVTQYISGGSLFSLLHEQKRILDLQSKLIIAV DVAKGMEYLHNLTQPIIHRDLNSHNILLYEDGHAWADFGESRFLQSLDEDNMTKQPGNLR WMAPEVFTQCTRYTIKADVFSYALCLWEILTGEIPFAHLKPAAAAADMAYHHIRPPIGYSIPKP ISSLLIRGWNACPE corresponding to amino acids 1 - 707 of NP_057062 (SEQ. ID NO:210), which also corresponds to amino acids 1 - 707 of C03950_3_P13 (SEQ. ID NO:218), and a second amino acid sequence being at least 70%, optionally at least 80%, preferably at least 85%, more preferably at least 90% and most preferably at least 95% homologous to a polypeptide having the sequence AKSRPSHYPVSSVYTETLKKKNEDRFGMWIEYLRR (SEQ. ID NO: 356) corresponding to amino acids 708 - 742 of C03950_3_P13 (SEQ. ID NO:218), wherein said first amino acid sequence and second amino acid sequence are contiguous and in a sequential order.

4. Comparison report between C03950_3_P13 (SEQ. ID NO:218) and Q6MZS9_HUMAN (SEQ. ID NO:211):

A. An isolated chimeric polypeptide encoding for C03950_3_P13 (SEQ. ID NO:218), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P13 (SEQ. ID NO:218), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHSGAD-IQQVGYGGLTALHI ATIAGHLEAADVLLQHGANVNIQDAVFFTPLHIAAYYGHEQVTRLLLKFGADVNVSGEV-GDR PLHLASAKGFLNIAKLLMEEGSKADVNAQDNEDHVPLHFCSRFGHHD corresponding to amino acids 132-367 of Q6MZS9_HUMAN (SEQ. ID NO:211), which also corresponds to amino acids 14 - 249 of C03950_3_P13 (SEQ. ID NO:218), a bridging amino acid I corresponding to amino acid 250 of C03950_3_P13 (SEQ. ID NO:218), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 251 - 366 of C03950_3_P13 (SEQ. ID NO:218), a bridging amino acid N corresponding to amino acid 367 of C03950_3_P13 (SEQ. ID NO:218), a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIAVDVAKGMEYLHNLTQPIIHIZDLN corresponding to amino acids 486 - 708 of Q6MZS9_HUMAN (SEQ. ID NO: 211), which also corresponds to amino acids 368 - 590 of C03950_3_P13 (SEQ. ID NO:218), and a fifth amino acid sequence being at least 70%, optionally at least 80%, preferably at least 85%, more preferably at least 90% and most preferably at least 95% homologous to a polypeptide having the sequence SHNILLYEDGHAWADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEAKSRPSHYPV SSVYTETLKKKNEDRFGMWIEYLRR (SEQ. ID NO: 358) corresponding to amino acids 591 - 742 of C03950_3_P13 (SEQ. ID NO:218), wherein said first amino acid sequence, second amino acid sequence, bridging amino acid, third amino acid sequence, bridging amino acid, fourth amino acid sequence and fifth amino acid sequence are contiguous and in a sequential order.

B. An isolated polypeptide encoding for an edge portion of C03950_3_P13 (SEQ. ID NO:218), comprising an amino acid sequence being at least 70%, optionally at least about 80%, preferably at least about 85%, more preferably at least about 90% and most preferably at least about 95% homologous to the sequence SHNILLYEDGHAWADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEAKSRPSHYPV SSVYTETLKKKNEDRFGMWIEYLRR (SEQ. ID NO: 358) of C03950_3_P13 (SEQ. ID NO:218).

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 99:

*Table 99 - InterPro domain(s)*

| Domain description | Analysis type | Position(s) on protein |
|---|---|---|
| Protein kinase | BlastProDom | 463-665 |
| Ankyrin | FPrintScan | 167-179, 352-364 |
| Ankyrin | HMMPfam | 66-99, 100-132, 133-165, 166-198, 199-233, 234-264, 269-302, 304-338, 339-371, 381-413 |
| Protein kinase | HMMPfam | 463-720 |
| Tyrosine protein kinase | HMMSmart | 463-721 |
| Serine | HMMSmart | 463-722 |
| Ankyrin | HMMSmart | 66-96, 100-129, 133-162, 166-195, 199-230, 234-265, 269-300, 304-335, 339-368, 381-410 |
| Protein kinase | ProfileScan | 463-725 |
| Ankyrin | ProfileScan | 100-132, 133-165, 169-198, 199-231, 269-301, 339-371 |
| Ankyrin | ProfileScan | 58-401 |
| Protein kinase | ScanRegExp | 469-490 |

Variant protein C03950_3_P13 (SEQ. ID NO:218) is encoded by the following transcript(s): C03950_3_T11 (SEQ. ID NO:162), for which the coding portion starts at position 389 and ends at position 2614. The transcript also has the following SNPs as listed in Table 100 (given according to their position on the nucleotide sequence, with the alternative nucleic acid listed.

*Table 100 - Nucleic acid SNPs*

| SNP position(s) on nucleotide sequence | Alternative nucleic acid(s) |
|---|---|
| 2727 | T -> C |

Variant protein C03950_3_P15 (SEQ. ID NO:219) according to the present invention is encoded by transcript C03950_3_T13 (SEQ. ID NO:163). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P15 (SEQ. ID NO:219) and TNI3K_HUMAN (SEQ. ID NO:396):

A. An isolated chimeric polypeptide encoding for C03950_3_P15 (SEQ. ID NO:219), comprising a first amino acid sequence being at least 70%, optionally at least 80%, preferably at least 85%, more preferably at least 90% and most preferably at least 95%, homologous to a polypeptide having the sequence AVRRGLREGGA (SEQ. ID NO: 342) corresponding to amino acids 1 - 11 of C03950-3-P15 (SEQ. ID NO:219), a second amino acid sequence being at least 90% homologous to MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHSDEWKKKVSES YVITIERLEDDLQIKEKELTELRNIFGSDEAFSKVNLNYRTENGLSLLHLCCICGGKKSHIRTLM LKGLRPSRLTRNGFTALHLAVYKDNAELITSLLHSGADIQQVGYGGLTALHIATIAGHLEAAD VLLQHGANVNIQDAVFFTPLHIAAYYGHEQVTRLLLKFGADVNVSGEVGDRPLHLASAKGFL NIAKLLMEEGSKADVNAQDNEDHVPLHFCSRFGHHDIVKYLLQSDLEVQPHWNIYGDTPLH LACYNGKFEVAKEIIQISGTESLTKENIFSETAFHSACTYGKSIDLVKFLLDQNVININHQGRDG HTGLHSACYHGHIRLVQFLLDNGADMNLVACDPSRSSGEKDEQTCLMWAYEKGHDAIVTLL KHYKRPQDELPCNEYSQPGGDGSYVSVPSPLGKIKSMTKEKADILLLRAGLPSHFHLQLSEIEF HEIIGSGSFGKVYKGRCRNKIVAIKRYRANTYCSKSDVDMFCREVSILCQLNHPCVIQFVGAC LNDPSQFAIVTQYISGGSLFSLLHEQKRILDLQSKLIIAVDVAKGMEYLHNLTQPIIHRDLN corresponding to amino acids 1 - 691 of TNI3K_HUMAN (SEQ. ID NO: 396), which also corresponds to amino acids 12 - 702 of C03950_3_P15 (SEQ. ID NO:219), and a third amino acid sequence being at least 70%, optionally at least 80%, preferably at least 85%, more preferably at least 90% and most preferably at least 95% homologous to a polypeptide having the sequence RYFFPK (SEQ. ID NO: 364) corresponding to amino acids 703 - 708 of C03950_3_P15 (SEQ. ID NO:219), wherein said first amino acid sequence, second amino acid sequence and third amino acid sequence are contiguous and in a sequential order.

B. An isolated polypeptide encoding for a head of C03950_3_P15 (SEQ. ID NO:219), comprising a polypeptide being at least 70%, optionally at least about 80%, preferably at least about 85%, more preferably at least about 90% and most preferably at least about 95% homologous to the sequence AVRRGLREGGA (SEQ. ID NO: 342) of C03950_3_P15 (SEQ. ID NO:219).

C. An isolated polypeptide encoding for an edge portion of C03950_3_P15 (SEQ. ID NO:219), comprising an amino acid sequence being at least 70%, optionally at least about 80%, preferably at least about 85%, more preferably at least about 90% and most preferably at least about 95% homologous to the sequence RYFFPK (SEQ. ID NO: 364) of C03950_3_P15 (SEQ. ID NO:219).

2. Comparison report between C03950_3_P15 (SEQ. ID NO:219) and Q6MZS9_HUMAN (SEQ. ID NO:211):

A. An isolated chimeric polypeptide encoding for C03950_3_P15 (SEQ. ID NO:219), comprising a first amino acid sequence being at least 70%, optionally at least 80%, preferably at least 85%, more preferably at least 90% and most preferably at least 95%, homologous to a polypeptide having the sequence AVRRGLR (SEQ. ID NO: 344) corresponding to amino acids 1 - 7 of C03950_3_P15 (SEQ. ID NO:219), a second amino acid sequence being at least 90% homologous to EGGAMAAARDPPEVSLREATQRKLRRFSELRGKLVARGEFW-DIVAITAADEKQELAYNQQLS EKLKRKELPLGVQYHVFVDPAGAKIGNGGSTLCALQCLEKLYGDKWNS-FTILLIHSDEWKKK VSESYVITIERLEDDLQIKEKELTELRNIFGSDEAFSKVNLNYRTENGLSLLHLCCICGG-KKSHI RTLMLKGLRPSRLTRNGFTALHLAVYKDNAELITSLLHSGADIQQVGYGGLTALHIATIAGHL EAADVLLQHGANVNIQDAVFFTPLHIAAYYGHEQVTRLLLKFGADVNVSGEVGDRPLHLAS AKGFLNIAKLLMEEGSKADVNAQDNEDHVPLHFCSRFGHHD corresponding to amino acids 14 - 367 of Q6MZS9_HUMAN (SEQ. ID NO:211), which also corresponds to amino acids 8 - 361 of C03950_3_P15 (SEQ. ID NO:219), a bridging amino acid I corresponding to amino acid 362 of C03950_3_P15 (SEQ. ID NO:219), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9 HUMAN (SEQ. ID NO:211), which also corresponds to amino acids 363 - 478 of C03950_3_P15 (SEQ. ID NO:219), a bridging amino acid N corresponding to amino acid 479 of C03950_3_P15 (SEQ. ID NO:219), and a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIAVDVAKGMEYLHNLTQPIIHRDLNRYFFPK corresponding to amino acids 486-714 of Q6MZS9_HUMAN (SEQ. ID NO:211), which also corresponds to amino acids 480 - 708 of C03950_3_P15 (SEQ. ID NO:219), wherein said first amino acid sequence, second amino acid sequence, bridging amino acid, third amino acid sequence, bridging amino acid and fourth amino acid sequence are contiguous and in a sequential order.

B. An isolated polypeptide encoding for a head of C03950_3_P15 (SEQ. ID NO:219), comprising a polypeptide being at least 70%, optionally at least about 80%, preferably at least about 85%, more preferably at least about 90% and most preferably at least about 95% homologous to the sequence AVRRGLR (SEQ. ID NO: 344) of C03950_3_P15 (SEQ. ID NO:219).

3. Comparison report between C03950_3_P15 (SEQ. ID NO:219) and NP_057062 (SEQ. ID NO:210):

A. An isolated chimeric polypeptide encoding for C03950_3_P15 (SEQ. ID NO:219), comprising a first amino acid sequence being at least 70%, optionally at least 80%, preferably at least 85%, more preferably at least 90% and most preferably at least 95%, homologous to a polypeptide having the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P15 (SEQ. ID NO:219), a second amino acid sequence being at least 90% homologous to DEWKKKVSESYVITIERLEDDLQIKEKELTELRNIFGSDEAFSKVNL-NYRTENGLSLLHLCCIC GGKKSHIRTLMLKGLRPSRLTRNGFTALHLAVYKDNAELITSLLHSGADIQQVGYG-GLTALHI ATIAGHLEAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHDIVKYLLQSDLEVQPH

VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLN corresponding to amino acids 14 - 590 of NP_057062 (SEQ. ID NO:210), which also corresponds to amino acids 126 - 702 of C03950_3_P15 (SEQ. ID NO:219), and a third amino acid sequence being at least 70%, optionally at least 80%, preferably at least 85%, more preferably at least 90% and most preferably at least 95% homologous to a polypeptide having the sequence RYFFPK (SEQ. ID NO: 364) corresponding to amino acids 703 - 708 of C03950_3_P15 (SEQ. ID NO:219), wherein said first amino acid sequence, second amino acid sequence and third amino acid sequence are contiguous and in a sequential order.

B. An isolated polypeptide encoding for a head of C03950_3_P15 (SEQ. ID NO:219), comprising a polypeptide being at least 70%, optionally at least about 80%, preferably at least about 85%, more preferably at least about 90% and most preferably at least about 95% homologous to the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) of C03950_3_P15 (SEQ. ID NO:219).

The localization of the variant protein was determined according to results from a number of different software programs and analyses, including analyses from SignalP and other specialized programs. The variant protein is believed to be located intracellularly.

Variant protein C03950_3_P15 (SEQ. ID NO:219) also has the following non-silent SNPs (Single Nucleotide Polymorphisms) as listed in Table 101, (given according to their position(s) on the amino acid sequence, with the alternative amino acid(s) listed.

*Table 101 - Amino acid mutations*

| SNP position(s) on amino acid sequence | Alternative amino acid(s) |
|---|---|
| 119 | T -> P |

The variant protein has the following domains, as determined by using InterPro. The domains are described in Table 102:

*Table 102 - InterPro domain(s)*

| Domain description | Analysis type | Position(s) on protein |
|---|---|---|
| Protein kinase | BlastProDom | 575-704 |
| Ankyrin | FPrintScan | 279-291, 464-476 |
| Ankyrin | HMMPfam | 178-211, 212-244, 245-277, 278-310, 311-345, 346-376, 381-414, 416-450, 451-483, 493-525 |
| Tyrosine protein kinase | HMMSmart | 575-708 |
| Serine | HMMSmart | 575-705 |
| Ankyrin | HMMSmart | 178-208, 212-241, 245-274, 278-307, 311-342, 346-377, 381-412, 416-447, 451-480, 493-522 |
| Protein kinase | ProfileScan | 575-708 |
| Ankyrin | ProfileScan | 212-244, 245-277, 281-310, 311-343, 381-413, 451-483 |
| Ankyrin | ProfileScan | 170-513 |
| Protein kinase | ScanRegExp | 581-602 |

Variant protein C03950_3_P15 (SEQ. ID NO:219) is encoded by the following transcript(s): C03950_3_T13 (SEQ. ID NO:163), for which the coding portion starts at position 3 and ends at position 2126. The transcript also has the following SNPs as listed in Table 103 (given according to their position on the nucleotide sequence, with the alternative nucleic acid listed.

*Table 103 - Nucleic acid SNPs*

| SNP position(s) on nucleotide sequence | Alternative nucleic acid(s) |
|---|---|
| 263 | A -> G |
| 357 | A -> C |
| 7207 | C -> A |

Variant protein C03950_3_P17 (SEQ. ID NO:220) according to the present invention is encoded by transcript C03950_3_T15 (SEQ. ID NO:164). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P17 (SEQ. ID NO:220) and TNI3K_HUMAN (SEQ. ID NO: 396):

A. An isolated chimeric polypeptide encoding for C03950_3_P17 (SEQ. ID NO:220), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P17 (SEQ. ID NO:220), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHS-GADIQQVGYGGLTALHI ATIAGHLEAADVLLQHGANVNIQDAVFFTPLHIAAYYGHEQVTRLLLKFGADVNVS-GEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLN corresponding to amino acids 115 - 691 of TNI3K_HUMAN (SEQ. ID NO: 396), which also corresponds to amino acids 14 - 590 of C03950_3_P17 (SEQ. ID NO:220), and a third amino acid sequence being at least 70%, optionally at least 80%, preferably at least 85%, more preferably at least 90% and most preferably at least 95% homologous to a polypeptide having the sequence RCCTGWLSCYHPD (SEQ. ID NO: 368) corresponding to amino acids 591 - 603 of C03950_3_P17 (SEQ. ID NO:220), wherein said first amino acid sequence, second amino acid sequence and third amino acid sequence are contiguous and in a sequential order.

B. An isolated polypeptide encoding for a head of C03950_3_P17 (SEQ. ID NO:220), comprising a polypeptide being at least 70%, optionally at least about 80%, preferably at least about 85%, more preferably at least about 90% and most preferably at least about 95% homologous to the sequence MGNYKSRPTQTCT (SEQ. ID NO: 346) of C03950_3_P17 (SEQ. ID NO:220).

C. An isolated polypeptide encoding for an edge portion of C03950_3_P17 (SEQ. ID NO:220), comprising an amino acid sequence being at least 70%, optionally at least about 80%, preferably at least about 85%, more preferably at least about 90% and most preferably at least about 95% homologous to the sequence CCTGWLSCYHPD (SEQ. ID NO: 368) of C03950_3_P17 (SEQ. ID NO:220).

2. Comparison report between C03950_3_P17 (SEQ. ID NO:220) and Q6MZS9_HUMAN (SEQ. ID NO:211):

A. An isolated chimeric polypeptide encoding for C03950_3_P17 (SEQ. ID NO:220), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P17 (SEQ. ID NO:220), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHS-GADIQQVGYGGLTALHI ATIAGHLEAADVLLQHGANVNIQDAVFFTPLHIAAYYGHEQVTRLLLKFGADVNVS-GEVGDR PLHLASAKGFLNIAKLLMEEGSKADVNAQDNEDHVPLHFCSRFGHHD corresponding to amino acids 132 - 367 of Q6MZS9_HUMAN (SEQ. ID NO:211), which also corresponds to amino acids 14 - 249 of C03950_3_P17 (SEQ. ID NO:220), a bridging amino acid I corresponding to amino acid 250 of C03950_3_P17 (SEQ. ID NO:220), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 251 - 366 of C03950_3_P17 (SEQ. ID NO:220), a bridging amino acid N corresponding to amino acid 367 of C03950_3_P17 (SEQ. ID NO:220), a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIAVDVAKGMEYLHNLTQPIIHRDLNR corresponding to amino acids 486 - 709 of Q6MZS9_HUMAN (SEQ. ID NO:211), which also corresponds to amino acids 368 - 591 of C03950_3_P17 (SEQ. ID NO:220), and a fifth amino acid sequence being at least 70%, optionally at least 80%, preferably at least 85%, more preferably at least 90% and most preferably at least 95% homologous to a polypeptide having the sequence CCTGWLSCYHPD (SEQ. ID NO: 369) corresponding to amino acids 592 - 603 of C03950_3_P17 (SEQ. ID NO:220), wherein said first amino acid sequence, second amino acid sequence, bridging amino acid, third amino acid sequence, bridging amino acid, fourth amino acid sequence and fifth amino acid sequence are contiguous and in a sequential order.

B. An isolated polypeptide encoding for a head of C03950_3_P17 (SEQ. ID NO:220), comprising a polypeptide being at least 70%, optionally at least about 80%, preferably at least about 85%, more preferably at least about 90% and most preferably at least about 95% homologous to the sequence MGNYKSRPTQTCT (SEQ. ID NO: 346) of C03950_3_P17 (SEQ. ID NO:220).

C. An isolated polypeptide encoding for an edge portion of C03950_3_P17 (SEQ. ID NO:220), comprising an amino acid sequence being at least 70%, optionally at least about 80%, preferably at least about 85%, more preferably at least about 90% and most preferably at least about 95% homologous to the sequence CCTGWLSCYHPD (SEQ. ID NO: 369) of C03950_3_P17 (SEQ. ID NO:220).

3. Comparison report between C03950_3_P17 (SEQ. ID NO:220) and NP_057062 (SEQ. ID NO:210):

A. An isolated chimeric polypeptide encoding for C03950_3_P17 (SEQ. ID NO:220), comprising a first amino acid sequence being at least 90% homologous to MGNYKSRPTQTCTDEWKKKVSESYVITIERLEDDL-QIKEKELTELRNIFGSDEAFSKVNLNYR TENGLSLLHLCCICGGKKSHIRTLMLKGLRPSRLTRNGFTALH-LAVYKDNAELITSLLHSGADI QQVGYGGLTALHIATIAGHLEAADVLLQHGANVNIQDAVFFTPLHIAAYY-GHEQVTRLLLKF GADVNVSGEVGDRPLHLASAKGFLNIAKLLMEEGSKADVNAQDNEDHVPLHFCSRF-GHHDI VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADMNLVACDPSRS SGEKDEQTCLMWAYEKGHDAIVTLLKHYKRPQDELPCNEYSQPGGDGSYVSVPSPLGKIKSM TKEKADILLLRAGLPSHFHLQLSEIEFHEIIGSGSFGKVYKGRCRNKIVAIKRYRANTYCSKSDV DMFCREVSILCQLNHPCVIQFVGACLNDPSQFAIVTQYISGGSLFSLLHEQKRILDLQSKLIIAV DVAKGMEYLHNLTQPIIHRDLN corresponding to amino acids 1 - 590 of NP_057062 (SEQ. ID NO:210), which also corresponds to amino acids 1 - 590 of C03950_3_P17 (SEQ. ID NO:220), and a second amino acid sequence being at least 70%, optionally at least 80%, preferably at least 85%, more preferably at least 90% and most preferably at least 95% homologous to a polypeptide having the sequence RCCTGWLSCYHPD (SEQ. ID NO: 368) corresponding to amino acids 591 - 603 of C03950_3_P17 (SEQ. ID NO:220), wherein said first amino acid sequence and second amino acid sequence are contiguous and in a sequential order.

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 104:

*Table 104 - InterPro domain(s)*

| Domain description | Analysis type | Position(s) on protein |
|---|---|---|
| Protein kinase | BlastProDom | 463-590 |
| Ankyrin | FPrintScan | 167-179, 352-364 |

(continued)

| Domain description | Analysis type | Position(s) on protein |
|---|---|---|
| Ankyrin | HMMPfam | 66-99, 100-132, 133-165, 166-198, 199-233, 234-264, 269-302, 304-338, 339-371, 381-413 |
| Tyrosine protein kinase | HMMSmart | 463-602 |
| Serine | HMMSmart | 463-603 |
| Ankyrin | HMMSmart | 66-96, 100-129, 133-162, 166-195, 199-230, 234-265, 269-300, 304-335, 339-368, 381-410 |
| Protein kinase | ProfileScan | 463-603 |
| Ankyrin | ProfileScan | 100-132, 133-165, 169-198, 199-231, 269-301, 339-371 |
| Ankyrin | ProfileScan | 58-401 |
| Protein kinase | ScanRegExp | 469-490 |

Variant protein C03950_3_P17 (SEQ. ID NO:220) is encoded by the following transcript(s): C03950_3_T15 (SEQ. ID NO:164), for which the coding portion starts at position 389 and ends at position 2197.

Variant protein C03950_3_P19 (SEQ. ID NO:221) according to the present invention is encoded by transcript C03950_3_T17 (SEQ. ID NO:165). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P19 (SEQ. ID NO:221) and TNI3K_HUMAN (SEQ. ID NO: 396):

A. An isolated chimeric polypeptide encoding for C03950_3_P19 (SEQ. ID NO:221), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P19 (SEQ. ID NO:221), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHS-GADIQQVGYGGLTALHI ATIAGHLEAADVLLQHGANVNIQDAVFFTPLHIAAYYGHEQVTRLLLKFGADVNVS-GEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYICRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLN corresponding to amino acids 115 - 691 of TNI3K_HUMAN (SEQ. ID NO: 396), which also corresponds to amino acids 14 - 590 of C03950_3_P19 (SEQ. ID NO:221), and a third amino acid sequence being at least 70%, optionally at least 80%, preferably at least 85%, more preferably at least 90% and most preferably at least 95% homologous to a polypeptide having the sequence RAS (SEQ. ID NO: 370) corresponding to amino acids 591 - 593 of C03950_3_P19 (SEQ. ID NO:221), wherein said first amino acid sequence, second amino acid sequence and third amino acid sequence are contiguous and in a sequential order.

B. An isolated polypeptide encoding for an edge portion of C03950_3_P19 (SEQ. ID NO:221), comprising an amino acid sequence being at least 70%, optionally at least about 80%, preferably at least about 85%, more preferably at least about 90% and most preferably at least about 95% homologous to the sequence RAS (SEQ. ID NO: 370) of C03950_3_P19 (SEQ. ID NO:221).

2. Comparison report between C03950_3_P19 (SEQ. ID NO:221) and Q6MZS9_HUMAN (SEQ. ID NO:211):

A. An isolated chimeric polypeptide encoding for C03950_3_P19 (SEQ. ID NO:221), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P19 (SEQ. ID NO:221), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHS-GADIQQVGYGGLTALHI ATIAGHLEAADVLLQHGANVNIQDAVFFTPLHIAAYYGHEQVTRLLLKFGADVNVS-GEVGDR PLHLASAKGFLNIAKLLMEEGSKADVNAQDNEDHVPLHFCSRFGHHD corresponding to amino acids 132 - 367 of Q6MZS9_HUMAN (SEQ. ID NO:211), which also corresponds to amino acids 14 - 249 of C03950_3_P19 (SEQ. ID NO:221), a bridging amino acid I corresponding to amino acid 250 of C03950_3_P19 (SEQ. ID NO:221), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 251 - 366 of C03950_3_P19 (SEQ. ID NO:221), a bridging amino acid N corresponding to amino acid 367 of C03950_3_P19 (SEQ. ID NO:221), a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIAVDVAKGMEYLHNLTQPIIHRDLNR corresponding to amino acids 486 - 709 of Q6MZS9_HUMAN (SEQ. ID NO:211), which also corresponds to amino acids 368 - 591 of C03950_3_P19 (SEQ. ID NO:221), and a fifth amino acid sequence being at least 70%, optionally at least 80%, preferably at least 85%, more preferably at least 90% and most preferably at least 95% homologous to a polypeptide having the sequence AS corresponding to amino acids 592 - 593 of C03950_3_P19 (SEQ. ID NO:221), wherein said first amino acid sequence, second amino acid sequence, bridging amino acid, third amino acid sequence, bridging amino acid, fourth amino acid sequence and fifth amino acid sequence are contiguous and in a sequential order.

3. Comparison report between C03950_3_P19 (SEQ. ID NO:221) and NP_057062 (SEQ. ID NO:210):

A. An isolated chimeric polypeptide encoding for C03950_3_P19 (SEQ. ID NO:221), comprising a first amino acid sequence being at least 90% homologous to MGNYKSRPTQTCTDEWKKKVSESYVITIERLEDDL-QIKEKELTELRNIFGSDEAFSKVNLNYR TENGLSLLHLCCICGGKKSHIRTLMLKGLRPSRLTRNGFTALH-LAVYKDNAELITSLLHSGADI QQVGYGGLTALHIATIAGHLEAADVLLQHGANVNIQDAVFFTPLHIAAYY-GHEQVTRLLLKF GADVNVSGEVGDRPLHLASAKGFLNIAKLLMEEGSKADVNAQDNEDHVPLHFCSRF-GHHDI VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADMNLVACDPSRS SGEKDEQTCLMWAYEKGHDAIVTLLKHYKRPQDELPCNEYSQPGGDGSYVSVPSPLGKIKSM TKEKADILLLRAGLPSHFHLQLSEIEFHEIIGSGSFGKVYKGRCRNKIVAIKRYRANTYCSKSDV DMFCREVSILCQLNHPCVIQFVGACLNDPSQFAIVTQYISGGSLFSLLHEQKRILDLQSKLIIAV DVAKGMEYLHNLTQPIIHRDLN corresponding to amino acids 1 - 590 of NP_057062 (SEQ. ID NO:210), which also corresponds to amino acids 1 - 590 of C03950_3_P19 (SEQ. ID NO:221), and a second amino acid sequence being at least 70%, optionally at least 80%, preferably at least 85%, more preferably at least 90% and most preferably at least 95% homologous to a polypeptide having the sequence RAS (SEQ. ID NO: 370) corresponding to amino acids 591 - 593 of C03950_3_P19 (SEQ. ID NO:221), wherein said first amino acid sequence and second amino acid sequence are contiguous and in a sequential order.

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 105:

*Table 105 - InterPro domain(s)*

| Domain description | Analysis type | Position(s) on protein |
|---|---|---|
| Protein kinase | BlastProDom | 463-590 |
| Ankyrin | FPrintScan | 167-179, 352-364 |
| Ankyrin | HMMPfam | 66-99, 100-132, 133-165, 166-198, 199-233, 234-264, 269-302, 304-338, 339-371, 381-413 |
| Tyrosine protein kinase | HMMSmart | 463-593 |

(continued)

| Domain description | Analysis type | Position(s) on protein |
|---|---|---|
| Serine | HMMSmart | 463-592 |
| Ankyrin | HMMSmart | 66-96, 100-129, 133-162, 166-195, 199-230, 234-265, 269-300, 304-335, 339-368, 381-410 |
| Protein kinase | ProfileScan | 463-593 |
| Ankyrin | ProfileScan | 100-132, 133-165, 169-198, 199-231, 269-301, 339-371 |
| Ankyrin | ProfileScan | 58-401 |
| Protein kinase | ScanRegExp | 469-490 |

Variant protein C03950_3_P19 (SEQ. ID NO:221) is encoded by the following transcript(s): C03950_3_T17 (SEQ. ID NO:165), for which the coding portion starts at position 389 and ends at position 2167.

Variant protein C03950_3_P20 (SEQ. ID NO:222) according to the present invention is encoded by transcript C03950_3_T18 (SEQ. ID NO:166). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P20 (SEQ. ID NO:222) and TNI3K_HUMAN (SEQ. ID NO: 396):

A. An isolated chimeric polypeptide encoding for C03950_3_P20 (SEQ. ID NO:222), comprising a first amino acid sequence being at least 70%, optionally at least 80%, preferably at least 85%, more preferably at least 90% and most preferably at least 95%, homologous to a polypeptide having the sequence AVRRGLREGGA (SEQ. ID NO: 342) corresponding to amino acids 1 - 11 of C03950_3_P20 (SEQ. ID NO:222), a second amino acid sequence being at least 90% homologous to MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLMSDEWKKKVSES YVITIERLEDDLQIKEKELTELRNIFGSDEAFSKVNLNYRTENGLSLLHLCCICGGKKSHIRTLM LKGLRPSRLTRNGFTALHLAVYKDNAELITSLLHSGADIQQVGYGGLTALHIATIAGHLEAAD VLLQHGANVNIQDAVFFTPLHIAAYYGHEQVTRLLLKFGADVNVSGEVGDRPLHLASAKGFL NIAKLLMEEGSKADVNAQDNEDHVPLHFCSRFGHHDIVKYLLQSDLEVQPHWNIYGDTPLH LACYNGKFEVAKEIIQISGTESLTKENIFSETAFHSACTYGKSIDLVKFLLDQNVININHQGRDG HTGLHSACYHGHIRLVQFLLDNGADMNLVACDPSRSSGEKDEQTCLMWAYEKGHDAIVTLL KHYKRPQDELPCNEYSQPGGDGSYVSVPSPLGKIKSMTKEKADILLLRAGLPSHFHLQLSEIEF HEIIGSGSFGKVYKGRCRNKIVAIKRYRANTYCSKSDVDMFCREVSILCQLNHPCVIQFVGAC LNDPSQFAIVTQYISGGSLFSLLHEQKR corresponding to amino acids 1 - 657 of TNI3K_HUMAN (SEQ. ID NO: 396), which also corresponds to amino acids 12 - 668 of C03950_3_P20 (SEQ. ID NO:222), and a third amino acid sequence being at least 70%, optionally at least 80%, preferably at least 85%, more preferably at least 90% and most preferably at least 95% homologous to a polypeptide having the sequence YGSFVLIYPWTFRRNYSCNTSEGFPLDEPSPFEI (SEQ. ID NO: 372) corresponding to amino acids 669 - 702 of C03950_3_P20 (SEQ. ID NO:222), wherein said first amino acid sequence, second amino acid sequence and third amino acid sequence are contiguous and in a sequential order.

B. An isolated polypeptide encoding for a head of C03950_3_P20 (SEQ. ID NO:222), comprising a polypeptide being at least 70%, optionally at least about 80%, preferably at least about 85%, more preferably at least about 90% and most preferably at least about 95% homologous to the sequence AVRRGLREGGA (SEQ. ID NO: 342) of C03950_3_P20 (SEQ. ID NO:222).

C. An isolated polypeptide encoding for an edge portion of C03950_3_P20 (SEQ. ID NO:222), comprising an amino acid sequence being at least 70%, optionally at least about 80%, preferably at least about 85%, more preferably at least about 90% and most preferably at least about 95% homologous to the sequence YGSFVLIYPWTFRRNYSCNTSEGFPLDEPSPFEI (SEQ. ID NO: 372) of C03950_3_P20 (SEQ. ID NO:222).

2. Comparison report between C03950_3_P20 (SEQ. ID NO:222) and Q6MZS9_HUMAN (SEQ. ID NO:211):

A. An isolated chimeric polypeptide encoding for C03950_3_P20 (SEQ. ID NO:222), comprising a first amino acid sequence being at least 70%, optionally at least 80%, preferably at least 85%, more preferably at least 90% and most preferably at least 95%, homologous to a polypeptide having the sequence AVRRGLR (SEQ. ID NO: 344) corresponding to amino acids 1 - 7 of C03950_3_P20 (SEQ. ID NO:222), a second amino acid sequence being at least 90% homologous to EGGAMAAARDPPEVSLREATQRKLRRFSELRGKLVARGEFW-DIVAITAADEKQELAYNQQLS EKLKRKELPLGVQYHVFVDPAGAKIGNGGSTLCALQCLEKLYGDKWNS-FTILLIHSDEWKKK VSESYVITIERLEDDLQIKEKELTELRNIFGSDEAFSKVNLNYRTENGLSLLHLCCICGG-KKSHI RTLMLKGLRPSRLTRNGFTALHLAVYKDNAELITSLLHSGADIQQVGYGGLTALHIATIAGHL EAADVLLQHGANVNIQDAVFFTPLHIAAYYGHEQVTRLLLKFGADVNVSGEVGDRPLHLAS AKGFLNIAKLLMEEGSKADVNAQDNEDHVPLHFCSRFGHHD corresponding to amino acids 14 - 367 of Q6MZS9_HUMAN (SEQ. ID NO:211), which also corresponds to amino acids 8 - 361 of C03950_3_P20 (SEQ. ID NO:222), a bridging amino acid I corresponding to amino acid 362 of C03950_3_P20 (SEQ. ID NO:222), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 363 - 478 of C03950_3_P20 (SEQ. ID NO:222), a bridging amino acid N corresponding to amino acid 479 of C03950_3_P20 (SEQ. ID NO:222), a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKR corresponding to amino acids 486 - 674 of Q6MZS9_HUMAN (SEQ. ID NO:211), which also corresponds to amino acids 480 - 668 of C03950_3_P20 (SEQ. ID NO:222), and a fifth amino acid sequence being at least 70%, optionally at least 80%, preferably at least 85%, more preferably at least 90% and most preferably at least 95% homologous to a polypeptide having the sequence YGSFVLIYPWTFRRNYSCNTSEGFPLDEPSPFEI (SEQ. ID NO: 372) corresponding to amino acids 669 - 702 of C03950_3_P20 (SEQ. ID NO:222), wherein said first amino acid sequence, second amino acid sequence, bridging amino acid, third amino acid sequence, bridging amino acid, fourth amino acid sequence and fifth amino acid sequence are contiguous and in a sequential order.

B. An isolated polypeptide encoding for a head of C03950_3_P20 (SEQ. ID NO:222), comprising a polypeptide being at least 70%, optionally at least about 80%, preferably at least about 85%, more preferably at least about 90% and most preferably at least about 95% homologous to the sequence AVRRGLR (SEQ. ID NO: 344) of C03950_3_P20 (SEQ. ID NO:222).

3. Comparison report between C03950_3_P20 (SEQ. ID NO:222) and NP_057062 (SEQ. ID NO:210):

A. An isolated chimeric polypeptide encoding for C03950_3_P20 (SEQ. ID NO:222), comprising a first amino acid sequence being at least 70%, optionally at least 80%, preferably at least 85%, more preferably at least 90% and most preferably at least 95%, homologous to a polypeptide having the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) corresponding to amino acids 1 - 125 of C03950_3_P20 (SEQ. ID NO:222), a second amino acid sequence being at least 90% homologous to DEWKKKVSESYVITIERLEDDLQIKEKELTELRNIFGSDEAFSKVNL-NYRTENGLSLLHLCCIC GGKKSHIRTLMLKGLRPSRLTRNGFTALHLAVYKDNAELITSLLHSGADIQQVGYG-GLTALHI ATIAGHLEAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKR corresponding to amino acids 14 - 556 of NP_057062 (SEQ. ID NO:210), which also corresponds to amino acids 126 - 668 of C03950_3_P20 (SEQ. ID NO:222), and a third amino acid sequence being at least 70%, optionally at least 80%, preferably at least 85%, more preferably at least 90% and most preferably at least 95% homologous to a polypeptide having the sequence YGSFVLIYPWTFRRNYSCNTSEGFPLDEPSPFEI (SEQ. ID NO: 372) corresponding to amino acids 669 - 702 of C03950_3_P20 (SEQ. ID NO:222), wherein said first amino acid sequence, second amino acid sequence and third amino acid sequence are contiguous and in a sequential order.

B. An isolated polypeptide encoding for a head of C03950_3_P20 (SEQ. ID NO:222), comprising a polypeptide being at least 70%, optionally at least about 80%, preferably at least about 85%, more preferably at least about 90% and most preferably at least about 95% homologous to the sequence AVRRGLREGGAMAAARDPPEVSLREATQRKLRRFSELRGKLVARGEFWDIVAITAADEKQEL AYNQQLSEKLKRKELPLGVQYHVFVDPAGAKIGNGGSTLCALQCLEKLYGDKWNSFTILLIH S (SEQ. ID NO: 343) of C03950_3_P20 (SEQ. ID NO:222).

The localization of the variant protein was determined according to results from a number of different software programs and analyses, including analyses from SignalP and other specialized programs. The variant protein is believed to be located intracellularly.

Variant protein C03950_3_P20 (SEQ. ID NO:222) also has the following non-silent SNPs (Single Nucleotide Polymorphisms) as listed in Table 106, (given according to their position(s) on the amino acid sequence, with the alternative amino acid(s) listed.

*Table 106 - Amino acid mutations*

| SNP position(s) on amino acid sequence | Alternative amino acid(s) |
|---|---|
| 119 | T -> P |

The variant protein has the following domains, as determined by using InterPro. The domains are described in Table 107:

*Table 107 - InterPro domain(s)*

| Domain description | Analysis type | Position(s) on protein |
|---|---|---|
| Protein kinase | BlastProDom | 575-668 |
| Ankyrin | FPrintScan | 279-291, 464-476 |
| Ankyrin | HMMPfam | 178-211, 212-244, 245-277, 278-310, 311-345, 346-376, 381-414, 416-450, 451-483, 493-525 |
| Tyrosine protein kinase | HMMSmart | 575-702 |
| Serine | HMMSmart | 575-700 |
| Ankyrin | HMMSmart | 178-208, 212-241, 245-274, 278-307, 311-342, 346-377, 381-412, 416-447, 451-480, 493-522 |
| Protein kinase | ProfileScan | 575-702 |
| Ankyrin | ProfileScan | 212-244, 245-277, 281-310, 311-343, 381-413, 451-483 |
| Ankyrin | ProfileScan | 170-513 |
| Protein kinase | ScanRegExp | 581-602 |

Variant protein C03950_3_P20 (SEQ. ID NO:222) is encoded by the following transcript(s): C03950_3_T18 (SEQ. ID NO:166), for which the coding portion starts at position 3 and ends at position 2108. The transcript also has the following SNPs as listed in Table 108 (given according to their position on the nucleotide sequence, with the alternative nucleic acid listed.

*Table 108 - Nucleic acid SNPs*

| SNP position(s) on nucleotide sequence | Alternative nucleic acid(s) |
|---|---|
| 263 | A -> G |
| 357 | A -> C |

Variant protein C03950_3_P21 (SEQ. ID NO:223) according to the present invention is encoded by transcript C03950_3_T19 (SEQ. ID NO:167). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P21 (SEQ. ID NO:223) and TNI3K_HUMAN (SEQ. ID NO: 396):

A. An isolated chimeric polypeptide encoding for C03950_3_P21 (SEQ. ID NO:223), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P21 (SEQ. ID NO:223), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHS-GADIQQVGYGGLTALHI ATIAGHLEAADVLLQHGANVNIQDAVFFTPLHIAAYYGHEQVTRLLLKFGADVNVS-GEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKR corresponding to amino acids 115 - 657 of TNI3K_HUMAN (SEQ. ID NO: 396), which also corresponds to amino acids 14 - 556 of C03950_3_P21 (SEQ. ID NO:223), and a third amino acid sequence being at least 70%, optionally at least 80%, preferably at least 85%, more preferably at least 90% and most preferably at least 95% homologous to a polypeptide having the sequence YGSFVLIYPWTFRRNYSCNTSEGFPLDEPSPFEI (SEQ. ID NO: 372) corresponding to amino acids 557 - 590 of C03950_3_P21 (SEQ. ID NO:223), wherein said first amino acid sequence, second amino acid sequence and third amino acid sequence are contiguous and in a sequential order.

B. An isolated polypeptide encoding for a head of C03950_3_P21 (SEQ. ID NO:223), comprising a polypeptide being at least 70%, optionally at least about 80%, preferably at least about 85%, more preferably at least about 90% and most preferably at least about 95% homologous to the sequence MGNYKSRPTQTCT (SEQ. ID NO: 346) of C03950_3_P21 (SEQ. ID NO:223).

C. An isolated polypeptide encoding for an edge portion of C03950_3_P21 (SEQ. ID NO:223), comprising an amino acid sequence being at least 70%, optionally at least about 80%, preferably at least about 85%, more preferably at least about 90% and most preferably at least about 95% homologous to the sequence YGSFVLIYPWTFRRNYSCNTSEGFPLDEPSPFEI (SEQ. ID NO: 372) of C03950_3_P21 (SEQ. ID NO:223).

2. Comparison report between C03950_3_P21 (SEQ. ID NO:223) and Q6MZS9_HUMAN (SEQ. ID NO:211):

A. An isolated chimeric polypeptide encoding for C03950_3_P21 (SEQ. ID NO:223), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P21 (SEQ. ID NO:223), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHS-GADIQQVGYGGLTALHI ATIAGHLEAADVLLQHGANVNIQDAVFFTPLHIAAYYGHEQVTRLLLKFGADVNVS-GEVGDR PLHLASAKGFLNIAKLLMEEGSKADVNAQDNEDHVPLHFCSRFGHHD corresponding to amino acids 132 - 367 of Q6MZS9_HUMAN (SEQ. ID NO:211), which also corresponds to amino acids 14 - 249 of C03950_3_P21 (SEQ. ID NO:223), a bridging amino acid I corresponding to amino acid 250 of C03950_3_P21 (SEQ. ID NO:223), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 251 - 366 of C03950_3_P21 (SEQ. ID NO:223), a bridging amino acid N corresponding to amino acid 367 of C03950_3_P21 (SEQ. ID NO:223), a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKR corresponding to amino acids 486 - 674 of Q6MZS9_HUMAN (SEQ. ID NO:211), which also corresponds to amino acids 368 - 556 of C03950_3_P21 (SEQ. ID NO:223), and a fifth amino acid sequence being at least 70%, optionally at least 80%, preferably at least 85%, more preferably at least 90% and most preferably at least 95% homologous to a polypeptide having the sequence YGSFVLIYPWTFRRNYSCNTSEGFPLDEPSPFEI (SEQ. ID NO: 372) corresponding to amino acids 557 - 590 of C03950_3_P21 (SEQ. ID NO:223), wherein said first amino acid sequence, second amino acid sequence, bridging amino acid, third amino acid sequence, bridging amino acid, fourth amino acid sequence and fifth amino acid sequence are contiguous and in a sequential order.

3. Comparison report between C03950_3_P21 (SEQ. ID NO:223) and NP_057062 (SEQ. ID NO:210):

A. An isolated chimeric polypeptide encoding for C03950_3_P21 (SEQ. ID NO:223), comprising a first amino acid sequence being at least 90% homologous to MGNYKSRPTQTCTDEWKKKVSESYVITIERLEDDL-QIKEKELTELRNIFGSDEAFSKVNLNYR TENGLSLLHLCCICGGKKSHIRTLMLKGLRPSRLTRNGFTALH-LAVYKDNAELITSLLHSGADI QQVGYGGLTALHIATIAGHLEAADVLLQHGANVNIQDAVFFTPLHIAAYY-GHEQVTRLLLKF GADVNVSGEVGDRPLHLASAKGFLNIAKLLMEEGSKADVNAQDNEDHVPLHFCSRF-GHHDI VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADMNLVACDPSRS SGEKDEQTCLMWAYEKGHDAIVTLLKHYKRPQDELPCNEYSQPGGDGSYVSVPSPLGKIKSM TKEKADILLLRAGLPSHFHLQLSEIEFHEIIGSGSFGKVYKGRCRNKIVAIKRYRANTYCSKSDV DMFCREVSILCQLNHPCVIQFVGACLNDPSQFAIVTQYISGGSLFSLLHEQKR corresponding to amino acids 1 - 556 of NP_057062 (SEQ. ID NO:210), which also corresponds to amino acids 1 - 556 of C03950_3_P21 (SEQ. ID NO:223), and a second amino acid sequence being at least 70%, optionally at least 80%, preferably at least 85%, more preferably at least 90% and most preferably at least 95% homologous to a polypeptide having the sequence YGSFVLIYPWTFRRNYSCNTSEGFPLDEPSPFEI (SEQ. ID NO: 372) corresponding to amino acids 557 - 590 of C03950_3_P21 (SEQ. ID NO:223), wherein said first amino acid sequence and second amino acid sequence are contiguous and in a sequential order.

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 109:

*Table 109 - InterPro domain(s)*

| Domain description | Analysis type | Position(s) on protein |
|---|---|---|
| Protein kinase | BlastProDom | 463-556 |
| Ankyrin | FPrintScan | 167-179, 352-364 |
| Ankyrin | HMMPfam | 66-99, 100-132, 133-165, 166-198, 199-233, 234-264, 269-302, 304-338, 339-371, 381-413 |
| Tyrosine protein kinase | HMMSmart | 463-590 |
| Serine | HMMSmart | 463-588 |
| Ankyrin | HMMSmart | 66-96, 100-129, 133-162, 166-195, 199-230, 234-265, 269-300, 304-335, 339-368, 381-410 |
| Protein kinase | ProfileScan | 463-590 |
| Ankyrin | ProfileScan | 100-132, 133-165, 169-198, 199-231, 269-301, 339-371 |
| Ankyrin | ProfileScan | 58-401 |
| Protein kinase | ScanRegExp | 469-490 |

Variant protein C03950_3_P21 (SEQ. ID NO:223) is encoded by the following transcript(s): C03950_3_T19 (SEQ. ID NO:167), for which the coding portion starts at position 389 and ends at position 2158.

Variant protein C03950_3_P23 (SEQ. ID NO:224) according to the present invention is encoded by transcript C03950_3_T21 (SEQ. ID NO:168). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P23 (SEQ. ID NO:224) and Q6MZS9_HUMAN (SEQ. ID NO:211):

A. An isolated chimeric polypeptide encoding for C03950_3_P23 (SEQ. ID NO:224), comprising a first amino acid sequence being at least 70%, optionally at least 80%, preferably at least 85%, more preferably at least 90% and most preferably at least 95%, homologous to a polypeptide having the sequence MGNYKSRPTQTCT (SEQ. ID NO: 346) corresponding to amino acids 1 - 13 of C03950_3_P23 (SEQ. ID NO:224), a second amino acid sequence being at least 90% homologous to DEWKKKVSESYVITIERLEDDLQIKEKELTELRNIFGSDEAF-SKVNLNYRTENGLSLLHLCCIC GGKKSHIRTLMLKGLRPSRLTRNGFTALHLAVYKDNAELITSLLHS-GADIQQVGYGGLTALHI ATIAGHLEAADVLLQHGANVNIQDAVFFTPLHIAAYYGHEQVTRLLLKFGADVNVS-GEVGDR PLHLASAKGFLNIAKLLMEEGSKADVNAQDNEDHVPLHFCSRFGHHD corresponding to amino acids 132 - 367 of Q6MZS9_HUMAN (SEQ. ID NO:211), which also corresponds to amino acids 14 - 249 of C03950_3_P23 (SEQ. ID NO:224), a bridging amino acid I corresponding to amino acid 250 of C03950_3_P23 (SEQ. ID NO:224), a third amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 251 - 366 of C03950_3_P23 (SEQ. ID NO:224), a bridging amino acid N corresponding to amino acid 367 of C03950_3_P23 (SEQ. ID NO:224), a fourth amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSG corresponding to amino acids 486 - 590 of Q6MZS9_HUMAN (SEQ. ID NO:211), which also corresponds to amino acids 368 - 472 of C03950_3_P23 (SEQ. ID NO:224), and a fifth amino acid sequence being at least 70%, optionally at least 80%, preferably at least 85%, more preferably at least 90% and most preferably at least 95% homologous to a polypeptide having the sequence NLK (SEQ. ID NO: 378) corresponding to amino acids 473 - 475 of C03950_3_P23 (SEQ. ID NO:224), wherein said first amino acid sequence, second amino acid sequence, bridging amino acid, third amino acid sequence, bridging amino acid, fourth amino acid sequence and fifth amino acid sequence are contiguous and in a sequential order.

B. An isolated polypeptide encoding for a head of C03950_3_P23 (SEQ. ID NO:224), comprising a polypeptide being at least 70%, optionally at least about 80%, preferably at least about 85%, more preferably at least about 90% and most preferably at least about 95% homologous to the sequence MGNYKSRPTQTCT (SEQ. ID NO: 346) of C03950_3_P23 (SEQ. ID NO:224).

C. An isolated polypeptide encoding for an edge portion of C03950_3_P23 (SEQ. ID NO:224), comprising an amino acid sequence being at least 70%, optionally at least about 80%, preferably at least about 85%, more preferably at least about 90% and most preferably at least about 95% homologous to the sequence NLK (SEQ. ID NO: 378) of C03950_3_P23 (SEQ. ID NO:224).

2. Comparison report between C03950_3_P23 (SEQ. ID NO:224) and NP_057062 (SEQ. ID NO:210):

A. An isolated chimeric polypeptide encoding for C03950_3_P23 (SEQ. ID NO:224), comprising a first amino acid sequence being at least 90% homologous to MGNYKSRPTQTCTDEWKKKVSESYVITIERLEDDL-QIKEKELTELRNIFGSDEAFSKVNLNYR TENGLSLLHLCCICGGKKSHIRTLMLKGLRPSRLTRNGFTALH-LAVYKDNAELITSLLHSGADI QQVGYGGLTALHIATIAGHLEAADVLLQHGANVNIQDAVFFTPLHIAAYY-GHEQVTRLLLKF GADVNVSGEVGDRPLHLASAKGFLNIAKLLMEEGSKADVNAQDNEDHVPLHFCSRF-GHHDI VKYLLQSDLEVQPHVVNIYGDTPLHLACYNGKFEVAKEIIQISGTESLTKENIFSETAFHSACT YGK-SIDLVKFLLDQNVININHQGRDGHTGLHSACYHGHIRLVQFLLDNGADMNLVACDPSRS SGEKDEQTCLMWAYEKGHDAIVTLLKHYKRPQDELPCNEYSQPGGDGSYVSVPSPLGKIKSM TKEKADIL-LLRAGLPSHFHLQLSEIEFHEIIGSG corresponding to amino acids 1 - 472 of NP_057062 (SEQ. ID NO:210), which also corresponds to amino acids 1 - 472 of C03950_3_P23 (SEQ. ID NO:224), and a second amino acid sequence being at least 70%, optionally at least 80%, preferably at least 85%, more preferably at least 90% and most preferably at least 95% homologous to a polypeptide having the sequence NLK (SEQ. ID NO: 378) corresponding to amino acids 473 - 475 of C03950_3_P23 (SEQ. ID NO:224), wherein said first amino acid sequence and second amino acid sequence are contiguous and in a sequential order.

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 110:

*Table 110 - InterPro domain(s)*

| Domain description | Analysis type | Position(s) on protein |
|---|---|---|
| Ankyrin | FPrintScan | 167-179, 352-364 |

(continued)

| Domain description | Analysis type | Position(s) on protein |
|---|---|---|
| Ankyrin | HMMPfam | 66-99, 100-132, 133-165, 166-198, 199-233, 234-264, 269-302, 304-338, 339-371, 381-413 |
| Ankyrin | HMMSmart | 66-96, 100-129, 133-162, 166-195, 199-230, 234-265, 269-300, 304-335, 339-368, 381-410 |
| Ankyrin | ProfileScan | 100-132, 133-165, 169-198, 199-231, 269-301, 339-371 |
| Ankyrin | ProfileScan | 58-401 |

Variant protein C03950_3_P23 (SEQ. ID NO:224) is encoded by the following transcript(s): C03950_3_T21 (SEQ. ID NO:168), for which the coding portion starts at position 389 and ends at position 1813.

Variant protein C03950_3_P24 (SEQ. ID NO:225) according to the present invention is encoded by transcript C03950_3_T22 (SEQ. ID NO:169).

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 111:

*Table 111 - InterPro domain(s)*

| Domain description | Analysis type | Position(s) on protein |
|---|---|---|
| Protein kinase | BlastProDom | 46-84 |
| Protein kinase | ProfileScan | 1-142 |

Variant protein C03950_3_P24 (SEQ. ID NO:225) is encoded by the following transcript(s): C03950_3_T22 (SEQ. ID NO:169), for which the coding portion starts at position 457 and ends at position 1218.

Variant protein C03950_3_P25 (SEQ. ID NO:226) according to the present invention is encoded by transcript C03950_3_T23 (SEQ. ID NO:170).

The localization of the variant protein was determined according to results from a number of different software programs and analyses, including analyses from SignalP and other specialized programs. The variant protein is believed to be located intracellularly.

The variant protein has the following domains, as determined by using InterPro. The domains are described in Table 112:

*Table 112 - InterPro domain(s)*

| Domain description | Analysis type | Position(s) on protein |
|---|---|---|
| Protein kinase | BlastProDom | 46-84 |
| Protein kinase | ProfileScan | 1-142 |

Variant protein C03950_3_P25 (SEQ. ID NO:226) is encoded by the following transcript(s): C03950_3_T23 (SEQ. ID NO:170), for which the coding portion starts at position 457 and ends at position 1155.

Variant protein C03950_3_P28 (SEQ. ID NO:227) according to the present invention is encoded by transcript C03950_3_T2 (SEQ. ID NO:156). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P28 (SEQ. ID NO:227) and TNI3K_HUMAN (SEQ. ID NO: 396):

A. An isolated chimeric polypeptide encoding for C03950_3_P28 (SEQ. ID NO:227), comprising a first amino acid sequence being at least 90% homologous to MAAARDPPEVSLREATQRKLRRFSELRGKLVARGEFWDI-

VAITAADEKQELAYNQQLSEKLK RKELPLGVQYHVFVDPAGAKIGNGGSTLCALQCLEKLYGDKWNSFTIL-LIHSDEWKKKVSES YVITIERLEDDLQIKEKELTELRNIFGSDEAFSKVNLNYRTENGLSLLHLCCICGGKK-SHIRTLM LKGLRPSRLTRNGFTALHLAVYKDNAELITSLLHSGADIQQVGYGGLTALHIATIAGHLEAAD VLL-QHGANVNIQDAVFFTPLHIAAYYGHEQVTRLLLKFGADVNVSGEVGDRPLHLASAKGFL NIAKLLMEEGSKADVNAQDNEDHVPLHFCSRFGHHDIVKYLLQSDLEVQPHVUMYGDTPLH LACYNGKFEVAKEIIQISGTESLTKENIFSETAFHSACTYGKSIDLVKFLLDQNVININHQGRDG HTGLHSACYHGHIRLVQFLLDNGADMNLVACDPSRSSGEKDEQTCLMWAYEKGHDAIVTLL KHYKRPQDELPCNEYSQPGGDGSYVSVPSPLGKIKSMTKEKADILLLRAGLPSHFHLQLSEIEF HEIIGSGSFGKVYKGRCRNKIVAIKRYRANTYCSKSDVDMFCREVSILCQLNHPCVIQFVGAC LNDPSQFAIVTQYISGGSLFSLLHEQKRILDLQSKLIIAVDVAKGMEYLHNLTQPIIHRDLN corresponding to amino acids 1 - 691 of TNI3K_HUMAN (SEQ. ID NO: 396), which also corresponds to amino acids 1 - 691 of C03950_3_P28 (SEQ. ID NO:227), a second amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 692 - 731 of C03950_3_P28 (SEQ. ID NO:227), and a third amino acid sequence being at least 90% homologous to ESRFLQSLDEDNMTKQPGNLRWMAPEVFTQCTRYTIKADVFSYALCLWEILTGEIP-FAHLKPA AAAADMAYHHIRPPIGYSIPKPISSLLIRGWNACPEGRPEFSEWMKLEECLCNIELMSPASSNS SGSLSPSSSSDCLVNRGGPGRSHVAALRSRFELEYALNARSYAALSQSAGQYSSQGLSLEEMK RSLQYT-PIDKYGYVSDPMSSMHFHSCRNSSSFEDSS corresponding to amino acids 710 - 936 of TNI3K_HUMAN (SEQ. ID NO: 396), which also corresponds to amino acids 732 - 958 of C03950_3_P28 (SEQ. ID NO:227), wherein said first amino acid sequence, second amino acid sequence and third amino acid sequence are contiguous and in a sequential order.

2. Comparison report between C03950_3_P28 (SEQ. ID NO:227) and NP_057062 (SEQ. ID NO:210):

A. An isolated chimeric polypeptide encoding for C03950_3_P28 (SEQ. ID NO:227), comprising a first amino acid sequence being at least 70%, optionally at least 80%, preferably at least 85%, more preferably at least 90% and most preferably at least 95%, homologous to a polypeptide having the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) corresponding to amino acids 1 - 114 of C03950_3_P28 (SEQ. ID NO:227), a second amino acid sequence being at least 90% homologous to DEWKKKVSESYVITIERLEDDLQIKEKELTELRNIFGSDEAFSKVNLNYRTENGLSLLHL-CCIC GGKKSHIRTLMLKGLRPSRLTRNGFTALHLAVYKDNAELITSLLHSGADIQQVGYGGLTALHI ATIAG-HLEAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLN corresponding to amino acids 14 - 590 of NP_057062 (SEQ. ID NO:210), which also corresponds to amino acids 115 - 691 of C03950_3_P28 (SEQ. ID NO:227), a third amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 692 - 731 of C03950_3_P28 (SEQ. ID NO:227), and a fourth amino acid sequence being at least 90% homologous to ESRFLQSLDEDNMTKQPGNLRWMAPEVFTQCTRYTIKADVFSYALCLWEILT-GEIPFAHLKPA AAAADMAYHHIRPPIGYSIPKPISSLLIRGWNACPEGRPEFSEWMKLEECLCNIELMSPASS-NS SGSLSPSSSSDCLVNRGGPGRSHVAALRSRFELEYALNARSYAALSQSAGQYSSQGLSLEEMK RSLQ-YTPIDKYGYVSDPMSSMHFHSCRNSSSFEDSS corresponding to amino acids 609 - 835 of NP_057062 (SEQ. ID NO:210), which also corresponds to amino acids 732 - 958 of C03950_3_P28 (SEQ. ID NO:227), wherein said first amino acid sequence, second amino acid sequence, third amino acid sequence and fourth amino acid sequence are contiguous and in a sequential order.

B. An isolated polypeptide encoding for a head of C03950_3_P28 (SEQ. ID NO:227), comprising a polypeptide being at least 70%, optionally at least about 80%, preferably at least about 85%, more preferably at least about 90% and most preferably at least about 95% homologous to the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) of C03950_3_P28 (SEQ. ID NO:227).

3. Comparison report between C03950_3_P28 (SEQ. ID NO:227) and Q9Y2V6_HUMAN (SEQ. ID NO:210):

A. An isolated chimeric polypeptide encoding for C03950_3_P28 (SEQ. ID NO:227), comprising a first amino acid sequence being at least 70%, optionally at least 80%, preferably at least 85%, more preferably at least 90% and most preferably at least 95%, homologous to a polypeptide having the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) corresponding to amino acids 1 - 114 of C03950_3_P28 (SEQ. ID NO:227), a second amino acid sequence being at least 90% homologous to DEWKKKVSESYVITIERLEDDLQIKEKELTELRNIFGSDEAFSKVNLNYRTENGLSLLHL-CCIC GGKKSHIRTLMLKGLRPSRLTRNGFTALHLAVYKDNAELITSLLHSGADIQQVGYGGLTALHI ATIAGH-LEAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLN corresponding to amino acids 14 - 590 of Q9Y2V6_HUMAN (SEQ. ID NO:210), which also corresponds to amino acids 115 - 691 of C03950_3_P28 (SEQ. ID NO:227), a third amino acid sequence being at least 70%, optionally at least 80%, preferably at least 85%, more preferably at least 90% and most preferably at least 95%, homologous to a polypeptide having the sequence RSAITSRIWITHSICIWRGAHYFNREECNFRCMLTSAILK corresponding to amino acids 692 - 731 of C03950_3_P28 (SEQ. ID NO:227), and a fourth amino acid sequence being at least 90% homologous to ESRFLQSLDEDNMTKQPGNLRWMAPEVFTQCTRYTIKADVFSYALCL-WEILTGEIPFAHLKPA AAAADMAYHHIRPPIGYSIPKPISSLLIRGWNACPEGRPEFSEWMKLEECLCNIELM-SPASSNS SGSLSPSSSSDCLVNRGGPGRSHVAALRSRFELEYALNARSYAALSQSAGQYSSQGLSLEEMK RSLQYTPIDKYGYVSDPMSSMHFHSCRNSSSFEDSS corresponding to amino acids 609 - 835 of Q9Y2V6_HUMAN (SEQ. ID NO:210), which also corresponds to amino acids 732 - 958 of C03950_3_P28 (SEQ. ID NO:227), wherein said first amino acid sequence, second amino acid sequence, third amino acid sequence and fourth amino acid sequence are contiguous and in a sequential order.

4. Comparison report between C03950_3_P28 (SEQ. ID NO:227) and Q6MZS9_HUMAN (SEQ. ID NO:211):

A. An isolated chimeric polypeptide encoding for C03950_3_P28 (SEQ. ID NO:227), comprising a first amino acid sequence being at least 90% homologous to MAAARDPPEVSLREATQRKLRRFSELRGKLVARGEFWDI-VAITAADEKQELAYNQQLSEKLK RKELPLGVQYHVFVDPAGAKIGNGGSTLCALQCLEKLYGDKWNSFTIL-LIHSDEWKKKVSES YVITIERLEDDLQIKEKELTELRNIFGSDEAFSKVNLNYRTENGLSLLHLCCICGGKK-SHIRTLM LKGLRPSRLTRNGFTALHLAVYKDNAELITSLLHSGADIQQVGYGGLTALHIATIAGHLEAAD VLLQ-HGANVNIQDAVFFTPLHIAAYYGHEQVTRLLLKFGADVNVSGEVGDRPLHLASAKGFL NIAKLLMEEGSKAD-VNAQDNEDHVPLHFCSRFGHHD corresponding to amino acids 18 - 367 of Q6MZS9_HUMAN (SEQ. ID NO: 211), which also corresponds to amino acids 1 - 350 of C03950_3_P28 (SEQ. ID NO:227), a bridging amino acid I corresponding to amino acid 351 of C03950_3_P28 (SEQ. ID NO:227), a second amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 352 - 467 of C03950_3_P28 (SEQ. ID NO:227), a bridging amino acid N corresponding to amino acid 468 of C03950_3_P28 (SEQ. ID NO:227), a third amino acid sequence being at least 90% homologous to LVACDPSRSSGEKDEQTCLMWAYEKGHDAIVTLLKHYKRPQDELPCNEYSQPGGDGSYVSV PSPLG-KIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIA-VDVAKGMEYLHNLTQPIIHRDLNR corresponding to amino acids 486 - 709 of Q6MZS9_HUMAN (SEQ. ID NO:211), which also corresponds to amino acids 469 - 692 of C03950_3_P28 (SEQ. ID NO:227), and a fourth amino acid sequence being at least 70%, optionally at least 80%, preferably at least 85%, more preferably at least 90% and most preferably at least 95% homologous to a polypeptide having the sequence SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEWMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) corresponding to amino acids 693 - 958 of C03950_3_P28 (SEQ. ID NO:227), wherein said , first amino acid sequence, bridging amino acid, second amino acid sequence, bridging amino acid, third amino acid sequence and fourth amino acid sequence are contiguous and in a sequential order.

B. An isolated polypeptide encoding for an edge portion of C03950_3_P28 (SEQ. ID NO:227), comprising an amino acid sequence being at least 70%, optionally at least about 80%, preferably at least about 85%, more preferably at least about 90% and most preferably at least about 95% homologous to the sequence SAITSRIWITHSICIWRGAHYFNREECNFRCMLTSAILKESRFLQSLDEDNMTKQPGNLRWMA PEVFTQCTRYTIKADVFSYALCLWEILTGEIPFAHLKPAAAAADMAYHHIRPPIGYSIPKPISSL LIRGWNACPEGRPEFSEWMKLEECLCNIELMSPASSNSSGSLSPSSSSDCLVNRGGPGRSHVA ALRSRFELEYALNARSYAALSQSAGQYSSQGLSLEEMKRSLQYTPIDKYGYVSDPMSSMHFH SCRNSSSFEDSS (SEQ. ID NO: 345) of C03950_3_P28 (SEQ. ID NO:227).

The localization of the variant protein was determined according to results from a number of different software programs and analyses, including analyses from SignalP and other specialized programs. The variant protein is believed to be located intracellularly.

Variant protein C03950_3_P28 (SEQ. ID NO:227) also has the following non-silent SNPs (Single Nucleotide Polymorphisms) as listed in Table 113, (given according to their position(s) on the amino acid sequence, with the alternative amino acid(s) listed.

*Table 113 - Amino acid mutations*

| SNP position(s) on amino acid sequence | Alternative amino acid(s) |
|---|---|
| 108 | T -> P |

The variant protein has the following domains, as determined by using InterPro. The domains are described in Table 114:

*Table 114 - InterPro domain(s)*

| Domain description | Analysis type | Position(s) on protein |
|---|---|---|
| Protein kinase | BlastProDom | 564-788 |
| Ankyrin | FPrintScan | 268-280, 453-465 |
| Ankyrin | HMMPfam | 167-200, 201-233, 234-266, 267-299, 300-334, 335-365, 370-403, 405-439, 440-472, 482-514 |
| Protein kinase | HMMPfam | 564-842 |
| Tyrosine protein kinase | HMMSmart | 564-842 |
| Serine | HMMSmart | 564-846 |
| Ankyrin | HMMSmart | 167-197, 201-230, 234-263, 267-296, 300-331, 335-366, 370-401, 405-436, 440-469, 482-511 |
| Protein kinase | ProfileScan | 564-846 |
| Ankyrin | ProfileScan | 201-233, 234-266, 270-299, 300-332, 370-402, 440-472 |
| Ankyrin | ProfileScan | 159-502 |
| Protein kinase | ScanRegExp | 570-591 |

Variant protein C03950_3_P28 (SEQ. ID NO:227) is encoded by the following transcript(s): C03950_3_T2 (SEQ. ID NO:156), for which the coding portion starts at position 36 and ends at position 2909. The transcript also has the following SNPs as listed in Table 115 (given according to their position on the nucleotide sequence, with the alternative nucleic acid listed.

*Table 115 - Nucleic acid SNPs*

| SNP position(s) on nucleotide sequence | Alternative nucleic acid(s) |
|---|---|
| 263 | A -> G |
| 357 | A -> C |

Variant protein C03950_3_P31 (SEQ. ID NO:228) according to the present invention is encoded by transcript C03950_3_T10 (SEQ. ID NO:161). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P31 (SEQ. ID NO:228) and TNI3K_HUMAN (SEQ. ID NO: 396):

A. An isolated chimeric polypeptide encoding for C03950_3_P31 (SEQ. ID NO:228), comprising a first amino acid sequence being at least 90% homologous to MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHSDEWKKKVSES YVITIERLEDDLQIKEKELTELRNIFGSDEAFSKVNLNYRTENGLSLLHLCCICGGKKSHIRTLM LKGLRPSRLTRNGFTALHLAVYKDNAELITSLLHSGADIQQVGYGGLTALHIATIAGHLEAAD VLLQHGANVNIQDAVFFTPLHIAAYYGHEQVTRLLLKFGADVNVSGEVGDRPLHLASAKGFL NIAKLLMEEGSKADVNAQDNEDHVPLHFCSRFGHHDIVKYLLQSDLEVQPHWNIYGDTPLH LACYNGKFEVAKEIIQISGTESLTKENIFSETAFHSACTYGKSIDLVKFLLDQNVININHQGRDG HTGLHSACYHGHIRLVQFLLDNGADMNLVACDPSRSSGEKDEQTCLMWAYEKGHDAIVTLL KHYKRPQDELPCNEYSQPGGDGSYVSVPSPLGKIKSMTKEKADILLLRAGLPSHFHLQLSEIEF HEIIGSGSFGKVYKGRCRNKIVAIKRYRANTYCSKSDVDMFCREVSILCQLNHPCVIQFVGAC LNDPSQFAIVTQYISGGSLFSLLHEQKRILDLQSKLIIAVDVAKGMEYLHNLTQPIIHRDLNSHN ILLYEDGHAWADFGESRFLQSLDEDNMTKQPGNLRWMAPEVFTQCTRYTIKADVFSYALCL WEILTGEIPFAHLKPAAAAADMAYHHIRPPIGYSIPKPISSLLIRGWNACPE corresponding to amino acids 1 - 808 of TNI3K_HUMAN (SEQ. ID NO: 396), which also corresponds to amino acids 1 - 808 of C03950_3_P31 (SEQ. ID NO:228), and a second amino acid sequence being at least 70%, optionally at least 80%, preferably at least 85%, more preferably at least 90% and most preferably at least 95% homologous to a polypeptide having the sequence AKSRPSHYPVSSVYTETLKKKNEDRFGMWIEYLRR (SEQ. ID NO: 356) corresponding to amino acids 809 - 843 of C03950_3_P31 (SEQ. ID NO:228), wherein said first amino acid sequence and second amino acid sequence are contiguous and in a sequential order.

B. An isolated polypeptide encoding for an edge portion of C03950_3_P31 (SEQ. ID NO:228), comprising an amino acid sequence being at least 70%, optionally at least about 80%, preferably at least about 85%, more preferably at least about 90% and most preferably at least about 95% homologous to the sequence AKSRPSHYPVSSVYTETLKKKNEDRFGMWIEYLRR (SEQ. ID NO: 356) of C03950_3_P31 (SEQ. ID NO:228).

2. Comparison report between C03950_3_P31 (SEQ. ID NO:228) and Q6MZS9_HUMAN (SEQ. ID NO:211):

A. An isolated chimeric polypeptide encoding for C03950_3_P31 (SEQ. ID NO:228), comprising a first amino acid sequence being at least 90% homologous to MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHSDEWKKKVSES YVITIERLEDDLQIKEKELTELRNIFGSDEAFSKVNLNYRTENGLSLLHLCCICGGKKSHIRTLM LKGLRPSRLTRNGFTALHLAVYKDNAELITSLLHSGADIQQVGYGGLTALHIATIAGHLEAAD VLLQHGANVNIQDAVFFTPLHIAAYYGHEQVTRLLLKFGADVNVSGEVGDRPLHLASAKGFL NIAKLLMEEGSKADVNAQDNEDHVPLHFCSRFGHHD corresponding to amino acids 18 - 367 of Q6MZS9_HUMAN (SEQ. ID NO:211), which also corresponds to amino acids 1 - 350 of C03950_3_P31 (SEQ. ID NO:228), a bridging amino acid I corresponding to amino acid 351 of C03950_3_P31 (SEQ. ID NO:228), a second amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 352 - 467 of C03950_3_P31 (SEQ. ID NO:228), a bridging amino acid N corresponding to amino acid 468 of C03950_3_P31 (SEQ. ID NO:228), a third amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIAVDVAKGMEYLHNLTQPIIHRDLN corresponding to amino acids 486 - 708 of Q6MZS9_HUMAN (SEQ. ID NO: 211), which also corresponds to amino acids 469 - 691 of C03950_3_P31 (SEQ. ID NO:228), and a fourth amino acid sequence being at least 70%, optionally at least 80%, preferably at least 85%, more preferably at least 90% and most preferably at least 95% homologous to a polypeptide having the sequence SHNILLYEDGHAWADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEAKSRPSHYPV SSVYTETLKKKNEDRFGMWIEYLRR (SEQ. ID NO: 358) corresponding to amino acids 692 - 843 ofC03950_3_P31 (SEQ. ID NO:228), wherein said , first amino acid sequence, bridging amino acid, second amino acid sequence, bridging amino acid, third amino acid sequence and fourth amino acid sequence are contiguous and in a sequential order.

B. An isolated polypeptide encoding for an edge portion of C03950_3_P31 (SEQ. ID NO:228), comprising an amino acid sequence being at least 70%, optionally at least about 80%, preferably at least about 85%, more preferably at least about 90% and most preferably at least about 95% homologous to the sequence SHNILLYEDGHAWADFGESRFLQSLDEDNMTKQPGNLRWMAPEVFTQCTRYTIKADVFSYA LCLWEILTGEIPFAHLKPAAAAADMAYHHIRPPIGYSIPKPISSLLIRGWNACPEAKSRPSHYPV SSVYTETLKKKNEDRFGMWIEYLRR (SEQ. ID NO: 358) of C03950_3_P31 (SEQ. ID NO:228).

3. Comparison report between C03950_3_P31 (SEQ. ID NO:228) and NP_057062 (SEQ. ID NO:210):

A. An isolated chimeric polypeptide encoding for C03950_3_P31 (SEQ. ID NO:228), comprising a first amino acid sequence being at least 70%, optionally at least 80%, preferably at least 85%, more preferably at least 90% and most preferably at least 95%, homologous to a polypeptide having the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) corresponding to amino acids 1 - 114 of C03950_3_P31 (SEQ. ID NO:228), a second amino acid sequence being at least 90% homologous to DEWKKKVSESYVITIERLEDDLQIKEKELTELRNIFGSDEAFSKVNLNYRTENGLSLLHLCCIC GGKKSHIRTLMLKGLRPSRLTRNGFTALHLAVYKDNAELITSLLHSGADIQQVGYGGLTALHI ATIAGHLEAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLNSHNILLYEDGHAWADFGESRFLQSLDEDNMTKQPGNLRWMAPEVFTQCTRYTI KADVFSYALCLWEILTGEIPFAHLKPAAAAADMAYHHIRPPIGYSIPKPISSLLIRGWNACPE corresponding to amino acids 14 - 707 of NP_057062 (SEQ. ID NO:210), which also corresponds to amino acids 115 - 808 of C03950_3_P31 (SEQ. ID NO:228), and a third amino acid sequence being at least 70%, optionally at least 80%, preferably at least 85%, more preferably at least 90% and most preferably at least 95% homologous to a polypeptide having the sequence AKSRPSHYPVSSVYTETLKKKNEDRFGMWIEYLRR (SEQ. ID NO: 356) corresponding to amino acids 809 - 843 of C03950_3_P31 (SEQ. ID NO:228), wherein said first amino acid sequence, second amino acid sequence and third amino acid sequence are contiguous and in a sequential order.

B. An isolated polypeptide encoding for a head of C03950_3_P31 (SEQ. ID NO:228), comprising a polypeptide being at least 70%, optionally at least about 80%, preferably at least about 85%, more preferably at least about 90% and most preferably at least about 95% homologous to the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) of C03950_3_P31 (SEQ. ID NO:228).

C. An isolated polypeptide encoding for an edge portion of C03950_3_P31 (SEQ. ID NO:228), comprising an amino acid sequence being at least 70%, optionally at least about 80%, preferably at least about 85%, more preferably at least about 90% and most preferably at least about 95% homologous to the sequence AKSRPSHYPVSSVYTETLKKKNEDRFGMWIEYLRR (SEQ. ID NO: 356) of C03950_3_P31 (SEQ. ID NO:228).

The localization of the variant protein was determined according to results from a number of different software programs and analyses, including analyses from SignalP and other specialized programs. The variant protein is believed to be located intracellularly.

Variant protein C03950_3_P31 (SEQ. ID NO:228) also has the following non-silent SNPs (Single Nucleotide Polymorphisms) as listed in Table 116, (given according to their position(s) on the amino acid sequence, with the alternative amino acid(s) listed.

*Table 116 - Amino acid mutations*

| SNP position(s) on amino acid sequence | Alternative amino acid(s) |
|---|---|
| 108 | T -> P |

The variant protein has the following domains, as determined by using InterPro. The domains are described in Table 117:

*Table 117 - InterPro domain(s)*

| Domain description | Analysis type | Position(s) on protein |
|---|---|---|
| Protein kinase | BlastProDom | 564-766 |
| Ankyrin | FPrintScan | 268-280, 453-465 |
| Ankyrin | HMMPfam | 167-200, 201-233, 234-266, 267-299, 300-334, 335-365, 370-403, 405-439, 440-472, 482-514 |
| Protein kinase | HMMPfam | 564-821 |
| Tyrosine protein kinase | HMMSmart | 564-822 |
| Serine | HMMSmart | 564-823 |
| Ankyrin | HMMSmart | 167-197, 201-230, 234-263, 267-296, 300-331, 335-366, 370-401, 405-436, 440-469, 482-511 |
| Protein kinase | ProfileScan | 564-826 |
| Ankyrin | ProfileScan | 201-233, 234-266, 270-299, 300-332, 370-402, 440-472 |
| Ankyrin | ProfileScan | 159-502 |
| Protein kinase | ScanRegExp | 570-591 |

Variant protein C03950_3_P31 (SEQ. ID NO:228) is encoded by the following transcript(s): C03950_3_T10 (SEQ. ID NO:161), for which the coding portion starts at position 36 and ends at position 2564. The transcript also has the following SNPs as listed in Table 118 (given according to their position on the nucleotide sequence, with the alternative nucleic acid listed.

*Table 118 - Nucleic acid SNPs*

| SNP position(s) on nucleotide sequence | Alternative nucleic acid(s) |
|---|---|
| 263 | A -> G |
| 357 | A -> C |
| 2677 | T -> C |

Variant protein C03950_3_P33 (SEQ. ID NO:229) according to the present invention is encoded by transcript C03950_3_T13 (SEQ. ID NO:163). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P33 (SEQ. ID NO:229) and TNI3K_HUMAN (SEQ. ID NO: 396):

A. An isolated chimeric polypeptide encoding for C03950_3_P33 (SEQ. ID NO:229), comprising a first amino acid sequence being at least 90% homologous to MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHSDEWKKKVSES YVITIERLEDDLQIKEKELTELRNIFGSDEAFSKVNLNYRTENGLSLLHLCCICGGKKSHIRTLM LKGLRPSRLTRNGFTALHLAVYKDNAELITSLLHSGADIQQVGYGGLTALHIATIAGHLEAAD VLLQHGANVNIQDAVFFTPLHIAAYYGHEQVTRLLLKTGADVNVSGEVGDRPLHLASAKGFL NIAKLLMEEGSKADVNAQDNEDHVPLHFCSRFGHHDIVKYLLQSDLEVQPHVUNIYGDTPLH LACYNGKFEVAKEIIQISGTESLTKENIFSETAFHSACTYGKSIDLVKFLLDQNVININHQGRDG HTGLHSACYHGHIRLVQFLLDNGADMNLVACDPSRSSGEKDEQTCLMWAYEKGHDAIVTLL KHYKRPQDELPCNEYSQPGGDGSYVSVPSPLGKIKSMTKEKADILLLRAGLPSHFHLQLSEIEF HEIIGSGSFGKVYKGRCRNKIVAIKRYRANTYCSKSDVDMFCREVSILCQLNHPCVIQFVGAC LNDPSQFAIVTQYISGGSLFSLLHEQKRILDLQSKLIIAVDVAKGMEYLHNLTQPIIHRDLN corresponding to amino acids 1 - 691 of TNI3K_HUMAN (SEQ. ID NO: 396), which also corresponds to amino acids 1 - 691 of C03950_3_P33 (SEQ. ID NO:229), and a second amino acid sequence being at least 70%, optionally at least 80%, preferably at least 85%, more preferably at least 90% and most preferably at least 95% homologous to a polypeptide having the sequence RYFFPK (SEQ. ID NO: 364) corresponding to amino acids 692 - 697 of C03950_3_P33 (SEQ. ID NO:229), wherein said first amino acid sequence and second amino acid sequence are contiguous and in a sequential order. B. An isolated polypeptide encoding for an edge portion of C03950_3_P33 (SEQ. ID NO:229), comprising an amino acid sequence being at least 70%, optionally at least about 80%, preferably at least about 85%, more preferably at least about 90% and most preferably at least about 95% homologous to the sequence RYFFPK (SEQ. ID NO: 364) of C03950_3_P33 (SEQ. ID NO:229).

2. Comparison report between C03950_3_P33 (SEQ. ID NO:229) and Q6MZS9_HUMAN (SEQ. ID NO:211):

A. An isolated chimeric polypeptide encoding for C03950_3_P33 (SEQ. ID NO:229), comprising a first amino acid sequence being at least 90% homologous to MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHSDEWKKKVSES YVITIERLEDDLQIKEKELTELRNIFGSDEAFSKVNLNYRTENGLSLLHLCCICGGKKSHIRTLM LKGLRPSRLTRNGFTALHLAVYKDNAELITSLLHSGADIQQVGYGGLTALHIATIAGHLEAAD VLLQHGANVNIQDAVFFTPLHIAAYYGHEQVTRLLLKFGADVNVSGEVGDRPLHLASAKGFL NIAKLLMEEGSKADVNAQDNEDHVPLHFCSRFGHHD corresponding to amino acids 18 - 367 of Q6MZS9_HUMAN (SEQ. ID NO: 211), which also corresponds to amino acids 1 - 350 of C03950_3_P33 (SEQ. ID NO:229), a bridging amino acid I corresponding to amino acid 351 of C03950_3_P33 (SEQ. ID NO:229), a second amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 352 - 467 of C03950_3_P33 (SEQ. ID NO:229), a bridging amino acid N corresponding to amino acid 468 of C03950_3_P33 (SEQ. ID NO:229), and a third amino acid sequence being at least 90% homologous to LVACDPSRSSGEKDEQTCLMWAYEKGHDAIVTLLKHYKRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKRI LDLQSKLIIAVDVAKGMEYLHNLTQPIIHRDLNRYFFPK corresponding to amino acids 486 - 714 of Q6MZS9_HUMAN (SEQ. ID NO:211), which also corresponds to amino acids 469 - 697 of C03950_3_P33 (SEQ. ID NO:229), wherein said , first amino acid sequence, bridging amino acid, second amino acid sequence, bridging amino acid and third amino acid sequence are contiguous and in a sequential order.

3. Comparison report between C03950_3_P33 (SEQ. ID NO:229) and NP_057062 (SEQ. ID NO:210):

A. An isolated chimeric polypeptide encoding for C03950_3_P33 (SEQ. ID NO:229), comprising a first amino acid sequence being at least 70%, optionally at least 80%, preferably at least 85%, more preferably at least 90% and most preferably at least 95%, homologous to a polypeptide having the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) corresponding to amino acids 1 - 114 of C03950_3_P33 (SEQ. ID NO:229), a second amino acid sequence being at least

90% homologous to DEWKKKVSESYVITIERLEDDLQIKEKELTELRNIFGSDEAFSKVNLNYRTENGLSLLHL-CCIC GGKKSHIRTLMLKGLRPSRLTRNGFTALHLAVYKDNAELITSLLHSGADIQQVGYGGLTALHI ATIAG-HLEAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKRILDLQSKLIIAVDVAKGMEYLHNLT QPIIHRDLN corresponding to amino acids 14 - 590 of NP_057062 (SEQ. ID NO:210), which also corresponds to amino acids 115 - 691 of C03950_3_P33 (SEQ. ID NO:229), and a third amino acid sequence being at least 70%, optionally at least 80%, preferably at least 85%, more preferably at least 90% and most preferably at least 95% homologous to a polypeptide having the sequence RYFFPK (SEQ. ID NO: 364) corresponding to amino acids 692 - 697 of C03950_3_P33 (SEQ. ID NO:229), wherein said first amino acid sequence, second amino acid sequence and third amino acid sequence are contiguous and in a sequential order.

B. An isolated polypeptide encoding for a head of C03950_3_P33 (SEQ. ID NO:229), comprising a polypeptide being at least 70%, optionally at least about 80%, preferably at least about 85%, more preferably at least about 90% and most preferably at least about 95% homologous to the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) of C03950_3_P33 (SEQ. ID NO:229).

C. An isolated polypeptide encoding for an edge portion of C03950_3_P33 (SEQ. ID NO:229), comprising an amino acid sequence being at least 70%, optionally at least about 80%, preferably at least about 85%, more preferably at least about 90% and most preferably at least about 95% homologous to the sequence RYFFPK (SEQ. ID NO: 364) of C03950_3_P33 (SEQ. ID NO:229).

The localization of the variant protein was determined according to results from a number of different software programs and analyses, including analyses from SignalP and other specialized programs. The variant protein is believed to be located intracellularly.

Variant protein C03950_3_P33 (SEQ. ID NO:229) also has the following non-silent SNPs (Single Nucleotide Polymorphisms) as listed in Table 119, (given according to their position(s) on the amino acid sequence, with the alternative amino acid(s) listed.

*Table 119 - Amino acid mutations*

| SNP position(s) on amino acid sequence | Alternative amino acid(s) |
|---|---|
| 108 | T -> P |

The variant protein has the following domains, as determined by using InterPro. The domains are described in Table 120:

*Table 120 - InterPro domain(s)*

| Domain description | Analysis type | Position(s) on protein |
|---|---|---|
| Protein kinase | BlastProDom | 564-693 |
| Ankyrin | FPrintScan | 268-280, 453-465 |
| Ankyrin | HMMPfam | 167-200, 201-233, 234-266, 267-299, 300-334, 335-365, 370-403, 405-439, 440-472, 482-514 |
| Tyrosine protein kinase | HMMSmart | 564-697 |
| Serine | HMMSmart | 564-694 |
| Ankyrin | HMMSmart | 167-197, 201-230, 234-263, 267-296, 300-331, 335-366, 370-401, 405-436, 440-469, 482-511 |
| Protein kinase | ProfileScan | 564-697 |
| Ankyrin | ProfileScan | 201-233, 234-266, 270-299, 300-332, 370-402, 440-472 |

(continued)

| Domain description | Analysis type | Position(s) on protein |
|---|---|---|
| Ankyrin | ProfileScan | 159-502 |
| Protein kinase | ScanRegExp | 570-591 |

Variant protein C03950_3_P33 (SEQ. ID NO:229) is encoded by the following transcript(s): C03950_3_T13 (SEQ. ID NO:163), for which the coding portion starts at position 36 and ends at position 2126. The transcript also has the following SNPs as listed in Table 121 (given according to their position on the nucleotide sequence, with the alternative nucleic acid listed.

*Table 121 - Nucleic acid SNPs*

| SNP position(s) on nucleotide sequence | Alternative nucleic acid(s) |
|---|---|
| 263 | A -> G |
| 357 | A -> C |
| 7207 | C -> A |

Variant protein C03950_3_P35 (SEQ. ID NO:230) according to the present invention is encoded by transcript C03950_3_T18 (SEQ. ID NO:166). One or more alignments to one or more previously published Serine/threonine-protein kinase TNNI3K (SEQ. ID NO:209) protein sequences are given in the alignment table on the attached CD-ROM. A brief description of the relationship of the variant protein according to the present invention to each such aligned protein is as follows:

1. Comparison report between C03950_3_P35 (SEQ. ID NO:230) and TNI3K_HUMAN (SEQ. ID NO: 396):

A. An isolated chimeric polypeptide encoding for C03950_3_P35 (SEQ. ID NO:230), comprising a first amino acid sequence being at least 90% homologous to MAAARDPPEVSLREATQRKLRRFSELRGKLVARGEFWDI-VAITAADEKQELAYNQQLSEKLK RKELPLGVQYHVFVDPAGAKIGNGGSTLCALQCLEKLYGDKWNSFTILLI-HSDEWKKKVSES YVITIERLEDDLQIKEKELTELRNIFGSDEAFSKVNLNYRTENGLSLLHLCCICGGKKSHI-RTLM LKGLRPSRLTRNGFTALHLAVYKDNAELITSLLHSGADIQQVGYGGLTALHIATIAGHLEAAD VLLQ-HGANVNIQDAVFFTPLHIAAYYGHEQVTRLLLKFGADVNVSGEVGDRPLHLASAKGFL NIAKLLMEEGSKADVNAQDNEDHVPLHFCSRFGHHDIVKYLLQSDLEVQPHWNIYGDTPLH LACYNGKFEVAKEIIQISGTESLTKENIFSETAFHSACTYGKSIDLVKFLLDQNVININHQGRDG HTGLHSACYHGHIRLVQFLLDNGADMNLVACDPSRSSGEKDEQTCLMWAYEKGHDAIVTLL KHYKRPQDELPCNEYSQPGGDGSYVSVPSPLGKIKSMTKEKADILLLRAGLPSHFHLQLSEIEF HEIIGSGSFGKVYKGRCRNKIVAIKRYRANTYCSKSDVDMFCREVSILCQLNHPCVIQFVGAC LNDPSQFAIVTQYISGGSLFSLLHEQKR corresponding to amino acids 1 - 657 of TNI3K_HUMAN (SEQ. ID NO: 396), which also corresponds to amino acids 1 - 657 of C03950_3_P35 (SEQ. ID NO:230), and a second amino acid sequence being at least 70%, optionally at least 80%, preferably at least 85%, more preferably at least 90% and most preferably at least 95% homologous to a polypeptide having the sequence YGSFVLIYPWTFRRNYSCNTSEGFPLDEPSPFEI (SEQ. ID NO: 372) corresponding to amino acids 658 - 691 of C03950_3_P3 (SEQ. ID NO:230), wherein said first amino acid sequence and second amino acid sequence are contiguous and in a sequential order.

B. An isolated polypeptide encoding for an edge portion of C03950_3_P35 (SEQ. ID NO:230), comprising an amino acid sequence being at least 70%, optionally at least about 80%, preferably at least about 85%, more preferably at least about 90% and most preferably at least about 95% homologous to the sequence YGSFVLIYPWTFRRNYSCNTSEGFPLDEPSPFEI (SEQ. ID NO: 372) of C03950_3_P35 (SEQ. ID NO:230).

2. Comparison report between C03950_3_P35 (SEQ. IDNO:230) and Q6MZS9_HUMAN (SEQ. ID NO:211):

A. An isolated chimeric polypeptide encoding for C03950_3_P35 (SEQ. ID NO:230), comprising a first amino acid sequence being at least 90% homologous to MAAARDPPEVSLREATQRKLRRFSELRGKLVARGEFWDI-VAITAADEKQELAYNQQLSEKLK RKELPLGVQYHVFVDPAGAKIGNGGSTLCALQCLEKLYGDKWNSFTIL-LIHSDEWKKKVSES YVITIERLEDDLQIKEKELTELRNIFGSDEAFSKVNLNYRTENGLSLLHLCCICGGKK-SHIRTLM LKGLRPSRLTRNGFTALHLAVYKDNAELITSLLHSGADIQQVGYGGLTALHIATIAGHLEAAD VLLQ-

HGANVNIQDAVFFTPLHIAAYYGHEQVTRLLLKFGADVNVSGEVGDRPLHLASAKGFL NIAKLLMEEGSKAD-VNAQDNEDHVPLHFCSRFGHHD corresponding to amino acids 18 - 367 of Q6MZS9_HUMAN (SEQ. ID NO: 211), which also corresponds to amino acids 1 - 350 of C03950_3_P35 (SEQ. ID NO:230), a bridging amino acid I corresponding to amino acid 351 of C03950_3_P35 (SEQ. ID NO:230), a second amino acid sequence being at least 90% homologous to VKYLLQSDLEVQPHVVNIYGDTPLHLACYNGKFEVAKEIIQISGTESLTKENIFSETAFHSACT YGKSIDLVKFLLDQNVININHQGRDGHTGLHSACYHGHIRLVQFLLDNGADM corresponding to amino acids 369 - 484 of Q6MZS9_HUMAN (SEQ. ID NO:211), which also corresponds to amino acids 352 - 467 of C03950_3_P35 (SEQ. ID NO:230), a bridging amino acid N corresponding to amino acid 468 of C03950_3_P35 (SEQ. ID NO:230), a third amino acid sequence being at least 90% homologous to LVACDPSRSSGEKDEQTCLMWAYEKGHDAIVTLLKHYICRPQDELPCNEYSQPGGDGSYVSV PSPLGKIKSMTKEKADILLLRAGLPSHFHLQLSEIEFHEIIGSGSFGKVYKGRCRNKIVAIKRYR ANTYCSKSDVDMFCREVSILCQLNHPCVIQFVGACLNDPSQFAIVTQYISGGSLFSLLHEQKR corresponding to amino acids 486 - 674 of Q6MZS9_HUMAN (SEQ. ID NO:211), which also corresponds to amino acids 469 - 657 of C03950_3_P35 (SEQ. ID NO:230), and a fourth amino acid sequence being at least 70%, optionally at least 80%, preferably at least 85%, more preferably at least 90% and most preferably at least 95% homologous to a polypeptide having the sequence YGSFVLIYPWTFRRNYSCNTSEGFPLDEPSPFEI (SEQ. ID NO: 372) corresponding to amino acids 658 - 691 of C03950_3_P35 (SEQ. ID NO:230), wherein said , first amino acid sequence, bridging amino acid, second amino acid sequence, bridging amino acid, third amino acid sequence and fourth amino acid sequence are contiguous and in a sequential order.

B. An isolated polypeptide encoding for an edge portion of C03950_3_P35 (SEQ. ID NO:230), comprising an amino acid sequence being at least 70%, optionally at least about 80%, preferably at least about 85%, more preferably at least about 90% and most preferably at least about 95% homologous to the sequence YGSFVLIYPWTFRRNYSCNTSEGFPLDEPSPFEI (SEQ. ID NO: 372) of C03950_3_P35 (SEQ. ID NO:230).

3. Comparison report between C03950_3_P35 (SEQ. ID NO:230) and NP_057062 (SEQ. ID NO:210):

A. An isolated chimeric polypeptide encoding for C03950_3_P35 (SEQ. ID NO:230), comprising a first amino acid sequence being at least 70%, optionally at least 80%, preferably at least 85%, more preferably at least 90% and most preferably at least 95%, homologous to a polypeptide having the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) corresponding to amino acids 1 - 114 of C03950_3_P35 (SEQ. ID NO:230), a second amino acid sequence being at least 90% homologous to DEWKKKVSESYVITIERLEDDLQIKEKELTELRNIFGSDEAFSKVNLNYRTENGLSLLHL-CCIC GGKKSHIRTLMLKGLRPSRLTRNGFTALHLAVYKDNAELITSLLHSGADIQQVGYGGLTALHI ATIAGHL-EAADVLLQHGANVNIQDAVFFTPLHIAAYYGHEQVTRLLLKFGADVNVSGEVGDR PLHLASAKGFLNIAKLLMEEGSKADVNAQDNEDHVPLHFCSRFGHHDIVKYLLQSDLEVQPH VVNIYGDTPLHLACYNGKFEVAKEIIQISGTESLTKENIFSETAFHSACTYGKSIDLVKFLLDQN VININHQGRDGHTGLHSACYHGHIRLVQFLLDNGADMNLVACDPSRSSGEKDEQTCLMWAY EKGHDAIVTLLKHYKRPQDELPCNEYSQPGGDGSYVSVPSPLGKIKSMTKEKADILLLRAGLP SHFHLQLSEIEFHEIIGSGSFGKVYKGRCRNKIVAIKRYRANTYCSKSDVDMFCREVSILCQLN HPCVIQFVGACLNDPSQFAIVTQYISGGSLFSLLHEQKR corresponding to amino acids 14 - 556 of NP_057062 (SEQ. ID NO:210), which also corresponds to amino acids 115 - 657 of C03950_3_P35 (SEQ. ID NO:230), and a third amino acid sequence being at least 70%, optionally at least 80%, preferably at least 85%, more preferably at least 90% and most preferably at least 95% homologous to a polypeptide having the sequence YGSFVLIYPWTFRRNYSCNTSEGFPLDEPSPFEI (SEQ. ID NO: 372) corresponding to amino acids 658 - 691 of C03950_3_P35 (SEQ. ID NO:230), wherein said first amino acid sequence, second amino acid sequence and third amino acid sequence are contiguous and in a sequential order.

B. An isolated polypeptide encoding for a head of C03950_3_P35 (SEQ. ID NO:230), comprising a polypeptide being at least 70%, optionally at least about 80%, preferably at least about 85%, more preferably at least about 90% and most preferably at least about 95% homologous to the sequence MAAARDPPEVSLREATQRKLRRFSELRGKLVARGEFWDIVAITAADEKQELAYNQQLSEKLK RKELPLGVQYHVFVDPAGAKIGNGGSTLCALQCLEKLYGDKWNSFTILLIHS (SEQ. ID NO: 379) of C03950_3_P35 (SEQ. ID NO:230).

The localization of the variant protein was determined according to results from a number of different software programs and analyses, including analyses from SignalP and other specialized programs. The variant protein is believed to be located intracellularly.

Variant protein C03950_3_P35 (SEQ. ID NO:230) also has the following non-silent SNPs (Single Nucleotide Polymorphisms) as listed in Table 122, (given according to their position(s) on the amino acid sequence, with the alternative amino acid(s) listed.

*Table 122 - Amino acid mutations*

| SNP position(s) on amino acid sequence | Alternative amino acid(s) |
|---|---|
| 108 | T->P |

The variant protein has the following domains, as determined by using InterPro. The domains are described in Table 123:

*Table 123 - InterPro domain(s)*

| Domain description | Analysis type | Position(s) on protein |
|---|---|---|
| Protein kinase | BlastProDom | 564-657 |
| Ankyrin | FPrintScan | 268-280, 453-465 |
| Ankyrin | HMMPfam | 167-200, 201-233, 234-266, 267-299, 300-334, 335-365, 370-403, 405-439, 440-472, 482-514 |
| Tyrosine protein kinase | HMMSmart | 564-691 |
| Serine | HMMSmart | 564-689 |
| Ankyrin | HMMSmart | 167-197, 201-230, 234-263, 267-296, 300-331, 335-366, 370-401, 405-436, 440-469, 482-511 |
| Protein kinase | ProfileScan | 564-691 |
| Ankyrin | ProfileScan | 201-233, 234-266, 270-299, 300-332, 370-402, 440-472 |
| Ankyrin | ProfileScan | 159-502 |
| Protein kinase | ScanRegExp | 570-591 |

Variant protein C03950_3_P35 (SEQ. ID NO:230) is encoded by the following transcript(s): C03950_3_T18 (SEQ. ID NO:166), for which the coding portion starts at position 36 and ends at position 2108. The transcript also has the following SNPs as listed in Table 124 (given according to their position on the nucleotide sequence, with the alternative nucleic acid listed.

*Table 124 - Nucleic acid SNPs*

| SNP position(s) on nucleotide sequence | Alternative nucleic acid(s) |
|---|---|
| 263 | A -> G |
| 357 | A -> C |

As noted above, cluster C03950 features 38 segment(s), which were listed in Table 86 above and for which the sequence(s) are given. These segment(s) are portions of nucleic acid sequence(s) which are described herein separately because they are of particular interest. A description of several segments according to the present invention is now provided.

Segment cluster C03950_3_N44 (SEQ. ID NO:176) according to the present invention is supported by 21 libraries. The number of libraries was determined as previously described. This segment can be found in the following transcript(s): C03950_3_T10 (SEQ. ID NO:161), C03950_3_T11 (SEQ. ID NO:162), C03950_3_T13 (SEQ. ID NO:163), C03950_3_T15 (SEQ. ID NO:164), C03950_3_T17 (SEQ. ID NO:165), C03950_3_T18 (SEQ. ID NO:166), C03950_3_T19 (SEQ. ID NO:167), C03950_3_T2 (SEQ. ID NO:156), C03950_3_T4 (SEQ. ID NO:157), C03950_3_T7 (SEQ. ID NO:158), C03950_3_T8 (SEQ. ID NO:159) and C03950_3_T9 (SEQ. ID NO:160). Table 125 below describes the starting and ending position of this segment on each transcript.

*Table 125 - Segment location on transcripts*

| Transcript name | Segment starting position | Segment ending position |
|---|---|---|
| C03950_3_T10 (SEQ. ID NO:161) | 1811 | 2005 |
| C03950_3_T11 (SEQ.IDNO:162) | 1861 | 2055 |
| C03950_3_T13 (SEQ. ID NO:163) | 1811 | 2005 |
| C03950_3_T15 (SEQ. ID NO:164) | 1861 | 2055 |
| C03950_3_T17 (SEQ. ID NO:165) | 1861 | 2055 |
| C03950_3_T18 (SEQ. ID NO:166) | 1811 | 2005 |
| C03950_3_T19 (SEQ. ID NO:167) | 1861 | 2055 |
| C03950_3_T2 (SEQ. ID NO:156) | 1811 | 2005 |
| C03950_3_T4 (SEQ. ID NO:157) | 1861 | 2055 |
| C03950_3_T7 (SEQ. ID NO:158) | 1861 | 2055 |
| C03950_3_T8 (SEQ. ID NO:159) | 1185 | 1379 |
| C03950_3_T9 (SEQ. ID NO:160) | 1185 | 1379 |

Segment cluster C03950_3_N45 (SEQ. ID NO:177) according to the present invention is supported by 2 libraries. The number of libraries was determined as previously described. This segment can be found in the following transcript (s): C03950_3_T18 (SEQ. ID NO:166) and C03950_3_T19 (SEQ. ID NO:167). Table 126 below describes the starting and ending position of this segment on each transcript.

*Table 126 - Segment location on transcripts*

| Transcript name | **Segment** starting position | **Segment** ending position |
|---|---|---|
| C03950_3_T18 (SEQ. ID NO:166) | 2006 | 2339 |
| C03950_3_T19 (SEQ. ID NO:167) | 2056 | 2389 |

Segment cluster C03950_3_N48 (SEQ. ID NO:178) according to the present invention is supported by 2 libraries. The number of libraries was determined as previously described. This segment can be found in the following transcript (s): C03950_3_T13 (SEQ. ID NO:163). Table 127 below describes the starting and ending position of this segment on each transcript.

*Table 127 - Segment location on transcripts*

| Transcript name | Segment starting position | Segment ending position |
|---|---|---|
| C03950_3_T13 (SEQ. ID NO:163) | 2111 | 7282 |

Segment cluster C03950_3_N49 (SEQ. ID NO:179) according to the present invention is supported by 10 libraries. The number of libraries was determined as previously described. This segment can be found in the following transcript(s): C03950_3_T13 (SEQ. ID NO:163) and C03950_3_T15 (SEQ. ID NO:164). Table 128 below describes the starting and ending position of this segment on each transcript.

*Table 128 - Segment location on transcripts*

| Transcript name | Segment starting position | Segment ending position |
|---|---|---|
| C03950_3_T13 (SEQ. ID NO:163) | 7283 | 7566 |
| C03950_3_T15 (SEQ. ID NO:164) | 2161 | 2444 |

Segment cluster C03950_3_N56 (SEQ. ID NO:180) according to the present invention is supported by 8 libraries. The number of libraries was determined as previously described. This segment can be found in the following transcript(s): C03950_3_T17 (SEQ. ID NO:165). Table 129 below describes the starting and ending position of this segment on each transcript.

*Table 129 - Segment location on transcripts*

| Transcript name | Segment starting position | Segment ending position |
|---|---|---|
| C03950_3_T17 (SEQ. ID N0:165) | 2161 | 2352 |

Segment cluster C03950_3_N62 (SEQ. ID NO:181) according to the present invention is supported by 2 libraries. The number of libraries was determined as previously described. This segment can be found in the following transcript (s): C03950_3_T22 (SEQ. ID NO:169) and C03950_3_T23 (SEQ. ID NO:170). Table 130 below describes the starting and ending position of this segment on each transcript.

*Table 130 - Segment location on transcripts*

| Transcript name | Segment starting position | Segment ending position |
|---|---|---|
| C03950_3_T22 (SEQ. ID NO:169) | 1 | 591 |
| C03950_3_T23 (SEQ. ID NO:170) | 1 | 591 |

Segment cluster C03950_3_N67 (SEQ. ID NO:183) according to the present invention is supported by 3 libraries. The number of libraries was determined as previously described. This segment can be found in the following transcript (s): C03950_3_T10 (SEQ. ID NO:161) and C03950_3_T11 (SEQ. ID NO:162). Table 131 below describes the starting and ending position of this segment on each transcript.

*Table 131 - Segment location on transcripts*

| Transcript name | Segment starting position | Segment ending position |
|---|---|---|
| C03950_3_T10 (SEQ. ID NO:161) | 2460 | 2750 |
| C03950_3_T11 (SEQ. ID NO:162) | 2510 | 2800 |

According to an optional embodiment of the present invention, short segments related to the above cluster are also provided. These segments are up to about 120 bp in length, and so are included in a separate description.

Segment cluster C03950_3_N17 (SEQ. ID NO:191) according to the present invention is supported by 1 libraries. The number of libraries was determined as previously described. This segment can be found in the following transcript (s): C03950_3_T8 (SEQ. ID NO:159) and C03950_3_T9 (SEQ. ID NO:160). Table 132 below describes the starting and ending position of this segment on each transcript.

*Table 132 - Segment location on transcripts*

| Transcript name | Segment starting position | Segment ending position |
|---|---|---|
| C03950 3 T8 (SEQ. ID NO:159) | 1 | 45 |
| C03950_3_T9 (SEQ.IDNO:160) | 1 | 45 |

Segment cluster C03950_3_N40 (SEQ. ID NO:199) according to the present invention is supported by 2 libraries. The number of libraries was determined as previously described. This segment can be found in the following transcript (s): C03950_3_T21 (SEQ. ID NO:168). Table 133 below describes the starting and ending position of this segment on each transcript.

*Table 133 - Segment location on transcripts*

| Transcript name | Segment starting position | Segment ending position |
|---|---|---|
| C03950_3_T21 SEQ. ID NO:168) | 1803 | 1829 |

Segment cluster C03950_3_N51 (SEQ. ID NO:202) according to the present invention is supported by 1 libraries. The number of libraries was determined as previously described. This segment can be found in the following transcript(s): C03950_3_T2 (SEQ. ID NO:156), C03950_3_T4 (SEQ. ID NO:157) and C03950_3_T9 (SEQ. ID NO:160). Table 134 below describes the starting and ending position of this segment on each transcript.

*Table 134 - Segment location on transcripts*

| Transcript name | Segment starting position | Segment ending position |
|---|---|---|
| C03950_3_T2 (SEQ. ID NO:156) | 2111 | 2229 |
| C03950_3_T4 (SEQ. ID NO:157) | 2161 | 2279 |
| C03950_3_T9 (SEQ. ID NO:160) | 1485 | 1603 |

Segment cluster C03950_3_N75 (SEQ. ID NO:208) according to the present invention is supported by 1 libraries. The number of libraries was determined as previously described. This segment can be found in the following transcript (s): C03950_3_T23 (SEQ. ID NO:170) and C03950_3_T7 (SEQ. ID NO:158). Table 135 below describes the starting and ending position of this segment on each transcript.

*Table 135 - Segment location on transcripts*

| Transcript name | Segment starting position | Segment ending position |
|---|---|---|
| C03950_3_T23 (SEQ. ID NO:170) | 1145 | 1218 |
| C03950_3_T7 (SEQ. ID NO:158) | 2820 | 2893 |

Expression of Homo sapiens TNNI3 interacting kinase (TNNI3K) C03950 transcripts which are detectable by amplicon as depicted in sequence name C03950_seg44WT (SEQ. ID NO: 233) specifically in heart tissue

Expression of Homo sapiens TNNI3 interacting kinase (TNNI3K) transcripts detectable by or according to seg44WT - C03950_seg44WT (SEQ. ID NO: 233) amplicon and primers C03950_seg44WTF (SEQ. ID NO: 231) and C03950_seg44WTR (SEQ. ID NO: 232) was measured by real time PCR. In parallel the expression of four housekeeping genes - SDHA (GenBank Accession No. NM_004168 (SEQ. ID NO: 33); amplicon - SDHA-amplicon (SEQ. ID NO:36) ), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24) ) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the median of the quantities of the heart samples (sample numbers 44, 45 and 46, Table 1_6 above), to obtain a value of relative expression for each sample relative to median of the heart samples.

Figure 24 is a histogram showing relative expression of the above-indicated Homo sapiens TNNI3 interacting kinase (TNNI3K) transcripts in heart tissue samples as opposed to other tissues.

As is evident from Figure 24, the expression of Homo sapiens TNNI3 interacting kinase (TNNI3K) transcripts detectable by the above amplicon in heart tissue samples was significantly higher than in most of the other samples (sample numbers 1, 2, 3, 4, 5, 6, 7, 8, 9, 10, 11, 12, 13, 14, 15, 16, 17, 19, 20, 75, 76, 77, 78, 21, 23, 24, 25, 26, 28, 29, 30, 31, 32, 33, 34, 35, 36, 37, 38, 39, 40, 41, 42, 43, 47, 48, 49, 50, 51, 52, 53, 54, 55, 57, 58, 59, 60, 61, 62, 63, 64, 65, 66, 67, 68, 69, 70, 71, 72, 73 and 74, Table 1_6 above) except for the brain samples.

Primer pairs are also optionally and preferably encompassed within the present invention; for example, for the above experiment, the following primer pair was used as a non-limiting illustrative example only of a suitable primer pair: C03950_seg44WTF (SEQ. ID NO: 231) forward primer; and C03950_seg44WTR (SEQ. ID NO: 232) reverse primer.

The present invention also preferably encompasses any amplicon obtained through the use of any suitable primer pair; for example, for the above experiment, the following amplicon was obtained as a non-limiting illustrative example only of a suitable amplicon: C03950_seg44WT (SEQ. ID NO: 233).

Forward Primer C03950_seg44WTF (SEQ. ID NO:231):GAGCCAATACCTACTGCTCCAAG
Reverse Primer C03950_seg44WTR (SEQ. ID NO:232): GCAAGCACCCACAAACTGAATTA
Amplicon (C03950_seg44WT (SEQ. ID NO: 233)):

GAGCCAATACCTACTGCTCCAAGTCAGATGTGGATATGTTTTGCCGAGAGGTGTC
CATTCTCTGCCAGCTCAATCATCCCTGCGTAATTCAGTTTGTGGGTGCTTGC

Expression of Homo sapiens TNNI3 interacting kinase (TNNI3K) C03950 transcripts which are detectable by amplicon as depicted in sequence name C03950_seg51 (SEQ. ID NO: 236) specifically in heart tissue

Expression of Homo sapiens TNNI3 interacting kinase (TNNI3K) transcripts detectable by or according to seg51 - C03950_seg51 (SEQ. ID NO: 236) amplicon and primers C03950_seg51F (SEQ. ID NO: 234) and C03950_seg51R (SEQ. ID NO: 235) was measured by real time PCR. In parallel the expression of four housekeeping genes - SDHA (GenBank Accession No. NM_004168 (SEQ. ID NO: 33); amplicon - SDHA-amplicon (SEQ. ID NO:36)), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24)) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the median of the quantities of the heart samples (sample numbers 44, 45 and 46, Table 1_6 above), to obtain a value of relative expression for each sample relative to median of the heart samples.

Figure 25 is a histogram showing relative expression of the above-indicated Homo sapiens TNNI3 interacting kinase (TNNI3K) transcripts in heart tissue samples as opposed to other tissues.

As is evident from Figure 25, the expression of Homo sapiens TNNI3 interacting kinase (TNNI3K) transcripts detectable by the above amplicon in heart tissue samples was significantly higher than in most of the other samples (sample numbers 1, 2, 3, 4, 5, 6, 7, 8, 9, 10, 11, 12, 13, 14, 15, 16, 17, 19, 20, 75, 76, 77, 78, 21, 23, 24, 25, 26, 28, 29, 30, 31, 32, 33, 34, 35, 36, 37, 38, 39, 40, 41, 42, 43, 47, 48, 49, 50, 51, 52, 53, 54, 55, 57, 58, 59, 60, 61, 62, 63, 64, 65, 66, 67, 68, 69, 70, 71, 72, 73 and 74, Table 1_6 above) except for the brain samples.

Primer pairs are also optionally and preferably encompassed within the present invention; for example, for the above experiment, the following primer pair was used as a non-limiting illustrative example only of a suitable primer pair: C03950_seg51F (SEQ. ID NO: 234) forward primer; and C03950_seg51R (SEQ. ID NO: 235) reverse primer.

The present invention also preferably encompasses any amplicon obtained through the use of any suitable primer pair; for example, for the above experiment, the following amplicon was obtained as a non-limiting illustrative example only of a suitable amplicon: C03950_seg51 (SEQ. ID NO: 236).

Forward Primer (C03950_seg51F (SEQ. ID NO: 234)):TCTGCCATTACCTCTAGGATCTGG

Reverse Primer (C03950_seg51R (SEQ. ID NO: 235)):GGCAGAAGTAAGCATACACCTGAAA

Amplicon (C03950_seg51 (SEQ. ID NO: 236)):

TCTGCCATTACCTCTAGGATCTGGATCACCCATAGTATTTGCATCTGGAGGGGAGC
TCATTACTTTAACAGGGAAGAATGCAATTTCAGGTGTATGCTTACTTCTGCC

Expression of Homo sapiens TNNI3 interacting kinase (TNNI3K) C03950 transcripts which are detectable by amplicon as depicted in sequence name C03950_seg67F2R2 (SEQ. ID NO: 239) specifically in heart tissue

Expression of Homo sapiens TNNI3 interacting kinase (TNNI3K) transcripts detectable by or according to seg67F2R2 - C03950_seg67F2R2 (SEQ. ID NO: 239) amplicon and primers C03950_seg67F2 (SEQ. ID NO: 237) and C03950_seg67R2 (SEQ. ID NO: 238) was measured by real time PCR. In parallel the expression of four housekeeping genes - SDHA (GenBank Accession No. NM_004168 (SEQ. ID NO: 33); amplicon - SDHA-amplicon (SEQ. ID NO:36)), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24)) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the median of the quantities of the heart samples (sample numbers 44, 45 and 46, Table 1_6 above), to obtain a value of relative expression for each sample relative to median of the heart samples.

Figure 26 is a histogram showing relative expression of the above-indicated Homo sapiens TNNI3 interacting kinase (TNNI3K) transcripts in heart tissue samples as opposed to other tissues.

As is evident from Figure 26, the expression of Homo sapiens TNNI3 interacting kinase (TNNI3K) transcripts detectable by the above amplicon in heart tissue samples was significantly higher than in most of the other samples (sample numbers 1, 2, 3, 4, 5, 6, 7, 8, 9, 10, 11, 12, 13, 14, 15, 16, 17, 19, 20, 75, 76, 77, 78, 21, 23, 24, 25, 26, 28, 29, 30, 31, 32, 33, 34, 35, 36, 37, 38, 39, 40, 41, 42, 43, 47, 48, 49, 50, 51, 52, 53, 54, 55, 57, 58, 59, 60, 61, 62, 63, 64, 65, 66, 67, 68, 69, 70, 71, 72, 73 and 74, Table 1_6 above) except for the brain samples.

Primer pairs are also optionally and preferably encompassed within the present invention; for example, for the above experiment, the following primer pair was used as a non-limiting illustrative example only of a suitable primer pair: C03950_seg67F2 (SEQ. ID NO: 237) forward primer; and C03950_seg67R2 (SEQ. ID NO: 238) reverse primer.

The present invention also preferably encompasses any amplicon obtained through the use of any suitable primer pair; for example, for the above experiment, the following amplicon was obtained as a non-limiting illustrative example only of a suitable amplicon: C03950_seg67F2R2 (SEQ. ID NO: 239).
Forward Primer C03950_seg67F2 (SEQ. ID NO:237):CGTTTTGGGATGTGGATTGAGT
Reverse Primer C03950_seg67R2 (SEQ. ID NO:238):ACCGCTTTCATGGAGCTAACA
Amplicon C03950_seg67F2R2 (SEQ. ID NO:239)

CGTTTTGGGATGTGGATTGAGTATCTCAGAAGATAACCTCTTATCCTGGCCATTCA
ACCTGATGTGTTACATGTTTATTTGTTTAGAATCTTCCATCACTACCAAAATGTTA
GCTCCATGAAAGCGGT

DESCRIPTION FOR CLUSTER R15601

Cluster R15601 features 2 transcript(s) and 25 segment(s) of interest, the names for which are given in Tables 136 and 137, respectively. The selected protein variants are given in table 138.

*Table 136 - Transcripts of interest*

| Transcript Name |
|---|
| R15601_T8(SEQ. ID NO:240) |
| R15601_T9 (SEQ. ID NO:241) |

*Table 137 - Segments of interest*

| Segment Name |
|---|
| R15601_N4 (SEQ. ID NO:242) |
| R15601_N6 (SEQ. ID NO:243) |
| R15601_N10 (SEQ. ID NO:244) |
| R15601_N14 (SEQ. ID NO:245) |
| R15601_N16 (SEQ. ID NO:246) |
| R15601_N18 (SEQ. ID NO:247) |
| R15601_N20 (SEQ. ID NO:248) |
| R15601_N22 (SEQ. ID NO:249) |
| R15601_N26 (SEQ. ID NO:250) |
| R15601_N28 (SEQ. ID NO:251) |
| R15601_N30 (SEQ. ID NO:252) |
| R15601_N32 (SEQ. ID NO:253) |
| R15601_N42 (SEQ. ID NO:254) |
| R15601_N45 (SEQ. ID NO:255) |
| R15601_N0 (SEQ. ID NO:256) |
| R15601_N3 (SEQ. ID NO:257) |
| R15601_N8 (SEQ. ID NO:258) |
| R15601_N12 (SEQ. ID NO:259) |
| R15601_N24 (SEQ. ID NO:260) |

(continued)

| Segment Name |
|---|
| R15601_N27 (SEQ. ID NO:261) |
| R15601_N34 (SEQ. ID NO:262) |
| R15601_N36 (SEQ. ID NO:263) |
| R15601_N38 (SEQ. ID NO:264) |
| R15601_N40 (SEQ. ID NO:265) |
| R15601_N44 (SEQ. ID NO:266) |

*Table 138 - Proteins of interest*

| Protein Name | Corresponding Transcript(s) |
|---|---|
| R15601_P2 (SEQ. ID NO:268) | R15601_T8 (SEQ. ID NO:240) |
| R15601_P3 (SEQ. ID NO:269) | R15601_T9 (SEQ. ID NO:241) |

These sequences are variants of the known protein cardiomyopathy associated 4 (SEQ. ID NO:267) (SwissProt accession identifier NP_775259 (SEQ. ID NO: 397)), referred to herein as the previously known protein.

The sequence for protein cardiomyopathy associated 4 (SEQ. ID NO:267) is given.

Non-limiting exemplary utilities for R15601 variants according to the present invention are described in greater detail below and also with regard to the previous section on clinical utility.The heart-selective diagnostic marker prediction engine provided the following results with regard to cluster R15601. Predictions were made for selective expression of transcripts of this contig in heart tissue, according to the previously described methods. The numbers on the y-axis of the first figure below refer to weighted expression of ESTs in each category, as "parts per million" (ratio of the expression of ESTs for a particular cluster to the expression of all ESTs in that category, according to parts per million).

Overall, the following results were obtained as shown with regard to the histogram in Figure 27, concerning the number of heart-specific clones in libraries/sequences.

This cluster was found to be selectively expressed in heart for the following reasons: in a comparison of the ratio of expression of the cluster in heart specific ESTs to the overall expression of the cluster in non-heart ESTs, which was found to be 16.3; the ratio of expression of the cluster in heart specific ESTs to the overall expression of the cluster in muscle-specific ESTs which was found to be 3.8; and fisher exact test P-values were computed both for library and weighted clone counts to check that the counts are statistically significant, and were found to be 2.70E-07.

One particularly important measure of specificity of expression of a cluster in heart tissue is the previously described comparison of the ratio of expression of the cluster in heart as opposed to muscle. This cluster was found to be specifically expressed in heart as opposed to non-heart ESTs as described above. However, many proteins have been shown to be generally expressed at a higher level in both heart and muscle, which is less desirable. For this cluster, as described above, the ratio of expression of the cluster in heart specific ESTs to the overall expression of the cluster in muscle-specific ESTs which was found to be 16.3, which clearly supports specific expression in heart tissue.

As noted above, cluster R15601 features 2 transcript(s), which were listed in Table 136 above. These transcript (s) encode for protein(s) which are variant(s) of protein cardiomyopathy associated 4 (SEQ. ID NO:267). A description of each variant protein according to the present invention is now provided.

Variant protein R15601_P2 (SEQ. ID NO:268) according to the present invention is encoded by transcript R15601_T8 (SEQ. ID NO:240). One or more alignments to one or more previously published cardiomyopathy associated 4 (SEQ. ID NO:267) protein sequences are given in the alignment table on the attached CD-ROM. A brief description of the relationship of the variant protein according to the present invention to each such aligned protein is as follows:

1. Comparison report between R15601_P2 (SEQ. ID NO:268) and NP_775259 (SEQ. ID NO: 397):

A. An isolated chimeric polypeptide encoding for R15601_P2 (SEQ. ID NO:268), comprising a first amino acid sequence being at least 70%, optionally at least 80%, preferably at least 85%, more preferably at least 90% and most preferably at least 95%, homologous to a polypeptide having the sequence MPRKDRNSSRAESAQCQVLSCVIHGILLMAREIAVVVLPLSQ (SEQ. ID NO: 388) corresponding to amino acids 1 - 42 of R15601_P2 (SEQ. ID NO:268), and a second amino acid sequence being at least 90% homologous to ESYVQAASDASRAIDINSSDIKALYRRCQALEHLGKLDQAFKDVQRCATLEPRNQNFQEMLR RLNTSIQEKLRVQFSTDSRVQKMFEILLDENSEADKREKAANNLIVLGREEAGAEKIFQNNGV ALLLQLLDTKKPELVLAAVRTLSGMCSGHQARATVILHAVRIDRICSLMAVENEEMSLAVCN LLQAIIDSLSGEDKREHRGKEEALVLDTKKDLKQITSHLLDMLVSKKVSGQGRDQALNLLNK NVPRKDLAIHDNSRTIYVVDNGLRKILKVVGQVPDLPSCLPLTDNTRMLASILINKLYDDLRC DPERDHFRKICEEYITGKFDPQDMDKNLNAIQTVSGILQGPFDLGNQLLGLKGVMEMMVALC GSERETDQLVAVEALIHASTKLSRATFIITNGVSLLKQIYKTTKNEKIKIRTLVGLCKLGSAGGT DYGLRQFAEGSTEKLAKQCRKWLCNMSIDTRTRRWAVEGLAYLTLDADVKDDFVQDVPAL QAMFELAKAGTSDKTILYSVATTLVNCTNSYDVKEVIPELVQLAKFSKQHVPEEHPKDKKDFI DMRVKRLLKAGVISALACMVKADSAILTDQTKELLARVFLALCDNPKDRGTIVAQGGGKALI PLALEGTDVGKVKAAHALAKIAAVSNPDIAFPGERVYEVVRPLVRLLDTQRDGLQNYEALLG LTNLSGRSDKLRQKIFKERALPDIENYMFENHDQLRQAATECMCNMVLHKEVQERFLADGN DRLKLVVLLCGEDDDKVQNAAAGALAMLTAAHKKLCLKMTQVTTQWLEILQRLCLHDQLS VQHRGLVIAYNLLAADAELAKKLVESELLEILTVVGKQEPDEKKAEVVQTARECLIKCMDYG FIKPVS corresponding to amino acids 57 - 931 of NP_775259 (SEQ. ID NO: 397), which also corresponds to amino acids 43 - 917 of R15601_P2 (SEQ. ID NO:268), wherein said first amino acid sequence and second amino acid sequence are contiguous and in a sequential order.

B. An isolated polypeptide encoding for a head of R15601_P2 (SEQ. ID NO:268), comprising a polypeptide being at least 70%, optionally at least about 80%, preferably at least about 85%, more preferably at least about 90% and most preferably at least about 95% homologous to the sequence MPRKDRNSSRAESAQCQVLSCVIHGILLMAREIAVVVLPLSQ (SEQ. ID NO: 388) of R15601_P2 (SEQ. ID NO:268).

The localization of the variant protein was determined according to results from a number of different software programs and analyses, including analyses from SignalP and other specialized programs. The variant protein is believed to be membranal with regard to the cell.

Variant protein R15601_P2 (SEQ. ID NO:268) also has the following non-silent SNPs (Single Nucleotide Polymorphisms) as listed in Table 139, (given according to their position(s) on the amino acid sequence, with the alternative amino acid(s) listed.

*Table 139 - Amino acid mutations*

| SNP position(s) on amino acid sequence | Alternative amino acid(s) |
|---|---|
| 46 | V -> I |
| 838 | I -> N |

Variant protein R15601_P2 (SEQ. ID NO:268) is encoded by the following transcript(s): R15601_T8 (SEQ. ID NO:240), for which the coding portion starts at position 56 and ends at position 2806. The transcript also has the following SNPs as listed in Table 140 (given according to their position on the nucleotide sequence, with the alternative nucleic acid listed.

*Table 140 - Nucleic acid SNPs*

| SNP position(s) on nucleotide sequence | Alternative nucleic acid(s) |
|---|---|
| 191 | G -> A |
| 394 | G -> A |
| 1480 | C -> T |
| 1807 | C -> T |
| 2568 | T -> A |
| 2822 | A -> C |
| 2880 | G -> A |
| 2919 | A -> G |
| 2919 | A -> T |

Variant protein R15601_P3 (SEQ. ID NO:269) according to the present invention is encoded by transcript R15601_T9 (SEQ. ID NO:241). One or more alignments to one or more previously published cardiomyopathy associated 4 (SEQ. ID NO:267) protein sequences are given in the alignment table on the attached CD-ROM. A brief description of the relationship of the variant protein according to the present invention to each such aligned protein is as follows:

1. Comparison report between R15601_P3 (SEQ. ID NO:269) and NP_775259 (SEQ. ID NO: 397):

A. An isolated chimeric polypeptide encoding for R15601_P3 (SEQ. ID NO:269), comprising a first amino acid sequence being at least 90% homologous to MAEVEAVQLKEEGNRHFQLQDYKAATNSYSQALKLTKDKA-LLATLYRNRAACGLKTESYV QAASDASRAIDINSSDIKALYRRCQALEHLGKLDQAFKDVQRCATLEPRNQN-FQEMLRRLNT SIQEKLRVQFSTDSRVQKMFEILLDENSEADKREKAANNLIVLGREEAGAEKIFQNNGVALLL QLLDTKKPELVLAAVRTLSGMCSGHQARATVILHAVRIDRICSLMAVENEEMSLAVCNLLQA IIDSLSGEDKREHRGKEEALVLDTKKDLKQITSHLLDMLVSKKVSGQGRDQALNLLNKNVPR KDLAIHDNSRTIYVVDNGLRKILKWGQVPDLPSCLPLTDNTRMLASILINKLYDDLRCDPER DHFRKICEEYITGKFDPQDMDKNLNAIQTVSGILQGPFDLGNQLLGLKGVMEMMVALCGSER ETDQLVAVEALIHASTKLSRATFIITNGVSLLKQIYKTTKNEKIKIRTLVGLCKLGSAGGTDYG LRQFAEGSTEKLAKQCRKWLCNMSIDTRTRRWAVEGLAYLTLDADVKDDFVQDVPALQAM FELAKAG corresponding to amino acids 1 - 565 of NP_775259 (SEQ. ID NO: 397), which also corresponds to amino acids 1 - 565 of R15601_P3 (SEQ. ID NO:269), and a second amino acid sequence being at least 70%, optionally at least 80%, preferably at least 85%, more preferably at least 90% and most preferably at least 95% homologous to a polypeptide having the sequence VGESGPTTNLRKGLLGPDPQGMDPSLPPGSTPYPCINMIGYFPLSGPHFT (SEQ. ID NO: 389) corresponding to amino acids 566 - 615 of R15601_P3 (SEQ. ID NO:269), wherein said first amino acid sequence and second amino acid sequence are contiguous and in a sequential order.

B. An isolated polypeptide encoding for an edge portion of R15601_P3 (SEQ. ID NO:269), comprising an amino acid sequence being at least 70%, optionally at least about 80%, preferably at least about 85%, more preferably at least about 90% and most preferably at least about 95% homologous to the sequence VGESGPTTNLRKGLLGPDPQGMDPSLPPGSTPYPCINMIGYFPLSGPHFT (SEQ. ID NO: 389) of R15601_P3 (SEQ. ID NO:269).

The localization of the variant protein was determined according to results from a number of different software programs and analyses, including analyses from SignalP and other specialized programs. The variant protein is believed to be located intracellularly.

Variant protein R15601_P3 (SEQ. ID NO:269) also has the following non-silent SNPs (Single Nucleotide Polymorphisms) as listed in Table 141, (given according to their position(s) on the amino acid sequence, with the alternative amino acid(s) listed.

*Table 141 - Amino acid mutations*

| SNP position(s) on amino acid sequence | Alternative amino acid(s) |
|---|---|
| 60 | V -> I |

Variant protein R15601_P3 (SEQ. ID NO:269) is encoded by the following transcript(s): R15601_T9 (SEQ. ID NO:241), for which the coding portion starts at position 95 and ends at position 1939. The transcript also has the following SNPs as listed in Table 142 (given according to their position on the nucleotide sequence, with the alternative nucleic acid listed.

*Table 142 - Nucleic acid SNPs*

| SNP position(s) on nucleotide sequence | Alternative nucleic acid(s) |
|---|---|
| 238 | C -> T |
| 272 | G -> A |
| 475 | G -> A |
| 1561 | C -> T |
| 1840 | A -> G |

As noted above, cluster R15601 features 25 segment(s), which were listed in Table 137 above and for which the sequence(s) are given. These segment(s) are portions of nucleic acid sequence(s) which are described herein separately because they are of particular interest. A description of several segments according to the present invention is now provided.

Segment cluster R15601_N6 (SEQ. ID NO:243) according to the present invention is supported by 1 libraries. The number of libraries was determined as previously described. This segment can be found in the following transcript (s): R15601_T8 (SEQ. ID NO:240). Table 143 below describes the starting and ending position of this segment on each transcript.

*Table 143 - Segment location on transcripts*

| Transcript name | Segment starting position | Segment ending position |
|---|---|---|
| R15601 T8 (SEQ. ID NO:240) | 1 | 181 |

Segment cluster R15601_N28 (SEQ. ID NO:251) according to the present invention is supported by 1 libraries. The number of libraries was determined as previously described. This segment can be found in the following transcript (s): R15601_T9 (SEQ. ID NO:241). Table 144 below describes the starting and ending position of this segment on each transcript.

*Table 144 - Segment location on transcripts*

| Transcript name | Segment starting position | Segment ending position |
|---|---|---|
| R15601 T9 (SEQ. ID NO:241) | 1790 | 1956 |

Segment cluster R15601_N30 (SEQ. ID NO:252) according to the present invention is supported by 13 libraries. The number of libraries was determined as previously described. This segment can be found in the following transcript (s): R15601_T8 (SEQ. ID NO:240). Table 145 below describes the starting and ending position of this segment on each transcript.

*Table 145 - Segment location on transcripts*

| Transcript name | Segment starting position | Segment ending position |
|---|---|---|
| R15601_T8 (SEQ. ID NO:240) | 1709 | 1849 |

Expression of Homo sapiens cardiomyopathy associated 4 (CMYA4) R15601 transcripts which are detectable by amplicon as depicted in sequence name R15601_seg28 (SEQ. ID NO: 272) specifically in heart tissue

Expression of Homo sapiens cardiomyopathy associated 4 (CMYA4) transcripts detectable by or according to seg28 - R15601_seg28 (SEQ. ID NO: 272) amplicon and primers R15601_seg28F (SEQ. ID NO: 270) and R15601_seg28R (SEQ. ID NO: 271) was measured by real time PCR. In parallel the expression of four housekeeping genes - SDHA (GenBank Accession No. NM_004168 (SEQ. ID NO: 33); amplicon - SDHA-amplicon (SEQ. ID NO:36) ), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24) ) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the median of the quantities of the heart samples (sample numbers 44, 45 and 46, Table 1_6 above), to obtain a value of relative expression for each sample relative to median of the heart samples.

Figure 28 is a histogram showing relative expression of the above-indicated Homo sapiens cardiomyopathy associated 4 (CMYA4) transcripts in heart tissue samples as opposed to other tissues. Values represent the average of duplicate experiments. Error bars indicate the minimal and maximal values obtained.

As is evident from Figure 28, the expression of Homo sapiens cardiomyopathy associated 4 (CMYA4) transcripts detectable by the above amplicon in heart tissue samples was significantly higher than in most of the other samples (sample numbers 1, 2, 3, 4, 5, 6, 7, 8, 9, 10, 11, 12, 13, 14, 15, 16, 17, 19, 20, 75, 76, 77, 78, 21, 23, 24, 25, 26, 28, 29, 30, 31, 32, 33, 34, 35, 36, 37, 38, 39, 40, 41, 42, 43, 47, 48, 49, 50, 51, 52, 53, 54, 55, 57, 58, 59, 60, 61, 62, 63, 64, 65, 66, 67, 68, 69, 70, 71, 72, 73 and 74, Table 1_6 above).

Primer pairs are also optionally and preferably encompassed within the present invention; for example, for the above experiment, the following primer pair was used as a non-limiting illustrative example only of a suitable primer pair:

R15601_seg28F (SEQ. ID NO: 270) forward primer; and R15601_seg28R (SEQ. ID NO: 271) reverse primer.

The present invention also preferably encompasses any amplicon obtained through the use of any suitable primer pair; for example, for the above experiment, the following amplicon was obtained as a non-limiting illustrative example only of a suitable amplicon: R15601_seg28 (SEQ. ID NO: 272).

Forward Primer (R15601_seg28F (SEQ. ID NO: 270)):GTCTGGCCCGACCACAAAC
Reverse Primer (R15601_seg28R (SEQ. ID NO: 271)):GGTAAGGAGTAGAGCCAGGAGGA
Amplicon (R15601_seg28 (SEQ. ID NO: 272)):

GTCTGGCCCGACCACAAACCTCAGGAAAGGTCTGCTGGGTCCAGACCCACAGGGA
ATGGATCCCAGTCTTCCTCCTGGCTCTACTCCTTACC

Expression of Homo sapiens cardiomyopathy associated 4 (CMYA4) R15601 transcripts which are detectable by amplicon as depicted in sequence name R15601_seg30WT (SEQ. ID NO: 275) specifically in heart tissue

Expression of Homo sapiens cardiomyopathy associated 4 (CMYA4) transcripts detectable by or according to seg30WT - R15601_seg30WT (SEQ. ID NO: 275) amplicon and primers R15601_seg30WTF (SEQ. ID NO: 273) and R15601_seg30WTR (SEQ. ID NO: 274) was measured by real time PCR. In parallel the expression of four housekeeping genes - SDHA (GenBank Accession No. NM_004168 (SEQ. ID NO: 33); amplicon - SDHA-amplicon (SEQ. ID NO:36)), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24)) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the median of the quantities of the heart samples (sample numbers 44, 45 and 46, Table 1_6 above), to obtain a value of relative expression for each sample relative to median of the heart samples.

Figure 29 is a histogram showing relative expression of the above-indicated Homo sapiens cardiomyopathy associated 4 (CMYA4) transcripts in heart tissue samples as opposed to other tissues.

As is evident from Figure 29, the expression of Homo sapiens cardiomyopathy associated 4 (CMYA4) transcripts detectable by the above amplicon in heart tissue samples was significantly higher than in most of the other samples (sample numbers 1, 2, 3, 4, 5, 6, 7, 8, 9, 10, 11, 12, 13, 14, 15, 16, 17, 19, 20, 75, 76, 77, 78, 21, 23, 24, 25, 26, 28, 29, 30, 31, 32, 33, 34, 35, 36, 37, 38, 39, 40, 41, 42, 43, 47, 48, 49, 50, 51, 52, 53, 54, 55, 57, 58, 59, 60, 61, 62, 63, 64, 65, 66, 67, 68, 69, 70, 71, 72, 73 and 74, Table 1_6 above).

Primer pairs are also optionally and preferably encompassed within the present invention; for example, for the above experiment, the following primer pair was used as a non-limiting illustrative example only of a suitable primer pair: R15601_seg30WTF (SEQ. ID NO: 273) forward primer; and R15601_seg30WTR (SEQ. ID NO: 274) reverse primer. The present invention also preferably encompasses any amplicon obtained through the use of any suitable primer pair; for example, for the above experiment, the following amplicon was obtained as a non-limiting illustrative example only of a suitable amplicon: R15601_seg30WT (SEQ. ID NO: 275).

Forward Primer (R15601_seg30WTF (SEQ. ID NO: 273)):ACCATCCTGTACTCGGTGGC
Reverse Primer (R15601_seg30WTR (SEQ. ID NO: 274)):CATGCTGCTTGGAGAACTTGG
Amplicon (R15601_seg30WT (SEQ. ID NO: 275)):

ACCATCCTGTACTCGGTGGCCACCACCCTGGTGAACTGCACCAACAGCTACGATG
TCAAGGAGGTCATCCCAGAGCTTGTCCAGCTCGCCAAGTTCTCCAAGCAGCATG

DESCRIPTION FOR CLUSTER T11811

Cluster T11811 features 6 transcript(s) and 20 segment(s) of interest, the names for which are given in Tables 146 and 147, respectively. The selected protein variants are given in table 148.

*Table 146 - Transcripts of interest*

| Transcript Name |
|---|
| T11811_T3 (SEQ. ID NO:276) |

(continued)

| Transcript Name |
|---|
| T11811_T6 (SEQ. ID NO:277) |
| T11811_T12 (SEQ. ID NO:278) |
| T11811_T13 (SEQ. ID NO:279) |
| T11811_T15 (SEQ. ID NO:280) |
| T11811_T24 (SEQ. ID NO:281) |

*Table 147 - Segments of interest*

| Segment Name |
|---|
| T11811_N0 (SEQ. ID NO:282) |
| T11811_N10 (SEQ. ID NO:283) |
| T11811_N26 (SEQ. ID NO:284) |
| T11811_N2 (SEQ. ID NO:285) |
| T11811_N4 (SEQ. ID NO:286) |
| T11811_N5 (SEQ. ID NO:287) |
| T11811_N7 (SEQ. ID NO:288) |
| T11811_N8 (SEQ. ID NO:289) |
| T11811_N9 (SEQ. ID NO:290) |
| T11811_N11 (SEQ. ID NO:291) |
| T11811_N12 (SEQ. ID NO:292) |
| T11811_N13 (SEQ. ID NO:293) |
| T11811_N14 (SEQ. ID NO:294) |
| T11811_N15 (SEQ. ID NO:295) |
| T11811_N17 (SEQ. ID NO:296) |
| T11811_N18 (SEQ. ID NO:297) |
| T11811_N20 (SEQ. ID NO:298) |
| T11811_N21 (SEQ. ID NO:299) |
| T11811_N22(SEQ. ID NO:300) |
| T11811_N23 (SEQ. ID NO:301) |

*Table 148 - Proteins of interest*

| Protein Name | Corresponding Transcript(s) |
|---|---|
| T11811_P2 (SEQ. ID NO:303) | T11811_T3 (SEQ. ID NO:276) |
| T11811_P4 (SEQ. ID NO:304) | T11811_T6 (SEQ. ID NO:277) |
| T11811_P7 (SEQ. ID NO:305) | T11811_T12 (SEQ. ID NO:278) |
| T11811_P8 (SEQ. ID NO:306) | T11811_T13 (SEQ. ID NO:279) |
| T11811_P10 (SEQ. ID NO:307) | T11811_T15 (SEQ. ID NO:280) |
| T11811_P15 (SEQ. ID NO:308) | T11811_T24 (SEQ. ID NO:281) |

These sequences are variants of the known protein Myosin regulatory light chain 2 (SEQ. ID NO:302), atrial isoform (SwissProt accession identifier MLRA_HUMAN (SEQ. ID NO: 398); known also according to the synonyms Myosin light chain 2a; MLC-2a; MLC2a; Myosin regulatory light chain 7), referred to herein as the previously known protein.

The sequence for protein Myosin regulatory light chain 2 (SEQ. ID NO:302), atrial isoform is given.

According to optional but preferred embodiments of the present invention, variants of this cluster according to the present invention (amino acid and/or nucleic acid sequences of T11811) may optionally have one or more of the following utilities, as described in greater detail below. It should be noted that these utilities are optionally and preferably suitable for human and non-human animals as subjects, except where otherwise noted. The reasoning is described with regard to biological and/or physiological and/or other information about the known protein, but is given to demonstrate particular diagnostic utility for the variants according to the present invention.

A non-limiting example of such a utility is the detection, diagnosis and/or determination of dilated cardiomyopathy (DCM). The method comprises detecting a T11811 variant, for example a variant protein, protein fragment, peptide, polynucleotide, polynucleotide fragment and/or oligonucleotide as described herein, optionally and preferably in a serum sample. The expression levels of the T11811 variant as determined in a patient can be further compared to those in a normal individual.

Differential expression of the known Myosin regulatory light chain 2 (SEQ. ID NO:302), atrial isoform is described with regard to PCT Application No. WO 03/040407, hereby incorporated by reference as if fully set forth herein. Differential expression was measured in samples taken from healthy cardiac tissue and also from samples taken from patients suffering from DCM, using total or amplified RNA.According to other optional embodiments of the present invention, variants of this cluster according to the present invention (amino acid and/or nucleic acid sequences of T11811) may optionally have one or more of the following utilities, some of which are related to utilities described above. It should be noted that these utilities are optionally and preferably suitable for human and non-human animals as subjects, except where otherwise noted.

The Table below (Table 149) describes diagnostic utilities for the cluster T11811 that were found through microarrays, including the statistical significance thereof and a reference. One or more T11811 variants according to the present invention may optionally have one or more of these utilities.

*Table 149 - Microarray data for T11811*

| | |
|---|---|
| identification of heart cellular damage, due to very high expression in heart. | jp_atlas, GNF1, GNF2, med_all_avg (internal database). |

The following GO Annotation(s) apply to the previously known protein. The following annotation(s) were found: actin filament-based movement; smooth muscle contraction, which are annotation(s) related to Biological Process; ATPase activity, coupled; calcium ion binding; microfilament motor activity, which are annotation (s) related to Molecular Function; and myosin, which are annotation(s) related to Cellular Component.

The GO assignment relies on information from one or more of the SwissProt/TremBl Protein knowledgebase, available from <http://www.expasy.ch/sprot/>; or Locuslink, available from <http://www.ncbi.nlm.nih.gov/projects/LocusLink/>.

As noted above, cluster T11811 features 6 transcript(s), which were listed in Table 146 above. These transcript (s) encode for protein(s) which are variant(s) of protein Myosin regulatory light chain 2 (SEQ. ID NO:302), atrial isoform. A description of each variant protein according to the present invention is now provided.

Variant protein T11811_P2 (SEQ. ID NO:303) according to the present invention is encoded by transcript T11811_T3 (SEQ. ID NO:276). One or more alignments to one or more previously published Myosin regulatory light chain 2 (SEQ. ID NO:302) protein sequences are given in the alignment table on the attached CD-ROM. A brief description of the relationship of the variant protein according to the present invention to each such aligned protein is as follows:

1. Comparison report between T11811_P2 (SEQ. ID NO:303) and MLRA_HUMAN (SEQ. ID NO: 398):

A. An isolated chimeric polypeptide encoding for T11811_P2 (SEQ. ID NO:303), comprising a first amino acid sequence being at least 90% homologous to MASRKAGTRGKVAATKQAQRGSSNVFSMFEQAQIQEFKEAFSCIDQNRDGIICKADLRETYS QLGKVSVPEEELDAMLQEGKGPINFTVFLTLFGEKLNGTDPEEAILSAFRMFDPSGKGVVNKD EFKQLLLTQADKFSPAE corresponding to amino acids 1 - 142 of MLRA_HUMAN (SEQ ID NO: 398), which also corresponds to amino acids 1 - 142 of T11811_P2 (SEQ. ID NO:303), a second amino acid sequence being at least 70%, optionally at least 80%, preferably at least 85%, more preferably at least 90% and most preferably at least 95% homologous to a polypeptide having the sequence VRLPSPFNTHPQHLLWAFTHDPEPSTSEAVAGR (SEQ. ID NO: 390) corresponding to amino acids 143 - 175 of T11811_P2 (SEQ. ID NO:303), and a third amino acid sequence being at least 90% homologous to VEQMFALTPMDLAGNIDYKSLCYIITHGDEKEE corresponding to amino acids 143 - 175 of MLRA_HUMAN (SEQ. ID NO: 398), which also corresponds to amino acids 176 - 208 of T11811_P2 (SEQ. ID NO:303), wherein said first amino acid sequence, second amino acid sequence and third amino acid sequence are contiguous and in a sequential order.
B. An isolated polypeptide encoding for an edge portion of T11811_P2 (SEQ. ID NO:303), comprising an amino acid sequence being at least 70%, optionally at least about 80%, preferably at least about 85%, more preferably at least about 90% and most preferably at least about 95% homologous to the sequence VRLPSPFNTHPQHLLWAFTHDPEPSTSEAVAGR (SEQ. ID NO: 390) of T11811_P2 (SEQ. ID NO:303).

The localization of the variant protein was determined according to results from a number of different software programs and analyses, including analyses from SignalP and other specialized programs. The variant protein is believed to be located intracellularly.
Variant protein T11811_P2 (SEQ. ID NO:303) also has the following non-silent SNPs (Single Nucleotide Polymorphisms) as listed in Table 150, (given according to their position(s) on the amino acid sequence, with the alternative amino acid(s) listed.

*Table 150 - Amino acid mutations*

| SNP position(s) on amino acid sequence | Alternative amino acid(s) |
|---|---|
| 8 | T-> |
| 37 | F -> |
| 54 | K -> |
| 58 | R -> |
| 69 | V -> |
| 77 | M -> I |
| 82 | K -> |
| 90 | F -> |
| 97 | K -> * |
| 97 | K -> |
| 102 | D -> |
| 105 | E -> |
| 118 | G -> R |

Variant protein T11811_P2 (SEQ. ID NO:303) is encoded by the following transcript(s): T11811_T3 (SEQ. ID NO:276), for which the coding portion starts at position 347 and ends at position 970. The transcript also has the following SNPs as listed in Table 151 (given according to their position on the nucleotide sequence, with the alternative nucleic acid listed.

*Table 151 - Nucleic acid SNPs*

| SNP position(s) on nucleotide sequence | Alternative nucleic acid(s) |
|---|---|
| 234 | A -> |
| 237 | G -> A |
| 319 | C -> T |
| 369 | C -> |
| 371 | C -> A |
| 455 | T -> |
| 463 | A -> G |
| 506 | A -> |

(continued)

| SNP position(s) on nucleotide sequence | Alternative nucleic acid(s) |
|---|---|
| 519 | G -> |
| 553 | C -> |
| 553 | C -> T |
| 577 | G -> T |
| 592 | G -> |
| 613 | C -> T |
| 616 | C -> |
| 635 | A -> |
| 635 | A -> T |
| 652 | C -> |
| 660 | A -> |
| 698 | G -> C |
| 988 | C -> |

Variant protein T11811_P4 (SEQ. ID NO:304) according to the present invention is encoded by transcript T11811-_T6 (SEQ. ID NO:277). One or more alignments to one or more previously published Myosin regulatory light chain 2 (SEQ. ID NO:302) protein sequences are given in the alignment table on the attached CD-ROM. A brief description of the relationship of the variant protein according to the present invention to each such aligned protein is as follows:

1. Comparison report between T11811_P4 (SEQ. ID NO:304) and MLRA_HUMAN (SEQ. ID NO: 398):

A. An isolated chimeric polypeptide encoding for T11811_P4 (SEQ. ID NO:304), comprising a first amino acid sequence being at least 90% homologous to

MASRKAGTRGKVAATKQAQRGSSNVFSMFEQAQIQEFKEAFSCIDQNRDGIICKADLRETYS
QLGKVSVPEEELDAMLQEGKGPINFTVFLTLFGEKLNGTDPEEAILSAFRMFDPSGKGVVNKD

corresponding to amino acids 1 - 125 of MLRA_HUMAN (SEQ. ID NO: 398), which also corresponds to amino acids 1 - 125 of T11811_P4 (SEQ. ID NO:304), and a second amino acid sequence being at least 70%, optionally at least 80%, preferably at least 85%, more preferably at least 90% and most preferably at least 95% homologous to a polypeptide having the sequence DQPFPAPWEPPYPPSLCSHSPAVSCSDPPHPPGSSSFS (SEQ. ID NO: 391) corresponding to amino acids 126 - 163 of T11811_P4 (SEQ. ID NO:304), wherein said first amino acid sequence and second amino acid sequence are contiguous and in a sequential order.

B. An isolated polypeptide encoding for an edge portion of T11811_P4 (SEQ. ID NO:304), comprising an amino acid sequence being at least 70%, optionally at least about 80%, preferably at least about 85%, more preferably at least about 90% and most preferably at least about 95% homologous to the sequence DQPFPAPWEPPYPPSLCSHSPAVSCSDPPHPPGSSSFS (SEQ. ID NO: 391) of T11811_P4 (SEQ. ID NO:304).

The localization of the variant protein was determined according to results from a number of different software programs and analyses, including analyses from SignalP and other specialized programs. The variant protein is believed to be located intracellularly.

Variant protein T11811_P4 (SEQ. ID NO:304) also has the following non-silent SNPs (Single Nucleotide Polymorphisms) as listed in Table 152, (given according to their position(s) on the amino acid sequence, with the alternative amino acid(s) listed.

*Table 152 - Amino acid mutations*

| SNP position(s) on amino acid sequence | Alternative amino acid(s) |
|---|---|
| 8 | T -> |
| 37 | F -> |
| 54 | K -> |
| 58 | R -> |
| 69 | V -> |
| 77 | M -> I |
| 82 | K -> |
| 90 | F -> |
| 97 | K -> * |
| 97 | K -> |
| 102 | D -> |
| 105 | E -> |
| 118 | G -> R |
| 138 | P -> S |
| 140 | S -> T |

Variant protein T11811_P4 (SEQ ID NO:304) is encoded by the following transcript(s): T11811_T6 (SEQ ID NO:277), for which the coding portion starts at position 347 and ends at position 835. The transcript also has the following SNPs as listed in Table 153 (given according to their position on the nucleotide sequence, with the alternative nucleic acid listed.

*Table 153 - Nucleic acid SNPs*

| SNP position(s) on nucleotide sequence | Alternative nucleic acid(s) |
|---|---|
| 234 | A -> |
| 237 | G -> A |
| 319 | C -> T |
| 369 | C -> |
| 371 | C -> A |
| 455 | T -> |
| 463 | A -> G |
| 506 | A -> |
| 519 | G -> |
| 553 | C -> |
| 553 | C -> T |
| 577 | G -> T |
| 592 | G -> |
| 613 | C -> T |
| 616 | C -> |
| 635 | A -> |
| 635 | A -> T |

(continued)

| SNP position(s) on nucleotide sequence | Alternative nucleic acid(s) |
|---|---|
| 652 | C -> |
| 660 | A -> |
| 698 | G -> C |
| 758 | C -> T |
| 764 | T -> A |
| 984 | C -> |

Variant protein T11811_P7 (SEQ. ID NO:305) according to the present invention is encoded by transcript T11811_T12 (SEQ. ID NO:278). One or more alignments to one or more previously published Myosin regulatory light chain 2 (SEQ. ID NO:302) protein sequences are given in the alignment table on the attached CD-ROM. A brief description of the relationship of the variant protein according to the present invention to each such aligned protein is as follows:

1. Comparison report between T11811_P7 (SEQ. ID NO:305) and MLRA_HUMAN (SEQ. ID NO: 398):

A. An isolated chimeric polypeptide encoding for T11811_P7 (SEQ. ID NO:305), comprising a first amino acid sequence being at least 90% homologous to MASRKAGTRGKVAATKQAQRGSSNVFSMFEQAQIQEFKE corresponding to amino acids 1 - 39 of MLRA_HUMAN (SEQ. ID NO: 398), which also corresponds to amino acids 1 - 39 of T11811_P7 (SEQ. ID NO:305), a second amino acid sequence being at least 70%, optionally at least 80%, preferably at least 85%, more preferably at least 90% and most preferably at least 95% homologous to a polypeptide having the sequence VSPPPPTFPRAGGCSHLKAPIPQ (SEQ. ID NO: 392) corresponding to amino acids 40 - 62 of T11811_P7 (SEQ. ID NO:305), and a third amino acid sequence being at least 90% homologous to

AFSCIDQNRDGIICKADLRETYSQLGKVSVPEEELDAMLQEGKGPINFTVFLTLFGEKLNGTDP
EEAILSAFRMFDPSGKGVVNKDEFKQLLLTQADKFSPAEVEQMFALTPMDLAGNIDYKSLCYI

ITHGDEKEE corresponding to amino acids 40 - 175 of MLRA_HUMAN (SEQ. ID NO: 398), which also corresponds to amino acids 63 - 198 of T11811_P7 (SEQ. ID NO:305), wherein said first amino acid sequence, second amino acid sequence and third amino acid sequence are contiguous and in a sequential order.

B. An isolated polypeptide encoding for an edge portion of T11811_P7 (SEQ. ID NO:305), comprising an amino acid sequence being at least 70%, optionally at least about 80%, preferably at least about 85%, more preferably at least about 90% and most preferably at least about 95% homologous to the sequence VSPPPPTFPRAGGCSHLKAPIPQ (SEQ. ID NO: 392) of T11811_P7 (SEQ. ID NO:305).

The localization of the variant protein was determined according to results from a number of different software programs and analyses, including analyses from SignalP and other specialized programs. The variant protein is believed to be located intracellularly.

Variant protein T11811_P7 (SEQ. ID NO:305) also has the following non-silent SNPs (Single Nucleotide Polymorphisms) as listed in Table 154, (given according to their position(s) on the amino acid sequence, with the alternative amino acid(s) listed.

*Table 154 - Amino acid mutations*

| SNP position(s) on amino acid sequence | Alternative amino acid(s) |
|---|---|
| 8 | T -> |
| 37 | F -> |
| 77 | K -> |
| 81 | R -> |

(continued)

| SNP position(s) on amino acid sequence | Alternative amino acid(s) |
|---|---|
| 92 | V -> |
| 100 | M -> I |
| 105 | K -> |
| 113 | F -> |
| 120 | K -> * |
| 120 | K -> |
| 125 | D -> |
| 128 | E -> |
| 141 | G -> R |

Variant protein T11811_P7 (SEQ. ID NO:305) is encoded by the following transcript(s): T11811_T12 (SEQ. ID NO:278), for which the coding portion starts at position 347 and ends at position 940. The transcript also has the following SNPs as listed in Table 155 (given according to their position on the nucleotide sequence, with the alternative nucleic acid listed.

*Table 155 - Nucleic acid SNPs*

| SNP position(s) on nucteotide sequence | Alternative nucleic acid(s) |
|---|---|
| 234 | A -> |
| 237 | G -> A |
| 319 | C -> T |
| 369 | C -> |
| 371 | C -> A |
| 455 | T -> |
| 463 | A -> G |
| 575 | A -> |
| 588 | G -> |
| 622 | C -> |
| 622 | C -> T |
| 646 | G -> T |
| 661 | G -> |
| 682 | C -> T |
| 685 | C -> |
| 704 | A -> |
| 704 | A -> T |
| 721 | C -> |
| 729 | A -> |
| 767 | G -> C |
| 958 | C -> |

Variant protein T11811_P8 (SEQ. ID NO:306) according to the present invention is encoded by transcript T11811_T13 (SEQ. ID NO:279). One or more alignments to one or more previously published Myosin regulatory light chain 2 (SEQ. ID NO:302) protein sequences are given in the alignment table on the attached CD-ROM. A brief description of the relationship of the variant protein according to the present invention to each such aligned protein is as follows:

1. Comparison report between T11811_P8 (SEQ. ID N0:306) and MLRA_HUMAN (SEQ. ID NO: 398):

A. An isolated chimeric polypeptide encoding for T11811_P8 (SEQ. ID NO:306), comprising a first amino acid sequence being at least 90% homologous to

MASRKAGTRGKVAATKQAQRGSSNVFSMFEQAQIQEFKEAFSCIDQNRDGIICKADLRETYS
QLGKVSVPEEELDAMLQEGKGPINFTVFLTLFGEKLNGTDPEEAILSAFRMFDPSGKGVVNKD

E corresponding to amino acids 1 - 126 of MLRA_HUMAN (SEQ. ID NO: 398), which also corresponds to amino acids 1 - 126 of T11811_P8 (SEQ. ID NO:306), and a second amino acid sequence being at least 70%, optionally at least 80%, preferably at least 85%, more preferably at least 90% and most preferably at least 95% homologous to a polypeptide having the sequence WSRCSP (SEQ. ID NO: 393) corresponding to amino acids 127 - 132 of T11811_P8 (SEQ. ID NO:306), wherein said first amino acid sequence and second amino acid sequence are contiguous and in a sequential order.

B. An isolated polypeptide encoding for an edge portion of T11811_P8 (SEQ. ID NO:306), comprising an amino acid sequence being at least 70%, optionally at least about 80%, preferably at least about 85%, more preferably at least about 90% and most preferably at least about 95% homologous to the sequence WSRCSP (SEQ. ID NO: 393) of T11811_P8 (SEQ. ID NO:306).

The localization of the variant protein was determined according to results from a number of different software programs and analyses, including analyses from SignalP and other specialized programs. The variant protein is believed to be located intracellularly.

Variant protein T11811_P8 (SEQ. ID NO:306) also has the following non-silent SNPs (Single Nucleotide Polymorphisms) as listed in Table 156, (given according to their position(s) on the amino acid sequence, with the alternative amino acid(s) listed.

*Table 156 - Amino acid mutations*

| SNP position(s) on amino acid sequence | Alternative amino acid(s) |
|---|---|
| 8 | T-> |
| 37 | F -> |
| 54 | K -> |
| 58 | R -> |
| 69 | V -> |
| 77 | M -> I |
| 82 | K -> |
| 90 | F -> |
| 97 | K -> * |
| 97 | K -> |
| 102 | D -> |
| 105 | E -> |
| 118 | G -> R |

Variant protein T11811_P8 (SEQ. ID NO:306) is encoded by the following transcript(s): T11811_T13 (SEQ. ID NO:279), for which the coding portion starts at position 347 and ends at position 742. The transcript also has the following SNPs as listed in Table 157 (given according to their position on the nucleotide sequence, with the alternative nucleic acid listed.

*Table 157 - Nucleic acid SNPs*

| SNP position(s) on nucleotide sequence | Alternative nucleic acid(s) |
|---|---|
| 234 | A -> |
| 237 | G -> A |
| 319 | C -> T |
| 369 | C -> |
| 371 | C -> A |
| 455 | T -> |
| 463 | A -> G |
| 506 | A -> |
| 519 | G -> |
| 553 | C -> |
| 553 | C -> T |
| 577 | G -> T |
| 592 | G -> |
| 613 | C -> T |
| 616 | C -> |
| 635 | A -> |
| 635 | A -> T |
| 652 | C -> |
| 660 | A -> |
| 698 | G -> C |
| 840 | C -> |

Variant protein T11811_P10 (SEQ. ID NO:307) according to the present invention is encoded by transcript T11811_T15 (SEQ. ID NO:280).

The localization of the variant protein was determined according to results from a number of different software programs and analyses, including analyses from SignalP and other specialized programs. The variant protein is believed to be located intracellularly.

Variant protein T11811_P10 (SEQ. ID NO:307) also has the following non-silent SNPs (Single Nucleotide Polymorphisms) as listed in Table 158, (given according to their position(s) on the amino acid sequence, with the alternative amino acid(s) listed.

*Table 158 - Amino acid mutations*

| SNP position(s) on amino acid sequence | Alternative amino acid(s) |
|---|---|
| 8 | T -> |
| 37 | F -> |
| 54 | K -> |
| 58 | R -> |
| 77 | P -> |
| 100 | P -> |
| 100 | P -> S |

(continued)

| SNP position(s) on amino acid sequence | Alternative amino acid(s) |
|---|---|
| 108 | A -> S |
| 113 | G -> |
| 120 | L -> F |
| 121 | P -> |
| 127 | E -> |
| 127 | E -> V |
| 133 | P -> |
| 135 | G -> |

Variant protein T11811_P10 (SEQ. ID NO:307) is encoded by the following transcript(s): T11811_T15 (SEQ. ID NO:280), for which the coding portion starts at position 347 and ends at position 778. The transcript also has the following SNPs as listed in Table 159 (given according to their position on the nucleotide sequence, with the alternative nucleic acid listed.

*Table 159 - Nucleic acid SNPs*

| SNP position(s) on nucleotide sequence | Alternative nucleic acid(s) |
|---|---|
| 234 | A -> |
| 237 | G -> A |
| 319 | C -> T |
| 369 | C -> |
| 371 | C -> A |
| 455 | T -> |
| 463 | A -> G |
| 506 | A -> |
| 519 | G-> |
| 576 | C -> |
| 577 | C -> |
| 644 | C -> |
| 644 | C -> T |
| 668 | G -> T |
| 683 | G -> |
| 704 | C -> T |
| 707 | C -> |
| 726 | A -> |
| 726 | A -> T |
| 743 | C -> |
| 751 | A -> |
| 789 | G -> C |
| 980 | C -> |

Variant protein T11811_P15 (SEQ. ID NO:308) according to the present invention is encoded by transcript T11811_T24 (SEQ. ID NO:281).

The localization of the variant protein was determined according to results from a number of different software programs and analyses, including analyses from SignalP and other specialized programs. The variant protein is believed to be located intracellularly.

Variant protein T11811_P15 (SEQ. ID NO:308) also has the following non-silent SNPs (Single Nucleotide Polymorphisms) as listed in Table 160, (given according to their position(s) on the amino acid sequence, with the alternative amino acid(s) listed.

*Table 160 - Amino acid mutations*

| SNP position(s) on amino acid sequence | Alternative amino acid(s) |
|---|---|
| 8 | T -> |
| 37 | F -> |
| 77 | K -> |
| 81 | R -> |

Variant protein T11811_P15 (SEQ. ID NO:308) is encoded by the following transcript(s): T11811_T24 (SEQ. ID NO:281), for which the coding portion starts at position 347 and ends at position 652. The transcript also has the following SNPs as listed in Table 161 (given according to their position on the nucleotide sequence, with the alternative nucleic acid listed.

*Table 161 - Nucleic acid SNPs*

| SNP position(s) on nucleotide sequence | Alternative nucleic acid(s) |
|---|---|
| 234 | A -> |
| 237 | G -> A |
| 319 | C -> T |
| 369 | C -> |
| 371 | C -> A |
| 455 | T -> |
| 463 | A -> G |
| 575 | A -> |
| 588 | G -> |
| 693 | T -> C |
| 736 | C -> T |
| 758 | C->G |
| 758 | C -> T |
| 839 | C -> |
| 840 | C -> |
| 907 | C -> |
| 907 | C -> T |
| 931 | G->T |
| 946 | G -> |
| 967 | C -> T |
| 970 | C -> |
| 989 | A -> |

(continued)

| SNP position(s) on nucleotide sequence | Alternative nucleic acid(s) |
|---|---|
| 989 | A -> T |
| 1006 | C -> |
| 1014 | A -> |
| 1052 | G -> C |
| 1243 | C -> |

As noted above, cluster T11811 features 20 segment(s), which were listed in Table 147 above and for which the sequence(s) are given. These segment(s) are portions of nucleic acid sequence(s) which are described herein separately because they are of particular interest. A description of several segments according to the present invention is now provided.

Segment cluster T11811_N10 (SEQ. ID NO:283) according to the present invention is supported by 17 libraries. The number of libraries was determined as previously described. This segment can be found in the following transcript (s): T11811_T24 (SEQ. ID NO:281). Table 162 below describes the starting and ending position of this segment on each transcript.

*Table 162 - Segment location on transcripts*

| Transcript name | Segment starting position | Segment ending position |
|---|---|---|
| T11811 T24 (SEQ. ID NO:281) | 609 | 802 |

Segment cluster T11811_N26 (SEQ. ID NO:284) according to the present invention is supported by 54 libraries. The number of libraries was determined as previously described. This segment can be found in the following transcript (s): T11811_T12 (SEQ. ID NO:278), T11811_T13 (SEQ. ID NO:279), T11811_T15 (SEQ. ID NO:280), T11811_T24 (SEQ. ID NO:281), T11811_T3 (SEQ. ID NO:276) and T11811_T6 (SEQ. ID NO:277). Table 163 below describes the starting and ending position of this segment on each transcript.

*Table 163 - Segment location on transcripts*

| transcript name | Segment starting position | Segment ending position |
|---|---|---|
| T11811_T12 (SEQ. ID NO:278) | 842 | 999 |
| T11811_T13 (SEQ. ID NO:279) | 724 | 881 |
| T11811_T15 (SEQ. ID NO:280) | 864 | 1021 |
| T11811_T24 (SEQ. ID NO:281) | 1127 | 1284 |
| T11811_T3 (SEQ. ID NO:276) | 872 | 1029 |
| T11811_T6 (SEQ. ID NO:277) | 868 | 1025 |

According to an optional embodiment of the present invention, short segments related to the above cluster are also provided. These segments are up to about 120 bp in length, and so are included in a separate description.

Segment cluster T11811_N7 (SEQ. ID NO:288) according to the present invention is supported by 22 libraries. The number of libraries was determined as previously described. This segment can be found in the following transcript (s): T11811_T12 (SEQ. ID NO:278) and T11811_T24 (SEQ. ID NO:281). Table 164 below describes the starting and ending position of this segment on each transcript.

*Table 164 - Segment location on transcripts*

| Transcript name | Segment starting position | Segment ending position |
|---|---|---|
| T11811_T12 (SEQ. ID NO:278) | 464 | 492 |
| T11811_T24 (SEQ. ID NO:281) | 464 | 492 |

Segment cluster T11811_N8 (SEQ. ID NO:289) according to the present invention is supported by 26 libraries. The number of libraries was determined as previously described. This segment can be found in the following transcript (s): T11811_T12 (SEQ. ID NO:278) and T11811_T24 (SEQ. ID NO:281). Table 165 below describes the starting and ending position of this segment on each transcript.

*Table 165 - Segment location on transcripts*

| Transcript name | Segment starting position | Segment ending position |
|---|---|---|
| T11811_T12 (SEQ. ID NO:278) | 493 | 532 |
| T11811_T24 (SEQ. ID NO:281) | 493 | 532 |

Segment cluster T11811_N9 (SEQ. ID NO:290) according to the present invention is supported by 64 libraries. The number of libraries was determined as previously described. This segment can be found in the following transcript (s): T11811_T12 (SEQ. ID NO:278), T11811_T13 (SEQ. ID NO:279), T11811_T15 (SEQ. ID N0:280), T11811_T24 (SEQ. ID NO:281), T11811_T3 (SEQ. ID NO:276) and T11811_T6 (SEQ. ID NO:277). Table 166 below describes the starting and ending position of this segment on each transcript.

*Table 166 - Segment location on transcripts*

| Transcript name | Segment starting position | Segment ending position |
|---|---|---|
| T11811_T12 (SEQ. ID NO:278) | 533 | 608 |
| T11811_T13 (SEQ. ID NO:279) | 464 | 539 |
| T11811_T15 (SEQ. ID NO:280) | 464 | 539 |
| T11811_T24(SEQ.ID NO:281) | 533 | 608 |
| T11811_T3 (SEQ.ID NO:276) | 464 | 539 |
| T11811_T6 (SEQ. ID NO:277) | 464 | 539 |

Segment cluster T11811_N11 (SEQ. ID NO:291) according to the present invention is supported by 14 libraries. The number of libraries was determined as previously described. This segment can be found in the following transcript (s): T11811-_T15 (SEQ. ID N0:280) and T11811_T24 (SEQ. ID NO:281). Table 167 below describes the starting and ending position of this segment on each transcript.

*Table 167 - Segment location on transcripts*

| Transcript name | Segment starting position | Segment ending position |
|---|---|---|
| T11811_T15 (SEQ. ID NO:280) | 540 | 563 |
| T11811_T24 (SEQ. ID NO:281) | 803 | 826 |

Segment cluster T11811_N12 (SEQ. ID NO:292) according to the present invention is supported by 13 libraries. The number of libraries was determined as previously described. This segment can be found in the following transcript (s): T11811_T15 (SEQ. ID NO:280) and T11811_T24 (SEQ. ID NO:281). Table 168 below describes the starting and ending position of this segment on each transcript.

*Table 168 - Segment location on transcripts*

| Transcript name | Segment starting position | Segment ending position |
|---|---|---|
| T11811_T15 (SEQ. ID NO:280) | 564 | 600 |
| T11811_T24 (SEQ. ID NO:281) | 827 | 863 |

Segment cluster T11811_N13 (SEQ. ID NO:293) according to the present invention is supported by 10 libraries. The number of libraries was determined as previously described. This segment can be found in the following transcript (s): T11811_T15 (SEQ. ID NO:280) and T11811_T24 (SEQ. ID NO:281). Table 169 below describes the starting and ending position of this segment on each transcript.

*Table 169 - Segment location on transcripts*

| Transcript name | Segment starting position | Segment ending position |
|---|---|---|
| T11811_T15 (SEQ. ID NO:280) | 601 | 630 |
| T11811_T24 (SEQ. ID NO:281) | 864 | 893 |

Segment cluster T11811_N14 (SEQ. ID NO:294) according to the present invention is supported by 57 libraries. The number of libraries was determined as previously described. This segment can be found in the following transcript (s): T11811_T12 (SEQ. ID NO:278), T11811_T13 (SEQ. ID NO:279), T11811_T15 (SEQ. ID NO:280), T11811_T24 (SEQ. ID NO:281), T11811_T3 (SEQ. ID NO:276) and T11811_T6 (SEQ. ID NO:277). Table 170 below describes the starting and ending position of this segment on each transcript.

*Table 170 - Segment location on transcripts*

| Transcript name | Segment starting position | Segment ending position |
|---|---|---|
| T11811_T12 (SEQ. ID NO:278) | 609 | 693 |
| T11811_T13 (SEQ. ID NO:279) | 540 | 624 |
| T11811_T15 (SEQ. ID NO:280) | 631 | 715 |
| T11811_T24 (SEQ. ID NO:281) | 894 | 978 |
| T11811_T3 (SEQ. ID NO:276) | 540 | 624 |
| T11811_T6 (SEQ. ID NO:277) | 540 | 624 |

Segment cluster T11811_N18 (SEQ. ID NO:297) according to the present invention is supported by 61 libraries. The number of libraries was determined as previously described. This segment can be found in the following transcript (s): T11811_T12 (SEQ. ID NO:278), T11811_T13 (SEQ. ID NO:279), T11811_T15 (SEQ. ID NO:280), T11811_T24 (SEQ. ID NO:281), T11811_T3 (SEQ. ID NO:276) and T11811_T6 (SEQ. ID NO:277). Table 171 below describes the starting and ending position of this segment on each transcript.

*Table 171 - Segment location on transcripts*

| Transcript name | Segment starting position | Segment ending position |
|---|---|---|
| T11811_T12 (SEQ. ID NO:278) | 720 | 792 |
| T11811_T13 (SEQ. ID NO:279) | 651 | 723 |
| T11811_T15 (SEQ. ID NO:280) | 742 | 814 |
| T11811_T24 (SEQ. ID NO:281) | 1005 | 1077 |
| T11811_T3 (SEQ. ID NO:276) | 651 | 723 |
| T11811_T6 (SEQ. ID NO:277) | 651 | 723 |

Segment cluster T11811_N20 (SEQ. ID NO:298) according to the present invention is supported by 7 libraries. The number of libraries was determined as previously described. This segment can be found in the following transcript (s): T11811_T6 (SEQ. ID NO:277). Table 172 below describes the starting and ending position of this segment on each transcript.

*Table 172 - Segment location on transcripts*

| Transcript name | Segment starting position | Segment ending position |
|---|---|---|
| T11811 T6 (SEQ. ID NO:277) | 724 | 818 |

Segment cluster T11811_N22 (SEQ. ID NO:300) according to the present invention is supported by 58 libraries. The number of libraries was determined as previously described. This segment can be found in the following transcript(s): T11811_T12 (SEQ. ID NO:278), T11811_T15 (SEQ. ID NO:280), T11811_T24 (SEQ. ID NO:281), T11811_T3 (SEQ. ID NO:276) and T11811_T6 (SEQ. ID NO:277). Table 173 below describes the starting and ending position of this segment on each transcript.

*Table 173 - Segment location on transcripts*

| Transcript name | Segment starting position | Segment ending position |
|---|---|---|
| T11811 T12 (SEQ. ID NO:278) | 809 | 841 |
| T11811_T15 (SEQ. ID NO:280) | 831 | 863 |
| T11811_T24 (SEQ. ID NO:281) | 1094 | 1126 |
| T11811_T3 (SEQ. ID NO:276) | 740 | 772 |
| T1181_T6 (SEQ. ID NO:277) | 835 | 867 |

Segment cluster T11811_N23 (SEQ. ID NO:301) according to the present invention is supported by 7 libraries. The number of libraries was determined as previously described. This segment can be found in the following transcript (s): T11811_T3 (SEQ. ID NO:276). Table 174 below describes the starting and ending position of this segment on each transcript.

*Table 174 - Segment location on transcripts*

| Transcript name | Segment starting position | Segment ending position |
|---|---|---|
| T11811_T3 (SEQ. ID NO:276) | 773 | 871 |

Expression of Homo sapiens myosin, light polypeptide 7, regulatory (MYL7) T11811 transcripts which are detectable by amplicon as depicted in sequence name T11811_seg14WT (SEQ. ID NO: 311) specifically in heart tissue

Expression of Homo sapiens myosin, light polypeptide 7, regulatory (MYL7) transcripts detectable by or according to seg14WT - T11811_seg14WT (SEQ. ID NO: 311) amplicon and primers T11811_seg14WTF (SEQ. ID NO: 309) and T11811_seg14WTR (SEQ. ID NO: 310) was measured by real time PCR. In parallel the expression of four housekeeping genes - SDHA (GenBank Accession No. NM_004168 (SEQ. ID NO: 33); amplicon - SDHA-amplicon (SEQ. ID NO:36)), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24)) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the average of the quantities of the heart samples (sample numbers 44, 45 and 46, Table 1_6 above), to obtain a value of relative expression of each sample relative to average of the heart samples.

Figure 30 is a histogram showing relative expression of the above-indicated Homo sapiens myosin, light polypeptide 7, regulatory (MYL7) transcripts in heart tissue samples as opposed to other tissues.

As is evident from Figure 30, the expression of Homo sapiens myosin, light polypeptide 7, regulatory (MYL7) transcripts detectable by the above amplicon in 2 of the heart tissue samples was significantly higher than in the other samples (sample numbers 1, 2, 3, 4, 5, 6, 7, 8, 9, 10, 11, 12, 13, 14, 15, 16, 17, 19, 20, 75, 76, 77, 78, 21, 23, 24, 25, 26, 28, 29, 30, 31, 32, 33, 34, 35, 36, 37, 38, 39, 40, 41, 42, 43, 47, 48, 49, 50, 51, 52, 53, 54, 55, 57, 58, 59, 60, 61, 62, 63, 64, 65, 66, 67, 68, 69, 70, 71, 72, 73 and 74, Table 1_6 above).

Primer pairs are also optionally and preferably encompassed within the present invention; for example, for the above experiment, the following primer pair was used as a non-limiting illustrative example only of a suitable primer pair: T11811_seg14WTF (SEQ. ID NO: 309) forward primer; and T11811_seg14WTR (SEQ. ID NO: 310) reverse primer.

The present invention also preferably encompasses any amplicon obtained through the use of any suitable primer pair; for example, for the above experiment, the following amplicon was obtained as a non-limiting illustrative example only of a suitable amplicon: T11811_seg14WT (SEQ. ID NO: 311).

Forward Primer (T11811_seg14WTF (SEQ. ID NO: 309)):GGAAGGTGAGTGTCCCAGAGG

Reverse Primer (T11811_seg14WTR (SEQ. ID NO: 310)):GTGAGGAAGACGGTGAAGTTGAT

Amplicon (T11811_seg14WT (SEQ. ID NO: 311)):

GGAAGGTGAGTGTCCCAGAGGAGGAGCTGGACGCCATGCTGCAAGAGGGCAAGG
GCCCCATCAACTTCACCGTCTTCCTCAC

Expression of Homo sapiens myosin, light polypeptide 7, regulatory (MYL7) T11811 transcripts which are detectable by amplicon as depicted in sequence name T11811_seg7-8-9 (SEQ. ID NO: 314) specifically in heart tissue

Expression of Homo sapiens myosin, light polypeptide 7, regulatory (MYL7) transcripts detectable by or according to seg7-8-9 - T11811_seg7-8-9 (SEQ. ID NO: 314) amplicon and primers T11811_seg7-8-9F (SEQ. ID NO: 312) and T11811_seg7-8-9R (SEQ. ID NO: 313) was measured by real time PCR. In parallel the expression of four housekeeping genes - SDHA (GenBank Accession No. NM_004168 (SEQ. ID NO: 33); amplicon - SDHA-amplicon (SEQ. ID NO:36) ), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24)) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the avarage of the quantities of the heart samples (sample numbers 44 and 45, Table 1_6 above), to obtain a value of relative expression of each sample relative to avarage of the heart samples.

Figure 31 is a histogram showing relative expression of the above-indicated Homo sapiens myosin, light polypeptide 7, regulatory (MYL7) transcripts in heart tissue samples as opposed to other tissues.

As is evident from Figure 31, the expression of Homo sapiens myosin, light polypeptide 7, regulatory (MYL7) transcripts detectable by the above amplicon in 2 of the heart tissue samples was significantly higher than in the other samples (sample numbers 1, 2, 3, 4, 5, 6, 7, 8, 9, 10, 11, 12, 13, 14, 15, 16, 17, 19, 20, 75, 76, 77, 78, 21, 23, 24, 25, 26, 28, 29, 30, 31, 32, 33, 34, 35, 36, 37, 38, 39, 40, 41, 42, 43, 47, 48, 49, 50, 51, 52, 53, 54, 55, 57, 58, 59, 60, 61, 62, 63, 64, 65, 66, 67, 68, 69, 70, 71, 72, 73 and 74, Table 1_6 above).

Primer pairs are also optionally and preferably encompassed within the present invention; for example, for the above experiment, the following primer pair was used as a non-limiting illustrative example only of a suitable primer pair: T11811_seg7-8-9F (SEQ. ID NO: 312) forward primer; and T11811_seg7-8-9R (SEQ. ID NO: 313) reverse primer.

The present invention also preferably encompasses any amplicon obtained through the use of any suitable primer pair; for example, for the above experiment, the following amplicon was obtained as a non-limiting illustrative example only of a suitable amplicon: T11811_seg7-8-9 (SEQ. ID NO: 314).

Forward Primer (T11811_seg7-8-9F (SEQ. ID NO: 312)):CCCACCTTCCCTAGAGCTGG

Reverse Primer (T11811_seg7-8-9R (SEQ. ID NO: 313)):AGGTCTGCCTTGCAGATGATG

Amplicon (T11811_seg7-8-9 (SEQ. ID NO: 314)):

CCCACCTTCCCTAGAGCTGGGGGCTGCTCCCACCTGAAGGCCCCCATCCCACAGG
CCTTCAGCTGTATCGACCAGAATCGTGATGGCATCATCTGCAAGGCAGACCT

Expression of Homo sapiens myosin, light polypeptide 7, regulatory (MYL7) T11811 transcripts which are detectable by amplicon as depicted in sequence name T11811_seg23 (SEQ. ID NO: 317) specifically in heart tissue

Expression of Homo sapiens myosin, light polypeptide 7, regulatory (MYL7) transcripts detectable by or according to seg23 - T11811_seg23_F2R2 (SEQ. ID NO: 317) amplicon and primers T11811_seg23F2 (SEQ. ID NO: 315) and T11811_seg23R2 (SEQ. ID NO: 316) was measured by real time PCR. In parallel the expression of four housekeeping genes - SDHA (GenBank Accession No. NM_004168 (SEQ. ID NO: 33); amplicon - SDHA-amplicon (SEQ. ID NO:36)), Ubiquitin (GenBank Accession No. BC000449 (SEQ. ID NO: 29); amplicon - Ubiquitin-amplicon (SEQ. ID NO: 32)), RPL19 (GenBank Accession No. NM_000981 (SEQ. ID NO: 21); RPL19 amplicon (SEQ. ID NO: 24) ) and TATA box (GenBank Accession No. NM_003194 (SEQ. ID NO: 25); TATA amplicon (SEQ. ID NO: 28)) was measured similarly. For each RT sample, the expression of the above amplicon was normalized to the geometric mean of the quantities of the housekeeping genes. The normalized quantity of each RT sample was then divided by the avarage of the quantities of the heart samples (sample numbers 44, 45 and 46, Table 1_6 above), to obtain a value of relative expression of each sample relative to avarage of the heart samples.

Figure 32 is a histogram showing relative expression of the above-indicated Homo sapiens myosin, light polypeptide 7, regulatory (MYL7) transcripts in heart tissue samples as opposed to other tissues.

As is evident from Figure 32, the expression of Homo sapiens myosin, light polypeptide 7, regulatory (MYL7) transcripts detectable by the above amplicon in 2 of the heart tissue samples was significantly higher than in the other samples (sample numbers 1, 2, 3, 4, 5, 6, 7, 8, 9, 10, 11, 12, 13, 14, 15, 16, 17, 19, 20, 75, 76, 77, 78, 21, 23, 24, 25, 26, 28, 29, 30, 31, 32, 33, 34, 35, 36, 37, 38, 39, 40, 41, 42, 43, 47, 48, 49, 50, 51, 52, 53, 54, 55, 57, 58, 59, 60, 61, 62, 63, 64, 65, 66, 67, 68, 69, 70, 71, 72, 73 and 74, Table 1_6 above).

Primer pairs are also optionally and preferably encompassed within the present invention; for example, for the above experiment, the following primer pair was used as a non-limiting illustrative example only of a suitable primer pair: T11811_seg23F2 (SEQ. ID NO: 315) forward primer; and T11811_seg23R2 (SEQ. ID NO: 316) reverse primer.

The present invention also preferably encompasses any amplicon obtained through the use of any suitable primer pair; for example, for the above experiment, the following amplicon was obtained as a non-limiting illustrative example only of a suitable amplicon: T11811_seg23F2R2 (SEQ. ID NO: 317).

Forward Primer (T11811_seg23F2 (SEQ. ID NO: 315)):AGGCAGACAAGTTCTCTCCAGCT

Reverse Primer (T11811_seg23R2 (SEQ. ID NO: 316)):GGTGAAGGCCCAGAGAAGG

Amplicon (T11811 _seg23_F2R2 (SEQ. ID NO: 317)):

AGGCAGACAAGTTCTCTCCAGCTGAGGTGAGGCTGCCCAGCCCCTTCAATACTCATCCCC
AGCACCTTCTCTGGGCCTTCACC

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. An isolated polypeptide comprising an amino acid sequence at least about 70%, 80%, 85%, 90% or 95% homologous to SEQ ID NO:58, preferably wherein the isolated polypeptide has an amino acid sequence as set forth in SEQ ID NO:58.

2. An isolated polynucleotide comprising a nucleic acid sequence set forth in SEQ ID NO:38 and homologues and fragments thereof,
preferably wherein the nucleic acid sequence encodes a polypeptide having an amino acid sequence set forth in SEQ ID NO:58; or
preferably wherein the nucleic acid sequence encodes a polypeptide comprising contiguous amino acids having at least about 70% homology to SEQ ID NO:327; or
preferably wherein the nucleic acid sequence encodes a polypeptide comprising contiguous amino acids having at least about 80% homology to SEQ ID NO:327; or preferably wherein the nucleic acid sequence encodes a polypeptide comprising contiguous amino acids having at least about 85% homology to SEQ ID NO:327; or
preferably wherein the nucleic acid sequence encodes a polypeptide comprising contiguous amino acids having at least about 90% homology to SEQ ID NO:327; or
preferably wherein the nucleic acid sequence encodes a polypeptide comprising contiguous amino acids having at least about 95% homology to SEQ ID NO:327; or
preferably wherein the nucleic acid sequence encodes a polypeptide comprising contiguous amino acids as set forth in SEQ ID NO:327.

3. An isolated polypeptide having an amino acid sequence encoded by any one of the nucleic acid sequence of claim 1. preferably wherein the polypeptide comprises a first portion having an amino acid sequence being at least about 90% homologous to amino acids 1 - 23 of SEQ ID NO:58, and a second portion having amino acid sequence being at least about 70%, 80%, 85%, 90% ,95% or 100% homologous to SEQ ID NO:327, wherein the first amino acid sequence and the second amino acid sequence are contiguous and in a sequential order.

4. An antibody which binds to at least one epitope of a polypeptide having an amino acid sequence according to any of claims 1 and 3,
preferably wherein said antibody is capable of differentiating between a polypeptide having the epitope and a corresponding known protein.

5. An isolated polynucleotide, comprising a nucleic acid sequence complementary to any one of the nucleic acid sequences of claims 2; or

comprising a nucleic acid sequence that hybridizes under stringent conditions to any one of the nucleic acid sequences of claims 2.

**6.** An expression vector comprising any one of the polynucleotide sequences according to claim 2.

**7.** A host cell comprising the vector according to claim 6.

**8.** A process for producing a polypeptide comprising:

culturing the host cell according to claim 7 under conditions suitable to produce the polypeptide encoded by said polynucleotide; and
recovering said polypeptide.

**9.** An isolated polynucleotide segment, consisting of a nucleic acid sequence as set forth in SEQ ID NO:48.

**10.** An isolated polynucleotide segment consisting of a nucleic acid sequence complementary to the nucleic acid sequence of claim 9; or
consisting of a nucleic acid sequence that hybridizes under stringent conditions to the nucleic acid sequence of claim 9.

**11.** A kit for detecting cancer, comprising a marker capable of detecting a DLL3 variant or a polynucleotide encoding same selected from the group consisting of:

a polypeptide according to any of claims 1, 3; or
a polynucleotide according to claims 2 or 9.

**12.** The kit of claim 11,
wherein the cancer is lung cancer; or
wherein the cancer is invasive or metastatic; or
wherein said kit comprises an antibody according to claim 7 and wherein said kit further comprises at least one reagent for performing an immunoassay, preferably wherein said immunoassay is selected from the group consisting of an ELISA, a RIA (radio immunoassay), a slot blot, immunohistochemical assay, FACS (fluorescence activated cell sorting), a radio-imaging assay or a Western blot.

**13.** The kit of claim 12, wherein said kit comprises at least one nucleotide probe or primer pair,
preferably wherein said kit comprises
at least one oligonucleotide, probe or primer pair capable of selectively hybridizing to a polynucleotide according to any of claims 2 or 9; or
wherein said at least one primer pair amplifies an amplicon comprising a nucleic acid sequence as set forth in any one of SEQ ID NOs:67 and 70, or optionally preferably comprising a pair of isolated oligonucleotides having a sequence of SEQ ID NOs:65 and 66; or SEQ ID NOs:66 and 69; or
wherein the probe has a nucleic acid sequence selected from the group consisting of SEQ ID NOs: 64, 68.

**14.** A method for at least one of detecting cancer, monitoring cancer progression, monitoring cancer-treatment efficacy, detecting acute or chronic exacerbation of cancer and selecting a therapy for cancer, comprising detecting in a sample differential expression of at least one DLL3 variant or at least one polynucleotide encoding same selected from the group consisting of: a polypeptide according to any of claims 1, 3 or a polynucleotide according to claims 2 or 9,
preferably wherein the cancer is lung cancer; or
preferably wherein said cancer is invasive or metastatic; or
preferably wherein detecting the differential expression of the polypeptide is performed with the antibody of claim 4; or preferably wherein detecting the differential expression of the at least one polynucleotide is performed with the pair of isolated oligonucleotides having a sequence of SEQ ID NOs:65 and 66; or SEQ ID NOs:66 and 69; or with the probe has a nucleic acid sequence selected from the group consisting of SEQ ID NOs: 64, 68; or performed by using a kit for detecting cancer according to claim 11, in a sample obtained from a subject.

**15.** The kit or method of any one of claims 11-14, wherein the polynucleotide or polypeptide is detected in a sample selected from the group consisting of blood, serum, plasma, urine, sputum, saliva, stool, spinal fluid or CSF, lymph fluid, the external secretions of the skin, respiratory, intestinal, and genitourinary tracts, any human organ or tissue, any sample obtained by lavage optionally of the bronchial system, or samples of in vivo cell culture constituents.

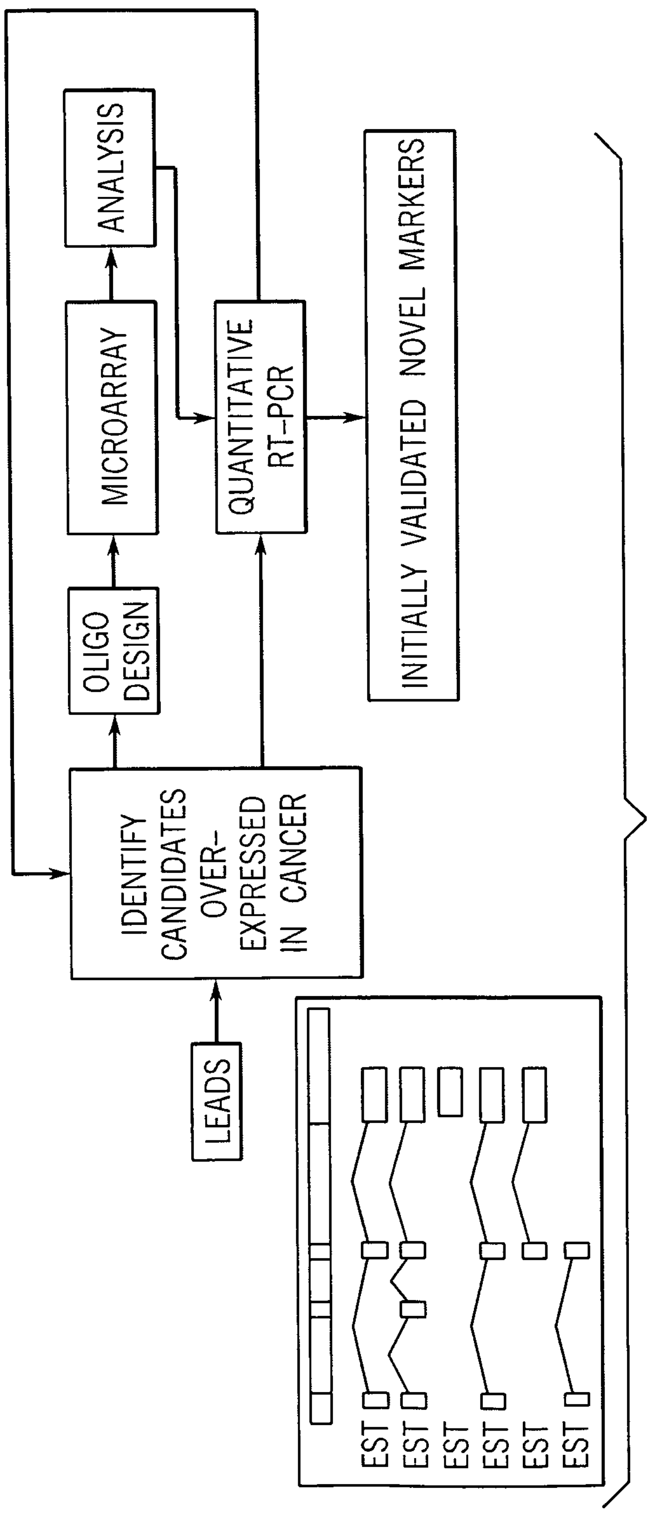
LEADS
IDENTIFY CANDIDATES OVER-EXPRESSED IN CANCER
OLIGO DESIGN
MICROARRAY
ANALYSIS
QUANTITATIVE RT-PCR
INITIALLY VALIDATED NOVEL MARKERS
EST
EST
EST
EST
EST
EST

FIG. 1

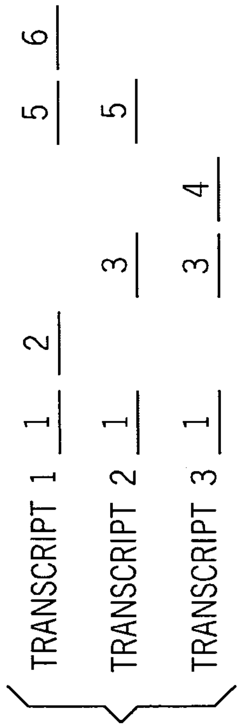
TRANSCRIPT 1
1
2
5
6
TRANSCRIPT 2
1
3
5
TRANSCRIPT 3
1
3
4

FIG. 2

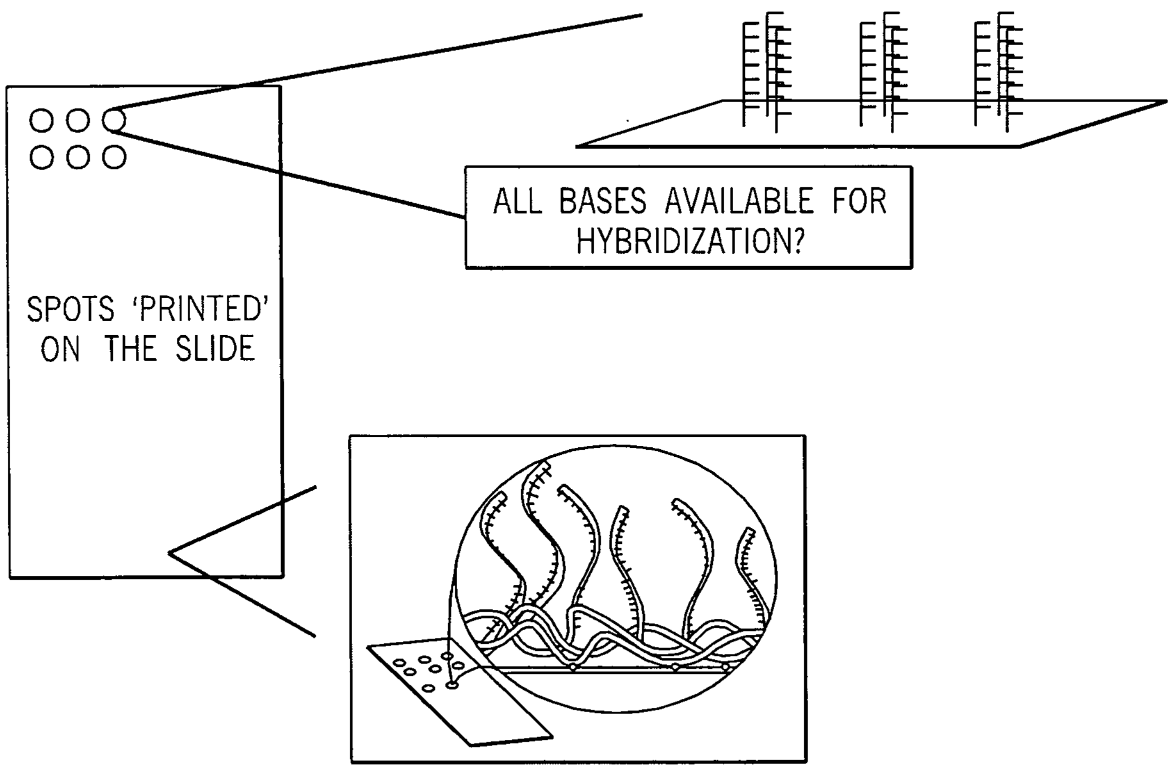
ALL BASES AVAILABLE FOR HYBRIDIZATION?
SPOTS 'PRINTED' ON THE SLIDE

FIG. 3

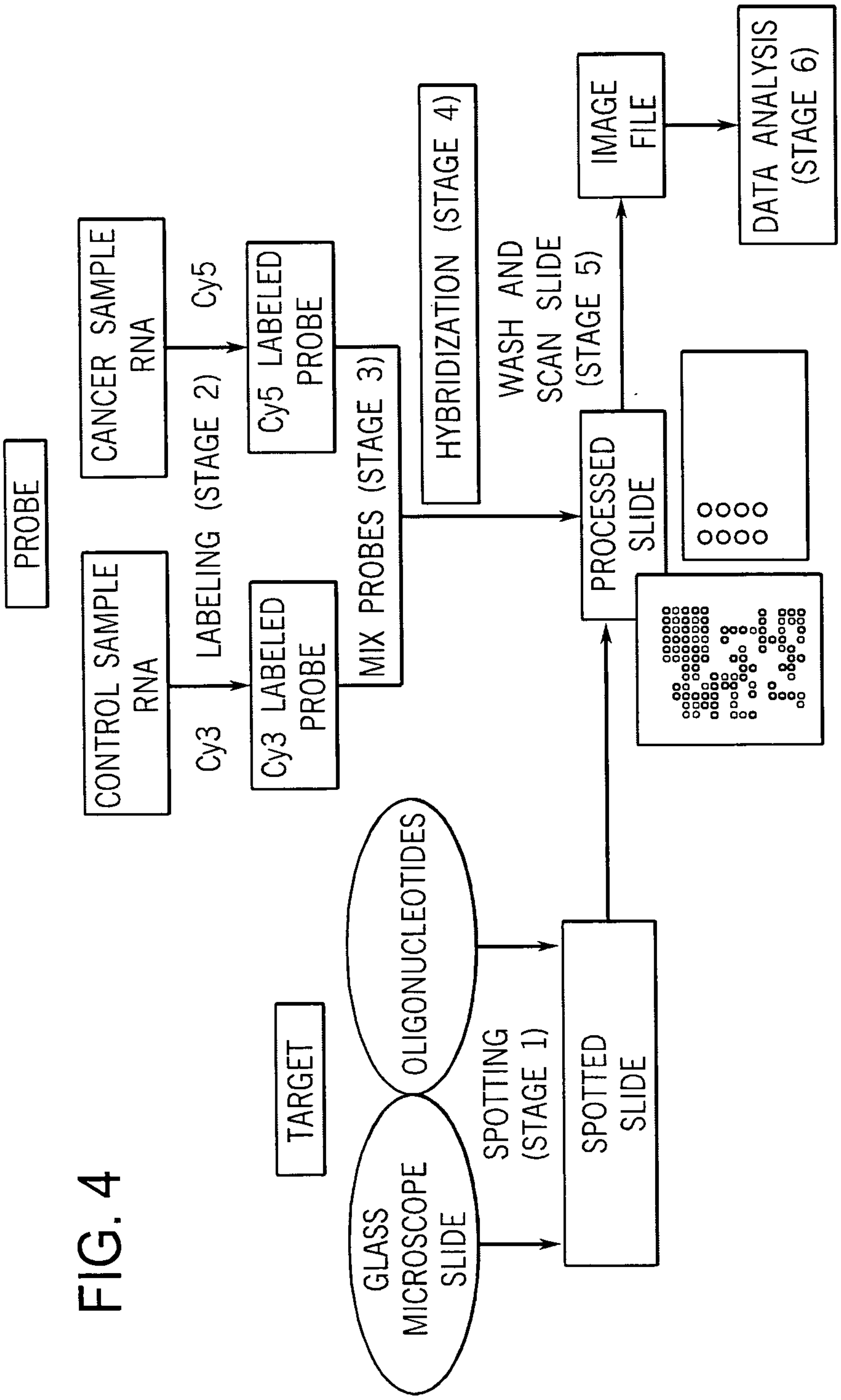
PROBE
CONTROL SAMPLE RNA
CANCER SAMPLE RNA
Cy3
LABELING (STAGE 2)
Cy5
Cy3 LABELED PROBE
Cy5 LABELED PROBE
MIX PROBES (STAGE 3)
HYBRIDIZATION (STAGE 4)
WASH AND SCAN SLIDE (STAGE 5)
IMAGE FILE
DATA ANALYSIS (STAGE 6)
PROCESSED SLIDE
TARGET
GLASS MICROSCOPE SLIDE
OLIGONUCLEOTIDES
SPOTTING (STAGE 1)
SPOTTED SLIDE

FIG. 4

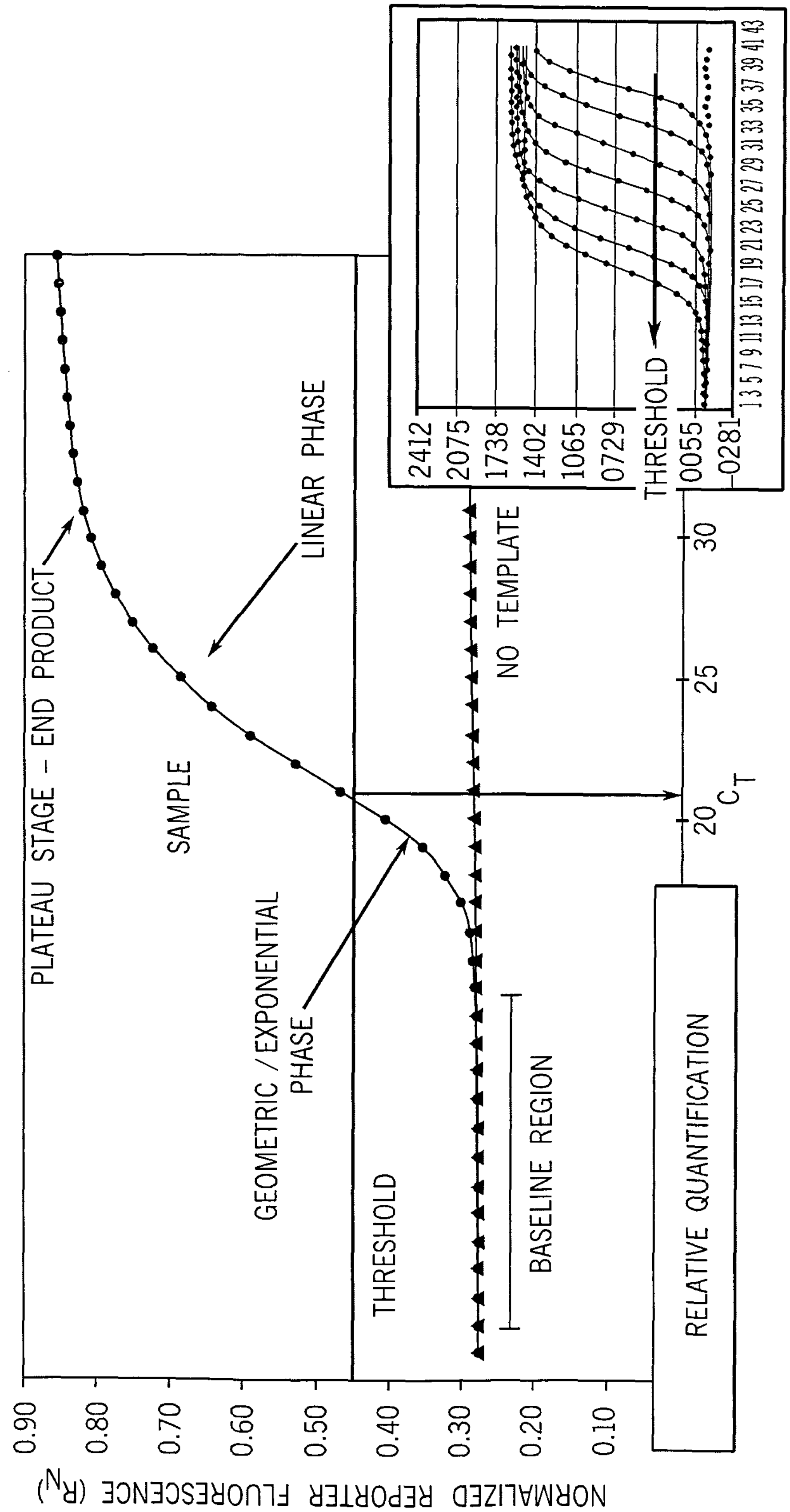
NORMALIZED REPORTER FLUORESCENCE (RN)
0.90
0.80
0.70
0.60
0.50
0.40
0.30
0.20
0.10
PLATEAU STAGE - END PRODUCT
LINEAR PHASE
SAMPLE
GEOMETRIC / EXPONENTIAL PHASE
THRESHOLD
NO TEMPLATE
BASELINE REGION
RELATIVE QUANTIFICATION
20
25
30
CT
2412
2075
1738
1402
1065
0729
THRESHOLD
0055
-0281
1 3 5 7 9 11 13 15 17 19 21 23 25 27 29 31 33 35 37 39 41 43

FIG. 5

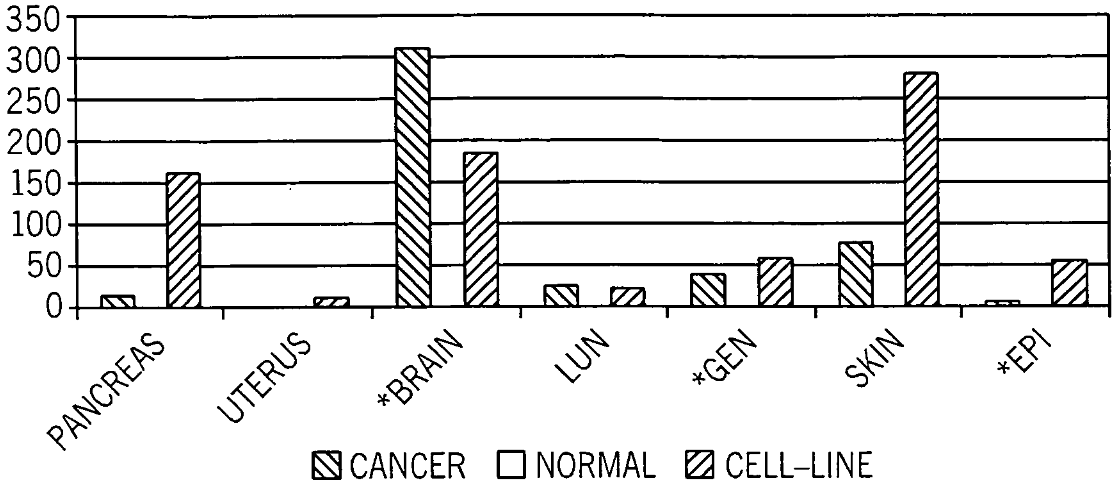
350
300
250
200
150
100
50
0
PANCREAS
UTERUS
*BRAIN
LUN
*GEN
SKIN
*EPI
CANCER
NORMAL
CELL-LINE

FIG. 6

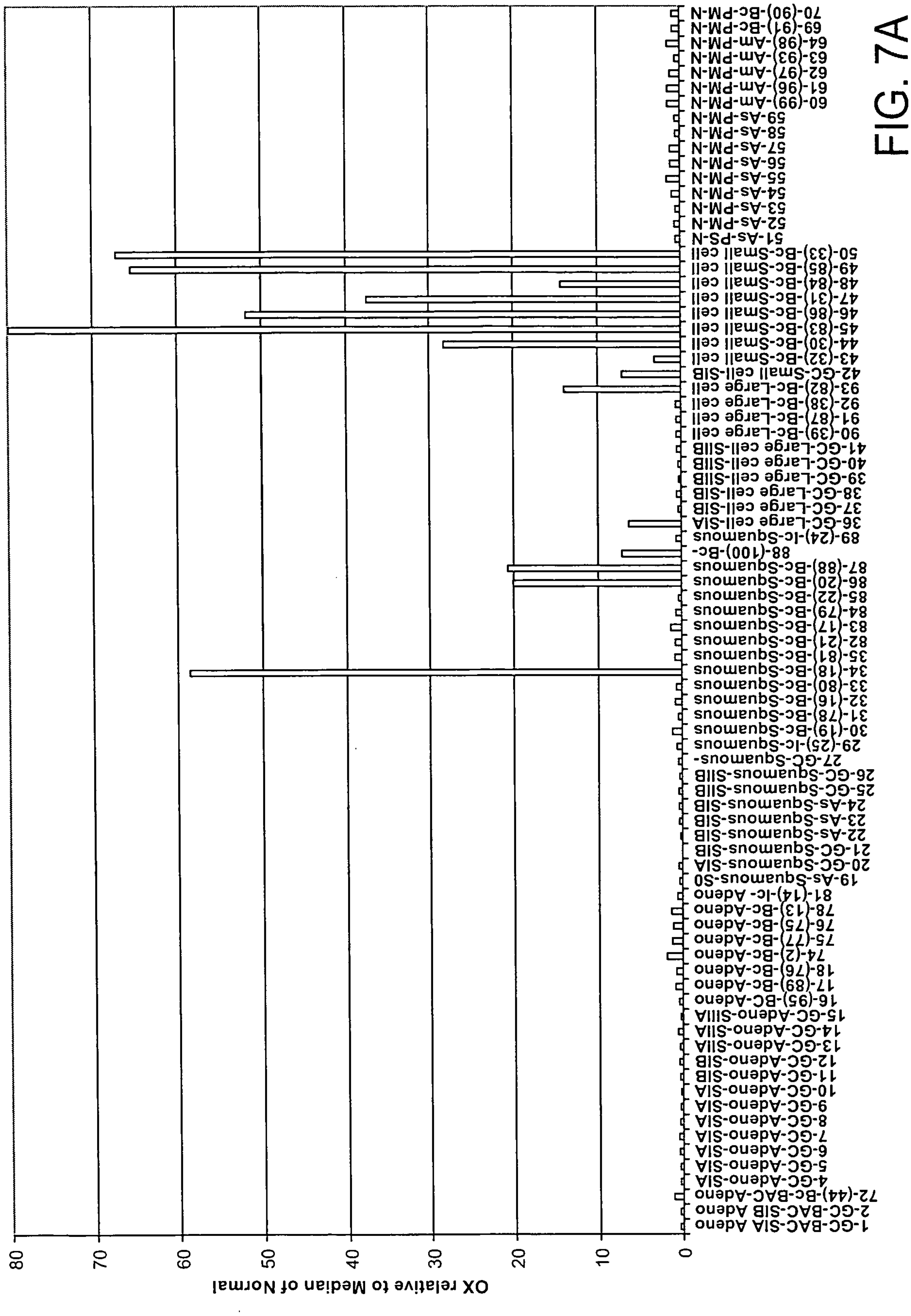
OX relative to Median of Normal
0
10
20
30
40
50
60
70
80
1-GC-BAC-SIA Adeno
2-GC-BAC-SIB Adeno
72-(44)-Bc-BAC-Adeno
4-GC-Adeno-SIA
5-GC-Adeno-SIA
6-GC-Adeno-SIA
7-GC-Adeno-SIA
8-GC-Adeno-SIA
9-GC-Adeno-SIA
10-GC-Adeno-SIA
11-GC-Adeno-SIB
12-GC-Adeno-SIB
13-GC-Adeno-SIIA
14-GC-Adeno-SIIA
15-GC-Adeno-SIIIA
16-(95)-BC-Adeno
17-(89)-Bc-Adeno
18-(76)-Bc-Adeno
74-(2)-Bc-Adeno
75-(77)-Bc-Adeno
76-(75)-Bc-Adeno
78-(13)-Bc-Adeno
81-(14)-Ic- Adeno
19-As-Squamous-S0
20-GC-Squamous-SIA
21-GC-Squamous-SIB
22-As-Squamous-SIB
23-As-Squamous-SIB
24-As-Squamous-SIB
25-GC-Squamous-SIIB
26-GC-Squamous-SIIB
27-GC-Squamous-
29-(25)-Ic-Squamous
30-(19)-Bc-Squamous
31-(78)-Bc-Squamous
32-(16)-Bc-Squamous
33-(80)-Bc-Squamous
34-(18)-Bc-Squamous
35-(81)-Bc-Squamous
82-(21)-Bc-Squamous
83-(17)-Bc-Squamous
84-(79)-Bc-Squamous
85-(22)-Bc-Squamous
86-(20)-Bc-Squamous
87-(88)-Bc-Squamous
88-(100)-Bc-
89-(24)-Ic-Squamous
36-GC-Large cell-SIA
37-GC-Large cell-SIB
38-GC-Large cell-SIB
39-GC-Large cell-SIIB
40-GC-Large cell-SIIB
41-GC-Large cell-SIIB
90-(39)-Bc-Large cell
91-(87)-Bc-Large cell
92-(38)-Bc-Large cell
93-(82)-Bc-Large cell
42-GC-Small cell-SIB
43-(32)-Bc-Small cell
44-(30)-Bc-Small cell
45-(83)-Bc-Small cell
46-(86)-Bc-Small cell
47-(31)-Bc-Small cell
48-(84)-Bc-Small cell
49-(85)-Bc-Small cell
50-(33)-Bc-Small cell
51-As-PS-N
52-As-PM-N
53-As-PM-N
54-As-PM-N
55-As-PM-N
56-As-PM-N
57-As-PM-N
58-As-PM-N
59-As-PM-N
60-(99)-Am-PM-N
61-(96)-Am-PM-N
62-(97)-Am-PM-N
63-(93)-Am-PM-N
64-(98)-Am-PM-N
69-(91)-Bc-PM-N
70-(90)-Bc-PM-N

FIG. 7A

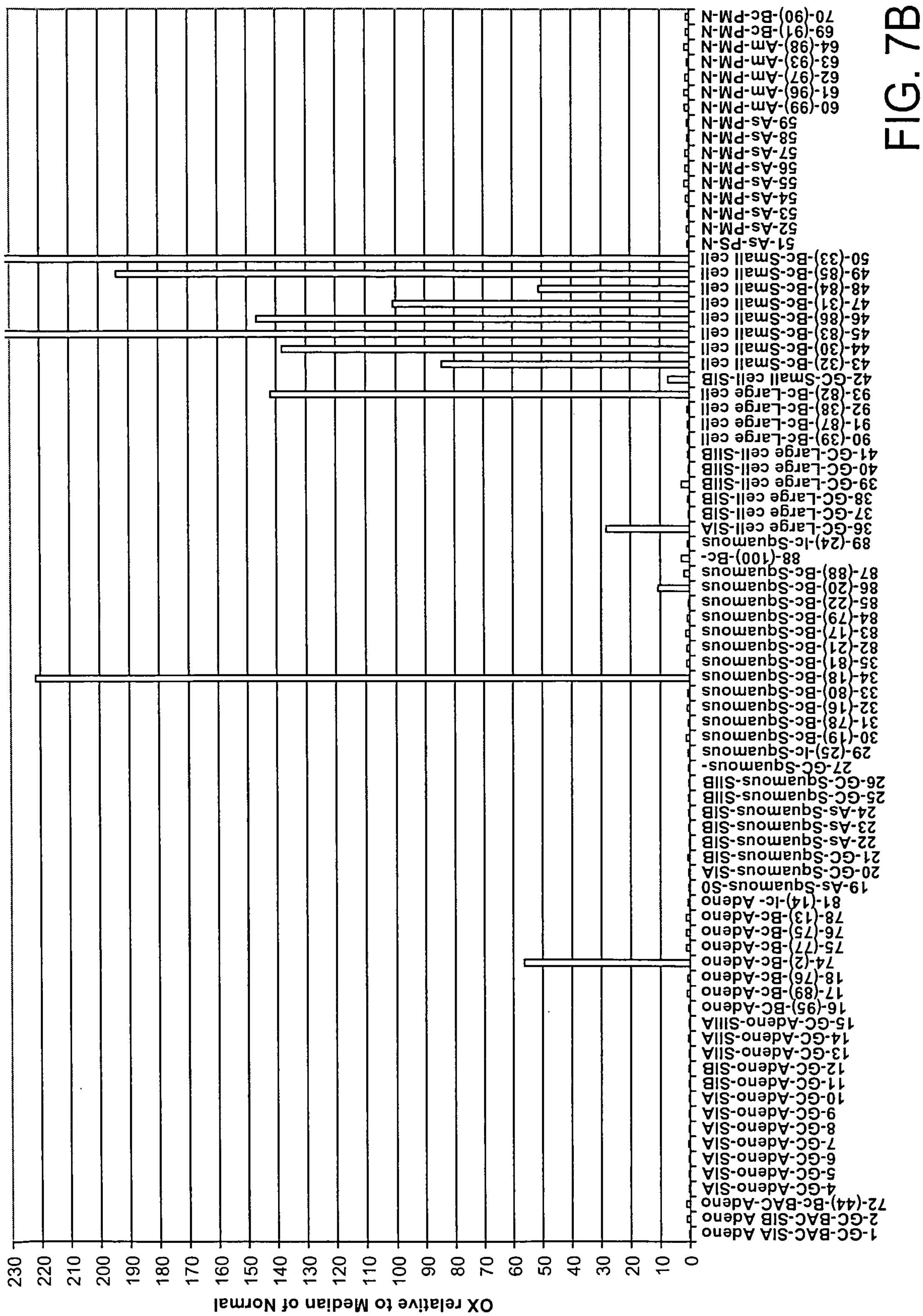

OX relative to Median of Normal
0
10
20
30
40
50
60
70
80
90
100
110
120
130
140
150
160
170
180
190
200
210
220
230
1-GC-BAC-SIA Adeno
2-GC-BAC-SIB Adeno
72-(44)-Bc-BAC-Adeno
4-GC-Adeno-SIA
5-GC-Adeno-SIA
6-GC-Adeno-SIA
7-GC-Adeno-SIA
8-GC-Adeno-SIA
9-GC-Adeno-SIA
10-GC-Adeno-SIA
11-GC-Adeno-SIB
12-GC-Adeno-SIB
13-GC-Adeno-SIIA
14-GC-Adeno-SIIA
15-GC-Adeno-SIIIA
16-(95)-BC-Adeno
17-(89)-Bc-Adeno
18-(76)-Bc-Adeno
74-(2)-Bc-Adeno
75-(77)-Bc-Adeno
76-(75)-Bc-Adeno
78-(13)-Bc-Adeno
81-(14)-Ic -Adeno
19-As-Squamous-S0
20-GC-Squamous-SIA
21-GC-Squamous-SIB
22-As-Squamous-SIB
23-As-Squamous-SIB
24-As-Squamous-SIB
25-GC-Squamous-SIIB
26-GC-Squamous-SIIB
27-GC-Squamous-
29-(25)-Ic-Squamous
30-(19)-Bc-Squamous
31-(78)-Bc-Squamous
32-(16)-Bc-Squamous
33-(80)-Bc-Squamous
34-(18)-Bc-Squamous
35-(81)-Bc-Squamous
82-(21)-Bc-Squamous
83-(17)-Bc-Squamous
84-(79)-Bc-Squamous
85-(22)-Bc-Squamous
86-(20)-Bc-Squamous
87-(88)-Bc-Squamous
88-(100)-Bc-
89-(24)-Ic-Squamous
36-GC-Large cell-SIA
37-GC-Large cell-SIB
38-GC-Large cell-SIB
39-GC-Large cell-SIIB
40-GC-Large cell-SIIB
41-GC-Large cell-SIIB
90-(39)-Bc-Large cell
91-(87)-Bc-Large cell
92-(38)-Bc-Large cell
93-(82)-Bc-Large cell
42-GC-Small cell-SIB
43-(32)-Bc-Small cell
44-(30)-Bc-Small cell
45-(83)-Bc-Small cell
46-(86)-Bc-Small cell
47-(31)-Bc-Small cell
48-(84)-Bc-Small cell
49-(85)-Bc-Small cell
50-(33)-Bc-Small cell
51-As-PS-N
52-As-PM-N
53-As-PM-N
54-As-PM-N
55-As-PM-N
56-As-PM-N
57-As-PM-N
58-As-PM-N
59-As-PM-N
60-(99)-Am-PM-N
61-(96)-Am-PM-N
62-(97)-Am-PM-N
63-(93)-Am-PM-N
64-(98)-Am-PM-N
69-(91)-Bc-PM-N
70-(90)-Bc-PM-N


FIG. 7B

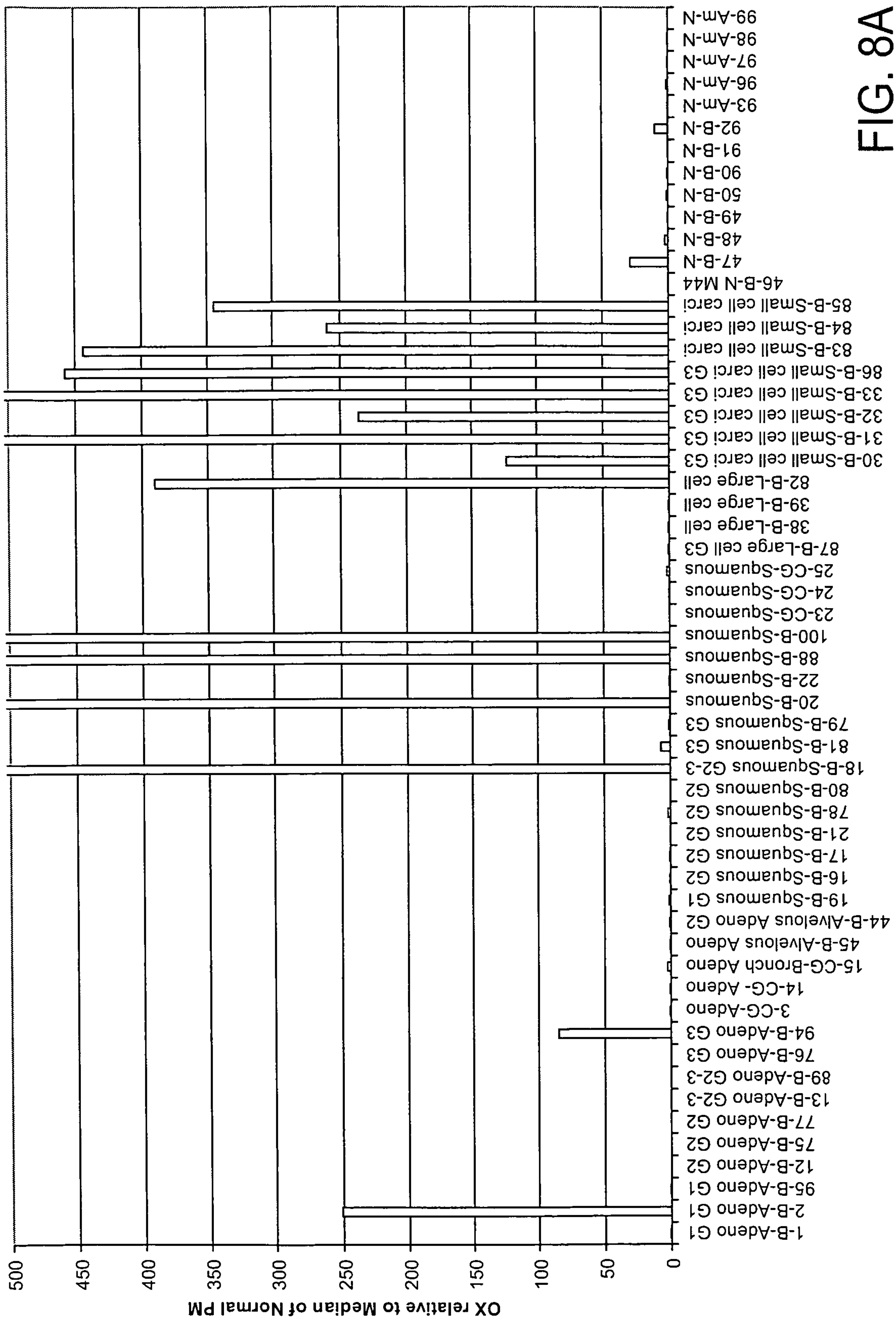

OX relative to Median of Normal PM
0
50
100
150
200
250
300
350
400
450
500
1-B-Adeno G1
2-B-Adeno G1
95-B-Adeno G1
12-B-Adeno G2
75-B-Adeno G2
77-B-Adeno G2
13-B-Adeno G2-3
89-B-Adeno G2-3
76-B-Adeno G3
94-B-Adeno G3
3-CG-Adeno
14-CG- Adeno
15-CG-Bronch Adeno
45-B-Alvelous Adeno
44-B-Alvelous Adeno G2
19-B-Squamous G1
16-B-Squamous G2
17-B-Squamous G2
21-B-Squamous G2
78-B-Squamous G2
80-B-Squamous G2
18-B-Squamous G2-3
81-B-Squamous G3
79-B-Squamous G3
20-B-Squamous
22-B-Squamous
88-B-Squamous
100-B-Squamous
23-CG-Squamous
24-CG-Squamous
25-CG-Squamous
87-B-Large cell G3
38-B-Large cell
39-B-Large cell
82-B-Large cell
30-B-Small cell carci G3
31-B-Small cell carci G3
32-B-Small cell carci G3
33-B-Small cell carci G3
86-B-Small cell carci G3
83-B-Small cell carci
84-B-Small cell carci
85-B-Small cell carci
46-B-N M44
47-B-N
48-B-N
49-B-N
50-B-N
90-B-N
91-B-N
92-B-N
93-Am-N
96-Am-N
97-Am-N
98-Am-N
99-Am-N


FIG. 8A

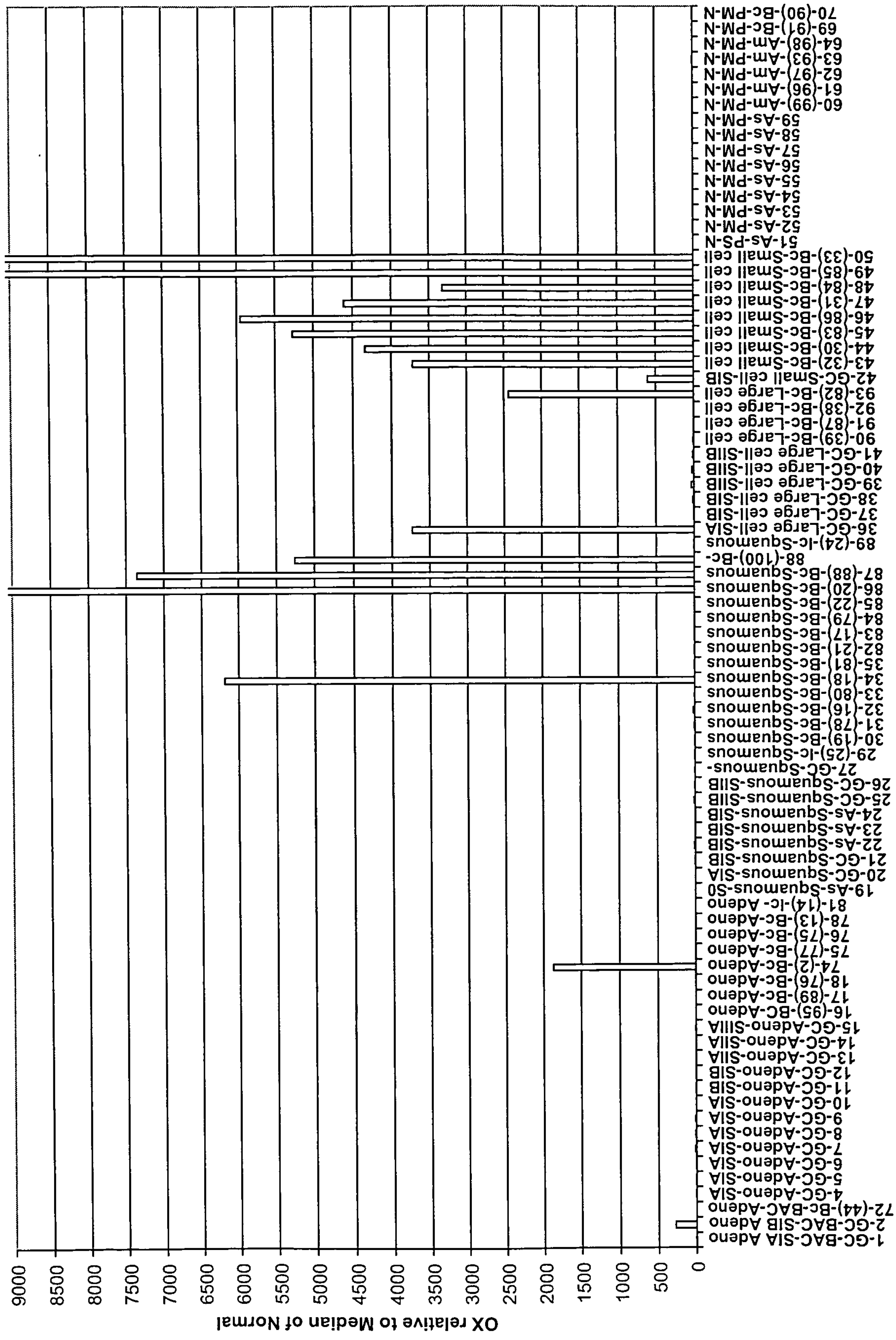

OX relative to Median of Normal
0
500
1000
1500
2000
2500
3000
3500
4000
4500
5000
5500
6000
6500
7000
7500
8000
8500
9000
1-GC-BAC-SIA Adeno
2-GC-BAC-SIB Adeno
72-(44)-Bc-BAC-Adeno
4-GC-Adeno-SIA
5-GC-Adeno-SIA
6-GC-Adeno-SIA
7-GC-Adeno-SIA
8-GC-Adeno-SIA
9-GC-Adeno-SIA
10-GC-Adeno-SIA
11-GC-Adeno-SIB
12-GC-Adeno-SIB
13-GC-Adeno-SIIA
14-GC-Adeno-SIIA
15-GC-Adeno-SIIIA
16-(95)-BC-Adeno
17-(89)-Bc-Adeno
18-(76)-Bc-Adeno
74-(2)-Bc-Adeno
75-(77)-Bc-Adeno
76-(75)-Bc-Adeno
78-(13)-Bc-Adeno
81-(14)-Ic- Adeno
19-As-Squamos-S0
20-GC-Squamos-SIA
21-GC-Squamos-SIB
22-As-Squamos-SIB
23-As-Squamos-SIB
24-As-Squamos-SIB
25-GC-Squamos-SIIB
26-GC-Squamos-SIIB
27-GC-Squamos-
29-(25)-Ic-Squamous
30-(19)-Bc-Squamous
31-(78)-Bc-Squamous
32-(16)-Bc-Squamous
33-(80)-Bc-Squamous
34-(18)-Bc-Squamous
35-(81)-Bc-Squamous
82-(21)-Bc-Squamous
83-(17)-Bc-Squamous
84-(79)-Bc-Squamous
85-(22)-Bc-Squamous
86-(20)-Bc-Squamous
87-(88)-Bc-Squamous
88-(100)-Bc-
89-(24)-Ic-Squamous
36-GC-Large cell-SIA
37-GC-Large cell-SIB
38-GC-Large cell-SIB
39-GC-Large cell-SIIB
40-GC-Large cell-SIIB
41-GC-Large cell-SIIB
90-(39)-Bc-Large cell
91-(87)-Bc-Large cell
92-(38)-Bc-Large cell
93-(82)-Bc-Large cell
42-GC-Small cell-SIB
43-(32)-Bc-Small cell
44-(30)-Bc-Small cell
45-(83)-Bc-Small cell
46-(86)-Bc-Small cell
47-(31)-Bc-Small cell
48-(84)-Bc-Small cell
49-(85)-Bc-Small cell
50-(33)-Bc-Small cell
51-As-PS-N
52-As-PM-N
53-As-PM-N
54-As-PM-N
55-As-PM-N
56-As-PM-N
57-As-PM-N
58-As-PM-N
59-As-PM-N
60-(99)-Am-PM-N
61-(96)-Am-PM-N
62-(97)-Am-PM-N
63-(93)-Am-PM-N
64-(98)-Am-PM-N
69-(91)-Bc-PM-N
70-(90)-Bc-PM-N


FIG. 8B

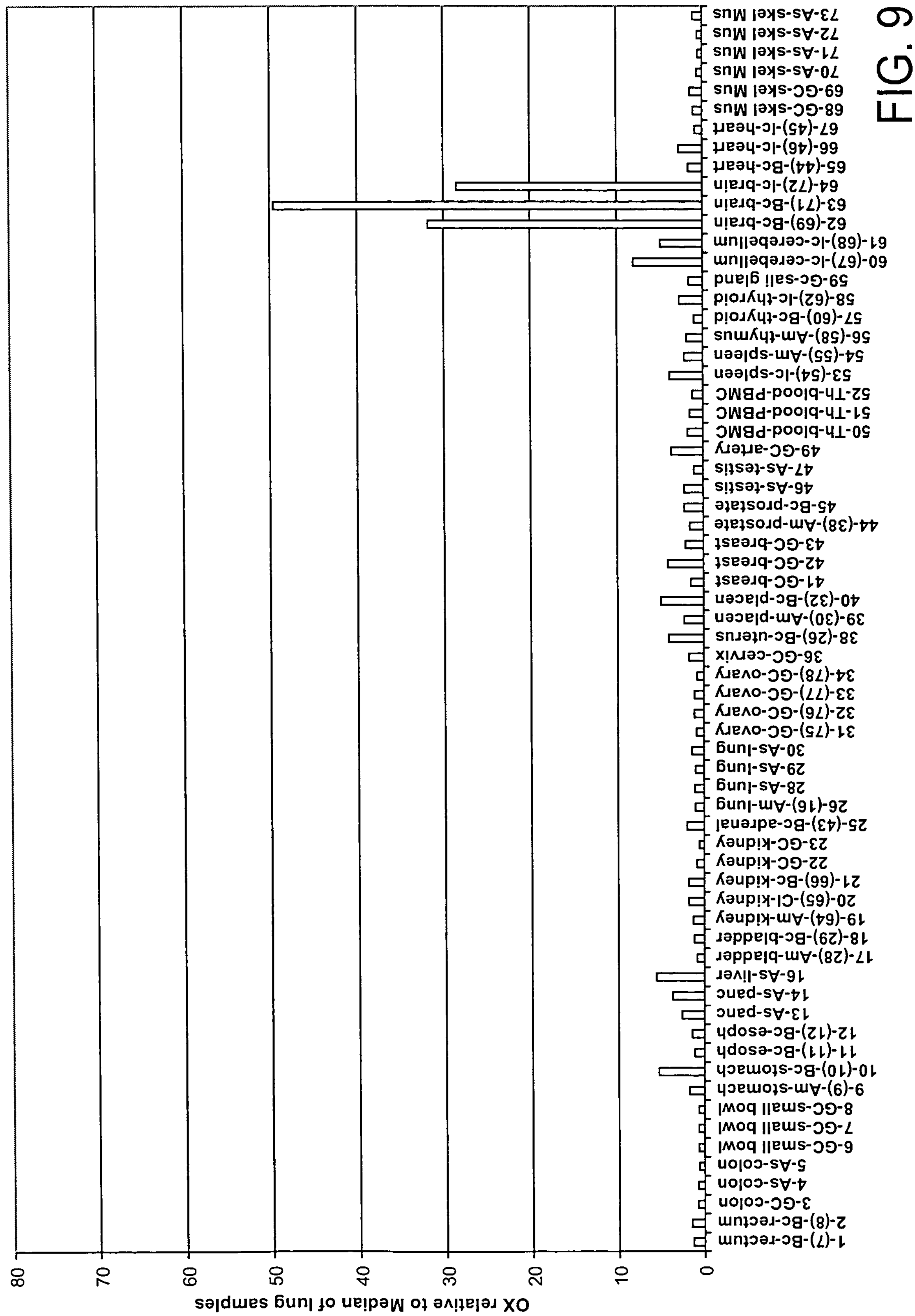
OX relative to Median of lung samples
0
10
20
30
40
50
60
70
80
1-(7)-Bc-rectum
2-(8)-Bc-rectum
3-GC-colon
4-As-colon
5-As-colon
6-GC-small bowl
7-GC-small bowl
8-GC-small bowl
9-(9)-Am-stomach
10-(10)-Bc-stomach
11-(11)-Bc-esoph
12-(12)-Bc-esoph
13-As-panc
14-As-panc
16-As-liver
17-(28)-Am-bladder
18-(29)-Bc-bladder
19-(64)-Am-kidney
20-(65)-Cl-kidney
21-(66)-Bc-kidney
22-GC-kidney
23-GC-kidney
25-(43)-Bc-adrenal
26-(16)-Am-lung
28-As-lung
29-As-lung
30-As-lung
31-(75)-GC-ovary
32-(76)-GC-ovary
33-(77)-GC-ovary
34-(78)-GC-ovary
36-GC-cervix
38-(26)-Bc-uterus
39-(30)-Am-placen
40-(32)-Bc-placen
41-GC-breast
42-GC-breast
43-GC-breast
44-(38)-Am-prostate
45-Bc-prostate
46-As-testis
47-As-testis
49-GC-artery
50-Th-blood-PBMC
51-Th-blood-PBMC
52-Th-blood-PBMC
53-(54)-lc-spleen
54-(55)-Am-spleen
56-(58)-Am-thymus
57-(60)-Bc-thyroid
58-(62)-lc-thyroid
59-Gc-sali gland
60-(67)-lc-cerebellum
61-(68)-lc-cerebellum
62-(69)-Bc-brain
63-(71)-Bc-brain
64-(72)-lc-brain
65-(44)-Bc-heart
66-(46)-lc-heart
67-(45)-lc-heart
68-GC-skel Mus
69-GC-skel Mus
70-As-skel Mus
71-As-skel Mus
72-As-skel Mus
73-As-skel Mus

FIG. 9

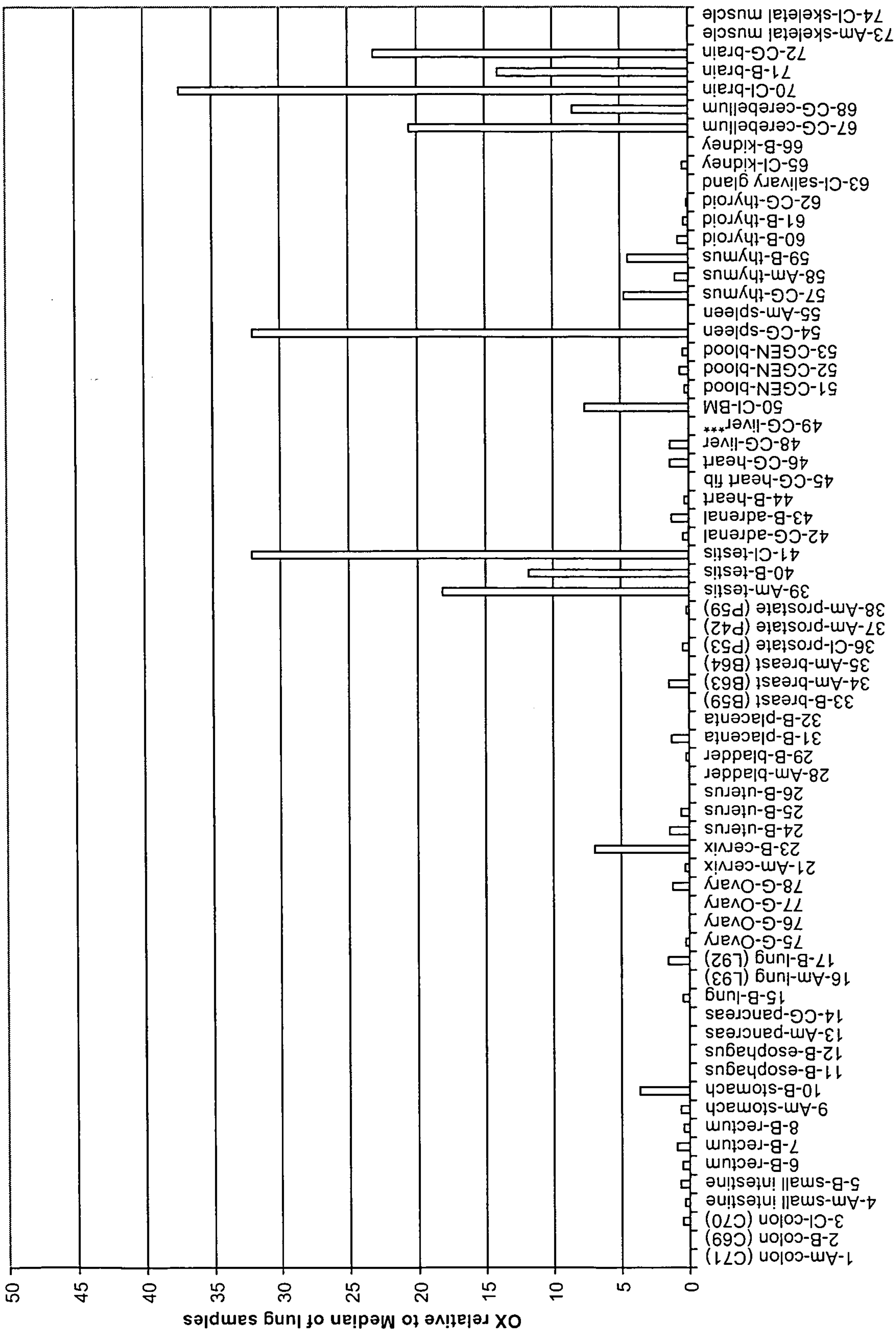

OX relative to Median of lung samples
0
5
10
15
20
25
30
35
40
45
50
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
65-Cl-kidney
66-B-kidney
67-CG-cerebellum
68-CG-cerebellum
70-Cl-brain
71-B-brain
72-CG-brain
73-Am-skeletal muscle
74-Cl-skeletal muscle


FIG. 10A

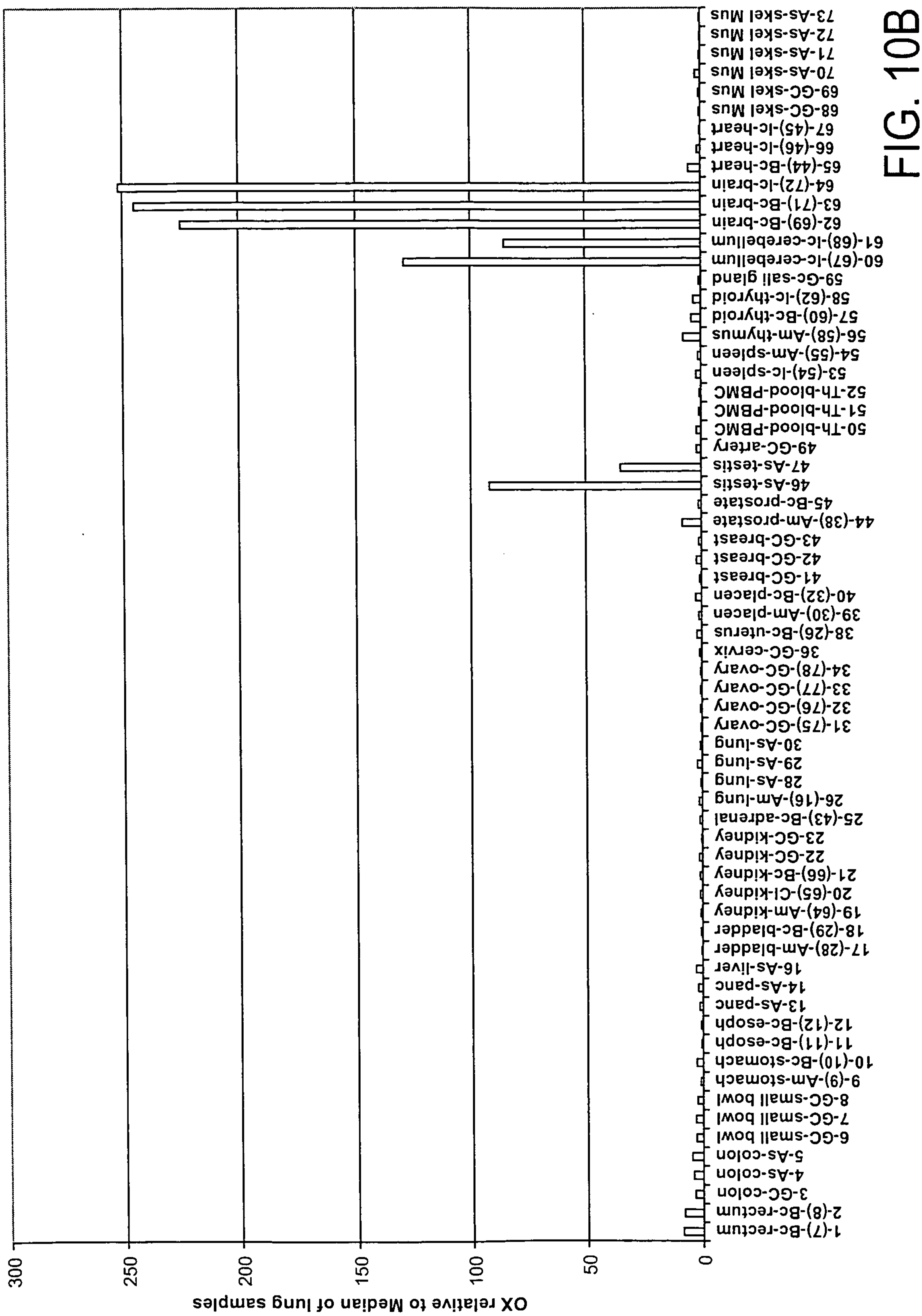
OX relative to Median of lung samples
0
50
100
150
200
250
300
1-(7)-Bc-rectum
2-(8)-Bc-rectum
3-GC-colon
4-As-colon
5-As-colon
6-GC-small bowl
7-GC-small bowl
8-GC-small bowl
9-(9)-Am-stomach
10-(10)-Bc-stomach
11-(11)-Bc-esoph
12-(12)-Bc-esoph
13-As-panc
14-As-panc
16-As-liver
17-(28)-Am-bladder
18-(29)-Bc-bladder
19-(64)-Am-kidney
20-(65)-Cl-kidney
21-(66)-Bc-kidney
22-GC-kidney
23-GC-kidney
25-(43)-Bc-adrenal
26-(16)-Am-lung
28-As-lung
29-As-lung
30-As-lung
31-(75)-GC-ovary
32-(76)-GC-ovary
33-(77)-GC-ovary
34-(78)-GC-ovary
36-GC-cervix
38-(26)-Bc-uterus
39-(30)-Am-placen
40-(32)-Bc-placen
41-GC-breast
42-GC-breast
43-GC-breast
44-(38)-Am-prostate
45-Bc-prostate
46-As-testis
47-As-testis
49-GC-artery
50-Th-blood-PBMC
51-Th-blood-PBMC
52-Th-blood-PBMC
53-(54)-lc-spleen
54-(55)-Am-spleen
56-(58)-Am-thymus
57-(60)-Bc-thyroid
58-(62)-lc-thyroid
59-Gc-sali gland
60-(67)-lc-cerebellum
61-(68)-lc-cerebellum
62-(69)-Bc-brain
63-(71)-Bc-brain
64-(72)-lc-brain
65-(44)-Bc-heart
66-(46)-lc-heart
67-(45)-lc-heart
68-GC-skel Mus
69-GC-skel Mus
70-As-skel Mus
71-As-skel Mus
72-As-skel Mus
73-As-skel Mus

FIG. 10B

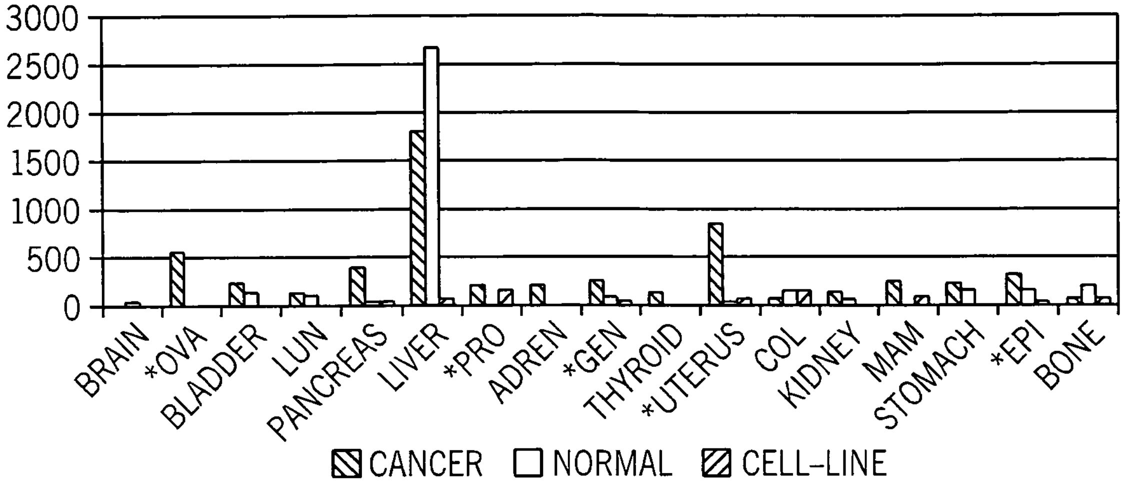
3000
2500
2000
1500
1000
500
0
BRAIN
*OVA
BLADDER
LUN
PANCREAS
LIVER
*PRO
ADREN
*GEN
THYROID
*UTERUS
COL
KIDNEY
MAM
STOMACH
*EPI
BONE
CANCER
NORMAL
CELL-LINE

FIG. 11

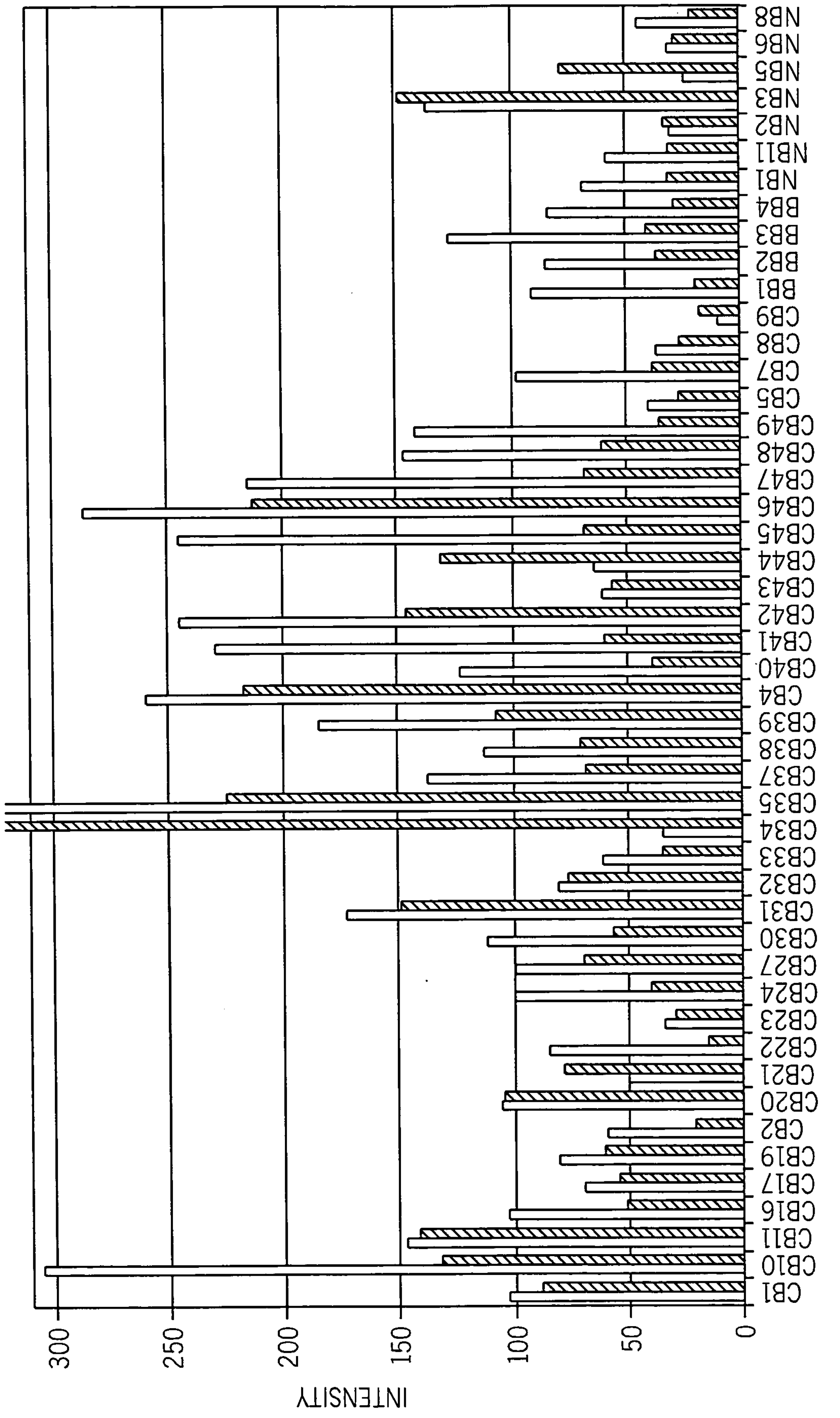
INTENSITY
0
50
100
150
200
250
300
CB1
CB10
CB11
CB16
CB17
CB19
CB2
CB20
CB21
CB22
CB23
CB24
CB27
CB30
CB31
CB32
CB33
CB34
CB35
CB37
CB38
CB39
CB4
CB40
CB41
CB42
CB43
CB44
CB45
CB46
CB47
CB48
CB49
CB5
CB7
CB8
CB9
BB1
BB2
BB3
BB4
NB1
NB11
NB2
NB3
NB5
NB6
NB8
D12115_0_24_19
D12115_0_62_120

FIG. 12A

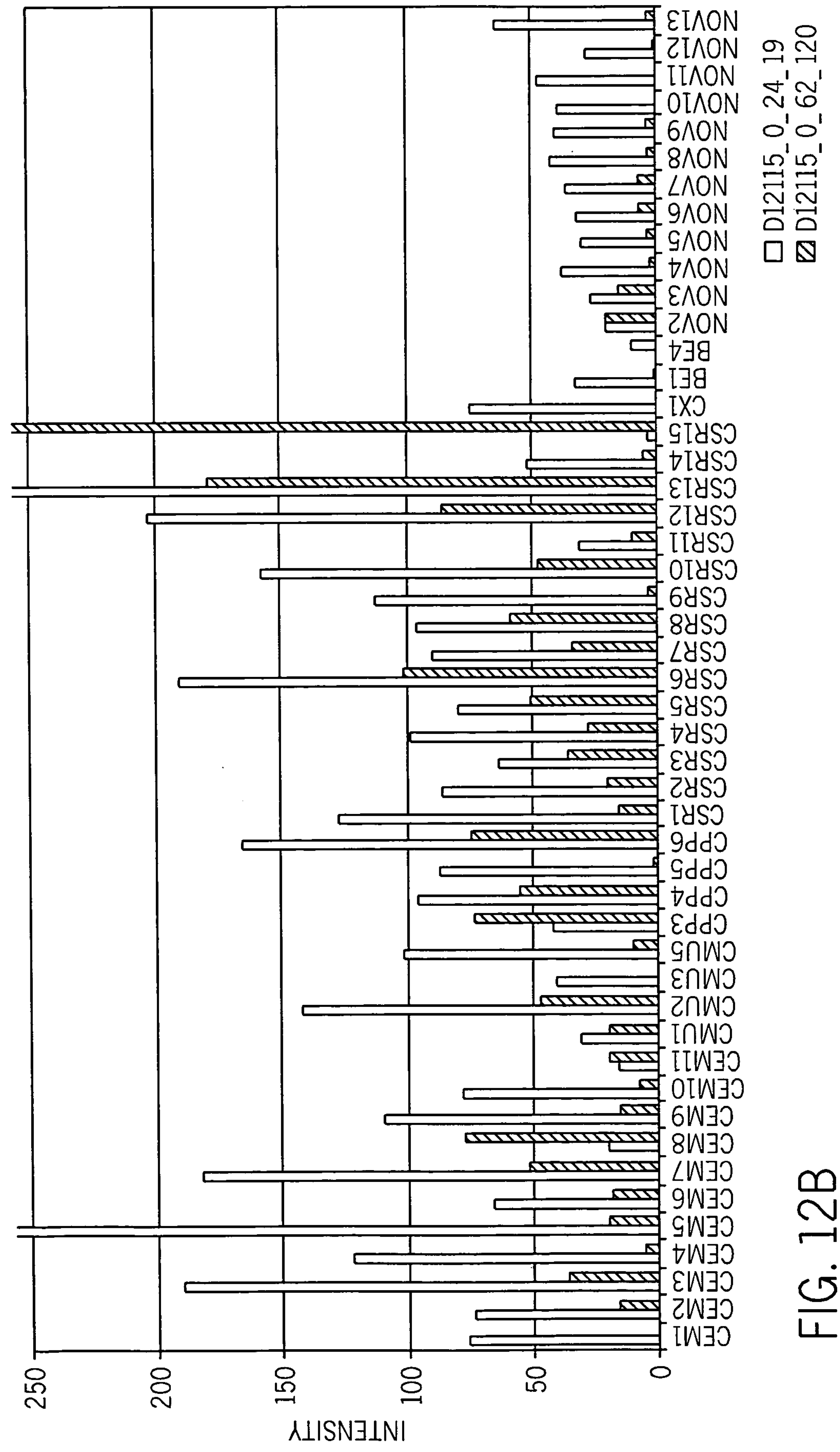
INTENSITY
0
50
100
150
200
250
CEM1
CEM2
CEM3
CEM4
CEM5
CEM6
CEM7
CEM8
CEM9
CEM10
CEM11
CMU1
CMU2
CMU3
CMU5
CPP3
CPP4
CPP5
CPP6
CSR1
CSR2
CSR3
CSR4
CSR5
CSR6
CSR7
CSR8
CSR9
CSR10
CSR11
CSR12
CSR13
CSR14
CSR15
CX1
BE1
BE4
NOV2
NOV3
NOV4
NOV5
NOV6
NOV7
NOV8
NOV9
NOV10
NOV11
NOV12
NOV13
D12115_0_24_19
D12115_0_62_120

FIG. 12B

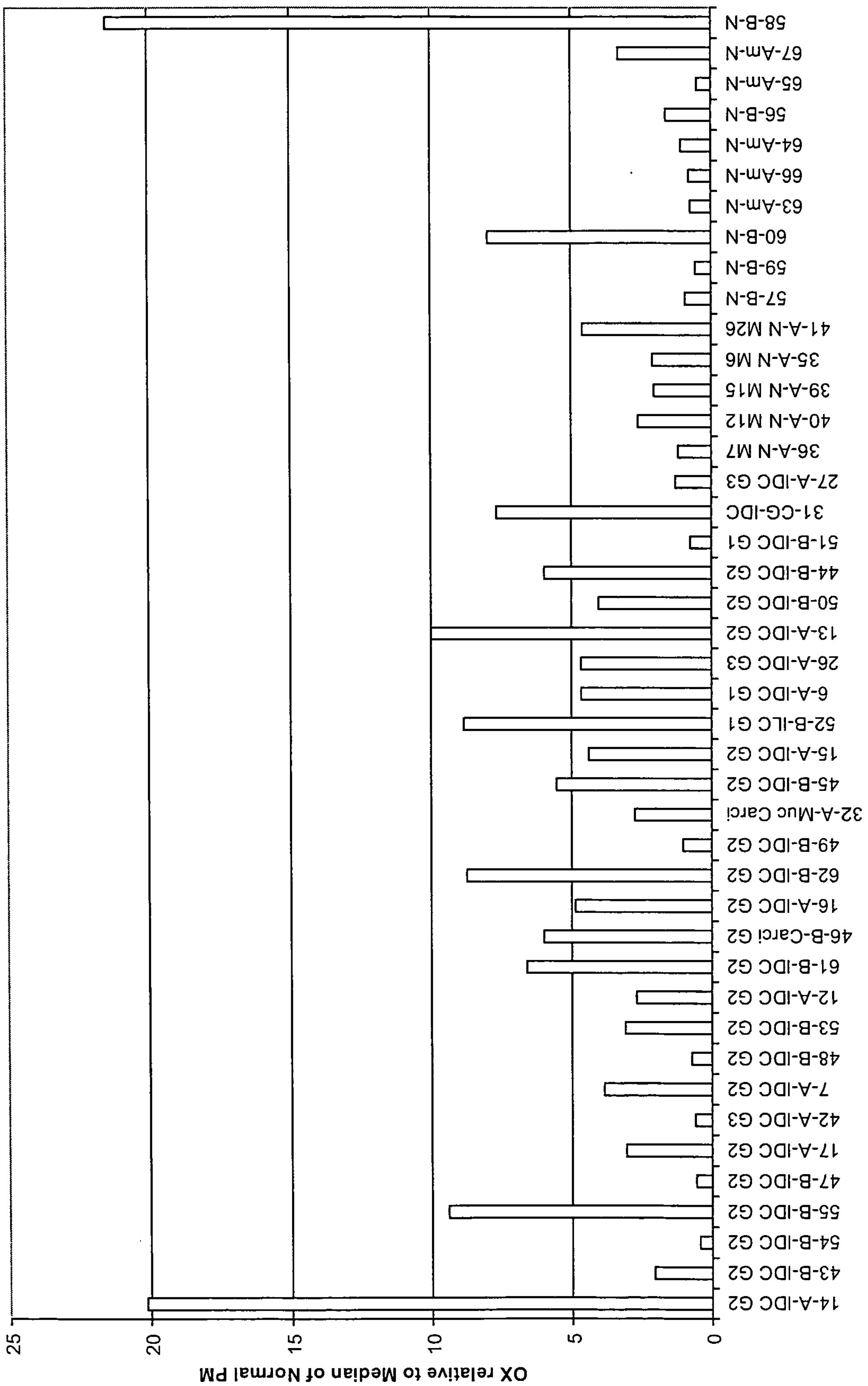
OX relative to Median of Normal PM
0
5
10
15
20
25
14-A-IDC G2
43-B-IDC G2
54-B-IDC G2
55-B-IDC G2
47-B-IDC G2
17-A-IDC G2
42-A-IDC G3
7-A-IDC G2
48-B-IDC G2
53-B-IDC G2
12-A-IDC G2
61-B-IDC G2
46-B-Carci G2
16-A-IDC G2
62-B-IDC G2
49-B-IDC G2
32-A-Muc Carci
45-B-IDC G2
15-A-IDC G2
52-B-ILC G1
6-A-IDC G1
26-A-IDC G3
13-A-IDC G2
50-B-IDC G2
44-B-IDC G2
51-B-IDC G1
31-CG-IDC
27-A-IDC G3
36-A-N M7
40-A-N M12
39-A-N M15
35-A-N M6
41-A-N M26
57-B-N
59-B-N
60-B-N
63-Am-N
66-Am-N
64-Am-N
56-B-N
65-Am-N
67-Am-N
58-B-N

FIG. 13

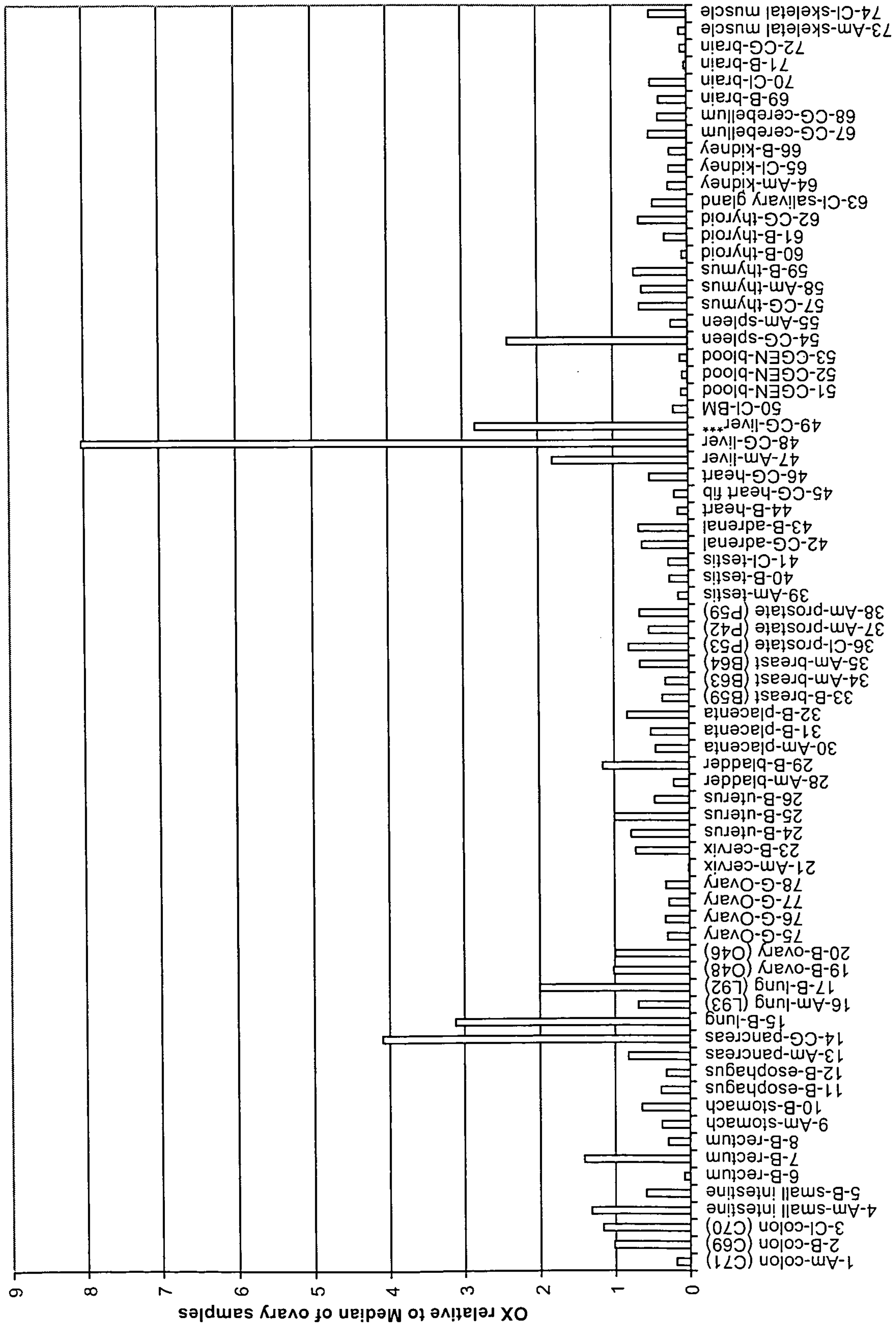

OX relative to Median of ovary samples
0
1
2
3
4
5
6
7
8
9
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle


FIG. 14

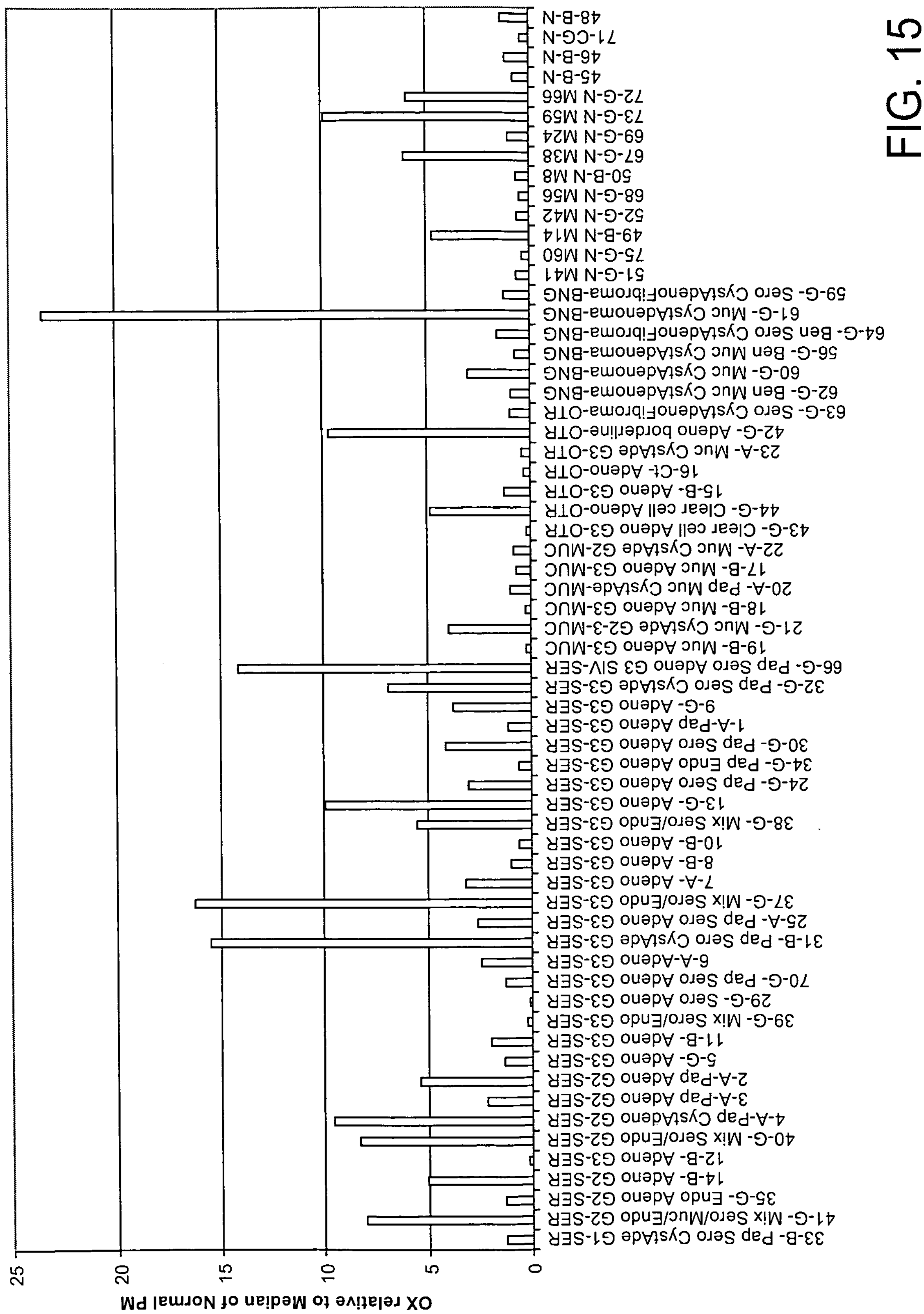
OX relative to Median of Normal PM
0
5
10
15
20
25
33-B- Pap Sero CystAde G1-SER
41-G- Mix Sero/Muc/Endo G2-SER
35-G- Endo Adeno G2-SER
14-B- Adeno G2-SER
12-B- Adeno G3-SER
40-G- Mix Sero/Endo G2-SER
4-A-Pap CystAdeno G2-SER
3-A-Pap Adeno G2-SER
2-A-Pap Adeno G2-SER
5-G- Adeno G3-SER
11-B- Adeno G3-SER
39-G- Mix Sero/Endo G3-SER
29-G- Sero Adeno G3-SER
70-G- Pap Sero Adeno G3-SER
6-A-Adeno G3-SER
31-B- Pap Sero CystAde G3-SER
25-A- Pap Sero Adeno G3-SER
37-G- Mix Sero/Endo G3-SER
7-A- Adeno G3-SER
8-B- Adeno G3-SER
10-B- Adeno G3-SER
38-G- Mix Sero/Endo G3-SER
13-G- Adeno G3-SER
24-G- Pap Sero Adeno G3-SER
34-G- Pap Endo Adeno G3-SER
30-G- Pap Sero Adeno G3-SER
1-A-Pap Adeno G3-SER
9-G- Adeno G3-SER
32-G- Pap Sero CystAde G3-SER
66-G- Pap Sero Adeno G3 SIV-SER
19-B- Muc Adeno G3-MUC
21-G- Muc CystAde G2-3-MUC
18-B- Muc Adeno G3-MUC
20-A- Pap Muc CystAde-MUC
17-B- Muc Adeno G3-MUC
22-A- Muc CystAde G2-MUC
43-G- Clear cell Adeno G3-OTR
44-G- Clear cell Adeno-OTR
15-B- Adeno G3-OTR
16-Ct- Adeno-OTR
23-A- Muc CystAde G3-OTR
42-G- Adeno borderline-OTR
63-G- Sero CystAdenoFibroma-OTR
62-G- Ben Muc CystAdenoma-BNG
60-G- Muc CystAdenoma-BNG
56-G- Ben Muc CystAdenoma-BNG
64-G- Ben Sero CystAdenoFibroma-BNG
61-G- Muc CystAdenoma-BNG
59-G- Sero CystAdenoFibroma-BNG
51-G-N M41
75-G-N M60
49-B-N M14
52-G-N M42
68-G-N M56
50-B-N M8
67-G-N M38
69-G-N M24
73-G-N M59
72-G-N M66
45-B-N
46-B-N
71-CG-N
48-B-N

FIG. 15

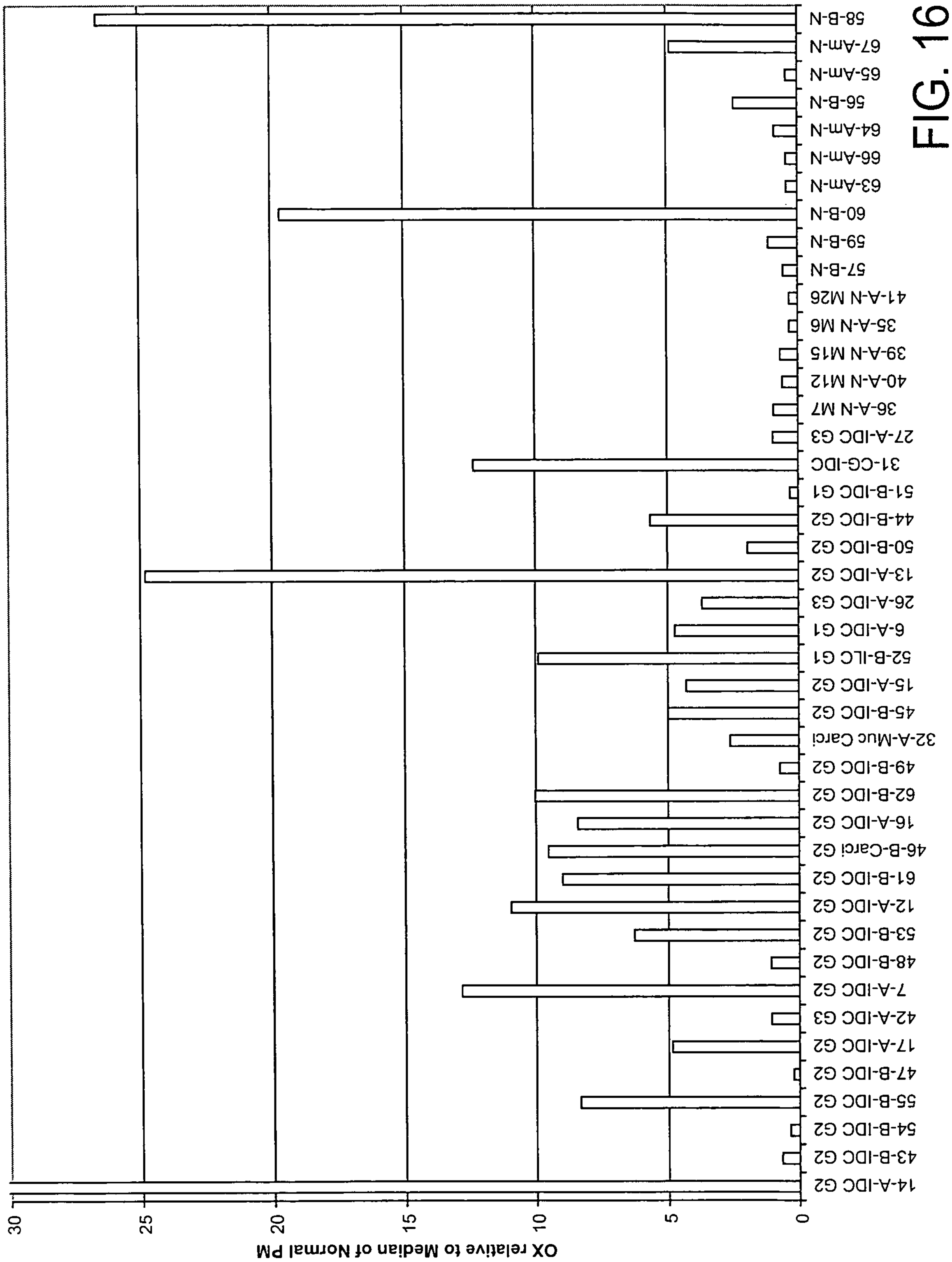
OX relative to Median of Normal PM
0
5
10
15
20
25
30
14-A-IDC G2
43-B-IDC G2
54-B-IDC G2
55-B-IDC G2
47-B-IDC G2
17-A-IDC G2
42-A-IDC G3
7-A-IDC G2
48-B-IDC G2
53-B-IDC G2
12-A-IDC G2
61-B-IDC G2
46-B-Carci G2
16-A-IDC G2
62-B-IDC G2
49-B-IDC G2
32-A-Muc Carci
45-B-IDC G2
15-A-IDC G2
52-B-ILC G1
6-A-IDC G1
26-A-IDC G3
13-A-IDC G2
50-B-IDC G2
44-B-IDC G2
51-B-IDC G1
31-CG-IDC
27-A-IDC G3
36-A-N M7
40-A-N M12
39-A-N M15
35-A-N M6
41-A-N M26
57-B-N
59-B-N
60-B-N
63-Am-N
66-Am-N
64-Am-N
56-B-N
65-Am-N
67-Am-N
58-B-N

FIG. 16

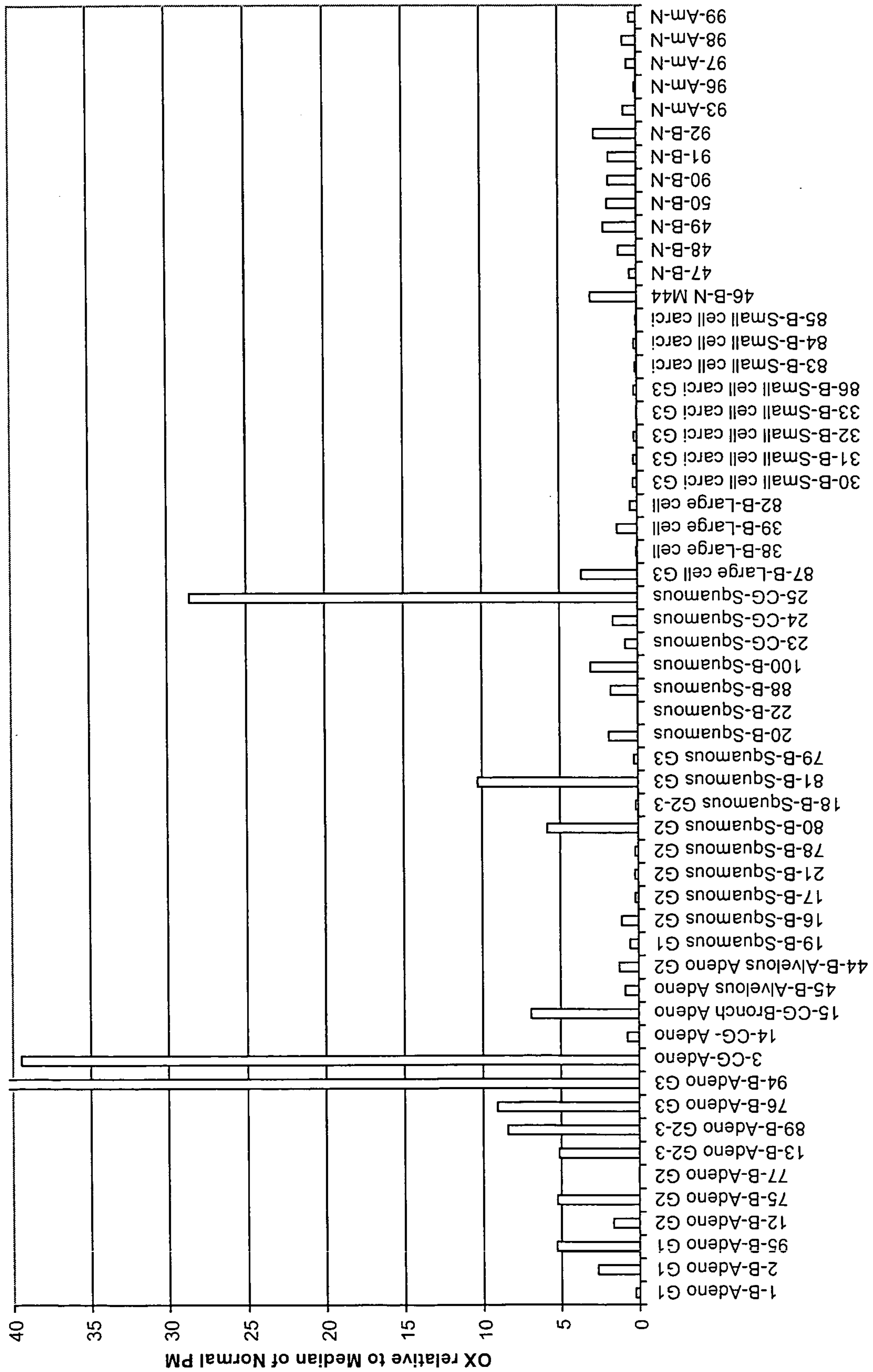

OX relative to Median of Normal PM
0
5
10
15
20
25
30
35
40
1-B-Adeno G1
2-B-Adeno G1
95-B-Adeno G1
12-B-Adeno G2
75-B-Adeno G2
77-B-Adeno G2
13-B-Adeno G2-3
89-B-Adeno G2-3
76-B-Adeno G3
94-B-Adeno G3
3-CG-Adeno
14-CG- Adeno
15-CG-Bronch Adeno
45-B-Alvelous Adeno
44-B-Alvelous Adeno G2
19-B-Squamous G1
16-B-Squamous G2
17-B-Squamous G2
21-B-Squamous G2
78-B-Squamous G2
80-B-Squamous G2
18-B-Squamous G2-3
81-B-Squamous G3
79-B-Squamous G3
20-B-Squamous
22-B-Squamous
88-B-Squamous
100-B-Squamous
23-CG-Squamous
24-CG-Squamous
25-CG-Squamous
87-B-Large cell G3
38-B-Large cell
39-B-Large cell
82-B-Large cell
30-B-Small cell carci G3
31-B-Small cell carci G3
32-B-Small cell carci G3
33-B-Small cell carci G3
86-B-Small cell carci G3
83-B-Small cell carci
84-B-Small cell carci
85-B-Small cell carci
46-B-N M44
47-B-N
48-B-N
49-B-N
50-B-N
90-B-N
91-B-N
92-B-N
93-Am-N
96-Am-N
97-Am-N
98-Am-N
99-Am-N


FIG. 17

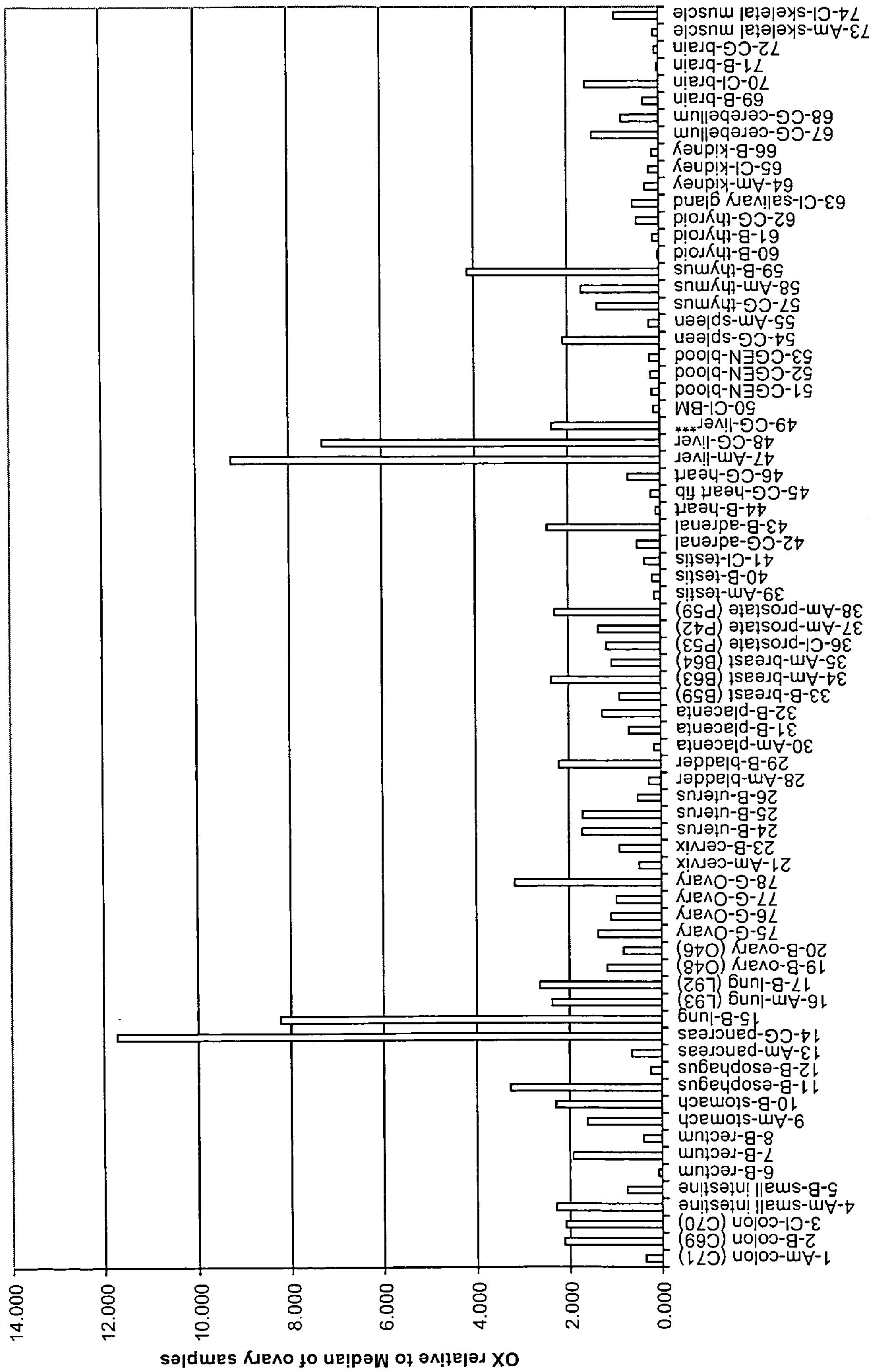
OX relative to Median of ovary samples
0.000
2.000
4.000
6.000
8.000
10.000
12.000
14.000
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle

FIG. 18

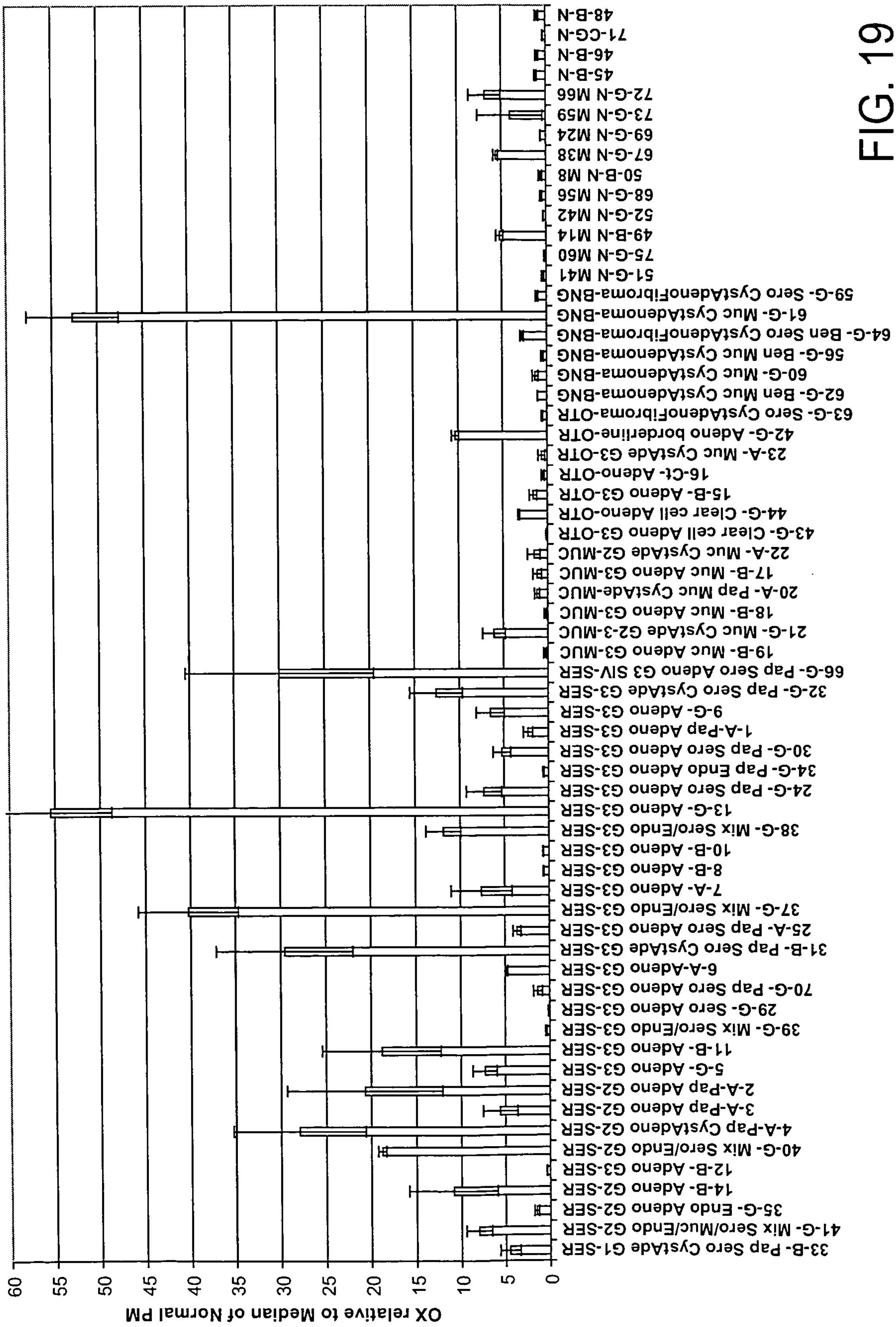
OX relative to Median of Normal PM
0
5
10
15
20
25
30
35
40
45
50
55
60
33-B- Pap Sero CystAde G1-SER
41-G- Mix Sero/Muc/Endo G2-SER
35-G- Endo Adeno G2-SER
14-B- Adeno G2-SER
12-B- Adeno G3-SER
40-G- Mix Sero/Endo G2-SER
4-A-Pap CystAdeno G2-SER
3-A-Pap Adeno G2-SER
2-A-Pap Adeno G2-SER
5-G- Adeno G3-SER
11-B- Adeno G3-SER
39-G- Mix Sero/Endo G3-SER
29-G- Sero Adeno G3-SER
70-G- Pap Sero Adeno G3-SER
6-A-Adeno G3-SER
31-B- Pap Sero CystAde G3-SER
25-A- Pap Sero Adeno G3-SER
37-G- Mix Sero/Endo G3-SER
7-A- Adeno G3-SER
8-B- Adeno G3-SER
10-B- Adeno G3-SER
38-G- Mix Sero/Endo G3-SER
13-G- Adeno G3-SER
24-G- Pap Sero Adeno G3-SER
34-G- Pap Endo Adeno G3-SER
30-G- Pap Sero Adeno G3-SER
1-A-Pap Adeno G3-SER
9-G- Adeno G3-SER
32-G- Pap Sero CystAde G3-SER
66-G- Pap Sero Adeno G3 SIV-SER
19-B- Muc Adeno G3-MUC
21-G- Muc CystAde G2-3-MUC
18-B- Muc Adeno G3-MUC
20-A- Pap Muc CystAde-MUC
17-B- Muc Adeno G3-MUC
22-A- Muc CystAde G2-MUC
43-G- Clear cell Adeno G3-OTR
44-G- Clear cell Adeno-OTR
15-B- Adeno G3-OTR
16-Ct- Adeno-OTR
23-A- Muc CystAde G3-OTR
42-G- Adeno borderline-OTR
63-G- Sero CystAdenoFibroma-OTR
62-G- Ben Muc CystAdenoma-BNG
60-G- Muc CystAdenoma-BNG
56-G- Ben Muc CystAdenoma-BNG
64-G- Ben Sero CystAdenoFibroma-BNG
61-G- Muc CystAdenoma-BNG
59-G- Sero CystAdenoFibroma-BNG
51-G-N M41
75-G-N M60
49-B-N M14
52-G-N M42
68-G-N M56
50-B-N M8
67-G-N M38
69-G-N M24
73-G-N M59
72-G-N M66
45-B-N
46-B-N
71-CG-N
48-B-N

FIG. 19

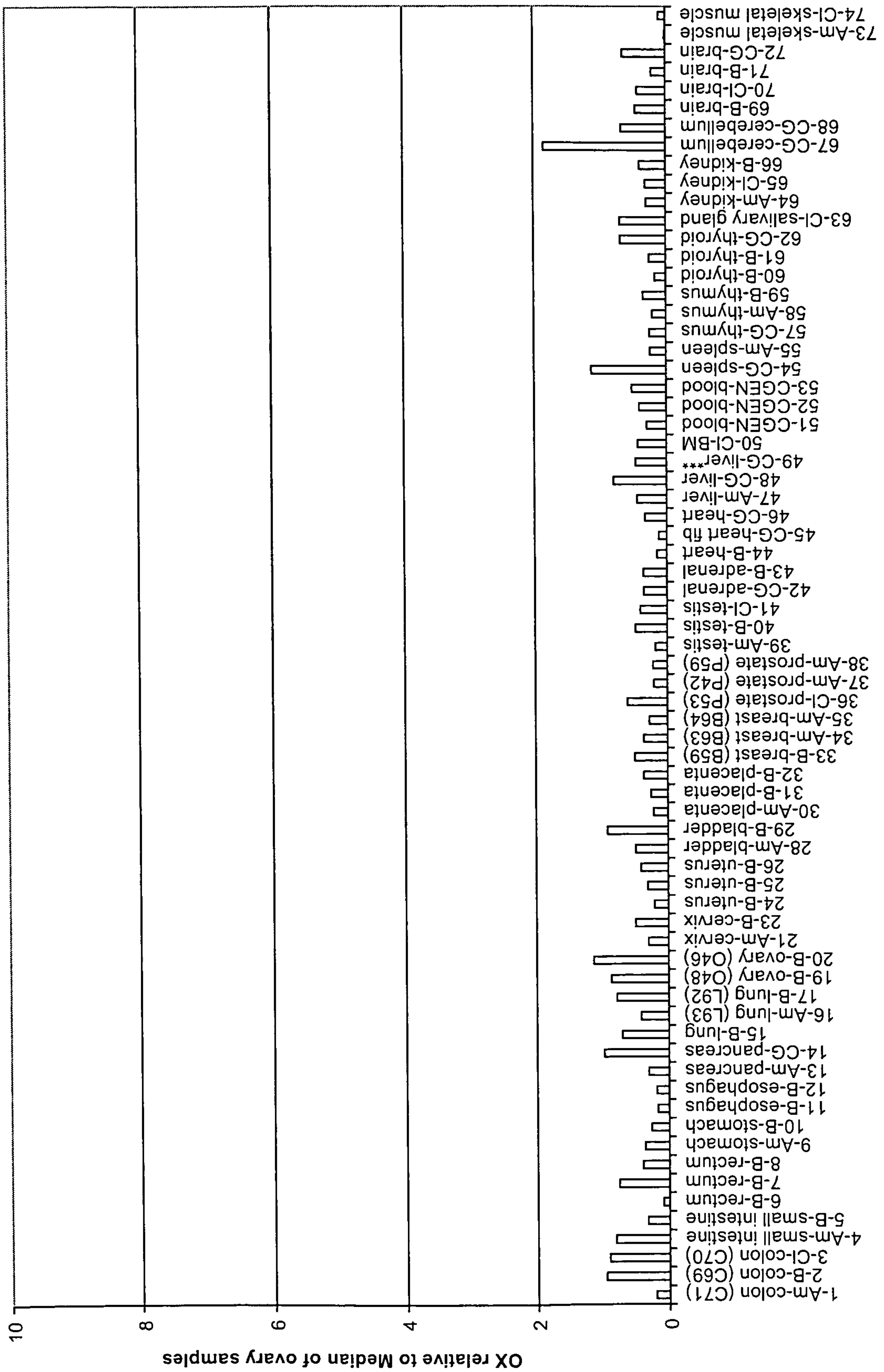
OX relative to Median of ovary samples
0
2
4
6
8
10
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle

FIG. 20

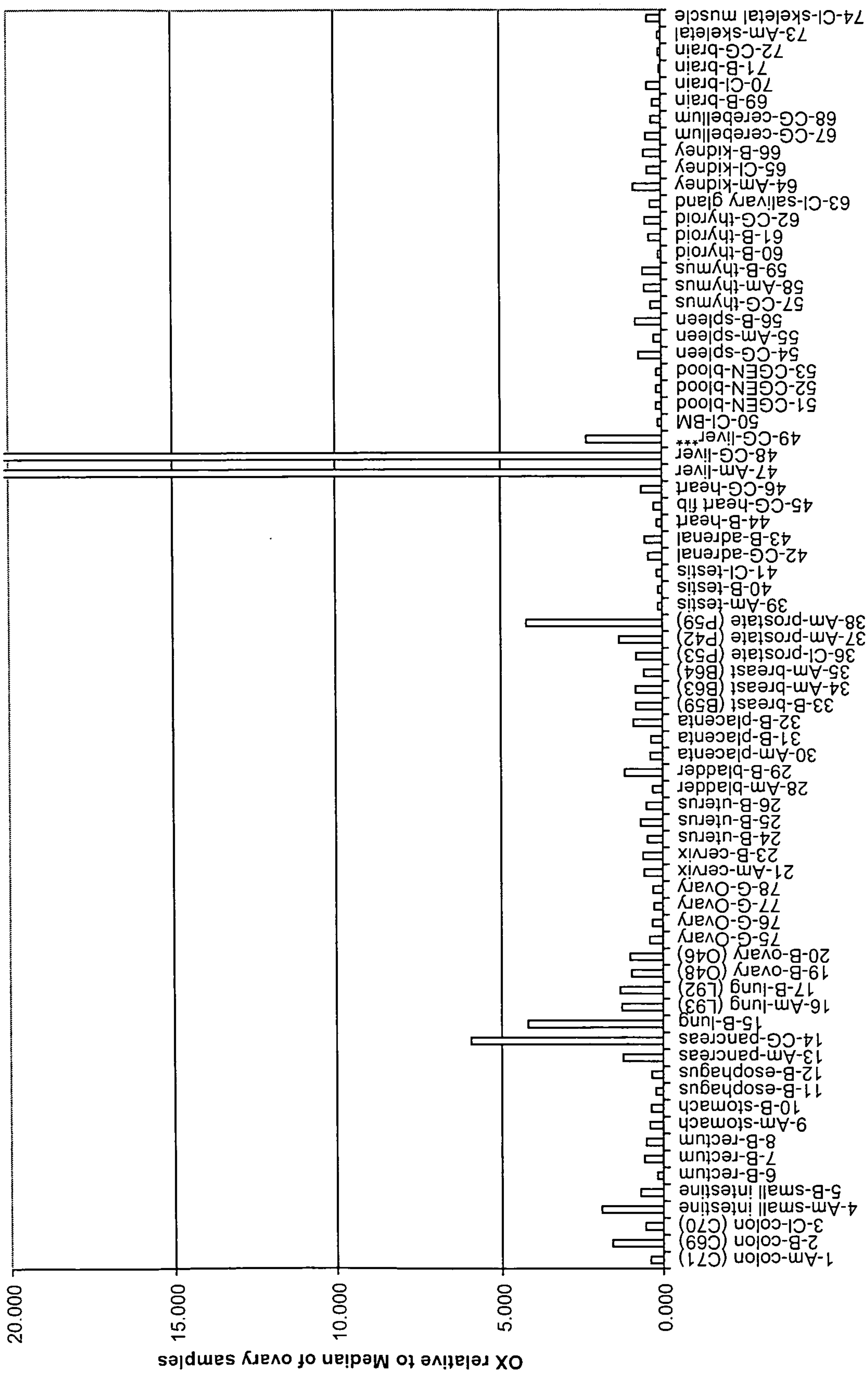
OX relative to Median of ovary samples
0.000
5.000
10.000
15.000
20.000
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
56-B-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal
74-Cl-skeletal muscle

FIG. 21

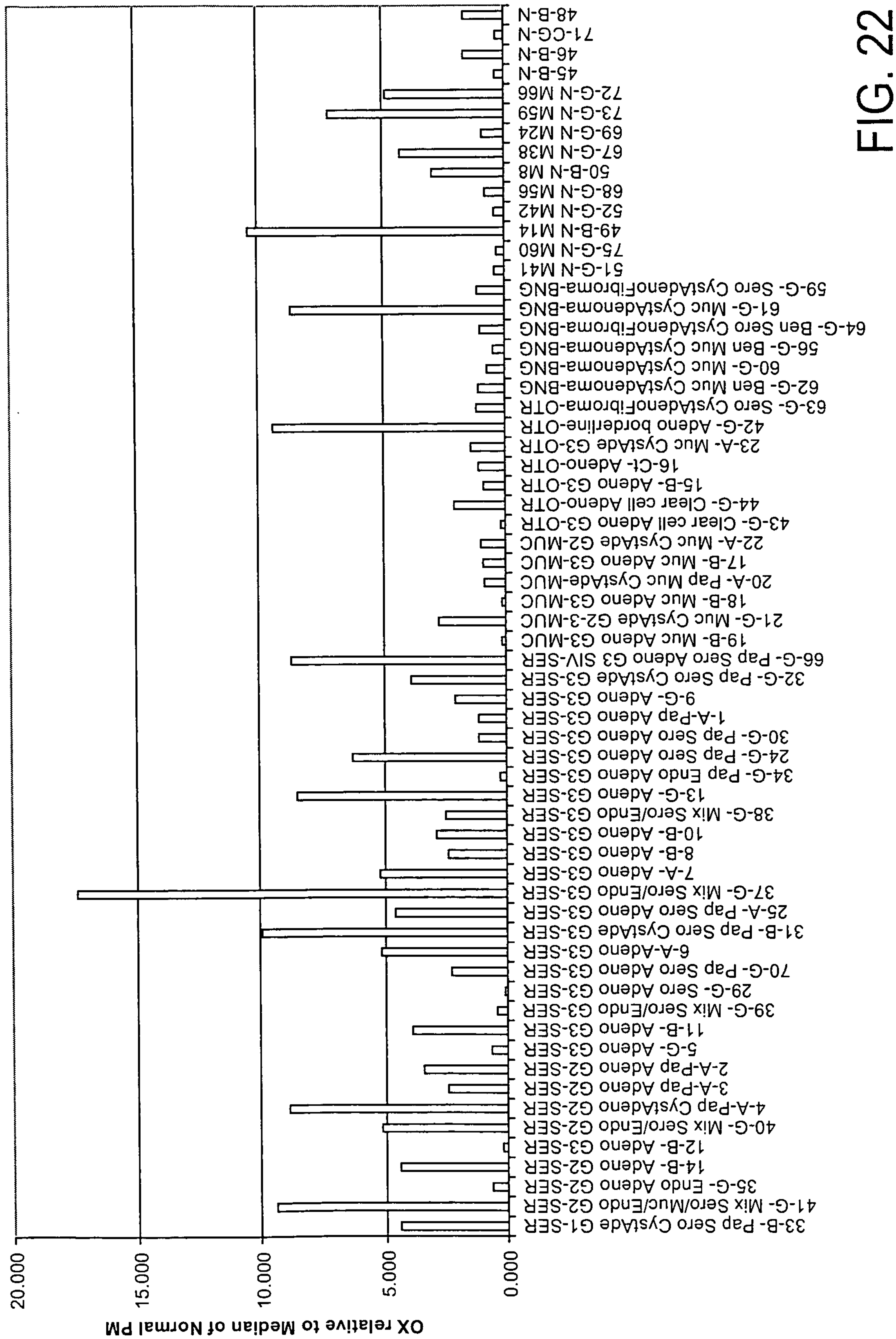

OX relative to Median of Normal PM
20.000
15.000
10.000
5.000
0.000
33-B- Pap Sero CystAde G1-SER
41-G- Mix Sero/Muc/Endo G2-SER
35-G- Endo Adeno G2-SER
14-B- Adeno G2-SER
12-B- Adeno G3-SER
40-G- Mix Sero/Endo G2-SER
4-A-Pap CystAdeno G2-SER
3-A-Pap Adeno G2-SER
2-A-Pap Adeno G2-SER
5-G- Adeno G3-SER
11-B- Adeno G3-SER
39-G- Mix Sero/Endo G3-SER
29-G- Sero Adeno G3-SER
70-G- Pap Sero Adeno G3-SER
6-A-Adeno G3-SER
31-B- Pap Sero CystAde G3-SER
25-A- Pap Sero Adeno G3-SER
37-G- Mix Sero/Endo G3-SER
7-A- Adeno G3-SER
8-B- Adeno G3-SER
10-B- Adeno G3-SER
38-G- Mix Sero/Endo G3-SER
13-G- Adeno G3-SER
34-G- Pap Endo Adeno G3-SER
24-G- Pap Sero Adeno G3-SER
30-G- Pap Sero Adeno G3-SER
1-A-Pap Adeno G3-SER
9-G- Adeno G3-SER
32-G- Pap Sero CystAde G3-SER
66-G- Pap Sero Adeno G3 SIV-SER
19-B- Muc Adeno G3-MUC
21-G- Muc CystAde G2-3-MUC
18-B- Muc Adeno G3-MUC
20-A- Pap Muc CystAde-MUC
17-B- Muc Adeno G3-MUC
22-A- Muc CystAde G2-MUC
43-G- Clear cell Adeno G3-OTR
44-G- Clear cell Adeno-OTR
15-B- Adeno G3-OTR
16-Ct- Adeno-OTR
23-A- Muc CystAde G3-OTR
42-G- Adeno borderline-OTR
63-G- Sero CystAdenoFibroma-OTR
62-G- Ben Muc CystAdenoma-BNG
60-G- Muc CystAdenoma-BNG
56-G- Ben Muc CystAdenoma-BNG
64-G- Ben Sero CystAdenoFibroma-BNG
61-G- Muc CystAdenoma-BNG
59-G- Sero CystAdenoFibroma-BNG
51-G-N M41
75-G-N M60
49-B-N M14
52-G-N M42
68-G-N M56
50-B-N M8
67-G-N M38
69-G-N M24
73-G-N M59
72-G-N M66
45-B-N
46-B-N
71-CG-N
48-B-N


FIG. 22

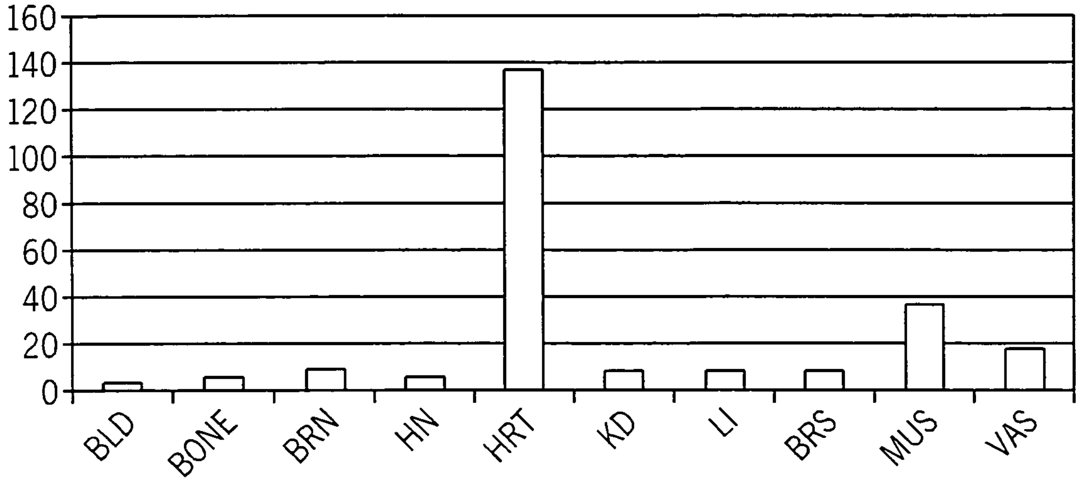


FIG. 23

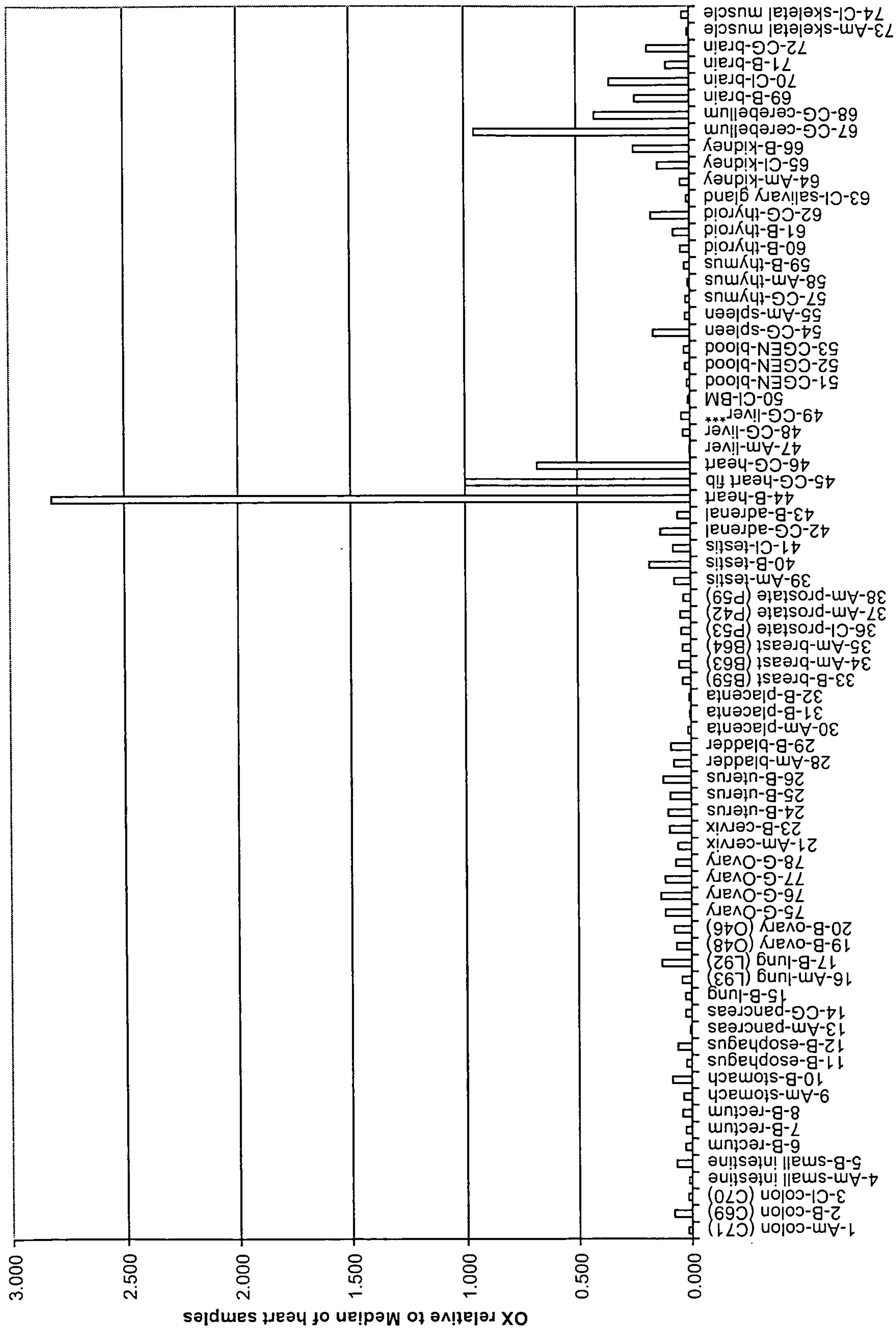
OX relative to Median of heart samples
0.000
0.500
1.000
1.500
2.000
2.500
3.000
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle

FIG. 24

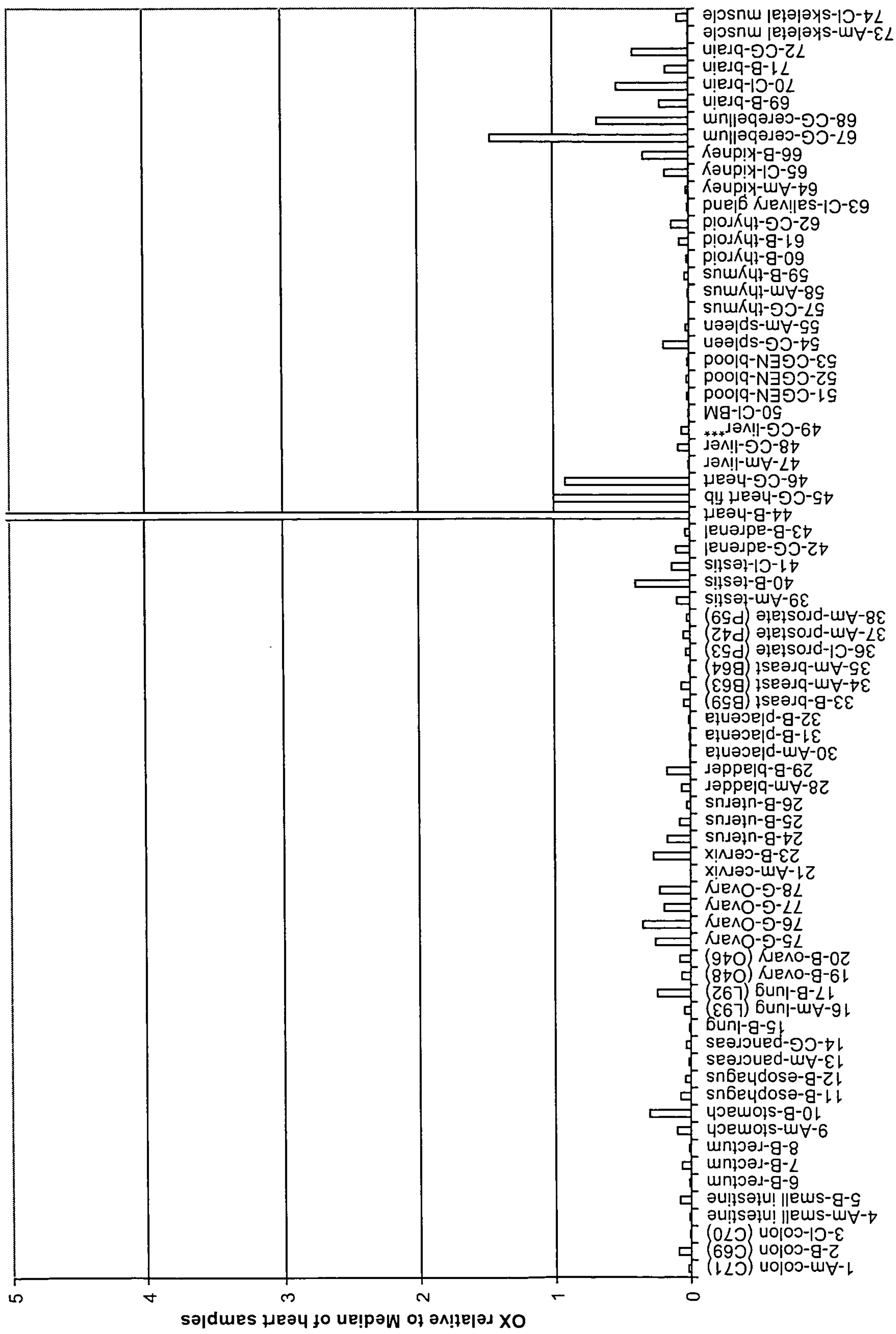
OX relative to Median of heart samples
0
1
2
3
4
5
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle

FIG. 25

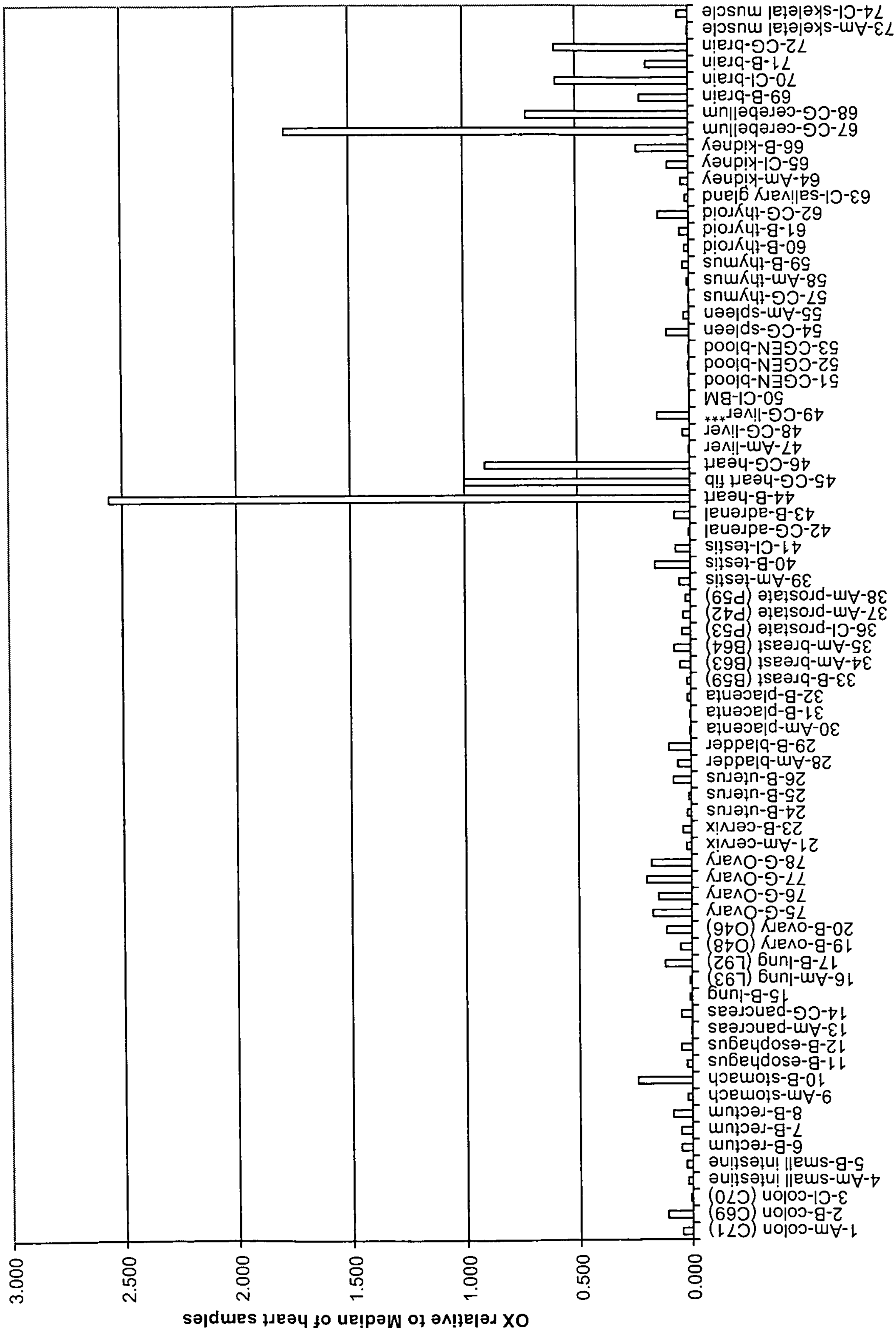
OX relative to Median of heart samples
3.000
2.500
2.000
1.500
1.000
0.500
0.000
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle

FIG. 26

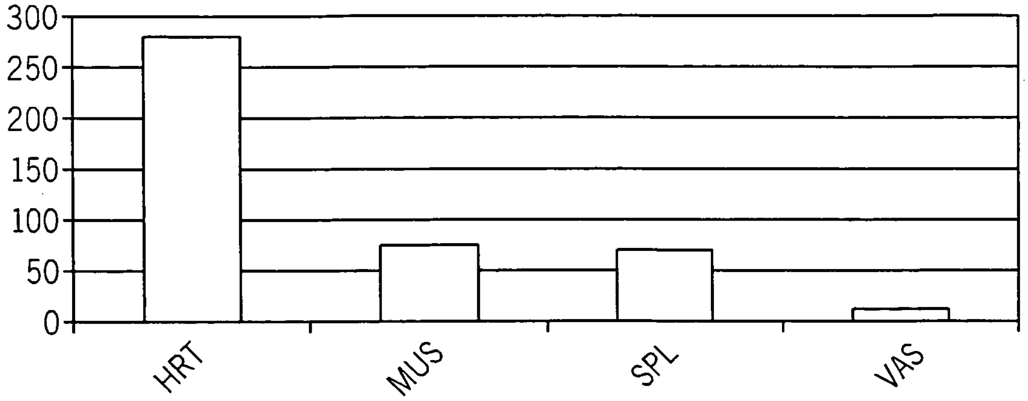


FIG. 27

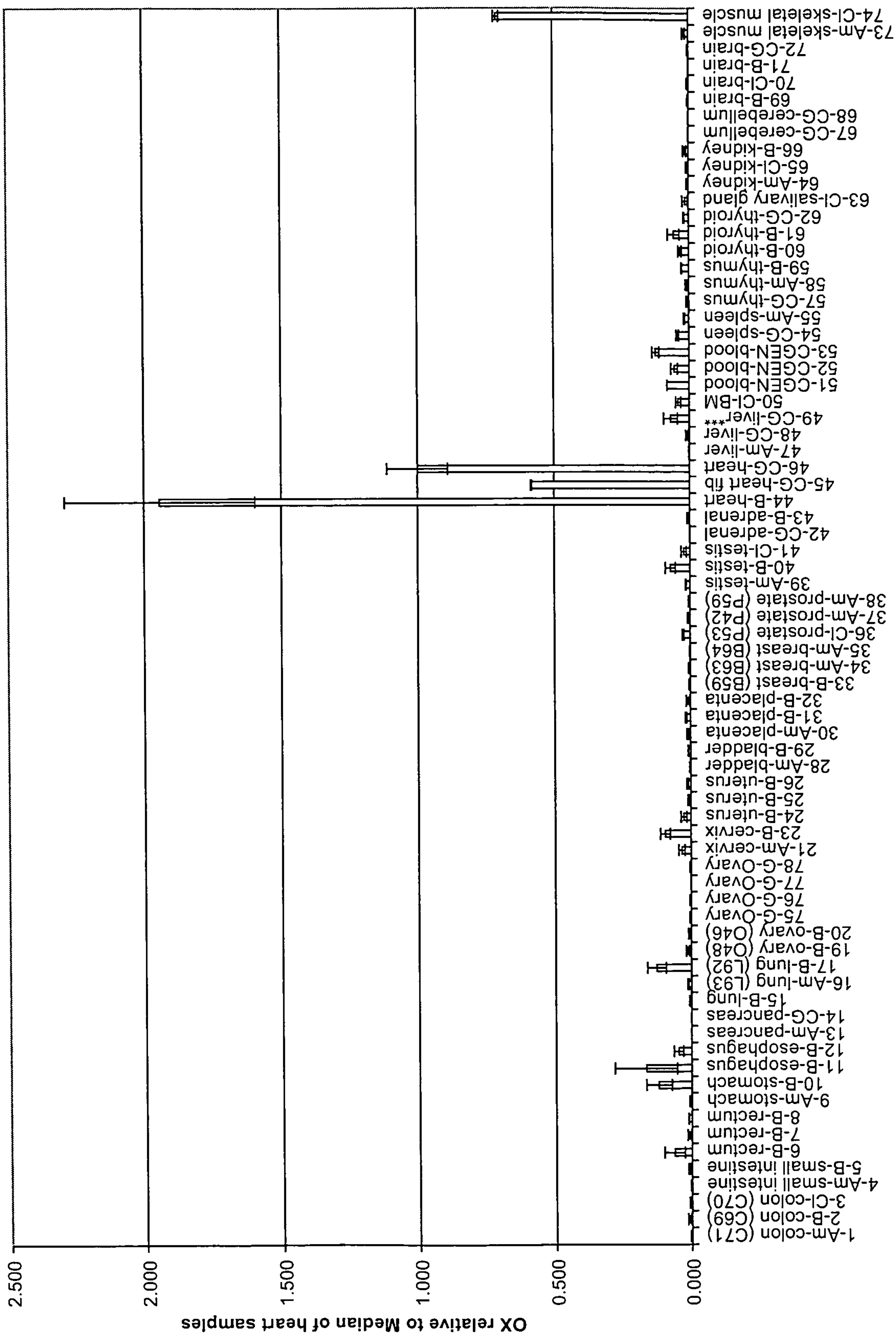
OX relative to Median of heart samples
0.000
0.500
1.000
1.500
2.000
2.500
1-Am-colon (C71)
2-B-colon (C69)
3-Cl-colon (C70)
4-Am-small intesine
5-B-small intesine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle

FIG. 28

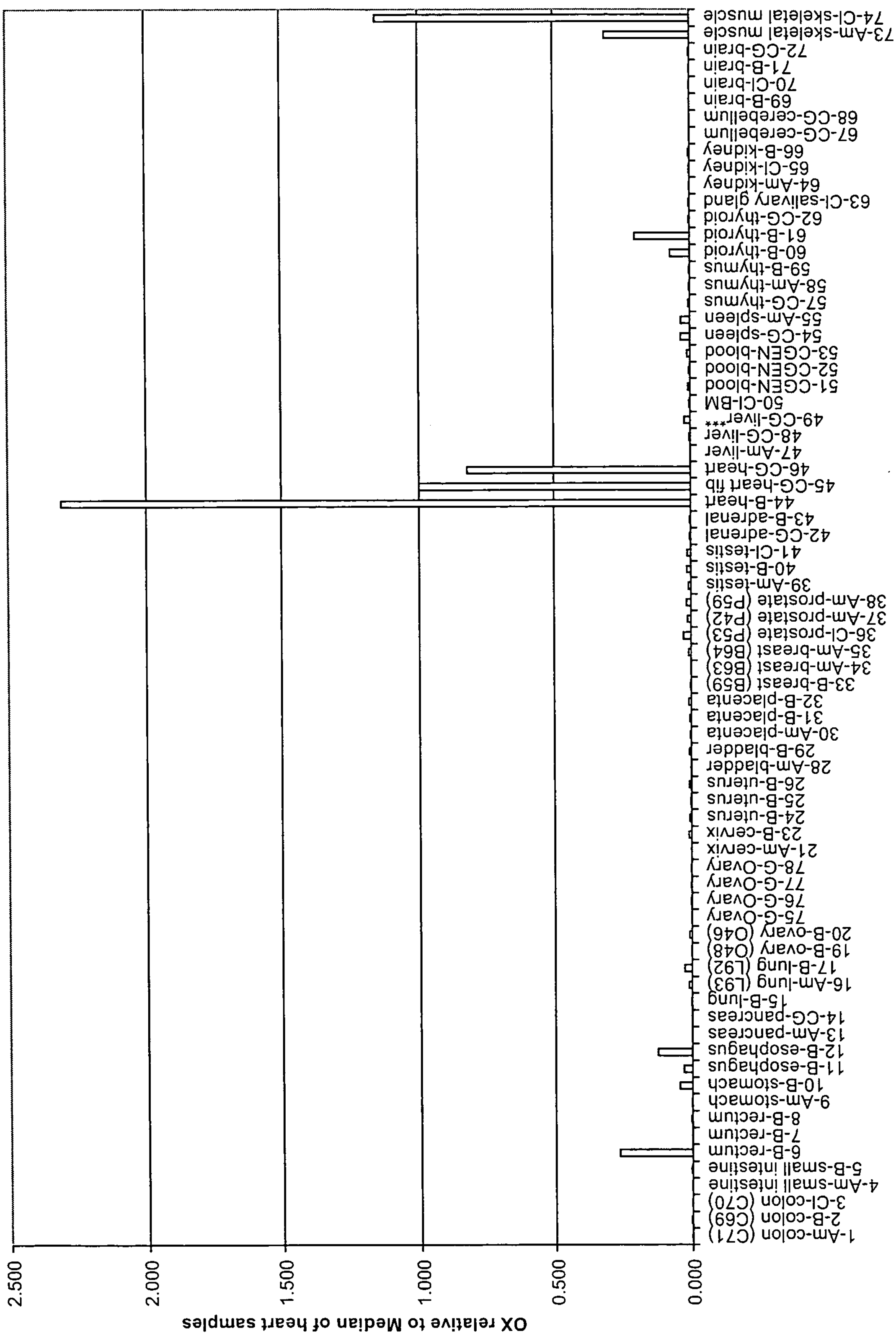
OX relative to Median of heart samples
0.000
0.500
1.000
1.500
2.000
2.500
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle
FIG. 29

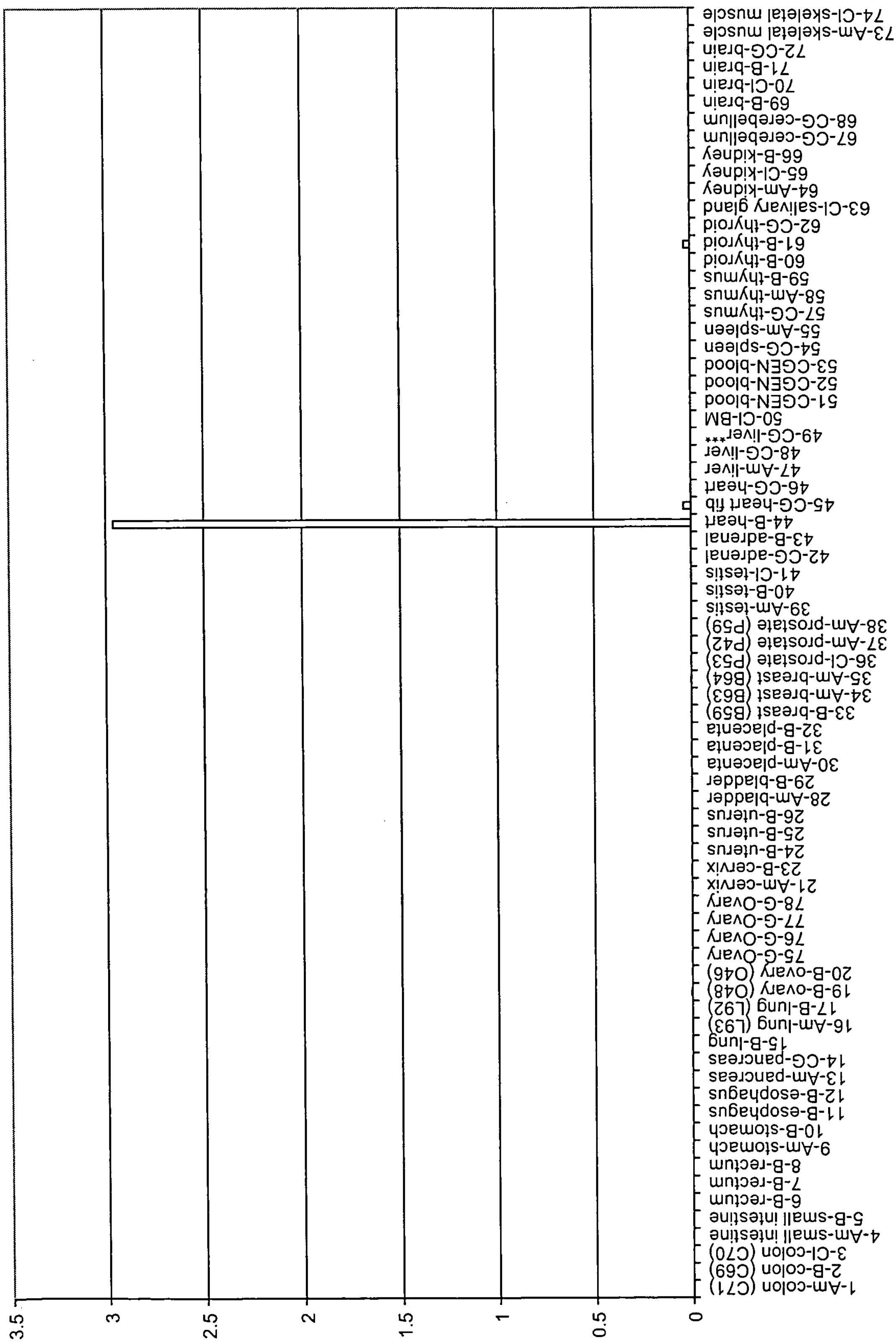

0
0.5
1
1.5
2
2.5
3
3.5
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle


FIG. 30

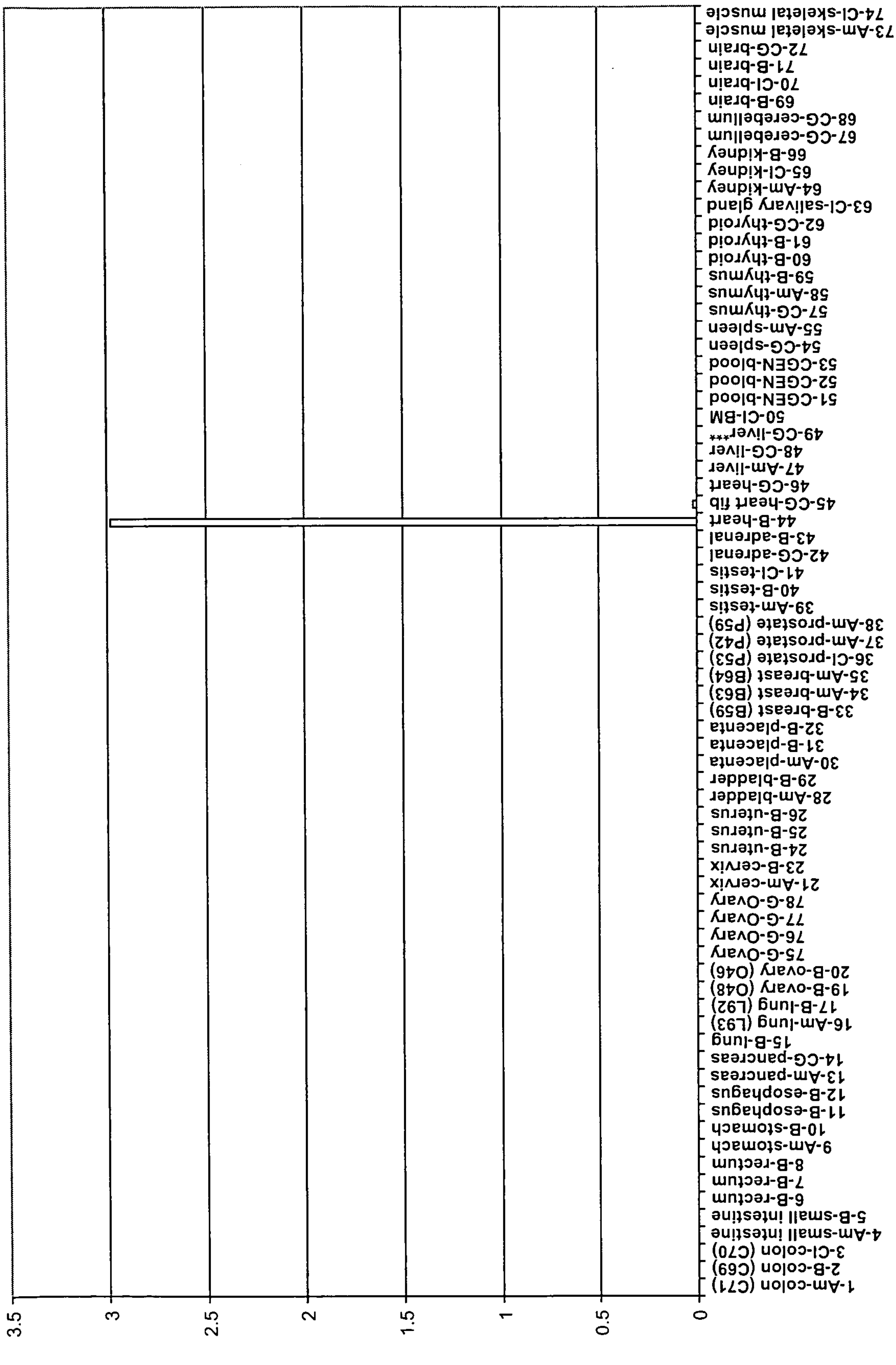
3.5
3
2.5
2
1.5
1
0.5
0
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle

FIG. 31

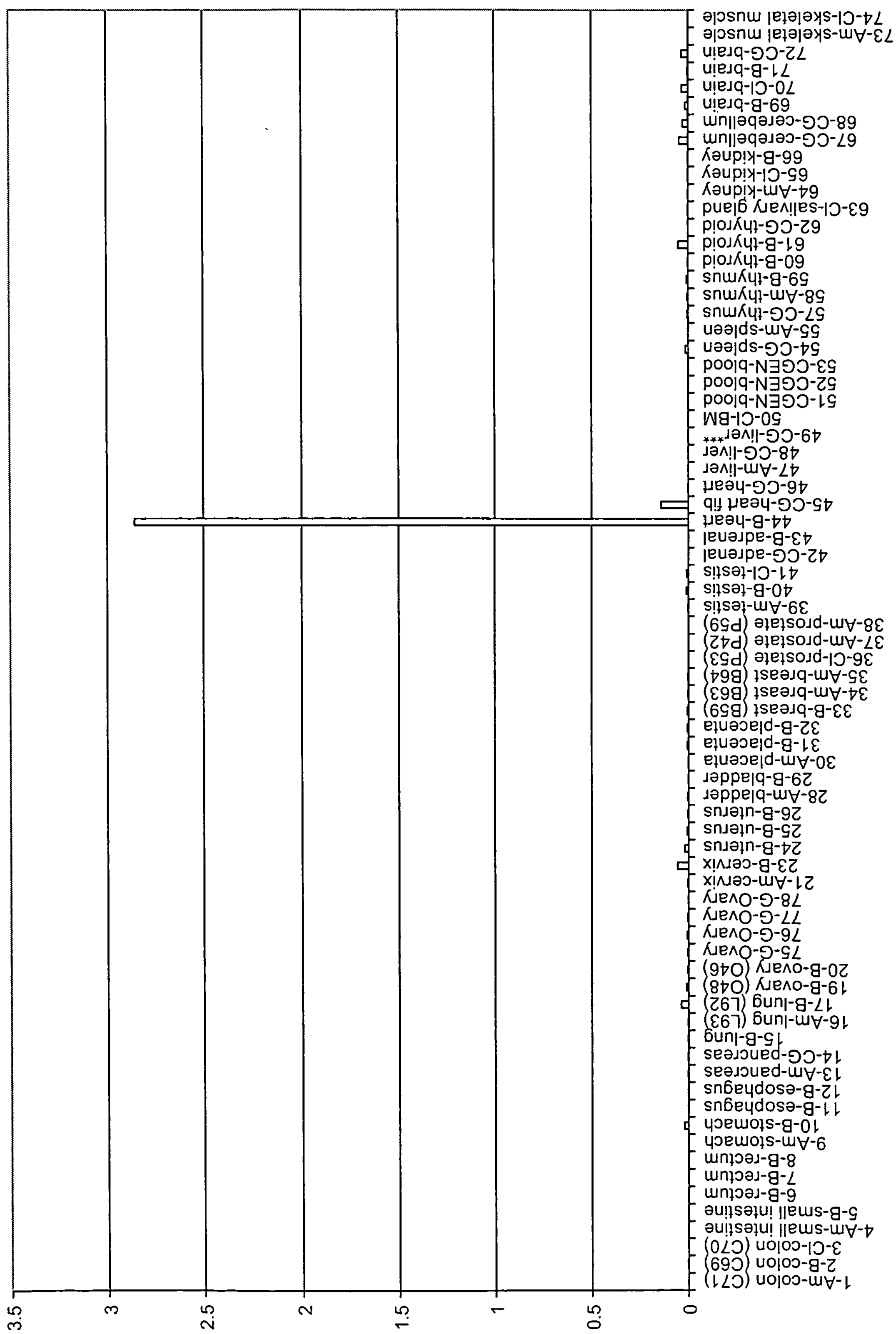
0
0.5
1
1.5
2
2.5
3
3.5
1-Am-colon (C71)
2-B-colon (C69)
3-Cl-colon (C70)
4-Am-small intestine
5-B-small intestine
6-B-rectum
7-B-rectum
8-B-rectum
9-Am-stomach
10-B-stomach
11-B-esophagus
12-B-esophagus
13-Am-pancreas
14-CG-pancreas
15-B-lung
16-Am-lung (L93)
17-B-lung (L92)
19-B-ovary (O48)
20-B-ovary (O46)
75-G-Ovary
76-G-Ovary
77-G-Ovary
78-G-Ovary
21-Am-cervix
23-B-cervix
24-B-uterus
25-B-uterus
26-B-uterus
28-Am-bladder
29-B-bladder
30-Am-placenta
31-B-placenta
32-B-placenta
33-B-breast (B59)
34-Am-breast (B63)
35-Am-breast (B64)
36-Cl-prostate (P53)
37-Am-prostate (P42)
38-Am-prostate (P59)
39-Am-testis
40-B-testis
41-Cl-testis
42-CG-adrenal
43-B-adrenal
44-B-heart
45-CG-heart fib
46-CG-heart
47-Am-liver
48-CG-liver
49-CG-liver***
50-Cl-BM
51-CGEN-blood
52-CGEN-blood
53-CGEN-blood
54-CG-spleen
55-Am-spleen
57-CG-thymus
58-Am-thymus
59-B-thymus
60-B-thyroid
61-B-thyroid
62-CG-thyroid
63-Cl-salivary gland
64-Am-kidney
65-Cl-kidney
66-B-kidney
67-CG-cerebellum
68-CG-cerebellum
69-B-brain
70-Cl-brain
71-B-brain
72-CG-brain
73-Am-skeletal muscle
74-Cl-skeletal muscle

FIG. 32

Europäisches Patentamt
European Patent Office
Office européen des brevets

# EUROPEAN SEARCH REPORT

Application Number
EP 10 15 0046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE Geneseq [Online] 24 March 2000 (2000-03-24), "Full length Human delta3 coding sequence." XP002588000 retrieved from EBI accession no. GSN:AAZ48825 Database accession no. AAZ48825 * compound * ----- | 2,5-7,10 | INV. C07H21/02 |
| X | DATABASE Geneseq [Online] 20 May 2004 (2004-05-20), "Full length human cDNA clone SeqID 3585." XP002588001 retrieved from EBI accession no. GSN:ADL31552 Database accession no. ADL31552 * compound; sequence 3585 * ----- | 2,5-7,10 | |
| X | WO 2004/093804 A2 (FIVE PRIME THERAPEUTICS INC [US]; LEE ERNESTINE [US]; HESTIR KEVIN [US) 4 November 2004 (2004-11-04) * sequence 167 * ----- | 2-8,10 | |

TECHNICAL FIELDS SEARCHED (IPC)
C07K

1

The present search report has been drawn up for all claims

| Place of search | Date of completion of the search | Examiner |
|---|---|---|
| The Hague | 21 June 2010 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 15 0046

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2010

| Patent document cited in search report | Publication date | Patent family member(s) | Publication date |
|---|---|---|---|
| WO 2004093804 A2 | 04-11-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040126767 A
- US 4469863 A
- US 4476301 A
- US 5023243 A
- US 5177196 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466 A
- US 677 A
- US 5476925 A
- US 5519126 A
- US 5536821 A
- US 5541306 A
- US 5550111 A
- US 5563253 A
- US 5571799 A
- US 5587361 A
- US 5625050 A
- US 5034506 A
- US 5166315 A
- US 5185444 A
- US 5214134 A
- US 5216141 A
- US 5235033 A
- US 5264562 A
- US 5264564 A
- US 5405938 A
- US 5434257 A
- US 5466677 A
- US 5470967 A
- US 5489677 A
- US 5541307 A
- US 5561225 A
- US 5596086 A
- US 5602240 A
- US 5610289 A
- US 5608046 A
- US 5618704 A
- US 5623070 A
- US 5663312 A
- US 5633360 A
- US 5677437 A
- US 5677439 A
- US 5539082 A
- US 5714331 A
- US 5719262 A
- US 6303374 B
- US 4873316 A
- EP 264166 A
- US 4683195 A
- US 4683202 A, Mullis
- WO 9001069 A1
- WO 9418219 A
- US 6096551 A
- US 4036945 A, Goldenberg
- US 4331647 A
- US 4946778 A
- US 4816567 A
- US 5545807 A
- US 5545806 A
- US 5569825 A
- US 5625126 A
- US 5633425 A
- US 5661016 A
- US 4366241 A
- US 4376110 A
- US 4517288 A
- US 4837168 A
- US 6696686 B
- US 6625545 B
- US 426002 A
- US 20040101876 A
- WO 2005071058 A
- WO 02088750 A
- WO 00055174 A
- US 2005095592 A
- US 6316213 B
- WO 9832390 A
- WO 04055519 A
- US 6335170 B
- WO 0603527 A
- WO 05024043 A
- WO 03040407 A

**Non-patent literature cited in the description**


- **Altschul S. F. et al.** *J Mol Biol,* 1990, vol. 215 (3), 403-10
- **Altschul S.F. et al.** *Nucleic Acids Res.,* 1997, vol. 25, 3389-3402
- **Singleton et al.** Dictionary of Microbiology and Molecular Biology. 1994
- The Cambridge Dictionary of Science and Technology. 1988
- The Glossary of Genetics. Springer Verlag, 1991
- **Hale ; Marham.** The Harper Collins Dictionary of Biology. 1991
- **Einat Hazkani-Covo ; Erez Levanon ; Galit Rotman ; Dan Graur ; Amit Novik.** Evolution of multicellularity in metazoa: comparative analysis of the subcellular localization of proteins in Saccharomyces, Drosophila and Caenorhabditis. *Cell Biology International,* 2004, vol. 28 (3), 171-8
- **Edgar et al.** *Nucleic Acids Research,* 2002, vol. 30 (1), 207-210
- **Su et al.** *Proc Natl Acad Sci U S A.,* 09 April 2004, vol. 101 (16), 6062-7
- **Hanley et al.** *Radiology,* 1982, vol. 143, 29-36
- **Dowdy ; Wearden.** Statistics for Research. John Wiley & Sons, 1983
- **Fischer et al.** *Intensive Care Med.,* 2003, vol. 29, 1043-51
- **Calame et al.** *Adv. Immunol.,* 1988, vol. 43, 235-275
- **Winoto et al.** *EMBO J.,* vol. 8, 729-733
- **Banerji et al.** *Cell,* 1983, 33729-740
- **Byrne et al.** *Proc. Natl. Acad. Sci. USA,* 1989, vol. 86, 5473-5477
- **Edlunch et al.** *Science,* 1985, vol. 230, 912-916
- **Tonkinson et al.** *Cancer Investigation,* 1996, vol. 14 (1), 54-65
- **Goeddel.** Gene Expression Technology: Methods in Enzymology. Academic Press, 1990, vol. 185
- **Smith ; Johnson.** Gene. Pharmacia Biotech Inc, 1988, vol. 67, 31-40
- **Amrann et al.** *Gene,* 1988, vol. 69, 301-315
- **Gottesman.** Gene Expression Technology: Methods in Enzymology. Academic Press, 1990, vol. 185, 119-128
- **Wada et al.** *Nucl. Acids Res.,* 1992, vol. 20, 2111-2118
- **Baldari et al.** *EMBO J.,* 1987, vol. 6, 229-234
- **Kurjan ; Herskowitz.** *Cell,* 1982, vol. 30, 933-943
- **Schultz et al.** *Gene,* 1987, vol. 54, 113-123
- **Smith et al.** *Mol. Cell. Biol.,* 1983, vol. 3, 2156-2165
- **Lucklow ; Summers.** *Virology,* 1989, vol. 170, 31-39
- **Seed.** *Nature,* 1987, vol. 329, 840
- **Kaufman et al.** *EMBO J.,* 1987, vol. 6, 187-195
- **Sambrook et al.** Molecular Cloning: A Laboratory Manual. Cold Spring Harbor Laboratory Press, 1989
- **Pinkert et al.** *Genes Dev.,* 1987, vol. 1, 268-277
- **Calame ; Eaton.** *Adv. Immunol.,* 1988, vol. 43, 235-275
- **Winoto ; Baltimore.** *EMBO J.,* 1989, vol. 8, 729-733
- **Banerji et al.** *Cell,* 1983, vol. 33, 729-740
- **Queen ; Baltimore.** *Cell,* 1983, vol. 33, 741-748
- **Byrne ; Ruddle.** *Proc. Natl. Acad. Sci. USA,* 1989, vol. 86, 5473-5477
- **Edlund et al.** *Science,* 1985, vol. 230, 912-916
- **Kessel ; Gruss.** *Science,* 1990, vol. 249, 374-379
- **Campes ; Tilghman.** *Genes Dev.,* 1989, vol. 3, 537-546
- **Weintraub et al.** Antisense RNA as a molecular tool for genetic analysis. *Reviews-Trends in Genetics,* 1986, vol. 1 (1
- **Nielsen et al.** *Current Opin. Biotechnol.,* 1999, vol. 10, 71-75
- **Kwoh et al.** *Proc. Natl. Acad. Sci. USA,* 1989, vol. 86, 1173-1177
- **Lizardi et al.** *BioTechnology,* vol. 6, 1197-1202
- **Malek et al.** *Methods Mol. Biol.,* 1994, vol. 28, 253-260
- **Sambrook et al.** Molecular Cloning -A Laboratory Manual. CSH Laboratories, 1989
- **Ausubel et al.** Current Protocols in Molecular Biology. John Wiley & Sons Inc, 1989
- **John Morrow Stewart ; Janis Dillaha Young.** Solid Phase Peptide Syntheses. Pierce Chemical Company
- **Creighton T.** Proteins, structures and molecular principles. WH Freeman and Co, 1983
- **Bitter et al.** *Methods in Enzymol.,* 1987, vol. 153, 516-544
- **Studier et al.** *Methods in Enzymol.,* 1990, vol. 185, 60-89
- **Brisson et al.** *Nature,* 1984, vol. 310, 511-514
- **Takamatsu et al.** *EMBO J.,* 1987, vol. 6, 307-311
- **Coruzzi et al.** *EMBO J.,* 1984, vol. 3, 1671-1680
- **Brogli et al.** *Science,* 1984, vol. 224, 838-843

- **Gurley et al.** *Mol. Cell. Biol.,* 1986, vol. 6, 559-565
- **Weissbach ; Weissbach.** Methods for Plant Molecular Biology. Academic Press, 1988, 421-463
- **Harlow ; Lane.** Antibodies: A Laboratory Manual. Cold Spring Harbor Laboratory, 1988
- **Porter, R. R.** *Biochem. J.,* 1959, vol. 73, 119-126
- **Inbar et al.** *Proc. Nat'1 Acad. Sci. USA,* 1972, vol. 69, 2659-62
- **Whitlow ; Filpula.** *Methods,* 1991, vol. 2, 97-105
- **Bird et al.** *Science,* 1988, vol. 242, 423-426
- **Pack et al.** *Bio/Technology,* 1993, vol. 11, 1271-77
- **Larrick ; Fry.** *Methods,* 1991, vol. 2, 106-10
- **Jones et al.** *Nature,* 1986, vol. 321, 522-525
- **Riechmann et al.** *Nature,* 1988, vol. 332, 323-327
- **Verhoeyen et al.** *Science,* 1988, vol. 239, 1534-1536
- **Hoogenboom ; Winter.** *J. Mol. Biol.,* 1991, vol. 227, 381
- **Marks et al.** *J. Mol. Biol.,* 1991, vol. 222, 581
- **Cole et al.** Monoclonal Antibodies and Cancer Therapy. Alan R. Liss, 1985, 77
- **Boerner et al.** *J. Immunol.,* 1991, vol. 147 (1), 86-95
- **Marks et al.** *Bio/Technology,* 1992, vol. 10, 779-783
- **Lonberg et al.** *Nature,* 1994, vol. 368, 856-859
- **Morrison.** *Nature,* 1994, vol. 368, 812-13
- **Fishwild et al.** *Nature Biotechnology,* 1996, vol. 14, 845-51
- **Neuberger.** *Nature Biotechnology,* vol. 14, 826
- **Lonberg ; Huszar.** *Intern. Rev. Immunol.,* 1995, vol. 13, 65-93
- **Young AC et al.** The three-dimensional structures of a polysaccharide binding antibody to Cryptococcus neoformans and its complex with a peptide from a phage display library: implications for the identification of peptide mimotopes. *J Mol Biol,* 12 December 1997, vol. 274 (4), 622-34
- **Giebel LB et al.** Screening of cyclic peptide phage libraries identifies ligands that bind streptavidin with high affinities. *Biochemistry,* 28 November 1995, vol. 34 (47), 15430-5
- **Davies EL et al.** Selection of specific phage-display antibodies using libraries derived from chicken immunoglobulin genes. *J Immunol Methods,* 12 October 1995, vol. 186 (1), 125-35
- **Jones C RT.** Current trends in molecular recognition and bioseparation. *J Chromatogr A,* 14 July 1995, vol. 707 (1), 3-22
- **Deng SJ et al.** Basis for selection of improved carbohydrate-binding single-chain antibodies from synthetic gene libraries. *Proc Natl Acad Sci U S A,* 23 May 1995, vol. 92 (11), 4992-6
- **Deng SJ et al.** Selection of antibody single-chain variable fragments with improved carbohydrate binding by phage display. *J Biol Chem,* 01 April 1994, vol. 269 (13), 9533-8
- **Boguski et al.** *Nat Genet.,* August 1993, vol. 4 (4), 332-3
- **Sorek, R. ; Ast, G. ; Graur, D.** Alu-containing exons are alternatively spliced. *Genome Res,* 2002, vol. 12, 1060-7
- **A. Shoshan et al.** Optical technologies and informatics. *Proceedings of SPIE,* 2001, vol. 4266, 86-95

SEQUENCE LISTING

<110> Compugen Ltd

<120> NOVEL NUCLEOTIDE AND AMINO ACID SEQUENCES, AND METHODS OF USE THEREOF FOR DIAGNOSIS

<130> COMP/026 PCT

<160> 398

<210> 1
<211> 1536
<212> DNA
<213> Homo sapiens

<400> 1

```
gtgacgcgag gctctgcgga gaccaggagt cagactgtag gacgacctcg ggtcccacgt     60
gtccccggta ctcgccggcc ggagcccccg gcttcccggg gccgggggac cttagcggca    120
cccacacaca gcctactttc caagcggagc catgtctggt aacggcaatg cggctgcaac    180
ggcggaagaa aacagcccaa agatgagagt gattcgcgtg ggtacccgca agagccagct    240
tgctcgcata cagacggaca gtgtggtggc aacattgaaa gcctcgtacc ctggcctgca    300
gtttgaaatc attgctatgt ccaccacagg ggacaagatt cttgatactg cactctctaa    360
gattggagag aaaagcctgt ttaccaagga gcttgaacat gccctggaga agaatgaagt    420
ggacctggtt gttcactcct tgaaggacct gcccactgtg cttcctcctg gcttcaccat    480
cggagccatc tgcaagcggg aaaaccctca tgatgctgtt gtctttcacc caaaatttgt    540
tgggaagacc ctagaaaccc tgccagagaa gagtgtggtg ggaaccagct ccctgcgaag    600
agcagcccag ctgcagagaa agttcccgca tctggagttc aggagtattc ggggaaacct    660
caacacccgg cttcggaagc tggacgagca gcaggagttc agtgccatca tcctggcaac    720
agctggcctg cagcgcatgg gctggcacaa ccgggtgggg cagatcctgc accctgagga    780
atgcatgtat gctgtgggcc aggggggcctt gggcgtggaa gtgcgagcca aggaccagga    840
catcttggat ctggtgggtg tgctgcacga tcccgagact ctgcttcgct gcatcgctga    900
aagggccttc ctgaggcacc tggaaggagg ctgcagtgtg ccagtagccg tgcatacagc    960
tatgaaggat gggcaactgt acctgactgg aggagtctgg agtctagacg gctcagatag   1020
catacaagag accatgcagg ctaccatcca tgtccctgcc cagcatgaag atggccctga   1080
ggatgaccca cagttggtag gcatcactgc tcgtaacatt ccacgagggc cccagttggc   1140
tgcccagaac ttgggcatca gcctggccaa cttgttgctg agcaaaggag ccaaaaacat   1200
cctggatgtt gcacggcagc ttaacgatgc ccattaactg gtttgtgggg cacagatgcc   1260
tgggttgctg ctgtccagtg cctacatccc gggcctcagt gccccattct cactgctatc   1320
```

tggggagtga ttaccccggg agactgaact gcagggttca agccttccag ggatttgcct 1380

caccttgggg ccttgatgac tgccttgcct cctcagtatg tgggggcttc atctctttag 1440

agaagtccaa gcaacagcct ttgaatgtaa ccaatcctac taataaacca gttctgaagg 1500

taaaaaaaaa aaaaaaaaaa aaaaaaaaaa aaaaaa 1536

<210> 2
<211> 19
<212> DNA
<213> Artificial Organism

<220>
<223> Synthetic oligonucleotide

<400> 2

tgagagtgat tcgcgtggg 19

<210> 3
<211> 21
<212> DNA
<213> Artificial Organism

<220>
<223> Synthetic oligonucleotide

<400> 3

ccagggtacg aggctttcaa t 21

<210> 4
<211> 91
<212> DNA
<213> Artificial Organism

<220>
<223> Synthetic oligonucleotide

<400> 4

tgagagtgat tcgcgtgggt acccgcaaga gccagcttgc tcgcatacag acggacagtg 60

tggtggcaac attgaaagcc tcgtaccctg g 91

<210> 5
<211> 1261
<212> DNA
<213> Homo sapiens

<400> 5

ccggccggct ccgttatggc gacccgcagc cctggcgtcg tgattagtga tgatgaacca 60

ggttatgacc ttgatttatt ttgcatacct aatcattatg ctgaggattt ggaaagggtg 120

tttattcctc atggactaat tatggacagg actgaacgtc ttgctcgaga tgtgatgaag 180

```
gagatgggag gccatcacat tgtagccctc tgtgtgctca agggggcta taaattcttt     240

gctgacctgc tggattacat caaagcactg aatagaaata gtgatagatc cattcctatg    300

actgtagatt ttatcagact gaagagctat tgtaatgacc agtcaacagg ggacataaaa    360

gtaattggtg gagatgatct ctcaacttta actggaaaga atgtcttgat tgtggaagat    420

ataattgaca ctggcaaaac aatgcagact ttgctttcct tggtcaggca gtataatcca    480

aagatggtca aggtcgcaag cttgctggtg aaaaggaccc cacgaagtgt tggatataag    540

ccagactttg ttggatttga aattccagac aagtttgttg taggatatgc ccttgactat    600

aatgaatact tcagggattt gaatcatgtt tgtgtcatta gtgaaactgg aaaagcaaaa    660

tacaaagcct aagatgagag ttcaagttga gtttggaaac atctggagtc ctattgacat    720

cgccagtaaa attatcaatg ttctagttct gtggccatct gcttagtaga gctttttgca    780

tgtatcttct aagaatttta tctgttttgt actttagaaa tgtcagttgc tgcattccta    840

aactgtttat ttgcactatg agcctataga ctatcagttc cctttgggcg gattgttgtt    900

taacttgtaa atgaaaaaat tctcttaaac cacagcacta ttgagtgaaa cattgaactc    960

atatctgtaa gaaataaaga gaagatatat tagtttttta attggtattt taatttttat   1020

atatgcagga aagaatagaa gtgattgaat attgttaatt ataccaccgt gtgttagaaa   1080

agtaagaagc agtcaatttt cacatcaaag acagcatcta agaagttttg ttctgtcctg   1140

gaattatttt agtagtgttt cagtaatgtt gactgtattt tccaacttgt tcaaattatt   1200

accagtgaat ctttgtcagc agttcccttt taaatgcaaa tcaataaatt cccaaaaatt   1260

t                                                                   1261


<210>  6
<211>  21
<212>  DNA
<213>  Artificial Organism

<220>
<223>  Synthetic oligonucleotide

<400> 6

tgacactggc aaaacaatgc a                                               21


<210>  7
<211>  21
<212>  DNA
<213>  Artificial Organism

<220>
<223>  Synthetic oligonucleotide

<400> 7

ggtccttttc accagcaagc t                                               21
```

<210> 8
<211> 94
<212> DNA
<213> Artificial Organism

<220>
<223> Synthetic oligonucleotide

<400> 8

tgacactggc aaaacaatgc agactttgct ttccttggtc aggcagtata atccaaagat 60

ggtcaaggtc gcaagcttgc tggtgaaaag gacc 94

<210> 9
<211> 1261
<212> DNA
<213> Homo sapiens

<400> 9

cttttgcgtc gccagccgag ccacatcgct cagacaccat ggggaaggtg aaggtcggag 60

tcaacggatt tggtcgtatt gggcgcctgg tcaccagggc tgcttttaac tctggtaaag 120

tggatattgt tgccatcaat gacccettca ttgacctcaa ctacatggtt tacatgttcc 180

aatatgattc cacccatggc aaattccatg gcaccgtcaa ggctgagaac gggaagcttg 240

tcatcaatgg aaatcccatc accatcttcc aggagcgaga tccctccaaa atcaagtggg 300

gcgatgctgg cgctgagtac gtcgtggagt ccactggcgt cttcaccacc atggagaagg 360

ctggggctca tttgcagggg ggagccaaaa gggtcatcat ctctgccccc tctgctgatg 420

cccccatgtt cgtcatgggt gtgaaccatg agaagtatga caacagcctc aagatcatca 480

gcaatgcctc ctgcaccacc aactgcttag cacccctggc caaggtcatc catgacaact 540

ttggtatcgt ggaaggactc atgaccacag tccatgccat cactgccacc cagaagactg 600

tggatggccc ctccgggaaa ctgtggcgtg atggccgcgg ggctctccag aacatcatcc 660

ctgcctctac tggcgctgcc aaggctgtgg gcaaggtcat ccctgagctg aacgggaagc 720

tcactggcat ggccttccgt gtccccactg ccaacgtgtc agtggtggac ctgacctgcc 780

gtctagaaaa acctgccaaa tatgatgaca tcaagaaggt ggtgaagcag gcgtcggagg 840

gcccccctcaa gggcatcctg ggctacactg agcaccaggt ggtctcctct gacttcaaca 900

gcgacaccca ctcctccacc tttgacgctg gggctggcat tgccctcaac gaccactttg 960

tcaagctcat ttcctggtat gacaacgaat ttggctacag caacagggtg gtggacctca 1020

tggcccacat ggcctccaag gagtaagacc cctggaccac cagccccagc aagagcacaa 1080

gaggaagaga gagaccctca ctgctgggga gtccctgcca cactcagtcc cccaccacac 1140

tgaatctccc ctcctcacag ttgccatgta gaccccttga agaggggagg ggcctaggga 1200

gccgcacctt gtcatgtacc atcaataaag taccctgtgc tcaaccaaaa aaaaaaaaaa 1260

a 1261

<210> 10
<211> 20
<212> DNA
<213> Artificial Organism

<220>
<223> Synthetic oligonucleotide

<400> 10

tgcaccacca actgcttagc 20

<210> 11
<211> 19
<212> DNA
<213> Artificial Organism

<220>
<223> Synthetic oligonucleotide

<400> 11

ccatcacgcc acagtttcc 19

<210> 12
<211> 116
<212> DNA
<213> Artificial Organism

<220>
<223> Synthetic oligonucleotide

<400> 12

tgcaccacca actgcttagc acccctggcc aaggtcatcc atgacaactt tggtatcgtg 60

gaaggactca tgaccacagt ccatgccatc actgccaccc agaagactgt ggatgg 116

<210> 13
<211> 2650
<212> DNA
<213> Homo sapiens

<400> 13

agggacagcc cagaggaggc gtggccacgc tgccggcgga agtggagccc tccgcgagcg 60

cgcgaggccg ccggggcagg cggggaaacc ggacagtagg ggcggggccg ggccggcgat 120

ggggatgcgg gagcactacg cggagctgca cccgtgcccg ccggaattgg ggatgcagag 180

cagcggcagc gggtatggca ggcagccggc gggccggcct ccagcgcagg tgcccgagag 240

gcaggggctg gcctgggatg cgcgcgcacc tgccctcgcc ccgccccgcc cgcacgaggg 300

gtggtggccg aggccccgcc ccgcacgcct cgcctgaggc gggtccgctc agcccaggcg 360
cccgcccccg cccccgccga ttaaatgggc cggcggggct cagcccccgg aaacggtcgt 420
aacttcgggg ctgcgagcgc ggagggcgac gacgacgaag cgcagacagc gtcatggcag 480
agcaggtggc cctgagccgg acccaggtgt gcgggatcct gcgggaagag cttttccagg 540
gcgatgcctt ccatcagtcg gatacacaca tattcatcat catgggtgca tcgggtgacc 600
tggccaagaa gaagatctac cccaccatct ggtggctgtt ccgggatggc cttctgcccg 660
aaaacacctt catcgtgggc tatgcccgtt cccgcctcac agtggctgac atccgcaaac 720
agagtgagcc cttcttcaag gccaccccag aggagaagct caagctggag gacttctttg 780
cccgcaactc ctatgtggct ggccagtacg atgatgcagc ctcctaccag cgcctcaaca 840
gccacatgga tgccctccac ctggggtcac aggccaaccg cctcttctac ctggccttgc 900
ccccgaccgt ctacgaggcc gtcaccaaga acattcacga gtcctgcatg agccagatag 960
gctggaaccg catcatcgtg gagaagccct tcgggaggga cctgcagagc tctgaccggc 1020
tgtccaacca catctcctcc ctgttccgtg aggaccagat ctaccgcatc gaccactacc 1080
tgggcaagga gatggtgcag aacctcatgg tgctgagatt tgccaacagg atcttcggcc 1140
ccatctggaa ccgggacaac atcgcctgcg ttatcctcac cttcaaggag ccctttggca 1200
ctgagggtcg cgggggctat ttcgatgaat ttgggatcat ccgggacgtg atgcagaacc 1260
acctactgca gatgctgtgt ctggtggcca tggagaagcc cgcctccacc aactcagatg 1320
acgtccgtga tgagaaggtc aaggtgttga aatgcatctc agaggtgcag gccaacaatg 1380
tggtcctggg ccagtacgtg gggaaccccg atggagaggg cgaggccacc aaagggtacc 1440
tggacgaccc cacggtgccc cgcgggtcca ccaccgccac ttttgcagcc gtcgtcctct 1500
atgtggagaa tgagaggtgg gatggggtgc ccttcatcct gcgctgcggc aaggccctga 1560
acgagcgcaa ggccgaggtg aggctgcagt tccatgatgt ggccggcgac atcttccacc 1620
agcagtgcaa gcgcaacgag ctggtgatcc gcgtgcagcc caacgaggcc gtgtacacca 1680
agatgatgac caagaagccg ggcatgttct tcaaccccga ggagtcggag ctggacctga 1740
cctacggcaa cagatacaag aacgtgaagc tccctgacgc ctacgagcgc ctcatcctgg 1800
acgtcttctg cgggagccag atgcacttcg tgcgcagcga cgagctccgt gaggcctggc 1860
gtattttcac cccactgctg caccagattg agctggagaa gcccaagccc atcccctata 1920
tttatggcag ccgaggcccc acggaggcag acgagctgat gaagagagtg ggtttccagt 1980
atgagggcac ctacaagtgg gtgaaccccc acaagctctg agccctgggc acccacctcc 2040
acccccgcca cggccaccct ccttcccgcc gcccgacccc gagtcgggag gactccggga 2100
ccattgacct cagctgcaca ttcctggccc cgggctctgg ccaccctggc ccgcccctcg 2160

ctgctgctac tacccgagcc cagctacatt cctcagctgc caagcactcg agaccatcct 2220

ggcccctcca gaccctgcct gagcccagga gctgagtcac ctcctccact cactccagcc 2280

caacagaagg aaggaggagg gcgcccattc gtctgtccca gagcttattg gccactgggt 2340

ctcactcctg agtggggcca gggtgggagg gagggacaag gggaggaaa ggggcgagca 2400

cccacgtgag agaatctgcc tgtggccttg cccgccagcc tcagtgccac ttgacattcc 2460

ttgtcaccag caacatctcg agccccctgg atgtcccctg tcccaccaac tctgcactcc 2520

atggccaccc cgtgccaccc gtaggcagcc tctctgctat aagaaaagca gacgcagcag 2580

ctgggacccc tcccaacctc aatgccctgc cattaaatcc gcaaacagcc aaaaaaaaaa 2640

aaaaaaaaaa 2650

<210> 14
<211> 20
<212> DNA
<213> Artificial Organism

<220>
<223> Synthetic oligonucleotide

<400> 14

gaggccgtca ccaagaacat 20

<210> 15
<211> 19
<212> DNA
<213> Artificial Organism

<220>
<223> Synthetic oligonucleotide

<400> 15

ggacagccgg tcagagctc 19

<210> 16
<211> 111
<212> DNA
<213> Artificial Organism

<220>
<223> Synthetic oligonucleotide

<400> 16

gaggccgtca ccaagaacat tcacgagtcc tgcatgagcc agataggctg gaaccgcatc 60

atcgtggaga agcccttcgg gagggacctg cagagctctg accggctgtc c 111

<210> 17
<211> 541
<212> DNA

<213>  Homo sapiens

<400> 17

```
cttttcgatc cgccatctgc ggtggagccg ccaccaaaat gcagattttc gtgaaaaccc     60
ttacggggaa gaccatcacc ctcgaggttg aaccctcgga tacgatagaa aatgtaaagg    120
ccaagatcca ggataaggaa ggaattcctc ctgatcagca gagactgatc tttgctggca    180
agcagctgga agatggacgt actttgtctg actacaatat tcaaaaggag tctactcttc    240
atcttgtgtt gagacttcgt ggtggtgcta agaaaaggaa gaagaagtct tacaccactc    300
ccaagaagaa taagcacaag agaaagaagg ttaagctggc tgtcctgaaa tattataagg    360
tggatgagaa tggcaaaatt agtcgccttc gtcgagagtg cccttctgat gaatgtggtg    420
ctggggtgtt tatggcaagt cactttgaca gacattattg tggcaaatgt tgtctgactt    480
actgtttcaa caaaccagaa gacaagtaac tgtatgagtt aataaaagac atgaactaac    540
a                                                                    541
```

<210>  18
<211>  20
<212>  DNA
<213>  Artificial Organism

<220>
<223>  Synthetic oligonucleotide

<400> 18

```
ctggcaagca gctggaagat                                                 20
```

<210>  19
<211>  23
<212>  DNA
<213>  Artificial Organism

<220>
<223>  Synthetic oligonucleotide

<400> 19

```
tttcttagca ccaccacgaa gtc                                             23
```

<210>  20
<211>  101
<212>  DNA
<213>  Artificial Organism

<220>
<223>  Synthetic oligonucleotide

<400> 20

```
ctggcaagca gctggaagat ggacgtactt tgtctgacta caatattcaa aaggagtcta     60
```

ctcttcatct tgtgttgaga cttcgtggtg gtgctaagaa a 101

<210> 21
<211> 748
<212> DNA
<213> Homo sapiens

<400> 21

gcagataatg ggaggagccg ggcccgagcg agctctttcc tttcgctgct gcggccgcag 60

ccatgagtat gctcaggctt cagaagaggc tcgcctctag tgtcctccgc tgtggcaaga 120

agaaggtctg gttagacccc aatgagacca atgaaatcgc caatgccaac tcccgtcagc 180

agatccggaa gctcatcaaa gatgggctga tcatccgcaa gcctgtgacg gtccattccc 240

gggctcgatg ccggaaaaac accttggccc gccggaaggg caggcacatg ggcataggta 300

agcggaaggg tacagccaat gcccgaatgc cagagaaggt cacatggatg aggagaatga 360

ggattttgcg ccggctgctc agaagatacc gtgaatctaa gaagatcgat cgccacatgt 420

atcacagcct gtacctgaag gtgaagggga atgtgttcaa aaacaagcgg attctcatgg 480

aacacatcca caagctgaag gcagacaagg cccgcaagaa gctcctggct gaccaggctg 540

aggcccgcag gtctaagacc aaggaagcac gcaagcgccg tgaagagcgc ctccaggcca 600

agaaggagga gatcatcaag actttatcca aggaggaaga gaccaagaaa taaaacctcc 660

cactttgtct gtacatactg gcctctgtga ttacatagat cagccattaa aataaaacaa 720

gccttaatct gcaaaaaaaa aaaaaaaa 748

<210> 22
<211> 23
<212> DNA
<213> Artificial Organism

<220>
<223> Synthetic oligonucleotide

<400> 22

tggcaagaag aaggtctggt tag 23

<210> 23
<211> 22
<212> DNA
<213> Artificial Organism

<220>
<223> Synthetic oligonucleotide

<400> 23

tgatcagccc atctttgatg ag 22

<210> 24
<211> 101
<212> DNA
<213> Artificial Organism

<220>
<223> Synthetic oligonucleotide

<400> 24

tggcaagaag aaggtctggt tagaccccaa tgagaccaat gaaatcgcca atgccaactc 60

ccgtcagcag atccggaagc tcatcaaaga tgggctgatc a 101

<210> 25
<211> 1867
<212> DNA
<213> Homo sapiens

<400> 25

ggttcgctgt ggcgggcgcc tgggccgccg gctgtttaac ttcgcttccg ctggcccata 60

gtgatctttg cagtgaccca gcagcatcac tgtttcttgg cgtgtgaaga taacccaagg 120

aattgaggaa gttgctgaga agagtgtgct ggagatgctc taggaaaaaa ttgaatagtg 180

agacgagttc cagcgcaagg gtttctggtt tgccaagaag aaagtgaaca tcatggatca 240

gaacaacagc ctgccacctt acgctcaggg cttggcctcc cctcagggtg ccatgactcc 300

cggaatccct atctttagtc caatgatgcc ttatggcact ggactgaccc cacagcctat 360

tcagaacacc aatagtctgt ctattttgga agagcaacaa aggcagcagc agcaacaaca 420

acagcagcag cagcagcagc agcagcaaca gcaacagcag cagcagcagc agcagcagca 480

gcagcagcag cagcagcagc agcagcagca gcaacaggca gtggcagctg cagccgttca 540

gcagtcaacg tcccagcagg caacacaggg aacctcaggc caggcaccac agctcttcca 600

ctcacagact ctcacaactg caccccttgcc gggcaccact ccactgtatc cctcccccat 660

gactcccatg acccccatca ctcctgccac gccagcttcg gagagttctg ggattgtacc 720

gcagctgcaa aatattgtat ccacagtgaa tcttggttgt aaacttgacc taaagaccat 780

tgcacttcgt gcccgaaacg ccgaatataa tcccaagcgg tttgctgcgg taatcatgag 840

gataagagag ccacgaacca cggcactgat tttcagttct gggaaaatgg tgtgcacagg 900

agccaagagt gaagaacagt ccagactggc agcaagaaaa tatgctagag ttgtacagaa 960

gttgggtttt ccagctaagt tcttggactt caagattcag aatatggtgg ggagctgtga 1020

tgtgaagttt cctataaggt tagaaggcct tgtgctcacc caccaacaat ttagtagtta 1080

tgagccagag ttatttcctg gtttaatcta cagaatgatc aaacccagaa ttgttctcct 1140

tatttttgtt tctggaaaag ttgtattaac aggtgctaaa gtcagagcag aaatttatga 1200

agcatttgaa aacatctacc ctattctaaa gggattcagg aagacgacgt aatggctctc 1260

atgtaccctt gcctccccca cccccttctt tttttttttt taaacaaatc agtttgtttt 1320

ggtaccttta aatggtggtg ttgtgagaag atggatgttg agttgcaggg tgtggcacca 1380

ggtgatgccc ttctgtaagt gcccaccgcg ggatgccggg aaggggcatt atttgtgcac 1440

tgagaacacc gcgcagcgtg actgtgagtt gctcataccg tgctgctatc tgggcagcgc 1500

tgcccattta tttatatgta gattttaaac actgctgttg acaagttggt ttgagggaga 1560

aaactttaag tgttaaagcc acctctataa ttgattggac tttttaattt taatgttttt 1620

ccccatgaac cacagttttt atatttctac cagaaaagta aaaatctttt ttaaaagtgt 1680

tgtttttcta atttataact cctaggggtt atttctgtgc cagacacatt ccacctctcc 1740

agtattgcag gacagaatat atgtgttaat gaaaatgaat ggctgtacat attttttttct 1800

ttcttcagag tactctgtac aataaatgca gtttataaaa gtgttaaaaa aaaaaaaaaa 1860

aaaaaaa 1867

<210> 26
<211> 20
<212> DNA
<213> Artificial Organism

<220>
<223> Synthetic oligonucleotide

<400> 26

cggtttgctg cggtaatcat 20

<210> 27
<211> 21
<212> DNA
<213> Artificial Organism

<220>
<223> Synthetic oligonucleotide

<400> 27

tttcttgctg ccagtctgga c 21

<210> 28
<211> 122
<212> DNA
<213> Artificial Organism

<220>
<223> Synthetic oligonucleotide

<400> 28

cggtttgctg cggtaatcat gaggataaga gagccacgaa ccacggcact gattttcagt 60

tctgggaaaa tggtgtgcac aggagccaag agtgaagaac agtccagact ggcagcaaga 120

aa 122

<210> 29
<211> 2201
<212> DNA
<213> Homo sapiens

<400> 29

cgggatttgg gtcgcggttc ttgtttgtgg atcgctgtga tcgtcacttg acaatgcaga 60

tcttcgtgaa gactctgact ggtaagacca tcaccctcga ggttgagccc agtgacacca 120

tcgagaatgt caaggcaaag atccaagata aggaaggcat ccctcctgac cagcagaggc 180

tgatctttgc tggaaaacag ctggaagatg ggcgcaccct gtctgactac aacatccaga 240

aagagtccac cctgcacctg gtgctccgtc tcagaggtgg gatgcaaatc ttcgtgaaga 300

cactcactgg caagaccatc acccttgagg tggagcccag tgacaccatc gagaacgtca 360

aagcaaagat ccaggacaag gaaggcattc ctcctgacca gcagaggttg atctttgccg 420

gaaagcagct ggaagatggg cgcaccctgt ctgactacaa catccagaaa gagtctaccc 480

tgcacctggt gctccgtctc agaggtggga tgcagatctt cgtgaagacc ctgactggta 540

agaccatcac cctcgaggtg gagcccagtg acaccatcga gaatgtcaag gcaaagatcc 600

aagataagga aggcattcct cctgatcagc agaggttgat ctttgccgga aaacagctgg 660

aagatggtcg taccctgtct gactacaaca tccagaaaga gtccaccttg cacctggtac 720

tccgtctcag aggtgggatg caaatcttcg tgaagacact cactggcaag accatcaccc 780

ttgaggtcga gcccagtgac actatcgaga acgtcaaagc aaagatccaa gacaaggaag 840

gcattcctcc tgaccagcag aggttgatct ttgccggaaa gcagctggaa gatgggcgca 900

ccctgtctga ctacaacatc cagaaagagt ctaccctgca cctggtgctc cgtctcagag 960

gtgggatgca gatcttcgtg aagaccctga ctggtaagac catcaccctc gaagtggagc 1020

cgagtgacac cattgagaat gtcaaggcaa agatccaaga caaggaaggc atccctcctg 1080

accagcagag gttgatcttt gccggaaaac agctggaaga tggtcgtacc ctgtctgact 1140

acaacatcca gaaagagtcc accttgcacc tggtgctccg tctcagaggt gggatgcaga 1200

tcttcgtgaa gaccctgact ggtaagacca tcactctcga ggtggagccg agtgacacca 1260

ttgagaatgt caaggcaaag atccaagaca aggaaggcat ccctcctgat cagcagaggt 1320

tgatctttgc tgggaaacag ctggaagatg gacgcaccct gtctgactac aacatccaga 1380

aagagtccac cctgcacctg gtgctccgtc ttagaggtgg gatgcagatc ttcgtgaaga 1440

ccctgactgg taagaccatc actctcgaag tggagccgag tgacaccatt gagaatgtca 1500

aggcaaagat ccaagacaag gaaggcatcc ctcctgacca gcagaggttg atctttgctg 1560

ggaaacagct ggaagatgga cgcaccctgt ctgactacaa catccagaaa gagtccaccc 1620

tgcacctggt gctccgtctt agaggtggga tgcagatctt cgtgaagacc ctgactggta 1680

agaccatcac tctcgaagtg gagccgagtg acaccattga gaatgtcaag gcaaagatcc 1740

aagacaagga aggcatccct cctgaccagc agaggttgat ctttgctggg aaacagctgg 1800

aagatggacg caccctgtct gactacaaca tccagaaaga gtccaccctg cacctggtgc 1860

tccgtctcag aggtgggatg cagatcttcg tgaagaccct gactggtaag accatcaccc 1920

tcgaggtgga gcccagtgac accatcgaga atgtcaaggc aaagatccaa gataaggaag 1980

gcatccctcc tgatcagcag aggttgatct ttgctgggaa acagctggaa gatggacgca 2040

ccctgtctga ctacaacatc cagaaagagt ccactctgca cttggtcctg cgcttgaggg 2100

ggggtgtcta agtttcccct tttaaggttt caacaaattt cattgcactt tcctttcaat 2160

aaagttgttg cattcccaaa aaaaaaaaaa aaaaaaaaaa a 2201

<210> 30
<211> 19
<212> DNA
<213> Artificial Organism

<220>
<223> Synthetic oligonucleotide

<400> 30

atttgggtcg cggttcttg 19

<210> 31
<211> 21
<212> DNA
<213> Artificial Organism

<220>
<223> Synthetic oligonucleotide

<400> 31

tgccttgaca ttctcgatgg t 21

<210> 32
<211> 133
<212> DNA
<213> Artificial Organism

<220>
<223> Synthetic oligonucleotide

<400> 32

atttgggtcg cggttcttgt ttgtggatcg ctgtgatcgt cacttgacaa tgcagatctt 60

cgtgaagact ctgactggta agaccatcac cctcgaggtt gagcccagtg acaccatcga 120

gaatgtcaag gca 133

<210> 33
<211> 2207
<212> DNA
<213> Homo sapiens

<400> 33

tggcgctggc caaggcgtgg ccaacagtgt tgcaaacagg aacccgaggt tttcacttca 60

ctgttgatgg gaacaagagg gcatctgcta aagtttcaga ttccatttct gctcagtatc 120

cagtagtgga tcatgaattt gatgcagtgg tggtaggcgc tggaggggca ggcttgcgag 180

ctgcatttgg cctttctgag gcagggttta atacagcatg tgttaccaag ctgtttccta 240

ccaggtcaca cactgttgca gcgcagggag gaatcaatgc tgctctgggg aacatggagg 300

aggacaactg gaggtggcat ttctacgaca ccgtgaaggg ctccgactgg ctgggggacc 360

aggatgccat ccactacatg acggagcagg cccccgccgc cgtggtcgag ctagaaaatt 420

atggcatgcc gtttagcaga actgaagatg ggaagattta tcagcgtgca tttggtggac 480

agagcctcaa gtttggaaag ggcgggcagg cccatcggtg ctgctgtgtg gctgatcgga 540

ctggccactc gctattgcac accttatatg gacggtctct gcgatatgat accagctatt 600

ttgtggagta ttttgccttg gatctcctga tggagaacgg ggagtgccgt ggtgtcatcg 660

cactgtgcat agaggacggg tccatccatc gcataagagc aaagaacact gttgttgcca 720

caggaggcta cgggcgcacc tacttcagct gcacgtctgc ccacaccagc actggcgacg 780

gcacggccat gatcaccagg gcaggccttc cttgccagga cctagagttt gttcagttcc 840

accccacagg catatatggt gctggttgtc tcattacgga aggatgtcgt ggagagggag 900

gcattctcat taacagtcaa ggcgaaaggt ttatggagcg atacgcccct gtcgcgaagg 960

acctggcgtc tagagatgtg gtgtctcggt cgatgactct ggagatccga gaaggaagag 1020

gctgtggccc tgagaaagat cacgtctacc tgcagctgca ccacctacct ccagagcagc 1080

tggccacgcg cctgcctggc atttcagaga cagccatgat cttcgctggc gtggacgtca 1140

cgaaggagcc gatccctgtc ctccccaccg tgcattataa catgggcggc attcccacca 1200

actacaaggg gcaggtcctg aggcacgtga atggccagga tcagattgtg cccggcctgt 1260

acgcctgtgg ggaggccgcc tgtgcctcgg tacatggtgc caaccgcctc ggggcaaact 1320

cgctcttgga cctggttgtc tttggtcggg catgtgccct gagcatcgaa gagtcatgca 1380

ggcctggaga taaagtccct ccaattaaac caaacgctgg ggaagaatct gtcatgaatc 1440

ttgacaaatt gagatttgct gatggaagca taagaacatc ggaactgcga ctcagcatgc 1500

agaagtcaat gcaaaatcat gctgccgtgt tccgtgtggg aagcgtgttg caagaaggtt 1560

gtgggaaaat cagcaagctc tatggagacc taaagcacct gaagacgttc gaccggggaa 1620

tggtctggaa cacagacctg gtggagaccc tggagctgca gaacctgatg ctgtgtgcgc 1680

tgcagaccat ctacggagca gaggcgcgga aggagtcacg ggcgcgcat gccagggaag 1740

actacaaggt gcggattgat gagtacgatt actccaagcc catccagggg caacagaaga 1800

agccctttga ggagcactgg aggaagcaca ccctgtcctt tgtggacgtt ggcactggga 1860

aggtcactct ggaatataga cccgtaatcg acaaaacttt gaacgaggct gactgtgcca 1920

ccatcccgcc agccattcgc tcctactgat gagacaagat gtggtgatga cagaatcagc 1980

ttttgtaatt atgtataata gctcatgcat gtgtccatgt cataactgtc ttcatacgct 2040

tctgcactct ggggaagaag gagtacattg aagggagatt ggcacctagt ggctgggagc 2100

ttgccaggaa cccagtggcc agggagcgtg gcacttacct ttgtcccttg cttcattctt 2160

gtgagatgat aaaactgggc acagctctta aataaaatat aaatgag 2207

<210> 34
<211> 20
<212> DNA
<213> Artificial Organism

<220>
<223> Synthetic oligonucleotide

<400> 34

tgggaacaag agggcatctg 20

<210> 35
<211> 22
<212> DNA
<213> Artificial Organism

<220>
<223> Synthetic oligonucleotide

<400> 35

ccaccactgc atcaaattca tg 22

<210> 36
<211> 86
<212> DNA
<213> Artificial Organism

<220>
<223> Synthetic oligonucleotide

<400> 36

tgggaacaag agggcatctg ctaaagtttc agattccatt tctgctcagt atccagtagt 60

ggatcatgaa tttgatgcag tggtgg 86

<210> 37
<211> 2017
<212> DNA
<213> Homo sapiens

<400> 37

```
ggccattctc tcagatataa ggcttggaag ccagcagctg cgactcccga gacccccca      60
ccagaaggcc atggtctccc cacggatgtc cgggctcctc tcccagactg tgatcctagc    120
gctcattttc ctcccccaga cacggcccgc tggcgtcttc gagctgcaga tccactcttt    180
cgggccgggt ccaggcccgg ctcccctgcc gcctcttctt cagagtctgc ctgaagcctg    240
ggctctcaga ggaggccgcc gagtccccgt gcgccctggg cgcggcgctg agtgcgcgcg    300
gaccggtcta caccgagcag cccggagcgc ccgcgcctga tctcccactg cccgacggcc    360
tcttgcaggt gcccttccgg gacgcctggc ctggcacctt ctctttcatc atcgaaacct    420
ggagagagga gttaggagac cagattggag ggcccgcctg gagcctgctg gcgcgcgtgg    480
ctggcaggcg gcgcttggca gccggaggcc cgtgggcccg ggacattcag cgcgcaggcg    540
cctgggagct gcgcttctcg taccgcgcgc gctgcgagcc gcctgccgtc gggaccgcgt    600
gcacgcgcct ctgccgtccg cgcagcgccc cctcgcggtg cggtccggga ctgcgcccct    660
gcgcaccgct cgaggacgaa tgtgaggcgc cgctggtgtg ccgagcaggc tgcagccctg    720
agcatggctt ctgtgaacag cccggtgaat gccgatgcct agagggctgg actggacccc    780
tctgcacggt ccctgtctcc accagcagct gcctcagccc caggggcccg tcctctgcta    840
ccaccggatg ccttgtccct gggcctgggc cctgtgacgg gaacccgtgt gccaatggag    900
gcagctgtag tgagacaccc aggtcctttg aatgcacctg cccgcgtggg ttctacgggc    960
tgcggtgtga ggtgagcggg gtgacatgtg cagatggacc ctgcttcaac ggcggcttgt   1020
gtgtcggggg tgcagaccct gactctgcct acatctgcca ctgcccaccc ggtttccaag   1080
gctccaactg tgagaagagg gtggaccggt gcagcctgca gccatgccgc aatggcggac   1140
tctgcctgga cctgggccac gccctgcgct gccgctgccg cgccggcttc gcgggtcctc   1200
gctgcgagca cgacctggac gactgcgcgg gccgcgcctg cgctaacggc ggcacgtgtg   1260
tggagggcgg cggcgcgcac cgctgctcct gcgcgctggg cttcggcggc cgcgactgcc   1320
gcgagcgcgc ggacccgtgc gccgcgcgcc cctgtgctca cggcggccgc tgctacgccc   1380
acttctccgg cctcgtctgc gcttgcgctc ccggctacat gggagcgcgg tgtgagttcc   1440
cagtgcaccc cgacggcgca agcgccttgc ccgcggcccc gccgggcctc aggcccgggg   1500
accctcagcg ctaccttttg cctccggctc tgggactgct cgtggccgcg ggcgtggccg   1560
gcgctgcgct cttgctggtc cacgtgcgcc gccgtggcca ctcccaggat gctgggtctc   1620
gcttgctggc tgggaccccg gagccgtcag tccacgcact cccggatgca ctcaacaacc   1680
```

taaggacgca ggagggttcc ggggatggtc cgagctcgtc cgtagattgg aatcgccctg 1740

aagatgtaga ccctcaaggg atttatgtca tatctgctcc ttccatctac gctcgggagg 1800

cctgacgcgt ctcctccatc cgcacctgga gtcagagcgt ggatttttgt atttgctcgg 1860

tggtgcccag tctctgcccc agaggctttg gagttcaatc ttgaaggggt gtctggggga 1920

actttactgt tgcaagttgt aaataatggt tatttatatc ctatttttttc tcaccccatc 1980

tctctagaaa cacctataaa ggctattatt gtgatca 2017

<210> 38
<211> 2057
<212> DNA
<213> Homo sapiens

<400> 38

ggccattctc tcagatataa ggcttggaag ccagcagctg cgactcccga gaccccccca 60

ccagaaggcc atggtctccc cacggatgtc cgggctcctc tcccagactg tgatcctagc 120

gctcattttc ctcccccagg tcagagccag acacggcccg ctggcgtctt cgagctgcag 180

atccactctt tcgggccggg tccaggccct ggggccccgc ggtccccctg cagcgcccgg 240

ctcccctgcc gcctcttctt cagagtctgc ctgaagcctg ggctctcaga ggaggccgcc 300

gagtccccgt gcgccctggg cgcggcgctg agtgcgcgcg gaccggtcta caccgagcag 360

cccggagcgc ccgcgcctga tctcccactg cccgacggcc tcttgcaggt gcccttccgg 420

gacgcctggc ctggcacctt ctctttcatc atcgaaacct ggagagagga gttaggagac 480

cagattggag ggcccgcctg gagcctgctg gcgcgcgtgg ctggcaggcg gcgcttggca 540

gccggaggcc cgtgggcccg ggacattcag cgcgcaggcg cctgggagct gcgcttctcg 600

taccgcgcgc gctgcgagcc gcctgccgtc gggaccgcgt gcacgcgcct ctgccgtccg 660

cgcagcgccc cctcgcggtg cggtccggga ctgcgcccct gcgcaccgct cgaggacgaa 720

tgtgaggcgc cgctggtgtg ccgagcaggc tgcagccctg agcatggctt ctgtgaacag 780

cccggtgaat gccgatgcct agagggctgg actggacccc tctgcacggt ccctgtctcc 840

accagcagct gcctcagccc caggggcccg tcctctgcta ccaccggatg ccttgtccct 900

gggcctgggc cctgtgacgg gaacccgtgt gccaatggag gcagctgtag tgagacaccc 960

aggtcctttg aatgcacctg cccgcgtggg ttctacgggc tgcggtgtga ggtgagcggg 1020

gtgacatgtg cagatggacc ctgcttcaac ggcggcttgt gtgtcggggg tgcagaccct 1080

gactctgcct acatctgcca ctgcccaccc ggtttccaag gctccaactg tgagaagagg 1140

gtggaccggt gcagcctgca gccatgccgc aatggcggac tctgcctgga cctgggccac 1200

gccctgcgct gccgctgccg cgccggcttc gcgggtcctc gctgcgagca cgacctggac 1260

gactgcgcgg gccgcgcctg cgctaacggc ggcacgtgtg tggagggcgg cggcgcgcac 1320

cgctgctcct gcgcgctggg cttcggcggc cgcgactgcc gcgagcgcgc ggacccgtgc 1380

gccgcgcgcc cctgtgctca cggcggccgc tgctacgccc acttctccgg cctcgtctgc 1440

gcttgcgctc ccggctacat gggagcgcgg tgtgagttcc cagtgcaccc cgacggcgca 1500

agcgccttgc ccgcggcccc gccgggcctc aggcccgggg accctcagcg ctaccttttg 1560

cctccggctc tgggactgct cgtggccgcg ggcgtggccg gcgctgcgct cttgctggtc 1620

cacgtgcgcc gccgtggcca ctcccaggat gctgggtctc gcttgctggc tgggaccccg 1680

gagccgtcag tccacgcact cccggatgca ctcaacaacc taaggacgca ggagggttcc 1740

ggggatggtc cgagctcgtc cgtagattgg aatcgccctg aagatgtaga ccctcaaggg 1800

atttatgtca tatctgctcc ttccatctac gctcgggagg cctgacgcgt ctcctccatc 1860

cgcacctgga gtcagagcgt ggatttttgt atttgctcgg tggtgcccag tctctgcccc 1920

agaggctttg gagttcaatc ttgaaggggt gtctggggga actttactgt tgcaagttgt 1980

aaataatggt tatttatatc ctatttttttc tcaccccatc tctctagaaa cacctataaa 2040

ggctattatt gtgatca 2057

<210> 39
<211> 1866
<212> DNA
<213> Homo sapiens

<400> 39

ggccattctc tcagatataa ggcttggaag ccagcagctg cgactcccga gacccccca 60

ccagaaggcc atggtctccc cacggatgtc cgggctcctc tcccagactg tgatcctagc 120

gctcattttc ctcccccaga cacggcccgc tggcgtcttc gagctgcaga tccactcttt 180

cgggccgggt ccaggccctg ggccccgcg gtccccctgc agcgcccggc tcccctgccg 240

cctcttcttc agagtctgcc tgaagcctgg gctctcagag gaggccgccg agtccccgtg 300

cgccctgggc gcggcgctga gtgcgcgcgg accggtctac accgagcagc ccggagcgcc 360

cgcgcctgat ctcccactgc ccgacggcct cttgcaggtg cccttccggg acgcctggcc 420

tggcaccttc tctttcatca tcgaaacctg gagagaggag ttaggagacc agattggagg 480

gcccgcctgg agcctgctgg cgcgcgtggc tggcaggcgg cgcttggcag ccggaggccc 540

gtgggcccgg gacattcagc gcgcaggcgc ctgggagctg cgcttctcgt accgcgcgcg 600

ctgcgagccg cctgccgtcg ggaccgcgtg cacgcgcctc tgccgtccgc gcagcgcccc 660

ctcgcggtgc ggtccgggac tgcgcccctg cgcaccgctc gaggacgaat gtgaggcgcc 720

gctggtgtgc cgagcaggct gcagccctga gcatggcttc tgtgaacagc ccggtgaatg 780

ccgatgccta gagggctgga ctggacccct ctgcacggtc cctgtctcca ccagcagctg 840

cctcagcccc aggggcccgt cctctgctac caccggatgc cttgtccctg ggcctgggcc 900

ctgtgacggg aacccgtgtg ccaatggagg cagctgtagt gagacaccca ggtcctttga 960

atgcacctgc ccgcgtgggt tctacgggct gcggtgtgag gtgagcgggg tgacatgtgc 1020

agatggaccc tgcttcaacg gcggcttgtg tgtcgggggt gcagaccctg actctgccta 1080

catctgccac tgcccacccg gtttccaagg ctccaactgt gagaagaggg tggaccggtg 1140

cagcctgcag ccatgccgca atggtgaggc ctggaggcct gaacggcgag ggatggggtg 1200

ggggtcctgg atggctcaga cagtccaggg ttggaatcct ggctttgact cttctaaccc 1260

tagggcctgg ggacctgacc ttccacctgc aagcctgtaa aatgggcaag gagacattcc 1320

ctatctcata actattaata tttactgaga atttactgtg tgccaggccc tattctaggc 1380

actgaggata cagcagggaa tgaaacagac aaagtccctg gccctgcctg atagagctga 1440

ggtgcctggt gtgctgagga taagcaggga agccagtgtg gttatactga gatgaggtca 1500

gggaggtgac tgaggcacat cttgtgggac cccctgggtc acaagaaggg gaactttcac 1560

ttttcccctg agtgagatgg agccacagga aggttctgag gagagaagag acctgatatc 1620

ggtgctaaaa gattaaatgc ggcccggcac ggcgactcac gcctgtaatt ccagcacttt 1680

gggaggccga ggcgggcaga tcacctgagc tcaggagttg gagaccagcc cgggccacat 1740

ggtgaaaccc cgtctctact aaaaatacaa aaaattagcc gggcgtgatg gcaggtgctt 1800

gtaatcccag ctactcggga ggctaaggcg gaagaatcac ttgaacccgg gaggcggagg 1860

ttgcag 1866

<210> 40
<211> 139
<212> DNA
<213> Homo sapiens

<400> 40

ggccattctc tcagatataa ggcttggaag ccagcagctg cgactcccga gacccccccca 60

ccagaaggcc atggtctccc cacggatgtc cgggctcctc tcccagactg tgatcctagc 120

gctcattttc ctcccccag 139

<210> 41
<211> 195
<212> DNA
<213> Homo sapiens

<400> 41

cggctcccct gccgcctctt cttcagagtc tgcctgaagc ctgggctctc agaggaggcc 60

gccgagtccc cgtgcgccct gggcgcggcg ctgagtgcgc gcggaccggt ctacaccgag 120

cagcccggag cgcccgcgcc tgatctccca ctgcccgacg gcctcttgca ggtgcccttc 180

cgggacgcct ggcct 195

<210> 42
<211> 231
<212> DNA
<213> Homo sapiens

<400> 42

ggcccgcctg gagcctgctg gcgcgcgtgg ctggcaggcg gcgcttggca gccggaggcc 60

cgtgggcccg ggacattcag cgcgcaggcg cctgggagct gcgcttctcg taccgcgcgc 120

gctgcgagcc gcctgccgtc gggaccgcgt gcacgcgcct ctgccgtccg cgcagcgccc 180

cctcgcggtg cggtccggga ctgcgcccct gcgcaccgct cgaggacgaa t 231

<210> 43
<211> 218
<212> DNA
<213> Homo sapiens

<400> 43

tggtgtgccg agcaggctgc agccctgagc atggcttctg tgaacagccc ggtgaatgcc 60

gatgcctaga gggctggact ggacccctct gcacggtccc tgtctccacc agcagctgcc 120

tcagccccag gggcccgtcc tctgctacca ccggatgcct tgtccctggg cctgggccct 180

gtgacgggaa cccgtgtgcc aatggaggca gctgtagt 218

<210> 44
<211> 223
<212> DNA
<213> Homo sapiens

<400> 44

gagacaccca ggtcctttga atgcacctgc ccgcgtgggt tctacgggct gcggtgtgag 60

gtgagcgggg tgacatgtgc agatggaccc tgcttcaacg gcggcttgtg tgtcgggggt 120

gcagaccctg actctgccta catctgccac tgcccacccg gtttccaagg ctccaactgt 180

gagaagaggg tggaccggtg cagcctgcag ccatgccgca atg 223

<210> 45
<211> 703
<212> DNA
<213> Homo sapiens

<400> 45

gtgaggcctg gaggcctgaa cggcgaggga tggggtgggg gtcctggatg gctcagacag 60

tccagggttg gaatcctggc tttgactctt ctaaccctag ggcctgggga cctgaccttc 120

cacctgcaag cctgtaaaat gggcaaggag acattcccta tctcataact attaatattt 180

actgagaatt tactgtgtgc caggccctat tctaggcact gaggatacag cagggaatga 240

aacagacaaa gtccctggcc ctgcctgata gagctgaggt gcctggtgtg ctgaggataa 300

gcagggaagc cagtgtggtt atactgagat gaggtcaggg aggtgactga ggcacatctt 360

gtgggacccc ctgggtcaca agaaggggaa ctttcacttt tcccctgagt gagatggagc 420

cacaggaagg ttctgaggag agaagagacc tgatatcggt gctaaaagat taaatgcggc 480

ccggcacggc gactcacgcc tgtaattcca gcactttggg aggccgaggc gggcagatca 540

cctgagctca ggagttggag accagcccgg gccacatggt gaaaccccgt ctctactaaa 600

aatacaaaaa attagccggg cgtgatggca ggtgcttgta atcccagcta ctcgggaggc 660

taaggcggaa gaatcacttg aacccgggag gcggaggttg cag 703

<210> 46
<211> 580
<212> DNA
<213> Homo sapiens

<400> 46

gcggactctg cctggacctg ggccacgccc tgcgctgccg ctgccgcgcc ggcttcgcgg 60

gtcctcgctg cgagcacgac ctggacgact gcgcgggccg cgcctgcgct aacggcggca 120

cgtgtgtgga gggcggcggc gcgcaccgct gctcctgcgc gctgggcttc ggcggccgcg 180

actgccgcga gcgcgcggac ccgtgcgccg cgcgcccctg tgctcacggc ggccgctgct 240

acgcccactt ctccggcctc gtctgcgctt gcgctcccgg ctacatggga gcgcggtgtg 300

agttcccagt gcaccccgac ggcgcaagcg ccttgcccgc ggccccgccg ggcctcaggc 360

ccggggaccc tcagcgctac cttttgcctc cggctctggg actgctcgtg gccgcgggcg 420

tggccggcgc tgcgctcttg ctggtccacg tgcgccgccg tggccactcc caggatgctg 480

ggtctcgctt gctggctggg accccggagc cgtcagtcca cgcactcccg gatgcactca 540

acaacctaag gacgcaggag ggttccgggg atggtccgag 580

<210> 47
<211> 419
<212> DNA
<213> Homo sapiens

<400> 47

gcctgacgcg tctcctccat ccgcacctgg agtcagagcg tggatttttg tatttgctcg 60

gtggtgccca gtctctgccc cagaggcttt ggagttcaat cttgaagggg tgtctggggg 120

aactttactg ttgcaagttg taaataatgg ttatttatat cctatttttt ctcaccccat 180

ctctctagaa acacctataa aggctattat tgtgatcagt tttgactaac gaggctgaat 240

gctttgtatt tgatgttggg gctgagggga agagattttg tctcctgagg agaatagtag 300

acaggactct ttatgttgta ggggtcggaa acgactcaaa ctgctttaaa tataaaagaa 360

tttatggctg ggtgcagtgg ctcacggctg taatcccact gaaccactga gtgagatca 419

<210> 48
<211> 11
<212> DNA
<213> Homo sapiens

<400> 48

gtcagagcca g 11

<210> 49
<211> 58
<212> DNA
<213> Homo sapiens

<400> 49

acacggcccg ctggcgtctt cgagctgcag atccactctt tcgggccggg tccaggcc 58

<210> 50
<211> 29
<212> DNA
<213> Homo sapiens

<400> 50

ctggggcccc gcggtccccc tgcagcgcc 29

<210> 51
<211> 58
<212> DNA
<213> Homo sapiens

<400> 51

ggcaccttct ctttcatcat cgaaacctgg agagaggagt taggagacca gattggag 58

<210> 52
<211> 12
<212> DNA
<213> Homo sapiens

<400> 52

gtgaggcgcc gc 12

<210> 53
<211> 85
<212> DNA
<213> Homo sapiens

<400> 53

```
ctcgtccgta gattggaatc gccctgaaga tgtagaccct caagggattt atgtcatatc     60

tgctccttcc atctacgctc gggag                                           85


<210>  54
<211>  618
<212>  PRT
<213>  Homo sapiens

<400> 54

Met Val Ser Pro Arg Met Ser Gly Leu Leu Ser Gln Thr Val Ile Leu
1               5                   10                  15


Ala Leu Ile Phe Leu Pro Gln Thr Arg Pro Ala Gly Val Phe Glu Leu
            20                  25                  30


Gln Ile His Ser Phe Gly Pro Gly Pro Gly Pro Gly Ala Pro Arg Ser
        35                  40                  45


Pro Cys Ser Ala Arg Leu Pro Cys Arg Leu Phe Phe Arg Val Cys Leu
    50                  55                  60


Lys Pro Gly Leu Ser Glu Glu Ala Ala Glu Ser Pro Cys Ala Leu Gly
65                  70                  75                  80


Ala Ala Leu Ser Ala Arg Gly Pro Val Tyr Thr Glu Gln Pro Gly Ala
                85                  90                  95


Pro Ala Pro Asp Leu Pro Leu Pro Asp Gly Leu Leu Gln Val Pro Phe
            100                 105                 110


Arg Asp Ala Trp Pro Gly Thr Phe Ser Phe Ile Ile Glu Thr Trp Arg
        115                 120                 125


Glu Glu Leu Gly Asp Gln Ile Gly Gly Pro Ala Trp Ser Leu Leu Ala
    130                 135                 140


Arg Val Ala Gly Arg Arg Arg Leu Ala Ala Gly Gly Pro Trp Ala Arg
145                 150                 155                 160


Asp Ile Gln Arg Ala Gly Ala Trp Glu Leu Arg Phe Ser Tyr Arg Ala
                165                 170                 175


Arg Cys Glu Pro Pro Ala Val Gly Thr Ala Cys Thr Arg Leu Cys Arg
            180                 185                 190


Pro Arg Ser Ala Pro Ser Arg Cys Gly Pro Gly Leu Arg Pro Cys Ala
```

195 200 205

Pro Leu Glu Asp Glu Cys Glu Ala Pro Leu Val Cys Arg Ala Gly Cys
210 215 220

Ser Pro Glu His Gly Phe Cys Glu Gln Pro Gly Glu Cys Arg Cys Leu
225 230 235 240

Glu Gly Trp Thr Gly Pro Leu Cys Thr Val Pro Val Ser Thr Ser Ser
245 250 255

Cys Leu Ser Pro Arg Gly Pro Ser Ser Ala Thr Thr Gly Cys Leu Val
260 265 270

Pro Gly Pro Gly Pro Cys Asp Gly Asn Pro Cys Ala Asn Gly Gly Ser
275 280 285

Cys Ser Glu Thr Pro Arg Ser Phe Glu Cys Thr Cys Pro Arg Gly Phe
290 295 300

Tyr Gly Leu Arg Cys Glu Val Ser Gly Val Thr Cys Ala Asp Gly Pro
305 310 315 320

Cys Phe Asn Gly Gly Leu Cys Val Gly Gly Ala Asp Pro Asp Ser Ala
325 330 335

Tyr Ile Cys His Cys Pro Pro Gly Phe Gln Gly Ser Asn Cys Glu Lys
340 345 350

Arg Val Asp Arg Cys Ser Leu Gln Pro Cys Arg Asn Gly Gly Leu Cys
355 360 365

Leu Asp Leu Gly His Ala Leu Arg Cys Arg Cys Arg Ala Gly Phe Ala
370 375 380

Gly Pro Arg Cys Glu His Asp Leu Asp Asp Cys Ala Gly Arg Ala Cys
385 390 395 400

Ala Asn Gly Gly Thr Cys Val Glu Gly Gly Gly Ala His Arg Cys Ser
405 410 415

Cys Ala Leu Gly Phe Gly Gly Arg Asp Cys Arg Glu Arg Ala Asp Pro
420 425 430

Cys Ala Ala Arg Pro Cys Ala His Gly Gly Arg Cys Tyr Ala His Phe
435 440 445

```
Ser Gly Leu Val Cys Ala Cys Ala Pro Gly Tyr Met Gly Ala Arg Cys
    450                 455                 460

Glu Phe Pro Val His Pro Asp Gly Ala Ser Ala Leu Pro Ala Ala Pro
465                 470                 475                 480

Pro Gly Leu Arg Pro Gly Asp Pro Gln Arg Tyr Leu Leu Pro Pro Ala
                485                 490                 495

Leu Gly Leu Leu Val Ala Ala Gly Val Ala Gly Ala Ala Leu Leu Leu
            500                 505                 510

Val His Val Arg Arg Arg Gly His Ser Gln Asp Ala Gly Ser Arg Leu
        515                 520                 525

Leu Ala Gly Thr Pro Glu Pro Ser Val His Ala Leu Pro Asp Ala Leu
    530                 535                 540

Asn Asn Leu Arg Thr Gln Glu Gly Ser Gly Asp Gly Pro Ser Ser Ser
545                 550                 555                 560

Val Asp Trp Asn Arg Pro Glu Asp Val Asp Pro Gln Gly Ile Tyr Val
                565                 570                 575

Ile Ser Ala Pro Ser Ile Tyr Ala Arg Glu Val Ala Thr Pro Leu Phe
            580                 585                 590

Pro Pro Leu His Thr Gly Arg Ala Gly Gln Arg Gln His Leu Leu Phe
        595                 600                 605

Pro Tyr Pro Ser Ser Ile Leu Ser Val Lys
    610                 615


<210>  55
<211>  587
<212>  PRT
<213>  Homo sapiens

<400> 55

Met Val Ser Pro Arg Met Ser Gly Leu Leu Ser Gln Thr Val Ile Leu
1               5                   10                  15

Ala Leu Ile Phe Leu Pro Gln Thr Arg Pro Ala Gly Val Phe Glu Leu
            20                  25                  30

Gln Ile His Ser Phe Gly Pro Gly Pro Gly Pro Gly Ala Pro Arg Ser
        35                  40                  45
```

```
Pro Cys Ser Ala Arg Leu Pro Cys Arg Leu Phe Phe Arg Val Cys Leu
    50                  55                  60

Lys Pro Gly Leu Ser Glu Glu Ala Ala Glu Ser Pro Cys Ala Leu Gly
65                  70                  75                  80

Ala Ala Leu Ser Ala Arg Gly Pro Val Tyr Thr Glu Gln Pro Gly Ala
                85                  90                  95

Pro Ala Pro Asp Leu Pro Leu Pro Asp Gly Leu Leu Gln Val Pro Phe
            100                 105                 110

Arg Asp Ala Trp Pro Gly Thr Phe Ser Phe Ile Ile Glu Thr Trp Arg
        115                 120                 125

Glu Glu Leu Gly Asp Gln Ile Gly Gly Pro Ala Trp Ser Leu Leu Ala
    130                 135                 140

Arg Val Ala Gly Arg Arg Arg Leu Ala Ala Gly Gly Pro Trp Ala Arg
145                 150                 155                 160

Asp Ile Gln Arg Ala Gly Ala Trp Glu Leu Arg Cys Ser Tyr Arg Ala
                165                 170                 175

Arg Cys Glu Pro Pro Ala Val Gly Thr Ala Cys Thr Arg Leu Cys Arg
            180                 185                 190

Pro Arg Ser Ala Pro Ser Arg Cys Gly Pro Gly Leu Arg Pro Cys Ala
        195                 200                 205

Pro Leu Glu Asp Glu Cys Glu Ala Pro Pro Val Cys Arg Ala Gly Cys
    210                 215                 220

Ser Pro Glu His Gly Phe Cys Glu Gln Pro Gly Glu Cys Arg Cys Leu
225                 230                 235                 240

Glu Gly Trp Thr Gly Pro Leu Cys Thr Val Pro Val Ser Thr Ser Ser
                245                 250                 255

Cys Leu Ser Pro Arg Gly Pro Ser Ser Ala Thr Thr Gly Cys Leu Val
            260                 265                 270

Pro Gly Pro Gly Pro Cys Asp Gly Asn Pro Cys Ala Asn Gly Gly Ser
        275                 280                 285

Cys Ser Glu Thr Pro Arg Ser Phe Glu Cys Thr Cys Pro Arg Gly Phe
    290                 295                 300
```

```
Tyr Gly Leu Arg Cys Glu Val Ser Gly Val Thr Cys Ala Asn Gly Pro
305                 310                 315                 320

Cys Phe Asn Gly Gly Leu Cys Val Gly Gly Ala Asp Pro Asp Ser Ala
                325                 330                 335

Tyr Ile Cys His Cys Pro Pro Gly Phe Gln Gly Ser Asn Cys Glu Lys
            340                 345                 350

Arg Val Asp Arg Cys Ser Leu Gln Pro Cys Arg Asn Gly Gly Leu Cys
        355                 360                 365

Leu Asp Leu Gly His Ala Leu Arg Cys Arg Cys Arg Ala Gly Phe Ala
    370                 375                 380

Gly Pro Arg Cys Glu His Asp Leu Asp Asp Cys Ala Gly Arg Ala Cys
385                 390                 395                 400

Ala Asn Gly Gly Thr Cys Val Glu Gly Gly Gly Ala His Arg Cys Ser
                405                 410                 415

Cys Ala Leu Gly Phe Gly Gly Arg Asp Cys Arg Glu Arg Ala Asp Pro
            420                 425                 430

Cys Ala Val Arg Pro Cys Ala His Gly Gly Arg Cys Tyr Ala His Phe
        435                 440                 445

Ser Gly Leu Val Cys Ala Cys Ala Pro Gly Tyr Met Gly Ala Arg Cys
    450                 455                 460

Glu Phe Pro Val His Pro Asp Gly Ala Ser Ala Leu Pro Ala Ala Pro
465                 470                 475                 480

Pro Gly Leu Arg Pro Gly Asp Pro Gln Arg Tyr Leu Leu Pro Pro Ala
                485                 490                 495

Leu Gly Leu Leu Val Ala Ala Gly Val Ala Gly Ala Ala Leu Leu Leu
            500                 505                 510

Val His Val Arg Arg Arg Gly His Ser Gln Asp Ala Gly Ser Arg Leu
        515                 520                 525

Leu Ala Gly Thr Pro Glu Pro Ser Val His Ala Leu Pro Asp Ala Leu
    530                 535                 540

Asn Asn Leu Arg Thr Gln Glu Gly Ser Gly Asp Gly Pro Ser Ser Ser
```

545 550 555 560

Val Asp Trp Asn Arg Pro Glu Asp Val Asp Pro Gln Gly Ile Tyr Val
565 570 575

Ile Ser Ala Pro Ser Ile Tyr Ala Arg Glu Ala
580 585

<210> 56
<211> 587
<212> PRT
<213> Homo sapiens

<400> 56

Met Val Ser Pro Arg Met Ser Gly Leu Leu Ser Gln Thr Val Ile Leu
1 5 10 15

Ala Leu Ile Phe Leu Pro Gln Thr Arg Pro Ala Gly Val Phe Glu Leu
20 25 30

Gln Ile His Ser Phe Gly Pro Gly Pro Gly Pro Gly Ala Pro Arg Ser
35 40 45

Pro Cys Ser Ala Arg Leu Pro Cys Arg Leu Phe Phe Arg Val Cys Leu
50 55 60

Lys Pro Gly Leu Ser Glu Glu Ala Ala Glu Ser Pro Cys Ala Leu Gly
65 70 75 80

Ala Ala Leu Ser Ala Arg Gly Pro Val Tyr Thr Glu Gln Pro Gly Ala
85 90 95

Pro Ala Pro Asp Leu Pro Leu Pro Asp Gly Leu Leu Gln Val Pro Phe
100 105 110

Arg Asp Ala Trp Pro Gly Thr Phe Ser Phe Ile Ile Glu Thr Trp Arg
115 120 125

Glu Glu Leu Gly Asp Gln Ile Gly Gly Pro Ala Trp Ser Leu Leu Ala
130 135 140

Arg Val Ala Gly Arg Arg Arg Leu Ala Ala Gly Gly Pro Trp Ala Arg
145 150 155 160

Asp Ile Gln Arg Ala Gly Ala Trp Glu Leu Arg Phe Ser Tyr Arg Ala
165 170 175

```
Arg Cys Glu Pro Pro Ala Val Gly Thr Ala Cys Thr Arg Leu Cys Arg
            180                 185                 190

Pro Arg Ser Ala Pro Ser Arg Cys Gly Pro Gly Leu Arg Pro Cys Ala
        195                 200                 205

Pro Leu Glu Asp Glu Cys Glu Ala Pro Leu Val Cys Arg Ala Gly Cys
    210                 215                 220

Ser Pro Glu His Gly Phe Cys Glu Gln Pro Gly Glu Cys Arg Cys Leu
225                 230                 235                 240

Glu Gly Trp Thr Gly Pro Leu Cys Thr Val Pro Val Ser Thr Ser Ser
                245                 250                 255

Cys Leu Ser Pro Arg Gly Pro Ser Ser Ala Thr Thr Gly Cys Leu Val
            260                 265                 270

Pro Gly Pro Gly Pro Cys Asp Gly Asn Pro Cys Ala Asn Gly Gly Ser
        275                 280                 285

Cys Ser Glu Thr Pro Arg Ser Phe Glu Cys Thr Cys Pro Arg Gly Phe
    290                 295                 300

Tyr Gly Leu Arg Cys Glu Val Ser Gly Val Thr Cys Ala Asp Gly Pro
305                 310                 315                 320

Cys Phe Asn Gly Gly Leu Cys Val Gly Gly Ala Asp Pro Asp Ser Ala
                325                 330                 335

Tyr Ile Cys His Cys Pro Pro Gly Phe Gln Gly Ser Asn Cys Glu Lys
            340                 345                 350

Arg Val Asp Arg Cys Ser Leu Gln Pro Cys Arg Asn Gly Gly Leu Cys
        355                 360                 365

Leu Asp Leu Gly His Ala Leu Arg Cys Arg Cys Arg Ala Gly Phe Ala
    370                 375                 380

Gly Pro Arg Cys Glu His Asp Leu Asp Asp Cys Ala Gly Arg Ala Cys
385                 390                 395                 400

Ala Asn Gly Gly Thr Cys Val Glu Gly Gly Gly Ala His Arg Cys Ser
                405                 410                 415

Cys Ala Leu Gly Phe Gly Gly Arg Asp Cys Arg Glu Arg Ala Asp Pro
            420                 425                 430
```

Cys Ala Ala Arg Pro Cys Ala His Gly Gly Arg Cys Tyr Ala His Phe
        435                 440                 445

Ser Gly Leu Val Cys Ala Cys Ala Pro Gly Tyr Met Gly Ala Arg Cys
    450                 455                 460

Glu Phe Pro Val His Pro Asp Gly Ala Ser Ala Leu Pro Ala Ala Pro
465                 470                 475                 480

Pro Gly Leu Arg Pro Gly Asp Pro Gln Arg Tyr Leu Leu Pro Pro Ala
                485                 490                 495

Leu Gly Leu Leu Val Ala Ala Gly Val Ala Gly Ala Ala Leu Leu Leu
            500                 505                 510

Val His Val Arg Arg Arg Gly His Ser Gln Asp Ala Gly Ser Arg Leu
        515                 520                 525

Leu Ala Gly Thr Pro Glu Pro Ser Val His Ala Leu Pro Asp Ala Leu
    530                 535                 540

Asn Asn Leu Arg Thr Gln Glu Gly Ser Gly Asp Gly Pro Ser Ser Ser
545                 550                 555                 560

Val Asp Trp Asn Arg Pro Glu Asp Val Asp Pro Gln Gly Ile Tyr Val
                565                 570                 575

Ile Ser Ala Pro Ser Ile Tyr Ala Arg Glu Ala
            580                 585

<210> 57
<211> 89
<212> PRT
<213> Homo sapiens

<400> 57

Met Val Ser Pro Arg Met Ser Gly Leu Leu Ser Gln Thr Val Ile Leu
1               5                   10                  15

Ala Leu Ile Phe Leu Pro Gln Thr Arg Pro Ala Gly Val Phe Glu Leu
            20                  25                  30

Gln Ile His Ser Phe Gly Pro Gly Pro Gly Pro Ala Pro Leu Pro Pro
        35                  40                  45

Leu Leu Gln Ser Leu Pro Glu Ala Trp Ala Leu Arg Gly Gly Arg Arg
    50                  55                  60

Val Pro Val Arg Pro Gly Arg Gly Ala Glu Cys Ala Arg Thr Gly Leu
65                  70                  75                  80

His Arg Ala Ala Arg Ser Ala Arg Ala
                85

<210> 58
<211> 67
<212> PRT
<213> Homo sapiens

<400> 58

Met Val Ser Pro Arg Met Ser Gly Leu Leu Ser Gln Thr Val Ile Leu
1               5                   10                  15

Ala Leu Ile Phe Leu Pro Gln Val Arg Ala Arg His Gly Pro Leu Ala
            20                  25                  30

Ser Ser Ser Cys Arg Ser Thr Leu Ser Gly Arg Val Gln Ala Leu Gly
        35                  40                  45

Pro Arg Gly Pro Pro Ala Ala Pro Gly Ser Pro Ala Ala Ser Ser Ser
    50                  55                  60

Glu Ser Ala
65

<210> 59
<211> 409
<212> PRT
<213> Homo sapiens

<400> 59

Met Val Ser Pro Arg Met Ser Gly Leu Leu Ser Gln Thr Val Ile Leu
1               5                   10                  15

Ala Leu Ile Phe Leu Pro Gln Thr Arg Pro Ala Gly Val Phe Glu Leu
            20                  25                  30

Gln Ile His Ser Phe Gly Pro Gly Pro Gly Pro Gly Ala Pro Arg Ser
        35                  40                  45

Pro Cys Ser Ala Arg Leu Pro Cys Arg Leu Phe Phe Arg Val Cys Leu
    50                  55                  60

Lys Pro Gly Leu Ser Glu Glu Ala Ala Glu Ser Pro Cys Ala Leu Gly

```
65                  70                  75                  80

Ala Ala Leu Ser Ala Arg Gly Pro Val Tyr Thr Glu Gln Pro Gly Ala
                85                  90                  95

Pro Ala Pro Asp Leu Pro Leu Pro Asp Gly Leu Leu Gln Val Pro Phe
            100                 105                 110

Arg Asp Ala Trp Pro Gly Thr Phe Ser Phe Ile Ile Glu Thr Trp Arg
        115                 120                 125

Glu Glu Leu Gly Asp Gln Ile Gly Gly Pro Ala Trp Ser Leu Leu Ala
    130                 135                 140

Arg Val Ala Gly Arg Arg Arg Leu Ala Ala Gly Gly Pro Trp Ala Arg
145                 150                 155                 160

Asp Ile Gln Arg Ala Gly Ala Trp Glu Leu Arg Phe Ser Tyr Arg Ala
                165                 170                 175

Arg Cys Glu Pro Pro Ala Val Gly Thr Ala Cys Thr Arg Leu Cys Arg
            180                 185                 190

Pro Arg Ser Ala Pro Ser Arg Cys Gly Pro Gly Leu Arg Pro Cys Ala
        195                 200                 205

Pro Leu Glu Asp Glu Cys Glu Ala Pro Leu Val Cys Arg Ala Gly Cys
    210                 215                 220

Ser Pro Glu His Gly Phe Cys Glu Gln Pro Gly Glu Cys Arg Cys Leu
225                 230                 235                 240

Glu Gly Trp Thr Gly Pro Leu Cys Thr Val Pro Val Ser Thr Ser Ser
                245                 250                 255

Cys Leu Ser Pro Arg Gly Pro Ser Ser Ala Thr Thr Gly Cys Leu Val
            260                 265                 270

Pro Gly Pro Gly Pro Cys Asp Gly Asn Pro Cys Ala Asn Gly Gly Ser
        275                 280                 285

Cys Ser Glu Thr Pro Arg Ser Phe Glu Cys Thr Cys Pro Arg Gly Phe
    290                 295                 300

Tyr Gly Leu Arg Cys Glu Val Ser Gly Val Thr Cys Ala Asp Gly Pro
305                 310                 315                 320
```

Cys Phe Asn Gly Gly Leu Cys Val Gly Gly Ala Asp Pro Asp Ser Ala
325 330 335

Tyr Ile Cys His Cys Pro Pro Gly Phe Gln Gly Ser Asn Cys Glu Lys
340 345 350

Arg Val Asp Arg Cys Ser Leu Gln Pro Cys Arg Asn Gly Glu Ala Trp
355 360 365

Arg Pro Glu Arg Arg Gly Met Gly Trp Gly Ser Trp Met Ala Gln Thr
370 375 380

Val Gln Gly Trp Asn Pro Gly Phe Asp Ser Ser Asn Pro Arg Ala Trp
385 390 395 400

Gly Pro Asp Leu Pro Pro Ala Ser Leu
405

<210> 60
<211> 18
<212> DNA
<213> Artificial Organism

<220>
<223> Synthetic oligonucleotide

<400> 60

tgtgaacagc ccggtgaa 18

<210> 61
<211> 20
<212> DNA
<213> Artificial Organism

<220>
<223> Synthetic oligonucleotide

<400> 61

gacaaggcat ccggtggtag 20

<210> 62
<211> 126
<212> DNA
<213> Artificial Organism

<220>
<223> Synthetic oligonucleotide

<400> 62

tgtgaacagc ccggtgaatg ccgatgccta gagggctgga ctggacccct ctgcacggtc 60

```
cctgtctcca ccagcagctg cctcagcccc aggggcccgt cctctgctac caccggatgc    120

cttgtc                                                               126


<210>  63
<211>  44
<212>  PRT
<213>  Artificial Organism

<220>
<223>  Synthetic peptide

<400> 63

Glu Ala Trp Arg Pro Glu Arg Arg Gly Met Gly Trp Gly Ser Trp Met
1               5                   10                  15


Ala Gln Thr Val Gln Gly Trp Asn Pro Gly Phe Asp Ser Ser Asn Pro
            20                  25                  30


Arg Ala Trp Gly Pro Asp Leu Pro Pro Ala Ser Leu
        35                  40



<210>  64
<211>  14
<212>  DNA
<213>  Homo sapiens

<400> 64

agccagacac ggcc                                                       14


<210>  65
<211>  20
<212>  DNA
<213>  Artificial Organism

<220>
<223>  Synthetic oligonucleotide

<400> 65

tcattttcct cccccaggtc                                                 20


<210>  66
<211>  21
<212>  DNA
<213>  Artificial Organism

<220>
<223>  Synthetic oligonucleotide

<400> 66

gtggatctgc agctcgaaga c                                               21
```

<210> 67
<211> 64
<212> DNA
<213> Artificial Organism

<220>
<223> Synthetic oligonucleotide

<400> 67

tcattttcct cccccaggtc agagccagac acggcccgct ggcgtcttcg agctgcagat 60

ccac 64

<210> 68
<211> 16
<212> DNA
<213> Homo sapiens

<400> 68

tcagagccag acacgg 16

<210> 69
<211> 22
<212> DNA
<213> Artificial Organism

<220>
<223> Synthetic oligonucleotide

<400> 69

tcctctccca gactgtgatc ct 22

<210> 70
<211> 91
<212> DNA
<213> Artificial Organism

<220>
<223> Synthetic oligonucleotide

<400> 70

tcctctccca gactgtgatc ctagcgctca ttttcctccc ccaggtcaga gccagacacg 60

gcccgctggc gtcttcgagc tgcagatcca c 91

<210> 71
<211> 15
<212> DNA
<213> Homo sapiens

<400> 71

cctcccccag acacg 15

<210> 72
<211> 17
<212> DNA
<213> Artificial Organism

<220>
<223> Synthetic oligonucleotide

<400> 72

acccggcccg aaagagt 17

<210> 73
<211> 95
<212> DNA
<213> Artificial Organism

<220>
<223> Synthetic oligonucleotide

<400> 73

tcctctccca gactgtgatc ctagcgctca ttttcctccc ccagacacgg cccgctggcg 60

tcttcgagct gcagatccac tctttcgggc cgggt 95

<210> 74
<211> 2359
<212> DNA
<213> Homo sapiens

<400> 74

actcccgaga cccccccacc agaaggccat ggtctcccca cggatgtccg ggctcctctc 60

ccagactgtg atcctagcgc tcattttcct cccccagaca cggcccgctg gcgtcttcga 120

gctgcagatc cactctttcg ggccgggtcc aggccctggg gccccgcggt ccccctgcag 180

cgcccggctc ccctgccgcc tcttcttcag agtctgcctg aagcctgggc tctcagagga 240

ggccgccgag tccccgtgcg ccctgggcgc ggcgctgagt gcgcgcggac cggtctacac 300

cgagcagccc ggagcgcccg cgcctgatct cccactgccc gacggcctct tgcaggtgcc 360

cttccgggac gcctggcctg gcaccttctc tttcatcatc gaaacctgga gagaggagtt 420

aggagaccag attggagggc ccgcctggag cctgctggcg cgcgtggctg gcaggcggcg 480

cttggcagcc ggaggcccgt gggcccggga cattcagcgc gcaggcgcct gggagctgcg 540

cttctcgtac cgcgcgcgct gcgagccgcc tgccgtcggg accgcgtgca cgcgcctctg 600

ccgtccgcgc agcgccccct cgcggtgcgg tccgggactg cgcccctgcg caccgctcga 660

ggacgaatgt gaggcgccgc tggtgtgccg agcaggctgc agccctgagc atggcttctg 720

tgaacagccc ggtgaatgcc gatgcctaga gggctggact ggacccctct gcacggtccc 780

tgtctccacc agcagctgcc tcagccccag ggcccgtcc tctgctacca ccggatgcct 840

tgtccctggg cctgggccct gtgacgggaa cccgtgtgcc aatggaggca gctgtagtga 900

gacacccagg tcctttgaat gcacctgccc gcgtgggttc tacgggctgc ggtgtgaggt 960

gagcggggtg acatgtgcag atggaccctg cttcaacggc ggcttgtgtg tcgggggtgc 1020

agaccctgac tctgcctaca tctgccactg cccacccggt ttccaaggct ccaactgtga 1080

gaagagggtg gaccggtgca gcctgcagcc atgccgcaat ggcggactct gcctggacct 1140

gggccacgcc ctgcgctgcc gctgccgcgc cggcttcgcg ggtcctcgct gcgagcacga 1200

cctggacgac tgcgcgggcc gcgcctgcgc taacggcggc acgtgtgtgg agggcggcgg 1260

cgcgcaccgc tgctcctgcg cgctgggctt cggcggccgc gactgccgcg agcgcgcgga 1320

cccgtgcgcc gcgcgcccct gtgctcacgg cggccgctgc tacgcccact tctccggcct 1380

cgtctgcgct tgcgctcccg gctacatggg agcgcggtgt gagttcccag tgcaccccga 1440

cggcgcaagc gccttgcccg cggccccgcc gggcctcagg cccggggacc ctcagcgcta 1500

ccttttgcct ccggctctgg gactgctcgt ggccgcgggc gtggccggcg ctgcgctctt 1560

gctggtccac gtgcgccgcc gtggccactc ccaggatgct gggtctcgct tgctggctgg 1620

gaccccggag ccgtcagtcc acgcactccc ggatgcactc aacaacctaa ggacgcagga 1680

gggttccggg gatggtccga gctcgtccgt agattggaat cgccctgaag atgtagaccc 1740

tcaagggatt tatgtcatat ctgctccttc catctacgct cgggaggtag cgacgcccct 1800

tttccccccg ctacacactg ggcgcgctgg gcagaggcag cacctgcttt ttccctaccc 1860

ttcctcgatt ctgtccgtga aatgaattgg gtagagtctc tggaaggttt taagcccatt 1920

ttcagttcta acttactttc atcctatttt gcatccctct tatcgttttg agctacctgc 1980

catcttctct ttgaaaaacc tatgggcttg aggaggtcac gatgccgact ccgccagagc 2040

ttttccactg attgtactca gcggggaggc aggggaggca gaggggcagc ctctctaatg 2100

cttcctactc attttgtttc taggcctgac gcgtctcctc catccgcacc tggagtcaga 2160

gcgtggattt ttgtatttgc tcggtggtgc ccagtctctg ccccagaggc tttggagttc 2220

aatcttgaag gggtgtctgg gggaacttta ctgttgcaag ttgtaaataa tggttattta 2280

tatcctattt tttctcaccc catctctcta gaaacaccta taaaggctat tattgtgatc 2340

aaaaaaaaaa aaaaaaaaa 2359

<210> 75
<211> 2022
<212> DNA
<213> Homo sapiens

<400> 75

actcccgaga cccccccacc agaaggccat ggtctcccca cggatgtccg ggctcctctc 60

```
ccagactgtg atcctagcgc tcattttcct cccccagaca cggcccgctg gcgtcttcga    120
gctgcagatc cactctttcg ggccgggtcc aggccctggg gccccgcggt ccccctgcag    180
cgcccggctc ccctgccgcc tcttcttcag agtctgcctg aagcctgggc tctcagagga    240
ggccgccgag tccccgtgcg ccctgggcgc ggcgctgagt gcgcgcggac cggtctacac    300
cgagcagccc ggagcgcccg cgcctgatct cccactgccc gacggcctct tgcaggtgcc    360
cttccgggac gcctggcctg gcaccttctc tttcatcatc gaaacctgga gagaggagtt    420
aggagaccag attggagggc ccgcctggag cctgctggcg cgcgtggctg gcaggcggcg    480
cttggcagcc ggaggcccgt gggcccggga cattcagcgc gcaggcgcct gggagctgcg    540
cttctcgtac cgcgcgcgct gcgagccgcc tgccgtcggg accgcgtgca cgcgcctctg    600
ccgtccgcgc agcgccccct cgcggtgcgg tccgggactg cgcccctgcg caccgctcga    660
ggacgaatgt gaggcgccgc tggtgtgccg agcaggctgc agccctgagc atggcttctg    720
tgaacagccc ggtgaatgcc gatgcctaga gggctggact ggacccctct gcacggtccc    780
tgtctccacc agcagctgcc tcagccccag ggcccgtcc tctgctacca ccggatgcct     840
tgtccctggg cctgggccct gtgacgggaa cccgtgtgcc aatggaggca gctgtagtga    900
gacacccagg tcctttgaat gcacctgccc gcgtgggttc tacgggctgc ggtgtgaggt    960
gagcggggtg acatgtgcag atggaccctg cttcaacggc ggcttgtgtg tcgggggtgc   1020
agaccctgac tctgcctaca tctgccactg cccacccggt ttccaaggct ccaactgtga   1080
gaagagggtg gaccggtgca gcctgcagcc atgccgcaat ggcggactct gcctggacct   1140
gggccacgcc ctgcgctgcc gctgccgcgc cggcttcgcg ggtcctcgct gcgagcacga   1200
cctggacgac tgcgcgggcc gcgcctgcgc taacggcggc acgtgtgtgg agggcggcgg   1260
cgcgcaccgc tgctcctgcg cgctgggctt cggcggccgc gactgccgcg agcgcgcgga   1320
cccgtgcgcc gcgcgcccct gtgctcacgg cggccgctgc tacgcccact tctccggcct   1380
cgtctgcgct tgcgctcccg gctacatggg agcgcggtgt gagttcccag tgcaccccga   1440
cggcgcaagc gccttgcccg cggccccgcc gggcctcagg cccggggacc ctcagcgcta   1500
ccttttgcct ccggctctgg gactgctcgt ggccgcgggc gtggccggcg ctgcgctctt   1560
gctggtccac gtgcgccgcc gtggccactc ccaggatgct gggtctcgct tgctggctgg   1620
gaccccggag ccgtcagtcc acgcactccc ggatgcactc aacaacctaa ggacgcagga   1680
gggttccggg gatggtccga gctcgtccgt agattggaat cgccctgaag atgtagaccc   1740
tcaagggatt tatgtcatat ctgctccttc catctacgct cgggaggcct gacgcgtctc   1800
ctccatccgc acctggagtc agagcgtgga tttttgtatt tgctcggtgg tgcccagtct   1860
ctgccccaga ggctttggag ttcaatcttg aaggggtgtc tgggggaact ttactgttgc   1920
aagttgtaaa taatggttat ttatatccta ttttttctca ccccatctct ctagaaacac   1980
```

ctataaaggc tattattgtg atcaaaaaaa aaaaaaaaaa aa 2022

<210> 76
<211> 44
<212> PRT
<213> Artificial Organism

<220>
<223> Synthetic peptide

<400> 76

```
Val Arg Ala Arg His Gly Pro Leu Ala Ser Ser Ser Cys Arg Ser Thr
1               5                   10                  15

Leu Ser Gly Arg Val Gln Ala Leu Gly Pro Arg Gly Pro Pro Ala Ala
            20                  25                  30

Pro Gly Ser Pro Ala Ala Ser Ser Ser Glu Ser Ala
        35                  40
```

<210> 77
<211> 46
<212> PRT
<213> Artificial Organism

<220>
<223> Synthetic peptide

<400> 77

```
Ala Pro Leu Pro Pro Leu Leu Gln Ser Leu Pro Glu Ala Trp Ala Leu
1               5                   10                  15

Arg Gly Gly Arg Arg Val Pro Val Arg Pro Gly Arg Gly Ala Glu Cys
            20                  25                  30

Ala Arg Thr Gly Leu His Arg Ala Ala Arg Ser Ala Arg Ala
        35                  40                  45
```

<210> 78
<211> 2809
<212> DNA
<213> Homo sapiens

<400> 78

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240
ctgcagtttc tgtttccttg actggcagct cagcggggcc ctcccgcttg gatgttccgg 300
gaaagtgatg tgggtaggac aggcggggcg agccgcaggt gccagaacac agattgtata 360
aaaggctggg ggctggtggg gagcagggga agggaatgtg accaggtcta ggtctggagt 420
ttcagcttgg acactgagcc aagcagacaa gcaaagcaag ccaggacaca ccatcctgcc 480
ccaggcccag cttctctcct gccttccaac gccatgggga gcaatctcag cccccaactc 540
tgcctgatgc cctttatctt gggcctcttg tctggaggtg tgaccaccac tccatggtct 600
ttggcccggc cccagggatc ctgctctctg gagggggtag agatcaaagg cggctccttc 660
cgacttctcc aagagggcca ggcactggag tacgtgtgtc cttctggctt ctacccgtac 720
cctgtgcaga cacgtacctg cagatctacg gggtcctgga gcaccctgaa gactcaagac 780
caaaagactg tcaggaaggc agagtgcaga gcaatccact gtccaagacc acacgacttc 840
gagaacgggg aatactggcc ccggtctccc tactacaatg tgagtgatga gatctctttc 900
cactgctatg acggttacac tctccggggc tctgccaatc gcacctgcca agtgaatggc 960
cggtggagtg ggcagacagc gatctgtgac aacggagcgg ggtactgctc caacccgggc 1020
atccccattg gcacaaggaa ggtgggcagc cagtaccgcc ttgaagacag cgtcacctac 1080
cactgcagcc gggggcttac cctgcgtggc tcccagcggc gaacgtgtca ggaaggtggc 1140
tcttggagcg ggacggagcc ttcctgccaa gactccttca tgtacgacac ccctcaagag 1200
gtggccgaag ctttcctgtc ttccctgaca gagaccatag aaggagtcga tgctgaggat 1260
gggcacggcc caggggaaca acagaagcgg aagatcgtcc tggacccttc aggctccatg 1320
aacatctacc tggtgctaga tggatcagac agcattgggg ccagcaactt cacaggagcc 1380
aaaaagtgtc tagtcaactt aattgagaag gtggcaagtt atggtgtgaa gccaagatat 1440
ggtctagtga catatgccac ataccccaaa atttgggtca aagtgtctga agcagacagc 1500
agtaatgcag actgggtcac gaagcagctc aatgaaatca attatgaaga ccacaagttg 1560
aagtcaggga ctaacaccaa gaaggccctc caggcagtgt acagcatgat gagctggcca 1620
gatgacgtcc ctcctgaagg ctggaaccgc acccgccatg tcatcatcct catgactgat 1680
ggattgcaca acatgggcgg ggacccaatt actgtcattg atgagatccg ggacttgcta 1740
tacattggca aggatcgcaa aaacccaagg gaggattatc tggatgtcta tgtgtttggg 1800
gtcgggcctt tggtgaacca agtgaacatc aatgctttgg cttccaagaa agacaatgag 1860
caacatgtgt tcaaagtcaa ggatatggaa aacctggaag atgttttcta ccaaatgatc 1920
gatgaaagcc agtctctgag tctctgtggc atggtttggg aacacaggaa gggtaccgat 1980
taccacaagc aaccatggca ggccaagatc tcagtcattc gcccttcaaa gggacacgag 2040
agctgtatgg gggctgtggt gtctgagtac tttgtgctga cagcagcaca ttgtttcact 2100

gtggatgaca aggaacactc aatcaaggtc agcgtaggag gggagaagcg ggacctggag 2160

atagaagtag tcctatttca ccccaactac aacattaatg ggaaaaaaga agcaggaatt 2220

cctgaatttt atgactatga cgttgccctg atcaagctca agaataagct gaaatatggc 2280

cagactatca ggcccatttg tctcccctgc accgagggaa caactcgagc tttgaggctt 2340

cctccaacta ccacttgcca gcaacaaaag gaagagctgc tccctgcaca ggatatcaaa 2400

gctctgtttg tgtctgagga ggagaaaaag ctgactcgga aggaggtcta catcaagaat 2460

ggggataaga aaggcagctg tgagagagat gctcaatatg ccccaggcta tgacaaagtc 2520

aaggacatct cagaggtggt cacccctcgg ttcctttgta ctggaggagt gagtccctat 2580

gctgacccca atacttgcag aggtgattct ggcggcccct tgatagttca caagagaagt 2640

cgtttcattc aagttggtgt aatcagctgg ggagtagtgg atgtctgcaa aaaccagaag 2700

cgtgctgccc tggctgaagg agaaactcca agatgaggat ttgggttttc tataaggggt 2760

ttcctgctgg acaggggcgt gggattgaat taaaacagct gcgacaaca 2809

<210> 79
<211> 3143
<212> DNA
<213> Homo sapiens

<400> 79

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240

ctgcagtttc tgtttccttg actggcagct cagcggggcc ctcccgcttg gatgttccgg 300

gaaagtgatg tgggtaggac aggcggggcg agccgcaggt gccagaacac agattgtata 360

aaaggctggg ggctggtggg gagcagggga agggaatgtg accaggtcta ggtctggagt 420

ttcagcttgg acactgagcc aagcagacaa gcaaagcaag ccaggacaca ccatcctgcc 480

ccaggcccag cttctctcct gccttccaac gccatgggga gcaatctcag cccccaactc 540

tgcctgatgc cctttatctt gggcctcttg tctggaggtg tgaccaccac tccatggtct 600

ttggcccggc cccagggatc ctgctctctg gagggggtag agatcaaagg cggctccttc 660

cgacttctcc aagagggcca ggcactggag tacgtgtgtc cttctggctt ctacccgtac 720

cctgtgcaga cacgtacctg cagatctacg gggtcctgga gcaccctgaa gactcaagac 780

caaaagactg tcaggaaggc agagtgcaga gcaatccact gtccaagacc acacgacttc 840

gagaacgggg aatactggcc ccggtctccc tactacaatg tgagtgatga gatctctttc 900

cactgctatg acggttacac tctccggggc tctgccaatc gcacctgcca agtgaatggc 960
cggtggagtg ggcagacagc gatctgtgac aacggagcgg ggtactgctc caacccgggc 1020
atccccattg gcacaaggaa ggtgggcagc cagtaccgcc ttgaagacag cgtcacctac 1080
cactgcagcc gggggcttac cctgcgtggc tcccagcggc gaacgtgtca ggaaggtggc 1140
tcttggagcg ggacggagcc ttcctgccaa gactccttca tgtacgacac ccctcaagag 1200
gtggccgaag ctttcctgtc ttccctgaca gagaccatag aaggagtcga tgctgaggat 1260
gggcacggcc caggggaaca acagaagcgg aagatcgtcc tggacccttc aggctccatg 1320
aacatctacc tggtgctaga tggatcagac agcattgggg ccagcaactt cacaggagcc 1380
aaaaagtgtc tagtcaactt aattgagaag gtggcaagtt atggtgtgaa gccaagatat 1440
ggtctagtga catatgccac ataccccaaa atttgggtca aagtgtctga agcagacagc 1500
agtaatgcag actgggtcac gaagcagctc aatgaaatca attatgaaga ccacaagttg 1560
aagtcaggga ctaacaccaa gaaggccctc caggcagtgt acagcatgat gagctggcca 1620
gatgacgtcc ctcctgaagg ctggaaccgc acccgccatg tcatcatcct catgactgat 1680
ggtcagaagg gacctctctc ctgtcccagc ctccccacct tctcagacca gcatgtggcc 1740
cttaagtcca cttgtaacac tatacccatg gttggggccc tgaatgtgac tcatagctgg 1800
ctgttcatct ctcctgtgac ccttcataag gaattcttcc taagccctgt gatcaactat 1860
ctctaaccct tcctcaactt gctcaccctg ccatgtgtat ccctgccttt agccagttta 1920
tcttccttat ctcctaccct catggtcctg tctcttctgc aggattgcac aacatgggcg 1980
gggacccaat tactgtcatt gatgagatcc gggacttgct atacattggc aaggatcgca 2040
aaaacccaag ggaggattat ctggatgtct atgtgtttgg ggtcgggcct ttggtgaacc 2100
aagtgaacat caatgctttg gcttccaaga aagacaatga gcaacatgtg ttcaaagtca 2160
aggatatgga aaacctggaa gatgttttct accaaatgat cgatgaaagc cagtctctga 2220
gtctctgtgg catggtttgg gaacacagga agggtaccga ttaccacaag caaccatggc 2280
aggccaagat ctcagtcatt cgcccttcaa agggacacga gagctgtatg ggggctgtgg 2340
tgtctgagta ctttgtgctg acagcagcac attgtttcac tgtggatgac aaggaacact 2400
caatcaaggt cagcgtagga ggggagaagc gggacctgga gatagaagta gtcctatttc 2460
accccaacta caacattaat gggaaaaaag aagcaggaat tcctgaattt tatgactatg 2520
acgttgccct gatcaagctc aagaataagc tgaaatatgg ccagactatc aggcccattt 2580
gtctcccctg caccgaggga acaactcgag ctttgaggct tcctccaact accacttgcc 2640
agcaacaaaa ggaagagctg ctccctgcac aggatatcaa agctctgttt gtgtctgagg 2700
aggagaaaaa gctgactcgg aaggaggtct acatcaagaa tggggataag aaaggcagct 2760
gtgagagaga tgctcaatat gccccaggct atgacaaagt caaggacatc tcagaggtgg 2820

tcacccctcg gttcctttgt actggaggag tgagtcccta tgctgacccc aatacttgca 2880

gaggtgattc tggcggcccc ttgatagttc acaagagaag tcgtttcatt caagttggtg 2940

taatcagctg gggagtagtg gatgtctgca aaaaccagaa gcggcaaaag caggtacctg 3000

ctcacgcccg agactttcac atcaacctct ttcaagtgct gccctggctg aaggagaaac 3060

tccaagatga ggatttgggt tttctataag ggtttcctg ctggacaggg gcgtgggatt 3120

gaattaaaac agctgcgaca aca 3143

<210> 80
<211> 3262
<212> DNA
<213> Homo sapiens

<400> 80

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240

ctgcagtttc tgtttccttg actggcagct cagcggggcc ctcccgcttg gatgttccgg 300

gaaagtgatg tgggtaggac aggcggggcg agccgcaggt gccagaacac agattgtata 360

aaaggctggg ggctggtggg gagcagggga agggaatgtg accaggtcta ggtctggagt 420

ttcagcttgg acactgagcc aagcagacaa gcaaagcaag ccaggacaca ccatcctgcc 480

ccaggcccag cttctctcct gccttccaac gccatgggga gcaatctcag cccccaactc 540

tgcctgatgc cctttatctt gggcctcttg tctggaggtg tgaccaccac tccatggtct 600

ttggcccggc cccagggatc ctgctctctg gagggggtag agatcaaagg cggctccttc 660

cgacttctcc aagagggcca ggcactggag tacgtgtgtc cttctggctt ctacccgtac 720

cctgtgcaga cacgtacctg cagatctacg gggtcctgga gcaccctgaa gactcaagac 780

caaaagactg tcaggaaggc agagtgcaga ggtttgaggg caatgagtgt gggcagtggc 840

ctaaggcaga aacagggcag gcggcagcaa ggtcaggact aggatgagac taggcagggt 900

gacaaggtgg gctgaccggg agtaggagca gttttagggt ggcaggcgga aagggggcaa 960

gaaaaagcgg agttaaccct tactaagcat ttaccctggg cttccaggca gccctggaag 1020

tcaagagaac actcagaaat ggggagggag aagcagtgga aatccatatg ggttgaggag 1080

taggtaagat gctgcttctg cgggactggg aatgcgctgt ttctcagtga catggtctcc 1140

gagaccagga gggatacacc taaggcagcc tttccctctt gatgacttct acttgtcccc 1200

ccttctcaaa gcaatccact gtccaagacc acacgacttc gagaacgggg aatactggcc 1260

ccggtctccc tactacaatg tgagtgatga gatctctttc cactgctatg acggttacac 1320

tctccggggc tctgccaatc gcacctgcca agtgaatggc cggtggagtg ggcagacagc 1380

gatctgtgac aacggagcgg ggtactgctc caacccgggc atccccattg gcacaaggaa 1440

ggtgggcagc cagtaccgcc ttgaagacag cgtcacctac cactgcagcc gggggcttac 1500

cctgcgtggc tcccagcggc gaacgtgtca ggaaggtggc tcttggagcg ggacggagcc 1560

ttcctgccaa gactccttca tgtacgacac ccctcaagag gtggccgaag ctttcctgtc 1620

ttccctgaca gagaccatag aaggagtcga tgctgaggat gggcacggcc caggggaaca 1680

acagaagcgg aagatcgtcc tggacccttc aggctccatg aacatctacc tggtgctaga 1740

tggatcagac agcattgggg ccagcaactt cacaggagcc aaaaagtgtc tagtcaactt 1800

aattgagaag gtggcaagtt atggtgtgaa gccaagatat ggtctagtga catatgccac 1860

ataccccaaa atttgggtca aagtgtctga agcagacagc agtaatgcag actgggtcac 1920

gaagcagctc aatgaaatca attatgaaga ccacaagttg aagtcaggga ctaacaccaa 1980

gaaggccctc caggcagtgt acagcatgat gagctggcca gatgacgtcc ctcctgaagg 2040

ctggaaccgc acccgccatg tcatcatcct catgactgat ggattgcaca acatgggcgg 2100

ggacccaatt actgtcattg atgagatccg ggacttgcta tacattggca aggatcgcaa 2160

aaacccaagg gaggattatc tggatgtcta tgtgtttggg gtcgggcctt tggtgaacca 2220

agtgaacatc aatgctttgg cttccaagaa agacaatgag caacatgtgt tcaaagtcaa 2280

ggatatggaa aacctggaag atgttttcta ccaaatgatc gatgaaagcc agtctctgag 2340

tctctgtggc atggtttggg aacacaggaa gggtaccgat taccacaagc aaccatggca 2400

ggccaagatc tcagtcattc gcccttcaaa gggacacgag agctgtatgg gggctgtggt 2460

gtctgagtac tttgtgctga cagcagcaca ttgtttcact gtggatgaca aggaacactc 2520

aatcaaggtc agcgtaggag gggagaagcg ggacctggag atagaagtag tcctatttca 2580

ccccaactac aacattaatg ggaaaaaaga agcaggaatt cctgaatttt atgactatga 2640

cgttgccctg atcaagctca agaataagct gaaatatggc cagactatca ggcccatttg 2700

tctcccctgc accgagggaa caactcgagc tttgaggctt cctccaacta ccacttgcca 2760

gcaacaaaag gaagagctgc tccctgcaca ggatatcaaa gctctgtttg tgtctgagga 2820

ggagaaaaag ctgactcgga aggaggtcta catcaagaat ggggataaga aaggcagctg 2880

tgagagagat gctcaatatg ccccaggcta tgacaaagtc aaggacatct cagaggtggt 2940

cacccctcgg ttcctttgta ctggaggagt gagtccctat gctgacccca atacttgcag 3000

aggtgattct ggcggcccct tgatagttca caagagaagt cgtttcattc aagttggtgt 3060

aatcagctgg ggagtagtgg atgtctgcaa aaaccagaag cggcaaaagc aggtacctgc 3120

tcacgcccga gactttcaca tcaacctctt tcaagtgctg ccctggctga aggagaaact 3180

ccaagatgag gatttgggtt ttctataagg ggtttcctgc tggacagggg cgtgggattg 3240

aattaaaaca gctgcgacaa ca 3262

<210> 81
<211> 3132
<212> DNA
<213> Homo sapiens

<400> 81

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240

ctgcagtttc tgtttccttg actggcagct cagcggggcc ctcccgcttg gatgttccgg 300

gaaagtgatg tgggtaggac aggcggggcg agccgcaggt gccagaacac agattgtata 360

aaaggctggg ggctggtggg gagcagggga agggaatgtg accaggtcta ggtctggagt 420

ttcagcttgg acactgagcc aagcagacaa gcaaagcaag ccaggacaca ccatcctgcc 480

ccaggcccag cttctctcct gccttccaac gccatgggga gcaatctcag cccccaactc 540

tgcctgatgc cctttatctt gggcctcttg tctggaggtg tgaccaccac tccatggtct 600

ttggcccggc cccagggatc ctgctctctg gagggggtag agatcaaagg cggctccttc 660

cgacttctcc aagagggcca ggcactggag tacgtgtgtc cttctggctt ctacccgtac 720

cctgtgcaga cacgtacctg cagatctacg gggtcctgga gcaccctgaa gactcaagac 780

caaaagactg tcaggaaggc agagtgcaga gcaatccact gtccaagacc acacgacttc 840

gagaacgggg aatactggcc ccggtctccc tactacaatg tgagtgatga gatctctttc 900

cactgctatg acggttacac tctccggggc tctgccaatc gcacctgcca agtgaatggc 960

cggtggagtg ggcagacagc gatctgtgac aacggagcgg ggtactgctc caacccgggc 1020

atccccattg gcacaaggaa ggtgggcagc cagtaccgcc ttgaagacag cgtcacctac 1080

cactgcagcc gggggcttac cctgcgtggc tcccagcggc gaacgtgtca ggaaggtggc 1140

tcttggagcg ggacggagcc ttcctgccaa gactccttca tgtacgacac ccctcaagag 1200

gtggccgaag ctttcctgtc ttccctgaca gagaccatag aaggagtcga tgctgaggat 1260

gggcacggcc caggggaaca acagaagcgg aagatcgtcc tggacccttc aggctccatg 1320

aacatctacc tggtgctaga tggatcagac agcattgggg ccagcaactt cacaggagcc 1380

aaaaagtgtc tagtcaactt aattgagaag gtggcaagtt atggtgtgaa gccaagatat 1440

ggtctagtga catatgccac ataccccaaa atttgggtca aagtgtctga agcagacagc 1500

agtaatgcag actgggtcac gaagcagctc aatgaaatca attatgaaga ccacaagttg 1560

aagtcaggga ctaacaccaa gaaggccctc caggcagtgt acagcatgat gagctggcca 1620

gatgacgtcc ctcctgaagg ctggaaccgc acccgccatg tcatcatcct catgactgat 1680

ggattgcaca acatgggcgg ggacccaatt actgtcattg atgagatccg ggacttgcta 1740

tacattggca aggatcgcaa aaacccaagg gaggattatc tggatgtcta tgtgtttggg 1800

gtcgggcctt tggtgaacca agtgaacatc aatgctttgg cttccaagaa agacaatgag 1860

caacatgtgt tcaaagtcaa ggatatggaa aacctggaag atgttttcta ccaaatgatc 1920

gatgaaagcc agtctctgag tctctgtggc atggtttggg aacacaggaa gggtaccgat 1980

taccacaagc aaccatggca ggccaagatc tcagtcattc gcccttcaaa gggacacgag 2040

agctgtatgg gggctgtggt gtctgagtac tttgtgctga cagcagcaca ttgtttcact 2100

gtggatgaca aggaacactc aatcaaggtc agcgtaggag gggagaagcg ggacctggag 2160

atagaagtag tcctatttca ccccaactac aacattaatg ggaaaaaaga agcaggaatt 2220

cctgaatttt atgactatga cgttgccctg atcaagctca agaataagct gaaatatggc 2280

cagactatca ggcccatttg tctcccctgc accgagggaa caactcgagc tttgaggctt 2340

cctccaacta ccacttgcca gcaacaaaag gaagagctgc tccctgcaca ggatatcaaa 2400

gctctgtttg tgtctgagga ggagaaaaag ctgactcgga aggaggtcta catcaagaat 2460

ggggataaga aaggcagctg tgagagagat gctcaatatg ccccaggcta tgacaaagtc 2520

aaggacatct cagaggtggt cacccctcgg ttcctttgta ctggaggagt gagtccctat 2580

gctgacccca atacttgcag aggtgattct ggcggcccct tgatagttca caagagaagt 2640

cgtttcattc aagtgagtcc tccctttcct atctggggag atgccaagtg gtcagcatgg 2700

gccccaaagc aggaaagctc aatgcatgtg gctagtaatt cgaggtaggc agagcctgcc 2760

tcaccttagg accgcatgtc ttgcctgcgt gtgtcaagaa cgaggctgag ctgggtccct 2820

agtctgattc ctttaggtca gctaagacgc aagcaggaac agccatgctt ccaggattag 2880

gaattctact gaatgatcca tggcacccca ctgcctctgc aggttggtgt aatcagctgg 2940

ggagtagtgg atgtctgcaa aaaccagaag cggcaaaagc aggtacctgc tcacgcccga 3000

gactttcaca tcaacctctt tcaagtgctg ccctggctga aggagaaact ccaagatgag 3060

gatttgggtt ttctataagg ggtttcctgc tggacagggg cgtgggattg aattaaaaca 3120

gctgcgacaa ca 3132

<210> 82
<211> 2860
<212> DNA
<213> Homo sapiens

<400> 82

ggccttgggg gagggggagg ccagaatgac tccaagagct acaggaaggc aggtcagaga 60
ccccactgga caaacagtgg ctggactctg caccataaca cacaatcaac aggggagtga 120
gctggatcct tatttctggt ccctaagtgg gtggtttggg cttactgggg aggagctaag 180
gccggagagg aggtactgaa ggggagagtc ctggaccttt ggcagcaaag ggtgggactt 240
ctgcagtttc tgtttccttg actggcagct cagcggggcc ctcccgcttg gatgttccgg 300
gaaagtgatg tgggtaggac aggcggggcg agccgcaggt gccagaacac agattgtata 360
aaaggctggg ggctggtggg gagcagggga agggaatgtg accaggtcta ggtctggagt 420
ttcagcttgg acactgagcc aagcagacaa gcaaagcaag ccaggacaca ccatcctgcc 480
ccaggcccag cttctctcct gccttccaac gccatgggga gcaatctcag cccccaactc 540
tgcctgatgc cctttatctt gggcctcttg tctggaggtg tgaccaccac tccatggtct 600
ttggcccggc cccagggatc ctgctctctg gagggggtag agatcaaagg cggctccttc 660
cgacttctcc aagagggcca ggcactggag tacgtgtgtc cttctggctt ctacccgtac 720
cctgtgcaga cacgtacctg cagatctacg gggtcctgga gcaccctgaa gactcaagac 780
caaaagactg tcaggaaggc agagtgcaga gcaatccact gtccaagacc acacgacttc 840
gagaacgggg aatactggcc ccggtctccc tactacaatg tgagtgatga gatctctttc 900
cactgctatg acggttacac tctccggggc tctgccaatc gcacctgcca agtgaatggc 960
cggtggagtg ggcagacagc gatctgtgac aacggagcgg ggtactgctc caacccgggc 1020
atccccattg gcacaaggaa ggtgggcagc cagtaccgcc ttgaagacag cgtcacctac 1080
cactgcagcc gggggcttac cctgcgtggc tcccagcggc gaacgtgtca ggaaggtggc 1140
tcttggagcg ggacggagcc ttcctgccaa gactccttca tgtacgacac ccctcaagag 1200
gtggccgaag ctttcctgtc ttccctgaca gagaccatag aaggagtcga tgctgaggat 1260
gggcacggcc caggggaaca acagaagcgg aagatcgtcc tggacccttc aggctccatg 1320
aacatctacc tggtgctaga tggatcagac agcattgggg ccagcaactt cacaggagcc 1380
aaaaagtgtc tagtcaactt aattgagaag gtggcaagtt atggtgtgaa gccaagatat 1440
ggtctagtga catatgccac ataccccaaa atttgggtca aagtgtctga agcagacagc 1500
agtaatgcag actgggtcac gaagcagctc aatgaaatca attatgaaga ccacaagttg 1560
aagtcaggga ctaacaccaa gaaggccctc caggcagtgt acagcatgat gagctggcca 1620
gatgacgtcc ctcctgaagg ctggaaccgc acccgccatg tcatcatcct catgactgat 1680
ggattgcaca acatgggcgg ggacccaatt actgtcattg atgagatccg ggacttgcta 1740
tacattggca aggatcgcaa aaacccaagg gaggattatc tggatgtcta tgtgtttggg 1800
gtcgggcctt tggtgaacca agtgaacatc aatgctttgg cttccaagaa agacaatgag 1860

caacatgtgt tcaaagtcaa ggatatggaa aacctggaag atgttttcta ccaaatgatc 1920

gatgaaagcc agtctctgag tctctgtggc atggtttggg aacacaggaa gggtaccgat 1980

taccacaagc aaccatggca ggccaagatc tcagtcattc gcccttcaaa gggacacgag 2040

agctgtatgg gggctgtggt gtctgagtac tttgtgctga cagcagcaca ttgtttcact 2100

gtggatgaca aggaacactc aatcaaggtc agcgtaggag gggagaagcg ggacctggag 2160

atagaagtag tcctatttca ccccaactac aacattaatg ggaaaaaaga agcaggaatt 2220

cctgaatttt atgactatga cgttgccctg atcaagctca agaataagct gaaatatggc 2280

cagactatca ggcccatttg tctcccctgc accgagggaa caactcgagc tttgaggctt 2340

cctccaacta ccacttgcca gcaacaaaga agagctgctc cctgcacagg atatcaaagc 2400

tctgtttgtg tctgaggagg agaaaaagct gactcggaag gaggtctaca tcaagaatgg 2460

ggataagaaa ggcagctgtg agagagatgc tcaatatgcc ccaggctatg acaaagtcaa 2520

ggacatctca gaggtggtca cccctcggtt cctttgtact ggaggagtga gtccctatgc 2580

tgaccccaat acttgcagag gtgattctgg cggccccttg atagttcaca agagaagtcg 2640

tttcattcaa gttggtgtaa tcagctgggg agtagtggat gtctgcaaaa accagaagcg 2700

gcaaaagcag gtacctgctc acgcccgaga ctttcacatc aacctctttc aagtgctgcc 2760

ctggctgaag gagaaactcc aagatgagga tttgggtttt ctataagggg tttcctgctg 2820

gacaggggcg tgggattgaa ttaaaacagc tgcgacaaca 2860

<210> 83
<211> 3130
<212> DNA
<213> Homo sapiens

<400> 83

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240

ctgcagtttc tgtttccttg actggcagct cagcggggcc ctcccgcttg gatgttccgg 300

gaaagtgatg tgggtaggac aggcggggcg agccgcaggt gccagaacac agattgtata 360

aaaggctggg ggctggtggg gagcagggga agggaatgtg accaggtcta ggtctggagt 420

ttcagcttgg acactgagcc aagcagacaa gcaaagcaag ccaggacaca ccatcctgcc 480

ccaggcccag cttctctcct gccttccaac gccatgggga gcaatctcag cccccaactc 540

tgcctgatgc cctttatctt gggcctcttg tctggaggtg tgaccaccac tccatggtct 600

ttggcccggc cccagggatc ctgctctctg gagggggtag agatcaaagg cggctccttc 660

```
cgacttctcc aagagggcca ggcactggag tacgtgtgtc cttctggctt ctacccgtac     720
cctgtgcaga cacgtacctg cagatctacg gggtcctgga gcaccctgaa gactcaagac     780
caaaagactg tcaggaaggc agagtgcaga gcaatccact gtccaagacc acacgacttc     840
gagaacgggg aatactggcc ccggtctccc tactacaatg tgagtgatga gatctctttc     900
cactgctatg acggttacac tctccggggc tctgccaatc gcacctgcca agtgaatggc     960
cggtggagtg ggcagacagc gatctgtgac aacggaggtg agaagcatcc cctcccccta    1020
cattgctgtc tccctgacgg cgcccagccc gaggagtggg cactcggctc cggacactgt    1080
aactcttgct ctctaccttg ctcacggggc ctcaggcttc agtgcttacc tcgatgtctc    1140
atacctctgc agcggggtac tgctccaacc cgggcatccc cattggcaca aggaaggtgg    1200
gcagccagta ccgccttgaa gacagcgtca cctaccactg cagccggggg cttaccctgc    1260
gtggctccca gcggcgaacg tgtcaggaag gtggctcttg gagcgggacg gagccttcct    1320
gccaagactc cttcatgtac gacacccctc aagaggtggc cgaagctttc ctgtcttccc    1380
tgacagagac catagaagga gtcgatgctg aggatgggca cggcccaggg gaacaacaga    1440
agcggaagat cgtcctggac ccttcaggct ccatgaacat ctacctggtg ctagatggat    1500
cagacagcat tggggccagc aacttcacag gagccaaaaa gtgtctagtc aacttaattg    1560
agaaggtgga atcctcctat ccctgaactc gggggaatgg aatctcgctg atcttccagg    1620
actagctccc tgatcattcc agcccctctg aacaacaggg ccccaggaaa atctccaggt    1680
ggcaagttat ggtgtgaagc caagatatgg tctagtgaca tatgccacat accccaaaat    1740
ttgggtcaaa gtgtctgaag cagacagcag taatgcagac tgggtcacga agcagctcaa    1800
tgaaatcaat tatgaagacc acaagttgaa gtcagggact aacaccaaga aggccctcca    1860
ggcagtgtac agcatgatga gctggccaga tgacgtccct cctgaaggct ggaaccgcac    1920
ccgccatgtc atcatcctca tgactgatgg attgcacaac atgggcgggg acccaattac    1980
tgtcattgat gagatccggg acttgctata cattggcaag gatcgcaaaa acccaaggga    2040
ggattatctg gatgtctatg tgtttggggt cgggcctttg gtgaaccaag tgaacatcaa    2100
tgctttggct tccaagaaag acaatgagca acatgtgttc aaagtcaagg atatggaaaa    2160
cctggaagat gttttctacc aaatgatcga tgaaagccag tctctgagtc tctgtggcat    2220
ggtttgggaa cacaggaagg gtaccgatta ccacaagcaa ccatggcagg ccaagatctc    2280
agtcattcgc ccttcaaagg gacacgagag ctgtatgggg gctgtggtgt ctgagtactt    2340
tgtgctgaca gcagcacatt gtttcactgt ggatgacaag gaacactcaa tcaaggtcag    2400
cgtaggaggg gagaagcggg acctggagat agaagtagtc ctatttcacc ccaactacaa    2460
cattaatggg aaaaaagaag caggaattcc tgaattttat gactatgacg ttgccctgat    2520
```

caagctcaag aataagctga aatatggcca gactatcagg cccatttgtc tcccctgcac 2580

cgagggaaca actcgagctt tgaggcttcc tccaactacc acttgccagc aacaaaagga 2640

agagctgctc cctgcacagg atatcaaagc tctgtttgtg tctgaggagg agaaaaagct 2700

gactcggaag gaggtctaca tcaagaatgg ggataagaaa ggcagctgtg agagagatgc 2760

tcaatatgcc ccaggctatg acaaagtcaa ggacatctca gaggtggtca cccctcggtt 2820

cctttgtact ggaggagtga gtccctatgc tgaccccaat acttgcagag gtgattctgg 2880

cggccccttg atagttcaca agagaagtcg tttcattcaa gttggtgtaa tcagctgggg 2940

agtagtggat gtctgcaaaa accagaagcg gcaaaagcag gtacctgctc acgcccgaga 3000

ctttcacatc aacctctttc aagtgctgcc ctggctgaag gagaaactcc aagatgagga 3060

tttgggtttt ctataagggg tttcctgctg gacaggggcg tgggattgaa ttaaaacagc 3120

tgcgacaaca 3130

<210> 84
<211> 2785
<212> DNA
<213> Homo sapiens

<400> 84

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240

ctgcagtttc tgtttccttg actggcagct cagcggggcc ctcccgcttg gatgttccgg 300

gaaagtgatg tgggtaggac aggcggggcg agccgcaggt gccagaacac agattgtata 360

aaaggctggg ggctggtggg gagcagggga agggaatgtg accaggtcta ggtctggagt 420

ttcagcttgg acactgagcc aagcagacaa gcaaagcaag ccaggacaca ccatcctgcc 480

ccaggcccag cttctctcct gccttccaac gccatgggga gcaatctcag cccccaactc 540

tgcctgatgc cctttatctt gggcctcttg tctggaggtg tgaccaccac tccatggtct 600

ttggcccggc cccagggatc ctgctctctg gagggggtag agatcaaagg cggctccttc 660

cgacttctcc aagagggcca ggcactggag tacgtgtgtc cttctggctt ctacccgtac 720

cctgtgcaga cacgtacctg cagatctacg ggtcctgga gcaccctgaa gactcaagac 780

caaaagactg tcaggaaggc agagtgcaga gcaatccact gtccaagacc acacgacttc 840

gagaacgggg aatactggcc ccggtctccc tactacaatg tgagtgatga gatctctttc 900

cactgctatg acggttacac tctccggggc tctgccaatc gcacctgcca agtgaatggc 960

cggtggagtg ggcagacagc gatctgtgac aacggagcgg ggtactgctc caacccgggc 1020

atccccattg gcacaaggaa ggtgggcagc cagtaccgcc ttgaagacag cgtcacctac 1080

cactgcagcc gggggcttac cctgcgtggc tcccagcggc gaacgtgtca ggaaggtggc 1140

tcttggagcg ggacggagcc ttcctgccaa gactccttca tgtacgacac ccctcaagag 1200

gtggccgaag ctttcctgtc ttccctgaca gagaccatag aaggagtcga tgctgaggat 1260

gggcacggcc caggggaaca acagaagcgg aagatcgtcc tggacccttc aggctccatg 1320

aacatctacc tggtgctaga tggatcagac agcattgggg ccagcaactt cacaggagcc 1380

aaaaagtgtc tagtcaactt aattgagaag gtggcaagtt atggtgtgaa gccaagatat 1440

ggtctagtga catatgccac ataccccaaa atttgggtca aagtgtctga agcagacagc 1500

agtaatgcag actgggtcac gaagcagctc aatgaaatca attatgaaga ccacaagttg 1560

aagtcaggga ctaacaccaa gaaggccctc caggcagtgt acagcatgat gagctggcca 1620

gatgacgtcc ctcctgaagg ctggaaccgc acccgccatg tcatcatcct catgactgat 1680

ggattgcaca acatgggcgg ggacccaatt actgtcattg atgagatccg ggacttgcta 1740

tacattggca aggatcgcaa aaacccaagg gaggattatc tggatgtcta tgtgtttggg 1800

gtcgggcctt tggtgaacca agtgaacatc aatgctttgg cttccaagaa agacaatgag 1860

caacatgtgt tcaaagtcaa ggatatggaa aacctggaag atgttttcta ccaaatgatc 1920

gatgaaagcc agtctctgag tctctgtggc atggtttggg aacacaggaa gggtaccgat 1980

taccacaagc aaccatggca ggccaagatc tcagtcattc gcccttcaaa gggacacgag 2040

agctgtatgg gggctgtggt gtctgagtac tttgtgctga cagcagcaca ttgtttcact 2100

gtggatgaca aggaacactc aatcaaggtc agcgtaggag gggagaagcg ggacctggag 2160

atagaagtag tcctatttca ccccaactac aacattaatg ggaaaaaaga agcaggaatt 2220

cctgaatttt atgactatga cgttgccctg atcaagctca agaataagct gaaatatggc 2280

cagactatca gaggaagagc tgctccctgc acaggatatc aaagctctgt ttgtgtctga 2340

ggaggagaaa aagctgactc ggaaggaggt ctacatcaag aatggggata agaaaggcag 2400

ctgtgagaga gatgctcaat atgccccagg ctatgacaaa gtcaaggaca tctcagaggt 2460

ggtcacccct cggttccttt gtactggagg agtgagtccc tatgctgacc ccaatacttg 2520

cagaggtgat tctggcggcc ccttgatagt tcacaagaga agtcgtttca ttcaagttgg 2580

tgtaatcagc tggggagtag tggatgtctg caaaaaccag aagcggcaaa agcaggtacc 2640

tgctcacgcc cgagactttc acatcaacct ctttcaagtg ctgccctggc tgaaggagaa 2700

actccaagat gaggatttgg gttttctata aggggtttcc tgctggacag gggcgtggga 2760

ttgaattaaa acagctgcga caaca 2785

<210> 85

<211> 3228
<212> DNA
<213> Homo sapiens

<400> 85

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240

ctgcagtttc tgtttccttg actggcagct cagcggggcc ctcccgcttg gatgttccgg 300

gaaagtgatg tgggtaggac aggcggggcg agccgcaggt gccagaacac agattgtata 360

aaaggctggg ggctggtggg gagcagggga agggaatgtg accaggtcta ggtctggagt 420

ttcagcttgg acactgagcc aagcagacaa gcaaagcaag ccaggacaca ccatcctgcc 480

ccaggcccag cttctctcct gccttccaac gccatgggga gcaatctcag cccccaactc 540

tgcctgatgc cctttatctt gggcctcttg tctggaggtg tgaccaccac tccatggtct 600

ttggcccggc cccagggatc ctgctctctg gagggggtag agatcaaagg cggctccttc 660

cgacttctcc aagagggcca ggcactggag tacgtgtgtc cttctggctt ctacccgtac 720

cctgtgcaga cacgtacctg cagatctacg gggtcctgga gcaccctgaa gactcaagac 780

caaaagactg tcaggaaggc agagtgcaga gcaatccact gtccaagacc acacgacttc 840

gagaacgggg aatactggcc ccggtctccc tactacaatg tgagtgatga gatctctttc 900

cactgctatg acggttacac tctccggggc tctgccaatc gcacctgcca agtgaatggc 960

cggtggagtg ggcagacagc gatctgtgac aacggagcgg ggtactgctc caacccgggc 1020

atccccattg gcacaaggaa ggtgggcagc cagtaccgcc ttgaagacag cgtcacctac 1080

cactgcagcc gggggcttac cctgcgtggc tcccagcggc gaacgtgtca ggaaggtggc 1140

tcttggagcg ggacggagcc ttcctgccaa gactccttca tgtacgacac ccctcaagag 1200

gtggccgaag ctttcctgtc ttccctgaca gagaccatag aaggagtcga tgctgaggat 1260

gggcacggcc caggggaaca acagaagcgg aagatcgtcc tggacccttc aggctccatg 1320

aacatctacc tggtgctaga tggatcagac agcattgggg ccagcaactt cacaggagcc 1380

aaaaagtgtc tagtcaactt aattgagaag gtggcaagtt atggtgtgaa gccaagatat 1440

ggtctagtga catatgccac ataccccaaa atttgggtca aagtgtctga agcagacagc 1500

agtaatgcag actgggtcac gaagcagctc aatgaaatca attatgaaga ccacaagttg 1560

aagtcaggga ctaacaccaa gaaggccctc caggcagtgt acagcatgat gagctggcca 1620

gatgacgtcc ctcctgaagg ctggaaccgc acccgccatg tcatcatcct catgactgat 1680

ggattgcaca acatgggcgg ggacccaatt actgtcattg atgagatccg ggacttgcta 1740

tacattggca aggatcgcaa aaacccaagg gaggattatc tggatgtcta tgtgtttggg 1800

gtcgggcctt tggtgaacca agtgaacatc aatgctttgg cttccaagaa agacaatgag 1860

caacatgtgt tcaaagtcaa ggatatggaa aacctggaag atgttttcta ccaaatgatc 1920

gatgaaagcc agtctctgag tctctgtggc atggtttggg aacacaggaa gggtaccgat 1980

taccacaagc aaccatggca ggccaagatc tcagtcattc gcccttcaaa gggacacgag 2040

agctgtatgg gggctgtggt gtctgagtac tttgtgctga cagcagcaca ttgtttcact 2100

gtggatgaca aggaacactc aatcaaggtc agcgtaggag gggagaagcg ggacctggag 2160

atagaagtag tcctatttca ccccaactac aacattaatg ggaaaaaaga agcaggaatt 2220

cctgaatttt atgactatga cgttgccctg atcaagctca agaataagct gaaatatggc 2280

cagactatca ggcccatttg tctcccctgc accgagggaa caactcgagc tttgaggctt 2340

cctccaacta ccacttgcca gcaacaaaag gaagagctgc tccctgcaca ggatatcaaa 2400

gctctgtttg tgtctgagga ggagaaaaag ctgactcgga aggaggtcta catcaagaat 2460

ggggataagg tgagaaacgg gcatcctaag gaggcactct aggccccaat ccttcctaag 2520

ccacttctgt tcattacttc tccatgcttc ccacctcccc tacagaaagg cagctgtgag 2580

agagatgctc aatatgcccc aggctatgac aaagtcaagg acatctcaga ggtggtcacc 2640

cctcggttcc tttgtactgg aggagtgagt ccctatgctg accccaatac ttgcagaggt 2700

gattctggcg gccccttgat agttcacaag agaagtcgtt tcattcaagt gagtcctccc 2760

tttcctatct ggggagatgc caagtggtca gcatgggccc caaagcagga aagctcaatg 2820

catgtggcta gtaattcgag gtaggcagag cctgcctcac cttaggaccg catgtcttgc 2880

ctgcgtgtgt caagaacgag gctgagctgg gtccctagtc tgattccttt aggtcagcta 2940

agacgcaagc aggaacagcc atgcttccag gattaggaat tctactgaat gatccatggc 3000

accccactgc ctctgcaggt tggtgtaatc agctggggag tagtggatgt ctgcaaaaac 3060

cagaagcggc aaaagcaggt acctgctcac gcccgagact ttcacatcaa cctctttcaa 3120

gtgctgccct ggctgaagga gaaactccaa gatgaggatt tgggttttct ataaggggtt 3180

tcctgctgga caggggcgtg ggattgaatt aaaacagctg cgacaaca 3228

<210> 86
<211> 2631
<212> DNA
<213> Homo sapiens

<400> 86

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180
gccggagagg aggtactgaa ggggagagtc ctggaccttt ggcagcaaag ggtgggactt 240
ctgcagtttc tgtttccttg actggcagct cagcggggcc ctcccgcttg gatgttccgg 300
gaaagtgatg tgggtaggac aggcggggcg agccgcaggt gccagaacac agattgtata 360
aaaggctggg ggctggtggg gagcagggga agggaatgtg accaggtcta ggtctggagt 420
ttcagcttgg acactgagcc aagcagacaa gcaaagcaag ccaggacaca ccatcctgcc 480
ccaggcccag cttctctcct gccttccaac gccatgggga gcaatctcag cccccaactc 540
tgcctgatgc cctttatctt gggcctcttg tctggaggtg tgaccaccac tccatggtct 600
ttggcccggc cccagggatc ctgctctctg gagggggtag agatcaaagg cggctccttc 660
cgacttctcc aagagggcca ggcactggag tacgtgtgtc cttctggctt ctacccgtac 720
cctgtgcaga cacgtacctg cagatctacg gggtcctgga gcaccctgaa gactcaagac 780
caaaagactg tcaggaaggc agagtgcaga gcaatccact gtccaagacc acacgacttc 840
gagaacgggg aatactggcc ccggtctccc tactacaatg tgagtgatga gatctctttc 900
cactgctatg acggttacac tctccggggc tctgccaatc gcacctgcca agtgaatggc 960
cggtggagtg ggcagacagc gatctgtgac aacggagcgg ggtactgctc caacccgggc 1020
atccccattg gcacaaggaa ggtgggcagc cagtaccgcc ttgaagacag cgtcacctac 1080
cactgcagcc gggggcttac cctgcgtggc tcccagcggc gaacgtgtca ggaaggtggc 1140
tcttggagcg ggacggagcc ttcctgccaa gactccttca tgtacgacac ccctcaagag 1200
gtggccgaag ctttcctgtc ttccctgaca gagaccatag aaggagtcga tgctgaggat 1260
gggcacggcc caggggaaca acagaagcgg aagatcgtcc tggacccttc aggctccatg 1320
aacatctacc tggtgctaga tggatcagac agcattgggg ccagcaactt cacaggagcc 1380
aaaaagtgtc tagtcaactt aattgagaag gtggcaagtt atggtgtgaa gccaagatat 1440
ggtctagtga catatgccac ataccccaaa atttgggtca aagtgtctga agcagacagc 1500
agtaatgcag actgggtcac gaagcagctc aatgaaatca attatgaaga ccacaagttg 1560
aagtcaggga ctaacaccaa gaaggccctc caggcagtgt acagcatgat gagctggcca 1620
gatgacgtcc ctcctgaagg ctggaaccgc acccgccatg tcatcatcct catgactgat 1680
ggattgcaca acatgggcgg ggacccaatt actgtcattg atgagatccg ggacttgcta 1740
tacattggca aggatcgcaa aaacccaagg gaggattatc tggatgtcta tgtgtttggg 1800
gtcgggcctt tggtgaacca agtgaacatc aatgctttgg cttccaagaa agacaatgag 1860
caacatgtgt tcaaagtcaa ggatatggaa aacctggaag atgttttcta ccaaatgatc 1920
gatgaaagcc agtctctgag tctctgtggc atggtttggg aacacaggaa gggtaccgat 1980
taccacaagc aaccatggca ggccaagatc tcagtcattc gcccttcaaa gggacacgag 2040

agctgtatgg gggctgtggt gtctgagtac tttgtgctga cagcagcaca ttgtttcact 2100

gtggatgaca aggaacactc aatcaaggtc agcgtagagg aagagctgct ccctgcacag 2160

gatatcaaag ctctgtttgt gtctgaggag gagaaaaagc tgactcggaa ggaggtctac 2220

atcaagaatg gggataagaa aggcagctgt gagagagatg ctcaatatgc cccaggctat 2280

gacaaagtca aggacatctc agaggtggtc acccctcggt tcctttgtac tggaggagtg 2340

agtccctatg ctgaccccaa tacttgcaga ggtgattctg gcggcccctt gatagttcac 2400

aagagaagtc gtttcattca agttggtgta atcagctggg gagtagtgga tgtctgcaaa 2460

aaccagaagc ggcaaaagca ggtacctgct cacgcccgag actttcacat caacctcttt 2520

caagtgctgc cctggctgaa ggagaaactc caagatgagg atttgggttt tctataaggg 2580

gtttcctgct ggacaggggc gtgggattga attaaaacag ctgcgacaac a 2631

<210> 87
<211> 1957
<212> DNA
<213> Homo sapiens

<400> 87

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240

ctgcagtttc tgtttccttg actggcagct cagcggggcc ctcccgcttg gatgttccgg 300

gaaagtgatg tgggtaggac aggcggggcg agccgcaggt gccagaacac agattgtata 360

aaaggctggg ggctggtggg gagcagggga agggaatgtg accaggtcta ggtctggagt 420

ttcagcttgg acactgagcc aagcagacaa gcaaagcaag ccaggacaca ccatcctgcc 480

ccaggcccag cttctctcct gccttccaac gccatgggga gcaatctcag cccccaactc 540

tgcctgatgc cctttatctt gggcctcttg tctggaggtg tgaccaccac tccatggtct 600

ttggcccggc cccagggatc ctgctctctg gagggggtag agatcaaagg cggctccttc 660

cgacttctcc aagagggcca ggcactggag tacgtgtgtc cttctggctt ctacccgtac 720

cctgtgcaga cacgtacctg cagatctacg gggtcctgga gcaccctgaa gactcaagac 780

caaaagactg tcaggaaggc agagtgcaga gcaatccact gtccaagacc acacgacttc 840

gagaacgggg aatactggcc ccggtctccc tactacaatg tgagtgatga gatctctttc 900

cactgctatg acggttacac tctccggggc tctgccaatc gcacctgcca agtgaatggc 960

cggtggagtg ggcagacagc gatctgtgac aacggagcgg ggtactgctc caacccgggc 1020

atccccattg gcacaaggaa ggtgggcagc cagtaccgcc ttgaagacag cgtcacctac 1080

cactgcagcc gggggcttac cctgcgtggc tcccagcggc gaacgtgtca ggaaggtggc 1140

tcttggagcg ggacggagcc ttcctgccaa gactccttca tgtacgacac ccctcaagag 1200

gtggccgaag ctttcctgtc ttccctgaca gagaccatag aaggagtcga tgctgaggat 1260

gggcacggcc caggggaaca acagaagcgg aagatcgtcc tggacccttc aggctccatg 1320

aacatctacc tggtgctaga tggatcagac agcattgggg ccagcaactt cacaggagcc 1380

aaaaagtgtc tagtcaactt aattgagaag gtggcaagtt atggtgtgaa gccaagatat 1440

ggtctagtga catatgccac ataccccaaa atttgggtca aagtgtctga agcagacagc 1500

agtaatgcag actgggtcac gaagcagctc aatgaaatca attatgaaga ccacaagttg 1560

aagtcaggga ctaacaccaa gaaggccctc caggcagtgt acagcatgat gagctggcca 1620

gatgacgtcc ctcctgaagg ctggaaccgc acccgccatg tcatcatcct catgactgat 1680

ggattgcaca acatgggcgg ggacccaatt actgtcattg atgagatccg ggacttgcta 1740

tacattggca aggatcgcaa aaacccaagg gaggattatc tggatgtcta tgtgtttggg 1800

gtcgggcctt tggtgaacca agtgaacatc aatgctttgg cttccaagaa agacaatgag 1860

caacatgtgt tcaaagtcaa ggatatggaa aacctggaag atgttttcta ccaaatgatc 1920

ggtagggaga tacaagggaa taaagaacac aactctc 1957

<210> 88
<211> 2238
<212> DNA
<213> Homo sapiens

<400> 88

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240

ctgcagtttc tgtttccttg actggcagct cagcggggcc ctcccgcttg gatgttccgg 300

gaaagtgatg tgggtaggac aggcggggcg agccgcaggt gccagaacac agattgtata 360

aaaggctggg ggctggtggg gagcagggga agggaatgtg accaggtcta ggtctggagt 420

ttcagcttgg acactgagcc aagcagacaa gcaaagcaag ccaggacaca ccatcctgcc 480

ccaggcccag cttctctcct gccttccaac gccatgggga gcaatctcag cccccaactc 540

tgcctgatgc cctttatctt gggcctcttg tctggaggtg tgaccaccac tccatggtct 600

ttggcccggc cccagggatc ctgctctctg gagggggtag agatcaaagg cggctccttc 660

cgacttctcc aagagggcca ggcactggag tacgtgtgtc cttctggctt ctacccgtac 720

cctgtgcaga cacgtacctg cagatctacg gggtcctgga gcaccctgaa gactcaagac 780

caaaagactg tcaggaaggc agagtgcaga gcaatccact gtccaagacc acacgacttc 840

gagaacgggg aatactggcc ccggtctccc tactacaatg tgagtgatga gatctctttc 900

cactgctatg acggttacac tctccggggc tctgccaatc gcacctgcca agtgaatggc 960

cggtggagtg ggcagacagc gatctgtgac aacggagcgg ggtactgctc caacccgggc 1020

atccccattg gcacaaggaa ggtgggcagc cagtaccgcc ttgaagacag cgtcacctac 1080

cactgcagcc gggggcttac cctgcgtggc tcccagcggc gaacgtgtca ggaaggtggc 1140

tcttggagcg ggacggagcc ttcctgccaa gactccttca tgtacgacac ccctcaagag 1200

gtggccgaag ctttcctgtc ttccctgaca gagaccatag aaggagtcga tgctgaggat 1260

gggcacggcc caggggaaca acagaagcgg aagatcgtcc tggacccttc aggctccatg 1320

aacatctacc tggtgctaga tggatcagac agcattgggg ccagcaactt cacaggagcc 1380

aaaaagtgtc tagtcaactt aattgagaag gtggcaagtt atggtgtgaa gccaagatat 1440

ggtctagtga catatgccac ataccccaaa atttgggtca aagtgtctga agcagacagc 1500

agtaatgcag actgggtcac gaagcagctc aatgaaatca attatgaaga ccacaagttg 1560

aagtcaggga ctaacaccaa gaaggccctc caggcagtgt acagcatgat gagctggcca 1620

gatgacgtcc ctcctgaagg ctggaaccgc acccgccatg tcatcatcct catgactgat 1680

ggtcagaagg gacctctctc ctgtcccagc ctccccacct tctcagacca gcatgtggcc 1740

cttaagtcca cttgtaacac tatacccatg gttggggccc tgaatgtgac tcatagctgg 1800

ctgttcatct ctcctgtgac ccttcataag gaattcttcc taagccctgt gatcaactat 1860

ctctaaccct tcctcaactt gctcaccctg ccatgtgtat ccctgccttt agccagttta 1920

tcttccttat ctcctaccct catggtcctg tctcttctgc aggattgcac aacatgggcg 1980

gggacccaat tactgtcatt gatgagatcc gggacttgct atacattggc aaggatcgca 2040

aaaacccaag ggaggattat ctggatgtct atgtgtttgg ggtcgggcct ttggtgaacc 2100

aagtgaacat caatgctttg gcttccaaga aagacaatga gcaacatgtg ttcaaagtca 2160

aggatatgga aaacctggaa gatgttttct accaaatgat cggtagggag atacaaggga 2220

ataaagaaca caactctc 2238

<210> 89
<211> 577
<212> DNA
<213> Homo sapiens

<400> 89

ggccttgggg gagggggagg ccagaatgac tccaagagct acaggaaggc aggtcagaga 60

ccccactgga caaacagtgg ctggactctg caccataaca cacaatcaac aggggagtga 120

gctggatcct tatttctggt ccctaagtgg gtggtttggg cttactgggg aggagctaag 180

gccggagagg aggtactgaa ggggagagtc ctggaccttt ggcagcaaag ggtgggactt 240

ctgcagtttc tgtttccttg actggcagct cagcggggcc ctcccgcttg gatgttccgg 300

gaaagtgatg tgggtaggac aggcggggcg agccgcaggt gccagaacac agattgtata 360

aaaggctggg ggctggtggg gagcagggga agggaatgtg accaggtcta ggtctggagt 420

ttcagcttgg acactgagcc aagcagacaa gcaaagcaag ccaggacaca ccatcctgcc 480

ccaggcccag cttctctcct gccttccaac gccatgggga gcaatctcag cccccaactc 540

tgcctgatgc cctttatctt gggcctcttg tctggag 577

<210> 90
<211> 156
<212> DNA
<213> Homo sapiens

<400> 90

gtgtgaccac cactccatgg tctttggccc ggccccaggg atcctgctct ctggaggggg 60

tagagatcaa aggcggctcc ttccgacttc tccaagaggg ccaggcactg gagtacgtgt 120

gtccttctgg cttctacccg taccctgtgc agacac 156

<210> 91
<211> 400
<212> DNA
<213> Homo sapiens

<400> 91

gtttgagggc aatgagtgtg ggcagtggcc taaggcagaa acagggcagg cggcagcaag 60

gtcaggacta ggatgagact aggcagggtg acaaggtggg ctgaccggga gtaggagcag 120

ttttagggtg gcaggcggaa agggggcaag aaaaagcgga gttaaccctt actaagcatt 180

taccctgggc ttccaggcag ccctggaagt caagagaaca ctcagaaatg gggagggaga 240

agcagtggaa atccatatgg gttgaggagt aggtaagatg ctgcttctgc gggactggga 300

atgcgctgtt tctcagtgac atggtctccg agaccaggag ggatacacct aaggcagcct 360

ttccctcttg atgacttcta cttgtccccc cttctcaaag 400

<210> 92
<211> 186
<212> DNA
<213> Homo sapiens

<400> 92

caatccactg tccaagacca cacgacttcg agaacgggga atactggccc cggtctccct 60

actacaatgt gagtgatgag atctctttcc actgctatga cggttacact ctccggggct 120

ctgccaatcg cacctgccaa gtgaatggcc ggtggagtgg gcagacagcg atctgtgaca 180

acggag 186

<210> 93
<211> 155
<212> DNA
<213> Homo sapiens

<400> 93

gtgagaagca tcccctcccc ctacattgct gtctccctga cggcgcccag cccgaggagt 60

gggcactcgg ctccggacac tgtaactctt gctctctacc ttgctcacgg ggcctcaggc 120

ttcagtgctt acctcgatgt ctcatacctc tgcag 155

<210> 94
<211> 174
<212> DNA
<213> Homo sapiens

<400> 94

cggggtactg ctccaacccg ggcatcccca ttggcacaag gaaggtgggc agccagtacc 60

gccttgaaga cagcgtcacc taccactgca gccgggggct taccctgcgt ggctcccagc 120

ggcgaacgtg tcaggaaggt ggctcttgga gcgggacgga gccttcctgc caag 174

<210> 95
<211> 132
<212> DNA
<213> Homo sapiens

<400> 95

accacaagtt gaagtcaggg actaacacca agaaggccct ccaggcagtg tacagcatga 60

tgagctggcc agatgacgtc cctcctgaag gctggaaccg cacccgccat gtcatcatcc 120

tcatgactga tg 132

<210> 96
<211> 281
<212> DNA
<213> Homo sapiens

<400> 96

gtcagaaggg acctctctcc tgtcccagcc tccccacctt ctcagaccag catgtggccc 60

ttaagtccac ttgtaacact atacccatgg ttggggccct gaatgtgact catagctggc 120

tgttcatctc tcctgtgacc cttcataagg aattcttcct aagccctgtg atcaactatc 180

tctaaccctt cctcaacttg ctcaccctgc catgtgtatc cctgccttta gccagtttat 240

cttccttatc tcctaccctc atggtcctgt ctcttctgca g 281

<210> 97
<211> 145
<212> DNA
<213> Homo sapiens

<400> 97

gtagggagat acaagggaat aaagaacaca actctcctca ggttcccctg aagtaattca 60

ttcttcctct acacctgaag ctctagttgc ctggaaagcc ttcttcattc ctccttctct 120

acctcagtgt cactattctt gtttc 145

<210> 98
<211> 154
<212> DNA
<213> Homo sapiens

<400> 98

gaggggagaa gcgggacctg gagatagaag tagtcctatt tcaccccaac tacaacatta 60

atgggaaaaa agaagcagga attcctgaat tttatgacta tgacgttgcc ctgatcaagc 120

tcaagaataa gctgaaatat ggccagacta tcag 154

<210> 99
<211> 270
<212> DNA
<213> Homo sapiens

<400> 99

gtgagtcctc cctttcctat ctggggagat gccaagtggt cagcatgggc cccaaagcag 60

gaaagctcaa tgcatgtggc tagtaattcg aggtaggcag agcctgcctc accttaggac 120

cgcatgtctt gcctgcgtgt gtcaagaacg aggctgagct gggtccctag tctgattcct 180

ttaggtcagc taagacgcaa gcaggaacag ccatgcttcc aggattagga attctactga 240

atgatccatg gcaccccact gcctctgcag 270

<210> 100
<211> 78
<212> DNA
<213> Homo sapiens

<400> 100

gtacctgcag atctacgggg tcctggagca ccctgaagac tcaagaccaa aagactgtca 60

ggaaggcaga gtgcagag 78

<210> 101
<211> 68
<212> DNA
<213> Homo sapiens

<400> 101

actccttcat gtacgacacc cctcaagagg tggccgaagc tttcctgtct tccctgacag 60

agaccata 68

<210> 102
<211> 34
<212> DNA
<213> Homo sapiens

<400> 102

gaaggagtcg atgctgagga tgggcacggc ccag 34

<210> 103
<211> 84
<212> DNA
<213> Homo sapiens

<400> 103

gggaacaaca gaagcggaag atcgtcctgg acccttcagg ctccatgaac atctacctgg 60

tgctagatgg atcagacagc attg 84

<210> 104
<211> 18
<212> DNA
<213> Homo sapiens

<400> 104

gggccagcaa cttcacag 18

<210> 105
<211> 35
<212> DNA
<213> Homo sapiens

<400> 105

gagccaaaaa gtgtctagtc aacttaattg agaag 35

<210> 106
<211> 113
<212> DNA
<213> Homo sapiens

<400> 106

gtggaatcct cctatccctg aactcggggg aatggaatct cgctgatctt ccaggactag 60

ctccctgatc attccagccc ctctgaacaa cagggcccca ggaaaatctc cag 113

<210> 107
<211> 56
<212> DNA
<213> Homo sapiens

<400> 107

gtggcaagtt atggtgtgaa gccaagatat ggtctagtga catatgccac ataccc 56

<210> 108
<211> 30
<212> DNA
<213> Homo sapiens

<400> 108

caaaatttgg gtcaaagtgt ctgaagcaga 30

<210> 109
<211> 24
<212> DNA
<213> Homo sapiens

<400> 109

cagcagtaat gcagactggg tcac 24

<210> 110
<211> 7
<212> DNA
<213> Homo sapiens

<400> 110

gaagcag 7

<210> 111
<211> 22
<212> DNA
<213> Homo sapiens

<400> 111

ctcaatgaaa tcaattatga ag 22

<210> 112
<211> 83
<212> DNA
<213> Homo sapiens

<400> 112

gattgcacaa catgggcggg gacccaatta ctgtcattga tgagatccgg gacttgctat 60

acattggcaa ggatcgcaaa aac 83

<210> 113
<211> 19
<212> DNA
<213> Homo sapiens

<400> 113

ccaagggagg attatctgg 19

<210> 114
<211> 35
<212> DNA
<213> Homo sapiens

<400> 114

atgtctatgt gtttggggtc gggcctttgg tgaac 35

<210> 115
<211> 9
<212> DNA
<213> Homo sapiens

<400> 115

caagtgaac 9

<210> 116
<211> 94
<212> DNA
<213> Homo sapiens

<400> 116

atcaatgctt tggcttccaa gaaagacaat gagcaacatg tgttcaaagt caaggatatg 60

gaaaacctgg aagatgtttt ctaccaaatg atcg 94

<210> 117
<211> 98
<212> DNA
<213> Homo sapiens

<400> 117

atgaaagcca gtctctgagt ctctgtggca tggtttggga acacaggaag ggtaccgatt 60

accacaagca accatggcag gccaagatct cagtcatt 98

<210> 118
<211> 64
<212> DNA
<213> Homo sapiens

<400> 118

cgcccttcaa agggacacga gagctgtatg ggggctgtgg tgtctgagta ctttgtgctg 60

acag 64

<210> 119
<211> 54
<212> DNA
<213> Homo sapiens

<400> 119

cagcacattg tttcactgtg gatgacaagg aacactcaat caaggtcagc gtag 54

<210> 120
<211> 77
<212> DNA
<213> Homo sapiens

<400> 120

gcccatttgt ctcccctgca ccgagggaac aactcgagct ttgaggcttc ctccaactac 60

cacttgccag caacaaa 77

<210> 121
<211> 2
<212> DNA
<213> Homo sapiens

<400> 121

ag 2

<210> 122
<211> 31
<212> DNA
<213> Homo sapiens

<400> 122

gaagagctgc tccctgcaca ggatatcaaa g 31

<210> 123
<211> 68
<212> DNA
<213> Homo sapiens

<400> 123

ctctgtttgt gtctgaggag gagaaaaagc tgactcggaa ggaggtctac atcaagaatg 60

gggataag 68

<210> 124
<211> 96
<212> DNA
<213> Homo sapiens

<400> 124

gtgagaaacg ggcatcctaa ggaggcactc taggccccaa tccttcctaa gccacttctg 60

ttcattactt ctccatgctt cccacctccc ctacag 96

<210> 125
<211> 97
<212> DNA
<213> Homo sapiens

<400> 125

aaaggcagct gtgagagaga tgctcaatat gccccaggct atgacaaagt caaggacatc 60

tcagaggtgg tcacccctcg gttcctttgt actggag 97

<210> 126
<211> 36
<212> DNA
<213> Homo sapiens

<400> 126

gagtgagtcc ctatgctgac cccaatactt gcagag 36

<210> 127
<211> 50
<212> DNA
<213> Homo sapiens

<400> 127

gtgattctgg cggccccttg atagttcaca agagaagtcg tttcattcaa 50

<210> 128
<211> 50
<212> DNA
<213> Homo sapiens

<400> 128

gttggtgtaa tcagctgggg agtagtggat gtctgcaaaa accagaagcg 50

<210> 129
<211> 53
<212> DNA
<213> Homo sapiens

<400> 129

gcaaaagcag gtacctgctc acgcccgaga ctttcacatc aacctctttc aag 53

<210> 130
<211> 107
<212> DNA

<213>  Homo sapiens

<400> 130

tgctgccctg gctgaaggag aaactccaag atgaggattt gggttttcta taaggggttt     60

cctgctggac aggggcgtgg gattgaatta aaacagctgc gacaaca                  107

<210>  131
<211>  764
<212>  PRT
<213>  Homo sapiens

<400> 131

```
Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190
```

```
Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
                405                 410                 415

Asn Pro Arg Glu Asp Tyr Leu Asp Val Tyr Val Phe Gly Val Gly Pro
            420                 425                 430

Leu Val Asn Gln Val Asn Ile Asn Ala Leu Ala Ser Lys Lys Asp Asn
        435                 440                 445
```

```
Glu Gln His Val Phe Lys Val Lys Asp Met Glu Asn Leu Glu Asp Val
    450                 455                 460

Phe Tyr Gln Met Ile Asp Glu Ser Gln Ser Leu Ser Leu Cys Gly Met
465                 470                 475                 480

Val Trp Glu His Arg Lys Gly Thr Asp Tyr His Lys Gln Pro Trp Gln
                485                 490                 495

Ala Lys Ile Ser Val Ile Arg Pro Ser Lys Gly His Glu Ser Cys Met
            500                 505                 510

Gly Ala Val Val Ser Glu Tyr Phe Val Leu Thr Ala Ala His Cys Phe
        515                 520                 525

Thr Val Asp Asp Lys Glu His Ser Ile Lys Val Ser Val Gly Gly Glu
    530                 535                 540

Lys Arg Asp Leu Glu Ile Glu Val Val Leu Phe His Pro Asn Tyr Asn
545                 550                 555                 560

Ile Asn Gly Lys Lys Glu Ala Gly Ile Pro Glu Phe Tyr Asp Tyr Asp
                565                 570                 575

Val Ala Leu Ile Lys Leu Lys Asn Lys Leu Lys Tyr Gly Gln Thr Ile
            580                 585                 590

Arg Pro Ile Cys Leu Pro Cys Thr Glu Gly Thr Thr Arg Ala Leu Arg
        595                 600                 605

Leu Pro Pro Thr Thr Thr Cys Gln Gln Gln Lys Glu Glu Leu Leu Pro
    610                 615                 620

Ala Gln Asp Ile Lys Ala Leu Phe Val Ser Glu Glu Glu Lys Lys Leu
625                 630                 635                 640

Thr Arg Lys Glu Val Tyr Ile Lys Asn Gly Asp Lys Lys Gly Ser Cys
                645                 650                 655

Glu Arg Asp Ala Gln Tyr Ala Pro Gly Tyr Asp Lys Val Lys Asp Ile
            660                 665                 670

Ser Glu Val Val Thr Pro Arg Phe Leu Cys Thr Gly Gly Val Ser Pro
        675                 680                 685

Tyr Ala Asp Pro Asn Thr Cys Arg Gly Asp Ser Gly Gly Pro Leu Ile
```

```
    690                 695                 700

Val His Lys Arg Ser Arg Phe Ile Gln Val Gly Val Ile Ser Trp Gly
705                 710                 715                 720

Val Val Asp Val Cys Lys Asn Gln Lys Arg Gln Lys Gln Val Pro Ala
                725                 730                 735

His Ala Arg Asp Phe His Ile Asn Leu Phe Gln Val Leu Pro Trp Leu
            740                 745                 750

Lys Glu Lys Leu Gln Asp Glu Asp Leu Gly Phe Leu
        755                 760
```

<210>  132
<211>  621
<212>  PRT
<213>  Homo sapiens

<400> 132

```
Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140
```

```
Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190

Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400
```

Val Ile Asp Glu Ile Arg Asp Leu Leu Tyr Ile Gly Lys Asp Arg Lys
405 410 415

Asn Pro Arg Glu Asp Tyr Leu Asp Val Tyr Val Phe Gly Val Gly Pro
420 425 430

Leu Val Asn Gln Val Asn Ile Asn Ala Leu Ala Ser Lys Lys Asp Asn
435 440 445

Glu Gln His Val Phe Lys Val Lys Asp Met Glu Asn Leu Glu Asp Val
450 455 460

Phe Tyr Gln Met Ile Asp Glu Ser Gln Ser Leu Ser Leu Cys Gly Met
465 470 475 480

Val Trp Glu His Arg Lys Gly Thr Asp Tyr His Lys Gln Pro Trp Gln
485 490 495

Ala Lys Ile Ser Val Ile Arg Pro Ser Lys Gly His Glu Ser Cys Met
500 505 510

Gly Ala Val Val Ser Glu Tyr Phe Val Leu Thr Ala Ala His Cys Phe
515 520 525

Thr Val Asp Asp Lys Glu His Ser Ile Lys Val Ser Val Gly Lys Asp
530 535 540

Ala Thr Glu Gly Pro Gly Leu His Leu Cys Ser Pro Gly Asn Thr Ser
545 550 555 560

His Phe Leu Gln Ile Leu His Ser Thr His Pro Gln Cys Ser Pro Ile
565 570 575

Pro Cys Thr Pro Asp Gln Ser Gly Met Gly Glu Asp Val Lys Leu Gly
580 585 590

Met Thr Arg Gly Gln Arg Gln Glu Ala Ala His Lys Glu Val Val Pro
595 600 605

Thr Leu Leu Leu Gln Glu Gly Arg Ser Gly Thr Trp Arg
610 615 620

<210> 133
<211> 764
<212> PRT
<213> Homo sapiens

<400> 133

Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Gln
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190

Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln

245 250 255

Gln Lys Arg Lys Ile Val Leu Asp Pro Ser Gly Ser Met Asn Ile Tyr
260 265 270

Leu Val Leu Asp Gly Ser Asp Ser Ile Gly Ala Ser Asn Phe Thr Gly
275 280 285

Ala Lys Lys Cys Leu Val Asn Leu Ile Glu Lys Val Ala Ser Tyr Gly
290 295 300

Val Lys Pro Arg Tyr Gly Leu Val Thr Tyr Ala Thr Tyr Pro Lys Ile
305 310 315 320

Trp Val Lys Val Ser Glu Ala Asp Ser Ser Asn Ala Asp Trp Val Thr
325 330 335

Lys Gln Leu Asn Glu Ile Asn Tyr Glu Asp His Lys Leu Lys Ser Gly
340 345 350

Thr Asn Thr Lys Lys Ala Leu Gln Ala Val Tyr Ser Met Met Ser Trp
355 360 365

Pro Asp Asp Val Pro Pro Glu Gly Trp Asn Arg Thr Arg His Val Ile
370 375 380

Ile Leu Met Thr Asp Gly Leu His Asn Met Gly Gly Asp Pro Ile Thr
385 390 395 400

Val Ile Asp Glu Ile Arg Asp Leu Leu Tyr Ile Gly Lys Asp Arg Lys
405 410 415

Asn Pro Arg Glu Asp Tyr Leu Asp Val Tyr Val Phe Gly Val Gly Pro
420 425 430

Leu Val Asn Gln Val Asn Ile Asn Ala Leu Ala Ser Lys Lys Asp Asn
435 440 445

Glu Gln His Val Phe Lys Val Lys Asp Met Glu Asn Leu Glu Asp Val
450 455 460

Phe Tyr Gln Met Ile Asp Glu Ser Gln Ser Leu Ser Leu Cys Gly Met
465 470 475 480

Val Trp Glu His Arg Lys Gly Thr Asp Tyr His Lys Gln Pro Trp Gln
485 490 495

```
Ala Lys Ile Ser Val Ile Arg Pro Ser Lys Gly His Glu Ser Cys Met
            500                 505                 510

Gly Ala Val Val Ser Glu Tyr Phe Val Leu Thr Ala Ala His Cys Phe
        515                 520                 525

Thr Val Asp Asp Lys Glu His Ser Ile Lys Val Ser Val Gly Gly Glu
    530                 535                 540

Lys Arg Asp Leu Glu Ile Glu Val Val Leu Phe His Pro Asn Tyr Asn
545                 550                 555                 560

Ile Asn Gly Lys Lys Glu Ala Gly Ile Pro Glu Phe Tyr Asp Tyr Asp
                565                 570                 575

Val Ala Leu Ile Lys Leu Lys Asn Lys Leu Lys Tyr Gly Gln Thr Ile
            580                 585                 590

Arg Pro Ile Cys Leu Pro Cys Thr Glu Gly Thr Thr Arg Ala Leu Arg
        595                 600                 605

Leu Pro Pro Thr Thr Thr Cys Gln Gln Gln Lys Glu Glu Leu Leu Pro
    610                 615                 620

Ala Gln Asp Ile Lys Ala Leu Phe Val Ser Glu Glu Glu Lys Lys Leu
625                 630                 635                 640

Thr Arg Lys Glu Val Tyr Ile Lys Asn Gly Asp Lys Lys Gly Ser Cys
                645                 650                 655

Glu Arg Asp Ala Gln Tyr Ala Pro Gly Tyr Asp Lys Val Lys Asp Ile
            660                 665                 670

Ser Glu Val Val Thr Pro Arg Phe Leu Cys Thr Gly Gly Val Ser Pro
        675                 680                 685

Tyr Ala Asp Pro Asn Thr Cys Arg Gly Asp Ser Gly Gly Pro Leu Ile
    690                 695                 700

Val His Lys Arg Ser Arg Phe Ile Gln Val Gly Val Ile Ser Trp Gly
705                 710                 715                 720

Val Val Asp Val Cys Lys Asn Gln Lys Arg Gln Lys Gln Val Pro Ala
                725                 730                 735

His Ala Arg Asp Phe His Ile Asn Leu Phe Gln Val Leu Pro Trp Leu
            740                 745                 750
```

Lys Glu Lys Leu Gln Asp Glu Asp Leu Gly Phe Leu
755 760

<210> 134
<211> 740
<212> PRT
<213> Homo sapiens

<400> 134

Met Gly Ser Asn Leu Ser Pro Gln Leu Cys Leu Met Pro Phe Ile Leu
1 5 10 15

Gly Leu Leu Ser Gly Gly Val Thr Thr Thr Pro Trp Ser Leu Ala Arg
20 25 30

Pro Gln Gly Ser Cys Ser Leu Glu Gly Val Glu Ile Lys Gly Gly Ser
35 40 45

Phe Arg Leu Leu Gln Glu Gly Gln Ala Leu Glu Tyr Val Cys Pro Ser
50 55 60

Gly Phe Tyr Pro Tyr Pro Val Gln Thr Arg Thr Cys Arg Ser Thr Gly
65 70 75 80

Ser Trp Ser Thr Leu Lys Thr Gln Asp Gln Lys Thr Val Arg Lys Ala
85 90 95

Glu Cys Arg Ala Ile His Cys Pro Arg Pro His Asp Phe Glu Asn Gly
100 105 110

Glu Tyr Trp Pro Arg Ser Pro Tyr Tyr Asn Val Ser Asp Glu Ile Ser
115 120 125

Phe His Cys Tyr Asp Gly Tyr Thr Leu Arg Gly Ser Ala Asn Arg Thr
130 135 140

Cys Gln Val Asn Gly Arg Trp Ser Gly Gln Thr Ala Ile Cys Asp Asn
145 150 155 160

Gly Ala Gly Tyr Cys Ser Asn Pro Gly Ile Pro Ile Gly Thr Arg Lys
165 170 175

Val Gly Ser Gln Tyr Arg Leu Glu Asp Ser Val Thr Tyr His Cys Ser
180 185 190

Arg Gly Leu Thr Leu Arg Gly Ser Gln Arg Arg Thr Cys Gln Glu Gly
195 200 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
                405                 410                 415

Asn Pro Arg Glu Asp Tyr Leu Asp Val Tyr Val Phe Gly Val Gly Pro
            420                 425                 430

Leu Val Asn Gln Val Asn Ile Asn Ala Leu Ala Ser Lys Lys Asp Asn
        435                 440                 445

Glu Gln His Val Phe Lys Val Lys Asp Met Glu Asn Leu Glu Asp Val

```
    450                 455                 460

Phe Tyr Gln Met Ile Asp Glu Ser Gln Ser Leu Ser Leu Cys Gly Met
465                 470                 475                 480

Val Trp Glu His Arg Lys Gly Thr Asp Tyr His Lys Gln Pro Trp Gln
                485                 490                 495

Ala Lys Ile Ser Val Ile Arg Pro Ser Lys Gly His Glu Ser Cys Met
            500                 505                 510

Gly Ala Val Val Ser Glu Tyr Phe Val Leu Thr Ala Ala His Cys Phe
        515                 520                 525

Thr Val Asp Asp Lys Glu His Ser Ile Lys Val Ser Val Gly Gly Glu
    530                 535                 540

Lys Arg Asp Leu Glu Ile Glu Val Val Leu Phe His Pro Asn Tyr Asn
545                 550                 555                 560

Ile Asn Gly Lys Lys Glu Ala Gly Ile Pro Glu Phe Tyr Asp Tyr Asp
                565                 570                 575

Val Ala Leu Ile Lys Leu Lys Asn Lys Leu Lys Tyr Gly Gln Thr Ile
            580                 585                 590

Arg Pro Ile Cys Leu Pro Cys Thr Glu Gly Thr Thr Arg Ala Leu Arg
        595                 600                 605

Leu Pro Pro Thr Thr Thr Cys Gln Gln Gln Lys Glu Glu Leu Leu Pro
    610                 615                 620

Ala Gln Asp Ile Lys Ala Leu Phe Val Ser Glu Glu Glu Lys Lys Leu
625                 630                 635                 640

Thr Arg Lys Glu Val Tyr Ile Lys Asn Gly Asp Lys Lys Gly Ser Cys
                645                 650                 655

Glu Arg Asp Ala Gln Tyr Ala Pro Gly Tyr Asp Lys Val Lys Asp Ile
            660                 665                 670

Ser Glu Val Val Thr Pro Arg Phe Leu Cys Thr Gly Gly Val Ser Pro
        675                 680                 685

Tyr Ala Asp Pro Asn Thr Cys Arg Gly Asp Ser Gly Gly Pro Leu Ile
    690                 695                 700
```

```
Val His Lys Arg Ser Arg Phe Ile Gln Val Gly Val Ile Ser Trp Gly
705                 710                 715                 720

Val Val Asp Val Cys Lys Asn Gln Lys Arg Ala Ala Leu Ala Glu Gly
                725                 730                 735

Glu Thr Pro Arg
            740


<210>  135
<211>  450
<212>  PRT
<213>  Homo sapiens

<400> 135

Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175
```

```
Val Gly Ser Gln Tyr Arg Leu Glu Asp Ser Val Thr Tyr His Cys Ser
          180                 185                 190

Arg Gly Leu Thr Leu Arg Gly Ser Gln Arg Arg Thr Cys Gln Glu Gly
      195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Gln Lys Gly Pro Leu Ser Cys Pro Ser Leu
385                 390                 395                 400

Pro Thr Phe Ser Asp Gln His Val Ala Leu Lys Ser Thr Cys Asn Thr
                405                 410                 415

Ile Pro Met Val Gly Ala Leu Asn Val Thr His Ser Trp Leu Phe Ile
            420                 425                 430
```

```
Ser Pro Val Thr Leu His Lys Glu Phe Phe Leu Ser Pro Val Ile Asn
        435                 440                 445

Tyr Leu
    450


<210>  136
<211>  744
<212>  PRT
<213>  Homo sapiens

<400> 136

Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
```

```
            180                 185                 190

Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
                405                 410                 415

Asn Pro Arg Glu Asp Tyr Leu Asp Val Tyr Val Phe Gly Val Gly Pro
            420                 425                 430
```

```
Leu Val Asn Gln Val Asn Ile Asn Ala Leu Ala Ser Lys Lys Asp Asn
          435                 440                 445

Glu Gln His Val Phe Lys Val Lys Asp Met Glu Asn Leu Glu Asp Val
    450                 455                 460

Phe Tyr Gln Met Ile Asp Glu Ser Gln Ser Leu Ser Leu Cys Gly Met
465                 470                 475                 480

Val Trp Glu His Arg Lys Gly Thr Asp Tyr His Lys Gln Pro Trp Gln
                485                 490                 495

Ala Lys Ile Ser Val Ile Arg Pro Ser Lys Gly His Glu Ser Cys Met
            500                 505                 510

Gly Ala Val Val Ser Glu Tyr Phe Val Leu Thr Ala Ala His Cys Phe
        515                 520                 525

Thr Val Asp Asp Lys Glu His Ser Ile Lys Val Ser Val Gly Gly Glu
    530                 535                 540

Lys Arg Asp Leu Glu Ile Glu Val Val Leu Phe His Pro Asn Tyr Asn
545                 550                 555                 560

Ile Asn Gly Lys Lys Glu Ala Gly Ile Pro Glu Phe Tyr Asp Tyr Asp
                565                 570                 575

Val Ala Leu Ile Lys Leu Lys Asn Lys Leu Lys Tyr Gly Gln Thr Ile
            580                 585                 590

Arg Pro Ile Cys Leu Pro Cys Thr Glu Gly Thr Thr Arg Ala Leu Arg
        595                 600                 605

Leu Pro Pro Thr Thr Thr Cys Gln Gln Gln Lys Glu Glu Leu Leu Pro
    610                 615                 620

Ala Gln Asp Ile Lys Ala Leu Phe Val Ser Glu Glu Glu Lys Lys Leu
625                 630                 635                 640

Thr Arg Lys Glu Val Tyr Ile Lys Asn Gly Asp Lys Lys Gly Ser Cys
                645                 650                 655

Glu Arg Asp Ala Gln Tyr Ala Pro Gly Tyr Asp Lys Val Lys Asp Ile
            660                 665                 670

Ser Glu Val Val Thr Pro Arg Phe Leu Cys Thr Gly Gly Val Ser Pro
        675                 680                 685
```

```
Tyr Ala Asp Pro Asn Thr Cys Arg Gly Asp Ser Gly Gly Pro Leu Ile
    690                 695                 700

Val His Lys Arg Ser Arg Phe Ile Gln Val Ser Pro Pro Phe Pro Ile
705                 710                 715                 720

Trp Gly Asp Ala Lys Trp Ser Ala Trp Ala Pro Lys Gln Glu Ser Ser
                725                 730                 735

Met His Val Ala Ser Asn Ser Arg
            740
```

<210> 137
<211> 633
<212> PRT
<213> Homo sapiens

<400> 137

```
Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160
```

```
Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190

Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
```

405 410 415

Asn Pro Arg Glu Asp Tyr Leu Asp Val Tyr Val Phe Gly Val Gly Pro
420 425 430

Leu Val Asn Gln Val Asn Ile Asn Ala Leu Ala Ser Lys Lys Asp Asn
435 440 445

Glu Gln His Val Phe Lys Val Lys Asp Met Glu Asn Leu Glu Asp Val
450 455 460

Phe Tyr Gln Met Ile Asp Glu Ser Gln Ser Leu Ser Leu Cys Gly Met
465 470 475 480

Val Trp Glu His Arg Lys Gly Thr Asp Tyr His Lys Gln Pro Trp Gln
485 490 495

Ala Lys Ile Ser Val Ile Arg Pro Ser Lys Gly His Glu Ser Cys Met
500 505 510

Gly Ala Val Val Ser Glu Tyr Phe Val Leu Thr Ala Ala His Cys Phe
515 520 525

Thr Val Asp Asp Lys Glu His Ser Ile Lys Val Ser Val Gly Gly Glu
530 535 540

Lys Arg Asp Leu Glu Ile Glu Val Val Leu Phe His Pro Asn Tyr Asn
545 550 555 560

Ile Asn Gly Lys Lys Glu Ala Gly Ile Pro Glu Phe Tyr Asp Tyr Asp
565 570 575

Val Ala Leu Ile Lys Leu Lys Asn Lys Leu Lys Tyr Gly Gln Thr Ile
580 585 590

Arg Pro Ile Cys Leu Pro Cys Thr Glu Gly Thr Thr Arg Ala Leu Arg
595 600 605

Leu Pro Pro Thr Thr Thr Cys Gln Gln Gln Arg Arg Ala Ala Pro Cys
610 615 620

Thr Gly Tyr Gln Ser Ser Val Cys Val
625 630

<210> 138
<211> 608
<212> PRT

<213>  Homo sapiens

<400> 138

```
Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190

Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240
```

```
Thr Ile Glu Gly Val Asp Ala Glu Asp Gly His Gly Pro Gly Glu Gln
            245                 250                 255

Gln Lys Arg Lys Ile Val Leu Asp Pro Ser Gly Ser Met Asn Ile Tyr
        260                 265                 270

Leu Val Leu Asp Gly Ser Asp Ser Ile Gly Ala Ser Asn Phe Thr Gly
    275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
            325                 330                 335

Lys Gln Leu Asn Glu Ile Asn Tyr Glu Asp His Lys Leu Lys Ser Gly
        340                 345                 350

Thr Asn Thr Lys Lys Ala Leu Gln Ala Val Tyr Ser Met Met Ser Trp
    355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
            405                 410                 415

Asn Pro Arg Glu Asp Tyr Leu Asp Val Tyr Val Phe Gly Val Gly Pro
        420                 425                 430

Leu Val Asn Gln Val Asn Ile Asn Ala Leu Ala Ser Lys Lys Asp Asn
    435                 440                 445

Glu Gln His Val Phe Lys Val Lys Asp Met Glu Asn Leu Glu Asp Val
    450                 455                 460

Phe Tyr Gln Met Ile Asp Glu Ser Gln Ser Leu Ser Leu Cys Gly Met
465                 470                 475                 480

Val Trp Glu His Arg Lys Gly Thr Asp Tyr His Lys Gln Pro Trp Gln
            485                 490                 495
```

```
Ala Lys Ile Ser Val Ile Arg Pro Ser Lys Gly His Glu Ser Cys Met
            500                 505                 510

Gly Ala Val Val Ser Glu Tyr Phe Val Leu Thr Ala Ala His Cys Phe
        515                 520                 525

Thr Val Asp Asp Lys Glu His Ser Ile Lys Val Ser Val Gly Gly Glu
    530                 535                 540

Lys Arg Asp Leu Glu Ile Glu Val Val Leu Phe His Pro Asn Tyr Asn
545                 550                 555                 560

Ile Asn Gly Lys Lys Glu Ala Gly Ile Pro Glu Phe Tyr Asp Tyr Asp
                565                 570                 575

Val Ala Leu Ile Lys Leu Lys Asn Lys Leu Lys Tyr Gly Gln Thr Ile
            580                 585                 590

Arg Gly Arg Ala Ala Pro Cys Thr Gly Tyr Gln Ser Ser Val Cys Val
        595                 600                 605


<210>  139
<211>  662
<212>  PRT
<213>  Homo sapiens

<400> 139

Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
```

```
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190

Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350
```

```
Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
                405                 410                 415

Asn Pro Arg Glu Asp Tyr Leu Asp Val Tyr Val Phe Gly Val Gly Pro
            420                 425                 430

Leu Val Asn Gln Val Asn Ile Asn Ala Leu Ala Ser Lys Lys Asp Asn
        435                 440                 445

Glu Gln His Val Phe Lys Val Lys Asp Met Glu Asn Leu Glu Asp Val
    450                 455                 460

Phe Tyr Gln Met Ile Asp Glu Ser Gln Ser Leu Ser Leu Cys Gly Met
465                 470                 475                 480

Val Trp Glu His Arg Lys Gly Thr Asp Tyr His Lys Gln Pro Trp Gln
                485                 490                 495

Ala Lys Ile Ser Val Ile Arg Pro Ser Lys Gly His Glu Ser Cys Met
            500                 505                 510

Gly Ala Val Val Ser Glu Tyr Phe Val Leu Thr Ala Ala His Cys Phe
        515                 520                 525

Thr Val Asp Asp Lys Glu His Ser Ile Lys Val Ser Val Gly Gly Glu
    530                 535                 540

Lys Arg Asp Leu Glu Ile Glu Val Val Leu Phe His Pro Asn Tyr Asn
545                 550                 555                 560

Ile Asn Gly Lys Lys Glu Ala Gly Ile Pro Glu Phe Tyr Asp Tyr Asp
                565                 570                 575

Val Ala Leu Ile Lys Leu Lys Asn Lys Leu Lys Tyr Gly Gln Thr Ile
            580                 585                 590

Arg Pro Ile Cys Leu Pro Cys Thr Glu Gly Thr Thr Arg Ala Leu Arg
        595                 600                 605
```

```
Leu Pro Pro Thr Thr Thr Cys Gln Gln Gln Lys Glu Glu Leu Leu Pro
    610                 615                 620

Ala Gln Asp Ile Lys Ala Leu Phe Val Ser Glu Glu Glu Lys Lys Leu
625                 630                 635                 640

Thr Arg Lys Glu Val Tyr Ile Lys Asn Gly Asp Lys Val Arg Asn Gly
                645                 650                 655

His Pro Lys Glu Ala Leu
            660


<210>  140
<211>  687
<212>  PRT
<213>  Homo sapiens

<400> 140

Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160
```

```
Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190

Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
```

405 410 415

Asn Pro Arg Glu Asp Tyr Leu Asp Val Tyr Val Phe Gly Val Gly Pro
420 425 430

Leu Val Asn Gln Val Asn Ile Asn Ala Leu Ala Ser Lys Lys Asp Asn
435 440 445

Glu Gln His Val Phe Lys Val Lys Asp Met Glu Asn Leu Glu Asp Val
450 455 460

Phe Tyr Gln Met Ile Asp Glu Ser Gln Ser Leu Ser Leu Cys Gly Met
465 470 475 480

Val Trp Glu His Arg Lys Gly Thr Asp Tyr His Lys Gln Pro Trp Gln
485 490 495

Ala Lys Ile Ser Val Ile Arg Pro Ser Lys Gly His Glu Ser Cys Met
500 505 510

Gly Ala Val Val Ser Glu Tyr Phe Val Leu Thr Ala Ala His Cys Phe
515 520 525

Thr Val Asp Asp Lys Glu His Ser Ile Lys Val Ser Val Glu Glu Glu
530 535 540

Leu Leu Pro Ala Gln Asp Ile Lys Ala Leu Phe Val Ser Glu Glu Glu
545 550 555 560

Lys Lys Leu Thr Arg Lys Glu Val Tyr Ile Lys Asn Gly Asp Lys Lys
565 570 575

Gly Ser Cys Glu Arg Asp Ala Gln Tyr Ala Pro Gly Tyr Asp Lys Val
580 585 590

Lys Asp Ile Ser Glu Val Val Thr Pro Arg Phe Leu Cys Thr Gly Gly
595 600 605

Val Ser Pro Tyr Ala Asp Pro Asn Thr Cys Arg Gly Asp Ser Gly Gly
610 615 620

Pro Leu Ile Val His Lys Arg Ser Arg Phe Ile Gln Val Gly Val Ile
625 630 635 640

Ser Trp Gly Val Val Asp Val Cys Lys Asn Gln Lys Arg Gln Lys Gln
645 650 655

```
Val Pro Ala His Ala Arg Asp Phe His Ile Asn Leu Phe Gln Val Leu
            660                 665                 670

Pro Trp Leu Lys Glu Lys Leu Gln Asp Glu Asp Leu Gly Phe Leu
        675                 680                 685


<210>  141
<211>  481
<212>  PRT
<213>  Homo sapiens

<400> 141

Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190
```

```
Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
                405                 410                 415

Asn Pro Arg Glu Asp Tyr Leu Asp Val Tyr Val Phe Gly Val Gly Pro
            420                 425                 430

Leu Val Asn Gln Val Asn Ile Asn Ala Leu Ala Ser Lys Lys Asp Asn
        435                 440                 445
```

```
Glu Gln His Val Phe Lys Val Lys Asp Met Glu Asn Leu Glu Asp Val
    450                 455                 460

Phe Tyr Gln Met Ile Gly Arg Glu Ile Gln Gly Asn Lys Glu His Asn
465                 470                 475                 480

Ser




<210>  142
<211>  289
<212>  PRT
<213>  Homo sapiens

<400> 142

Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Gly Glu Lys His Pro Leu Pro Leu His Cys Cys Leu Pro Asp Gly
```

165 170 175

Ala Gln Pro Glu Glu Trp Ala Leu Gly Ser Gly His Cys Asn Ser Cys
180 185 190

Ser Leu Pro Cys Ser Arg Gly Leu Arg Leu Gln Cys Leu Pro Arg Cys
195 200 205

Leu Ile Pro Leu Gln Arg Gly Thr Ala Pro Thr Arg Ala Ser Pro Leu
210 215 220

Ala Gln Gly Arg Trp Ala Ala Ser Thr Ala Leu Lys Thr Ala Ser Pro
225 230 235 240

Thr Thr Ala Ala Gly Gly Leu Pro Cys Val Ala Pro Ser Gly Glu Arg
245 250 255

Val Arg Lys Val Ala Leu Gly Ala Gly Arg Ser Leu Pro Ala Lys Thr
260 265 270

Pro Ser Cys Thr Thr Pro Leu Lys Arg Trp Pro Lys Leu Ser Cys Leu
275 280 285

Pro

<210> 143
<211> 122
<212> PRT
<213> Homo sapiens

<400> 143

Met Gly Ser Asn Leu Ser Pro Gln Leu Cys Leu Met Pro Phe Ile Leu
1 5 10 15

Gly Leu Leu Ser Gly Gly Val Thr Thr Thr Pro Trp Ser Leu Ala Arg
20 25 30

Pro Gln Gly Ser Cys Ser Leu Glu Gly Val Glu Ile Lys Gly Gly Ser
35 40 45

Phe Arg Leu Leu Gln Glu Gly Gln Ala Leu Glu Tyr Val Cys Pro Ser
50 55 60

Gly Phe Tyr Pro Tyr Pro Val Gln Thr Arg Thr Cys Arg Ser Thr Gly
65 70 75 80

```
Ser Trp Ser Thr Leu Lys Thr Gln Asp Gln Lys Thr Val Arg Lys Ala
                85                  90                  95

Glu Cys Arg Gly Leu Arg Ala Met Ser Val Gly Ser Gly Leu Arg Gln
            100                 105                 110

Lys Gln Gly Arg Arg Gln Gln Gly Gln Asp
        115                 120


<210>  144
<211>  21
<212>  DNA
<213>  Artificial Organism

<220>
<223>  Synthetic oligonucleotide

<400> 144

gtttgagggc aatgagtgtg g                                               21


<210>  145
<211>  21
<212>  DNA
<213>  Artificial Organism

<220>
<223>  Synthetic oligonucleotide

<400> 145

aaactgctcc tactcccggt c                                               21


<210>  146
<211>  123
<212>  DNA
<213>  Artificial Organism

<220>
<223>  Synthetic oligonucleotide

<400> 146

gtttgagggc aatgagtgtg ggcagtggcc taaggcagaa acagggcagg cggcagcaag     60

gtcaggacta ggatgagact aggcagggtg acaaggtggg ctgaccggga gtaggagcag    120

ttt                                                                  123


<210>  147
<211>  22
<212>  DNA
<213>  Artificial Organism

<220>
<223>  Synthetic oligonucleotide
```

<400> 147

ctacattgct gtctccctga cg 22

<210> 148
<211> 22
<212> DNA
<213> Artificial Organism

<220>
<223> Synthetic oligonucleotide

<400> 148

aggtaagcac tgaagcctga gg 22

<210> 149
<211> 114
<212> DNA
<213> Artificial Organism

<220>
<223> Synthetic oligonucleotide

<400> 149

ctacattgct gtctccctga cggcgcccag cccgaggagt gggcactcgg ctccggacac 60

tgtaactctt gctctctacc ttgctcacgg ggcctcaggc ttcagtgctt acct 114

<210> 150
<211> 21
<212> DNA
<213> Artificial Organism

<220>
<223> Synthetic oligonucleotide

<400> 150

aggcaacacc tcccactttc t 21

<210> 151
<211> 19
<212> DNA
<213> Artificial Organism

<220>
<223> Synthetic oligonucleotide

<400> 151

ttcacgtctt cccccatcc 19

<210> 152
<211> 101
<212> DNA

<213> Artificial Organism

<220>
<223> Synthetic oligonucleotide

<400> 152

aggcaacacc tcccactttc tacagatcct acactccacc catcctcaat gcagccccat 60

tccttgcacc ccagaccagt cagggatggg ggaagacgtg a 101

<210> 153
<211> 18
<212> DNA
<213> Artificial Organism

<220>
<223> Synthetic oligonucleotide

<400> 153

gaagagctgc tccctgca 18

<210> 154
<211> 22
<212> DNA
<213> Artificial Organism

<220>
<223> Synthetic oligonucleotide

<400> 154

ccccattctt gatgtagacc tc 22

<210> 155
<211> 94
<212> DNA
<213> Artificial Organism

<220>
<223> Synthetic oligonucleotide

<400> 155

gaagagctgc tccctgcaca ggatatcaaa gctctgtttg tgtctgagga ggagaaaaag 60

ctgactcgga aggaggtcta catcaagaat gggg 94

<210> 156
<211> 3362
<212> DNA
<213> Homo sapiens

<400> 156

gtgctgtgcg gcgcggtctc agggaaggtg gggctatggc agctgctagg gaccctccgg 60

aagtatcgct gcgagaagcc acccagcgaa aattgcggag gttttccgag ctaagaggca 120

```
aacttgtagc acgtggagaa ttctgggaca tagttgcaat aacagcggct gatgaaaaac    180
aggaacttgc ttacaaccaa cagctgtcag aaaagctgaa aagaaaggag ttaccccttg    240
gagttcaata tcacgttttt gtagatcctg ctggagccaa aattggaaat ggaggatcaa    300
cactttgtgc ccttcaatgt ttggaaaagc tatatggaga taaatggaat tcttttacca    360
tcttattaat tcactctgat gaatggaaga aaaaagtcag tgaatcatat gttatcacaa    420
tagaaagatt agaagatgac ctgcagatca aggaaaaaga actgacagaa ctaaggaata    480
tatttggctc tgatgaagcc ttcagtaaag tcaatttaaa ttaccgcact gaaaatgggc    540
tgtctctact tcatttatgt tgcatttgtg gaggcaagaa atcacatatt cgaactctta    600
tgttgaaagg gctccgccca tctcgactga caagaaatgg atttacagcc ttgcatttag    660
cagtttacaa ggataatgca gaattgatca cttctctgct tcacagtgga gctgatatac    720
agcaggttgg atacggtggc ctcactgccc tccatattgc tacaatagct ggccacctag    780
aggctgctga tgtgctgttg caacatggag ctaatgtcaa tattcaagat gcagtttttt    840
tcactccatt gcatattgca gcgtactatg gacatgaaca ggtaactcgc cttcttttga    900
aatttggtgc tgatgtaaat gtaagtggtg aagttggaga tagacccctc cacctagcat    960
ctgcaaaagg attcttgaat attgcaaaac tcttgatgga agaaggcagc aaagcagatg   1020
tgaatgctca agataatgaa gaccatgtcc cactccattt ctgttctcga tttggacacc   1080
atgatatagt taagtatctg ctgcaaagtg atttggaagt tcaacctcat gttgttaata   1140
tctatggaga taccccctta cacctggcat gctacaatgg caaatttgaa gttgccaagg   1200
aaatcatcca aatatcagga acagaaagtc tgactaagga aaacatcttc agtgaaacag   1260
cttttcatag tgcttgtacc tatggcaaga gcattgacct agtcaaattt cttcttgatc   1320
agaatgtcat aaacatcaac caccaaggaa gggatgggca cactggatta cactctgctt   1380
gctaccacgg tcacattcgc ctggttcagt tcttactgga taatggagct gatatgaatc   1440
tagtggcttg tgatcccagc aggtctagtg gtgaaaaaga tgagcagaca tgtttgatgt   1500
gggcttatga aaaagggcat gatgccattg tcacactcct gaagcattat aagagaccac   1560
aagatgaatt gccctgtaat gaatattctc agcctggagg agatggctcc tatgtgtctg   1620
ttccatcacc cttggggaag attaaaagca tgacaaaaga gaaggcagat attctcctcc   1680
taagagctgg attgccttca catttccatc ttcagctctc agaaattgag ttccatgaga   1740
ttattggctc aggttctttt gggaaagtat ataaaggacg atgcagaaat aaaatagtgg   1800
ctataaaacg ttatcgagcc aatacctact gctccaagtc agatgtggat atgttttgcc   1860
gagaggtgtc cattctctgc cagctcaatc atccctgcgt aattcagttt gtgggtgctt   1920
gcttgaatga tcccagccag tttgccattg tcactcaata catatcaggg ggttctctgt   1980
```

tctccctcct tcatgagcag aagaggattc ttgatttgca gtctaaatta attattgcag 2040

tagatgttgc caaaggcatg gagtaccttc acaacctgac acagccaatt atacatcgtg 2100

acttgaacag gtctgccatt acctctagga tctggatcac ccatagtatt tgcatctgga 2160

ggggagctca ttactttaac agggaagaat gcaatttcag gtgtatgctt acttctgcca 2220

tcctaaaaga atcaagattt ctacagtctc tggatgaaga caacatgaca aaacaacctg 2280

ggaacctccg ttggatggct cctgaggtgt tcacgcagtg cactcggtac accatcaaag 2340

cagatgtctt cagctatgct ctgtgtctgt gggaaattct cactggcgaa attccattcg 2400

ctcatctcaa gccagcggct gcggcagcag acatggctta ccaccacatc agacctccca 2460

ttggctattc cattcccaag cccatatcat ctctgctgat acgagggtgg aacgcatgtc 2520

ctgaaggaag acccgaattt tctgaagttg tcatgaagtt agaagagtgt ctctgcaaca 2580

ttgagctgat gtctcctgca tcaagtaaca gcagtgggtc tctctcacct tcttcttctt 2640

ctgattgcct ggtgaaccgg ggaggacctg gccggagtca tgtggcagca ttaagaagtc 2700

gtttcgaatt ggaatatgct ctaaatgcaa ggtcctatgc tgctttgtcc caaagtgctg 2760

gacaatattc ctctcaaggt ctgtctttgg aggagatgaa aagaagtctt caatacacac 2820

ccattgacaa atatggctat gtatccgatc ccatgagctc aatgcatttt cattcttgcc 2880

gaaatagtag cagctttgag gacagcagct gacagcattc ggcgtatacc taaggagagt 2940

tttttccccg aactgacagc aacgattcca accacggcaa gctggcttcc aactataaca 3000

ttttactctc aaaggtctcc ttaaattggg cttgttttta cttgtcctat ttaattcccc 3060

actattagca ggctttggat ttgtgcctaa ggaataatat gcaaaagaac caagacagaa 3120

tgtatatgaa gaattgtttt taattttgta aattaaaaaa aaatttagat cgttacttgg 3180

aaatggagcc taagtctgtg gtggacagat aataattatg ttttcctggg ctgaattatg 3240

tagacttgtg tttgacagct atgggtttat ttcttagaac attgttcatt ttcttttctc 3300

attatgttac ttctagtgtt cacctctgtg attaaagatt ctttggtgaa atagaaaatt 3360

ga 3362

<210> 157
<211> 3412
<212> DNA
<213> Homo sapiens

<400> 157

cttgaaaaca ggtctatgta gttttaacag ctcttttgtg aacttcggat gtaactgaat 60

cattttctag atttatctgt taaataaatg gtccatgaaa gtatgtatgg gtagataatt 120

gatggaagca aataatccta ttgaaacatt tgctatttat ttttgataca gttcttttaa 180

acagactgat tagttccctt tatgatttca ccctcgaaag cacacacgat tttgttcttt 240

atcattgttg catgacattc catttggcag ttgggcttat ttttagccag actatcaaag 300
tatttttcaa ctggactgtc actgcacttg aacttggaat cttataactt gaagaactgc 360
cctggagaaa ggaagaaact tataataaat gggaaattat aaatctagac caacccaaac 420
ttgtactgat gaatggaaga aaaaagtcag tgaatcatat gttatcacaa tagaaagatt 480
agaagatgac ctgcagatca aggaaaaaga actgacagaa ctaaggaata tatttggctc 540
tgatgaagcc ttcagtaaag tcaatttaaa ttaccgcact gaaaatgggc tgtctctact 600
tcatttatgt tgcatttgtg gaggcaagaa atcacatatt cgaactctta tgttgaaagg 660
gctccgccca tctcgactga caagaaatgg atttacagcc ttgcatttag cagtttacaa 720
ggataatgca gaattgatca cttctctgct tcacagtgga gctgatatac agcaggttgg 780
atacggtggc ctcactgccc tccatattgc tacaatagct ggccacctag aggctgctga 840
tgtgctgttg caacatggag ctaatgtcaa tattcaagat gcagtttttt tcactccatt 900
gcatattgca gcgtactatg gacatgaaca ggtaactcgc cttcttttga aatttggtgc 960
tgatgtaaat gtaagtggtg aagttggaga tagacccctc cacctagcat ctgcaaaagg 1020
attcttgaat attgcaaaac tcttgatgga agaaggcagc aaagcagatg tgaatgctca 1080
agataatgaa gaccatgtcc cactccattt ctgttctcga tttggacacc atgatatagt 1140
taagtatctg ctgcaaagtg atttggaagt tcaacctcat gttgttaata tctatggaga 1200
tacccctta cacctggcat gctacaatgg caaatttgaa gttgccaagg aaatcatcca 1260
aatatcagga acagaaagtc tgactaagga aaacatcttc agtgaaacag cttttcatag 1320
tgcttgtacc tatggcaaga gcattgacct agtcaaattt cttcttgatc agaatgtcat 1380
aaacatcaac caccaaggaa gggatgggca cactggatta cactctgctt gctaccacgg 1440
tcacattcgc ctggttcagt tcttactgga taatggagct gatatgaatc tagtggcttg 1500
tgatcccagc aggtctagtg gtgaaaaaga tgagcagaca tgtttgatgt gggcttatga 1560
aaaagggcat gatgccattg tcacactcct gaagcattat aagagaccac aagatgaatt 1620
gccctgtaat gaatattctc agcctggagg agatggctcc tatgtgtctg ttccatcacc 1680
cttggggaag attaaaagca tgacaaaaga gaaggcagat attctcctcc taagagctgg 1740
attgccttca catttccatc ttcagctctc agaaattgag ttccatgaga ttattggctc 1800
aggttctttt gggaaagtat ataaaggacg atgcagaaat aaaatagtgg ctataaaacg 1860
ttatcgagcc aatacctact gctccaagtc agatgtggat atgttttgcc gagaggtgtc 1920
cattctctgc cagctcaatc atccctgcgt aattcagttt gtgggtgctt gcttgaatga 1980
tcccagccag tttgccattg tcactcaata catatcaggg ggttctctgt tctccctcct 2040
tcatgagcag aagaggattc ttgatttgca gtctaaatta attattgcag tagatgttgc 2100

caaaggcatg gagtaccttc acaacctgac acagccaatt atacatcgtg acttgaacag 2160
gtctgccatt acctctagga tctggatcac ccatagtatt tgcatctgga ggggagctca 2220
ttactttaac agggaagaat gcaatttcag gtgtatgctt acttctgcca tcctaaaaga 2280
atcaagattt ctacagtctc tggatgaaga caacatgaca aaacaacctg ggaacctccg 2340
ttggatggct cctgaggtgt tcacgcagtg cactcggtac accatcaaag cagatgtctt 2400
cagctatgct ctgtgtctgt gggaaattct cactggcgaa attccattcg ctcatctcaa 2460
gccagcggct gcggcagcag acatggctta ccaccacatc agacctccca ttggctattc 2520
cattcccaag cccatatcat ctctgctgat acgagggtgg aacgcatgtc ctgaaggaag 2580
acccgaattt tctgaagttg tcatgaagtt agaagagtgt ctctgcaaca ttgagctgat 2640
gtctcctgca tcaagtaaca gcagtgggtc tctctcacct tcttcttctt ctgattgcct 2700
ggtgaaccgg ggaggacctg gccggagtca tgtggcagca ttaagaagtc gtttcgaatt 2760
ggaatatgct ctaaatgcaa ggtcctatgc tgctttgtcc caaagtgctg gacaatattc 2820
ctctcaaggt ctgtctttgg aggagatgaa aagaagtctt caatacacac ccattgacaa 2880
atatggctat gtatccgatc ccatgagctc aatgcatttt cattcttgcc gaaatagtag 2940
cagctttgag gacagcagct gacagcattc ggcgtatacc taaggagagt tttttccccg 3000
aactgacagc aacgattcca accacggcaa gctggcttcc aactataaca ttttactctc 3060
aaaggtctcc ttaaattggg cttgttttta cttgtcctat ttaattcccc actattagca 3120
ggctttggat ttgtgcctaa ggaataatat gcaaaagaac caagacagaa tgtatatgaa 3180
gaattgtttt taattttgta aattaaaaaa aaatttagat cgttacttgg aaatggagcc 3240
taagtctgtg gtggacagat aataattatg ttttcctggg ctgaattatg tagacttgtg 3300
tttgacagct atgggtttat ttcttagaac attgttcatt ttcttttctc attatgttac 3360
ttctagtgtt cacctctgtg attaaagatt ctttggtgaa atagaaaatt ga 3412

<210> 158
<211> 3420
<212> DNA
<213> Homo sapiens

<400> 158

cttgaaaaca ggtctatgta gttttaacag ctcttttgtg aacttcggat gtaactgaat 60
cattttctag atttatctgt taaataaatg gtccatgaaa gtatgtatgg gtagataatt 120
gatggaagca aataatccta ttgaaacatt tgctatttat ttttgataca gttcttttaa 180
acagactgat tagttccctt tatgatttca ccctcgaaag cacacacgat tttgttcttt 240
atcattgttg catgacattc catttggcag ttgggcttat ttttagccag actatcaaag 300
tatttttcaa ctggactgtc actgcacttg aacttggaat cttataactt gaagaactgc 360

cctggagaaa ggaagaaact tataataaat gggaaattat aaatctagac caacccaaac 420
ttgtactgat gaatggaaga aaaaagtcag tgaatcatat gttatcacaa tagaaagatt 480
agaagatgac ctgcagatca aggaaaaaga actgacagaa ctaaggaata tatttggctc 540
tgatgaagcc ttcagtaaag tcaatttaaa ttaccgcact gaaaatgggc tgtctctact 600
tcatttatgt tgcatttgtg gaggcaagaa atcacatatt cgaactctta tgttgaaagg 660
gctccgccca tctcgactga caagaaatgg atttacagcc ttgcatttag cagtttacaa 720
ggataatgca gaattgatca cttctctgct tcacagtgga gctgatatac agcaggttgg 780
atacggtggc ctcactgccc tccatattgc tacaatagct ggccacctag aggctgctga 840
tgtgctgttg caacatggag ctaatgtcaa tattcaagat gcagtttttt tcactccatt 900
gcatattgca gcgtactatg gacatgaaca ggtaactcgc cttcttttga aatttggtgc 960
tgatgtaaat gtaagtggtg aagttggaga tagacccctc cacctagcat ctgcaaaagg 1020
attcttgaat attgcaaaac tcttgatgga agaaggcagc aaagcagatg tgaatgctca 1080
agataatgaa gaccatgtcc cactccattt ctgttctcga tttggacacc atgatatagt 1140
taagtatctg ctgcaaagtg atttggaagt tcaacctcat gttgttaata tctatggaga 1200
taccccctta cacctggcat gctacaatgg caaatttgaa gttgccaagg aaatcatcca 1260
aatatcagga acagaaagtc tgactaagga aaacatcttc agtgaaacag cttttcatag 1320
tgcttgtacc tatggcaaga gcattgacct agtcaaattt cttcttgatc agaatgtcat 1380
aaacatcaac caccaaggaa gggatgggca cactggatta cactctgctt gctaccacgg 1440
tcacattcgc ctggttcagt tcttactgga taatggagct gatatgaatc tagtggcttg 1500
tgatcccagc aggtctagtg gtgaaaaaga tgagcagaca tgtttgatgt gggcttatga 1560
aaaagggcat gatgccattg tcacactcct gaagcattat aagagaccac aagatgaatt 1620
gccctgtaat gaatattctc agcctggagg agatggctcc tatgtgtctg ttccatcacc 1680
cttggggaag attaaaagca tgacaaaaga gaaggcagat attctcctcc taagagctgg 1740
attgccttca catttccatc ttcagctctc agaaattgag ttccatgaga ttattggctc 1800
aggttctttt gggaaagtat ataaaggacg atgcagaaat aaaatagtgg ctataaaacg 1860
ttatcgagcc aatacctact gctccaagtc agatgtggat atgttttgcc gagaggtgtc 1920
cattctctgc cagctcaatc atccctgcgt aattcagttt gtgggtgctt gcttgaatga 1980
tcccagccag tttgccattg tcactcaata catatcaggg ggttctctgt tctccctcct 2040
tcatgagcag aagaggattc ttgatttgca gtctaaatta attattgcag tagatgttgc 2100
caaaggcatg gagtaccttc acaacctgac acagccaatt atacatcgtg acttgaacag 2160
tcacaatatt cttctctatg aggatgggca tgctgtggtg gcagattttg gagaatcaag 2220

atttctacag tctctggatg aagacaacat gacaaaacaa cctgggaacc tccgttggat 2280

ggctcctgag gtgttcacgc agtgcactcg gtacaccatc aaagcagatg tcttcagcta 2340

tgctctgtgt ctgtgggaaa ttctcactgg cgaaattcca ttcgctcatc tcaagccagc 2400

ggctgcggca gcagacatgg cttaccacca catcagacct cccattggct attccattcc 2460

caagcccata tcatctctgc tgatacgagg gtggaacgca tgtcctgaag gaagacccga 2520

attttctgaa gttgtcatga agttagaaga gtgtctctgc aacattgagc tgatgtctcc 2580

tgcatcaagt aacagcagtg ggtctctctc accttcttct tcttctgatt gcctggtgaa 2640

ccggggagga cctggccgga gtcatgtggc agcattaaga agtcgtttcg aattggaata 2700

tgctctaaat gcaaggtcct atgctgcttt gtcccaaagt gctggacaat attcctctca 2760

aggtctgtct ttggaggaga tgaaaagaag tcttcaatac acacccattg acaaatatga 2820

tgtaacttca taaacacaaa acatagggca tcctttgaaa catttgcttt gaccagaagt 2880

cttccctttt gtggctatgt atccgatccc atgagctcaa tgcattttca ttcttgccga 2940

aatagtagca gctttgagga cagcagctga cagcattcgg cgtataccta aggagagttt 3000

tttccccgaa ctgacagcaa cgattccaac cacggcaagc tggcttccaa ctataacatt 3060

ttactctcaa aggtctcctt aaattgggct tgtttttact tgtcctattt aattccccac 3120

tattagcagg ctttggattt gtgcctaagg aataatatgc aaaagaacca agacagaatg 3180

tatatgaaga attgttttta attttgtaaa ttaaaaaaaa atttagatcg ttacttggaa 3240

atggagccta agtctgtggt ggacagataa taattatgtt ttcctgggct gaattatgta 3300

gacttgtgtt tgacagctat gggtttattt cttagaacat tgttcatttt cttttctcat 3360

tatgttactt ctagtgttca cctctgtgat taaagattct ttggtgaaat agaaaattga 3420

<210> 159
<211> 2670
<212> DNA
<213> Homo sapiens

<400> 159

ccttctccat gtggcctgca ttttcttata ccatggctga cacaggataa tgcagaattg 60

atcacttctc tgcttcacag tggagctgat atacagcagg ttggatacgg tggcctcact 120

gccctccata ttgctacaat agctggccac ctagaggctg ctgatgtgct gttgcaacat 180

ggagctaatg tcaatattca agatgcagtt tttttcactc cattgcatat tgcagcgtac 240

tatggacatg aacaggtaac tcgccttctt ttgaaatttg gtgctgatgt aaatgtaagt 300

ggtgaagttg gagatagacc cctccaccta gcatctgcaa aaggattctt gaatattgca 360

aaactcttga tggaagaagg cagcaaagca gatgtgaatg ctcaagataa tgaagaccat 420

gtcccactcc atttctgttc tcgatttgga caccatgata tagttaagta tctgctgcaa 480

agtgatttgg aagttcaacc tcatgttgtt aatatctatg gagatacccc cttacacctg 540
gcatgctaca atggcaaatt tgaagttgcc aaggaaatca tccaaatatc aggaacagaa 600
agtctgacta aggaaaacat cttcagtgaa acagcttttc atagtgcttg tacctatggc 660
aagagcattg acctagtcaa atttcttctt gatcagaatg tcataaacat caaccaccaa 720
ggaagggatg ggcacactgg attacactct gcttgctacc acggtcacat tcgcctggtt 780
cagttcttac tggataatgg agctgatatg aatctagtgg cttgtgatcc cagcaggtct 840
agtggtgaaa aagatgagca gacatgtttg atgtgggctt atgaaaaagg gcatgatgcc 900
attgtcacac tcctgaagca ttataagaga ccacaagatg aattgccctg taatgaatat 960
tctcagcctg gaggagatgg ctcctatgtg tctgttccat cacccttggg gaagattaaa 1020
agcatgacaa aagagaaggc agatattctc ctcctaagag ctggattgcc ttcacatttc 1080
catcttcagc tctcagaaat tgagttccat gagattattg gctcaggttc ttttgggaaa 1140
gtatataaag gacgatgcag aaataaaata gtggctataa aacgttatcg agccaatacc 1200
tactgctcca agtcagatgt ggatatgttt tgccgagagg tgtccattct ctgccagctc 1260
aatcatccct gcgtaattca gtttgtgggt gcttgcttga atgatcccag ccagtttgcc 1320
attgtcactc aatacatatc aggggggttct ctgttctccc tccttcatga gcagaagagg 1380
attcttgatt tgcagtctaa attaattatt gcagtagatg ttgccaaagg catggagtac 1440
cttcacaacc tgacacagcc aattatacat cgtgacttga acagtcacaa tattcttctc 1500
tatgaggatg ggcatgctgt ggtggcagat tttggagaat caagatttct acagtctctg 1560
gatgaagaca acatgacaaa acaacctggg aacctccgtt ggatggctcc tgaggtgttc 1620
acgcagtgca ctcggtacac catcaaagca gatgtcttca gctatgctct gtgtctgtgg 1680
gaaattctca ctggcgaaat tccattcgct catctcaagc cagcggctgc ggcagcagac 1740
atggcttacc accacatcag acctcccatt ggctattcca ttcccaagcc catatcatct 1800
ctgctgatac gagggtggaa cgcatgtcct gaaggaagac ccgaattttc tgaagttgtc 1860
atgaagttag aagagtgtct ctgcaacatt gagctgatgt ctcctgcatc aagtaacagc 1920
agtgggtctc tctcaccttc ttcttcttct gattgcctgg tgaaccgggg aggacctggc 1980
cggagtcatg tggcagcatt aagaagtcgt ttcgaattgg aatatgctct aaatgcaagg 2040
tcctatgctg ctttgtccca aagtgctgga caatattcct ctcaaggtct gtctttggag 2100
gagatgaaaa gaagtcttca atacacaccc attgacaaat atggctatgt atccgatccc 2160
atgagctcaa tgcattttca ttcttgccga aatagtagca gctttgagga cagcagctga 2220
cagcattcgg cgtataccta aggagagttt tttccccgaa ctgacagcaa cgattccaac 2280
cacggcaagc tggcttccaa ctataacatt ttactctcaa aggtctcctt aaattgggct 2340

tgtttttact tgtcctattt aattccccac tattagcagg ctttggattt gtgcctaagg 2400

aataatatgc aaaagaacca agacagaatg tatatgaaga attgttttta attttgtaaa 2460

ttaaaaaaaa atttagatcg ttacttggaa atggagccta agtctgtggt ggacagataa 2520

taattatgtt ttcctgggct gaattatgta gacttgtgtt tgacagctat gggtttattt 2580

cttagaacat tgttcatttt cttttctcat tatgttactt ctagtgttca cctctgtgat 2640

taaagattct ttggtgaaat agaaaattga 2670

<210> 160
<211> 2736
<212> DNA
<213> Homo sapiens

<400> 160

ccttctccat gtggcctgca ttttcttata ccatggctga cacaggataa tgcagaattg 60

atcacttctc tgcttcacag tggagctgat atacagcagg ttggatacgg tggcctcact 120

gccctccata ttgctacaat agctggccac ctagaggctg ctgatgtgct gttgcaacat 180

ggagctaatg tcaatattca agatgcagtt tttttcactc cattgcatat tgcagcgtac 240

tatggacatg aacaggtaac tcgccttctt ttgaaatttg gtgctgatgt aaatgtaagt 300

ggtgaagttg gagatagacc cctccaccta gcatctgcaa aaggattctt gaatattgca 360

aaactcttga tggaagaagg cagcaaagca gatgtgaatg ctcaagataa tgaagaccat 420

gtcccactcc atttctgttc tcgatttgga caccatgata tagttaagta tctgctgcaa 480

agtgatttgg aagttcaacc tcatgttgtt aatatctatg gagatacccc cttacacctg 540

gcatgctaca atggcaaatt tgaagttgcc aaggaaatca tccaaatatc aggaacagaa 600

agtctgacta aggaaaacat cttcagtgaa acagcttttc atagtgcttg tacctatggc 660

aagagcattg acctagtcaa atttcttctt gatcagaatg tcataaacat caaccaccaa 720

ggaagggatg ggcacactgg attacactct gcttgctacc acggtcacat tcgcctggtt 780

cagttcttac tggataatgg agctgatatg aatctagtgg cttgtgatcc cagcaggtct 840

agtggtgaaa aagatgagca gacatgtttg atgtgggctt atgaaaaagg gcatgatgcc 900

attgtcacac tcctgaagca ttataagaga ccacaagatg aattgccctg taatgaatat 960

tctcagcctg gaggagatgg ctcctatgtg tctgttccat cacccttggg gaagattaaa 1020

agcatgacaa aagagaaggc agatattctc ctcctaagag ctggattgcc ttcacatttc 1080

catcttcagc tctcagaaat tgagttccat gagattattg gctcaggttc ttttgggaaa 1140

gtatataaag gacgatgcag aaataaaata gtggctataa aacgttatcg agccaatacc 1200

tactgctcca agtcagatgt ggatatgttt tgccgagagg tgtccattct ctgccagctc 1260

aatcatccct gcgtaattca gtttgtgggt gcttgcttga atgatcccag ccagtttgcc 1320

attgtcactc aatacatatc aggggggttct ctgttctccc tccttcatga gcagaagagg 1380

attcttgatt tgcagtctaa attaattatt gcagtagatg ttgccaaagg catggagtac 1440

cttcacaacc tgacacagcc aattatacat cgtgacttga acaggtctgc cattacctct 1500

aggatctgga tcacccatag tatttgcatc tggaggggag ctcattactt taacagggaa 1560

gaatgcaatt tcaggtgtat gcttacttct gccatcctaa aagaatcaag atttctacag 1620

tctctggatg aagacaacat gacaaaacaa cctgggaacc tccgttggat ggctcctgag 1680

gtgttcacgc agtgcactcg gtacaccatc aaagcagatg tcttcagcta tgctctgtgt 1740

ctgtgggaaa ttctcactgg cgaaattcca ttcgctcatc tcaagccagc ggctgcggca 1800

gcagacatgg cttaccacca catcagacct cccattggct attccattcc caagcccata 1860

tcatctctgc tgatacgagg gtggaacgca tgtcctgaag gaagacccga attttctgaa 1920

gttgtcatga agttagaaga gtgtctctgc aacattgagc tgatgtctcc tgcatcaagt 1980

aacagcagtg ggtctctctc accttcttct tcttctgatt gcctggtgaa ccggggagga 2040

cctggccgga gtcatgtggc agcattaaga agtcgtttcg aattggaata tgctctaaat 2100

gcaaggtcct atgctgcttt gtcccaaagt gctggacaat attcctctca aggtctgtct 2160

ttggaggaga tgaaaagaag tcttcaatac acacccattg acaaatatgg ctatgtatcc 2220

gatcccatga gctcaatgca ttttcattct tgccgaaata gtagcagctt tgaggacagc 2280

agctgacagc attcggcgta tacctaagga gagtttttttc cccgaactga cagcaacgat 2340

tccaaccacg gcaagctggc ttccaactat aacattttac tctcaaaggt ctccttaaat 2400

tgggcttgtt tttacttgtc ctatttaatt ccccactatt agcaggcttt ggatttgtgc 2460

ctaaggaata atatgcaaaa gaaccaagac agaatgtata tgaagaattg tttttaattt 2520

tgtaaattaa aaaaaaattt agatcgttac ttggaaatgg agcctaagtc tgtggtggac 2580

agataataat tatgttttcc tgggctgaat tatgtagact tgtgtttgac agctatgggt 2640

ttatttctta gaacattgtt cattttcttt tctcattatg ttacttctag tgttcacctc 2700

tgtgattaaa gattctttgg tgaaatagaa aattga 2736

<210> 161
<211> 2750
<212> DNA
<213> Homo sapiens

<400> 161

gtgctgtgcg gcgcggtctc agggaaggtg gggctatggc agctgctagg gaccctccgg 60

aagtatcgct gcgagaagcc acccagcgaa aattgcggag gttttccgag ctaagaggca 120

aacttgtagc acgtggagaa ttctgggaca tagttgcaat aacagcggct gatgaaaaac 180

aggaacttgc ttacaaccaa cagctgtcag aaaagctgaa aagaaaggag ttaccccttg 240
gagttcaata tcacgttttt gtagatcctg ctggagccaa aattggaaat ggaggatcaa 300
cactttgtgc ccttcaatgt ttggaaaagc tatatggaga taaatggaat tcttttacca 360
tcttattaat tcactctgat gaatggaaga aaaaagtcag tgaatcatat gttatcacaa 420
tagaaagatt agaagatgac ctgcagatca aggaaaaaga actgacagaa ctaaggaata 480
tatttggctc tgatgaagcc ttcagtaaag tcaatttaaa ttaccgcact gaaaatgggc 540
tgtctctact tcatttatgt tgcatttgtg gaggcaagaa atcacatatt cgaactctta 600
tgttgaaagg gctccgccca tctcgactga caagaaatgg atttacagcc ttgcatttag 660
cagtttacaa ggataatgca gaattgatca cttctctgct tcacagtgga gctgatatac 720
agcaggttgg atacggtggc ctcactgccc tccatattgc tacaatagct ggccacctag 780
aggctgctga tgtgctgttg caacatggag ctaatgtcaa tattcaagat gcagtttttt 840
tcactccatt gcatattgca gcgtactatg gacatgaaca ggtaactcgc cttcttttga 900
aatttggtgc tgatgtaaat gtaagtggtg aagttggaga tagacccctc cacctagcat 960
ctgcaaaagg attcttgaat attgcaaaac tcttgatgga agaaggcagc aaagcagatg 1020
tgaatgctca agataatgaa gaccatgtcc cactccattt ctgttctcga tttggacacc 1080
atgatatagt taagtatctg ctgcaaagtg atttggaagt tcaacctcat gttgttaata 1140
tctatggaga taccccctta cacctggcat gctacaatgg caaatttgaa gttgccaagg 1200
aaatcatcca aatatcagga acagaaagtc tgactaagga aaacatcttc agtgaaacag 1260
cttttcatag tgcttgtacc tatggcaaga gcattgacct agtcaaattt cttcttgatc 1320
agaatgtcat aaacatcaac caccaaggaa gggatgggca cactggatta cactctgctt 1380
gctaccacgg tcacattcgc ctggttcagt tcttactgga taatggagct gatatgaatc 1440
tagtggcttg tgatcccagc aggtctagtg gtgaaaaaga tgagcagaca tgtttgatgt 1500
gggcttatga aaaagggcat gatgccattg tcacactcct gaagcattat aagagaccac 1560
aagatgaatt gccctgtaat gaatattctc agcctggagg agatggctcc tatgtgtctg 1620
ttccatcacc cttggggaag attaaaagca tgacaaaaga gaaggcagat attctcctcc 1680
taagagctgg attgccttca catttccatc ttcagctctc agaaattgag ttccatgaga 1740
ttattggctc aggttctttt gggaaagtat ataaaggacg atgcagaaat aaaatagtgg 1800
ctataaaacg ttatcgagcc aatacctact gctccaagtc agatgtggat atgttttgcc 1860
gagaggtgtc cattctctgc cagctcaatc atccctgcgt aattcagttt gtgggtgctt 1920
gcttgaatga tcccagccag tttgccattg tcactcaata catatcaggg ggttctctgt 1980
tctccctcct tcatgagcag aagaggattc ttgatttgca gtctaaatta attattgcag 2040
tagatgttgc caaaggcatg gagtaccttc acaacctgac acagccaatt atacatcgtg 2100

acttgaacag tcacaatatt cttctctatg aggatgggca tgctgtggtg gcagattttg 2160
gagaatcaag atttctacag tctctggatg aagacaacat gacaaaacaa cctgggaacc 2220
tccgttggat ggctcctgag gtgttcacgc agtgcactcg gtacaccatc aaagcagatg 2280
tcttcagcta tgctctgtgt ctgtgggaaa ttctcactgg cgaaattcca ttcgctcatc 2340
tcaagccagc ggctgcggca gcagacatgg cttaccacca catcagacct cccattggct 2400
attccattcc caagcccata tcatctctgc tgatacgagg gtggaacgca tgtcctgaag 2460
caaaatccag accaagtcat tacccagtct catctgtgta cacagaaact cttaagaaga 2520
aaaatgaaga tcgttttggg atgtggattg agtatctcag aagataacct cttatcctgg 2580
ccattcaacc tgatgtgtta catgtttatt tgtttagaat cttccatcac taccaaaatg 2640
ttagctccat gaaagcggtc ctttttgttt attttgttca ctgctataac actaacatct 2700
aaagctggat gcttaagaat gtttgttgag taaatgaatg aataccagta 2750

<210> 162
<211> 2800
<212> DNA
<213> Homo sapiens

<400> 162

cttgaaaaca ggtctatgta gttttaacag ctcttttgtg aacttcggat gtaactgaat 60
cattttctag atttatctgt taaataaatg gtccatgaaa gtatgtatgg gtagataatt 120
gatggaagca aataatccta ttgaaacatt tgctatttat ttttgataca gttcttttaa 180
acagactgat tagttccctt tatgatttca ccctcgaaag cacacacgat tttgttcttt 240
atcattgttg catgacattc catttggcag ttgggcttat ttttagccag actatcaaag 300
tatttttcaa ctggactgtc actgcacttg aacttggaat cttataactt gaagaactgc 360
cctggagaaa ggaagaaact tataataaat gggaaattat aaatctagac caacccaaac 420
ttgtactgat gaatggaaga aaaaagtcag tgaatcatat gttatcacaa tagaaagatt 480
agaagatgac ctgcagatca aggaaaaaga actgacagaa ctaaggaata tatttggctc 540
tgatgaagcc ttcagtaaag tcaatttaaa ttaccgcact gaaaatgggc tgtctctact 600
tcatttatgt tgcatttgtg gaggcaagaa atcacatatt cgaactctta tgttgaaagg 660
gctccgccca tctcgactga caagaaatgg atttacagcc ttgcatttag cagtttacaa 720
ggataatgca gaattgatca cttctctgct tcacagtgga gctgatatac agcaggttgg 780
atacggtggc ctcactgccc tccatattgc tacaatagct ggccacctag aggctgctga 840
tgtgctgttg caacatggag ctaatgtcaa tattcaagat gcagtttttt tcactccatt 900
gcatattgca gcgtactatg gacatgaaca ggtaactcgc cttcttttga aatttggtgc 960

tgatgtaaat gtaagtggtg aagttggaga tagacccctc cacctagcat ctgcaaaagg 1020
attcttgaat attgcaaaac tcttgatgga agaaggcagc aaagcagatg tgaatgctca 1080
agataatgaa gaccatgtcc cactccattt ctgttctcga tttggacacc atgatatagt 1140
taagtatctg ctgcaaagtg atttggaagt tcaacctcat gttgttaata tctatggaga 1200
tacccccttta cacctggcat gctacaatgg caaatttgaa gttgccaagg aaatcatcca 1260
aatatcagga acagaaagtc tgactaagga aaacatcttc agtgaaacag cttttcatag 1320
tgcttgtacc tatggcaaga gcattgacct agtcaaattt cttcttgatc agaatgtcat 1380
aaacatcaac caccaaggaa gggatgggca cactggatta cactctgctt gctaccacgg 1440
tcacattcgc ctggttcagt tcttactgga taatggagct gatatgaatc tagtggcttg 1500
tgatcccagc aggtctagtg gtgaaaaaga tgagcagaca tgtttgatgt gggcttatga 1560
aaaagggcat gatgccattg tcacactcct gaagcattat aagagaccac aagatgaatt 1620
gccctgtaat gaatattctc agcctggagg agatggctcc tatgtgtctg ttccatcacc 1680
cttggggaag attaaaagca tgacaaaaga gaaggcagat attctcctcc taagagctgg 1740
attgccttca catttccatc ttcagctctc agaaattgag ttccatgaga ttattggctc 1800
aggttctttt gggaaagtat ataaaggacg atgcagaaat aaaatagtgg ctataaaacg 1860
ttatcgagcc aatacctact gctccaagtc agatgtggat atgttttgcc gagaggtgtc 1920
cattctctgc cagctcaatc atccctgcgt aattcagttt gtgggtgctt gcttgaatga 1980
tcccagccag tttgccattg tcactcaata catatcaggg ggttctctgt tctccctcct 2040
tcatgagcag aagaggattc ttgatttgca gtctaaatta attattgcag tagatgttgc 2100
caaaggcatg gagtaccttc acaacctgac acagccaatt atacatcgtg acttgaacag 2160
tcacaatatt cttctctatg aggatgggca tgctgtggtg gcagattttg gagaatcaag 2220
atttctacag tctctggatg aagacaacat gacaaaacaa cctgggaacc tccgttggat 2280
ggctcctgag gtgttcacgc agtgcactcg gtacaccatc aaagcagatg tcttcagcta 2340
tgctctgtgt ctgtgggaaa ttctcactgg cgaaattcca ttcgctcatc tcaagccagc 2400
ggctgcggca gcagacatgg cttaccacca catcagacct cccattggct attccattcc 2460
caagcccata tcatctctgc tgatacgagg gtggaacgca tgtcctgaag caaaatccag 2520
accaagtcat tacccagtct catctgtgta cacagaaact cttaagaaga aaaatgaaga 2580
tcgttttggg atgtggattg agtatctcag aagataacct cttatcctgg ccattcaacc 2640
tgatgtgtta catgtttatt tgtttagaat cttccatcac taccaaaatg ttagctccat 2700
gaaagcggtc ctttttgttt attttgttca ctgctataac actaacatct aaagctggat 2760
gcttaagaat gtttgttgag taaatgaatg aataccagta 2800

<210> 163
<211> 7566
<212> DNA
<213> Homo sapiens

<400> 163

gtgctgtgcg gcgcggtctc agggaaggtg gggctatggc agctgctagg gaccctccgg 60

aagtatcgct gcgagaagcc acccagcgaa aattgcggag gttttccgag ctaagaggca 120

aacttgtagc acgtggagaa ttctgggaca tagttgcaat aacagcggct gatgaaaaac 180

aggaacttgc ttacaaccaa cagctgtcag aaaagctgaa aagaaaggag ttaccccttg 240

gagttcaata tcacgttttt gtagatcctg ctggagccaa aattggaaat ggaggatcaa 300

cactttgtgc ccttcaatgt ttggaaaagc tatatggaga taaatggaat tcttttacca 360

tcttattaat tcactctgat gaatggaaga aaaaagtcag tgaatcatat gttatcacaa 420

tagaaagatt agaagatgac ctgcagatca aggaaaaaga actgacagaa ctaaggaata 480

tatttggctc tgatgaagcc ttcagtaaag tcaatttaaa ttaccgcact gaaaatgggc 540

tgtctctact tcatttatgt tgcatttgtg gaggcaagaa atcacatatt cgaactctta 600

tgttgaaagg gctccgccca tctcgactga caagaaatgg atttacagcc ttgcatttag 660

cagtttacaa ggataatgca gaattgatca cttctctgct tcacagtgga gctgatatac 720

agcaggttgg atacggtggc ctcactgccc tccatattgc tacaatagct ggccacctag 780

aggctgctga tgtgctgttg caacatggag ctaatgtcaa tattcaagat gcagtttttt 840

tcactccatt gcatattgca gcgtactatg gacatgaaca ggtaactcgc cttcttttga 900

aatttggtgc tgatgtaaat gtaagtggtg aagttggaga tagacccctc cacctagcat 960

ctgcaaaagg attcttgaat attgcaaaac tcttgatgga agaaggcagc aaagcagatg 1020

tgaatgctca agataatgaa gaccatgtcc cactccattt ctgttctcga tttggacacc 1080

atgatatagt taagtatctg ctgcaaagtg atttggaagt tcaacctcat gttgttaata 1140

tctatggaga taccccctta cacctggcat gctacaatgg caaatttgaa gttgccaagg 1200

aaatcatcca aatatcagga acagaaagtc tgactaagga aaacatcttc agtgaaacag 1260

cttttcatag tgcttgtacc tatggcaaga gcattgacct agtcaaattt cttcttgatc 1320

agaatgtcat aaacatcaac caccaaggaa gggatgggca cactggatta cactctgctt 1380

gctaccacgg tcacattcgc ctggttcagt tcttactgga taatggagct gatatgaatc 1440

tagtggcttg tgatcccagc aggtctagtg gtgaaaaaga tgagcagaca tgtttgatgt 1500

gggcttatga aaaagggcat gatgccattg tcacactcct gaagcattat aagagaccac 1560

aagatgaatt gccctgtaat gaatattctc agcctggagg agatggctcc tatgtgtctg 1620

ttccatcacc cttggggaag attaaaagca tgacaaaaga gaaggcagat attctcctcc 1680

taagagctgg attgccttca catttccatc ttcagctctc agaaattgag ttccatgaga 1740
ttattggctc aggttctttt gggaaagtat ataaaggacg atgcagaaat aaaatagtgg 1800
ctataaaacg ttatcgagcc aatacctact gctccaagtc agatgtggat atgttttgcc 1860
gagaggtgtc cattctctgc cagctcaatc atccctgcgt aattcagttt gtgggtgctt 1920
gcttgaatga tcccagccag tttgccattg tcactcaata catatcaggg ggttctctgt 1980
tctccctcct tcatgagcag aagaggattc ttgatttgca gtctaaatta attattgcag 2040
tagatgttgc caaaggcatg gagtaccttc acaacctgac acagccaatt atacatcgtg 2100
acttgaacag gtattttttt cctaaataat gaactcagaa gggtatgact aactgggagt 2160
ttaagacaga tttcagtgaa gatacatttt agacttattg cagatcaggg tactctgtgg 2220
ttaagaggac aggctaccat aagcttaggc gatagaaagt tttgtcattg tcttttaagg 2280
agtagaaagc tgtataactg cttctcaggg atcttttaaa tctatgaaga atattcacaa 2340
atatagtaat atctgtaaga tgcaccagct aactagttcc caggtcagta aggtcacatc 2400
aagcagtgag ctaaatggct attaaaaaaa tgatatgtct cctataaagt gaagtgttgg 2460
agtagctcct gatataaaat tatctgaaac gtactttgtg ctaccgtaaa ttcagcttaa 2520
ccacaacagc ttatttacct agttatcagg taaagaggat ttcatgtgaa acaaatataa 2580
aatcagagtg ttccagtggt ggaaccccaa gtgacctcaa aacttttcct aaatagtgaa 2640
aactttctct gagtaaagtc ttataggaaa ggccaatata caaaatggag cagtgttggg 2700
tgaaggtaaa gtggggagga gaaaactcta tttacttccc atttcccact gctaaggaga 2760
gagattcctg caccacctct gtggaagccc aaaggtgctg tagagaatag ttggacaaac 2820
actgatttag tttatgaagc aagtcagatt attccacatc aatcactttc attttatgat 2880
agtcaataag attcaaatag attgtgtata tagtccttta tagaagcttt acagttctga 2940
agctagtaaa tattgattac tctagtcagt catttggtca acaactatgt ttgaacatat 3000
atgtttaaag actattttca gcacagtaga gcttacaaag tgggacgttc atagactaca 3060
ttcagggagt tttcacactc tatgtagagg agaaaaatat ttacctaaat gtatttgagg 3120
tataaaataa taagttatat acaagattta gtacaaaaat ttcaggaaca agaatattaa 3180
ttgcttctga ggattctaac aacaactcta tggaagggat gacatatgag cttggtcctg 3240
aaagatgggt agtttggaag agcatcctat aggcttaggg aagacataat aaaatgcata 3300
gagattagaa agttaaaggt atatatagga actatagttt cattttagta tacaaagtgt 3360
ataaatgtat gtaatgtgta taatgtaaga agtggacaac tggaaaaagg tatagaaaaa 3420
tagaaaaaga gcacagacac cagtgaatgc tattactaag gtatttcaga ttttttttcc 3480
tgtaaggaga ccataaagag gtttgagctg aaaatcacaa atttggagct atattattga 3540
aagatgaatc cagcatctgg taaaatatca acttgaaggg aaagtcaaga aactagtgag 3600

ggagttgttg cagtaatcta gataagaagt aacgaaaagc agaatttcac cggtggcagt 3660
ggatatgaaa ggaagagaca tgtcaacttt aaataacaaa atacagagtt tattcaagct 3720
caaagcttga ggatcattac ccatgagcat ggactcaagt tgtcctgaat acatactcca 3780
attagcagtg gttacaagat agtttttaag aaaaaaaaaa aaaaaaaaaa aagcagtttt 3840
taagttgttt acaaagaatt tacataaata atataaacta ttgattggtt gtaccttgtt 3900
ctttaatcaa aaattatagg aacataaaga taatgggtaa cacagctagt caggaacaaa 3960
atacctttac actattgccc cagggaaatg gagattggag agatgactaa agtcccatgc 4020
tcatgtctct ctgggcctga taaattttgt gttgctcata cagctcagac tgccctcagc 4080
tatttttctc ttctcagata gatatgaaag ataggagaga taaatcagat acgtgagtgg 4140
tagaatctta aatattgaaa gtaaatgtaa ataagtctca atctatttat aattatttac 4200
tagttttcac ataaaagtca tttcttaatt tttagctatt agttctttac ctttcttaat 4260
ttcaaaccaa acatttacgt taacaaacat ttaactattt cagacacaga tttagatgat 4320
aaattagcat aatataaacc gatgattttg agttttaaga gtattatctt tccatcccaa 4380
acatttttgc acatggtatt aaaacaaatg agtaatcatt gcaataagta atcattgcaa 4440
gttttctcta gctgtaactc ttcatttgtg gggccattat atattctgtt gccctgagtt 4500
atgtgcaagt tgttaccacc agttgtcagg caggaccaaa aatataaatg ttgattaaaa 4560
taaaaacaga acatttacta tatagctgat ataagtttat atctacaatt tttgctgcct 4620
ctaatctaac ctaaatgctt aattgcatca aaaagttttt aaatcttatg aaacaccatt 4680
ttgtaggtgc cttcattatg acagagtccc aaactattgt atcaattatt tgtagactat 4740
tttctatcaa tctttaatct ggtagcaaca gcaaaacatt gtttttcaga tgcatgcata 4800
tgtagcattc tacatggctc aaaagagcct gggtcctttc cctcagagag cttactgcac 4860
tacttggcaa ataagacata tgcccagaaa gcaactagag ttccacttta tatagaaaat 4920
aattaagggt caaaatgatg aaagagcaac tttcagagga tttcaagtaa gtcaaagatg 4980
aaagttgagt agaatgcttc ttagcctatg tggagctaaa cgtagagttt gaagaacagg 5040
cattaatctt ttctcaggct tttcacagca tactaacctc tgctttagca tggtacctag 5100
aaagtctcac tcaaagaagt agagagaaag atgcacatca gagactggga aactcataca 5160
cttatttgca tttgtattga catggtgtgt ttatattttt atattcccca ttagcctggg 5220
caagtcaggg tttcaggggc agagactgct ctcttcatct cacattttct gaactaagca 5280
ttgtgtctta cacagagaag atgtctaatt aatatttttt aactgaacca aattattgtg 5340
gatttctatg agtgtcattt cacgaagcaa tgacctaatt ggtataagta tgactacttg 5400
tgtatagcat ccaacttgcc caataacttt tataatttta aagggaaaag gatgtttgag 5460

cccacgggat ggggcacttt tatatttgag tttgtggttg ttgcaattgt ttgagtgaaa 5520

aaaaagaact ctacttttcc catggcaagc aaagccagct gagggtttgt accatccagt 5580

tctgaaagtt aattttgatc ttcagtaaat gtcccttggc atctgcaggg gattggttcc 5640

aggacctctc atggatacca aagtctgaga atgctcaagt ccctggtatt aattgctgta 5700

gcatttgcat atttcctatg cacatcctct cctgtacttt aaatcattga aagagtacct 5760

ataatatcta gtacaatgta aatgctctgt aaatagtttt tgcactatat tgtctaggga 5820

ataatgacaa aggaaaaaga gtctatacat cttcagtaca gacacaatct ttaaaaaaat 5880

tttttgatcc atggttagtt gaatccacag aagtgaaacc tatggattta gagggccaac 5940

tatatatact aaggaaaagt tcaaggaagg agtaagaaag tagttatttt caatgtaaat 6000

gatgataatg ataataccta ccatttattt atcaccatat gaaaggagct ttgctaagtt 6060

tttcatgctt tcatttaacc acgcaactct atgggatagt tgttattatt tccattttac 6120

agataaggaa actgactaaa gagactaagt ctgtagtatt ctgtatcttc tactgtaaca 6180

acaacaacag caaaaccatg tgagaagaga gagatagaag gagcgtactg atgatattga 6240

atgggcatca gcaatattta tcagccttac tctctgtact ttaatctttt aaaaagtcaa 6300

ttcctgtgac ccaaacaatt tctcatatca tcttatgctt ctgtgttcct gctttatttc 6360

ttctttcaca ctccatgttc tgctggtgtg gaacagatgt tgcatagaaa gcatgcagat 6420

aggttttgtt tggcccatag tgtttaaata ttttgaaaat gttcataatt caagcaggaa 6480

tgtacctctc taatttgctg tagacctcac cacctaccaa cttatatttg gtctaattta 6540

tacattcatt ttgtggcatt ggagtttgct agtcattatt tatagcttaa aggatttaaa 6600

ctttttactt aatactagaa gctgtgtgtt tttcttggaa aagtatattt ttcaaattgt 6660

ttgacactga agcagaaaca tcactgcaaa catacatgga tcaaatacat gatattctcc 6720

ttctataaag atatagtatt atatgttata ttaacactgt aaggaattaa atagtgataa 6780

cctccatcac aaagtgcaca tggcattcac atgaaatatg tatatgaaat cattttataa 6840

actgtgaagc accatacaaa atgcattttt tattactctt aattcctcat ttgttccctt 6900

taatattggg gatggaaaaa cactccacaa ttaaaaccac ctttgcaaaa actacaactg 6960

agaaaattat gactgaaaga tctgacttga ctccactttg cttctaacct gcaagctgtt 7020

cttgttcatt catggacata ggccaaaata actttgggag gaacttgaaa ctaagacaat 7080

aatagccctt tcacaaaaca aacctctttc ctgcctgggg actacactac cttcatagga 7140

ctaacaaatt agccacaaga ttagaaataa tggtttagga atcacatagc tggagactga 7200

aagattcgaa acctccccag attgcttctg gggataaatc actattgcaa aacctaagat 7260

cagtgcttga gatattttgc agatgctgta caggatggct cagctgctac cacccagact 7320

gataaactgg gtcatctggt ctcctggccc caggagactg actcagcatc aagaggacaa 7380

ctttgacacc ctatgatatc atctccaacc caaccaatca gcagtcccca ctttcagacc 7440

cctacccacc aagttttcct taaaaccccc atcctcaagt ttttgagaag actgatttga 7500

gtaataataa cactcttctc tcctgtacag ctggctgtgc gtgaattaaa cttttctcta 7560

ttgcaa 7566

<210> 164
<211> 2444
<212> DNA
<213> Homo sapiens

<400> 164

cttgaaaaca ggtctatgta gttttaacag ctcttttgtg aacttcggat gtaactgaat 60

cattttctag atttatctgt taaataaatg gtccatgaaa gtatgtatgg gtagataatt 120

gatggaagca aataatccta ttgaaacatt tgctatttat ttttgataca gttcttttaa 180

acagactgat tagttccctt tatgatttca ccctcgaaag cacacacgat tttgttcttt 240

atcattgttg catgacattc catttggcag ttgggcttat ttttagccag actatcaaag 300

tatttttcaa ctggactgtc actgcacttg aacttggaat cttataactt gaagaactgc 360

cctggagaaa ggaagaaact tataataaat gggaaattat aaatctagac caacccaaac 420

ttgtactgat gaatggaaga aaaaagtcag tgaatcatat gttatcacaa tagaaagatt 480

agaagatgac ctgcagatca aggaaaaaga actgacagaa ctaaggaata tatttggctc 540

tgatgaagcc ttcagtaaag tcaatttaaa ttaccgcact gaaaatgggc tgtctctact 600

tcatttatgt tgcatttgtg gaggcaagaa atcacatatt cgaactctta tgttgaaagg 660

gctccgccca tctcgactga caagaaatgg atttacagcc ttgcatttag cagtttacaa 720

ggataatgca gaattgatca cttctctgct tcacagtgga gctgatatac agcaggttgg 780

atacggtggc ctcactgccc tccatattgc tacaatagct ggccacctag aggctgctga 840

tgtgctgttg caacatggag ctaatgtcaa tattcaagat gcagtttttt tcactccatt 900

gcatattgca gcgtactatg gacatgaaca ggtaactcgc cttcttttga aatttggtgc 960

tgatgtaaat gtaagtggtg aagttggaga tagacccctc cacctagcat ctgcaaaagg 1020

attcttgaat attgcaaaac tcttgatgga agaaggcagc aaagcagatg tgaatgctca 1080

agataatgaa gaccatgtcc cactccattt ctgttctcga tttggacacc atgatatagt 1140

taagtatctg ctgcaaagtg atttggaagt tcaacctcat gttgttaata tctatggaga 1200

tacccccctta cacctggcat gctacaatgg caaatttgaa gttgccaagg aaatcatcca 1260

aatatcagga acagaaagtc tgactaagga aaacatcttc agtgaaacag cttttcatag 1320

tgcttgtacc tatggcaaga gcattgacct agtcaaattt cttcttgatc agaatgtcat 1380

aaacatcaac caccaaggaa gggatgggca cactggatta cactctgctt gctaccacgg 1440

tcacattcgc ctggttcagt tcttactgga taatggagct gatatgaatc tagtggcttg 1500

tgatcccagc aggtctagtg gtgaaaaaga tgagcagaca tgtttgatgt gggcttatga 1560

aaaagggcat gatgccattg tcacactcct gaagcattat aagagaccac aagatgaatt 1620

gccctgtaat gaatattctc agcctggagg agatggctcc tatgtgtctg ttccatcacc 1680

cttggggaag attaaaagca tgacaaaaga gaaggcagat attctcctcc taagagctgg 1740

attgccttca catttccatc ttcagctctc agaaattgag ttccatgaga ttattggctc 1800

aggttctttt gggaaagtat ataaaggacg atgcagaaat aaaatagtgg ctataaaacg 1860

ttatcgagcc aatacctact gctccaagtc agatgtggat atgttttgcc gagaggtgtc 1920

cattctctgc cagctcaatc atccctgcgt aattcagttt gtgggtgctt gcttgaatga 1980

tcccagccag tttgccattg tcactcaata catatcaggg ggttctctgt tctccctcct 2040

tcatgagcag aagaggattc ttgatttgca gtctaaatta attattgcag tagatgttgc 2100

caaaggcatg gagtaccttc acaacctgac acagccaatt atacatcgtg acttgaacag 2160

atgctgtaca ggatggctca gctgctacca cccagactga taaactgggt catctggtct 2220

cctggcccca ggagactgac tcagcatcaa gaggacaact ttgacaccct atgatatcat 2280

ctccaaccca accaatcagc agtccccact ttcagacccc tacccaccaa gttttcctta 2340

aaacccccat cctcaagttt ttgagaagac tgatttgagt aataataaca ctcttctctc 2400

ctgtacagct ggctgtgcgt gaattaaact tttctctatt gcaa 2444

<210> 165
<211> 2352
<212> DNA
<213> Homo sapiens

<400> 165

cttgaaaaca ggtctatgta gttttaacag ctcttttgtg aacttcggat gtaactgaat 60

cattttctag atttatctgt taaataaatg gtccatgaaa gtatgtatgg gtagataatt 120

gatggaagca aataatccta ttgaaacatt tgctatttat ttttgataca gttcttttaa 180

acagactgat tagttccctt tatgatttca ccctcgaaag cacacacgat tttgttcttt 240

atcattgttg catgacattc catttggcag ttgggcttat ttttagccag actatcaaag 300

tatttttcaa ctggactgtc actgcacttg aacttggaat cttataactt gaagaactgc 360

cctggagaaa ggaagaaact tataataaat gggaaattat aaatctagac caacccaaac 420

ttgtactgat gaatggaaga aaaaagtcag tgaatcatat gttatcacaa tagaaagatt 480

agaagatgac ctgcagatca aggaaaaaga actgacagaa ctaaggaata tatttggctc 540

tgatgaagcc ttcagtaaag tcaatttaaa ttaccgcact gaaaatgggc tgtctctact 600

tcatttatgt tgcatttgtg gaggcaagaa atcacatatt cgaactctta tgttgaaagg 660

gctccgccca tctcgactga caagaaatgg atttacagcc ttgcatttag cagtttacaa 720

ggataatgca gaattgatca cttctctgct tcacagtgga gctgatatac agcaggttgg 780

atacggtggc ctcactgccc tccatattgc tacaatagct ggccacctag aggctgctga 840

tgtgctgttg caacatggag ctaatgtcaa tattcaagat gcagtttttt tcactccatt 900

gcatattgca gcgtactatg gacatgaaca ggtaactcgc cttcttttga aatttggtgc 960

tgatgtaaat gtaagtggtg aagttggaga tagacccctc cacctagcat ctgcaaaagg 1020

attcttgaat attgcaaaac tcttgatgga agaaggcagc aaagcagatg tgaatgctca 1080

agataatgaa gaccatgtcc cactccattt ctgttctcga tttggacacc atgatatagt 1140

taagtatctg ctgcaaagtg atttggaagt tcaacctcat gttgttaata tctatggaga 1200

tacccccctta cacctggcat gctacaatgg caaatttgaa gttgccaagg aaatcatcca 1260

aatatcagga acagaaagtc tgactaagga aaacatcttc agtgaaacag cttttcatag 1320

tgcttgtacc tatggcaaga gcattgacct agtcaaattt cttcttgatc agaatgtcat 1380

aaacatcaac caccaaggaa gggatgggca cactggatta cactctgctt gctaccacgg 1440

tcacattcgc ctggttcagt tcttactgga taatggagct gatatgaatc tagtggcttg 1500

tgatcccagc aggtctagtg gtgaaaaaga tgagcagaca tgtttgatgt gggcttatga 1560

aaaagggcat gatgccattg tcacactcct gaagcattat aagagaccac aagatgaatt 1620

gccctgtaat gaatattctc agcctggagg agatggctcc tatgtgtctg ttccatcacc 1680

cttggggaag attaaaagca tgacaaaaga gaaggcagat attctcctcc taagagctgg 1740

attgccttca catttccatc ttcagctctc agaaattgag ttccatgaga ttattggctc 1800

aggttctttt gggaaagtat ataaaggacg atgcagaaat aaaatagtgg ctataaaacg 1860

ttatcgagcc aatacctact gctccaagtc agatgtggat atgttttgcc gagaggtgtc 1920

cattctctgc cagctcaatc atccctgcgt aattcagttt gtgggtgctt gcttgaatga 1980

tcccagccag tttgccattg tcactcaata catatcaggg ggttctctgt tctccctcct 2040

tcatgagcag aagaggattc ttgatttgca gtctaaatta attattgcag tagatgttgc 2100

caaaggcatg gagtaccttc acaacctgac acagccaatt atacatcgtg acttgaacag 2160

ggcttcctga actatgtatg tgattgctag agctcaagca gccatctttg aacatgaggc 2220

agtgcatcag aaagagcaga gcagcaagaa agaaggcatc tcagttccta acgaatgaag 2280

tcaccacatc agccatagaa gcctgcctct aggactctga aataaaaaag agaaataaac 2340

tcttagttta ag 2352

<210> 166

<211> 2339
<212> DNA
<213> Homo sapiens

<400> 166

```
gtgctgtgcg gcgcggtctc agggaaggtg gggctatggc agctgctagg gaccctccgg      60
aagtatcgct gcgagaagcc acccagcgaa aattgcggag gttttccgag ctaagaggca     120
aacttgtagc acgtggagaa ttctgggaca tagttgcaat aacagcggct gatgaaaaac     180
aggaacttgc ttacaaccaa cagctgtcag aaaagctgaa aagaaaggag ttaccccttg     240
gagttcaata tcacgttttt gtagatcctg ctggagccaa aattggaaat ggaggatcaa     300
cactttgtgc ccttcaatgt ttggaaaagc tatatggaga taaatggaat tcttttacca     360
tcttattaat tcactctgat gaatggaaga aaaaagtcag tgaatcatat gttatcacaa     420
tagaaagatt agaagatgac ctgcagatca aggaaaaaga actgacagaa ctaaggaata     480
tatttggctc tgatgaagcc ttcagtaaag tcaatttaaa ttaccgcact gaaaatgggc     540
tgtctctact tcatttatgt tgcatttgtg gaggcaagaa atcacatatt cgaactctta     600
tgttgaaagg gctccgccca tctcgactga caagaaatgg atttacagcc ttgcatttag     660
cagtttacaa ggataatgca gaattgatca cttctctgct tcacagtgga gctgatatac     720
agcaggttgg atacggtggc ctcactgccc tccatattgc tacaatagct ggccacctag     780
aggctgctga tgtgctgttg caacatggag ctaatgtcaa tattcaagat gcagtttttt     840
tcactccatt gcatattgca gcgtactatg gacatgaaca ggtaactcgc cttcttttga     900
aatttggtgc tgatgtaaat gtaagtggtg aagttggaga tagacccctc cacctagcat     960
ctgcaaaagg attcttgaat attgcaaaac tcttgatgga agaaggcagc aaagcagatg    1020
tgaatgctca agataatgaa gaccatgtcc cactccattt ctgttctcga tttggacacc    1080
atgatatagt taagtatctg ctgcaaagtg atttggaagt tcaacctcat gttgttaata    1140
tctatggaga taccccctta cacctggcat gctacaatgg caaatttgaa gttgccaagg    1200
aaatcatcca aatatcagga acagaaagtc tgactaagga aaacatcttc agtgaaacag    1260
cttttcatag tgcttgtacc tatggcaaga gcattgacct agtcaaattt cttcttgatc    1320
agaatgtcat aaacatcaac caccaaggaa gggatgggca cactggatta cactctgctt    1380
gctaccacgg tcacattcgc ctggttcagt tcttactgga taatggagct gatatgaatc    1440
tagtggcttg tgatcccagc aggtctagtg gtgaaaaaga tgagcagaca tgtttgatgt    1500
gggcttatga aaaagggcat gatgccattg tcacactcct gaagcattat aagagaccac    1560
aagatgaatt gccctgtaat gaatattctc agcctggagg agatggctcc tatgtgtctg    1620
ttccatcacc cttggggaag attaaaagca tgacaaaaga gaaggcagat attctcctcc    1680
taagagctgg attgccttca catttccatc ttcagctctc agaaattgag ttccatgaga    1740
```

ttattggctc aggttctttt gggaaagtat ataaaggacg atgcagaaat aaaatagtgg 1800

ctataaaacg ttatcgagcc aatacctact gctccaagtc agatgtggat atgttttgcc 1860

gagaggtgtc cattctctgc cagctcaatc atccctgcgt aattcagttt gtgggtgctt 1920

gcttgaatga tcccagccag tttgccattg tcactcaata catatcaggg ggttctctgt 1980

tctccctcct tcatgagcag aagaggtatg ggtcttttgt tctgatttat ccttggacat 2040

tccgtagaaa ctactcttgc aatacttcag agggtttccc attggatgag ccttcacctt 2100

ttgaaatctg agtgctgcta gcaagtgtgt catttttaat atcgctaata atttatgcct 2160

aataactgtg tttcaatgaa tagaaatact ttatgctttc ttagcagaaa ttcagttgaa 2220

gataacaaat ttaaggaatt taaattggaa actggagctt aaaataatta ggctaaattt 2280

gaaatactga taaagaagat atatgtaagc tcagaataaa ataaactttt aaaaaagtg 2339

<210> 167
<211> 2389
<212> DNA
<213> Homo sapiens

<400> 167

cttgaaaaca ggtctatgta gttttaacag ctcttttgtg aacttcggat gtaactgaat 60

cattttctag atttatctgt taaataaatg gtccatgaaa gtatgtatgg gtagataatt 120

gatggaagca aataatccta ttgaaacatt tgctatttat ttttgataca gttcttttaa 180

acagactgat tagttccctt tatgatttca ccctcgaaag cacacacgat tttgttcttt 240

atcattgttg catgacattc catttggcag ttgggcttat ttttagccag actatcaaag 300

tatttttcaa ctggactgtc actgcacttg aacttggaat cttataactt gaagaactgc 360

cctggagaaa ggaagaaact tataataaat gggaaattat aaatctagac caacccaaac 420

ttgtactgat gaatggaaga aaaaagtcag tgaatcatat gttatcacaa tagaaagatt 480

agaagatgac ctgcagatca aggaaaaaga actgacagaa ctaaggaata tatttggctc 540

tgatgaagcc ttcagtaaag tcaatttaaa ttaccgcact gaaaatgggc tgtctctact 600

tcatttatgt tgcatttgtg gaggcaagaa atcacatatt cgaactctta tgttgaaagg 660

gctccgccca tctcgactga caagaaatgg atttacagcc ttgcatttag cagtttacaa 720

ggataatgca gaattgatca cttctctgct tcacagtgga gctgatatac agcaggttgg 780

atacggtggc ctcactgccc tccatattgc tacaatagct ggccacctag aggctgctga 840

tgtgctgttg caacatggag ctaatgtcaa tattcaagat gcagtttttt tcactccatt 900

gcatattgca gcgtactatg gacatgaaca ggtaactcgc cttcttttga aatttggtgc 960

tgatgtaaat gtaagtggtg aagttggaga tagacccctc cacctagcat ctgcaaaagg 1020

```
attcttgaat attgcaaaac tcttgatgga agaaggcagc aaagcagatg tgaatgctca   1080
agataatgaa gaccatgtcc cactccattt ctgttctcga tttggacacc atgatatagt   1140
taagtatctg ctgcaaagtg atttggaagt tcaacctcat gttgttaata tctatggaga   1200
taccccctta cacctggcat gctacaatgg caaatttgaa gttgccaagg aaatcatcca   1260
aatatcagga acagaaagtc tgactaagga aaacatcttc agtgaaacag cttttcatag   1320
tgcttgtacc tatggcaaga gcattgacct agtcaaattt cttcttgatc agaatgtcat   1380
aaacatcaac caccaaggaa gggatgggca cactggatta cactctgctt gctaccacgg   1440
tcacattcgc ctggttcagt tcttactgga taatggagct gatatgaatc tagtggcttg   1500
tgatcccagc aggtctagtg gtgaaaaaga tgagcagaca tgtttgatgt gggcttatga   1560
aaaagggcat gatgccattg tcacactcct gaagcattat aagagaccac aagatgaatt   1620
gccctgtaat gaatattctc agcctggagg agatggctcc tatgtgtctg ttccatcacc   1680
cttggggaag attaaaagca tgacaaaaga gaaggcagat attctcctcc taagagctgg   1740
attgccttca catttccatc ttcagctctc agaaattgag ttccatgaga ttattggctc   1800
aggttctttt gggaaagtat ataaaggacg atgcagaaat aaaatagtgg ctataaaacg   1860
ttatcgagcc aatacctact gctccaagtc agatgtggat atgttttgcc gagaggtgtc   1920
cattctctgc cagctcaatc atccctgcgt aattcagttt gtgggtgctt gcttgaatga   1980
tcccagccag tttgccattg tcactcaata catatcaggg ggttctctgt tctccctcct   2040
tcatgagcag aagaggtatg ggtcttttgt tctgatttat ccttggacat tccgtagaaa   2100
ctactcttgc aatacttcag agggtttccc attggatgag ccttcacctt ttgaaatctg   2160
agtgctgcta gcaagtgtgt catttttaat atcgctaata atttatgcct aataactgtg   2220
tttcaatgaa tagaaatact ttatgctttc ttagcagaaa ttcagttgaa gataacaaat   2280
ttaaggaatt taaattggaa actggagctt aaaataatta ggctaaattt gaaatactga   2340
taaagaagat atatgtaagc tcagaataaa ataaactttt aaaaaagtg              2389
```

<210> 168
<211> 1829
<212> DNA
<213> Homo sapiens

<400> 168

```
cttgaaaaca ggtctatgta gttttaacag ctcttttgtg aacttcggat gtaactgaat     60
cattttctag atttatctgt taaataaatg gtccatgaaa gtatgtatgg gtagataatt    120
gatggaagca aataatccta ttgaaacatt tgctatttat ttttgataca gttcttttaa    180
acagactgat tagttccctt tatgatttca ccctcgaaag cacacacgat tttgttcttt    240
atcattgttg catgacattc catttggcag ttgggcttat ttttagccag actatcaaag    300
```

```
tatttttcaa ctggactgtc actgcacttg aacttggaat cttataactt gaagaactgc     360
cctggagaaa ggaagaaact tataataaat gggaaattat aaatctagac caacccaaac     420
ttgtactgat gaatggaaga aaaaagtcag tgaatcatat gttatcacaa tagaaagatt     480
agaagatgac ctgcagatca aggaaaaaga actgacagaa ctaaggaata tatttggctc     540
tgatgaagcc ttcagtaaag tcaatttaaa ttaccgcact gaaaatgggc tgtctctact     600
tcatttatgt tgcatttgtg gaggcaagaa atcacatatt cgaactctta tgttgaaagg     660
gctccgccca tctcgactga caagaaatgg atttacagcc ttgcatttag cagtttacaa     720
ggataatgca gaattgatca cttctctgct tcacagtgga gctgatatac agcaggttgg     780
atacggtggc ctcactgccc tccatattgc tacaatagct ggccacctag aggctgctga     840
tgtgctgttg caacatggag ctaatgtcaa tattcaagat gcagtttttt tcactccatt     900
gcatattgca gcgtactatg gacatgaaca ggtaactcgc cttcttttga aatttggtgc     960
tgatgtaaat gtaagtggtg aagttggaga tagacccctc cacctagcat ctgcaaaagg    1020
attcttgaat attgcaaaac tcttgatgga agaaggcagc aaagcagatg tgaatgctca    1080
agataatgaa gaccatgtcc cactccattt ctgttctcga tttggacacc atgatatagt    1140
taagtatctg ctgcaaagtg atttggaagt tcaacctcat gttgttaata tctatggaga    1200
tacccccttta cacctggcat gctacaatgg caaatttgaa gttgccaagg aaatcatcca    1260
aatatcagga acagaaagtc tgactaagga aaacatcttc agtgaaacag cttttcatag    1320
tgcttgtacc tatggcaaga gcattgacct agtcaaattt cttcttgatc agaatgtcat    1380
aaacatcaac caccaaggaa gggatgggca cactggatta cactctgctt gctaccacgg    1440
tcacattcgc ctggttcagt tcttactgga taatggagct gatatgaatc tagtggcttg    1500
tgatcccagc aggtctagtg gtgaaaaaga tgagcagaca tgtttgatgt gggcttatga    1560
aaaagggcat gatgccattg tcacactcct gaagcattat aagagaccac aagatgaatt    1620
gccctgtaat gaatattctc agcctggagg agatggctcc tatgtgtctg ttccatcacc    1680
cttggggaag attaaaagca tgacaaaaga gaaggcagat attctcctcc taagagctgg    1740
attgccttca catttccatc ttcagctctc agaaattgag ttccatgaga ttattggctc    1800
aggtaaccta aaataaataa ataaataaa                                      1829
```

```
<210>  169
<211>  1671
<212>  DNA
<213>  Homo sapiens

<400> 169

acctgatcca gccagtgata aatctacttc ataggaatat tctaggaact cctcgggagc       60
```

```
catttctctc ctcagccatg agcacactat ttatgggcct aatttagtaa cttcctaaat    120
tacaacctga agacttgaag gaggtgcctc ccacagtgtg tgattcactc ccagggagac    180
atagggtccc tggatgaatc catggttggc tattctgaac cttgtaaggc ttctaaggga    240
ggaagatgtt tatagattta aaaaaataat gctgcttctc attgtataag aggcagactt    300
acttgatgat taattacttg tgtgatgttt taggaagaat tatgaacttt atgatcttcc    360
aatgattaaa aaatgccctt tgaggaactg aacagccaca gtaaacacac tgtgtatgta    420
taaacatgtt tattgcacac actgagggca atatgtatgg atgttaattt atatttatgc    480
cttttgagag catcgggaga acacagtaaa ttctcactaa gaagaatgct actctgcagt    540
ggaagaacca aacacttggt aaatggcttg tggatgtttc ttgatgtgca gaacctccgt    600
tggatggctc ctgaggtgtt cacgcagtgc actcggtaca ccatcaaagc agatgtcttc    660
agctatgctc tgtgtctgtg ggaaattctc actggcgaaa ttccattcgc tcatctcaag    720
ccagcggctg cggcagcaga catggcttac caccacatca gacctcccat tggctattcc    780
attcccaagc ccatatcatc tctgctgata cgagggtgga acgcatgtcc tgaaggaaga    840
cccgaatttt ctgaagttgt catgaagtta gaagagtgtc tctgcaacat tgagctgatg    900
tctcctgcat caagtaacag cagtgggtct ctctcacctt cttcttcttc tgattgcctg    960
gtgaaccggg gaggacctgg ccggagtcat gtggcagcat taagaagtcg tttcgaattg   1020
gaatatgctc taaatgcaag gtcctatgct gctttgtccc aaagtgctgg acaatattcc   1080
tctcaaggtc tgtctttgga ggagatgaaa agaagtcttc aatacacacc cattgacaaa   1140
tatggctatg tatccgatcc catgagctca atgcattttc attcttgccg aaatagtagc   1200
agctttgagg acagcagctg acagcattcg gcgtatacct aaggagagtt ttttccccga   1260
actgacagca acgattccaa ccacggcaag ctggcttcca actataacat tttactctca   1320
aaggtctcct taaattgggc ttgtttttac ttgtcctatt taattcccca ctattagcag   1380
gctttggatt tgtgcctaag gaataatatg caaaagaacc aagacagaat gtatatgaag   1440
aattgttttt aattttgtaa attaaaaaaa aatttagatc gttacttgga aatggagcct   1500
aagtctgtgg tggacagata ataattatgt tttcctgggc tgaattatgt agacttgtgt   1560
ttgacagcta tgggtttatt tcttagaaca ttgttcattt tcttttctca ttatgttact   1620
tctagtgttc acctctgtga ttaaagattc tttggtgaaa tagaaaattg a            1671
```

<210> 170
<211> 1745
<212> DNA
<213> Homo sapiens

<400> 170

```
acctgatcca gccagtgata aatctacttc ataggaatat tctaggaact cctcgggagc     60
```

catttctctc ctcagccatg agcacactat ttatgggcct aatttagtaa cttcctaaat 120
tacaacctga agacttgaag gaggtgcctc ccacagtgtg tgattcactc ccagggagac 180
atagggtccc tggatgaatc catggttggc tattctgaac cttgtaaggc ttctaaggga 240
ggaagatgtt tatagattta aaaaaataat gctgcttctc attgtataag aggcagactt 300
acttgatgat taattacttg tgtgatgttt taggaagaat tatgaacttt atgatcttcc 360
aatgattaaa aaatgccctt tgaggaactg aacagccaca gtaaacacac tgtgtatgta 420
taaacatgtt tattgcacac actgagggca atatgtatgg atgttaattt atatttatgc 480
cttttgagag catcgggaga acacagtaaa ttctcactaa gaagaatgct actctgcagt 540
ggaagaacca aacacttggt aaatggcttg tggatgtttc ttgatgtgca gaacctccgt 600
tggatggctc ctgaggtgtt cacgcagtgc actcggtaca ccatcaaagc agatgtcttc 660
agctatgctc tgtgtctgtg ggaaattctc actggcgaaa ttccattcgc tcatctcaag 720
ccagcggctg cggcagcaga catggcttac caccacatca gacctcccat tggctattcc 780
attcccaagc ccatatcatc tctgctgata cgagggtgga acgcatgtcc tgaaggaaga 840
cccgaatttt ctgaagttgt catgaagtta gaagagtgtc tctgcaacat tgagctgatg 900
tctcctgcat caagtaacag cagtgggtct ctctcacctt cttcttcttc tgattgcctg 960
gtgaaccggg gaggacctgg ccggagtcat gtggcagcat taagaagtcg tttcgaattg 1020
gaatatgctc taaatgcaag gtcctatgct gctttgtccc aaagtgctgg acaatattcc 1080
tctcaaggtc tgtctttgga ggagatgaaa agaagtcttc aatacacacc cattgacaaa 1140
tatgatgtaa cttcataaac acaaaacata gggcatcctt tgaaacattt gctttgacca 1200
gaagtcttcc cttttgtggc tatgtatccg atcccatgag ctcaatgcat tttcattctt 1260
gccgaaatag tagcagcttt gaggacagca gctgacagca ttcggcgtat acctaaggag 1320
agtttttttcc ccgaactgac agcaacgatt ccaaccacgg caagctggct tccaactata 1380
acattttact ctcaaaggtc tccttaaatt gggcttgttt ttacttgtcc tatttaattc 1440
cccactatta gcaggctttg gatttgtgcc taaggaataa tatgcaaaag aaccaagaca 1500
gaatgtatat gaagaattgt ttttaatttt gtaaattaaa aaaaaattta gatcgttact 1560
tggaaatgga gcctaagtct gtggtggaca gataataatt atgttttcct gggctgaatt 1620
atgtagactt gtgtttgaca gctatgggtt tatttcttag aacattgttc attttctttt 1680
ctcattatgt tacttctagt gttcacctct gtgattaaag attctttggt gaaatagaaa 1740
attga 1745

<210> 171
<211> 168
<212> DNA

<213>  Homo sapiens

<400> 171

gcaaacttgt agcacgtgga gaattctggg acatagttgc aataacagcg gctgatgaaa 60

aacaggaact tgcttacaac caacagctgt cagaaaagct gaaaagaaag gagttacccc 120

ttggagttca atatcacgtt tttgtagatc ctgctggagc caaaattg 168

<210>  172
<211>  428
<212>  DNA
<213>  Homo sapiens

<400> 172

cttgaaaaca ggtctatgta gttttaacag ctcttttgtg aacttcggat gtaactgaat 60

cattttctag atttatctgt taaataaatg gtccatgaaa gtatgtatgg gtagataatt 120

gatggaagca aataatccta ttgaaacatt tgctatttat ttttgataca gttcttttaa 180

acagactgat tagttccctt tatgatttca ccctcgaaag cacacacgat tttgttcttt 240

atcattgttg catgacattc catttggcag ttgggcttat ttttagccag actatcaaag 300

tatttttcaa ctggactgtc actgcacttg aacttggaat cttataactt gaagaactgc 360

cctggagaaa ggaagaaact tataataaat gggaaattat aaatctagac caacccaaac 420

ttgtactg 428

<210>  173
<211>  139
<212>  DNA
<213>  Homo sapiens

<400> 173

gtaactcgcc ttcttttgaa atttggtgct gatgtaaatg taagtggtga agttggagat 60

agacccctcc acctagcatc tgcaaaagga ttcttgaata ttgcaaaact cttgatggaa 120

gaaggcagca aagcagatg 139

<210>  174
<211>  145
<212>  DNA
<213>  Homo sapiens

<400> 174

tgaatgctca agataatgaa gaccatgtcc cactccattt ctgttctcga tttggacacc 60

atgatatagt taagtatctg ctgcaaagtg atttggaagt tcaacctcat gttgttaata 120

tctatggaga tacccccttta cacct 145

<210> 175
<211> 150
<212> DNA
<213> Homo sapiens

<400> 175

gattacactc tgcttgctac cacggtcaca ttcgcctggt tcagttctta ctggataatg 60

gagctgatat gaatctagtg gcttgtgatc ccagcaggtc tagtggtgaa aaagatgagc 120

agacatgttt gatgtgggct tatgaaaaag 150

<210> 176
<211> 195
<212> DNA
<213> Homo sapiens

<400> 176

ttatcgagcc aatacctact gctccaagtc agatgtggat atgttttgcc gagaggtgtc 60

cattctctgc cagctcaatc atccctgcgt aattcagttt gtgggtgctt gcttgaatga 120

tcccagccag tttgccattg tcactcaata catatcaggg ggttctctgt tctccctcct 180

tcatgagcag aagag 195

<210> 177
<211> 334
<212> DNA
<213> Homo sapiens

<400> 177

gtatgggtct tttgttctga tttatccttg gacattccgt agaaactact cttgcaatac 60

ttcagagggt ttcccattgg atgagccttc accttttgaa atctgagtgc tgctagcaag 120

tgtgtcattt ttaatatcgc taataattta tgcctaataa ctgtgtttca atgaatagaa 180

atactttatg ctttcttagc agaaattcag ttgaagataa caaatttaag gaatttaaat 240

tggaaactgg agcttaaaat aattaggcta aatttgaaat actgataaag aagatatatg 300

taagctcaga ataaaataaa cttttaaaaa agtg 334

<210> 178
<211> 5172
<212> DNA
<213> Homo sapiens

<400> 178

gtattttttt cctaaataat gaactcagaa gggtatgact aactgggagt ttaagacaga 60

tttcagtgaa gatacatttt agacttattg cagatcaggg tactctgtgg ttaagaggac 120

aggctaccat aagcttaggc gatagaaagt tttgtcattg tcttttaagg agtagaaagc 180

```
tgtataactg cttctcaggg atcttttaaa tctatgaaga atattcacaa atatagtaat    240
atctgtaaga tgcaccagct aactagttcc caggtcagta aggtcacatc aagcagtgag    300
ctaaatggct attaaaaaaa tgatatgtct cctataaagt gaagtgttgg agtagctcct    360
gatataaaat tatctgaaac gtactttgtg ctaccgtaaa ttcagcttaa ccacaacagc    420
ttatttacct agttatcagg taaagaggat ttcatgtgaa acaaatataa aatcagagtg    480
ttccagtggt ggaaccccaa gtgacctcaa aacttttcct aaatagtgaa aactttctct    540
gagtaaagtc ttataggaaa ggccaatata caaaatggag cagtgttggg tgaaggtaaa    600
gtggggagga gaaaactcta tttacttccc atttcccact gctaaggaga gagattcctg    660
caccacctct gtggaagccc aaaggtgctg tagagaatag ttggacaaac actgatttag    720
tttatgaagc aagtcagatt attccacatc aatcactttc attttatgat agtcaataag    780
attcaaatag attgtgtata tagtccttta tagaagcttt acagttctga agctagtaaa    840
tattgattac tctagtcagt catttggtca acaactatgt ttgaacatat atgtttaaag    900
actattttca gcacagtaga gcttacaaag tgggacgttc atagactaca ttcagggagt    960
tttcacactc tatgtagagg agaaaaatat ttacctaaat gtatttgagg tataaaataa   1020
taagttatat acaagattta gtacaaaaat ttcaggaaca agaatattaa ttgcttctga   1080
ggattctaac aacaactcta tggaagggat gacatatgag cttggtcctg aaagatgggt   1140
agtttggaag agcatcctat aggcttaggg aagacataat aaaatgcata gagattagaa   1200
agttaaaggt atatatagga actatagttt cattttagta tacaaagtgt ataaatgtat   1260
gtaatgtgta taatgtaaga agtggacaac tggaaaaagg tatagaaaaa tagaaaaaga   1320
gcacagacac cagtgaatgc tattactaag gtatttcaga ttttttttcc tgtaaggaga   1380
ccataaagag gtttgagctg aaaatcacaa atttggagct atattattga aagatgaatc   1440
cagcatctgg taaaatatca acttgaaggg aaagtcaaga aactagtgag ggagttgttg   1500
cagtaatcta gataagaagt aacgaaaagc agaatttcac cggtggcagt ggatatgaaa   1560
ggaagagaca tgtcaacttt aaataacaaa atacagagtt tattcaagct caaagcttga   1620
ggatcattac ccatgagcat ggactcaagt tgtcctgaat acatactcca attagcagtg   1680
gttacaagat agtttttaag aaaaaaaaaa aaaaaaaaaa aagcagtttt taagttgttt   1740
acaaagaatt tacataaata atataaacta ttgattggtt gtaccttgtt ctttaatcaa   1800
aaattatagg aacataaaga taatgggtaa cacagctagt caggaacaaa atacctttac   1860
actattgccc cagggaaatg gagattggag agatgactaa agtcccatgc tcatgtctct   1920
ctgggcctga taaattttgt gttgctcata cagctcagac tgccctcagc tatttttctc   1980
ttctcagata gatatgaaag ataggagaga taaatcagat acgtgagtgg tagaatctta   2040
aatattgaaa gtaaatgtaa ataagtctca atctatttat aattatttac tagttttcac   2100
```

ataaaagtca tttcttaatt tttagctatt agttctttac ctttcttaat ttcaaaccaa 2160
acatttacgt taacaaacat ttaactattt cagacacaga tttagatgat aaattagcat 2220
aatataaacc gatgattttg agttttaaga gtattatctt tccatcccaa acatttttgc 2280
acatggtatt aaaacaaatg agtaatcatt gcaataagta atcattgcaa gttttctcta 2340
gctgtaactc ttcatttgtg gggccattat atattctgtt gccctgagtt atgtgcaagt 2400
tgttaccacc agttgtcagg caggaccaaa aatataaatg ttgattaaaa taaaaacaga 2460
acatttacta tatagctgat ataagtttat atctacaatt tttgctgcct ctaatctaac 2520
ctaaatgctt aattgcatca aaaagttttt aaatcttatg aaacaccatt ttgtaggtgc 2580
cttcattatg acagagtccc aaactattgt atcaattatt tgtagactat tttctatcaa 2640
tctttaatct ggtagcaaca gcaaaacatt gtttttcaga tgcatgcata tgtagcattc 2700
tacatggctc aaaagagcct gggtcctttc cctcagagag cttactgcac tacttggcaa 2760
ataagacata tgcccagaaa gcaactagag ttccacttta tatagaaaat aattaagggt 2820
caaaatgatg aaagagcaac tttcagagga tttcaagtaa gtcaaagatg aaagttgagt 2880
agaatgcttc ttagcctatg tggagctaaa cgtagagttt gaagaacagg cattaatctt 2940
ttctcaggct tttcacagca tactaacctc tgctttagca tggtacctag aaagtctcac 3000
tcaaagaagt agagagaaag atgcacatca gagactggga aactcataca cttatttgca 3060
tttgtattga catggtgtgt ttatattttt atattcccca ttagcctggg caagtcaggg 3120
tttcaggggc agagactgct ctcttcatct cacattttct gaactaagca ttgtgtctta 3180
cacagagaag atgtctaatt aatatttttt aactgaacca aattattgtg gatttctatg 3240
agtgtcattt cacgaagcaa tgacctaatt ggtataagta tgactacttg tgtatagcat 3300
ccaacttgcc caataacttt tataatttta aagggaaaag gatgtttgag cccacgggat 3360
ggggcacttt tatatttgag tttgtggttg ttgcaattgt ttgagtgaaa aaaaagaact 3420
ctacttttcc catggcaagc aaagccagct gagggtttgt accatccagt tctgaaagtt 3480
aattttgatc ttcagtaaat gtcccttggc atctgcaggg gattggttcc aggacctctc 3540
atggatacca aagtctgaga atgctcaagt ccctggtatt aattgctgta gcatttgcat 3600
atttcctatg cacatcctct cctgtacttt aaatcattga aagagtacct ataatatcta 3660
gtacaatgta aatgctctgt aaatagtttt tgcactatat tgtctaggga ataatgacaa 3720
aggaaaaaga gtctatacat cttcagtaca gacacaatct ttaaaaaaat tttttgatcc 3780
atggttagtt gaatccacag aagtgaaacc tatggattta gagggccaac tatatatact 3840
aaggaaaagt tcaaggaagg agtaagaaag tagttatttt caatgtaaat gatgataatg 3900
ataataccta ccatttattt atcaccatat gaaaggagct ttgctaagtt tttcatgctt 3960

tcatttaacc acgcaactct atgggatagt tgttattatt tccattttac agataaggaa 4020

actgactaaa gagactaagt ctgtagtatt ctgtatcttc tactgtaaca acaacaacag 4080

caaaaccatg tgagaagaga gagatagaag gagcgtactg atgatattga atgggcatca 4140

gcaatattta tcagccttac tctctgtact ttaatctttt aaaaagtcaa ttcctgtgac 4200

ccaaacaatt tctcatatca tcttatgctt ctgtgttcct gctttatttc ttctttcaca 4260

ctccatgttc tgctggtgtg gaacagatgt tgcatagaaa gcatgcagat aggttttgtt 4320

tggcccatag tgtttaaata ttttgaaaat gttcataatt caagcaggaa tgtacctctc 4380

taatttgctg tagacctcac cacctaccaa cttatatttg gtctaattta tacattcatt 4440

ttgtggcatt ggagtttgct agtcattatt tatagcttaa aggatttaaa ctttttactt 4500

aatactagaa gctgtgtgtt tttcttggaa aagtatattt ttcaaattgt ttgacactga 4560

agcagaaaca tcactgcaaa catacatgga tcaaatacat gatattctcc ttctataaag 4620

atatagtatt atatgttata ttaacactgt aaggaattaa atagtgataa cctccatcac 4680

aaagtgcaca tggcattcac atgaaatatg tatatgaaat cattttataa actgtgaagc 4740

accatacaaa atgcattttt tattactctt aattcctcat ttgttccctt taatattggg 4800

gatggaaaaa cactccacaa ttaaaaccac ctttgcaaaa actacaactg agaaaattat 4860

gactgaaaga tctgacttga ctccactttg cttctaacct gcaagctgtt cttgttcatt 4920

catggacata ggccaaaata actttgggag gaacttgaaa ctaagacaat aatagccctt 4980

tcacaaaaca aacctctttc ctgcctgggg actacactac cttcatagga ctaacaaatt 5040

agccacaaga ttagaaataa tggtttagga atcacatagc tggagactga aagattcgaa 5100

acctccccag attgcttctg gggataaatc actattgcaa aacctaagat cagtgcttga 5160

gatattttgc ag 5172

<210> 179
<211> 284
<212> DNA
<213> Homo sapiens

<400> 179

atgctgtaca ggatggctca gctgctacca cccagactga taaactgggt catctggtct 60

cctggcccca ggagactgac tcagcatcaa gaggacaact ttgacaccct atgatatcat 120

ctccaaccca accaatcagc agtccccact ttcagacccc tacccaccaa gttttcctta 180

aaaccccat cctcaagttt ttgagaagac tgatttgagt aataataaca ctcttctctc 240

ctgtacagct ggctgtgcgt gaattaaact tttctctatt gcaa 284

<210> 180
<211> 192

<212> DNA
<213> Homo sapiens

<400> 180

ggcttcctga actatgtatg tgattgctag agctcaagca gccatctttg aacatgaggc 60

agtgcatcag aaagagcaga gcagcaagaa agaaggcatc tcagttccta acgaatgaag 120

tcaccacatc agccatagaa gcctgcctct aggactctga aataaaaaag agaaataaac 180

tcttagttta ag 192

<210> 181
<211> 591
<212> DNA
<213> Homo sapiens

<400> 181

acctgatcca gccagtgata aatctacttc ataggaatat tctaggaact cctcgggagc 60

catttctctc ctcagccatg agcacactat ttatgggcct aatttagtaa cttcctaaat 120

tacaacctga agacttgaag gaggtgcctc ccacagtgtg tgattcactc ccagggagac 180

atagggtccc tggatgaatc catggttggc tattctgaac cttgtaaggc ttctaaggga 240

ggaagatgtt tatagattta aaaaaataat gctgcttctc attgtataag aggcagactt 300

acttgatgat taattacttg tgtgatgttt taggaagaat tatgaacttt atgatcttcc 360

aatgattaaa aaatgccctt tgaggaactg aacagccaca gtaaacacac tgtgtatgta 420

taaacatgtt tattgcacac actgagggca atatgtatgg atgttaattt atatttatgc 480

cttttgagag catcgggaga acacagtaaa ttctcactaa gaagaatgct actctgcagt 540

ggaagaacca aacacttggt aaatggcttg tggatgtttc ttgatgtgca g 591

<210> 182
<211> 133
<212> DNA
<213> Homo sapiens

<400> 182

aacctccgtt ggatggctcc tgaggtgttc acgcagtgca ctcggtacac catcaaagca 60

gatgtcttca gctatgctct gtgtctgtgg gaaattctca ctggcgaaat tccattcgct 120

catctcaagc cag 133

<210> 183
<211> 291
<212> DNA
<213> Homo sapiens

<400> 183

gcaaaatcca gaccaagtca ttacccagtc tcatctgtgt acacagaaac tcttaagaag 60

aaaaatgaag atcgttttgg gatgtggatt gagtatctca gaagataacc tcttatcctg 120

gccattcaac ctgatgtgtt acatgtttat ttgtttagaa tcttccatca ctaccaaaat 180

gttagctcca tgaaagcggt cctttttgtt tattttgttc actgctataa cactaacatc 240

taaagctgga tgcttaagaa tgtttgttga gtaaatgaat gaataccagt a 291

<210> 184
<211> 170
<212> DNA
<213> Homo sapiens

<400> 184

ctgatgtctc ctgcatcaag taacagcagt gggtctctct caccttcttc ttcttctgat 60

tgcctggtga accggggagg acctggccgg agtcatgtgg cagcattaag aagtcgtttc 120

gaattggaat atgctctaaa tgcaaggtcc tatgctgctt tgtcccaaag 170

<210> 185
<211> 527
<212> DNA
<213> Homo sapiens

<400> 185

gctatgtatc cgatcccatg agctcaatgc attttcattc ttgccgaaat agtagcagct 60

ttgaggacag cagctgacag cattcggcgt atacctaagg agagtttttt ccccgaactg 120

acagcaacga ttccaaccac ggcaagctgg cttccaacta taacatttta ctctcaaagg 180

tctccttaaa ttgggcttgt tttacttgt cctatttaat tccccactat tagcaggctt 240

tggatttgtg cctaaggaat aatatgcaaa agaaccaaga cagaatgtat atgaagaatt 300

gtttttaatt ttgtaaatta aaaaaaaatt tagatcgtta cttggaaatg gagcctaagt 360

ctgtggtgga cagataataa ttatgttttc ctgggctgaa ttatgtagac ttgtgtttga 420

cagctatggg tttatttctt agaacattgt tcattttctt ttctcattat gttacttcta 480

gtgttcacct ctgtgattaa agattctttg gtgaaataga aaattga 527

<210> 186
<211> 117
<212> DNA
<213> Homo sapiens

<400> 186

gtgctgtgcg gcgcggtctc agggaaggtg gggctatggc agctgctagg gaccctccgg 60

aagtatcgct gcgagaagcc acccagcgaa aattgcggag gttttccgag ctaagag 117

<210> 187
<211> 93
<212> DNA
<213> Homo sapiens

<400> 187

gaaatggagg atcaacactt tgtgcccttc aatgtttgga aaagctatat ggagataaat 60

ggaattcttt taccatctta ttaattcact ctg 93

<210> 188
<211> 109
<212> DNA
<213> Homo sapiens

<400> 188

atgaatggaa gaaaaaagtc agtgaatcat atgttatcac aatagaaaga ttagaagatg 60

acctgcagat caaggaaaaa gaactgacag aactaaggaa tatatttgg 109

<210> 189
<211> 86
<212> DNA
<213> Homo sapiens

<400> 189

ctctgatgaa gccttcagta aagtcaattt aaattaccgc actgaaaatg ggctgtctct 60

acttcattta tgttgcattt gtggag 86

<210> 190
<211> 98
<212> DNA
<213> Homo sapiens

<400> 190

gcaagaaatc acatattcga actcttatgt tgaaagggct ccgcccatct cgactgacaa 60

gaaatggatt tacagccttg catttagcag tttacaag 98

<210> 191
<211> 45
<212> DNA
<213> Homo sapiens

<400> 191

ccttctccat gtggcctgca ttttcttata ccatggctga cacag 45

<210> 192
<211> 111
<212> DNA
<213> Homo sapiens

<400> 192

gataatgcag aattgatcac ttctctgctt cacagtggag ctgatataca gcaggttgga 60

tacggtggcc tcactgccct ccatattgct acaatagctg gccacctaga g 111

<210> 193
<211> 99
<212> DNA
<213> Homo sapiens

<400> 193

gctgctgatg tgctgttgca acatggagct aatgtcaata ttcaagatgc agttttttttc 60

actccattgc atattgcagc gtactatgga catgaacag 99

<210> 194
<211> 105
<212> DNA
<213> Homo sapiens

<400> 194

ggcatgctac aatggcaaat ttgaagttgc caaggaaatc atccaaatat caggaacaga 60

aagtctgact aaggaaaaca tcttcagtga aacagctttt catag 105

<210> 195
<211> 95
<212> DNA
<213> Homo sapiens

<400> 195

tgcttgtacc tatggcaaga gcattgacct agtcaaattt cttcttgatc agaatgtcat 60

aaacatcaac caccaaggaa gggatgggca cactg 95

<210> 196
<211> 87
<212> DNA
<213> Homo sapiens

<400> 196

ggcatgatgc cattgtcaca ctcctgaagc attataagag accacaagat gaattgccct 60

gtaatgaata ttctcagcct ggaggag 87

<210> 197
<211> 57
<212> DNA
<213> Homo sapiens

<400> 197

atggctccta tgtgtctgtt ccatcaccct tggggaagat taaaagcatg acaaaag 57

<210> 198
<211> 93
<212> DNA
<213> Homo sapiens

<400> 198

agaaggcaga tattctcctc ctaagagctg gattgccttc acatttccat cttcagctct 60

cagaaattga gttccatgag attattggct cag 93

<210> 199
<211> 27
<212> DNA
<213> Homo sapiens

<400> 199

gtaacctaaa ataaataaat aaataaa 27

<210> 200
<211> 58
<212> DNA
<213> Homo sapiens

<400> 200

gttcttttgg gaaagtatat aaaggacgat gcagaaataa aatagtggct ataaaacg 58

<210> 201
<211> 105
<212> DNA
<213> Homo sapiens

<400> 201

gattcttgat ttgcagtcta aattaattat tgcagtagat gttgccaaag gcatggagta 60

ccttcacaac ctgacacagc caattataca tcgtgacttg aacag 105

<210> 202
<211> 119
<212> DNA
<213> Homo sapiens

<400> 202

gtctgccatt acctctagga tctggatcac ccatagtatt tgcatctgga ggggagctca 60

ttactttaac agggaagaat gcaatttcag gtgtatgctt acttctgcca tcctaaaag 119

<210> 203
<211> 53
<212> DNA
<213> Homo sapiens

<400> 203

tcacaatatt cttctctatg aggatgggca tgctgtggtg gcagattttg gag 53

<210> 204
<211> 53
<212> DNA
<213> Homo sapiens

<400> 204

aatcaagatt tctacagtct ctggatgaag acaacatgac aaaacaacct ggg 53

<210> 205
<211> 110
<212> DNA
<213> Homo sapiens

<400> 205

cggctgcggc agcagacatg gcttaccacc acatcagacc tcccattggc tattccattc 60

ccaagcccat atcatctctg ctgatacgag ggtggaacgc atgtcctgaa 110

<210> 206
<211> 60
<212> DNA
<213> Homo sapiens

<400> 206

ggaagacccg aattttctga agttgtcatg aagttagaag agtgtctctg caacattgag 60

<210> 207
<211> 80
<212> DNA
<213> Homo sapiens

<400> 207

tgctggacaa tattcctctc aaggtctgtc tttggaggag atgaaaagaa gtcttcaata 60

cacacccatt gacaaatatg 80

<210> 208
<211> 74
<212> DNA
<213> Homo sapiens

<400> 208

atgtaacttc ataaacacaa aacatagggc atcctttgaa acatttgctt tgaccagaag 60

tcttcccttt tgtg 74

<210> 209
<211> 936

<212> PRT
<213> Homo sapiens

<400> 209

```
Met Ala Ala Ala Arg Asp Pro Pro Glu Val Ser Leu Arg Glu Ala Thr
1               5                   10                  15

Gln Arg Lys Leu Arg Arg Phe Ser Glu Leu Arg Gly Lys Leu Val Ala
            20                  25                  30

Arg Gly Glu Phe Trp Asp Ile Val Ala Ile Thr Ala Ala Asp Glu Lys
        35                  40                  45

Gln Glu Leu Ala Tyr Asn Gln Gln Leu Ser Glu Lys Leu Lys Arg Lys
    50                  55                  60

Glu Leu Pro Leu Gly Val Gln Tyr His Val Phe Val Asp Pro Ala Gly
65                  70                  75                  80

Ala Lys Ile Gly Asn Gly Gly Ser Thr Leu Cys Ala Leu Gln Cys Leu
                85                  90                  95

Glu Lys Leu Tyr Gly Asp Lys Trp Asn Ser Phe Thr Ile Leu Leu Ile
            100                 105                 110

His Ser Asp Glu Trp Lys Lys Lys Val Ser Glu Ser Tyr Val Ile Thr
        115                 120                 125

Ile Glu Arg Leu Glu Asp Asp Leu Gln Ile Lys Glu Lys Glu Leu Thr
    130                 135                 140

Glu Leu Arg Asn Ile Phe Gly Ser Asp Glu Ala Phe Ser Lys Val Asn
145                 150                 155                 160

Leu Asn Tyr Arg Thr Glu Asn Gly Leu Ser Leu Leu His Leu Cys Cys
                165                 170                 175

Ile Cys Gly Gly Lys Lys Ser His Ile Arg Thr Leu Met Leu Lys Gly
            180                 185                 190

Leu Arg Pro Ser Arg Leu Thr Arg Asn Gly Phe Thr Ala Leu His Leu
        195                 200                 205

Ala Val Tyr Lys Asp Asn Ala Glu Leu Ile Thr Ser Leu Leu His Ser
    210                 215                 220

Gly Ala Asp Ile Gln Gln Val Gly Tyr Gly Gly Leu Thr Ala Leu His
225                 230                 235                 240
```

```
Ile Ala Thr Ile Ala Gly His Leu Glu Ala Ala Asp Val Leu Leu Gln
                245                 250                 255

His Gly Ala Asn Val Asn Ile Gln Asp Ala Val Phe Phe Thr Pro Leu
            260                 265                 270

His Ile Ala Ala Tyr Tyr Gly His Glu Gln Val Thr Arg Leu Leu Leu
        275                 280                 285

Lys Phe Gly Ala Asp Val Asn Val Ser Gly Glu Val Gly Asp Arg Pro
    290                 295                 300

Leu His Leu Ala Ser Ala Lys Gly Phe Leu Asn Ile Ala Lys Leu Leu
305                 310                 315                 320

Met Glu Glu Gly Ser Lys Ala Asp Val Asn Ala Gln Asp Asn Glu Asp
                325                 330                 335

His Val Pro Leu His Phe Cys Ser Arg Phe Gly His His Asp Ile Val
            340                 345                 350

Lys Tyr Leu Leu Gln Ser Asp Leu Glu Val Gln Pro His Val Val Asn
        355                 360                 365

Ile Tyr Gly Asp Thr Pro Leu His Leu Ala Cys Tyr Asn Gly Lys Phe
    370                 375                 380

Glu Val Ala Lys Glu Ile Ile Gln Ile Ser Gly Thr Glu Ser Leu Thr
385                 390                 395                 400

Lys Glu Asn Ile Phe Ser Glu Thr Ala Phe His Ser Ala Cys Thr Tyr
                405                 410                 415

Gly Lys Ser Ile Asp Leu Val Lys Phe Leu Leu Asp Gln Asn Val Ile
            420                 425                 430

Asn Ile Asn His Gln Gly Arg Asp Gly His Thr Gly Leu His Ser Ala
        435                 440                 445

Cys Tyr His Gly His Ile Arg Leu Val Gln Phe Leu Leu Asp Asn Gly
    450                 455                 460

Ala Asp Met Asn Leu Val Ala Cys Asp Pro Ser Arg Ser Ser Gly Glu
465                 470                 475                 480

Lys Asp Glu Gln Thr Cys Leu Met Trp Ala Tyr Glu Lys Gly His Asp
```

```
                485                 490                 495

Ala Ile Val Thr Leu Leu Lys His Tyr Lys Arg Pro Gln Asp Glu Leu
            500                 505                 510

Pro Cys Asn Glu Tyr Ser Gln Pro Gly Gly Asp Gly Ser Tyr Val Ser
        515                 520                 525

Val Pro Ser Pro Leu Gly Lys Ile Lys Ser Met Thr Lys Glu Lys Ala
    530                 535                 540

Asp Ile Leu Leu Leu Arg Ala Gly Leu Pro Ser His Phe His Leu Gln
545                 550                 555                 560

Leu Ser Glu Ile Glu Phe His Glu Ile Ile Gly Ser Gly Ser Phe Gly
                565                 570                 575

Lys Val Tyr Lys Gly Arg Cys Arg Asn Lys Ile Val Ala Ile Lys Arg
            580                 585                 590

Tyr Arg Ala Asn Thr Tyr Cys Ser Lys Ser Asp Val Asp Met Phe Cys
        595                 600                 605

Arg Glu Val Ser Ile Leu Cys Gln Leu Asn His Pro Cys Val Ile Gln
    610                 615                 620

Phe Val Gly Ala Cys Leu Asn Asp Pro Ser Gln Phe Ala Ile Val Thr
625                 630                 635                 640

Gln Tyr Ile Ser Gly Gly Ser Leu Phe Ser Leu Leu His Glu Gln Lys
                645                 650                 655

Arg Ile Leu Asp Leu Gln Ser Lys Leu Ile Ile Ala Val Asp Val Ala
            660                 665                 670

Lys Gly Met Glu Tyr Leu His Asn Leu Thr Gln Pro Ile Ile His Arg
        675                 680                 685

Asp Leu Asn Ser His Asn Ile Leu Leu Tyr Glu Asp Gly His Ala Val
    690                 695                 700

Val Ala Asp Phe Gly Glu Ser Arg Phe Leu Gln Ser Leu Asp Glu Asp
705                 710                 715                 720

Asn Met Thr Lys Gln Pro Gly Asn Leu Arg Trp Met Ala Pro Glu Val
                725                 730                 735
```

```
Phe Thr Gln Cys Thr Arg Tyr Thr Ile Lys Ala Asp Val Phe Ser Tyr
            740                 745                 750

Ala Leu Cys Leu Trp Glu Ile Leu Thr Gly Glu Ile Pro Phe Ala His
        755                 760                 765

Leu Lys Pro Ala Ala Ala Ala Ala Asp Met Ala Tyr His His Ile Arg
    770                 775                 780

Pro Pro Ile Gly Tyr Ser Ile Pro Lys Pro Ile Ser Ser Leu Leu Ile
785                 790                 795                 800

Arg Gly Trp Asn Ala Cys Pro Glu Gly Arg Pro Glu Phe Ser Glu Val
                805                 810                 815

Val Met Lys Leu Glu Glu Cys Leu Cys Asn Ile Glu Leu Met Ser Pro
            820                 825                 830

Ala Ser Ser Asn Ser Ser Gly Ser Leu Ser Pro Ser Ser Ser Ser Asp
        835                 840                 845

Cys Leu Val Asn Arg Gly Gly Pro Gly Arg Ser His Val Ala Ala Leu
    850                 855                 860

Arg Ser Arg Phe Glu Leu Glu Tyr Ala Leu Asn Ala Arg Ser Tyr Ala
865                 870                 875                 880

Ala Leu Ser Gln Ser Ala Gly Gln Tyr Ser Ser Gln Gly Leu Ser Leu
                885                 890                 895

Glu Glu Met Lys Arg Ser Leu Gln Tyr Thr Pro Ile Asp Lys Tyr Gly
            900                 905                 910

Tyr Val Ser Asp Pro Met Ser Ser Met His Phe His Ser Cys Arg Asn
        915                 920                 925

Ser Ser Ser Phe Glu Asp Ser Ser
    930                 935


<210>  210
<211>  835
<212>  PRT
<213>  Homo sapiens

<400> 210

Met Gly Asn Tyr Lys Ser Arg Pro Thr Gln Thr Cys Thr Asp Glu Trp
1               5                   10                  15
```

```
Lys Lys Lys Val Ser Glu Ser Tyr Val Ile Thr Ile Glu Arg Leu Glu
            20                  25                  30

Asp Asp Leu Gln Ile Lys Glu Lys Glu Leu Thr Glu Leu Arg Asn Ile
        35                  40                  45

Phe Gly Ser Asp Glu Ala Phe Ser Lys Val Asn Leu Asn Tyr Arg Thr
    50                  55                  60

Glu Asn Gly Leu Ser Leu Leu His Leu Cys Cys Ile Cys Gly Gly Lys
65                  70                  75                  80

Lys Ser His Ile Arg Thr Leu Met Leu Lys Gly Leu Arg Pro Ser Arg
                85                  90                  95

Leu Thr Arg Asn Gly Phe Thr Ala Leu His Leu Ala Val Tyr Lys Asp
            100                 105                 110

Asn Ala Glu Leu Ile Thr Ser Leu Leu His Ser Gly Ala Asp Ile Gln
        115                 120                 125

Gln Val Gly Tyr Gly Gly Leu Thr Ala Leu His Ile Ala Thr Ile Ala
    130                 135                 140

Gly His Leu Glu Ala Ala Asp Val Leu Leu Gln His Gly Ala Asn Val
145                 150                 155                 160

Asn Ile Gln Asp Ala Val Phe Phe Thr Pro Leu His Ile Ala Ala Tyr
                165                 170                 175

Tyr Gly His Glu Gln Val Thr Arg Leu Leu Leu Lys Phe Gly Ala Asp
            180                 185                 190

Val Asn Val Ser Gly Glu Val Gly Asp Arg Pro Leu His Leu Ala Ser
        195                 200                 205

Ala Lys Gly Phe Leu Asn Ile Ala Lys Leu Leu Met Glu Glu Gly Ser
    210                 215                 220

Lys Ala Asp Val Asn Ala Gln Asp Asn Glu Asp His Val Pro Leu His
225                 230                 235                 240

Phe Cys Ser Arg Phe Gly His His Asp Ile Val Lys Tyr Leu Leu Gln
                245                 250                 255

Ser Asp Leu Glu Val Gln Pro His Val Val Asn Ile Tyr Gly Asp Thr
            260                 265                 270
```

```
Pro Leu His Leu Ala Cys Tyr Asn Gly Lys Phe Glu Val Ala Lys Glu
        275                 280                 285

Ile Ile Gln Ile Ser Gly Thr Glu Ser Leu Thr Lys Glu Asn Ile Phe
    290                 295                 300

Ser Glu Thr Ala Phe His Ser Ala Cys Thr Tyr Gly Lys Ser Ile Asp
305                 310                 315                 320

Leu Val Lys Phe Leu Leu Asp Gln Asn Val Ile Asn Ile Asn His Gln
                325                 330                 335

Gly Arg Asp Gly His Thr Gly Leu His Ser Ala Cys Tyr His Gly His
            340                 345                 350

Ile Arg Leu Val Gln Phe Leu Leu Asp Asn Gly Ala Asp Met Asn Leu
        355                 360                 365

Val Ala Cys Asp Pro Ser Arg Ser Ser Gly Glu Lys Asp Glu Gln Thr
    370                 375                 380

Cys Leu Met Trp Ala Tyr Glu Lys Gly His Asp Ala Ile Val Thr Leu
385                 390                 395                 400

Leu Lys His Tyr Lys Arg Pro Gln Asp Glu Leu Pro Cys Asn Glu Tyr
                405                 410                 415

Ser Gln Pro Gly Gly Asp Gly Ser Tyr Val Ser Val Pro Ser Pro Leu
            420                 425                 430

Gly Lys Ile Lys Ser Met Thr Lys Glu Lys Ala Asp Ile Leu Leu Leu
        435                 440                 445

Arg Ala Gly Leu Pro Ser His Phe His Leu Gln Leu Ser Glu Ile Glu
    450                 455                 460

Phe His Glu Ile Ile Gly Ser Gly Ser Phe Gly Lys Val Tyr Lys Gly
465                 470                 475                 480

Arg Cys Arg Asn Lys Ile Val Ala Ile Lys Arg Tyr Arg Ala Asn Thr
                485                 490                 495

Tyr Cys Ser Lys Ser Asp Val Asp Met Phe Cys Arg Glu Val Ser Ile
            500                 505                 510

Leu Cys Gln Leu Asn His Pro Cys Val Ile Gln Phe Val Gly Ala Cys
```

515 520 525

Leu Asn Asp Pro Ser Gln Phe Ala Ile Val Thr Gln Tyr Ile Ser Gly
530 535 540

Gly Ser Leu Phe Ser Leu Leu His Glu Gln Lys Arg Ile Leu Asp Leu
545 550 555 560

Gln Ser Lys Leu Ile Ile Ala Val Asp Val Ala Lys Gly Met Glu Tyr
565 570 575

Leu His Asn Leu Thr Gln Pro Ile Ile His Arg Asp Leu Asn Ser His
580 585 590

Asn Ile Leu Leu Tyr Glu Asp Gly His Ala Val Val Ala Asp Phe Gly
595 600 605

Glu Ser Arg Phe Leu Gln Ser Leu Asp Glu Asp Asn Met Thr Lys Gln
610 615 620

Pro Gly Asn Leu Arg Trp Met Ala Pro Glu Val Phe Thr Gln Cys Thr
625 630 635 640

Arg Tyr Thr Ile Lys Ala Asp Val Phe Ser Tyr Ala Leu Cys Leu Trp
645 650 655

Glu Ile Leu Thr Gly Glu Ile Pro Phe Ala His Leu Lys Pro Ala Ala
660 665 670

Ala Ala Ala Asp Met Ala Tyr His His Ile Arg Pro Pro Ile Gly Tyr
675 680 685

Ser Ile Pro Lys Pro Ile Ser Ser Leu Leu Ile Arg Gly Trp Asn Ala
690 695 700

Cys Pro Glu Gly Arg Pro Glu Phe Ser Glu Val Val Met Lys Leu Glu
705 710 715 720

Glu Cys Leu Cys Asn Ile Glu Leu Met Ser Pro Ala Ser Ser Asn Ser
725 730 735

Ser Gly Ser Leu Ser Pro Ser Ser Ser Ser Asp Cys Leu Val Asn Arg
740 745 750

Gly Gly Pro Gly Arg Ser His Val Ala Ala Leu Arg Ser Arg Phe Glu
755 760 765

```
Leu Glu Tyr Ala Leu Asn Ala Arg Ser Tyr Ala Ala Leu Ser Gln Ser
    770                 775                 780

Ala Gly Gln Tyr Ser Ser Gln Gly Leu Ser Leu Glu Glu Met Lys Arg
785                 790                 795                 800

Ser Leu Gln Tyr Thr Pro Ile Asp Lys Tyr Gly Tyr Val Ser Asp Pro
                805                 810                 815

Met Ser Ser Met His Phe His Ser Cys Arg Asn Ser Ser Ser Phe Glu
            820                 825                 830

Asp Ser Ser
        835


<210>  211
<211>  714
<212>  PRT
<213>  Homo sapiens

<400> 211

Ser Ser Gly Ile Asn Ala Glu Trp Pro Leu Trp Pro Gly Glu Gly Gly
1               5                   10                  15

Ala Met Ala Ala Ala Arg Asp Pro Pro Glu Val Ser Leu Arg Glu Ala
            20                  25                  30

Thr Gln Arg Lys Leu Arg Arg Phe Ser Glu Leu Arg Gly Lys Leu Val
        35                  40                  45

Ala Arg Gly Glu Phe Trp Asp Ile Val Ala Ile Thr Ala Ala Asp Glu
    50                  55                  60

Lys Gln Glu Leu Ala Tyr Asn Gln Gln Leu Ser Glu Lys Leu Lys Arg
65                  70                  75                  80

Lys Glu Leu Pro Leu Gly Val Gln Tyr His Val Phe Val Asp Pro Ala
                85                  90                  95

Gly Ala Lys Ile Gly Asn Gly Gly Ser Thr Leu Cys Ala Leu Gln Cys
            100                 105                 110

Leu Glu Lys Leu Tyr Gly Asp Lys Trp Asn Ser Phe Thr Ile Leu Leu
        115                 120                 125

Ile His Ser Asp Glu Trp Lys Lys Lys Val Ser Glu Ser Tyr Val Ile
    130                 135                 140
```

```
Thr Ile Glu Arg Leu Glu Asp Asp Leu Gln Ile Lys Glu Lys Glu Leu
145                 150                 155                 160

Thr Glu Leu Arg Asn Ile Phe Gly Ser Asp Glu Ala Phe Ser Lys Val
                165                 170                 175

Asn Leu Asn Tyr Arg Thr Glu Asn Gly Leu Ser Leu Leu His Leu Cys
            180                 185                 190

Cys Ile Cys Gly Gly Lys Lys Ser His Ile Arg Thr Leu Met Leu Lys
        195                 200                 205

Gly Leu Arg Pro Ser Arg Leu Thr Arg Asn Gly Phe Thr Ala Leu His
    210                 215                 220

Leu Ala Val Tyr Lys Asp Asn Ala Glu Leu Ile Thr Ser Leu Leu His
225                 230                 235                 240

Ser Gly Ala Asp Ile Gln Gln Val Gly Tyr Gly Gly Leu Thr Ala Leu
                245                 250                 255

His Ile Ala Thr Ile Ala Gly His Leu Glu Ala Ala Asp Val Leu Leu
            260                 265                 270

Gln His Gly Ala Asn Val Asn Ile Gln Asp Ala Val Phe Phe Thr Pro
        275                 280                 285

Leu His Ile Ala Ala Tyr Tyr Gly His Glu Gln Val Thr Arg Leu Leu
    290                 295                 300

Leu Lys Phe Gly Ala Asp Val Asn Val Ser Gly Glu Val Gly Asp Arg
305                 310                 315                 320

Pro Leu His Leu Ala Ser Ala Lys Gly Phe Leu Asn Ile Ala Lys Leu
                325                 330                 335

Leu Met Glu Glu Gly Ser Lys Ala Asp Val Asn Ala Gln Asp Asn Glu
            340                 345                 350

Asp His Val Pro Leu His Phe Cys Ser Arg Phe Gly His His Asp Met
        355                 360                 365

Val Lys Tyr Leu Leu Gln Ser Asp Leu Glu Val Gln Pro His Val Val
    370                 375                 380

Asn Ile Tyr Gly Asp Thr Pro Leu His Leu Ala Cys Tyr Asn Gly Lys
385                 390                 395                 400
```

```
Phe Glu Val Ala Lys Glu Ile Ile Gln Ile Ser Gly Thr Glu Ser Leu
                405                 410                 415

Thr Lys Glu Asn Ile Phe Ser Glu Thr Ala Phe His Ser Ala Cys Thr
            420                 425                 430

Tyr Gly Lys Ser Ile Asp Leu Val Lys Phe Leu Leu Asp Gln Asn Val
        435                 440                 445

Ile Asn Ile Asn His Gln Gly Arg Asp Gly His Thr Gly Leu His Ser
    450                 455                 460

Ala Cys Tyr His Gly His Ile Arg Leu Val Gln Phe Leu Leu Asp Asn
465                 470                 475                 480

Gly Ala Asp Met Ser Leu Val Ala Cys Asp Pro Ser Arg Ser Ser Gly
                485                 490                 495

Glu Lys Asp Glu Gln Thr Cys Leu Met Trp Ala Tyr Glu Lys Gly His
            500                 505                 510

Asp Ala Ile Val Thr Leu Leu Lys His Tyr Lys Arg Pro Gln Asp Glu
        515                 520                 525

Leu Pro Cys Asn Glu Tyr Ser Gln Pro Gly Gly Asp Gly Ser Tyr Val
    530                 535                 540

Ser Val Pro Ser Pro Leu Gly Lys Ile Lys Ser Met Thr Lys Glu Lys
545                 550                 555                 560

Ala Asp Ile Leu Leu Leu Arg Ala Gly Leu Pro Ser His Phe His Leu
                565                 570                 575

Gln Leu Ser Glu Ile Glu Phe His Glu Ile Ile Gly Ser Gly Ser Phe
            580                 585                 590

Gly Lys Val Tyr Lys Gly Arg Cys Arg Asn Lys Ile Val Ala Ile Lys
        595                 600                 605

Arg Tyr Arg Ala Asn Thr Tyr Cys Ser Lys Ser Asp Val Asp Met Phe
    610                 615                 620

Cys Arg Glu Val Ser Ile Leu Cys Gln Leu Asn His Pro Cys Val Ile
625                 630                 635                 640

Gln Phe Val Gly Ala Cys Leu Asn Asp Pro Ser Gln Phe Ala Ile Val
```

645 650 655

Thr Gln Tyr Ile Ser Gly Gly Ser Leu Phe Ser Leu Leu His Glu Gln
660 665 670

Lys Arg Ile Leu Asp Leu Gln Ser Lys Leu Ile Ile Ala Val Asp Val
675 680 685

Ala Lys Gly Met Glu Tyr Leu His Asn Leu Thr Gln Pro Ile Ile His
690 695 700

Arg Asp Leu Asn Arg Tyr Phe Phe Pro Lys
705 710

<210> 212
<211> 969
<212> PRT
<213> Homo sapiens

<400> 212

Ala Val Arg Arg Gly Leu Arg Glu Gly Gly Ala Met Ala Ala Ala Arg
1 5 10 15

Asp Pro Pro Glu Val Ser Leu Arg Glu Ala Thr Gln Arg Lys Leu Arg
20 25 30

Arg Phe Ser Glu Leu Arg Gly Lys Leu Val Ala Arg Gly Glu Phe Trp
35 40 45

Asp Ile Val Ala Ile Thr Ala Ala Asp Glu Lys Gln Glu Leu Ala Tyr
50 55 60

Asn Gln Gln Leu Ser Glu Lys Leu Lys Arg Lys Glu Leu Pro Leu Gly
65 70 75 80

Val Gln Tyr His Val Phe Val Asp Pro Ala Gly Ala Lys Ile Gly Asn
85 90 95

Gly Gly Ser Thr Leu Cys Ala Leu Gln Cys Leu Glu Lys Leu Tyr Gly
100 105 110

Asp Lys Trp Asn Ser Phe Thr Ile Leu Leu Ile His Ser Asp Glu Trp
115 120 125

Lys Lys Lys Val Ser Glu Ser Tyr Val Ile Thr Ile Glu Arg Leu Glu
130 135 140

```
Asp Asp Leu Gln Ile Lys Glu Lys Glu Leu Thr Glu Leu Arg Asn Ile
145                 150                 155                 160

Phe Gly Ser Asp Glu Ala Phe Ser Lys Val Asn Leu Asn Tyr Arg Thr
                165                 170                 175

Glu Asn Gly Leu Ser Leu Leu His Leu Cys Cys Ile Cys Gly Gly Lys
            180                 185                 190

Lys Ser His Ile Arg Thr Leu Met Leu Lys Gly Leu Arg Pro Ser Arg
        195                 200                 205

Leu Thr Arg Asn Gly Phe Thr Ala Leu His Leu Ala Val Tyr Lys Asp
    210                 215                 220

Asn Ala Glu Leu Ile Thr Ser Leu Leu His Ser Gly Ala Asp Ile Gln
225                 230                 235                 240

Gln Val Gly Tyr Gly Gly Leu Thr Ala Leu His Ile Ala Thr Ile Ala
                245                 250                 255

Gly His Leu Glu Ala Ala Asp Val Leu Leu Gln His Gly Ala Asn Val
            260                 265                 270

Asn Ile Gln Asp Ala Val Phe Phe Thr Pro Leu His Ile Ala Ala Tyr
        275                 280                 285

Tyr Gly His Glu Gln Val Thr Arg Leu Leu Leu Lys Phe Gly Ala Asp
    290                 295                 300

Val Asn Val Ser Gly Glu Val Gly Asp Arg Pro Leu His Leu Ala Ser
305                 310                 315                 320

Ala Lys Gly Phe Leu Asn Ile Ala Lys Leu Leu Met Glu Glu Gly Ser
                325                 330                 335

Lys Ala Asp Val Asn Ala Gln Asp Asn Glu Asp His Val Pro Leu His
            340                 345                 350

Phe Cys Ser Arg Phe Gly His His Asp Ile Val Lys Tyr Leu Leu Gln
        355                 360                 365

Ser Asp Leu Glu Val Gln Pro His Val Val Asn Ile Tyr Gly Asp Thr
    370                 375                 380

Pro Leu His Leu Ala Cys Tyr Asn Gly Lys Phe Glu Val Ala Lys Glu
385                 390                 395                 400
```

```
Ile Ile Gln Ile Ser Gly Thr Glu Ser Leu Thr Lys Glu Asn Ile Phe
                405                 410                 415

Ser Glu Thr Ala Phe His Ser Ala Cys Thr Tyr Gly Lys Ser Ile Asp
            420                 425                 430

Leu Val Lys Phe Leu Leu Asp Gln Asn Val Ile Asn Ile Asn His Gln
        435                 440                 445

Gly Arg Asp Gly His Thr Gly Leu His Ser Ala Cys Tyr His Gly His
    450                 455                 460

Ile Arg Leu Val Gln Phe Leu Leu Asp Asn Gly Ala Asp Met Asn Leu
465                 470                 475                 480

Val Ala Cys Asp Pro Ser Arg Ser Ser Gly Glu Lys Asp Glu Gln Thr
                485                 490                 495

Cys Leu Met Trp Ala Tyr Glu Lys Gly His Asp Ala Ile Val Thr Leu
            500                 505                 510

Leu Lys His Tyr Lys Arg Pro Gln Asp Glu Leu Pro Cys Asn Glu Tyr
        515                 520                 525

Ser Gln Pro Gly Gly Asp Gly Ser Tyr Val Ser Val Pro Ser Pro Leu
    530                 535                 540

Gly Lys Ile Lys Ser Met Thr Lys Glu Lys Ala Asp Ile Leu Leu Leu
545                 550                 555                 560

Arg Ala Gly Leu Pro Ser His Phe His Leu Gln Leu Ser Glu Ile Glu
                565                 570                 575

Phe His Glu Ile Ile Gly Ser Gly Ser Phe Gly Lys Val Tyr Lys Gly
            580                 585                 590

Arg Cys Arg Asn Lys Ile Val Ala Ile Lys Arg Tyr Arg Ala Asn Thr
        595                 600                 605

Tyr Cys Ser Lys Ser Asp Val Asp Met Phe Cys Arg Glu Val Ser Ile
    610                 615                 620

Leu Cys Gln Leu Asn His Pro Cys Val Ile Gln Phe Val Gly Ala Cys
625                 630                 635                 640

Leu Asn Asp Pro Ser Gln Phe Ala Ile Val Thr Gln Tyr Ile Ser Gly
                645                 650                 655
```

```
Gly Ser Leu Phe Ser Leu Leu His Glu Gln Lys Arg Ile Leu Asp Leu
            660                 665                 670

Gln Ser Lys Leu Ile Ile Ala Val Asp Val Ala Lys Gly Met Glu Tyr
        675                 680                 685

Leu His Asn Leu Thr Gln Pro Ile Ile His Arg Asp Leu Asn Arg Ser
    690                 695                 700

Ala Ile Thr Ser Arg Ile Trp Ile Thr His Ser Ile Cys Ile Trp Arg
705                 710                 715                 720

Gly Ala His Tyr Phe Asn Arg Glu Glu Cys Asn Phe Arg Cys Met Leu
                725                 730                 735

Thr Ser Ala Ile Leu Lys Glu Ser Arg Phe Leu Gln Ser Leu Asp Glu
            740                 745                 750

Asp Asn Met Thr Lys Gln Pro Gly Asn Leu Arg Trp Met Ala Pro Glu
        755                 760                 765

Val Phe Thr Gln Cys Thr Arg Tyr Thr Ile Lys Ala Asp Val Phe Ser
    770                 775                 780

Tyr Ala Leu Cys Leu Trp Glu Ile Leu Thr Gly Glu Ile Pro Phe Ala
785                 790                 795                 800

His Leu Lys Pro Ala Ala Ala Ala Ala Asp Met Ala Tyr His His Ile
                805                 810                 815

Arg Pro Pro Ile Gly Tyr Ser Ile Pro Lys Pro Ile Ser Ser Leu Leu
            820                 825                 830

Ile Arg Gly Trp Asn Ala Cys Pro Glu Gly Arg Pro Glu Phe Ser Glu
        835                 840                 845

Val Val Met Lys Leu Glu Glu Cys Leu Cys Asn Ile Glu Leu Met Ser
    850                 855                 860

Pro Ala Ser Ser Asn Ser Ser Gly Ser Leu Ser Pro Ser Ser Ser Ser
865                 870                 875                 880

Asp Cys Leu Val Asn Arg Gly Gly Pro Gly Arg Ser His Val Ala Ala
                885                 890                 895

Leu Arg Ser Arg Phe Glu Leu Glu Tyr Ala Leu Asn Ala Arg Ser Tyr
```

```
            900                 905                 910

Ala Ala Leu Ser Gln Ser Ala Gly Gln Tyr Ser Ser Gln Gly Leu Ser
        915                 920                 925

Leu Glu Glu Met Lys Arg Ser Leu Gln Tyr Thr Pro Ile Asp Lys Tyr
    930                 935                 940

Gly Tyr Val Ser Asp Pro Met Ser Ser Met His Phe His Ser Cys Arg
945                 950                 955                 960

Asn Ser Ser Ser Phe Glu Asp Ser Ser
                965



<210>  213
<211>  857
<212>  PRT
<213>  Homo sapiens

<400> 213

Met Gly Asn Tyr Lys Ser Arg Pro Thr Gln Thr Cys Thr Asp Glu Trp
1               5                   10                  15

Lys Lys Lys Val Ser Glu Ser Tyr Val Ile Thr Ile Glu Arg Leu Glu
            20                  25                  30

Asp Asp Leu Gln Ile Lys Glu Lys Glu Leu Thr Glu Leu Arg Asn Ile
        35                  40                  45

Phe Gly Ser Asp Glu Ala Phe Ser Lys Val Asn Leu Asn Tyr Arg Thr
    50                  55                  60

Glu Asn Gly Leu Ser Leu Leu His Leu Cys Cys Ile Cys Gly Gly Lys
65                  70                  75                  80

Lys Ser His Ile Arg Thr Leu Met Leu Lys Gly Leu Arg Pro Ser Arg
                85                  90                  95

Leu Thr Arg Asn Gly Phe Thr Ala Leu His Leu Ala Val Tyr Lys Asp
            100                 105                 110

Asn Ala Glu Leu Ile Thr Ser Leu Leu His Ser Gly Ala Asp Ile Gln
        115                 120                 125

Gln Val Gly Tyr Gly Gly Leu Thr Ala Leu His Ile Ala Thr Ile Ala
    130                 135                 140
```

```
Gly His Leu Glu Ala Ala Asp Val Leu Leu Gln His Gly Ala Asn Val
145                 150                 155                 160

Asn Ile Gln Asp Ala Val Phe Phe Thr Pro Leu His Ile Ala Ala Tyr
                165                 170                 175

Tyr Gly His Glu Gln Val Thr Arg Leu Leu Leu Lys Phe Gly Ala Asp
            180                 185                 190

Val Asn Val Ser Gly Glu Val Gly Asp Arg Pro Leu His Leu Ala Ser
        195                 200                 205

Ala Lys Gly Phe Leu Asn Ile Ala Lys Leu Leu Met Glu Glu Gly Ser
    210                 215                 220

Lys Ala Asp Val Asn Ala Gln Asp Asn Glu Asp His Val Pro Leu His
225                 230                 235                 240

Phe Cys Ser Arg Phe Gly His His Asp Ile Val Lys Tyr Leu Leu Gln
                245                 250                 255

Ser Asp Leu Glu Val Gln Pro His Val Val Asn Ile Tyr Gly Asp Thr
            260                 265                 270

Pro Leu His Leu Ala Cys Tyr Asn Gly Lys Phe Glu Val Ala Lys Glu
        275                 280                 285

Ile Ile Gln Ile Ser Gly Thr Glu Ser Leu Thr Lys Glu Asn Ile Phe
    290                 295                 300

Ser Glu Thr Ala Phe His Ser Ala Cys Thr Tyr Gly Lys Ser Ile Asp
305                 310                 315                 320

Leu Val Lys Phe Leu Leu Asp Gln Asn Val Ile Asn Ile Asn His Gln
                325                 330                 335

Gly Arg Asp Gly His Thr Gly Leu His Ser Ala Cys Tyr His Gly His
            340                 345                 350

Ile Arg Leu Val Gln Phe Leu Leu Asp Asn Gly Ala Asp Met Asn Leu
        355                 360                 365

Val Ala Cys Asp Pro Ser Arg Ser Ser Gly Glu Lys Asp Glu Gln Thr
    370                 375                 380

Cys Leu Met Trp Ala Tyr Glu Lys Gly His Asp Ala Ile Val Thr Leu
385                 390                 395                 400
```

```
Leu Lys His Tyr Lys Arg Pro Gln Asp Glu Leu Pro Cys Asn Glu Tyr
                405                 410                 415

Ser Gln Pro Gly Gly Asp Gly Ser Tyr Val Ser Val Pro Ser Pro Leu
            420                 425                 430

Gly Lys Ile Lys Ser Met Thr Lys Glu Lys Ala Asp Ile Leu Leu Leu
        435                 440                 445

Arg Ala Gly Leu Pro Ser His Phe His Leu Gln Leu Ser Glu Ile Glu
    450                 455                 460

Phe His Glu Ile Ile Gly Ser Gly Ser Phe Gly Lys Val Tyr Lys Gly
465                 470                 475                 480

Arg Cys Arg Asn Lys Ile Val Ala Ile Lys Arg Tyr Arg Ala Asn Thr
                485                 490                 495

Tyr Cys Ser Lys Ser Asp Val Asp Met Phe Cys Arg Glu Val Ser Ile
            500                 505                 510

Leu Cys Gln Leu Asn His Pro Cys Val Ile Gln Phe Val Gly Ala Cys
        515                 520                 525

Leu Asn Asp Pro Ser Gln Phe Ala Ile Val Thr Gln Tyr Ile Ser Gly
    530                 535                 540

Gly Ser Leu Phe Ser Leu Leu His Glu Gln Lys Arg Ile Leu Asp Leu
545                 550                 555                 560

Gln Ser Lys Leu Ile Ile Ala Val Asp Val Ala Lys Gly Met Glu Tyr
                565                 570                 575

Leu His Asn Leu Thr Gln Pro Ile Ile His Arg Asp Leu Asn Arg Ser
            580                 585                 590

Ala Ile Thr Ser Arg Ile Trp Ile Thr His Ser Ile Cys Ile Trp Arg
        595                 600                 605

Gly Ala His Tyr Phe Asn Arg Glu Glu Cys Asn Phe Arg Cys Met Leu
    610                 615                 620

Thr Ser Ala Ile Leu Lys Glu Ser Arg Phe Leu Gln Ser Leu Asp Glu
625                 630                 635                 640

Asp Asn Met Thr Lys Gln Pro Gly Asn Leu Arg Trp Met Ala Pro Glu
                645                 650                 655
```

Val Phe Thr Gln Cys Thr Arg Tyr Thr Ile Lys Ala Asp Val Phe Ser
660 665 670

Tyr Ala Leu Cys Leu Trp Glu Ile Leu Thr Gly Glu Ile Pro Phe Ala
675 680 685

His Leu Lys Pro Ala Ala Ala Ala Ala Asp Met Ala Tyr His His Ile
690 695 700

Arg Pro Pro Ile Gly Tyr Ser Ile Pro Lys Pro Ile Ser Ser Leu Leu
705 710 715 720

Ile Arg Gly Trp Asn Ala Cys Pro Glu Gly Arg Pro Glu Phe Ser Glu
725 730 735

Val Val Met Lys Leu Glu Glu Cys Leu Cys Asn Ile Glu Leu Met Ser
740 745 750

Pro Ala Ser Ser Asn Ser Ser Gly Ser Leu Ser Pro Ser Ser Ser Ser
755 760 765

Asp Cys Leu Val Asn Arg Gly Gly Pro Gly Arg Ser His Val Ala Ala
770 775 780

Leu Arg Ser Arg Phe Glu Leu Glu Tyr Ala Leu Asn Ala Arg Ser Tyr
785 790 795 800

Ala Ala Leu Ser Gln Ser Ala Gly Gln Tyr Ser Ser Gln Gly Leu Ser
805 810 815

Leu Glu Glu Met Lys Arg Ser Leu Gln Tyr Thr Pro Ile Asp Lys Tyr
820 825 830

Gly Tyr Val Ser Asp Pro Met Ser Ser Met His Phe His Ser Cys Arg
835 840 845

Asn Ser Ser Ser Phe Glu Asp Ser Ser
850 855

<210> 214
<211> 814
<212> PRT
<213> Homo sapiens

<400> 214

Met Gly Asn Tyr Lys Ser Arg Pro Thr Gln Thr Cys Thr Asp Glu Trp

1 5 10 15

Lys Lys Lys Val Ser Glu Ser Tyr Val Ile Thr Ile Glu Arg Leu Glu
20 25 30

Asp Asp Leu Gln Ile Lys Glu Lys Glu Leu Thr Glu Leu Arg Asn Ile
35 40 45

Phe Gly Ser Asp Glu Ala Phe Ser Lys Val Asn Leu Asn Tyr Arg Thr
50 55 60

Glu Asn Gly Leu Ser Leu Leu His Leu Cys Cys Ile Cys Gly Gly Lys
65 70 75 80

Lys Ser His Ile Arg Thr Leu Met Leu Lys Gly Leu Arg Pro Ser Arg
85 90 95

Leu Thr Arg Asn Gly Phe Thr Ala Leu His Leu Ala Val Tyr Lys Asp
100 105 110

Asn Ala Glu Leu Ile Thr Ser Leu Leu His Ser Gly Ala Asp Ile Gln
115 120 125

Gln Val Gly Tyr Gly Gly Leu Thr Ala Leu His Ile Ala Thr Ile Ala
130 135 140

Gly His Leu Glu Ala Ala Asp Val Leu Leu Gln His Gly Ala Asn Val
145 150 155 160

Asn Ile Gln Asp Ala Val Phe Phe Thr Pro Leu His Ile Ala Ala Tyr
165 170 175

Tyr Gly His Glu Gln Val Thr Arg Leu Leu Leu Lys Phe Gly Ala Asp
180 185 190

Val Asn Val Ser Gly Glu Val Gly Asp Arg Pro Leu His Leu Ala Ser
195 200 205

Ala Lys Gly Phe Leu Asn Ile Ala Lys Leu Leu Met Glu Glu Gly Ser
210 215 220

Lys Ala Asp Val Asn Ala Gln Asp Asn Glu Asp His Val Pro Leu His
225 230 235 240

Phe Cys Ser Arg Phe Gly His His Asp Ile Val Lys Tyr Leu Leu Gln
245 250 255

```
Ser Asp Leu Glu Val Gln Pro His Val Val Asn Ile Tyr Gly Asp Thr
            260                 265                 270

Pro Leu His Leu Ala Cys Tyr Asn Gly Lys Phe Glu Val Ala Lys Glu
        275                 280                 285

Ile Ile Gln Ile Ser Gly Thr Glu Ser Leu Thr Lys Glu Asn Ile Phe
    290                 295                 300

Ser Glu Thr Ala Phe His Ser Ala Cys Thr Tyr Gly Lys Ser Ile Asp
305                 310                 315                 320

Leu Val Lys Phe Leu Leu Asp Gln Asn Val Ile Asn Ile Asn His Gln
                325                 330                 335

Gly Arg Asp Gly His Thr Gly Leu His Ser Ala Cys Tyr His Gly His
            340                 345                 350

Ile Arg Leu Val Gln Phe Leu Leu Asp Asn Gly Ala Asp Met Asn Leu
        355                 360                 365

Val Ala Cys Asp Pro Ser Arg Ser Ser Gly Glu Lys Asp Glu Gln Thr
    370                 375                 380

Cys Leu Met Trp Ala Tyr Glu Lys Gly His Asp Ala Ile Val Thr Leu
385                 390                 395                 400

Leu Lys His Tyr Lys Arg Pro Gln Asp Glu Leu Pro Cys Asn Glu Tyr
                405                 410                 415

Ser Gln Pro Gly Gly Asp Gly Ser Tyr Val Ser Val Pro Ser Pro Leu
            420                 425                 430

Gly Lys Ile Lys Ser Met Thr Lys Glu Lys Ala Asp Ile Leu Leu Leu
        435                 440                 445

Arg Ala Gly Leu Pro Ser His Phe His Leu Gln Leu Ser Glu Ile Glu
    450                 455                 460

Phe His Glu Ile Ile Gly Ser Gly Ser Phe Gly Lys Val Tyr Lys Gly
465                 470                 475                 480

Arg Cys Arg Asn Lys Ile Val Ala Ile Lys Arg Tyr Arg Ala Asn Thr
                485                 490                 495

Tyr Cys Ser Lys Ser Asp Val Asp Met Phe Cys Arg Glu Val Ser Ile
            500                 505                 510
```

```
Leu Cys Gln Leu Asn His Pro Cys Val Ile Gln Phe Val Gly Ala Cys
        515                 520                 525

Leu Asn Asp Pro Ser Gln Phe Ala Ile Val Thr Gln Tyr Ile Ser Gly
    530                 535                 540

Gly Ser Leu Phe Ser Leu Leu His Glu Gln Lys Arg Ile Leu Asp Leu
545                 550                 555                 560

Gln Ser Lys Leu Ile Ile Ala Val Asp Val Ala Lys Gly Met Glu Tyr
                565                 570                 575

Leu His Asn Leu Thr Gln Pro Ile Ile His Arg Asp Leu Asn Ser His
            580                 585                 590

Asn Ile Leu Leu Tyr Glu Asp Gly His Ala Val Val Ala Asp Phe Gly
        595                 600                 605

Glu Ser Arg Phe Leu Gln Ser Leu Asp Glu Asp Asn Met Thr Lys Gln
    610                 615                 620

Pro Gly Asn Leu Arg Trp Met Ala Pro Glu Val Phe Thr Gln Cys Thr
625                 630                 635                 640

Arg Tyr Thr Ile Lys Ala Asp Val Phe Ser Tyr Ala Leu Cys Leu Trp
                645                 650                 655

Glu Ile Leu Thr Gly Glu Ile Pro Phe Ala His Leu Lys Pro Ala Ala
            660                 665                 670

Ala Ala Ala Asp Met Ala Tyr His His Ile Arg Pro Pro Ile Gly Tyr
        675                 680                 685

Ser Ile Pro Lys Pro Ile Ser Ser Leu Leu Ile Arg Gly Trp Asn Ala
    690                 695                 700

Cys Pro Glu Gly Arg Pro Glu Phe Ser Glu Val Val Met Lys Leu Glu
705                 710                 715                 720

Glu Cys Leu Cys Asn Ile Glu Leu Met Ser Pro Ala Ser Ser Asn Ser
                725                 730                 735

Ser Gly Ser Leu Ser Pro Ser Ser Ser Ser Asp Cys Leu Val Asn Arg
            740                 745                 750

Gly Gly Pro Gly Arg Ser His Val Ala Ala Leu Arg Ser Arg Phe Glu
        755                 760                 765
```

```
Leu Glu Tyr Ala Leu Asn Ala Arg Ser Tyr Ala Ala Leu Ser Gln Ser
    770                 775                 780

Ala Gly Gln Tyr Ser Ser Gln Gly Leu Ser Leu Glu Glu Met Lys Arg
785                 790                 795                 800

Ser Leu Gln Tyr Thr Pro Ile Asp Lys Tyr Asp Val Thr Ser
                805                 810



<210>  215
<211>  739
<212>  PRT
<213>  Homo sapiens

<400> 215

Pro Ser Pro Cys Gly Leu His Phe Leu Ile Pro Trp Leu Thr Gln Asp
1               5                   10                  15

Asn Ala Glu Leu Ile Thr Ser Leu Leu His Ser Gly Ala Asp Ile Gln
            20                  25                  30

Gln Val Gly Tyr Gly Gly Leu Thr Ala Leu His Ile Ala Thr Ile Ala
        35                  40                  45

Gly His Leu Glu Ala Ala Asp Val Leu Leu Gln His Gly Ala Asn Val
    50                  55                  60

Asn Ile Gln Asp Ala Val Phe Phe Thr Pro Leu His Ile Ala Ala Tyr
65                  70                  75                  80

Tyr Gly His Glu Gln Val Thr Arg Leu Leu Leu Lys Phe Gly Ala Asp
                85                  90                  95

Val Asn Val Ser Gly Glu Val Gly Asp Arg Pro Leu His Leu Ala Ser
            100                 105                 110

Ala Lys Gly Phe Leu Asn Ile Ala Lys Leu Leu Met Glu Glu Gly Ser
        115                 120                 125

Lys Ala Asp Val Asn Ala Gln Asp Asn Glu Asp His Val Pro Leu His
    130                 135                 140

Phe Cys Ser Arg Phe Gly His His Asp Ile Val Lys Tyr Leu Leu Gln
145                 150                 155                 160

Ser Asp Leu Glu Val Gln Pro His Val Val Asn Ile Tyr Gly Asp Thr
```

165 170 175

Pro Leu His Leu Ala Cys Tyr Asn Gly Lys Phe Glu Val Ala Lys Glu
180 185 190

Ile Ile Gln Ile Ser Gly Thr Glu Ser Leu Thr Lys Glu Asn Ile Phe
195 200 205

Ser Glu Thr Ala Phe His Ser Ala Cys Thr Tyr Gly Lys Ser Ile Asp
210 215 220

Leu Val Lys Phe Leu Leu Asp Gln Asn Val Ile Asn Ile Asn His Gln
225 230 235 240

Gly Arg Asp Gly His Thr Gly Leu His Ser Ala Cys Tyr His Gly His
245 250 255

Ile Arg Leu Val Gln Phe Leu Leu Asp Asn Gly Ala Asp Met Asn Leu
260 265 270

Val Ala Cys Asp Pro Ser Arg Ser Ser Gly Glu Lys Asp Glu Gln Thr
275 280 285

Cys Leu Met Trp Ala Tyr Glu Lys Gly His Asp Ala Ile Val Thr Leu
290 295 300

Leu Lys His Tyr Lys Arg Pro Gln Asp Glu Leu Pro Cys Asn Glu Tyr
305 310 315 320

Ser Gln Pro Gly Gly Asp Gly Ser Tyr Val Ser Val Pro Ser Pro Leu
325 330 335

Gly Lys Ile Lys Ser Met Thr Lys Glu Lys Ala Asp Ile Leu Leu Leu
340 345 350

Arg Ala Gly Leu Pro Ser His Phe His Leu Gln Leu Ser Glu Ile Glu
355 360 365

Phe His Glu Ile Ile Gly Ser Gly Ser Phe Gly Lys Val Tyr Lys Gly
370 375 380

Arg Cys Arg Asn Lys Ile Val Ala Ile Lys Arg Tyr Arg Ala Asn Thr
385 390 395 400

Tyr Cys Ser Lys Ser Asp Val Asp Met Phe Cys Arg Glu Val Ser Ile
405 410 415

```
Leu Cys Gln Leu Asn His Pro Cys Val Ile Gln Phe Val Gly Ala Cys
            420                 425                 430

Leu Asn Asp Pro Ser Gln Phe Ala Ile Val Thr Gln Tyr Ile Ser Gly
        435                 440                 445

Gly Ser Leu Phe Ser Leu Leu His Glu Gln Lys Arg Ile Leu Asp Leu
    450                 455                 460

Gln Ser Lys Leu Ile Ile Ala Val Asp Val Ala Lys Gly Met Glu Tyr
465                 470                 475                 480

Leu His Asn Leu Thr Gln Pro Ile Ile His Arg Asp Leu Asn Ser His
                485                 490                 495

Asn Ile Leu Leu Tyr Glu Asp Gly His Ala Val Val Ala Asp Phe Gly
            500                 505                 510

Glu Ser Arg Phe Leu Gln Ser Leu Asp Glu Asp Asn Met Thr Lys Gln
        515                 520                 525

Pro Gly Asn Leu Arg Trp Met Ala Pro Glu Val Phe Thr Gln Cys Thr
    530                 535                 540

Arg Tyr Thr Ile Lys Ala Asp Val Phe Ser Tyr Ala Leu Cys Leu Trp
545                 550                 555                 560

Glu Ile Leu Thr Gly Glu Ile Pro Phe Ala His Leu Lys Pro Ala Ala
                565                 570                 575

Ala Ala Ala Asp Met Ala Tyr His His Ile Arg Pro Pro Ile Gly Tyr
            580                 585                 590

Ser Ile Pro Lys Pro Ile Ser Ser Leu Leu Ile Arg Gly Trp Asn Ala
        595                 600                 605

Cys Pro Glu Gly Arg Pro Glu Phe Ser Glu Val Val Met Lys Leu Glu
    610                 615                 620

Glu Cys Leu Cys Asn Ile Glu Leu Met Ser Pro Ala Ser Ser Asn Ser
625                 630                 635                 640

Ser Gly Ser Leu Ser Pro Ser Ser Ser Ser Asp Cys Leu Val Asn Arg
                645                 650                 655

Gly Gly Pro Gly Arg Ser His Val Ala Ala Leu Arg Ser Arg Phe Glu
            660                 665                 670
```

```
Leu Glu Tyr Ala Leu Asn Ala Arg Ser Tyr Ala Ala Leu Ser Gln Ser
        675                 680                 685

Ala Gly Gln Tyr Ser Ser Gln Gly Leu Ser Leu Glu Glu Met Lys Arg
    690                 695                 700

Ser Leu Gln Tyr Thr Pro Ile Asp Lys Tyr Gly Tyr Val Ser Asp Pro
705                 710                 715                 720

Met Ser Ser Met His Phe His Ser Cys Arg Asn Ser Ser Ser Phe Glu
                725                 730                 735

Asp Ser Ser



<210>  216
<211>  761
<212>  PRT
<213>  Homo sapiens

<400> 216

Pro Ser Pro Cys Gly Leu His Phe Leu Ile Pro Trp Leu Thr Gln Asp
1               5                   10                  15

Asn Ala Glu Leu Ile Thr Ser Leu Leu His Ser Gly Ala Asp Ile Gln
            20                  25                  30

Gln Val Gly Tyr Gly Gly Leu Thr Ala Leu His Ile Ala Thr Ile Ala
        35                  40                  45

Gly His Leu Glu Ala Ala Asp Val Leu Leu Gln His Gly Ala Asn Val
    50                  55                  60

Asn Ile Gln Asp Ala Val Phe Phe Thr Pro Leu His Ile Ala Ala Tyr
65                  70                  75                  80

Tyr Gly His Glu Gln Val Thr Arg Leu Leu Leu Lys Phe Gly Ala Asp
                85                  90                  95

Val Asn Val Ser Gly Glu Val Gly Asp Arg Pro Leu His Leu Ala Ser
            100                 105                 110

Ala Lys Gly Phe Leu Asn Ile Ala Lys Leu Leu Met Glu Glu Gly Ser
        115                 120                 125

Lys Ala Asp Val Asn Ala Gln Asp Asn Glu Asp His Val Pro Leu His
    130                 135                 140
```

```
Phe Cys Ser Arg Phe Gly His His Asp Ile Val Lys Tyr Leu Leu Gln
145                 150                 155                 160

Ser Asp Leu Glu Val Gln Pro His Val Val Asn Ile Tyr Gly Asp Thr
                165                 170                 175

Pro Leu His Leu Ala Cys Tyr Asn Gly Lys Phe Glu Val Ala Lys Glu
            180                 185                 190

Ile Ile Gln Ile Ser Gly Thr Glu Ser Leu Thr Lys Glu Asn Ile Phe
        195                 200                 205

Ser Glu Thr Ala Phe His Ser Ala Cys Thr Tyr Gly Lys Ser Ile Asp
    210                 215                 220

Leu Val Lys Phe Leu Leu Asp Gln Asn Val Ile Asn Ile Asn His Gln
225                 230                 235                 240

Gly Arg Asp Gly His Thr Gly Leu His Ser Ala Cys Tyr His Gly His
                245                 250                 255

Ile Arg Leu Val Gln Phe Leu Leu Asp Asn Gly Ala Asp Met Asn Leu
            260                 265                 270

Val Ala Cys Asp Pro Ser Arg Ser Ser Gly Glu Lys Asp Glu Gln Thr
        275                 280                 285

Cys Leu Met Trp Ala Tyr Glu Lys Gly His Asp Ala Ile Val Thr Leu
    290                 295                 300

Leu Lys His Tyr Lys Arg Pro Gln Asp Glu Leu Pro Cys Asn Glu Tyr
305                 310                 315                 320

Ser Gln Pro Gly Gly Asp Gly Ser Tyr Val Ser Val Pro Ser Pro Leu
                325                 330                 335

Gly Lys Ile Lys Ser Met Thr Lys Glu Lys Ala Asp Ile Leu Leu Leu
            340                 345                 350

Arg Ala Gly Leu Pro Ser His Phe His Leu Gln Leu Ser Glu Ile Glu
        355                 360                 365

Phe His Glu Ile Ile Gly Ser Gly Ser Phe Gly Lys Val Tyr Lys Gly
    370                 375                 380

Arg Cys Arg Asn Lys Ile Val Ala Ile Lys Arg Tyr Arg Ala Asn Thr
```

```
385                 390                 395                 400

Tyr Cys Ser Lys Ser Asp Val Asp Met Phe Cys Arg Glu Val Ser Ile
                405                 410                 415

Leu Cys Gln Leu Asn His Pro Cys Val Ile Gln Phe Val Gly Ala Cys
            420                 425                 430

Leu Asn Asp Pro Ser Gln Phe Ala Ile Val Thr Gln Tyr Ile Ser Gly
        435                 440                 445

Gly Ser Leu Phe Ser Leu Leu His Glu Gln Lys Arg Ile Leu Asp Leu
    450                 455                 460

Gln Ser Lys Leu Ile Ile Ala Val Asp Val Ala Lys Gly Met Glu Tyr
465                 470                 475                 480

Leu His Asn Leu Thr Gln Pro Ile Ile His Arg Asp Leu Asn Arg Ser
                485                 490                 495

Ala Ile Thr Ser Arg Ile Trp Ile Thr His Ser Ile Cys Ile Trp Arg
            500                 505                 510

Gly Ala His Tyr Phe Asn Arg Glu Glu Cys Asn Phe Arg Cys Met Leu
        515                 520                 525

Thr Ser Ala Ile Leu Lys Glu Ser Arg Phe Leu Gln Ser Leu Asp Glu
    530                 535                 540

Asp Asn Met Thr Lys Gln Pro Gly Asn Leu Arg Trp Met Ala Pro Glu
545                 550                 555                 560

Val Phe Thr Gln Cys Thr Arg Tyr Thr Ile Lys Ala Asp Val Phe Ser
                565                 570                 575

Tyr Ala Leu Cys Leu Trp Glu Ile Leu Thr Gly Glu Ile Pro Phe Ala
            580                 585                 590

His Leu Lys Pro Ala Ala Ala Ala Ala Asp Met Ala Tyr His His Ile
        595                 600                 605

Arg Pro Pro Ile Gly Tyr Ser Ile Pro Lys Pro Ile Ser Ser Leu Leu
    610                 615                 620

Ile Arg Gly Trp Asn Ala Cys Pro Glu Gly Arg Pro Glu Phe Ser Glu
625                 630                 635                 640
```

Val Val Met Lys Leu Glu Glu Cys Leu Cys Asn Ile Glu Leu Met Ser
645 650 655

Pro Ala Ser Ser Asn Ser Ser Gly Ser Leu Ser Pro Ser Ser Ser Ser
660 665 670

Asp Cys Leu Val Asn Arg Gly Gly Pro Gly Arg Ser His Val Ala Ala
675 680 685

Leu Arg Ser Arg Phe Glu Leu Glu Tyr Ala Leu Asn Ala Arg Ser Tyr
690 695 700

Ala Ala Leu Ser Gln Ser Ala Gly Gln Tyr Ser Ser Gln Gly Leu Ser
705 710 715 720

Leu Glu Glu Met Lys Arg Ser Leu Gln Tyr Thr Pro Ile Asp Lys Tyr
725 730 735

Gly Tyr Val Ser Asp Pro Met Ser Ser Met His Phe His Ser Cys Arg
740 745 750

Asn Ser Ser Ser Phe Glu Asp Ser Ser
755 760

<210> 217
<211> 854
<212> PRT
<213> Homo sapiens

<400> 217

Ala Val Arg Arg Gly Leu Arg Glu Gly Gly Ala Met Ala Ala Ala Arg
1 5 10 15

Asp Pro Pro Glu Val Ser Leu Arg Glu Ala Thr Gln Arg Lys Leu Arg
20 25 30

Arg Phe Ser Glu Leu Arg Gly Lys Leu Val Ala Arg Gly Glu Phe Trp
35 40 45

Asp Ile Val Ala Ile Thr Ala Ala Asp Glu Lys Gln Glu Leu Ala Tyr
50 55 60

Asn Gln Gln Leu Ser Glu Lys Leu Lys Arg Lys Glu Leu Pro Leu Gly
65 70 75 80

Val Gln Tyr His Val Phe Val Asp Pro Ala Gly Ala Lys Ile Gly Asn
85 90 95

```
Gly Gly Ser Thr Leu Cys Ala Leu Gln Cys Leu Glu Lys Leu Tyr Gly
            100                 105                 110

Asp Lys Trp Asn Ser Phe Thr Ile Leu Leu Ile His Ser Asp Glu Trp
        115                 120                 125

Lys Lys Lys Val Ser Glu Ser Tyr Val Ile Thr Ile Glu Arg Leu Glu
    130                 135                 140

Asp Asp Leu Gln Ile Lys Glu Lys Glu Leu Thr Glu Leu Arg Asn Ile
145                 150                 155                 160

Phe Gly Ser Asp Glu Ala Phe Ser Lys Val Asn Leu Asn Tyr Arg Thr
                165                 170                 175

Glu Asn Gly Leu Ser Leu Leu His Leu Cys Cys Ile Cys Gly Gly Lys
            180                 185                 190

Lys Ser His Ile Arg Thr Leu Met Leu Lys Gly Leu Arg Pro Ser Arg
        195                 200                 205

Leu Thr Arg Asn Gly Phe Thr Ala Leu His Leu Ala Val Tyr Lys Asp
    210                 215                 220

Asn Ala Glu Leu Ile Thr Ser Leu Leu His Ser Gly Ala Asp Ile Gln
225                 230                 235                 240

Gln Val Gly Tyr Gly Gly Leu Thr Ala Leu His Ile Ala Thr Ile Ala
                245                 250                 255

Gly His Leu Glu Ala Ala Asp Val Leu Leu Gln His Gly Ala Asn Val
            260                 265                 270

Asn Ile Gln Asp Ala Val Phe Phe Thr Pro Leu His Ile Ala Ala Tyr
        275                 280                 285

Tyr Gly His Glu Gln Val Thr Arg Leu Leu Leu Lys Phe Gly Ala Asp
    290                 295                 300

Val Asn Val Ser Gly Glu Val Gly Asp Arg Pro Leu His Leu Ala Ser
305                 310                 315                 320

Ala Lys Gly Phe Leu Asn Ile Ala Lys Leu Leu Met Glu Glu Gly Ser
                325                 330                 335

Lys Ala Asp Val Asn Ala Gln Asp Asn Glu Asp His Val Pro Leu His
            340                 345                 350
```

```
Phe Cys Ser Arg Phe Gly His His Asp Ile Val Lys Tyr Leu Leu Gln
        355                 360                 365

Ser Asp Leu Glu Val Gln Pro His Val Val Asn Ile Tyr Gly Asp Thr
    370                 375                 380

Pro Leu His Leu Ala Cys Tyr Asn Gly Lys Phe Glu Val Ala Lys Glu
385                 390                 395                 400

Ile Ile Gln Ile Ser Gly Thr Glu Ser Leu Thr Lys Glu Asn Ile Phe
                405                 410                 415

Ser Glu Thr Ala Phe His Ser Ala Cys Thr Tyr Gly Lys Ser Ile Asp
            420                 425                 430

Leu Val Lys Phe Leu Leu Asp Gln Asn Val Ile Asn Ile Asn His Gln
        435                 440                 445

Gly Arg Asp Gly His Thr Gly Leu His Ser Ala Cys Tyr His Gly His
    450                 455                 460

Ile Arg Leu Val Gln Phe Leu Leu Asp Asn Gly Ala Asp Met Asn Leu
465                 470                 475                 480

Val Ala Cys Asp Pro Ser Arg Ser Ser Gly Glu Lys Asp Glu Gln Thr
                485                 490                 495

Cys Leu Met Trp Ala Tyr Glu Lys Gly His Asp Ala Ile Val Thr Leu
            500                 505                 510

Leu Lys His Tyr Lys Arg Pro Gln Asp Glu Leu Pro Cys Asn Glu Tyr
        515                 520                 525

Ser Gln Pro Gly Gly Asp Gly Ser Tyr Val Ser Val Pro Ser Pro Leu
    530                 535                 540

Gly Lys Ile Lys Ser Met Thr Lys Glu Lys Ala Asp Ile Leu Leu Leu
545                 550                 555                 560

Arg Ala Gly Leu Pro Ser His Phe His Leu Gln Leu Ser Glu Ile Glu
                565                 570                 575

Phe His Glu Ile Ile Gly Ser Gly Ser Phe Gly Lys Val Tyr Lys Gly
            580                 585                 590

Arg Cys Arg Asn Lys Ile Val Ala Ile Lys Arg Tyr Arg Ala Asn Thr
```

```
        595                 600                 605

Tyr Cys Ser Lys Ser Asp Val Asp Met Phe Cys Arg Glu Val Ser Ile
    610                 615                 620

Leu Cys Gln Leu Asn His Pro Cys Val Ile Gln Phe Val Gly Ala Cys
625                 630                 635                 640

Leu Asn Asp Pro Ser Gln Phe Ala Ile Val Thr Gln Tyr Ile Ser Gly
                645                 650                 655

Gly Ser Leu Phe Ser Leu Leu His Glu Gln Lys Arg Ile Leu Asp Leu
            660                 665                 670

Gln Ser Lys Leu Ile Ile Ala Val Asp Val Ala Lys Gly Met Glu Tyr
        675                 680                 685

Leu His Asn Leu Thr Gln Pro Ile Ile His Arg Asp Leu Asn Ser His
    690                 695                 700

Asn Ile Leu Leu Tyr Glu Asp Gly His Ala Val Val Ala Asp Phe Gly
705                 710                 715                 720

Glu Ser Arg Phe Leu Gln Ser Leu Asp Glu Asp Asn Met Thr Lys Gln
                725                 730                 735

Pro Gly Asn Leu Arg Trp Met Ala Pro Glu Val Phe Thr Gln Cys Thr
            740                 745                 750

Arg Tyr Thr Ile Lys Ala Asp Val Phe Ser Tyr Ala Leu Cys Leu Trp
        755                 760                 765

Glu Ile Leu Thr Gly Glu Ile Pro Phe Ala His Leu Lys Pro Ala Ala
    770                 775                 780

Ala Ala Ala Asp Met Ala Tyr His His Ile Arg Pro Pro Ile Gly Tyr
785                 790                 795                 800

Ser Ile Pro Lys Pro Ile Ser Ser Leu Leu Ile Arg Gly Trp Asn Ala
                805                 810                 815

Cys Pro Glu Ala Lys Ser Arg Pro Ser His Tyr Pro Val Ser Ser Val
            820                 825                 830

Tyr Thr Glu Thr Leu Lys Lys Lys Asn Glu Asp Arg Phe Gly Met Trp
        835                 840                 845
```

Ile Glu Tyr Leu Arg Arg
    850

<210>  218
<211>  742
<212>  PRT
<213>  Homo sapiens

<400> 218

Met Gly Asn Tyr Lys Ser Arg Pro Thr Gln Thr Cys Thr Asp Glu Trp
1               5                   10                  15

Lys Lys Lys Val Ser Glu Ser Tyr Val Ile Thr Ile Glu Arg Leu Glu
            20                  25                  30

Asp Asp Leu Gln Ile Lys Glu Lys Glu Leu Thr Glu Leu Arg Asn Ile
        35                  40                  45

Phe Gly Ser Asp Glu Ala Phe Ser Lys Val Asn Leu Asn Tyr Arg Thr
    50                  55                  60

Glu Asn Gly Leu Ser Leu Leu His Leu Cys Cys Ile Cys Gly Gly Lys
65                  70                  75                  80

Lys Ser His Ile Arg Thr Leu Met Leu Lys Gly Leu Arg Pro Ser Arg
                85                  90                  95

Leu Thr Arg Asn Gly Phe Thr Ala Leu His Leu Ala Val Tyr Lys Asp
            100                 105                 110

Asn Ala Glu Leu Ile Thr Ser Leu Leu His Ser Gly Ala Asp Ile Gln
        115                 120                 125

Gln Val Gly Tyr Gly Gly Leu Thr Ala Leu His Ile Ala Thr Ile Ala
    130                 135                 140

Gly His Leu Glu Ala Ala Asp Val Leu Leu Gln His Gly Ala Asn Val
145                 150                 155                 160

Asn Ile Gln Asp Ala Val Phe Phe Thr Pro Leu His Ile Ala Ala Tyr
                165                 170                 175

Tyr Gly His Glu Gln Val Thr Arg Leu Leu Leu Lys Phe Gly Ala Asp
            180                 185                 190

Val Asn Val Ser Gly Glu Val Gly Asp Arg Pro Leu His Leu Ala Ser
        195                 200                 205

```
Ala Lys Gly Phe Leu Asn Ile Ala Lys Leu Leu Met Glu Glu Gly Ser
    210                 215                 220

Lys Ala Asp Val Asn Ala Gln Asp Asn Glu Asp His Val Pro Leu His
225                 230                 235                 240

Phe Cys Ser Arg Phe Gly His His Asp Ile Val Lys Tyr Leu Leu Gln
                245                 250                 255

Ser Asp Leu Glu Val Gln Pro His Val Val Asn Ile Tyr Gly Asp Thr
            260                 265                 270

Pro Leu His Leu Ala Cys Tyr Asn Gly Lys Phe Glu Val Ala Lys Glu
        275                 280                 285

Ile Ile Gln Ile Ser Gly Thr Glu Ser Leu Thr Lys Glu Asn Ile Phe
    290                 295                 300

Ser Glu Thr Ala Phe His Ser Ala Cys Thr Tyr Gly Lys Ser Ile Asp
305                 310                 315                 320

Leu Val Lys Phe Leu Leu Asp Gln Asn Val Ile Asn Ile Asn His Gln
                325                 330                 335

Gly Arg Asp Gly His Thr Gly Leu His Ser Ala Cys Tyr His Gly His
            340                 345                 350

Ile Arg Leu Val Gln Phe Leu Leu Asp Asn Gly Ala Asp Met Asn Leu
        355                 360                 365

Val Ala Cys Asp Pro Ser Arg Ser Ser Gly Glu Lys Asp Glu Gln Thr
    370                 375                 380

Cys Leu Met Trp Ala Tyr Glu Lys Gly His Asp Ala Ile Val Thr Leu
385                 390                 395                 400

Leu Lys His Tyr Lys Arg Pro Gln Asp Glu Leu Pro Cys Asn Glu Tyr
                405                 410                 415

Ser Gln Pro Gly Gly Asp Gly Ser Tyr Val Ser Val Pro Ser Pro Leu
            420                 425                 430

Gly Lys Ile Lys Ser Met Thr Lys Glu Lys Ala Asp Ile Leu Leu Leu
        435                 440                 445

Arg Ala Gly Leu Pro Ser His Phe His Leu Gln Leu Ser Glu Ile Glu
    450                 455                 460
```

```
Phe His Glu Ile Ile Gly Ser Gly Ser Phe Gly Lys Val Tyr Lys Gly
465                 470                 475                 480

Arg Cys Arg Asn Lys Ile Val Ala Ile Lys Arg Tyr Arg Ala Asn Thr
                485                 490                 495

Tyr Cys Ser Lys Ser Asp Val Asp Met Phe Cys Arg Glu Val Ser Ile
            500                 505                 510

Leu Cys Gln Leu Asn His Pro Cys Val Ile Gln Phe Val Gly Ala Cys
        515                 520                 525

Leu Asn Asp Pro Ser Gln Phe Ala Ile Val Thr Gln Tyr Ile Ser Gly
    530                 535                 540

Gly Ser Leu Phe Ser Leu Leu His Glu Gln Lys Arg Ile Leu Asp Leu
545                 550                 555                 560

Gln Ser Lys Leu Ile Ile Ala Val Asp Val Ala Lys Gly Met Glu Tyr
                565                 570                 575

Leu His Asn Leu Thr Gln Pro Ile Ile His Arg Asp Leu Asn Ser His
            580                 585                 590

Asn Ile Leu Leu Tyr Glu Asp Gly His Ala Val Val Ala Asp Phe Gly
        595                 600                 605

Glu Ser Arg Phe Leu Gln Ser Leu Asp Glu Asp Asn Met Thr Lys Gln
    610                 615                 620

Pro Gly Asn Leu Arg Trp Met Ala Pro Glu Val Phe Thr Gln Cys Thr
625                 630                 635                 640

Arg Tyr Thr Ile Lys Ala Asp Val Phe Ser Tyr Ala Leu Cys Leu Trp
                645                 650                 655

Glu Ile Leu Thr Gly Glu Ile Pro Phe Ala His Leu Lys Pro Ala Ala
            660                 665                 670

Ala Ala Ala Asp Met Ala Tyr His His Ile Arg Pro Pro Ile Gly Tyr
        675                 680                 685

Ser Ile Pro Lys Pro Ile Ser Ser Leu Leu Ile Arg Gly Trp Asn Ala
    690                 695                 700

Cys Pro Glu Ala Lys Ser Arg Pro Ser His Tyr Pro Val Ser Ser Val
```

705 710 715 720

Tyr Thr Glu Thr Leu Lys Lys Lys Asn Glu Asp Arg Phe Gly Met Trp
725 730 735

Ile Glu Tyr Leu Arg Arg
740

<210> 219
<211> 708
<212> PRT
<213> Homo sapiens

<400> 219

Ala Val Arg Arg Gly Leu Arg Glu Gly Gly Ala Met Ala Ala Ala Arg
1 5 10 15

Asp Pro Pro Glu Val Ser Leu Arg Glu Ala Thr Gln Arg Lys Leu Arg
20 25 30

Arg Phe Ser Glu Leu Arg Gly Lys Leu Val Ala Arg Gly Glu Phe Trp
35 40 45

Asp Ile Val Ala Ile Thr Ala Ala Asp Glu Lys Gln Glu Leu Ala Tyr
50 55 60

Asn Gln Gln Leu Ser Glu Lys Leu Lys Arg Lys Glu Leu Pro Leu Gly
65 70 75 80

Val Gln Tyr His Val Phe Val Asp Pro Ala Gly Ala Lys Ile Gly Asn
85 90 95

Gly Gly Ser Thr Leu Cys Ala Leu Gln Cys Leu Glu Lys Leu Tyr Gly
100 105 110

Asp Lys Trp Asn Ser Phe Thr Ile Leu Leu Ile His Ser Asp Glu Trp
115 120 125

Lys Lys Lys Val Ser Glu Ser Tyr Val Ile Thr Ile Glu Arg Leu Glu
130 135 140

Asp Asp Leu Gln Ile Lys Glu Lys Glu Leu Thr Glu Leu Arg Asn Ile
145 150 155 160

Phe Gly Ser Asp Glu Ala Phe Ser Lys Val Asn Leu Asn Tyr Arg Thr
165 170 175

```
Glu Asn Gly Leu Ser Leu Leu His Leu Cys Cys Ile Cys Gly Gly Lys
            180                 185                 190

Lys Ser His Ile Arg Thr Leu Met Leu Lys Gly Leu Arg Pro Ser Arg
        195                 200                 205

Leu Thr Arg Asn Gly Phe Thr Ala Leu His Leu Ala Val Tyr Lys Asp
    210                 215                 220

Asn Ala Glu Leu Ile Thr Ser Leu Leu His Ser Gly Ala Asp Ile Gln
225                 230                 235                 240

Gln Val Gly Tyr Gly Gly Leu Thr Ala Leu His Ile Ala Thr Ile Ala
                245                 250                 255

Gly His Leu Glu Ala Ala Asp Val Leu Leu Gln His Gly Ala Asn Val
            260                 265                 270

Asn Ile Gln Asp Ala Val Phe Phe Thr Pro Leu His Ile Ala Ala Tyr
        275                 280                 285

Tyr Gly His Glu Gln Val Thr Arg Leu Leu Leu Lys Phe Gly Ala Asp
    290                 295                 300

Val Asn Val Ser Gly Glu Val Gly Asp Arg Pro Leu His Leu Ala Ser
305                 310                 315                 320

Ala Lys Gly Phe Leu Asn Ile Ala Lys Leu Leu Met Glu Glu Gly Ser
                325                 330                 335

Lys Ala Asp Val Asn Ala Gln Asp Asn Glu Asp His Val Pro Leu His
            340                 345                 350

Phe Cys Ser Arg Phe Gly His His Asp Ile Val Lys Tyr Leu Leu Gln
        355                 360                 365

Ser Asp Leu Glu Val Gln Pro His Val Val Asn Ile Tyr Gly Asp Thr
    370                 375                 380

Pro Leu His Leu Ala Cys Tyr Asn Gly Lys Phe Glu Val Ala Lys Glu
385                 390                 395                 400

Ile Ile Gln Ile Ser Gly Thr Glu Ser Leu Thr Lys Glu Asn Ile Phe
                405                 410                 415

Ser Glu Thr Ala Phe His Ser Ala Cys Thr Tyr Gly Lys Ser Ile Asp
            420                 425                 430
```

```
Leu Val Lys Phe Leu Leu Asp Gln Asn Val Ile Asn Ile Asn His Gln
        435                 440                 445

Gly Arg Asp Gly His Thr Gly Leu His Ser Ala Cys Tyr His Gly His
    450                 455                 460

Ile Arg Leu Val Gln Phe Leu Leu Asp Asn Gly Ala Asp Met Asn Leu
465                 470                 475                 480

Val Ala Cys Asp Pro Ser Arg Ser Ser Gly Glu Lys Asp Glu Gln Thr
                485                 490                 495

Cys Leu Met Trp Ala Tyr Glu Lys Gly His Asp Ala Ile Val Thr Leu
            500                 505                 510

Leu Lys His Tyr Lys Arg Pro Gln Asp Glu Leu Pro Cys Asn Glu Tyr
        515                 520                 525

Ser Gln Pro Gly Gly Asp Gly Ser Tyr Val Ser Val Pro Ser Pro Leu
    530                 535                 540

Gly Lys Ile Lys Ser Met Thr Lys Glu Lys Ala Asp Ile Leu Leu Leu
545                 550                 555                 560

Arg Ala Gly Leu Pro Ser His Phe His Leu Gln Leu Ser Glu Ile Glu
                565                 570                 575

Phe His Glu Ile Ile Gly Ser Gly Ser Phe Gly Lys Val Tyr Lys Gly
            580                 585                 590

Arg Cys Arg Asn Lys Ile Val Ala Ile Lys Arg Tyr Arg Ala Asn Thr
        595                 600                 605

Tyr Cys Ser Lys Ser Asp Val Asp Met Phe Cys Arg Glu Val Ser Ile
    610                 615                 620

Leu Cys Gln Leu Asn His Pro Cys Val Ile Gln Phe Val Gly Ala Cys
625                 630                 635                 640

Leu Asn Asp Pro Ser Gln Phe Ala Ile Val Thr Gln Tyr Ile Ser Gly
                645                 650                 655

Gly Ser Leu Phe Ser Leu Leu His Glu Gln Lys Arg Ile Leu Asp Leu
            660                 665                 670

Gln Ser Lys Leu Ile Ile Ala Val Asp Val Ala Lys Gly Met Glu Tyr
        675                 680                 685
```

Leu His Asn Leu Thr Gln Pro Ile Ile His Arg Asp Leu Asn Arg Tyr
    690                 695                 700

Phe Phe Pro Lys
705

<210>  220
<211>  603
<212>  PRT
<213>  Homo sapiens

<400> 220

Met Gly Asn Tyr Lys Ser Arg Pro Thr Gln Thr Cys Thr Asp Glu Trp
1               5                   10                  15

Lys Lys Lys Val Ser Glu Ser Tyr Val Ile Thr Ile Glu Arg Leu Glu
            20                  25                  30

Asp Asp Leu Gln Ile Lys Glu Lys Glu Leu Thr Glu Leu Arg Asn Ile
        35                  40                  45

Phe Gly Ser Asp Glu Ala Phe Ser Lys Val Asn Leu Asn Tyr Arg Thr
    50                  55                  60

Glu Asn Gly Leu Ser Leu Leu His Leu Cys Cys Ile Cys Gly Gly Lys
65                  70                  75                  80

Lys Ser His Ile Arg Thr Leu Met Leu Lys Gly Leu Arg Pro Ser Arg
                85                  90                  95

Leu Thr Arg Asn Gly Phe Thr Ala Leu His Leu Ala Val Tyr Lys Asp
            100                 105                 110

Asn Ala Glu Leu Ile Thr Ser Leu Leu His Ser Gly Ala Asp Ile Gln
        115                 120                 125

Gln Val Gly Tyr Gly Gly Leu Thr Ala Leu His Ile Ala Thr Ile Ala
    130                 135                 140

Gly His Leu Glu Ala Ala Asp Val Leu Leu Gln His Gly Ala Asn Val
145                 150                 155                 160

Asn Ile Gln Asp Ala Val Phe Phe Thr Pro Leu His Ile Ala Ala Tyr
                165                 170                 175

Tyr Gly His Glu Gln Val Thr Arg Leu Leu Leu Lys Phe Gly Ala Asp

```
            180                 185                 190

Val Asn Val Ser Gly Glu Val Gly Asp Arg Pro Leu His Leu Ala Ser
        195                 200                 205

Ala Lys Gly Phe Leu Asn Ile Ala Lys Leu Leu Met Glu Glu Gly Ser
    210                 215                 220

Lys Ala Asp Val Asn Ala Gln Asp Asn Glu Asp His Val Pro Leu His
225                 230                 235                 240

Phe Cys Ser Arg Phe Gly His His Asp Ile Val Lys Tyr Leu Leu Gln
                245                 250                 255

Ser Asp Leu Glu Val Gln Pro His Val Val Asn Ile Tyr Gly Asp Thr
            260                 265                 270

Pro Leu His Leu Ala Cys Tyr Asn Gly Lys Phe Glu Val Ala Lys Glu
        275                 280                 285

Ile Ile Gln Ile Ser Gly Thr Glu Ser Leu Thr Lys Glu Asn Ile Phe
    290                 295                 300

Ser Glu Thr Ala Phe His Ser Ala Cys Thr Tyr Gly Lys Ser Ile Asp
305                 310                 315                 320

Leu Val Lys Phe Leu Leu Asp Gln Asn Val Ile Asn Ile Asn His Gln
                325                 330                 335

Gly Arg Asp Gly His Thr Gly Leu His Ser Ala Cys Tyr His Gly His
            340                 345                 350

Ile Arg Leu Val Gln Phe Leu Leu Asp Asn Gly Ala Asp Met Asn Leu
        355                 360                 365

Val Ala Cys Asp Pro Ser Arg Ser Ser Gly Glu Lys Asp Glu Gln Thr
    370                 375                 380

Cys Leu Met Trp Ala Tyr Glu Lys Gly His Asp Ala Ile Val Thr Leu
385                 390                 395                 400

Leu Lys His Tyr Lys Arg Pro Gln Asp Glu Leu Pro Cys Asn Glu Tyr
                405                 410                 415

Ser Gln Pro Gly Gly Asp Gly Ser Tyr Val Ser Val Pro Ser Pro Leu
            420                 425                 430
```

```
Gly Lys Ile Lys Ser Met Thr Lys Glu Lys Ala Asp Ile Leu Leu Leu
        435                 440                 445

Arg Ala Gly Leu Pro Ser His Phe His Leu Gln Leu Ser Glu Ile Glu
    450                 455                 460

Phe His Glu Ile Ile Gly Ser Gly Ser Phe Gly Lys Val Tyr Lys Gly
465                 470                 475                 480

Arg Cys Arg Asn Lys Ile Val Ala Ile Lys Arg Tyr Arg Ala Asn Thr
                485                 490                 495

Tyr Cys Ser Lys Ser Asp Val Asp Met Phe Cys Arg Glu Val Ser Ile
            500                 505                 510

Leu Cys Gln Leu Asn His Pro Cys Val Ile Gln Phe Val Gly Ala Cys
        515                 520                 525

Leu Asn Asp Pro Ser Gln Phe Ala Ile Val Thr Gln Tyr Ile Ser Gly
    530                 535                 540

Gly Ser Leu Phe Ser Leu Leu His Glu Gln Lys Arg Ile Leu Asp Leu
545                 550                 555                 560

Gln Ser Lys Leu Ile Ile Ala Val Asp Val Ala Lys Gly Met Glu Tyr
                565                 570                 575

Leu His Asn Leu Thr Gln Pro Ile Ile His Arg Asp Leu Asn Arg Cys
            580                 585                 590

Cys Thr Gly Trp Leu Ser Cys Tyr His Pro Asp
        595                 600



<210>  221
<211>  593
<212>  PRT
<213>  Homo sapiens

<400> 221

Met Gly Asn Tyr Lys Ser Arg Pro Thr Gln Thr Cys Thr Asp Glu Trp
1               5                   10                  15

Lys Lys Lys Val Ser Glu Ser Tyr Val Ile Thr Ile Glu Arg Leu Glu
            20                  25                  30

Asp Asp Leu Gln Ile Lys Glu Lys Glu Leu Thr Glu Leu Arg Asn Ile
        35                  40                  45
```

```
Phe Gly Ser Asp Glu Ala Phe Ser Lys Val Asn Leu Asn Tyr Arg Thr
    50                  55                  60

Glu Asn Gly Leu Ser Leu Leu His Leu Cys Cys Ile Cys Gly Gly Lys
65                  70                  75                  80

Lys Ser His Ile Arg Thr Leu Met Leu Lys Gly Leu Arg Pro Ser Arg
                85                  90                  95

Leu Thr Arg Asn Gly Phe Thr Ala Leu His Leu Ala Val Tyr Lys Asp
            100                 105                 110

Asn Ala Glu Leu Ile Thr Ser Leu Leu His Ser Gly Ala Asp Ile Gln
        115                 120                 125

Gln Val Gly Tyr Gly Gly Leu Thr Ala Leu His Ile Ala Thr Ile Ala
    130                 135                 140

Gly His Leu Glu Ala Ala Asp Val Leu Leu Gln His Gly Ala Asn Val
145                 150                 155                 160

Asn Ile Gln Asp Ala Val Phe Phe Thr Pro Leu His Ile Ala Ala Tyr
                165                 170                 175

Tyr Gly His Glu Gln Val Thr Arg Leu Leu Leu Lys Phe Gly Ala Asp
            180                 185                 190

Val Asn Val Ser Gly Glu Val Gly Asp Arg Pro Leu His Leu Ala Ser
        195                 200                 205

Ala Lys Gly Phe Leu Asn Ile Ala Lys Leu Leu Met Glu Glu Gly Ser
    210                 215                 220

Lys Ala Asp Val Asn Ala Gln Asp Asn Glu Asp His Val Pro Leu His
225                 230                 235                 240

Phe Cys Ser Arg Phe Gly His His Asp Ile Val Lys Tyr Leu Leu Gln
                245                 250                 255

Ser Asp Leu Glu Val Gln Pro His Val Val Asn Ile Tyr Gly Asp Thr
            260                 265                 270

Pro Leu His Leu Ala Cys Tyr Asn Gly Lys Phe Glu Val Ala Lys Glu
        275                 280                 285

Ile Ile Gln Ile Ser Gly Thr Glu Ser Leu Thr Lys Glu Asn Ile Phe
    290                 295                 300
```

```
Ser Glu Thr Ala Phe His Ser Ala Cys Thr Tyr Gly Lys Ser Ile Asp
305                 310                 315                 320

Leu Val Lys Phe Leu Leu Asp Gln Asn Val Ile Asn Ile Asn His Gln
                325                 330                 335

Gly Arg Asp Gly His Thr Gly Leu His Ser Ala Cys Tyr His Gly His
            340                 345                 350

Ile Arg Leu Val Gln Phe Leu Leu Asp Asn Gly Ala Asp Met Asn Leu
        355                 360                 365

Val Ala Cys Asp Pro Ser Arg Ser Ser Gly Glu Lys Asp Glu Gln Thr
    370                 375                 380

Cys Leu Met Trp Ala Tyr Glu Lys Gly His Asp Ala Ile Val Thr Leu
385                 390                 395                 400

Leu Lys His Tyr Lys Arg Pro Gln Asp Glu Leu Pro Cys Asn Glu Tyr
                405                 410                 415

Ser Gln Pro Gly Gly Asp Gly Ser Tyr Val Ser Val Pro Ser Pro Leu
            420                 425                 430

Gly Lys Ile Lys Ser Met Thr Lys Glu Lys Ala Asp Ile Leu Leu Leu
        435                 440                 445

Arg Ala Gly Leu Pro Ser His Phe His Leu Gln Leu Ser Glu Ile Glu
    450                 455                 460

Phe His Glu Ile Ile Gly Ser Gly Ser Phe Gly Lys Val Tyr Lys Gly
465                 470                 475                 480

Arg Cys Arg Asn Lys Ile Val Ala Ile Lys Arg Tyr Arg Ala Asn Thr
                485                 490                 495

Tyr Cys Ser Lys Ser Asp Val Asp Met Phe Cys Arg Glu Val Ser Ile
            500                 505                 510

Leu Cys Gln Leu Asn His Pro Cys Val Ile Gln Phe Val Gly Ala Cys
        515                 520                 525

Leu Asn Asp Pro Ser Gln Phe Ala Ile Val Thr Gln Tyr Ile Ser Gly
    530                 535                 540

Gly Ser Leu Phe Ser Leu Leu His Glu Gln Lys Arg Ile Leu Asp Leu
```

545 550 555 560

Gln Ser Lys Leu Ile Ile Ala Val Asp Val Ala Lys Gly Met Glu Tyr
565 570 575

Leu His Asn Leu Thr Gln Pro Ile Ile His Arg Asp Leu Asn Arg Ala
580 585 590

Ser

<210> 222
<211> 702
<212> PRT
<213> Homo sapiens

<400> 222

Ala Val Arg Arg Gly Leu Arg Glu Gly Gly Ala Met Ala Ala Ala Arg
1 5 10 15

Asp Pro Pro Glu Val Ser Leu Arg Glu Ala Thr Gln Arg Lys Leu Arg
20 25 30

Arg Phe Ser Glu Leu Arg Gly Lys Leu Val Ala Arg Gly Glu Phe Trp
35 40 45

Asp Ile Val Ala Ile Thr Ala Ala Asp Glu Lys Gln Glu Leu Ala Tyr
50 55 60

Asn Gln Gln Leu Ser Glu Lys Leu Lys Arg Lys Glu Leu Pro Leu Gly
65 70 75 80

Val Gln Tyr His Val Phe Val Asp Pro Ala Gly Ala Lys Ile Gly Asn
85 90 95

Gly Gly Ser Thr Leu Cys Ala Leu Gln Cys Leu Glu Lys Leu Tyr Gly
100 105 110

Asp Lys Trp Asn Ser Phe Thr Ile Leu Leu Ile His Ser Asp Glu Trp
115 120 125

Lys Lys Lys Val Ser Glu Ser Tyr Val Ile Thr Ile Glu Arg Leu Glu
130 135 140

Asp Asp Leu Gln Ile Lys Glu Lys Glu Leu Thr Glu Leu Arg Asn Ile
145 150 155 160

```
Phe Gly Ser Asp Glu Ala Phe Ser Lys Val Asn Leu Asn Tyr Arg Thr
                165                 170                 175

Glu Asn Gly Leu Ser Leu Leu His Leu Cys Cys Ile Cys Gly Gly Lys
            180                 185                 190

Lys Ser His Ile Arg Thr Leu Met Leu Lys Gly Leu Arg Pro Ser Arg
        195                 200                 205

Leu Thr Arg Asn Gly Phe Thr Ala Leu His Leu Ala Val Tyr Lys Asp
    210                 215                 220

Asn Ala Glu Leu Ile Thr Ser Leu Leu His Ser Gly Ala Asp Ile Gln
225                 230                 235                 240

Gln Val Gly Tyr Gly Gly Leu Thr Ala Leu His Ile Ala Thr Ile Ala
                245                 250                 255

Gly His Leu Glu Ala Ala Asp Val Leu Leu Gln His Gly Ala Asn Val
            260                 265                 270

Asn Ile Gln Asp Ala Val Phe Phe Thr Pro Leu His Ile Ala Ala Tyr
        275                 280                 285

Tyr Gly His Glu Gln Val Thr Arg Leu Leu Leu Lys Phe Gly Ala Asp
    290                 295                 300

Val Asn Val Ser Gly Glu Val Gly Asp Arg Pro Leu His Leu Ala Ser
305                 310                 315                 320

Ala Lys Gly Phe Leu Asn Ile Ala Lys Leu Leu Met Glu Glu Gly Ser
                325                 330                 335

Lys Ala Asp Val Asn Ala Gln Asp Asn Glu Asp His Val Pro Leu His
            340                 345                 350

Phe Cys Ser Arg Phe Gly His His Asp Ile Val Lys Tyr Leu Leu Gln
        355                 360                 365

Ser Asp Leu Glu Val Gln Pro His Val Val Asn Ile Tyr Gly Asp Thr
    370                 375                 380

Pro Leu His Leu Ala Cys Tyr Asn Gly Lys Phe Glu Val Ala Lys Glu
385                 390                 395                 400

Ile Ile Gln Ile Ser Gly Thr Glu Ser Leu Thr Lys Glu Asn Ile Phe
                405                 410                 415
```

```
Ser Glu Thr Ala Phe His Ser Ala Cys Thr Tyr Gly Lys Ser Ile Asp
            420                 425                 430

Leu Val Lys Phe Leu Leu Asp Gln Asn Val Ile Asn Ile Asn His Gln
        435                 440                 445

Gly Arg Asp Gly His Thr Gly Leu His Ser Ala Cys Tyr His Gly His
    450                 455                 460

Ile Arg Leu Val Gln Phe Leu Leu Asp Asn Gly Ala Asp Met Asn Leu
465                 470                 475                 480

Val Ala Cys Asp Pro Ser Arg Ser Ser Gly Glu Lys Asp Glu Gln Thr
                485                 490                 495

Cys Leu Met Trp Ala Tyr Glu Lys Gly His Asp Ala Ile Val Thr Leu
            500                 505                 510

Leu Lys His Tyr Lys Arg Pro Gln Asp Glu Leu Pro Cys Asn Glu Tyr
        515                 520                 525

Ser Gln Pro Gly Gly Asp Gly Ser Tyr Val Ser Val Pro Ser Pro Leu
    530                 535                 540

Gly Lys Ile Lys Ser Met Thr Lys Glu Lys Ala Asp Ile Leu Leu Leu
545                 550                 555                 560

Arg Ala Gly Leu Pro Ser His Phe His Leu Gln Leu Ser Glu Ile Glu
                565                 570                 575

Phe His Glu Ile Ile Gly Ser Gly Ser Phe Gly Lys Val Tyr Lys Gly
            580                 585                 590

Arg Cys Arg Asn Lys Ile Val Ala Ile Lys Arg Tyr Arg Ala Asn Thr
        595                 600                 605

Tyr Cys Ser Lys Ser Asp Val Asp Met Phe Cys Arg Glu Val Ser Ile
    610                 615                 620

Leu Cys Gln Leu Asn His Pro Cys Val Ile Gln Phe Val Gly Ala Cys
625                 630                 635                 640

Leu Asn Asp Pro Ser Gln Phe Ala Ile Val Thr Gln Tyr Ile Ser Gly
                645                 650                 655

Gly Ser Leu Phe Ser Leu Leu His Glu Gln Lys Arg Tyr Gly Ser Phe
            660                 665                 670
```

```
Val Leu Ile Tyr Pro Trp Thr Phe Arg Arg Asn Tyr Ser Cys Asn Thr
        675                 680                 685

Ser Glu Gly Phe Pro Leu Asp Glu Pro Ser Pro Phe Glu Ile
    690                 695                 700


<210>  223
<211>  590
<212>  PRT
<213>  Homo sapiens

<400> 223

Met Gly Asn Tyr Lys Ser Arg Pro Thr Gln Thr Cys Thr Asp Glu Trp
1               5                   10                  15

Lys Lys Lys Val Ser Glu Ser Tyr Val Ile Thr Ile Glu Arg Leu Glu
            20                  25                  30

Asp Asp Leu Gln Ile Lys Glu Lys Glu Leu Thr Glu Leu Arg Asn Ile
        35                  40                  45

Phe Gly Ser Asp Glu Ala Phe Ser Lys Val Asn Leu Asn Tyr Arg Thr
    50                  55                  60

Glu Asn Gly Leu Ser Leu Leu His Leu Cys Cys Ile Cys Gly Gly Lys
65                  70                  75                  80

Lys Ser His Ile Arg Thr Leu Met Leu Lys Gly Leu Arg Pro Ser Arg
                85                  90                  95

Leu Thr Arg Asn Gly Phe Thr Ala Leu His Leu Ala Val Tyr Lys Asp
            100                 105                 110

Asn Ala Glu Leu Ile Thr Ser Leu Leu His Ser Gly Ala Asp Ile Gln
        115                 120                 125

Gln Val Gly Tyr Gly Gly Leu Thr Ala Leu His Ile Ala Thr Ile Ala
    130                 135                 140

Gly His Leu Glu Ala Ala Asp Val Leu Leu Gln His Gly Ala Asn Val
145                 150                 155                 160

Asn Ile Gln Asp Ala Val Phe Phe Thr Pro Leu His Ile Ala Ala Tyr
                165                 170                 175

Tyr Gly His Glu Gln Val Thr Arg Leu Leu Leu Lys Phe Gly Ala Asp
```

```
            180                 185                 190

Val Asn Val Ser Gly Glu Val Gly Asp Arg Pro Leu His Leu Ala Ser
        195                 200                 205

Ala Lys Gly Phe Leu Asn Ile Ala Lys Leu Leu Met Glu Glu Gly Ser
    210                 215                 220

Lys Ala Asp Val Asn Ala Gln Asp Asn Glu Asp His Val Pro Leu His
225                 230                 235                 240

Phe Cys Ser Arg Phe Gly His His Asp Ile Val Lys Tyr Leu Leu Gln
                245                 250                 255

Ser Asp Leu Glu Val Gln Pro His Val Val Asn Ile Tyr Gly Asp Thr
            260                 265                 270

Pro Leu His Leu Ala Cys Tyr Asn Gly Lys Phe Glu Val Ala Lys Glu
        275                 280                 285

Ile Ile Gln Ile Ser Gly Thr Glu Ser Leu Thr Lys Glu Asn Ile Phe
    290                 295                 300

Ser Glu Thr Ala Phe His Ser Ala Cys Thr Tyr Gly Lys Ser Ile Asp
305                 310                 315                 320

Leu Val Lys Phe Leu Leu Asp Gln Asn Val Ile Asn Ile Asn His Gln
                325                 330                 335

Gly Arg Asp Gly His Thr Gly Leu His Ser Ala Cys Tyr His Gly His
            340                 345                 350

Ile Arg Leu Val Gln Phe Leu Leu Asp Asn Gly Ala Asp Met Asn Leu
        355                 360                 365

Val Ala Cys Asp Pro Ser Arg Ser Ser Gly Glu Lys Asp Glu Gln Thr
    370                 375                 380

Cys Leu Met Trp Ala Tyr Glu Lys Gly His Asp Ala Ile Val Thr Leu
385                 390                 395                 400

Leu Lys His Tyr Lys Arg Pro Gln Asp Glu Leu Pro Cys Asn Glu Tyr
                405                 410                 415

Ser Gln Pro Gly Gly Asp Gly Ser Tyr Val Ser Val Pro Ser Pro Leu
            420                 425                 430
```

```
Gly Lys Ile Lys Ser Met Thr Lys Glu Lys Ala Asp Ile Leu Leu Leu
        435                 440                 445

Arg Ala Gly Leu Pro Ser His Phe His Leu Gln Leu Ser Glu Ile Glu
    450                 455                 460

Phe His Glu Ile Ile Gly Ser Gly Ser Phe Gly Lys Val Tyr Lys Gly
465                 470                 475                 480

Arg Cys Arg Asn Lys Ile Val Ala Ile Lys Arg Tyr Arg Ala Asn Thr
                485                 490                 495

Tyr Cys Ser Lys Ser Asp Val Asp Met Phe Cys Arg Glu Val Ser Ile
            500                 505                 510

Leu Cys Gln Leu Asn His Pro Cys Val Ile Gln Phe Val Gly Ala Cys
        515                 520                 525

Leu Asn Asp Pro Ser Gln Phe Ala Ile Val Thr Gln Tyr Ile Ser Gly
    530                 535                 540

Gly Ser Leu Phe Ser Leu Leu His Glu Gln Lys Arg Tyr Gly Ser Phe
545                 550                 555                 560

Val Leu Ile Tyr Pro Trp Thr Phe Arg Arg Asn Tyr Ser Cys Asn Thr
                565                 570                 575

Ser Glu Gly Phe Pro Leu Asp Glu Pro Ser Pro Phe Glu Ile
            580                 585                 590


<210>  224
<211>  475
<212>  PRT
<213>  Homo sapiens

<400> 224

Met Gly Asn Tyr Lys Ser Arg Pro Thr Gln Thr Cys Thr Asp Glu Trp
1               5                   10                  15

Lys Lys Lys Val Ser Glu Ser Tyr Val Ile Thr Ile Glu Arg Leu Glu
            20                  25                  30

Asp Asp Leu Gln Ile Lys Glu Lys Glu Leu Thr Glu Leu Arg Asn Ile
        35                  40                  45

Phe Gly Ser Asp Glu Ala Phe Ser Lys Val Asn Leu Asn Tyr Arg Thr
    50                  55                  60
```

```
Glu Asn Gly Leu Ser Leu Leu His Leu Cys Cys Ile Cys Gly Gly Lys
65                  70                  75                  80

Lys Ser His Ile Arg Thr Leu Met Leu Lys Gly Leu Arg Pro Ser Arg
                85                  90                  95

Leu Thr Arg Asn Gly Phe Thr Ala Leu His Leu Ala Val Tyr Lys Asp
            100                 105                 110

Asn Ala Glu Leu Ile Thr Ser Leu Leu His Ser Gly Ala Asp Ile Gln
        115                 120                 125

Gln Val Gly Tyr Gly Gly Leu Thr Ala Leu His Ile Ala Thr Ile Ala
    130                 135                 140

Gly His Leu Glu Ala Ala Asp Val Leu Leu Gln His Gly Ala Asn Val
145                 150                 155                 160

Asn Ile Gln Asp Ala Val Phe Phe Thr Pro Leu His Ile Ala Ala Tyr
                165                 170                 175

Tyr Gly His Glu Gln Val Thr Arg Leu Leu Leu Lys Phe Gly Ala Asp
            180                 185                 190

Val Asn Val Ser Gly Glu Val Gly Asp Arg Pro Leu His Leu Ala Ser
        195                 200                 205

Ala Lys Gly Phe Leu Asn Ile Ala Lys Leu Leu Met Glu Glu Gly Ser
    210                 215                 220

Lys Ala Asp Val Asn Ala Gln Asp Asn Glu Asp His Val Pro Leu His
225                 230                 235                 240

Phe Cys Ser Arg Phe Gly His His Asp Ile Val Lys Tyr Leu Leu Gln
                245                 250                 255

Ser Asp Leu Glu Val Gln Pro His Val Val Asn Ile Tyr Gly Asp Thr
            260                 265                 270

Pro Leu His Leu Ala Cys Tyr Asn Gly Lys Phe Glu Val Ala Lys Glu
        275                 280                 285

Ile Ile Gln Ile Ser Gly Thr Glu Ser Leu Thr Lys Glu Asn Ile Phe
    290                 295                 300

Ser Glu Thr Ala Phe His Ser Ala Cys Thr Tyr Gly Lys Ser Ile Asp
305                 310                 315                 320
```

```
Leu Val Lys Phe Leu Leu Asp Gln Asn Val Ile Asn Ile Asn His Gln
                325                 330                 335

Gly Arg Asp Gly His Thr Gly Leu His Ser Ala Cys Tyr His Gly His
            340                 345                 350

Ile Arg Leu Val Gln Phe Leu Leu Asp Asn Gly Ala Asp Met Asn Leu
        355                 360                 365

Val Ala Cys Asp Pro Ser Arg Ser Ser Gly Glu Lys Asp Glu Gln Thr
    370                 375                 380

Cys Leu Met Trp Ala Tyr Glu Lys Gly His Asp Ala Ile Val Thr Leu
385                 390                 395                 400

Leu Lys His Tyr Lys Arg Pro Gln Asp Glu Leu Pro Cys Asn Glu Tyr
                405                 410                 415

Ser Gln Pro Gly Gly Asp Gly Ser Tyr Val Ser Val Pro Ser Pro Leu
            420                 425                 430

Gly Lys Ile Lys Ser Met Thr Lys Glu Lys Ala Asp Ile Leu Leu Leu
        435                 440                 445

Arg Ala Gly Leu Pro Ser His Phe His Leu Gln Leu Ser Glu Ile Glu
    450                 455                 460

Phe His Glu Ile Ile Gly Ser Gly Asn Leu Lys
465                 470                 475


<210>  225
<211>  254
<212>  PRT
<213>  Homo sapiens

<400> 225

Met Asp Val Asn Leu Tyr Leu Cys Leu Leu Arg Ala Ser Gly Glu His
1               5                   10                  15

Ser Lys Phe Ser Leu Arg Arg Met Leu Leu Cys Ser Gly Arg Thr Lys
            20                  25                  30

His Leu Val Asn Gly Leu Trp Met Phe Leu Asp Val Gln Asn Leu Arg
        35                  40                  45

Trp Met Ala Pro Glu Val Phe Thr Gln Cys Thr Arg Tyr Thr Ile Lys
```

50 55 60

Ala Asp Val Phe Ser Tyr Ala Leu Cys Leu Trp Glu Ile Leu Thr Gly
65 70 75 80

Glu Ile Pro Phe Ala His Leu Lys Pro Ala Ala Ala Ala Ala Asp Met
85 90 95

Ala Tyr His His Ile Arg Pro Pro Ile Gly Tyr Ser Ile Pro Lys Pro
100 105 110

Ile Ser Ser Leu Leu Ile Arg Gly Trp Asn Ala Cys Pro Glu Gly Arg
115 120 125

Pro Glu Phe Ser Glu Val Val Met Lys Leu Glu Glu Cys Leu Cys Asn
130 135 140

Ile Glu Leu Met Ser Pro Ala Ser Ser Asn Ser Ser Gly Ser Leu Ser
145 150 155 160

Pro Ser Ser Ser Ser Asp Cys Leu Val Asn Arg Gly Gly Pro Gly Arg
165 170 175

Ser His Val Ala Ala Leu Arg Ser Arg Phe Glu Leu Glu Tyr Ala Leu
180 185 190

Asn Ala Arg Ser Tyr Ala Ala Leu Ser Gln Ser Ala Gly Gln Tyr Ser
195 200 205

Ser Gln Gly Leu Ser Leu Glu Glu Met Lys Arg Ser Leu Gln Tyr Thr
210 215 220

Pro Ile Asp Lys Tyr Gly Tyr Val Ser Asp Pro Met Ser Ser Met His
225 230 235 240

Phe His Ser Cys Arg Asn Ser Ser Ser Phe Glu Asp Ser Ser
245 250

<210> 226
<211> 233
<212> PRT
<213> Homo sapiens

<400> 226

Met Asp Val Asn Leu Tyr Leu Cys Leu Leu Arg Ala Ser Gly Glu His
1 5 10 15

```
Ser Lys Phe Ser Leu Arg Arg Met Leu Leu Cys Ser Gly Arg Thr Lys
            20                  25                  30

His Leu Val Asn Gly Leu Trp Met Phe Leu Asp Val Gln Asn Leu Arg
        35                  40                  45

Trp Met Ala Pro Glu Val Phe Thr Gln Cys Thr Arg Tyr Thr Ile Lys
    50                  55                  60

Ala Asp Val Phe Ser Tyr Ala Leu Cys Leu Trp Glu Ile Leu Thr Gly
65                  70                  75                  80

Glu Ile Pro Phe Ala His Leu Lys Pro Ala Ala Ala Ala Ala Asp Met
                85                  90                  95

Ala Tyr His His Ile Arg Pro Pro Ile Gly Tyr Ser Ile Pro Lys Pro
            100                 105                 110

Ile Ser Ser Leu Leu Ile Arg Gly Trp Asn Ala Cys Pro Glu Gly Arg
        115                 120                 125

Pro Glu Phe Ser Glu Val Val Met Lys Leu Glu Glu Cys Leu Cys Asn
    130                 135                 140

Ile Glu Leu Met Ser Pro Ala Ser Ser Asn Ser Ser Gly Ser Leu Ser
145                 150                 155                 160

Pro Ser Ser Ser Ser Asp Cys Leu Val Asn Arg Gly Gly Pro Gly Arg
                165                 170                 175

Ser His Val Ala Ala Leu Arg Ser Arg Phe Glu Leu Glu Tyr Ala Leu
            180                 185                 190

Asn Ala Arg Ser Tyr Ala Ala Leu Ser Gln Ser Ala Gly Gln Tyr Ser
        195                 200                 205

Ser Gln Gly Leu Ser Leu Glu Glu Met Lys Arg Ser Leu Gln Tyr Thr
    210                 215                 220

Pro Ile Asp Lys Tyr Asp Val Thr Ser
225                 230
```

<210> 227
<211> 958
<212> PRT
<213> Homo sapiens

<400> 227

```
Met Ala Ala Ala Arg Asp Pro Pro Glu Val Ser Leu Arg Glu Ala Thr
1               5                   10                  15

Gln Arg Lys Leu Arg Arg Phe Ser Glu Leu Arg Gly Lys Leu Val Ala
            20                  25                  30

Arg Gly Glu Phe Trp Asp Ile Val Ala Ile Thr Ala Ala Asp Glu Lys
        35                  40                  45

Gln Glu Leu Ala Tyr Asn Gln Gln Leu Ser Glu Lys Leu Lys Arg Lys
    50                  55                  60

Glu Leu Pro Leu Gly Val Gln Tyr His Val Phe Val Asp Pro Ala Gly
65                  70                  75                  80

Ala Lys Ile Gly Asn Gly Gly Ser Thr Leu Cys Ala Leu Gln Cys Leu
                85                  90                  95

Glu Lys Leu Tyr Gly Asp Lys Trp Asn Ser Phe Thr Ile Leu Leu Ile
            100                 105                 110

His Ser Asp Glu Trp Lys Lys Lys Val Ser Glu Ser Tyr Val Ile Thr
        115                 120                 125

Ile Glu Arg Leu Glu Asp Asp Leu Gln Ile Lys Glu Lys Glu Leu Thr
    130                 135                 140

Glu Leu Arg Asn Ile Phe Gly Ser Asp Glu Ala Phe Ser Lys Val Asn
145                 150                 155                 160

Leu Asn Tyr Arg Thr Glu Asn Gly Leu Ser Leu Leu His Leu Cys Cys
                165                 170                 175

Ile Cys Gly Gly Lys Lys Ser His Ile Arg Thr Leu Met Leu Lys Gly
            180                 185                 190

Leu Arg Pro Ser Arg Leu Thr Arg Asn Gly Phe Thr Ala Leu His Leu
        195                 200                 205

Ala Val Tyr Lys Asp Asn Ala Glu Leu Ile Thr Ser Leu Leu His Ser
    210                 215                 220

Gly Ala Asp Ile Gln Gln Val Gly Tyr Gly Gly Leu Thr Ala Leu His
225                 230                 235                 240

Ile Ala Thr Ile Ala Gly His Leu Glu Ala Ala Asp Val Leu Leu Gln
                245                 250                 255
```

His Gly Ala Asn Val Asn Ile Gln Asp Ala Val Phe Phe Thr Pro Leu
260 265 270

His Ile Ala Ala Tyr Tyr Gly His Glu Gln Val Thr Arg Leu Leu Leu
275 280 285

Lys Phe Gly Ala Asp Val Asn Val Ser Gly Glu Val Gly Asp Arg Pro
290 295 300

Leu His Leu Ala Ser Ala Lys Gly Phe Leu Asn Ile Ala Lys Leu Leu
305 310 315 320

Met Glu Glu Gly Ser Lys Ala Asp Val Asn Ala Gln Asp Asn Glu Asp
325 330 335

His Val Pro Leu His Phe Cys Ser Arg Phe Gly His His Asp Ile Val
340 345 350

Lys Tyr Leu Leu Gln Ser Asp Leu Glu Val Gln Pro His Val Val Asn
355 360 365

Ile Tyr Gly Asp Thr Pro Leu His Leu Ala Cys Tyr Asn Gly Lys Phe
370 375 380

Glu Val Ala Lys Glu Ile Ile Gln Ile Ser Gly Thr Glu Ser Leu Thr
385 390 395 400

Lys Glu Asn Ile Phe Ser Glu Thr Ala Phe His Ser Ala Cys Thr Tyr
405 410 415

Gly Lys Ser Ile Asp Leu Val Lys Phe Leu Leu Asp Gln Asn Val Ile
420 425 430

Asn Ile Asn His Gln Gly Arg Asp Gly His Thr Gly Leu His Ser Ala
435 440 445

Cys Tyr His Gly His Ile Arg Leu Val Gln Phe Leu Leu Asp Asn Gly
450 455 460

Ala Asp Met Asn Leu Val Ala Cys Asp Pro Ser Arg Ser Ser Gly Glu
465 470 475 480

Lys Asp Glu Gln Thr Cys Leu Met Trp Ala Tyr Glu Lys Gly His Asp
485 490 495

Ala Ile Val Thr Leu Leu Lys His Tyr Lys Arg Pro Gln Asp Glu Leu

500 505 510

Pro Cys Asn Glu Tyr Ser Gln Pro Gly Gly Asp Gly Ser Tyr Val Ser
515 520 525

Val Pro Ser Pro Leu Gly Lys Ile Lys Ser Met Thr Lys Glu Lys Ala
530 535 540

Asp Ile Leu Leu Leu Arg Ala Gly Leu Pro Ser His Phe His Leu Gln
545 550 555 560

Leu Ser Glu Ile Glu Phe His Glu Ile Ile Gly Ser Gly Ser Phe Gly
565 570 575

Lys Val Tyr Lys Gly Arg Cys Arg Asn Lys Ile Val Ala Ile Lys Arg
580 585 590

Tyr Arg Ala Asn Thr Tyr Cys Ser Lys Ser Asp Val Asp Met Phe Cys
595 600 605

Arg Glu Val Ser Ile Leu Cys Gln Leu Asn His Pro Cys Val Ile Gln
610 615 620

Phe Val Gly Ala Cys Leu Asn Asp Pro Ser Gln Phe Ala Ile Val Thr
625 630 635 640

Gln Tyr Ile Ser Gly Gly Ser Leu Phe Ser Leu Leu His Glu Gln Lys
645 650 655

Arg Ile Leu Asp Leu Gln Ser Lys Leu Ile Ile Ala Val Asp Val Ala
660 665 670

Lys Gly Met Glu Tyr Leu His Asn Leu Thr Gln Pro Ile Ile His Arg
675 680 685

Asp Leu Asn Arg Ser Ala Ile Thr Ser Arg Ile Trp Ile Thr His Ser
690 695 700

Ile Cys Ile Trp Arg Gly Ala His Tyr Phe Asn Arg Glu Glu Cys Asn
705 710 715 720

Phe Arg Cys Met Leu Thr Ser Ala Ile Leu Lys Glu Ser Arg Phe Leu
725 730 735

Gln Ser Leu Asp Glu Asp Asn Met Thr Lys Gln Pro Gly Asn Leu Arg
740 745 750

Trp Met Ala Pro Glu Val Phe Thr Gln Cys Thr Arg Tyr Thr Ile Lys
755 760 765

Ala Asp Val Phe Ser Tyr Ala Leu Cys Leu Trp Glu Ile Leu Thr Gly
770 775 780

Glu Ile Pro Phe Ala His Leu Lys Pro Ala Ala Ala Ala Ala Asp Met
785 790 795 800

Ala Tyr His His Ile Arg Pro Pro Ile Gly Tyr Ser Ile Pro Lys Pro
805 810 815

Ile Ser Ser Leu Leu Ile Arg Gly Trp Asn Ala Cys Pro Glu Gly Arg
820 825 830

Pro Glu Phe Ser Glu Val Val Met Lys Leu Glu Glu Cys Leu Cys Asn
835 840 845

Ile Glu Leu Met Ser Pro Ala Ser Ser Asn Ser Ser Gly Ser Leu Ser
850 855 860

Pro Ser Ser Ser Ser Asp Cys Leu Val Asn Arg Gly Gly Pro Gly Arg
865 870 875 880

Ser His Val Ala Ala Leu Arg Ser Arg Phe Glu Leu Glu Tyr Ala Leu
885 890 895

Asn Ala Arg Ser Tyr Ala Ala Leu Ser Gln Ser Ala Gly Gln Tyr Ser
900 905 910

Ser Gln Gly Leu Ser Leu Glu Glu Met Lys Arg Ser Leu Gln Tyr Thr
915 920 925

Pro Ile Asp Lys Tyr Gly Tyr Val Ser Asp Pro Met Ser Ser Met His
930 935 940

Phe His Ser Cys Arg Asn Ser Ser Ser Phe Glu Asp Ser Ser
945 950 955

<210> 228
<211> 843
<212> PRT
<213> Homo sapiens

<400> 228

Met Ala Ala Ala Arg Asp Pro Pro Glu Val Ser Leu Arg Glu Ala Thr
1 5 10 15

```
Gln Arg Lys Leu Arg Arg Phe Ser Glu Leu Arg Gly Lys Leu Val Ala
            20                  25                  30

Arg Gly Glu Phe Trp Asp Ile Val Ala Ile Thr Ala Ala Asp Glu Lys
        35                  40                  45

Gln Glu Leu Ala Tyr Asn Gln Gln Leu Ser Glu Lys Leu Lys Arg Lys
    50                  55                  60

Glu Leu Pro Leu Gly Val Gln Tyr His Val Phe Val Asp Pro Ala Gly
65                  70                  75                  80

Ala Lys Ile Gly Asn Gly Gly Ser Thr Leu Cys Ala Leu Gln Cys Leu
                85                  90                  95

Glu Lys Leu Tyr Gly Asp Lys Trp Asn Ser Phe Thr Ile Leu Leu Ile
            100                 105                 110

His Ser Asp Glu Trp Lys Lys Lys Val Ser Glu Ser Tyr Val Ile Thr
        115                 120                 125

Ile Glu Arg Leu Glu Asp Asp Leu Gln Ile Lys Glu Lys Glu Leu Thr
    130                 135                 140

Glu Leu Arg Asn Ile Phe Gly Ser Asp Glu Ala Phe Ser Lys Val Asn
145                 150                 155                 160

Leu Asn Tyr Arg Thr Glu Asn Gly Leu Ser Leu Leu His Leu Cys Cys
                165                 170                 175

Ile Cys Gly Gly Lys Lys Ser His Ile Arg Thr Leu Met Leu Lys Gly
            180                 185                 190

Leu Arg Pro Ser Arg Leu Thr Arg Asn Gly Phe Thr Ala Leu His Leu
        195                 200                 205

Ala Val Tyr Lys Asp Asn Ala Glu Leu Ile Thr Ser Leu Leu His Ser
    210                 215                 220

Gly Ala Asp Ile Gln Gln Val Gly Tyr Gly Gly Leu Thr Ala Leu His
225                 230                 235                 240

Ile Ala Thr Ile Ala Gly His Leu Glu Ala Ala Asp Val Leu Leu Gln
                245                 250                 255

His Gly Ala Asn Val Asn Ile Gln Asp Ala Val Phe Phe Thr Pro Leu
            260                 265                 270
```

```
His Ile Ala Ala Tyr Tyr Gly His Glu Gln Val Thr Arg Leu Leu Leu
        275                 280                 285

Lys Phe Gly Ala Asp Val Asn Val Ser Gly Glu Val Gly Asp Arg Pro
    290                 295                 300

Leu His Leu Ala Ser Ala Lys Gly Phe Leu Asn Ile Ala Lys Leu Leu
305                 310                 315                 320

Met Glu Glu Gly Ser Lys Ala Asp Val Asn Ala Gln Asp Asn Glu Asp
                325                 330                 335

His Val Pro Leu His Phe Cys Ser Arg Phe Gly His His Asp Ile Val
            340                 345                 350

Lys Tyr Leu Leu Gln Ser Asp Leu Glu Val Gln Pro His Val Val Asn
        355                 360                 365

Ile Tyr Gly Asp Thr Pro Leu His Leu Ala Cys Tyr Asn Gly Lys Phe
    370                 375                 380

Glu Val Ala Lys Glu Ile Ile Gln Ile Ser Gly Thr Glu Ser Leu Thr
385                 390                 395                 400

Lys Glu Asn Ile Phe Ser Glu Thr Ala Phe His Ser Ala Cys Thr Tyr
                405                 410                 415

Gly Lys Ser Ile Asp Leu Val Lys Phe Leu Leu Asp Gln Asn Val Ile
            420                 425                 430

Asn Ile Asn His Gln Gly Arg Asp Gly His Thr Gly Leu His Ser Ala
        435                 440                 445

Cys Tyr His Gly His Ile Arg Leu Val Gln Phe Leu Leu Asp Asn Gly
    450                 455                 460

Ala Asp Met Asn Leu Val Ala Cys Asp Pro Ser Arg Ser Ser Gly Glu
465                 470                 475                 480

Lys Asp Glu Gln Thr Cys Leu Met Trp Ala Tyr Glu Lys Gly His Asp
                485                 490                 495

Ala Ile Val Thr Leu Leu Lys His Tyr Lys Arg Pro Gln Asp Glu Leu
            500                 505                 510

Pro Cys Asn Glu Tyr Ser Gln Pro Gly Gly Asp Gly Ser Tyr Val Ser
```

515 520 525

Val Pro Ser Pro Leu Gly Lys Ile Lys Ser Met Thr Lys Glu Lys Ala
530 535 540

Asp Ile Leu Leu Leu Arg Ala Gly Leu Pro Ser His Phe His Leu Gln
545 550 555 560

Leu Ser Glu Ile Glu Phe His Glu Ile Ile Gly Ser Gly Ser Phe Gly
565 570 575

Lys Val Tyr Lys Gly Arg Cys Arg Asn Lys Ile Val Ala Ile Lys Arg
580 585 590

Tyr Arg Ala Asn Thr Tyr Cys Ser Lys Ser Asp Val Asp Met Phe Cys
595 600 605

Arg Glu Val Ser Ile Leu Cys Gln Leu Asn His Pro Cys Val Ile Gln
610 615 620

Phe Val Gly Ala Cys Leu Asn Asp Pro Ser Gln Phe Ala Ile Val Thr
625 630 635 640

Gln Tyr Ile Ser Gly Gly Ser Leu Phe Ser Leu Leu His Glu Gln Lys
645 650 655

Arg Ile Leu Asp Leu Gln Ser Lys Leu Ile Ile Ala Val Asp Val Ala
660 665 670

Lys Gly Met Glu Tyr Leu His Asn Leu Thr Gln Pro Ile Ile His Arg
675 680 685

Asp Leu Asn Ser His Asn Ile Leu Leu Tyr Glu Asp Gly His Ala Val
690 695 700

Val Ala Asp Phe Gly Glu Ser Arg Phe Leu Gln Ser Leu Asp Glu Asp
705 710 715 720

Asn Met Thr Lys Gln Pro Gly Asn Leu Arg Trp Met Ala Pro Glu Val
725 730 735

Phe Thr Gln Cys Thr Arg Tyr Thr Ile Lys Ala Asp Val Phe Ser Tyr
740 745 750

Ala Leu Cys Leu Trp Glu Ile Leu Thr Gly Glu Ile Pro Phe Ala His
755 760 765

```
Leu Lys Pro Ala Ala Ala Ala Ala Asp Met Ala Tyr His His Ile Arg
    770                 775                 780

Pro Pro Ile Gly Tyr Ser Ile Pro Lys Pro Ile Ser Ser Leu Leu Ile
785                 790                 795                 800

Arg Gly Trp Asn Ala Cys Pro Glu Ala Lys Ser Arg Pro Ser His Tyr
                805                 810                 815

Pro Val Ser Ser Val Tyr Thr Glu Thr Leu Lys Lys Lys Asn Glu Asp
            820                 825                 830

Arg Phe Gly Met Trp Ile Glu Tyr Leu Arg Arg
        835                 840


<210>  229
<211>  697
<212>  PRT
<213>  Homo sapiens

<400> 229

Met Ala Ala Ala Arg Asp Pro Pro Glu Val Ser Leu Arg Glu Ala Thr
1               5                   10                  15

Gln Arg Lys Leu Arg Arg Phe Ser Glu Leu Arg Gly Lys Leu Val Ala
            20                  25                  30

Arg Gly Glu Phe Trp Asp Ile Val Ala Ile Thr Ala Ala Asp Glu Lys
        35                  40                  45

Gln Glu Leu Ala Tyr Asn Gln Gln Leu Ser Glu Lys Leu Lys Arg Lys
    50                  55                  60

Glu Leu Pro Leu Gly Val Gln Tyr His Val Phe Val Asp Pro Ala Gly
65                  70                  75                  80

Ala Lys Ile Gly Asn Gly Gly Ser Thr Leu Cys Ala Leu Gln Cys Leu
                85                  90                  95

Glu Lys Leu Tyr Gly Asp Lys Trp Asn Ser Phe Thr Ile Leu Leu Ile
            100                 105                 110

His Ser Asp Glu Trp Lys Lys Lys Val Ser Glu Ser Tyr Val Ile Thr
        115                 120                 125

Ile Glu Arg Leu Glu Asp Asp Leu Gln Ile Lys Glu Lys Glu Leu Thr
    130                 135                 140
```

Glu Leu Arg Asn Ile Phe Gly Ser Asp Glu Ala Phe Ser Lys Val Asn
145 150 155 160

Leu Asn Tyr Arg Thr Glu Asn Gly Leu Ser Leu Leu His Leu Cys Cys
165 170 175

Ile Cys Gly Gly Lys Lys Ser His Ile Arg Thr Leu Met Leu Lys Gly
180 185 190

Leu Arg Pro Ser Arg Leu Thr Arg Asn Gly Phe Thr Ala Leu His Leu
195 200 205

Ala Val Tyr Lys Asp Asn Ala Glu Leu Ile Thr Ser Leu Leu His Ser
210 215 220

Gly Ala Asp Ile Gln Gln Val Gly Tyr Gly Gly Leu Thr Ala Leu His
225 230 235 240

Ile Ala Thr Ile Ala Gly His Leu Glu Ala Ala Asp Val Leu Leu Gln
245 250 255

His Gly Ala Asn Val Asn Ile Gln Asp Ala Val Phe Phe Thr Pro Leu
260 265 270

His Ile Ala Ala Tyr Tyr Gly His Glu Gln Val Thr Arg Leu Leu Leu
275 280 285

Lys Phe Gly Ala Asp Val Asn Val Ser Gly Glu Val Gly Asp Arg Pro
290 295 300

Leu His Leu Ala Ser Ala Lys Gly Phe Leu Asn Ile Ala Lys Leu Leu
305 310 315 320

Met Glu Glu Gly Ser Lys Ala Asp Val Asn Ala Gln Asp Asn Glu Asp
325 330 335

His Val Pro Leu His Phe Cys Ser Arg Phe Gly His His Asp Ile Val
340 345 350

Lys Tyr Leu Leu Gln Ser Asp Leu Glu Val Gln Pro His Val Val Asn
355 360 365

Ile Tyr Gly Asp Thr Pro Leu His Leu Ala Cys Tyr Asn Gly Lys Phe
370 375 380

Glu Val Ala Lys Glu Ile Ile Gln Ile Ser Gly Thr Glu Ser Leu Thr
385 390 395 400

```
Lys Glu Asn Ile Phe Ser Glu Thr Ala Phe His Ser Ala Cys Thr Tyr
                405                 410                 415

Gly Lys Ser Ile Asp Leu Val Lys Phe Leu Leu Asp Gln Asn Val Ile
            420                 425                 430

Asn Ile Asn His Gln Gly Arg Asp Gly His Thr Gly Leu His Ser Ala
        435                 440                 445

Cys Tyr His Gly His Ile Arg Leu Val Gln Phe Leu Leu Asp Asn Gly
    450                 455                 460

Ala Asp Met Asn Leu Val Ala Cys Asp Pro Ser Arg Ser Ser Gly Glu
465                 470                 475                 480

Lys Asp Glu Gln Thr Cys Leu Met Trp Ala Tyr Glu Lys Gly His Asp
                485                 490                 495

Ala Ile Val Thr Leu Leu Lys His Tyr Lys Arg Pro Gln Asp Glu Leu
            500                 505                 510

Pro Cys Asn Glu Tyr Ser Gln Pro Gly Gly Asp Gly Ser Tyr Val Ser
        515                 520                 525

Val Pro Ser Pro Leu Gly Lys Ile Lys Ser Met Thr Lys Glu Lys Ala
    530                 535                 540

Asp Ile Leu Leu Leu Arg Ala Gly Leu Pro Ser His Phe His Leu Gln
545                 550                 555                 560

Leu Ser Glu Ile Glu Phe His Glu Ile Ile Gly Ser Gly Ser Phe Gly
                565                 570                 575

Lys Val Tyr Lys Gly Arg Cys Arg Asn Lys Ile Val Ala Ile Lys Arg
            580                 585                 590

Tyr Arg Ala Asn Thr Tyr Cys Ser Lys Ser Asp Val Asp Met Phe Cys
        595                 600                 605

Arg Glu Val Ser Ile Leu Cys Gln Leu Asn His Pro Cys Val Ile Gln
    610                 615                 620

Phe Val Gly Ala Cys Leu Asn Asp Pro Ser Gln Phe Ala Ile Val Thr
625                 630                 635                 640

Gln Tyr Ile Ser Gly Gly Ser Leu Phe Ser Leu Leu His Glu Gln Lys
```

```
                645                 650                 655

Arg Ile Leu Asp Leu Gln Ser Lys Leu Ile Ile Ala Val Asp Val Ala
            660                 665                 670

Lys Gly Met Glu Tyr Leu His Asn Leu Thr Gln Pro Ile Ile His Arg
        675                 680                 685

Asp Leu Asn Arg Tyr Phe Phe Pro Lys
    690                 695
```

<210>  230
<211>  691
<212>  PRT
<213>  Homo sapiens

<400> 230

```
Met Ala Ala Ala Arg Asp Pro Pro Glu Val Ser Leu Arg Glu Ala Thr
1               5                   10                  15

Gln Arg Lys Leu Arg Arg Phe Ser Glu Leu Arg Gly Lys Leu Val Ala
            20                  25                  30

Arg Gly Glu Phe Trp Asp Ile Val Ala Ile Thr Ala Ala Asp Glu Lys
        35                  40                  45

Gln Glu Leu Ala Tyr Asn Gln Gln Leu Ser Glu Lys Leu Lys Arg Lys
    50                  55                  60

Glu Leu Pro Leu Gly Val Gln Tyr His Val Phe Val Asp Pro Ala Gly
65                  70                  75                  80

Ala Lys Ile Gly Asn Gly Gly Ser Thr Leu Cys Ala Leu Gln Cys Leu
                85                  90                  95

Glu Lys Leu Tyr Gly Asp Lys Trp Asn Ser Phe Thr Ile Leu Leu Ile
            100                 105                 110

His Ser Asp Glu Trp Lys Lys Lys Val Ser Glu Ser Tyr Val Ile Thr
        115                 120                 125

Ile Glu Arg Leu Glu Asp Asp Leu Gln Ile Lys Glu Lys Glu Leu Thr
    130                 135                 140

Glu Leu Arg Asn Ile Phe Gly Ser Asp Glu Ala Phe Ser Lys Val Asn
145                 150                 155                 160
```

```
Leu Asn Tyr Arg Thr Glu Asn Gly Leu Ser Leu Leu His Leu Cys Cys
                165                 170                 175

Ile Cys Gly Gly Lys Lys Ser His Ile Arg Thr Leu Met Leu Lys Gly
            180                 185                 190

Leu Arg Pro Ser Arg Leu Thr Arg Asn Gly Phe Thr Ala Leu His Leu
        195                 200                 205

Ala Val Tyr Lys Asp Asn Ala Glu Leu Ile Thr Ser Leu Leu His Ser
    210                 215                 220

Gly Ala Asp Ile Gln Gln Val Gly Tyr Gly Gly Leu Thr Ala Leu His
225                 230                 235                 240

Ile Ala Thr Ile Ala Gly His Leu Glu Ala Ala Asp Val Leu Leu Gln
                245                 250                 255

His Gly Ala Asn Val Asn Ile Gln Asp Ala Val Phe Phe Thr Pro Leu
            260                 265                 270

His Ile Ala Ala Tyr Tyr Gly His Glu Gln Val Thr Arg Leu Leu Leu
        275                 280                 285

Lys Phe Gly Ala Asp Val Asn Val Ser Gly Glu Val Gly Asp Arg Pro
    290                 295                 300

Leu His Leu Ala Ser Ala Lys Gly Phe Leu Asn Ile Ala Lys Leu Leu
305                 310                 315                 320

Met Glu Glu Gly Ser Lys Ala Asp Val Asn Ala Gln Asp Asn Glu Asp
                325                 330                 335

His Val Pro Leu His Phe Cys Ser Arg Phe Gly His His Asp Ile Val
            340                 345                 350

Lys Tyr Leu Leu Gln Ser Asp Leu Glu Val Gln Pro His Val Val Asn
        355                 360                 365

Ile Tyr Gly Asp Thr Pro Leu His Leu Ala Cys Tyr Asn Gly Lys Phe
    370                 375                 380

Glu Val Ala Lys Glu Ile Ile Gln Ile Ser Gly Thr Glu Ser Leu Thr
385                 390                 395                 400

Lys Glu Asn Ile Phe Ser Glu Thr Ala Phe His Ser Ala Cys Thr Tyr
                405                 410                 415
```

```
Gly Lys Ser Ile Asp Leu Val Lys Phe Leu Leu Asp Gln Asn Val Ile
            420                 425                 430

Asn Ile Asn His Gln Gly Arg Asp Gly His Thr Gly Leu His Ser Ala
        435                 440                 445

Cys Tyr His Gly His Ile Arg Leu Val Gln Phe Leu Leu Asp Asn Gly
    450                 455                 460

Ala Asp Met Asn Leu Val Ala Cys Asp Pro Ser Arg Ser Ser Gly Glu
465                 470                 475                 480

Lys Asp Glu Gln Thr Cys Leu Met Trp Ala Tyr Glu Lys Gly His Asp
                485                 490                 495

Ala Ile Val Thr Leu Leu Lys His Tyr Lys Arg Pro Gln Asp Glu Leu
            500                 505                 510

Pro Cys Asn Glu Tyr Ser Gln Pro Gly Gly Asp Gly Ser Tyr Val Ser
        515                 520                 525

Val Pro Ser Pro Leu Gly Lys Ile Lys Ser Met Thr Lys Glu Lys Ala
    530                 535                 540

Asp Ile Leu Leu Leu Arg Ala Gly Leu Pro Ser His Phe His Leu Gln
545                 550                 555                 560

Leu Ser Glu Ile Glu Phe His Glu Ile Ile Gly Ser Gly Ser Phe Gly
                565                 570                 575

Lys Val Tyr Lys Gly Arg Cys Arg Asn Lys Ile Val Ala Ile Lys Arg
            580                 585                 590

Tyr Arg Ala Asn Thr Tyr Cys Ser Lys Ser Asp Val Asp Met Phe Cys
        595                 600                 605

Arg Glu Val Ser Ile Leu Cys Gln Leu Asn His Pro Cys Val Ile Gln
    610                 615                 620

Phe Val Gly Ala Cys Leu Asn Asp Pro Ser Gln Phe Ala Ile Val Thr
625                 630                 635                 640

Gln Tyr Ile Ser Gly Gly Ser Leu Phe Ser Leu Leu His Glu Gln Lys
                645                 650                 655

Arg Tyr Gly Ser Phe Val Leu Ile Tyr Pro Trp Thr Phe Arg Arg Asn
            660                 665                 670
```

```
Tyr Ser Cys Asn Thr Ser Glu Gly Phe Pro Leu Asp Glu Pro Ser Pro
        675                 680                 685

Phe Glu Ile
    690


<210>  231
<211>  23
<212>  DNA
<213>  Artificial Organism

<220>
<223>  Synthetic oligonucleotide

<400> 231

gagccaatac ctactgctcc aag                                             23


<210>  232
<211>  23
<212>  DNA
<213>  Artificial Organism

<220>
<223>  Synthetic oligonucleotide

<400> 232

gcaagcaccc acaaactgaa tta                                             23


<210>  233
<211>  107
<212>  DNA
<213>  Artificial Organism

<220>
<223>  Synthetic oligonucleotide

<400> 233

gagccaatac ctactgctcc aagtcagatg tggatatgtt ttgccgagag gtgtccattc     60

tctgccagct caatcatccc tgcgtaattc agtttgtggg tgcttgc                  107


<210>  234
<211>  24
<212>  DNA
<213>  Artificial Organism

<220>
<223>  Synthetic oligonucleotide

<400> 234

tctgccatta cctctaggat ctgg                                            24
```

<210> 235
<211> 25
<212> DNA
<213> Artificial Organism

<220>
<223> Synthetic oligonucleotide

<400> 235

ggcagaagta agcatacacc tgaaa 25

<210> 236
<211> 108
<212> DNA
<213> Artificial Organism

<220>
<223> Synthetic oligonucleotide

<400> 236

tctgccatta cctctaggat ctggatcacc catagtattt gcatctggag gggagctcat 60

tactttaaca gggaagaatg caatttcagg tgtatgctta cttctgcc 108

<210> 237
<211> 22
<212> DNA
<213> Artificial Organism

<220>
<223> Synthetic oligonucleotide

<400> 237

cgttttggga tgtggattga gt 22

<210> 238
<211> 21
<212> DNA
<213> Artificial Organism

<220>
<223> Synthetic oligonucleotide

<400> 238

accgctttca tggagctaac a 21

<210> 239
<211> 128
<212> DNA
<213> Artificial Organism

<220>
<223> Synthetic oligonucleotide

<400> 239

cgttttggga tgtggattga gtatctcaga agataacctc ttatcctggc cattcaacct 60

gatgtgttac atgtttattt gtttagaatc ttccatcact accaaaatgt tagctccatg 120

aaagcggt 128

<210> 240
<211> 2955
<212> DNA
<213> Homo sapiens

<400> 240

gccgactctc ctggatgcct ggggttggag aagggtgcct ccaaccccct gacccatgcc 60

ccggaaagac agaaattcat ccagagcaga atcagcccaa tgccaggtgc tttcatgtgt 120

tattcatggc atcttactaa tggcccgtga gatagctgtt gttgtcctcc ccctatcgca 180

ggagagctac gtccaggcag cttcagatgc ctccagagcc atcgacatca actcctcgga 240

catcaaggct ctgtatcggc gatgccaggc actggagcac ctggggaagc tggaccaggc 300

cttcaaagac gtgcagcgtt gtgccaccct cgagccacgg aaccagaact tccaggagat 360

gctgaggaga ctcaacacca gcattcagga gaagctccga gtgcagttct ccacagactc 420

gagggtacag aagatgtttg agatcctctt ggatgaaaac agtgaggctg ataagcggga 480

aaaggctgcc aacaatctca ttgtcctagg ccgtgaggaa gcaggggctg agaagatctt 540

ccagaacaat ggagtagcct tgctactgca gcttctggac actaagaagc ctgagctggt 600

gctggctgca gtgcggaccc tgtcgggcat gtgcagcggc caccaagcca gagccacagt 660

gattctgcat gcagtgcgga tagaccgaat ctgtagcctc atggccgtgg agaatgagga 720

gatgtctctg gctgtctgca acctgctcca agccatcatt gactccttgt ctggggagga 780

caagcgggag catcgaggga aggaggaggc cctggttcta gacaccaaga aggacctgaa 840

gcagatcacc agccacctgc tggacatgct agtcagcaag aaggtgtctg gccagggcag 900

ggatcaggcg ctgaacctgc tcaataagaa tgttcccagg aaggaccttg ccattcatga 960

caactcacgt accatctatg tggtggataa tggtctgagg aagatcctga aggttgtggg 1020

gcaggttcca gatctgccat cctgcctgcc cctgactgac aacacccgca tgctggcctc 1080

tatcctcatc aacaagctct atgatgacct gcgctgtgac ccggagcgcg atcacttccg 1140

caagatctgt gaggaatata tcacgggcaa gtttgacccc caggacatgg acaagaactt 1200

gaatgccatc cagacagtgt cagggatcct gcagggcccc tttgacctgg gcaaccagct 1260

gctgggactg aaaggtgtga tggagatgat ggtggcacta tgtggctcag agcgcgagac 1320

ggaccagctg gtggccgtgg aggccctcat ccatgcctcc acgaagctca gccgcgccac 1380

cttcatcatc accaatggag tgtcactgct caaacagatc tacaagacca ccaaaaatga 1440

gaagatcaag atccgcacac tggtgggact ctgtaagctc ggctctgcag gtggcacaga 1500

ctacggtctc aggcagtttg cggaagggtc gacagaaaaa ctggccaaac agtgtcgcaa 1560

gtggctgtgc aatatgtcca tagacactcg gacccgacgc tgggcagtgg agggcctggc 1620

ctacctcacg ctggacgctg atgtgaagga cgactttgtc caggacgtcc ctgccctgca 1680

ggccatgttt gagctggcca aggcaggtac cagtgacaag accatcctgt actcggtggc 1740

caccaccctg gtgaactgca ccaacagcta cgatgtcaag gaggtcatcc cagagcttgt 1800

ccagctcgcc aagttctcca agcagcatgt gcccgaggaa caccccaagg acaagaagga 1860

ctttatagac atgcgggtga agcggcttct gaaggcgggt gtcatctctg ccctggcttg 1920

catggtgaaa gcagatagtg ccatcctcac tgaccagacc aaggagctgc tggccagggt 1980

attcctggca ctgtgtgaca acccaaagga ccgaggcacc attgtggctc aaggtggtgg 2040

caaggccctg attcccctgg ctttggaggg cacagatgtg ggcaaggtga aggcagccca 2100

cgctctagca aagatcgctg ctgtctccaa tccggacatt gcttttcctg ggagcgggt 2160

gtatgaggtg gtgcggcccc ttgtaagact cttggacaca cagagggatg ggcttcagaa 2220

ctatgaggct ctcctaggcc tcaccaacct gtctgggcgg agtgacaaac tccggcagaa 2280

gatctttaag gagagggcct tgccagacat cgagaactac atgtttgaga atcatgatca 2340

gctgcggcag gcggccaccg agtgcatgtg caacatggtg ctccacaagg aggtacagga 2400

aaggttcttg gctgacggga atgaccggct gaagctggtg gtgctgctct gcggggagga 2460

tgatgataag gtgcagaatg cggctgcagg ggctctggcc atgctgacag cagcacacaa 2520

gaaactgtgc ctcaagatga ctcaagtgac aacccagtgg ttggagatcc tccagcggct 2580

ttgcctgcac gaccagctgt ctgtccaaca ccggggcctg gtcattgcct acaacctact 2640

ggcagccgat gctgagctgg ccaagaagct ggtggagagt gagctgctgg agatcctgac 2700

tgtggtgggc aaacaggagc cagatgagaa gaaggcagaa gtggttcaga cagcccgaga 2760

atgtctcatc aagtgcatgg attatggttt cattaaacca gtgtcttaga cagcgaccct 2820

cagggatgct gggagtggtc ctgtactgtg cagagtcctg ggttggttgg gttctcctgg 2880

agagtcaggt catctaggga tcatagcagt gacaatgaag tctcaatata aaggaaagac 2940

ttgattgttc tctga 2955

<210> 241
<211> 1956
<212> DNA
<213> Homo sapiens

<400> 241

agactgaggc tggctggctg gaggttgaca caggagtgct caggggagca gcatcacaag 60

agggcagatc gaaagcatcg tccttgctga aaaaatggca gaggtggaag cggtacagct 120
gaaggaggaa ggaaaccggc atttccagct ccaggactac aaggccgcca caaatagcta 180
cagccaggcc ctgaagctga ccaaggacaa ggccctgctg gccacgcttt atcggaaccg 240
ggcagcctgt ggcctgaaaa cggagagcta cgtccaggca gcttcagatg cctccagagc 300
catcgacatc aactcctcgg acatcaaggc tctgtatcgg cgatgccagg cactggagca 360
cctggggaag ctggaccagg ccttcaaaga cgtgcagcgt tgtgccaccc tcgagccacg 420
gaaccagaac ttccaggaga tgctgaggag actcaacacc agcattcagg agaagctccg 480
agtgcagttc tccacagact cgagggtaca gaagatgttt gagatcctct tggatgaaaa 540
cagtgaggct gataagcggg aaaaggctgc caacaatctc attgtcctag gccgtgagga 600
agcaggggct gagaagatct tccagaacaa tggagtagcc ttgctactgc agcttctgga 660
cactaagaag cctgagctgg tgctggctgc agtgcggacc ctgtcgggca tgtgcagcgg 720
ccaccaagcc agagccacag tgattctgca tgcagtgcgg atagaccgaa tctgtagcct 780
catggccgtg gagaatgagg agatgtctct ggctgtctgc aacctgctcc aagccatcat 840
tgactccttg tctggggagg acaagcggga gcatcgaggg aaggaggagg ccctggttct 900
agacaccaag aaggacctga agcagatcac cagccacctg ctggacatgc tagtcagcaa 960
gaaggtgtct ggccagggca gggatcaggc gctgaacctg ctcaataaga atgttcccag 1020
gaaggacctt gccattcatg acaactcacg taccatctat gtggtggata atggtctgag 1080
gaagatcctg aaggttgtgg ggcaggttcc agatctgcca tcctgcctgc ccctgactga 1140
caacacccgc atgctggcct ctatcctcat caacaagctc tatgatgacc tgcgctgtga 1200
cccggagcgc gatcacttcc gcaagatctg tgaggaatat atcacgggca agtttgaccc 1260
ccaggacatg gacaagaact tgaatgccat ccagacagtg tcagggatcc tgcagggccc 1320
ctttgacctg ggcaaccagc tgctgggact gaaaggtgtg atggagatga tggtggcact 1380
atgtggctca gagcgcgaga cggaccagct ggtggccgtg gaggccctca tccatgcctc 1440
cacgaagctc agccgcgcca ccttcatcat caccaatgga gtgtcactgc tcaaacagat 1500
ctacaagacc accaaaaatg agaagatcaa gatccgcaca ctggtgggac tctgtaagct 1560
cggctctgca ggtggcacag actacggtct caggcagttt gcggaagggt cgacagaaaa 1620
actggccaaa cagtgtcgca agtggctgtg caatatgtcc atagacactc ggacccgacg 1680
ctgggcagtg gagggcctgg cctacctcac gctggacgct gatgtgaagg acgactttgt 1740
ccaggacgtc cctgccctgc aggccatgtt tgagctggcc aaggcaggtg tcggggagtc 1800
tggcccgacc acaaacctca ggaaaggtct gctgggtcca gacccacagg gaatggatcc 1860
cagtcttcct cctggctcta ctccttaccc ctgtataaac atgattggtt atttccccct 1920

ttctggccct cattttacct gattgtaaaa tggact 1956

<210> 242
<211> 152
<212> DNA
<213> Homo sapiens

<400> 242

cggtacagct gaaggaggaa ggaaaccggc atttccagct ccaggactac aaggccgcca 60

caaatagcta cagccaggcc ctgaagctga ccaaggacaa ggccctgctg gccacgcttt 120

atcggaaccg ggcagcctgt ggcctgaaaa cg 152

<210> 243
<211> 181
<212> DNA
<213> Homo sapiens

<400> 243

gccgactctc ctggatgcct ggggttggag aagggtgcct ccaacccccct gacccatgcc 60

ccggaaagac agaaattcat ccagagcaga atcagcccaa tgccaggtgc tttcatgtgt 120

tattcatggc atcttactaa tggcccgtga gatagctgtt gttgtcctcc ccctatcgca 180

g 181

<210> 244
<211> 176
<212> DNA
<213> Homo sapiens

<400> 244

ccatcgacat caactcctcg gacatcaagg ctctgtatcg gcgatgccag gcactggagc 60

acctggggaa gctggaccag gccttcaaag acgtgcagcg ttgtgccacc ctcgagccac 120

ggaaccagaa cttccaggag atgctgagga gactcaacac cagcattcag gagaag 176

<210> 245
<211> 168
<212> DNA
<213> Homo sapiens

<400> 245

gctgccaaca atctcattgt cctaggccgt gaggaagcag ggctgagaa gatcttccag 60

aacaatggag tagccttgct actgcagctt ctggacacta agaagcctga gctggtgctg 120

gctgcagtgc ggaccctgtc gggcatgtgc agcggccacc aagccaga 168

<210> 246
<211> 169

<212> DNA
<213> Homo sapiens

<400> 246

gccacagtga ttctgcatgc agtgcggata gaccgaatct gtagcctcat ggccgtggag 60

aatgaggaga tgtctctggc tgtctgcaac ctgctccaag ccatcattga ctccttgtct 120

ggggaggaca agcgggagca tcgagggaag gaggaggccc tggttctag 169

<210> 247
<211> 171
<212> DNA
<213> Homo sapiens

<400> 247

acaccaagaa ggacctgaag cagatcacca gccacctgct ggacatgcta gtcagcaaga 60

aggtgtctgg ccagggcagg gatcaggcgc tgaacctgct caataagaat gttcccagga 120

aggaccttgc cattcatgac aactcacgta ccatctatgt ggtggataat g 171

<210> 248
<211> 172
<212> DNA
<213> Homo sapiens

<400> 248

gtctgaggaa gatcctgaag gttgtggggc aggttccaga tctgccatcc tgcctgcccc 60

tgactgacaa cacccgcatg ctggcctcta tcctcatcaa caagctctat gatgacctgc 120

gctgtgaccc ggagcgcgat cacttccgca agatctgtga ggaatatatc ac 172

<210> 249
<211> 301
<212> DNA
<213> Homo sapiens

<400> 249

gggcaagttt gacccccagg acatggacaa gaacttgaat gccatccaga cagtgtcagg 60

gatcctgcag ggcccctttg acctgggcaa ccagctgctg ggactgaaag gtgtgatgga 120

gatgatggtg gcactatgtg gctcagagcg cgagacggac cagctggtgg ccgtggaggc 180

cctcatccat gcctccacga agctcagccg cgccaccttc atcatcacca atggagtgtc 240

actgctcaaa cagatctaca agaccaccaa aaatgagaag atcaagatcc gcacactggt 300

g 301

<210> 250
<211> 142
<212> DNA

<213>  Homo sapiens

<400> 250

gtggctgtgc aatatgtcca tagacactcg gacccgacgc tgggcagtgg agggcctggc     60

ctacctcacg ctggacgctg atgtgaagga cgactttgtc caggacgtcc ctgccctgca    120

ggccatgttt gagctggcca ag                                             142

<210>  251
<211>  167
<212>  DNA
<213>  Homo sapiens

<400> 251

gtcggggagt ctggcccgac cacaaacctc aggaaaggtc tgctgggtcc agacccacag     60

ggaatggatc ccagtcttcc tcctggctct actccttacc cctgtataaa catgattggt    120

tatttccccc tttctggccc tcattttacc tgattgtaaa atggact                  167

<210>  252
<211>  141
<212>  DNA
<213>  Homo sapiens

<400> 252

accagtgaca agaccatcct gtactcggtg gccaccaccc tggtgaactg caccaacagc     60

tacgatgtca aggaggtcat cccagagctt gtccagctcg ccaagttctc caagcagcat    120

gtgcccgagg aacaccccaa g                                              141

<210>  253
<211>  128
<212>  DNA
<213>  Homo sapiens

<400> 253

gacaagaagg actttataga catgcgggtg aagcggcttc tgaaggcggg tgtcatctct     60

gccctggctt gcatggtgaa agcagatagt gccatcctca ctgaccagac caaggagctg    120

ctggccag                                                             128

<210>  254
<211>  156
<212>  DNA
<213>  Homo sapiens

<400> 254

gtacaggaaa ggttcttggc tgacgggaat gaccggctga agctggtggt gctgctctgc     60

ggggaggatg atgataaggt gcagaatgcg gctgcagggg ctctggccat gctgacagca    120

gcacacaaga aactgtgcct caagatgact caagtg 156

<210> 255
<211> 3019
<212> DNA
<213> Homo sapiens

<400> 255

cggctttgcc tgcacgacca gctgtctgtc caacaccggg gcctggtcat tgcctacaac 60

ctactggcag ccgatgctga gctggccaag aagctggtgg agagtgagct gctggagatc 120

ctgactgtgg tgggcaaaca ggagccagat gagaagaagg cagaagtggt tcagacagcc 180

cgagaatgtc tcatcaagtg catggattat ggtttcatta aaccagtgtc ttagacagcg 240

accctcaggg atgctgggag tggtcctgta ctgtgcagag tcctgggttg gttgggttct 300

cctggagagt caggtcatct agggatcata gcagtgacaa tgaagtctca atataaagga 360

aagacttgat tgttctctga gttgtgagtc ttctcctttg tcctgacaga gtttggatgt 420

ttcactctct ctcttgcttc ctgtctcctt atatttgtca tgtttcagaa attcccagaa 480

taattttcac ccatgattag aaataggttg gatcattctt tctgtaccca ttctgaaggc 540

caggataata ggttgaagtt ctttatattt tggaaaatgg attttgtggt aaggtaggaa 600

ctaacgggtg tgtgcatata aaggttaatg ctgttgtatt tggatgctga tctgtgctgt 660

tttcatgacc tctctcccac actatttgaa tcagtctgtt cagaactgtg tgtttcacct 720

gcagtgctct gtcccagtcc catgcccatt cccatcactt cactgtgatg gaaatagagt 780

ttatgttcaa cagtggggca tgacctctgc aatttgcttg ggaaagtctt ccaagcaaga 840

tggacatgat gaatacaaat aagatctact caaagtactt caaacaaaaa ataaataatt 900

cattggctca tgtatcttgg ccacccaggg aaggtctgac attgttagtt agatccagag 960

tttcaaatgt catcaccatg gatgtgtctt tttctctctc tcatttcccc tccccatatc 1020

ttgtctttta tttattatag gtttgtctca ttccctggca ggctctctcc ctgtgatagg 1080

aaagagagtc cccagcagcc ccagggtgac atagttgtta tagttcatta tggaatagaa 1140

gagagaagag cattctcaat aacccggcaa agttcccagg gatgactctg atatgtctat 1200

gtctcaggtc acatttccat ctatgaacca atcatattca gaggtggaat gctaattggc 1260

caggcctggg tcatatatac aagtctaggg aagaaatgag cttcatccct gtccaattga 1320

catggactga ttaggggtat taatggaaga ggtgtgccac cacaaaagaa tgtaccctgg 1380

gcagatcaaa gaacatattc tgtatgtcag gcttggccac aaaagaatga cacaagtaat 1440

atgctgtaga tcagaacctc tctgctaata ttgccttttt agcatggtta agatagctaa 1500

gatctagtac tgtcactcca gtatgtccca attctaccta cgtttattga agggtcaaca 1560

gttctgatct cagcattggg taaagggtgg gacattcaga tttacggtcc ttgataaaaa 1620

caatttacaa cgttccgttg tgtaataaat gtaagtgtac atatgcctgg gacatcagct 1680

ggaaaaggga cagactatca gagagttgca ctgttgcggt atgggccaaa tccaacataa 1740

tacccgctgt acctctagag aactaaaacc ttaatttctc agatcttttc tgcactaatg 1800

gtctttacat acagcctaca ttttaactaa ctcttgcatg ggcttgtttc acagcaggaa 1860

actatattca tcatatcctt attatgatag agaatgacaa cattcaaaag ggtgtggtgc 1920

ttctgaaaat atacacaata aatggcatga tttgacctct gtcaaattct tctttcctgc 1980

tttcctcata tgtgggcttg agtgggtgta tttagctcct ttaaaggaaa ctctttcaca 2040

ggcagggcca cactcttact cgttttgatc tcatgaggtt acatctgcca gtggcgttca 2100

gggccagtta gataaagttc atcttgtttg gattggagag actttttttt tttttttttt 2160

tgagacaggg tcttgctctg taacctaggc tcaagtgcag tgcagtggtg cagtcatggc 2220

tcaccgcagc cttggtctcc tgggctcaag tgatctttct acttcagcct cctcaatagc 2280

tgggactaca ggcacacacc accacccccca ggtaattaaa aaattttttt ttgtagagac 2340

agggtctcac tatgttgccc agtctgggag accacattct ttaacatgaa tattttgtaa 2400

atagtccaag aagatctaga ggaaagctga attccttcat ctgatttttg ccactgggct 2460

gcctcccatt gtctggctaa gtcttcatgg gaggccttta agaaaagctg tgacaaatac 2520

ctttgtttcc cactgcctag gttacataag tagttggctt ttccagccta gactcttata 2580

ttgttgcttg taagagggcc cttctttgca gatttctttt ttttttcatc tatgctttca 2640

aataggcaga tgctagcctg agatcatctc tttattgagg tagtttgccc aaaaacagca 2700

aggaacagcc aatacctgcc aactttctag gtttttccaa ccaattctct tacagaaaat 2760

ctataaatat cttagaggta tgtttcttag tttcccaaca tataatggga aaattcatca 2820

tttatattat tacttttaac tgaagtatgt tgttatcaga gaacttggtc tgtgtcatat 2880

taatcctttg ccatttgaga agcatacagt tttaaagttt atatctccaa aaggatattt 2940

tgtaaaaatt taacccccttt gactcttagt cttctcattt gtgaaaggta aataataaag 3000

aatttctgtt cctagtaag 3019

<210> 256
<211> 94
<212> DNA
<213> Homo sapiens

<400> 256

agactgaggc tggctggctg gaggttgaca caggagtgct caggggagca gcatcacaag 60

agggcagatc gaaagcatcg tccttgctga aaaa 94

<210>  257
<211>  16
<212>  DNA
<213>  Homo sapiens

<400> 257

atggcagagg tggaag    16

<210>  258
<211>  37
<212>  DNA
<213>  Homo sapiens

<400> 258

gagagctacg tccaggcagc ttcagatgcc tccagag    37

<210>  259
<211>  90
<212>  DNA
<213>  Homo sapiens

<400> 259

ctccgagtgc agttctccac agactcgagg gtacagaaga tgtttgagat cctcttggat    60

gaaaacagtg aggctgataa gcgggaaaag    90

<210>  260
<211>  95
<212>  DNA
<213>  Homo sapiens

<400> 260

ggactctgta agctcggctc tgcaggtggc acagactacg gtctcaggca gtttgcggaa    60

gggtcgacag aaaaactggc caaacagtgt cgcaa    95

<210>  261
<211>  6
<212>  DNA
<213>  Homo sapiens

<400> 261

gcaggt    6

<210>  262
<211>  67
<212>  DNA
<213>  Homo sapiens

<400> 262

ggtattcctg gcactgtgtg acaacccaaa ggaccgaggc accattgtgg ctcaaggtgg    60

tggcaag 67

<210> 263
<211> 114
<212> DNA
<213> Homo sapiens

<400> 263

gccctgattc ccctggcttt ggagggcaca gatgtgggca aggtgaaggc agcccacgct 60

ctagcaaaga tcgctgctgt ctccaatccg gacattgctt ttcctgggga gcgg 114

<210> 264
<211> 116
<212> DNA
<213> Homo sapiens

<400> 264

gtgtatgagg tggtgcggcc ccttgtaaga ctcttggaca cacagaggga tgggcttcag 60

aactatgagg ctctcctagg cctcaccaac ctgtctgggc ggagtgacaa actccg 116

<210> 265
<211> 118
<212> DNA
<213> Homo sapiens

<400> 265

gcagaagatc tttaaggaga gggccttgcc agacatcgag aactacatgt ttgagaatca 60

tgatcagctg cggcaggcgg ccaccgagtg catgtgcaac atggtgctcc acaaggag 118

<210> 266
<211> 27
<212> DNA
<213> Homo sapiens

<400> 266

acaacccagt ggttggagat cctccag 27

<210> 267
<211> 931
<212> PRT
<213> Homo sapiens

<400> 267

```
Met Ala Glu Val Glu Ala Val Gln Leu Lys Glu Glu Gly Asn Arg His
1               5                   10                  15

Phe Gln Leu Gln Asp Tyr Lys Ala Ala Thr Asn Ser Tyr Ser Gln Ala
            20                  25                  30
```

```
Leu Lys Leu Thr Lys Asp Lys Ala Leu Leu Ala Thr Leu Tyr Arg Asn
        35                  40                  45

Arg Ala Ala Cys Gly Leu Lys Thr Glu Ser Tyr Val Gln Ala Ala Ser
    50                  55                  60

Asp Ala Ser Arg Ala Ile Asp Ile Asn Ser Ser Asp Ile Lys Ala Leu
65                  70                  75                  80

Tyr Arg Arg Cys Gln Ala Leu Glu His Leu Gly Lys Leu Asp Gln Ala
                85                  90                  95

Phe Lys Asp Val Gln Arg Cys Ala Thr Leu Glu Pro Arg Asn Gln Asn
            100                 105                 110

Phe Gln Glu Met Leu Arg Arg Leu Asn Thr Ser Ile Gln Glu Lys Leu
        115                 120                 125

Arg Val Gln Phe Ser Thr Asp Ser Arg Val Gln Lys Met Phe Glu Ile
    130                 135                 140

Leu Leu Asp Glu Asn Ser Glu Ala Asp Lys Arg Glu Lys Ala Ala Asn
145                 150                 155                 160

Asn Leu Ile Val Leu Gly Arg Glu Glu Ala Gly Ala Glu Lys Ile Phe
                165                 170                 175

Gln Asn Asn Gly Val Ala Leu Leu Leu Gln Leu Leu Asp Thr Lys Lys
            180                 185                 190

Pro Glu Leu Val Leu Ala Ala Val Arg Thr Leu Ser Gly Met Cys Ser
        195                 200                 205

Gly His Gln Ala Arg Ala Thr Val Ile Leu His Ala Val Arg Ile Asp
    210                 215                 220

Arg Ile Cys Ser Leu Met Ala Val Glu Asn Glu Glu Met Ser Leu Ala
225                 230                 235                 240

Val Cys Asn Leu Leu Gln Ala Ile Ile Asp Ser Leu Ser Gly Glu Asp
                245                 250                 255

Lys Arg Glu His Arg Gly Lys Glu Glu Ala Leu Val Leu Asp Thr Lys
            260                 265                 270

Lys Asp Leu Lys Gln Ile Thr Ser His Leu Leu Asp Met Leu Val Ser
        275                 280                 285
```

```
Lys Lys Val Ser Gly Gln Gly Arg Asp Gln Ala Leu Asn Leu Leu Asn
    290                 295                 300

Lys Asn Val Pro Arg Lys Asp Leu Ala Ile His Asp Asn Ser Arg Thr
305                 310                 315                 320

Ile Tyr Val Val Asp Asn Gly Leu Arg Lys Ile Leu Lys Val Val Gly
                325                 330                 335

Gln Val Pro Asp Leu Pro Ser Cys Leu Pro Leu Thr Asp Asn Thr Arg
            340                 345                 350

Met Leu Ala Ser Ile Leu Ile Asn Lys Leu Tyr Asp Asp Leu Arg Cys
        355                 360                 365

Asp Pro Glu Arg Asp His Phe Arg Lys Ile Cys Glu Glu Tyr Ile Thr
    370                 375                 380

Gly Lys Phe Asp Pro Gln Asp Met Asp Lys Asn Leu Asn Ala Ile Gln
385                 390                 395                 400

Thr Val Ser Gly Ile Leu Gln Gly Pro Phe Asp Leu Gly Asn Gln Leu
                405                 410                 415

Leu Gly Leu Lys Gly Val Met Glu Met Met Val Ala Leu Cys Gly Ser
            420                 425                 430

Glu Arg Glu Thr Asp Gln Leu Val Ala Val Glu Ala Leu Ile His Ala
        435                 440                 445

Ser Thr Lys Leu Ser Arg Ala Thr Phe Ile Ile Thr Asn Gly Val Ser
    450                 455                 460

Leu Leu Lys Gln Ile Tyr Lys Thr Thr Lys Asn Glu Lys Ile Lys Ile
465                 470                 475                 480

Arg Thr Leu Val Gly Leu Cys Lys Leu Gly Ser Ala Gly Gly Thr Asp
                485                 490                 495

Tyr Gly Leu Arg Gln Phe Ala Glu Gly Ser Thr Glu Lys Leu Ala Lys
            500                 505                 510

Gln Cys Arg Lys Trp Leu Cys Asn Met Ser Ile Asp Thr Arg Thr Arg
        515                 520                 525

Arg Trp Ala Val Glu Gly Leu Ala Tyr Leu Thr Leu Asp Ala Asp Val
```

```
    530                 535                 540

Lys Asp Asp Phe Val Gln Asp Val Pro Ala Leu Gln Ala Met Phe Glu
545                 550                 555                 560

Leu Ala Lys Ala Gly Thr Ser Asp Lys Thr Ile Leu Tyr Ser Val Ala
                565                 570                 575

Thr Thr Leu Val Asn Cys Thr Asn Ser Tyr Asp Val Lys Glu Val Ile
            580                 585                 590

Pro Glu Leu Val Gln Leu Ala Lys Phe Ser Lys Gln His Val Pro Glu
        595                 600                 605

Glu His Pro Lys Asp Lys Lys Asp Phe Ile Asp Met Arg Val Lys Arg
    610                 615                 620

Leu Leu Lys Ala Gly Val Ile Ser Ala Leu Ala Cys Met Val Lys Ala
625                 630                 635                 640

Asp Ser Ala Ile Leu Thr Asp Gln Thr Lys Glu Leu Leu Ala Arg Val
                645                 650                 655

Phe Leu Ala Leu Cys Asp Asn Pro Lys Asp Arg Gly Thr Ile Val Ala
            660                 665                 670

Gln Gly Gly Gly Lys Ala Leu Ile Pro Leu Ala Leu Glu Gly Thr Asp
        675                 680                 685

Val Gly Lys Val Lys Ala Ala His Ala Leu Ala Lys Ile Ala Ala Val
    690                 695                 700

Ser Asn Pro Asp Ile Ala Phe Pro Gly Glu Arg Val Tyr Glu Val Val
705                 710                 715                 720

Arg Pro Leu Val Arg Leu Leu Asp Thr Gln Arg Asp Gly Leu Gln Asn
                725                 730                 735

Tyr Glu Ala Leu Leu Gly Leu Thr Asn Leu Ser Gly Arg Ser Asp Lys
            740                 745                 750

Leu Arg Gln Lys Ile Phe Lys Glu Arg Ala Leu Pro Asp Ile Glu Asn
        755                 760                 765

Tyr Met Phe Glu Asn His Asp Gln Leu Arg Gln Ala Ala Thr Glu Cys
    770                 775                 780
```

Met Cys Asn Met Val Leu His Lys Glu Val Gln Glu Arg Phe Leu Ala
785 790 795 800

Asp Gly Asn Asp Arg Leu Lys Leu Val Val Leu Leu Cys Gly Glu Asp
805 810 815

Asp Asp Lys Val Gln Asn Ala Ala Ala Gly Ala Leu Ala Met Leu Thr
820 825 830

Ala Ala His Lys Lys Leu Cys Leu Lys Met Thr Gln Val Thr Thr Gln
835 840 845

Trp Leu Glu Ile Leu Gln Arg Leu Cys Leu His Asp Gln Leu Ser Val
850 855 860

Gln His Arg Gly Leu Val Ile Ala Tyr Asn Leu Leu Ala Ala Asp Ala
865 870 875 880

Glu Leu Ala Lys Lys Leu Val Glu Ser Glu Leu Leu Glu Ile Leu Thr
885 890 895

Val Val Gly Lys Gln Glu Pro Asp Glu Lys Lys Ala Glu Val Val Gln
900 905 910

Thr Ala Arg Glu Cys Leu Ile Lys Cys Met Asp Tyr Gly Phe Ile Lys
915 920 925

Pro Val Ser
930

<210> 268
<211> 917
<212> PRT
<213> Homo sapiens

<400> 268

Met Pro Arg Lys Asp Arg Asn Ser Ser Arg Ala Glu Ser Ala Gln Cys
1 5 10 15

Gln Val Leu Ser Cys Val Ile His Gly Ile Leu Leu Met Ala Arg Glu
20 25 30

Ile Ala Val Val Val Leu Pro Leu Ser Gln Glu Ser Tyr Val Gln Ala
35 40 45

Ala Ser Asp Ala Ser Arg Ala Ile Asp Ile Asn Ser Ser Asp Ile Lys
50 55 60

```
Ala Leu Tyr Arg Arg Cys Gln Ala Leu Glu His Leu Gly Lys Leu Asp
65                  70                  75                  80

Gln Ala Phe Lys Asp Val Gln Arg Cys Ala Thr Leu Glu Pro Arg Asn
                85                  90                  95

Gln Asn Phe Gln Glu Met Leu Arg Arg Leu Asn Thr Ser Ile Gln Glu
            100                 105                 110

Lys Leu Arg Val Gln Phe Ser Thr Asp Ser Arg Val Gln Lys Met Phe
        115                 120                 125

Glu Ile Leu Leu Asp Glu Asn Ser Glu Ala Asp Lys Arg Glu Lys Ala
    130                 135                 140

Ala Asn Asn Leu Ile Val Leu Gly Arg Glu Glu Ala Gly Ala Glu Lys
145                 150                 155                 160

Ile Phe Gln Asn Asn Gly Val Ala Leu Leu Leu Gln Leu Leu Asp Thr
                165                 170                 175

Lys Lys Pro Glu Leu Val Leu Ala Ala Val Arg Thr Leu Ser Gly Met
            180                 185                 190

Cys Ser Gly His Gln Ala Arg Ala Thr Val Ile Leu His Ala Val Arg
        195                 200                 205

Ile Asp Arg Ile Cys Ser Leu Met Ala Val Glu Asn Glu Glu Met Ser
    210                 215                 220

Leu Ala Val Cys Asn Leu Leu Gln Ala Ile Ile Asp Ser Leu Ser Gly
225                 230                 235                 240

Glu Asp Lys Arg Glu His Arg Gly Lys Glu Glu Ala Leu Val Leu Asp
                245                 250                 255

Thr Lys Lys Asp Leu Lys Gln Ile Thr Ser His Leu Leu Asp Met Leu
            260                 265                 270

Val Ser Lys Lys Val Ser Gly Gln Gly Arg Asp Gln Ala Leu Asn Leu
        275                 280                 285

Leu Asn Lys Asn Val Pro Arg Lys Asp Leu Ala Ile His Asp Asn Ser
    290                 295                 300

Arg Thr Ile Tyr Val Val Asp Asn Gly Leu Arg Lys Ile Leu Lys Val
305                 310                 315                 320
```

```
Val Gly Gln Val Pro Asp Leu Pro Ser Cys Leu Pro Leu Thr Asp Asn
                325                 330                 335

Thr Arg Met Leu Ala Ser Ile Leu Ile Asn Lys Leu Tyr Asp Asp Leu
            340                 345                 350

Arg Cys Asp Pro Glu Arg Asp His Phe Arg Lys Ile Cys Glu Glu Tyr
        355                 360                 365

Ile Thr Gly Lys Phe Asp Pro Gln Asp Met Asp Lys Asn Leu Asn Ala
    370                 375                 380

Ile Gln Thr Val Ser Gly Ile Leu Gln Gly Pro Phe Asp Leu Gly Asn
385                 390                 395                 400

Gln Leu Leu Gly Leu Lys Gly Val Met Glu Met Met Val Ala Leu Cys
                405                 410                 415

Gly Ser Glu Arg Glu Thr Asp Gln Leu Val Ala Val Glu Ala Leu Ile
            420                 425                 430

His Ala Ser Thr Lys Leu Ser Arg Ala Thr Phe Ile Ile Thr Asn Gly
        435                 440                 445

Val Ser Leu Leu Lys Gln Ile Tyr Lys Thr Thr Lys Asn Glu Lys Ile
    450                 455                 460

Lys Ile Arg Thr Leu Val Gly Leu Cys Lys Leu Gly Ser Ala Gly Gly
465                 470                 475                 480

Thr Asp Tyr Gly Leu Arg Gln Phe Ala Glu Gly Ser Thr Glu Lys Leu
                485                 490                 495

Ala Lys Gln Cys Arg Lys Trp Leu Cys Asn Met Ser Ile Asp Thr Arg
            500                 505                 510

Thr Arg Arg Trp Ala Val Glu Gly Leu Ala Tyr Leu Thr Leu Asp Ala
        515                 520                 525

Asp Val Lys Asp Asp Phe Val Gln Asp Val Pro Ala Leu Gln Ala Met
    530                 535                 540

Phe Glu Leu Ala Lys Ala Gly Thr Ser Asp Lys Thr Ile Leu Tyr Ser
545                 550                 555                 560

Val Ala Thr Thr Leu Val Asn Cys Thr Asn Ser Tyr Asp Val Lys Glu
```

565 570 575

Val Ile Pro Glu Leu Val Gln Leu Ala Lys Phe Ser Lys Gln His Val
580 585 590

Pro Glu Glu His Pro Lys Asp Lys Lys Asp Phe Ile Asp Met Arg Val
595 600 605

Lys Arg Leu Leu Lys Ala Gly Val Ile Ser Ala Leu Ala Cys Met Val
610 615 620

Lys Ala Asp Ser Ala Ile Leu Thr Asp Gln Thr Lys Glu Leu Leu Ala
625 630 635 640

Arg Val Phe Leu Ala Leu Cys Asp Asn Pro Lys Asp Arg Gly Thr Ile
645 650 655

Val Ala Gln Gly Gly Gly Lys Ala Leu Ile Pro Leu Ala Leu Glu Gly
660 665 670

Thr Asp Val Gly Lys Val Lys Ala Ala His Ala Leu Ala Lys Ile Ala
675 680 685

Ala Val Ser Asn Pro Asp Ile Ala Phe Pro Gly Glu Arg Val Tyr Glu
690 695 700

Val Val Arg Pro Leu Val Arg Leu Leu Asp Thr Gln Arg Asp Gly Leu
705 710 715 720

Gln Asn Tyr Glu Ala Leu Leu Gly Leu Thr Asn Leu Ser Gly Arg Ser
725 730 735

Asp Lys Leu Arg Gln Lys Ile Phe Lys Glu Arg Ala Leu Pro Asp Ile
740 745 750

Glu Asn Tyr Met Phe Glu Asn His Asp Gln Leu Arg Gln Ala Ala Thr
755 760 765

Glu Cys Met Cys Asn Met Val Leu His Lys Glu Val Gln Glu Arg Phe
770 775 780

Leu Ala Asp Gly Asn Asp Arg Leu Lys Leu Val Val Leu Leu Cys Gly
785 790 795 800

Glu Asp Asp Asp Lys Val Gln Asn Ala Ala Ala Gly Ala Leu Ala Met
805 810 815

```
Leu Thr Ala Ala His Lys Lys Leu Cys Leu Lys Met Thr Gln Val Thr
            820                 825                 830

Thr Gln Trp Leu Glu Ile Leu Gln Arg Leu Cys Leu His Asp Gln Leu
        835                 840                 845

Ser Val Gln His Arg Gly Leu Val Ile Ala Tyr Asn Leu Leu Ala Ala
    850                 855                 860

Asp Ala Glu Leu Ala Lys Lys Leu Val Glu Ser Glu Leu Leu Glu Ile
865                 870                 875                 880

Leu Thr Val Val Gly Lys Gln Glu Pro Asp Glu Lys Lys Ala Glu Val
                885                 890                 895

Val Gln Thr Ala Arg Glu Cys Leu Ile Lys Cys Met Asp Tyr Gly Phe
            900                 905                 910

Ile Lys Pro Val Ser
        915


<210>  269
<211>  615
<212>  PRT
<213>  Homo sapiens

<400> 269

Met Ala Glu Val Glu Ala Val Gln Leu Lys Glu Glu Gly Asn Arg His
1               5                   10                  15

Phe Gln Leu Gln Asp Tyr Lys Ala Ala Thr Asn Ser Tyr Ser Gln Ala
            20                  25                  30

Leu Lys Leu Thr Lys Asp Lys Ala Leu Leu Ala Thr Leu Tyr Arg Asn
        35                  40                  45

Arg Ala Ala Cys Gly Leu Lys Thr Glu Ser Tyr Val Gln Ala Ala Ser
    50                  55                  60

Asp Ala Ser Arg Ala Ile Asp Ile Asn Ser Ser Asp Ile Lys Ala Leu
65                  70                  75                  80

Tyr Arg Arg Cys Gln Ala Leu Glu His Leu Gly Lys Leu Asp Gln Ala
                85                  90                  95

Phe Lys Asp Val Gln Arg Cys Ala Thr Leu Glu Pro Arg Asn Gln Asn
            100                 105                 110
```

```
Phe Gln Glu Met Leu Arg Arg Leu Asn Thr Ser Ile Gln Glu Lys Leu
        115                 120                 125

Arg Val Gln Phe Ser Thr Asp Ser Arg Val Gln Lys Met Phe Glu Ile
    130                 135                 140

Leu Leu Asp Glu Asn Ser Glu Ala Asp Lys Arg Glu Lys Ala Ala Asn
145                 150                 155                 160

Asn Leu Ile Val Leu Gly Arg Glu Glu Ala Gly Ala Glu Lys Ile Phe
                165                 170                 175

Gln Asn Asn Gly Val Ala Leu Leu Leu Gln Leu Leu Asp Thr Lys Lys
            180                 185                 190

Pro Glu Leu Val Leu Ala Ala Val Arg Thr Leu Ser Gly Met Cys Ser
        195                 200                 205

Gly His Gln Ala Arg Ala Thr Val Ile Leu His Ala Val Arg Ile Asp
    210                 215                 220

Arg Ile Cys Ser Leu Met Ala Val Glu Asn Glu Glu Met Ser Leu Ala
225                 230                 235                 240

Val Cys Asn Leu Leu Gln Ala Ile Ile Asp Ser Leu Ser Gly Glu Asp
                245                 250                 255

Lys Arg Glu His Arg Gly Lys Glu Glu Ala Leu Val Leu Asp Thr Lys
            260                 265                 270

Lys Asp Leu Lys Gln Ile Thr Ser His Leu Leu Asp Met Leu Val Ser
        275                 280                 285

Lys Lys Val Ser Gly Gln Gly Arg Asp Gln Ala Leu Asn Leu Leu Asn
    290                 295                 300

Lys Asn Val Pro Arg Lys Asp Leu Ala Ile His Asp Asn Ser Arg Thr
305                 310                 315                 320

Ile Tyr Val Val Asp Asn Gly Leu Arg Lys Ile Leu Lys Val Val Gly
                325                 330                 335

Gln Val Pro Asp Leu Pro Ser Cys Leu Pro Leu Thr Asp Asn Thr Arg
            340                 345                 350

Met Leu Ala Ser Ile Leu Ile Asn Lys Leu Tyr Asp Asp Leu Arg Cys
        355                 360                 365
```

```
Asp Pro Glu Arg Asp His Phe Arg Lys Ile Cys Glu Glu Tyr Ile Thr
    370                 375                 380

Gly Lys Phe Asp Pro Gln Asp Met Asp Lys Asn Leu Asn Ala Ile Gln
385                 390                 395                 400

Thr Val Ser Gly Ile Leu Gln Gly Pro Phe Asp Leu Gly Asn Gln Leu
                405                 410                 415

Leu Gly Leu Lys Gly Val Met Glu Met Met Val Ala Leu Cys Gly Ser
            420                 425                 430

Glu Arg Glu Thr Asp Gln Leu Val Ala Val Glu Ala Leu Ile His Ala
        435                 440                 445

Ser Thr Lys Leu Ser Arg Ala Thr Phe Ile Ile Thr Asn Gly Val Ser
    450                 455                 460

Leu Leu Lys Gln Ile Tyr Lys Thr Thr Lys Asn Glu Lys Ile Lys Ile
465                 470                 475                 480

Arg Thr Leu Val Gly Leu Cys Lys Leu Gly Ser Ala Gly Gly Thr Asp
                485                 490                 495

Tyr Gly Leu Arg Gln Phe Ala Glu Gly Ser Thr Glu Lys Leu Ala Lys
            500                 505                 510

Gln Cys Arg Lys Trp Leu Cys Asn Met Ser Ile Asp Thr Arg Thr Arg
        515                 520                 525

Arg Trp Ala Val Glu Gly Leu Ala Tyr Leu Thr Leu Asp Ala Asp Val
    530                 535                 540

Lys Asp Asp Phe Val Gln Asp Val Pro Ala Leu Gln Ala Met Phe Glu
545                 550                 555                 560

Leu Ala Lys Ala Gly Val Gly Glu Ser Gly Pro Thr Thr Asn Leu Arg
                565                 570                 575

Lys Gly Leu Leu Gly Pro Asp Pro Gln Gly Met Asp Pro Ser Leu Pro
            580                 585                 590

Pro Gly Ser Thr Pro Tyr Pro Cys Ile Asn Met Ile Gly Tyr Phe Pro
        595                 600                 605

Leu Ser Gly Pro His Phe Thr
```

610 615

<210> 270
<211> 19
<212> DNA
<213> Artificial Organism

<220>
<223> Synthetic oligonucleotide

<400> 270

gtctggcccg accacaaac 19

<210> 271
<211> 23
<212> DNA
<213> Artificial Organism

<220>
<223> Synthetic oligonucleotide

<400> 271

ggtaaggagt agagccagga gga 23

<210> 272
<211> 92
<212> DNA
<213> Artificial Organism

<220>
<223> Synthetic oligonucleotide

<400> 272

gtctggcccg accacaaacc tcaggaaagg tctgctgggt ccagacccac agggaatgga 60

tcccagtctt cctcctggct ctactcctta cc 92

<210> 273
<211> 20
<212> DNA
<213> Artificial Organism

<220>
<223> Synthetic oligonucleotide

<400> 273

accatcctgt actcggtggc 20

<210> 274
<211> 21
<212> DNA
<213> Artificial Organism

<220>
<223>  Synthetic oligonucleotide

<400> 274

catgctgctt ggagaacttg g                                                21

<210>  275
<211>  109
<212>  DNA
<213>  Artificial Organism

<220>
<223>  Synthetic oligonucleotide

<400> 275

accatcctgt actcggtggc caccaccctg gtgaactgca ccaacagcta cgatgtcaag      60

gaggtcatcc cagagcttgt ccagctcgcc aagttctcca agcagcatg                 109

<210>  276
<211>  1029
<212>  DNA
<213>  Homo sapiens

<400> 276

aaagggtatt gggtctgaca gataagagct cctgcactct accagccagc tactgacaga      60

cataggtctg gctccagtgg aggggcagca gccagtgagc ccagcctggg gtggcccact     120

cctgctgcct ccaggatgtc ccctgtttcc ccagcccctc tgctgtgccc tcggccccag     180

aagctggcga gactgcttct ctggaacagc atcacgcagg cctgcccatc ggcaccgctg     240

tgcaccaggc cttctgggga tacagatgtc aaccaggtga taaggctgag acataaagga     300

ggctgggccc tgccaccacg acagcagcca cacctctgca gagagaatgg ccagcaggaa     360

ggcggggacc cggggcaagg tggcagccac caagcaggcc caacgtggtt cttccaacgt     420

cttttccatg tttgaacaag cccagataca ggagttcaaa gaagccttca gctgtatcga     480

ccagaatcgt gatggcatca tctgcaaggc agacctgagg gagacctact cccagctggg     540

gaaggtgagt gtcccagagg aggagctgga cgccatgctg caagagggca agggccccat     600

caacttcacc gtcttcctca cgctctttgg ggagaagctc aatgggacag accccgagga     660

agccatcctg agtgccttcc gcatgtttga ccccagcggc aaaggggtgg tgaacaagga     720

tgagttcaag cagcttctcc tgacccaggc agacaagttc tctccagctg aggtgaggct     780

gcccagcccc ttcaatactc atccccagca ccttctctgg gccttcaccc atgacccaga     840

gcccagtacc agtgaggcag ttgctggaag ggtggagcag atgttcgccc tgacacccat     900

ggacctggcg gggaacatcg actacaagtc actgtgctac atcatcaccc atggagacga     960

gaaagaggaa tgaggggcag ggccaggccc acgggggggc acctcaataa actctgttgc    1020

aaaattgga 1029

<210> 277
<211> 1025
<212> DNA
<213> Homo sapiens

<400> 277

aaagggtatt gggtctgaca gataagagct cctgcactct accagccagc tactgacaga 60

cataggtctg gctccagtgg aggggcagca gccagtgagc ccagcctggg gtggcccact 120

cctgctgcct ccaggatgtc ccctgtttcc ccagcccctc tgctgtgccc tcggccccag 180

aagctggcga gactgcttct ctggaacagc atcacgcagg cctgcccatc ggcaccgctg 240

tgcaccaggc cttctgggga tacagatgtc aaccaggtga taaggctgag acataaagga 300

ggctgggccc tgccaccacg acagcagcca cacctctgca gagagaatgg ccagcaggaa 360

ggcggggacc cggggcaagg tggcagccac caagcaggcc caacgtggtt cttccaacgt 420

cttttccatg tttgaacaag cccagataca ggagttcaaa gaagccttca gctgtatcga 480

ccagaatcgt gatggcatca tctgcaaggc agacctgagg gagacctact cccagctggg 540

gaaggtgagt gtcccagagg aggagctgga cgccatgctg caagagggca agggccccat 600

caacttcacc gtcttcctca cgctctttgg ggagaagctc aatgggacag accccgagga 660

agccatcctg agtgccttcc gcatgtttga ccccagcggc aaaggggtgg tgaacaagga 720

tgaccaaccc ttccctgcgc catgggagcc tccgtaccca ccttccctgt gcagtcactc 780

ccccgcagtc tcctgctcag accctcctca ccccccaggt tcaagcagct tctcctgacc 840

caggcagaca agttctctcc agctgaggtg gagcagatgt tcgccctgac acccatggac 900

ctggcgggga acatcgacta caagtcactg tgctacatca tcacccatgg agacgagaaa 960

gaggaatgag gggcagggcc aggcccacgg gggggcacct caataaactc tgttgcaaaa 1020

ttgga 1025

<210> 278
<211> 999
<212> DNA
<213> Homo sapiens

<400> 278

aaagggtatt gggtctgaca gataagagct cctgcactct accagccagc tactgacaga 60

cataggtctg gctccagtgg aggggcagca gccagtgagc ccagcctggg gtggcccact 120

cctgctgcct ccaggatgtc ccctgtttcc ccagcccctc tgctgtgccc tcggccccag 180

aagctggcga gactgcttct ctggaacagc atcacgcagg cctgcccatc ggcaccgctg 240

tgcaccaggc cttctgggga tacagatgtc aaccaggtga taaggctgag acataaagga 300

ggctgggccc tgccaccacg acagcagcca cacctctgca gagagaatgg ccagcaggaa 360

ggcggggacc cggggcaagg tggcagccac caagcaggcc caacgtggtt cttccaacgt 420

cttttccatg tttgaacaag cccagataca ggagttcaaa gaagtctctc ccccacctcc 480

caccttccct agagctgggg gctgctccca cctgaaggcc cccatcccac aggccttcag 540

ctgtatcgac cagaatcgtg atggcatcat ctgcaaggca gacctgaggg agacctactc 600

ccagctgggg aaggtgagtg tcccagagga ggagctggac gccatgctgc aagagggcaa 660

gggccccatc aacttcaccg tcttcctcac gctctttggg gagaagctca atgggacaga 720

ccccgaggaa gccatcctga gtgccttccg catgtttgac cccagcggca aaggggtggt 780

gaacaaggat gagttcaagc agcttctcct gacccaggca gacaagttct ctccagctga 840

ggtggagcag atgttcgccc tgacacccat ggacctggcg gggaacatcg actacaagtc 900

actgtgctac atcatcaccc atggagacga gaaagaggaa tgaggggcag ggccaggccc 960

acggggggc acctcaataa actctgttgc aaaattgga 999

<210> 279
<211> 881
<212> DNA
<213> Homo sapiens

<400> 279

aaagggtatt gggtctgaca gataagagct cctgcactct accagccagc tactgacaga 60

cataggtctg gctccagtgg aggggcagca gccagtgagc ccagcctggg gtggcccact 120

cctgctgcct ccaggatgtc ccctgtttcc ccagcccctc tgctgtgccc tcggccccag 180

aagctggcga gactgcttct ctggaacagc atcacgcagg cctgcccatc ggcaccgctg 240

tgcaccaggc cttctgggga tacagatgtc aaccaggtga taaggctgag acataaagga 300

ggctgggccc tgccaccacg acagcagcca cacctctgca gagagaatgg ccagcaggaa 360

ggcggggacc cggggcaagg tggcagccac caagcaggcc caacgtggtt cttccaacgt 420

cttttccatg tttgaacaag cccagataca ggagttcaaa gaagccttca gctgtatcga 480

ccagaatcgt gatggcatca tctgcaaggc agacctgagg gagacctact cccagctggg 540

gaaggtgagt gtcccagagg aggagctgga cgccatgctg caagagggca agggccccat 600

caacttcacc gtcttcctca cgctctttgg ggagaagctc aatgggacag accccgagga 660

agccatcctg agtgccttcc gcatgtttga ccccagcggc aaaggggtgg tgaacaagga 720

tgagtggagc agatgttcgc cctgacaccc atggacctgg cggggaacat cgactacaag 780

tcactgtgct acatcatcac ccatggagac gagaaagagg aatgaggggc agggccaggc 840

ccacgggggg gcacctcaat aaactctgtt gcaaaattgg a 881

<210> 280
<211> 1021
<212> DNA
<213> Homo sapiens

<400> 280

aaagggtatt gggtctgaca gataagagct cctgcactct accagccagc tactgacaga 60

cataggtctg gctccagtgg aggggcagca gccagtgagc ccagcctggg gtggcccact 120

cctgctgcct ccaggatgtc ccctgtttcc ccagcccctc tgctgtgccc tcggccccag 180

aagctggcga gactgcttct ctggaacagc atcacgcagg cctgcccatc ggcaccgctg 240

tgcaccaggc cttctgggga tacagatgtc aaccaggtga taaggctgag acataaagga 300

ggctgggccc tgccaccacg acagcagcca cacctctgca gagagaatgg ccagcaggaa 360

ggcggggacc cggggcaagg tggcagccac caagcaggcc caacgtggtt cttccaacgt 420

cttttccatg tttgaacaag cccagataca ggagttcaaa gaagccttca gctgtatcga 480

ccagaatcgt gatggcatca tctgcaaggc agacctgagg gagacctact cccagctggc 540

ccctgtggcc aggatggagg gagggcggcc tgggcccttc tgggggacac ccagggtccc 600

tgtgtgcacc tcatgcccca cccccaccag ggaaggtgag tgtcccagag gaggagctgg 660

acgccatgct gcaagagggc aagggcccca tcaacttcac cgtcttcctc acgctctttg 720

gggagaagct caatggggaca gaccccgagg aagccatcct gagtgccttc cgcatgtttg 780

accccagcgg caaaggggtg gtgaacaagg atgagttcaa gcagcttctc ctgacccagg 840

cagacaagtt ctctccagct gaggtggagc agatgttcgc cctgacaccc atggacctgg 900

cggggaacat cgactacaag tcactgtgct acatcatcac ccatggagac gagaaagagg 960

aatgaggggc agggccaggc ccacgggggg gcacctcaat aaactctgtt gcaaaattgg 1020

a 1021

<210> 281
<211> 1284
<212> DNA
<213> Homo sapiens

<400> 281

aaagggtatt gggtctgaca gataagagct cctgcactct accagccagc tactgacaga 60

cataggtctg gctccagtgg aggggcagca gccagtgagc ccagcctggg gtggcccact 120

cctgctgcct ccaggatgtc ccctgtttcc ccagcccctc tgctgtgccc tcggccccag 180

aagctggcga gactgcttct ctggaacagc atcacgcagg cctgcccatc ggcaccgctg 240

tgcaccaggc cttctgggga tacagatgtc aaccaggtga taaggctgag acataaagga 300

ggctgggccc tgccaccacg acagcagcca cacctctgca gagagaatgg ccagcaggaa 360

ggcggggacc cggggcaagg tggcagccac caagcaggcc caacgtggtt cttccaacgt 420

cttttccatg tttgaacaag cccagataca ggagttcaaa gaagtctctc ccccacctcc 480

caccttccct agagctgggg gctgctccca cctgaaggcc cccatcccac aggccttcag 540

ctgtatcgac cagaatcgtg atggcatcat ctgcaaggca gacctgaggg agacctactc 600

ccagctgggt gcgtgcaccc acctcccacc ctgcgcactg gggtccctac tctgagctgc 660

tgggcgggtg ggagtggctg gggggacagg actctgctcc cctgcttccc ctcctccccg 720

tctcctcaca ctgcccttcc ccccttgtca cgccttgctt ccacttcacc ttcccgaccc 780

acagctgcct ctgcccctcc agcccctgtg gccaggatgg agggagggcg gcctgggccc 840

ttctggggga cacccagggt ccctgtgtgc acctcatgcc ccaccccac cagggaaggt 900

gagtgtccca gaggaggagc tggacgccat gctgcaagag ggcaagggcc ccatcaactt 960

caccgtcttc ctcacgctct ttggggagaa gctcaatggg acagaccccg aggaagccat 1020

cctgagtgcc ttccgcatgt ttgaccccag cggcaaaggg gtggtgaaca aggatgagtt 1080

caagcagctt ctcctgaccc aggcagacaa gttctctcca gctgaggtgg agcagatgtt 1140

cgccctgaca cccatggacc tggcggggaa catcgactac aagtcactgt gctacatcat 1200

cacccatgga gacgagaaag aggaatgagg ggcagggcca ggcccacggg ggggcacctc 1260

aataaactct gttgcaaaat tgga 1284

<210> 282
<211> 276
<212> DNA
<213> Homo sapiens

<400> 282

aaagggtatt gggtctgaca gataagagct cctgcactct accagccagc tactgacaga 60

cataggtctg gctccagtgg aggggcagca gccagtgagc ccagcctggg gtggcccact 120

cctgctgcct ccaggatgtc ccctgtttcc ccagcccctc tgctgtgccc tcggccccag 180

aagctggcga gactgcttct ctggaacagc atcacgcagg cctgcccatc ggcaccgctg 240

tgcaccaggc cttctgggga tacagatgtc aaccag 276

<210> 283
<211> 194
<212> DNA
<213> Homo sapiens

<400> 283

gtgcgtgcac ccacctccca ccctgcgcac tggggtccct actctgagct gctgggcggg 60

tgggagtggc tggggggaca ggactctgct cccctgcttc ccctcctccc cgtctcctca 120

cactgccctt cccccttgt cacgccttgc ttccacttca ccttcccgac ccacagctgc 180

ctctgcccct ccag 194

<210> 284
<211> 1257
<212> DNA
<213> Homo sapiens

<400> 284

gtggagcaga tgttcgccct gacacccatg gacctggcgg ggaacatcga ctacaagtca 60

ctgtgctaca tcatcaccca tggagacgag aaagaggaat gaggggcagg gccaggccca 120

cgggggggca cctcaataaa ctctgttgca aaattggaat tgctgtggtg tcttgtctgt 180

gacagatggg ttggggacca gccaaggggg atcccagggt ctcagtgcgc acatcaccat 240

gatcatggcc accatctacc tcctgggagc tggcccctcg ccagctcacc ttgattcact 300

cccatgatgc caagtgaagt gtgaactatg atcatgccta gtttacagat gaggacactg 360

aggcccagaa agtgtgagca tcttaccaag gccagccctc tagaagagga gatggtggga 420

tttacaccac ctccaccaag cccaggaatg agccacaaag tgggcactgc ccagctactt 480

ggggctgtgc agagaagagg ctgcttgctg ggcactcagc aaactctgcc caacagccca 540

gcgggtgggc agcagccctg ggacccccac acccaaccac acagcctccc ctggcccact 600

gctcgcaccc catctcaata cactggcttg ggtgcctccc tgcatgggcc ctttgtgaaa 660

ggcagagagg tacccatttg aaacacaacc agcttctcat tgcaaataca ggcaaggcac 720

taagacatga ggaacatgga caccaaagca ggggccaggt aacatgcaaa tttctagagg 780

aaatgcccag aacctggcat catgcctcct gagcccctca tgcgccgtga ggggtaagag 840

ggtcagacag ctggagtgta gggagacgac ttctcaggag agaatagtta gtgctcccgt 900

caccccttcat ctgagaaccc aagagctaga ggagaaagtg atcctcatga gtaccagagg 960

agcagcaggg gacatccaaa gcaccagaga gagaaacaga gacagagaga caggcagtga 1020

cagctcaaac ctcagccaga tccagagcat acaaagtctc ctgcctacag gacagcccag 1080

taagagctct cagcttgcct ccttccctcc ccacaagccc tgctgcaatc cctgtacctg 1140

ggggtcagtg ggaaggaggt gagcgagaaa ggaggggcac cccttcctga aggccccaag 1200

aggaaaggcg ttttcaccca gacaggtgtt cagttttgat tttatctggc gcctggc 1257

<210> 285
<211> 73
<212> DNA
<213> Homo sapiens

<400> 285

gtgataaggc tgagacataa aggaggctgg gccctgccac cacgacagca gccacacctc 60

tgcagagaga atg 73

<210> 286
<211> 96
<212> DNA
<213> Homo sapiens

<400> 286

gccagcagga aggcggggac ccggggcaag gtggcagcca ccaagcaggc ccaacgtggt 60

tcttccaacg tcttttccat gtttgaacaa gcccag 96

<210> 287
<211> 18
<212> DNA
<213> Homo sapiens

<400> 287

atacaggagt tcaaagaa 18

<210> 288
<211> 29
<212> DNA
<213> Homo sapiens

<400> 288

gtctctcccc cacctcccac cttccctag 29

<210> 289
<211> 40
<212> DNA
<213> Homo sapiens

<400> 289

agctgggggc tgctcccacc tgaaggcccc catcccacag 40

<210> 290
<211> 76
<212> DNA
<213> Homo sapiens

<400> 290

gccttcagct gtatcgacca gaatcgtgat ggcatcatct gcaaggcaga cctgagggag 60

acctactccc agctgg 76

<210> 291
<211> 24
<212> DNA
<213> Homo sapiens

<400> 291

cccctgtggc caggatggag ggag 24

<210> 292
<211> 37
<212> DNA
<213> Homo sapiens

<400> 292

ggcggcctgg gcccttctgg gggacaccca gggtccc 37

<210> 293
<211> 30
<212> DNA
<213> Homo sapiens

<400> 293

tgtgtgcacc tcatgcccca cccccaccag 30

<210> 294
<211> 85
<212> DNA
<213> Homo sapiens

<400> 294

ggaaggtgag tgtcccagag gaggagctgg acgccatgct gcaagagggc aagggcccca 60

tcaacttcac cgtcttcctc acgct 85

<210> 295
<211> 20
<212> DNA
<213> Homo sapiens

<400> 295

ctttggggag aagctcaatg 20

<210> 296
<211> 6
<212> DNA
<213> Homo sapiens

<400> 296

ggacag 6

<210> 297
<211> 73
<212> DNA
<213> Homo sapiens

<400> 297

accccgagga agccatcctg agtgccttcc gcatgtttga ccccagcggc aaaggggtgg 60

tgaacaagga tga 73

<210> 298
<211> 95
<212> DNA
<213> Homo sapiens

<400> 298

ccaacccttc cctgcgccat gggagcctcc gtacccacct tccctgtgca gtcactcccc 60

cgcagtctcc tgctcagacc ctcctcaccc cccag 95

<210> 299
<211> 16
<212> DNA
<213> Homo sapiens

<400> 299

gttcaagcag cttctc 16

<210> 300
<211> 33
<212> DNA
<213> Homo sapiens

<400> 300

ctgacccagg cagacaagtt ctctccagct gag 33

<210> 301
<211> 99
<212> DNA
<213> Homo sapiens

<400> 301

gtgaggctgc ccagcccctt caatactcat ccccagcacc ttctctgggc cttcacccat 60

gacccagagc ccagtaccag tgaggcagtt gctggaagg 99

<210> 302
<211> 175
<212> PRT
<213> Homo sapiens

<400> 302

Met Ala Ser Arg Lys Ala Gly Thr Arg Gly Lys Val Ala Ala Thr Lys
1 5 10 15

Gln Ala Gln Arg Gly Ser Ser Asn Val Phe Ser Met Phe Glu Gln Ala

```
            20                  25                  30

Gln Ile Gln Glu Phe Lys Glu Ala Phe Ser Cys Ile Asp Gln Asn Arg
        35                  40                  45

Asp Gly Ile Ile Cys Lys Ala Asp Leu Arg Glu Thr Tyr Ser Gln Leu
    50                  55                  60

Gly Lys Val Ser Val Pro Glu Glu Glu Leu Asp Ala Met Leu Gln Glu
65                  70                  75                  80

Gly Lys Gly Pro Ile Asn Phe Thr Val Phe Leu Thr Leu Phe Gly Glu
                85                  90                  95

Lys Leu Asn Gly Thr Asp Pro Glu Glu Ala Ile Leu Ser Ala Phe Arg
            100                 105                 110

Met Phe Asp Pro Ser Gly Lys Gly Val Val Asn Lys Asp Glu Phe Lys
        115                 120                 125

Gln Leu Leu Leu Thr Gln Ala Asp Lys Phe Ser Pro Ala Glu Val Glu
    130                 135                 140

Gln Met Phe Ala Leu Thr Pro Met Asp Leu Ala Gly Asn Ile Asp Tyr
145                 150                 155                 160

Lys Ser Leu Cys Tyr Ile Ile Thr His Gly Asp Glu Lys Glu Glu
                165                 170                 175
```

<210> 303
<211> 208
<212> PRT
<213> Homo sapiens

<400> 303

```
Met Ala Ser Arg Lys Ala Gly Thr Arg Gly Lys Val Ala Ala Thr Lys
1               5                   10                  15

Gln Ala Gln Arg Gly Ser Ser Asn Val Phe Ser Met Phe Glu Gln Ala
            20                  25                  30

Gln Ile Gln Glu Phe Lys Glu Ala Phe Ser Cys Ile Asp Gln Asn Arg
        35                  40                  45

Asp Gly Ile Ile Cys Lys Ala Asp Leu Arg Glu Thr Tyr Ser Gln Leu
    50                  55                  60
```

Gly Lys Val Ser Val Pro Glu Glu Glu Leu Asp Ala Met Leu Gln Glu
65                  70                  75                  80

Gly Lys Gly Pro Ile Asn Phe Thr Val Phe Leu Thr Leu Phe Gly Glu
                85                  90                  95

Lys Leu Asn Gly Thr Asp Pro Glu Glu Ala Ile Leu Ser Ala Phe Arg
            100                 105                 110

Met Phe Asp Pro Ser Gly Lys Gly Val Val Asn Lys Asp Glu Phe Lys
        115                 120                 125

Gln Leu Leu Leu Thr Gln Ala Asp Lys Phe Ser Pro Ala Glu Val Arg
    130                 135                 140

Leu Pro Ser Pro Phe Asn Thr His Pro Gln His Leu Leu Trp Ala Phe
145                 150                 155                 160

Thr His Asp Pro Glu Pro Ser Thr Ser Glu Ala Val Ala Gly Arg Val
                165                 170                 175

Glu Gln Met Phe Ala Leu Thr Pro Met Asp Leu Ala Gly Asn Ile Asp
            180                 185                 190

Tyr Lys Ser Leu Cys Tyr Ile Ile Thr His Gly Asp Glu Lys Glu Glu
        195                 200                 205

<210>  304
<211>  163
<212>  PRT
<213>  Homo sapiens

<400> 304

Met Ala Ser Arg Lys Ala Gly Thr Arg Gly Lys Val Ala Ala Thr Lys
1               5                   10                  15

Gln Ala Gln Arg Gly Ser Ser Asn Val Phe Ser Met Phe Glu Gln Ala
            20                  25                  30

Gln Ile Gln Glu Phe Lys Glu Ala Phe Ser Cys Ile Asp Gln Asn Arg
        35                  40                  45

Asp Gly Ile Ile Cys Lys Ala Asp Leu Arg Glu Thr Tyr Ser Gln Leu
    50                  55                  60

Gly Lys Val Ser Val Pro Glu Glu Glu Leu Asp Ala Met Leu Gln Glu
65                  70                  75                  80

```
Gly Lys Gly Pro Ile Asn Phe Thr Val Phe Leu Thr Leu Phe Gly Glu
                85                  90                  95

Lys Leu Asn Gly Thr Asp Pro Glu Glu Ala Ile Leu Ser Ala Phe Arg
            100                 105                 110

Met Phe Asp Pro Ser Gly Lys Gly Val Val Asn Lys Asp Asp Gln Pro
        115                 120                 125

Phe Pro Ala Pro Trp Glu Pro Pro Tyr Pro Pro Ser Leu Cys Ser His
    130                 135                 140

Ser Pro Ala Val Ser Cys Ser Asp Pro Pro His Pro Pro Gly Ser Ser
145                 150                 155                 160

Ser Phe Ser



<210>  305
<211>  198
<212>  PRT
<213>  Homo sapiens

<400> 305

Met Ala Ser Arg Lys Ala Gly Thr Arg Gly Lys Val Ala Ala Thr Lys
1               5                   10                  15

Gln Ala Gln Arg Gly Ser Ser Asn Val Phe Ser Met Phe Glu Gln Ala
            20                  25                  30

Gln Ile Gln Glu Phe Lys Glu Val Ser Pro Pro Pro Pro Thr Phe Pro
        35                  40                  45

Arg Ala Gly Gly Cys Ser His Leu Lys Ala Pro Ile Pro Gln Ala Phe
    50                  55                  60

Ser Cys Ile Asp Gln Asn Arg Asp Gly Ile Ile Cys Lys Ala Asp Leu
65                  70                  75                  80

Arg Glu Thr Tyr Ser Gln Leu Gly Lys Val Ser Val Pro Glu Glu Glu
                85                  90                  95

Leu Asp Ala Met Leu Gln Glu Gly Lys Gly Pro Ile Asn Phe Thr Val
            100                 105                 110

Phe Leu Thr Leu Phe Gly Glu Lys Leu Asn Gly Thr Asp Pro Glu Glu
        115                 120                 125
```

```
Ala Ile Leu Ser Ala Phe Arg Met Phe Asp Pro Ser Gly Lys Gly Val
    130                 135                 140

Val Asn Lys Asp Glu Phe Lys Gln Leu Leu Leu Thr Gln Ala Asp Lys
145                 150                 155                 160

Phe Ser Pro Ala Glu Val Glu Gln Met Phe Ala Leu Thr Pro Met Asp
                165                 170                 175

Leu Ala Gly Asn Ile Asp Tyr Lys Ser Leu Cys Tyr Ile Ile Thr His
            180                 185                 190

Gly Asp Glu Lys Glu Glu
        195


<210>  306
<211>  132
<212>  PRT
<213>  Homo sapiens

<400> 306

Met Ala Ser Arg Lys Ala Gly Thr Arg Gly Lys Val Ala Ala Thr Lys
1               5                   10                  15

Gln Ala Gln Arg Gly Ser Ser Asn Val Phe Ser Met Phe Glu Gln Ala
            20                  25                  30

Gln Ile Gln Glu Phe Lys Glu Ala Phe Ser Cys Ile Asp Gln Asn Arg
        35                  40                  45

Asp Gly Ile Ile Cys Lys Ala Asp Leu Arg Glu Thr Tyr Ser Gln Leu
    50                  55                  60

Gly Lys Val Ser Val Pro Glu Glu Glu Leu Asp Ala Met Leu Gln Glu
65                  70                  75                  80

Gly Lys Gly Pro Ile Asn Phe Thr Val Phe Leu Thr Leu Phe Gly Glu
                85                  90                  95

Lys Leu Asn Gly Thr Asp Pro Glu Glu Ala Ile Leu Ser Ala Phe Arg
            100                 105                 110

Met Phe Asp Pro Ser Gly Lys Gly Val Val Asn Lys Asp Glu Trp Ser
        115                 120                 125

Arg Cys Ser Pro
```

130

<210> 307
<211> 144
<212> PRT
<213> Homo sapiens

<400> 307

```
Met Ala Ser Arg Lys Ala Gly Thr Arg Gly Lys Val Ala Ala Thr Lys
1               5                   10                  15

Gln Ala Gln Arg Gly Ser Ser Asn Val Phe Ser Met Phe Glu Gln Ala
            20                  25                  30

Gln Ile Gln Glu Phe Lys Glu Ala Phe Ser Cys Ile Asp Gln Asn Arg
        35                  40                  45

Asp Gly Ile Ile Cys Lys Ala Asp Leu Arg Glu Thr Tyr Ser Gln Leu
    50                  55                  60

Ala Pro Val Ala Arg Met Glu Gly Gly Arg Pro Gly Pro Phe Trp Gly
65                  70                  75                  80

Thr Pro Arg Val Pro Val Cys Thr Ser Cys Pro Thr Pro Thr Arg Glu
                85                  90                  95

Gly Glu Cys Pro Arg Gly Gly Ala Gly Arg His Ala Ala Arg Gly Gln
            100                 105                 110

Gly Pro His Gln Leu His Arg Leu Pro His Ala Leu Trp Gly Glu Ala
        115                 120                 125

Gln Trp Asp Arg Pro Arg Gly Ser His Pro Glu Cys Leu Pro His Val
    130                 135                 140
```

<210> 308
<211> 102
<212> PRT
<213> Homo sapiens

<400> 308

```
Met Ala Ser Arg Lys Ala Gly Thr Arg Gly Lys Val Ala Ala Thr Lys
1               5                   10                  15

Gln Ala Gln Arg Gly Ser Ser Asn Val Phe Ser Met Phe Glu Gln Ala
            20                  25                  30
```

```
Gln Ile Gln Glu Phe Lys Glu Val Ser Pro Pro Pro Pro Thr Phe Pro
        35                  40                  45

Arg Ala Gly Gly Cys Ser His Leu Lys Ala Pro Ile Pro Gln Ala Phe
    50                  55                  60

Ser Cys Ile Asp Gln Asn Arg Asp Gly Ile Ile Cys Lys Ala Asp Leu
65                  70                  75                  80

Arg Glu Thr Tyr Ser Gln Leu Gly Ala Cys Thr His Leu Pro Pro Cys
                85                  90                  95

Ala Leu Gly Ser Leu Leu
            100
```

<210>  309
<211>  21
<212>  DNA
<213>  Artificial Organism

<220>
<223>  Synthetic oligonucleotide

<400> 309

ggaaggtgag tgtcccagag g     21

<210>  310
<211>  23
<212>  DNA
<213>  Artificial Organism

<220>
<223>  Synthetic oligonucleotide

<400> 310

gtgaggaaga cggtgaagtt gat     23

<210>  311
<211>  82
<212>  DNA
<213>  Artificial Organism

<220>
<223>  Synthetic oligonucleotide

<400> 311

ggaaggtgag tgtcccagag gaggagctgg acgccatgct gcaagagggc aagggcccca     60

tcaacttcac cgtcttcctc ac     82

<210>  312

<211> 20
<212> DNA
<213> Artificial Organism

<220>
<223> Synthetic oligonucleotide

<400> 312

cccaccttcc ctagagctgg 20

<210> 313
<211> 21
<212> DNA
<213> Artificial Organism

<220>
<223> Synthetic oligonucleotide

<400> 313

aggtctgcct tgcagatgat g 21

<210> 314
<211> 107
<212> DNA
<213> Artificial Organism

<220>
<223> Synthetic oligonucleotide

<400> 314

cccaccttcc ctagagctgg gggctgctcc cacctgaagg cccccatccc acaggccttc 60

agctgtatcg accagaatcg tgatggcatc atctgcaagg cagacct 107

<210> 315
<211> 23
<212> DNA
<213> Artificial Organism

<220>
<223> Synthetic oligonucleotide

<400> 315

aggcagacaa gttctctcca gct 23

<210> 316
<211> 19
<212> DNA
<213> Artificial Organism

<220>
<223> Synthetic oligonucleotide

<400> 316

ggtgaaggcc cagagaagg 19

<210> 317
<211> 83
<212> DNA
<213> Artificial Organism

<220>
<223> Synthetic oligonucleotide

<400> 317

aggcagacaa gttctctcca gctgaggtga ggctgcccag ccccttcaat actcatcccc 60

agcaccttct ctgggccttc acc 83

<210> 318
<211> 17
<212> DNA
<213> Artificial Organism

<220>
<223> Synthetic oligonucleotide

<400> 318

tgtcccagcc tccccac 17

<210> 319
<211> 19
<212> DNA
<213> Artificial Organism

<220>
<223> Synthetic oligonucleotide

<400> 319

gagtcacatt cagggcccc 19

<210> 320
<211> 91
<212> DNA
<213> Artificial Organism

<220>
<223> Synthetic oligonucleotide

<400> 320

tgtcccagcc tccccacctt ctcagaccag catgtggccc ttaagtccac ttgtaacact 60

atacccatgg ttggggccct gaatgtgact c 91

<210> 321
<211> 21
<212> DNA
<213> Artificial Organism

<220>
<223>  Synthetic oligonucleotide

<400> 321

caactctcct caggttcccc t    21

<210>  322
<211>  24
<212>  DNA
<213>  Artificial Organism

<220>
<223>  Synthetic oligonucleotide

<400> 322

gagaaggagg aatgaagaag gctt    24

<210>  323
<211>  91
<212>  DNA
<213>  Artificial Organism

<220>
<223>  Synthetic oligonucleotide

<400> 323

caactctcct caggttcccc tgaagtaatt cattcttcct ctacacctga agctctagtt    60

gcctggaaag ccttcttcat tcctccttct c    91

<210>  324
<211>  50
<212>  DNA
<213>  Homo sapiens

<400> 324

agtgggcact cggctccgga cactgtaact cttgctctct accttgctca    50

<210>  325
<211>  50
<212>  DNA
<213>  Homo sapiens

<400> 325

atgcccttta tcttgggcct cttgtctgga ggtgtgacca ccactccatg    50

<210>  326
<211>  46
<212>  PRT
<213>  Artificial Organism

<220>

<223>  Synthetic peptide

<400> 326

```
Ala Pro Leu Pro Pro Leu Leu Gln Ser Leu Pro Glu Ala Trp Ala Leu
1               5                   10                  15

Arg Gly Gly Arg Arg Val Pro Val Arg Pro Gly Arg Gly Ala Glu Cys
            20                  25                  30

Ala Arg Thr Gly Leu His Arg Ala Ala Arg Ser Ala Arg Ala
        35                  40                  45
```

<210>  327
<211>  44
<212>  PRT
<213>  Artificial Organism

<220>
<223>  Synthetic peptide

<400> 327

```
Val Arg Ala Arg His Gly Pro Leu Ala Ser Ser Ser Cys Arg Ser Thr
1               5                   10                  15

Leu Ser Gly Arg Val Gln Ala Leu Gly Pro Arg Gly Pro Pro Ala Ala
            20                  25                  30

Pro Gly Ser Pro Ala Ala Ser Ser Ser Glu Ser Ala
        35                  40
```

<210>  328
<211>  44
<212>  PRT
<213>  Artificial Organism

<220>
<223>  Synthetic peptide

<400> 328

```
Glu Ala Trp Arg Pro Glu Arg Arg Gly Met Gly Trp Gly Ser Trp Met
1               5                   10                  15

Ala Gln Thr Val Gln Gly Trp Asn Pro Gly Phe Asp Ser Ser Asn Pro
            20                  25                  30

Arg Ala Trp Gly Pro Asp Leu Pro Pro Ala Ser Leu
        35                  40
```

<210> 329
<211> 10
<212> PRT
<213> Artificial Organism

<220>
<223> Synthetic peptide

<400> 329

```
Ala Ala Leu Ala Glu Gly Glu Thr Pro Arg
1               5                   10
```

<210> 330
<211> 198
<212> PRT
<213> Artificial Organism

<220>
<223> Synthetic peptide

<400> 330

```
Gly Glu Lys Arg Asp Leu Glu Ile Glu Val Val Leu Phe His Pro Asn
1               5                   10                  15

Tyr Asn Ile Asn Gly Lys Lys Glu Ala Gly Ile Pro Glu Phe Tyr Asp
            20                  25                  30

Tyr Asp Val Ala Leu Ile Lys Leu Lys Asn Lys Leu Lys Tyr Gly Gln
        35                  40                  45

Thr Ile Arg Pro Ile Cys Leu Pro Cys Thr Glu Gly Thr Thr Arg Ala
    50                  55                  60

Leu Arg Leu Pro Pro Thr Thr Thr Cys Gln Gln Gln Lys Glu Glu Leu
65                  70                  75                  80

Leu Pro Ala Gln Asp Ile Lys Ala Leu Phe Val Ser Glu Glu Glu Lys
                85                  90                  95

Lys Leu Thr Arg Lys Glu Val Tyr Ile Lys Asn Gly Asp Lys Lys Gly
            100                 105                 110

Ser Cys Glu Arg Asp Ala Gln Tyr Ala Pro Gly Tyr Asp Lys Val Lys
        115                 120                 125

Asp Ile Ser Glu Val Val Thr Pro Arg Phe Leu Cys Thr Gly Gly Val
    130                 135                 140

Ser Pro Tyr Ala Asp Pro Asn Thr Cys Arg Gly Asp Ser Gly Gly Pro
145                 150                 155                 160
```

```
Leu Ile Val His Lys Arg Ser Arg Phe Ile Gln Val Gly Val Ile Ser
                165                 170                 175

Trp Gly Val Val Asp Val Cys Lys Asn Gln Lys Arg Ala Ala Leu Ala
            180                 185                 190

Glu Gly Glu Thr Pro Arg
        195
```

<210> 331
<211> 60
<212> PRT
<213> Artificial Organism

<220>
<223> Synthetic peptide

<400> 331

```
Gln Lys Gly Pro Leu Ser Cys Pro Ser Leu Pro Thr Phe Ser Asp Gln
1               5                   10                  15

His Val Ala Leu Lys Ser Thr Cys Asn Thr Ile Pro Met Val Gly Ala
            20                  25                  30

Leu Asn Val Thr His Ser Trp Leu Phe Ile Ser Pro Val Thr Leu His
        35                  40                  45

Lys Glu Phe Phe Leu Ser Pro Val Ile Asn Tyr Leu
    50                  55                  60
```

<210> 332
<211> 30
<212> PRT
<213> Artificial Organism

<220>
<223> Synthetic peptide

<400> 332

```
Ser Pro Pro Phe Pro Ile Trp Gly Asp Ala Lys Trp Ser Ala Trp Ala
1               5                   10                  15

Pro Lys Gln Glu Ser Ser Met His Val Ala Ser Asn Ser Arg
            20                  25                  30
```

<210> 333
<211> 202

<212> PRT
<213> Artificial Organism

<220>
<223> Synthetic peptide

<400> 333

```
Gly Glu Lys Arg Asp Leu Glu Ile Glu Val Val Leu Phe His Pro Asn
1               5                   10                  15

Tyr Asn Ile Asn Gly Lys Lys Glu Ala Gly Ile Pro Glu Phe Tyr Asp
            20                  25                  30

Tyr Asp Val Ala Leu Ile Lys Leu Lys Asn Lys Leu Lys Tyr Gly Gln
        35                  40                  45

Thr Ile Arg Pro Ile Cys Leu Pro Cys Thr Glu Gly Thr Thr Arg Ala
    50                  55                  60

Leu Arg Leu Pro Pro Thr Thr Thr Cys Gln Gln Gln Lys Glu Glu Leu
65                  70                  75                  80

Leu Pro Ala Gln Asp Ile Lys Ala Leu Phe Val Ser Glu Glu Glu Lys
                85                  90                  95

Lys Leu Thr Arg Lys Glu Val Tyr Ile Lys Asn Gly Asp Lys Lys Gly
            100                 105                 110

Ser Cys Glu Arg Asp Ala Gln Tyr Ala Pro Gly Tyr Asp Lys Val Lys
        115                 120                 125

Asp Ile Ser Glu Val Val Thr Pro Arg Phe Leu Cys Thr Gly Gly Val
    130                 135                 140

Ser Pro Tyr Ala Asp Pro Asn Thr Cys Arg Gly Asp Ser Gly Gly Pro
145                 150                 155                 160

Leu Ile Val His Lys Arg Ser Arg Phe Ile Gln Val Ser Pro Pro Phe
                165                 170                 175

Pro Ile Trp Gly Asp Ala Lys Trp Ser Ala Trp Ala Pro Lys Gln Glu
            180                 185                 190

Ser Ser Met His Val Ala Ser Asn Ser Arg
        195                 200
```

<210> 334
<211> 15

<212> PRT
<213> Artificial Organism

<220>
<223> Synthetic peptide

<400> 334

```
Arg Arg Ala Ala Pro Cys Thr Gly Tyr Gln Ser Ser Val Cys Val
1               5                   10                  15
```

<210> 335
<211> 91
<212> PRT
<213> Artificial Organism

<220>
<223> Synthetic peptide

<400> 335

```
Gly Glu Lys Arg Asp Leu Glu Ile Glu Val Val Leu Phe His Pro Asn
1               5                   10                  15

Tyr Asn Ile Asn Gly Lys Lys Glu Ala Gly Ile Pro Glu Phe Tyr Asp
            20                  25                  30

Tyr Asp Val Ala Leu Ile Lys Leu Lys Asn Lys Leu Lys Tyr Gly Gln
        35                  40                  45

Thr Ile Arg Pro Ile Cys Leu Pro Cys Thr Glu Gly Thr Thr Arg Ala
    50                  55                  60

Leu Arg Leu Pro Pro Thr Thr Thr Cys Gln Gln Gln Arg Arg Ala Ala
65                  70                  75                  80

Pro Cys Thr Gly Tyr Gln Ser Ser Val Cys Val
                85                  90
```

<210> 336
<211> 15
<212> PRT
<213> Artificial Organism

<220>
<223> Synthetic peptide

<400> 336

```
Gly Arg Ala Ala Pro Cys Thr Gly Tyr Gln Ser Ser Val Cys Val
1               5                   10                  15
```

<210> 337
<211> 66
<212> PRT
<213> Artificial Organism

<220>
<223> Synthetic peptide

<400> 337

```
Gly Glu Lys Arg Asp Leu Glu Ile Glu Val Val Leu Phe His Pro Asn
1               5                   10                  15

Tyr Asn Ile Asn Gly Lys Lys Glu Ala Gly Ile Pro Glu Phe Tyr Asp
            20                  25                  30

Tyr Asp Val Ala Leu Ile Lys Leu Lys Asn Lys Leu Lys Tyr Gly Gln
        35                  40                  45

Thr Ile Arg Gly Arg Ala Ala Pro Cys Thr Gly Tyr Gln Ser Ser Val
    50                  55                  60

Cys Val
65
```

<210> 338
<211> 10
<212> PRT
<213> Artificial Organism

<220>
<223> Synthetic peptide

<400> 338

```
Val Arg Asn Gly His Pro Lys Glu Ala Leu
1               5                   10
```

<210> 339
<211> 120
<212> PRT
<213> Artificial Organism

<220>
<223> Synthetic peptide

<400> 339

```
Gly Glu Lys Arg Asp Leu Glu Ile Glu Val Val Leu Phe His Pro Asn
1               5                   10                  15

Tyr Asn Ile Asn Gly Lys Lys Glu Ala Gly Ile Pro Glu Phe Tyr Asp
            20                  25                  30
```

```
Tyr Asp Val Ala Leu Ile Lys Leu Lys Asn Lys Leu Lys Tyr Gly Gln
        35                  40                  45

Thr Ile Arg Pro Ile Cys Leu Pro Cys Thr Glu Gly Thr Thr Arg Ala
    50                  55                  60

Leu Arg Leu Pro Pro Thr Thr Thr Cys Gln Gln Gln Lys Glu Glu Leu
65                  70                  75                  80

Leu Pro Ala Gln Asp Ile Lys Ala Leu Phe Val Ser Glu Glu Glu Lys
                85                  90                  95

Lys Leu Thr Arg Lys Glu Val Tyr Ile Lys Asn Gly Asp Lys Val Arg
            100                 105                 110

Asn Gly His Pro Lys Glu Ala Leu
        115                 120


<210>  340
<211>  146
<212>  PRT
<213>  Artificial Organism

<220>
<223>  Synthetic peptide

<400> 340

Glu Glu Glu Leu Leu Pro Ala Gln Asp Ile Lys Ala Leu Phe Val Ser
1               5                   10                  15

Glu Glu Glu Lys Lys Leu Thr Arg Lys Glu Val Tyr Ile Lys Asn Gly
            20                  25                  30

Asp Lys Lys Gly Ser Cys Glu Arg Asp Ala Gln Tyr Ala Pro Gly Tyr
        35                  40                  45

Asp Lys Val Lys Asp Ile Ser Glu Val Val Thr Pro Arg Phe Leu Cys
    50                  55                  60

Thr Gly Gly Val Ser Pro Tyr Ala Asp Pro Asn Thr Cys Arg Gly Asp
65                  70                  75                  80

Ser Gly Gly Pro Leu Ile Val His Lys Arg Ser Arg Phe Ile Gln Val
                85                  90                  95

Gly Val Ile Ser Trp Gly Val Val Asp Val Cys Lys Asn Gln Lys Arg
            100                 105                 110
```

Gln Lys Gln Val Pro Ala His Ala Arg Asp Phe His Ile Asn Leu Phe
        115                 120                 125

Gln Val Leu Pro Trp Leu Lys Glu Lys Leu Gln Asp Glu Asp Leu Gly
    130                 135                 140

Phe Leu
145

<210>  341
<211>  12
<212>  PRT
<213>  Artificial Organism

<220>
<223>  Synthetic peptide

<400> 341

Gly Arg Glu Ile Gln Gly Asn Lys Glu His Asn Ser
1               5                   10

<210>  342
<211>  11
<212>  PRT
<213>  Artificial Organism

<220>
<223>  Synthetic peptide

<400> 342

Ala Val Arg Arg Gly Leu Arg Glu Gly Gly Ala
1               5                   10

<210>  343
<211>  125
<212>  PRT
<213>  Artificial Organism

<220>
<223>  Synthetic peptide

<400> 343

Ala Val Arg Arg Gly Leu Arg Glu Gly Gly Ala Met Ala Ala Ala Arg
1               5                   10                  15

Asp Pro Pro Glu Val Ser Leu Arg Glu Ala Thr Gln Arg Lys Leu Arg
            20                  25                  30

Arg Phe Ser Glu Leu Arg Gly Lys Leu Val Ala Arg Gly Glu Phe Trp

35 40 45

Asp Ile Val Ala Ile Thr Ala Ala Asp Glu Lys Gln Glu Leu Ala Tyr
50 55 60

Asn Gln Gln Leu Ser Glu Lys Leu Lys Arg Lys Glu Leu Pro Leu Gly
65 70 75 80

Val Gln Tyr His Val Phe Val Asp Pro Ala Gly Ala Lys Ile Gly Asn
85 90 95

Gly Gly Ser Thr Leu Cys Ala Leu Gln Cys Leu Glu Lys Leu Tyr Gly
100 105 110

Asp Lys Trp Asn Ser Phe Thr Ile Leu Leu Ile His Ser
115 120 125

<210> 344
<211> 7
<212> PRT
<213> Artificial Organism

<220>
<223> Synthetic peptide

<400> 344

Ala Val Arg Arg Gly Leu Arg
1 5

<210> 345
<211> 266
<212> PRT
<213> Artificial Organism

<220>
<223> Synthetic peptide

<400> 345

Ser Ala Ile Thr Ser Arg Ile Trp Ile Thr His Ser Ile Cys Ile Trp
1 5 10 15

Arg Gly Ala His Tyr Phe Asn Arg Glu Glu Cys Asn Phe Arg Cys Met
20 25 30

Leu Thr Ser Ala Ile Leu Lys Glu Ser Arg Phe Leu Gln Ser Leu Asp
35 40 45

Glu Asp Asn Met Thr Lys Gln Pro Gly Asn Leu Arg Trp Met Ala Pro
50 55 60

```
Glu Val Phe Thr Gln Cys Thr Arg Tyr Thr Ile Lys Ala Asp Val Phe
65                  70                  75                  80

Ser Tyr Ala Leu Cys Leu Trp Glu Ile Leu Thr Gly Glu Ile Pro Phe
                85                  90                  95

Ala His Leu Lys Pro Ala Ala Ala Ala Ala Asp Met Ala Tyr His His
            100                 105                 110

Ile Arg Pro Pro Ile Gly Tyr Ser Ile Pro Lys Pro Ile Ser Ser Leu
        115                 120                 125

Leu Ile Arg Gly Trp Asn Ala Cys Pro Glu Gly Arg Pro Glu Phe Ser
    130                 135                 140

Glu Val Val Met Lys Leu Glu Glu Cys Leu Cys Asn Ile Glu Leu Met
145                 150                 155                 160

Ser Pro Ala Ser Ser Asn Ser Ser Gly Ser Leu Ser Pro Ser Ser Ser
                165                 170                 175

Ser Asp Cys Leu Val Asn Arg Gly Gly Pro Gly Arg Ser His Val Ala
            180                 185                 190

Ala Leu Arg Ser Arg Phe Glu Leu Glu Tyr Ala Leu Asn Ala Arg Ser
        195                 200                 205

Tyr Ala Ala Leu Ser Gln Ser Ala Gly Gln Tyr Ser Ser Gln Gly Leu
    210                 215                 220

Ser Leu Glu Glu Met Lys Arg Ser Leu Gln Tyr Thr Pro Ile Asp Lys
225                 230                 235                 240

Tyr Gly Tyr Val Ser Asp Pro Met Ser Ser Met His Phe His Ser Cys
                245                 250                 255

Arg Asn Ser Ser Ser Phe Glu Asp Ser Ser
            260                 265
```

<210>  346
<211>  13
<212>  PRT
<213>  Artificial Organism

<220>
<223>  Synthetic peptide

<400> 346

```
Met Gly Asn Tyr Lys Ser Arg Pro Thr Gln Thr Cys Thr
1               5                   10
```

```
<210>  347
<211>  266
<212>  PRT
<213>  Artificial Organism

<220>
<223>  Synthetic peptide

<400> 347

Ser Ala Ile Thr Ser Arg Ile Trp Ile Thr His Ser Ile Cys Ile Trp
1               5                   10                  15

Arg Gly Ala His Tyr Phe Asn Arg Glu Glu Cys Asn Phe Arg Cys Met
            20                  25                  30

Leu Thr Ser Ala Ile Leu Lys Glu Ser Arg Phe Leu Gln Ser Leu Asp
        35                  40                  45

Glu Asp Asn Met Thr Lys Gln Pro Gly Asn Leu Arg Trp Met Ala Pro
    50                  55                  60

Glu Val Phe Thr Gln Cys Thr Arg Tyr Thr Ile Lys Ala Asp Val Phe
65                  70                  75                  80

Ser Tyr Ala Leu Cys Leu Trp Glu Ile Leu Thr Gly Glu Ile Pro Phe
                85                  90                  95

Ala His Leu Lys Pro Ala Ala Ala Ala Ala Asp Met Ala Tyr His His
            100                 105                 110

Ile Arg Pro Pro Ile Gly Tyr Ser Ile Pro Lys Pro Ile Ser Ser Leu
        115                 120                 125

Leu Ile Arg Gly Trp Asn Ala Cys Pro Glu Gly Arg Pro Glu Phe Ser
    130                 135                 140

Glu Val Val Met Lys Leu Glu Glu Cys Leu Cys Asn Ile Glu Leu Met
145                 150                 155                 160

Ser Pro Ala Ser Ser Asn Ser Ser Gly Ser Leu Ser Pro Ser Ser Ser
                165                 170                 175

Ser Asp Cys Leu Val Asn Arg Gly Gly Pro Gly Arg Ser His Val Ala
            180                 185                 190
```

```
Ala Leu Arg Ser Arg Phe Glu Leu Glu Tyr Ala Leu Asn Ala Arg Ser
        195                 200                 205

Tyr Ala Ala Leu Ser Gln Ser Ala Gly Gln Tyr Ser Ser Gln Gly Leu
    210                 215                 220

Ser Leu Glu Glu Met Lys Arg Ser Leu Gln Tyr Thr Pro Ile Asp Lys
225                 230                 235                 240

Tyr Gly Tyr Val Ser Asp Pro Met Ser Ser Met His Phe His Ser Cys
                245                 250                 255

Arg Asn Ser Ser Ser Phe Glu Asp Ser Ser
            260                 265
```

<210> 348
<211> 13
<212> PRT
<213> Artificial Organism

<220>
<223> Synthetic peptide

<400> 348

```
Met Gly Asn Tyr Lys Ser Arg Pro Thr Gln Thr Cys Thr
1               5                   10
```

<210> 349
<211> 4
<212> PRT
<213> Artificial Organism

<220>
<223> Synthetic peptide

<400> 349

```
Asp Val Thr Ser
1
```

<210> 350
<211> 224
<212> PRT
<213> Artificial Organism

<220>
<223> Synthetic peptide

<400> 350

```
Ser His Asn Ile Leu Leu Tyr Glu Asp Gly His Ala Val Val Ala Asp
1               5                   10                  15

Phe Gly Glu Ser Arg Phe Leu Gln Ser Leu Asp Glu Asp Asn Met Thr
            20                  25                  30

Lys Gln Pro Gly Asn Leu Arg Trp Met Ala Pro Glu Val Phe Thr Gln
        35                  40                  45

Cys Thr Arg Tyr Thr Ile Lys Ala Asp Val Phe Ser Tyr Ala Leu Cys
    50                  55                  60

Leu Trp Glu Ile Leu Thr Gly Glu Ile Pro Phe Ala His Leu Lys Pro
65                  70                  75                  80

Ala Ala Ala Ala Ala Asp Met Ala Tyr His His Ile Arg Pro Pro Ile
                85                  90                  95

Gly Tyr Ser Ile Pro Lys Pro Ile Ser Ser Leu Leu Ile Arg Gly Trp
            100                 105                 110

Asn Ala Cys Pro Glu Gly Arg Pro Glu Phe Ser Glu Val Val Met Lys
        115                 120                 125

Leu Glu Glu Cys Leu Cys Asn Ile Glu Leu Met Ser Pro Ala Ser Ser
    130                 135                 140

Asn Ser Ser Gly Ser Leu Ser Pro Ser Ser Ser Ser Asp Cys Leu Val
145                 150                 155                 160

Asn Arg Gly Gly Pro Gly Arg Ser His Val Ala Ala Leu Arg Ser Arg
                165                 170                 175

Phe Glu Leu Glu Tyr Ala Leu Asn Ala Arg Ser Tyr Ala Ala Leu Ser
            180                 185                 190

Gln Ser Ala Gly Gln Tyr Ser Ser Gln Gly Leu Ser Leu Glu Glu Met
        195                 200                 205

Lys Arg Ser Leu Gln Tyr Thr Pro Ile Asp Lys Tyr Asp Val Thr Ser
    210                 215                 220
```

<210>  351
<211>  15
<212>  PRT
<213>  Artificial Organism

<220>

<223>  Synthetic peptide

<400> 351

```
Pro Ser Pro Cys Gly Leu His Phe Leu Ile Pro Trp Leu Thr Gln
1               5                   10                  15
```

<210>  352
<211>  245
<212>  PRT
<213>  Artificial Organism

<220>
<223>  Synthetic peptide

<400> 352

```
Ser His Asn Ile Leu Leu Tyr Glu Asp Gly His Ala Val Val Ala Asp
1               5                   10                  15

Phe Gly Glu Ser Arg Phe Leu Gln Ser Leu Asp Glu Asp Asn Met Thr
            20                  25                  30

Lys Gln Pro Gly Asn Leu Arg Trp Met Ala Pro Glu Val Phe Thr Gln
        35                  40                  45

Cys Thr Arg Tyr Thr Ile Lys Ala Asp Val Phe Ser Tyr Ala Leu Cys
    50                  55                  60

Leu Trp Glu Ile Leu Thr Gly Glu Ile Pro Phe Ala His Leu Lys Pro
65                  70                  75                  80

Ala Ala Ala Ala Ala Asp Met Ala Tyr His His Ile Arg Pro Pro Ile
                85                  90                  95

Gly Tyr Ser Ile Pro Lys Pro Ile Ser Ser Leu Leu Ile Arg Gly Trp
            100                 105                 110

Asn Ala Cys Pro Glu Gly Arg Pro Glu Phe Ser Glu Val Val Met Lys
        115                 120                 125

Leu Glu Glu Cys Leu Cys Asn Ile Glu Leu Met Ser Pro Ala Ser Ser
    130                 135                 140

Asn Ser Ser Gly Ser Leu Ser Pro Ser Ser Ser Ser Asp Cys Leu Val
145                 150                 155                 160

Asn Arg Gly Gly Pro Gly Arg Ser His Val Ala Ala Leu Arg Ser Arg
                165                 170                 175
```

```
Phe Glu Leu Glu Tyr Ala Leu Asn Ala Arg Ser Tyr Ala Ala Leu Ser
            180                 185                 190

Gln Ser Ala Gly Gln Tyr Ser Ser Gln Gly Leu Ser Leu Glu Glu Met
        195                 200                 205

Lys Arg Ser Leu Gln Tyr Thr Pro Ile Asp Lys Tyr Gly Tyr Val Ser
    210                 215                 220

Asp Pro Met Ser Ser Met His Phe His Ser Cys Arg Asn Ser Ser Ser
225                 230                 235                 240

Phe Glu Asp Ser Ser
                245



<210>  353
<211>  15
<212>  PRT
<213>  Artificial Organism

<220>
<223>  Synthetic peptide

<400> 353

Pro Ser Pro Cys Gly Leu His Phe Leu Ile Pro Trp Leu Thr Gln
1               5                   10                  15



<210>  354
<211>  266
<212>  PRT
<213>  Artificial Organism

<220>
<223>  Synthetic peptide

<400> 354

Ser Ala Ile Thr Ser Arg Ile Trp Ile Thr His Ser Ile Cys Ile Trp
1               5                   10                  15

Arg Gly Ala His Tyr Phe Asn Arg Glu Glu Cys Asn Phe Arg Cys Met
            20                  25                  30

Leu Thr Ser Ala Ile Leu Lys Glu Ser Arg Phe Leu Gln Ser Leu Asp
        35                  40                  45

Glu Asp Asn Met Thr Lys Gln Pro Gly Asn Leu Arg Trp Met Ala Pro
    50                  55                  60

Glu Val Phe Thr Gln Cys Thr Arg Tyr Thr Ile Lys Ala Asp Val Phe
```

```
65                  70                  75                  80

Ser Tyr Ala Leu Cys Leu Trp Glu Ile Leu Thr Gly Glu Ile Pro Phe
                85                  90                  95

Ala His Leu Lys Pro Ala Ala Ala Ala Ala Asp Met Ala Tyr His His
            100                 105                 110

Ile Arg Pro Pro Ile Gly Tyr Ser Ile Pro Lys Pro Ile Ser Ser Leu
        115                 120                 125

Leu Ile Arg Gly Trp Asn Ala Cys Pro Glu Gly Arg Pro Glu Phe Ser
    130                 135                 140

Glu Val Val Met Lys Leu Glu Glu Cys Leu Cys Asn Ile Glu Leu Met
145                 150                 155                 160

Ser Pro Ala Ser Ser Asn Ser Ser Gly Ser Leu Ser Pro Ser Ser Ser
                165                 170                 175

Ser Asp Cys Leu Val Asn Arg Gly Gly Pro Gly Arg Ser His Val Ala
            180                 185                 190

Ala Leu Arg Ser Arg Phe Glu Leu Glu Tyr Ala Leu Asn Ala Arg Ser
        195                 200                 205

Tyr Ala Ala Leu Ser Gln Ser Ala Gly Gln Tyr Ser Ser Gln Gly Leu
    210                 215                 220

Ser Leu Glu Glu Met Lys Arg Ser Leu Gln Tyr Thr Pro Ile Asp Lys
225                 230                 235                 240

Tyr Gly Tyr Val Ser Asp Pro Met Ser Ser Met His Phe His Ser Cys
                245                 250                 255

Arg Asn Ser Ser Ser Phe Glu Asp Ser Ser
            260                 265
```

<210> 355
<211> 11
<212> PRT
<213> Artificial Organism

<220>
<223> Synthetic peptide

<400> 355

Ala Val Arg Arg Gly Leu Arg Glu Gly Gly Ala

1 5 10

<210> 356
<211> 35
<212> PRT
<213> Artificial Organism

<220>
<223> Synthetic peptide

<400> 356

Ala Lys Ser Arg Pro Ser His Tyr Pro Val Ser Ser Val Tyr Thr Glu
1 5 10 15

Thr Leu Lys Lys Lys Asn Glu Asp Arg Phe Gly Met Trp Ile Glu Tyr
20 25 30

Leu Arg Arg
35

<210> 357
<211> 7
<212> PRT
<213> Artificial Organism

<220>
<223> Synthetic peptide

<400> 357

Ala Val Arg Arg Gly Leu Arg
1 5

<210> 358
<211> 152
<212> PRT
<213> Artificial Organism

<220>
<223> Synthetic peptide

<400> 358

Ser His Asn Ile Leu Leu Tyr Glu Asp Gly His Ala Val Val Ala Asp
1 5 10 15

Phe Gly Glu Ser Arg Phe Leu Gln Ser Leu Asp Glu Asp Asn Met Thr
20 25 30

Lys Gln Pro Gly Asn Leu Arg Trp Met Ala Pro Glu Val Phe Thr Gln
35 40 45

```
Cys Thr Arg Tyr Thr Ile Lys Ala Asp Val Phe Ser Tyr Ala Leu Cys
    50                  55                  60

Leu Trp Glu Ile Leu Thr Gly Glu Ile Pro Phe Ala His Leu Lys Pro
65                  70                  75                  80

Ala Ala Ala Ala Ala Asp Met Ala Tyr His His Ile Arg Pro Pro Ile
                85                  90                  95

Gly Tyr Ser Ile Pro Lys Pro Ile Ser Ser Leu Leu Ile Arg Gly Trp
            100                 105                 110

Asn Ala Cys Pro Glu Ala Lys Ser Arg Pro Ser His Tyr Pro Val Ser
        115                 120                 125

Ser Val Tyr Thr Glu Thr Leu Lys Lys Lys Asn Glu Asp Arg Phe Gly
    130                 135                 140

Met Trp Ile Glu Tyr Leu Arg Arg
145                 150


<210>  359
<211>  125
<212>  PRT
<213>  Artificial Organism

<220>
<223>  Synthetic peptide

<400> 359

Ala Val Arg Arg Gly Leu Arg Glu Gly Gly Ala Met Ala Ala Ala Arg
1               5                   10                  15

Asp Pro Pro Glu Val Ser Leu Arg Glu Ala Thr Gln Arg Lys Leu Arg
            20                  25                  30

Arg Phe Ser Glu Leu Arg Gly Lys Leu Val Ala Arg Gly Glu Phe Trp
        35                  40                  45

Asp Ile Val Ala Ile Thr Ala Ala Asp Glu Lys Gln Glu Leu Ala Tyr
    50                  55                  60

Asn Gln Gln Leu Ser Glu Lys Leu Lys Arg Lys Glu Leu Pro Leu Gly
65                  70                  75                  80

Val Gln Tyr His Val Phe Val Asp Pro Ala Gly Ala Lys Ile Gly Asn
                85                  90                  95
```

```
Gly Gly Ser Thr Leu Cys Ala Leu Gln Cys Leu Glu Lys Leu Tyr Gly
            100                 105                 110

Asp Lys Trp Asn Ser Phe Thr Ile Leu Leu Ile His Ser
        115                 120                 125



<210>  360
<211>  13
<212>  PRT
<213>  Artificial Organism

<220>
<223>  Synthetic peptide

<400> 360

Met Gly Asn Tyr Lys Ser Arg Pro Thr Gln Thr Cys Thr
1               5                   10



<210>  361
<211>  35
<212>  PRT
<213>  Artificial Organism

<220>
<223>  Synthetic peptide

<400> 361

Ala Lys Ser Arg Pro Ser His Tyr Pro Val Ser Ser Val Tyr Thr Glu
1               5                   10                  15

Thr Leu Lys Lys Lys Asn Glu Asp Arg Phe Gly Met Trp Ile Glu Tyr
            20                  25                  30

Leu Arg Arg
        35



<210>  362
<211>  152
<212>  PRT
<213>  Artificial Organism

<220>
<223>  Synthetic peptide

<400> 362

Ser His Asn Ile Leu Leu Tyr Glu Asp Gly His Ala Val Val Ala Asp
1               5                   10                  15

Phe Gly Glu Ser Arg Phe Leu Gln Ser Leu Asp Glu Asp Asn Met Thr
```

20 25 30

Lys Gln Pro Gly Asn Leu Arg Trp Met Ala Pro Glu Val Phe Thr Gln
35 40 45

Cys Thr Arg Tyr Thr Ile Lys Ala Asp Val Phe Ser Tyr Ala Leu Cys
50 55 60

Leu Trp Glu Ile Leu Thr Gly Glu Ile Pro Phe Ala His Leu Lys Pro
65 70 75 80

Ala Ala Ala Ala Ala Asp Met Ala Tyr His His Ile Arg Pro Pro Ile
85 90 95

Gly Tyr Ser Ile Pro Lys Pro Ile Ser Ser Leu Leu Ile Arg Gly Trp
100 105 110

Asn Ala Cys Pro Glu Ala Lys Ser Arg Pro Ser His Tyr Pro Val Ser
115 120 125

Ser Val Tyr Thr Glu Thr Leu Lys Lys Lys Asn Glu Asp Arg Phe Gly
130 135 140

Met Trp Ile Glu Tyr Leu Arg Arg
145 150

<210> 363
<211> 11
<212> PRT
<213> Artificial Organism

<220>
<223> Synthetic peptide

<400> 363

Ala Val Arg Arg Gly Leu Arg Glu Gly Gly Ala
1 5 10

<210> 364
<211> 6
<212> PRT
<213> Artificial Organism

<220>
<223> Synthetic peptide

<400> 364

Arg Tyr Phe Phe Pro Lys
1 5

<210> 365
<211> 7
<212> PRT
<213> Artificial Organism

<220>
<223> Synthetic peptide

<400> 365

```
Ala Val Arg Arg Gly Leu Arg
1               5
```

<210> 366
<211> 125
<212> PRT
<213> Artificial Organism

<220>
<223> Synthetic peptide

<400> 366

```
Ala Val Arg Arg Gly Leu Arg Glu Gly Gly Ala Met Ala Ala Ala Arg
1               5                   10                  15

Asp Pro Pro Glu Val Ser Leu Arg Glu Ala Thr Gln Arg Lys Leu Arg
            20                  25                  30

Arg Phe Ser Glu Leu Arg Gly Lys Leu Val Ala Arg Gly Glu Phe Trp
        35                  40                  45

Asp Ile Val Ala Ile Thr Ala Ala Asp Glu Lys Gln Glu Leu Ala Tyr
    50                  55                  60

Asn Gln Gln Leu Ser Glu Lys Leu Lys Arg Lys Glu Leu Pro Leu Gly
65                  70                  75                  80

Val Gln Tyr His Val Phe Val Asp Pro Ala Gly Ala Lys Ile Gly Asn
                85                  90                  95

Gly Gly Ser Thr Leu Cys Ala Leu Gln Cys Leu Glu Lys Leu Tyr Gly
            100                 105                 110

Asp Lys Trp Asn Ser Phe Thr Ile Leu Leu Ile His Ser
        115                 120                 125
```

<210> 367
<211> 13

<212>  PRT
<213>  Artificial Organism

<220>
<223>  Synthetic peptide

<400> 367

Met Gly Asn Tyr Lys Ser Arg Pro Thr Gln Thr Cys Thr
1               5                   10

<210>  368
<211>  13
<212>  PRT
<213>  Artificial Organism

<220>
<223>  Synthetic peptide

<400> 368

Arg Cys Cys Thr Gly Trp Leu Ser Cys Tyr His Pro Asp
1               5                   10

<210>  369
<211>  12
<212>  PRT
<213>  Artificial Organism

<220>
<223>  Synthetic peptide

<400> 369

Cys Cys Thr Gly Trp Leu Ser Cys Tyr His Pro Asp
1               5                   10

<210>  370
<211>  3
<212>  PRT
<213>  Artificial Organism

<220>
<223>  Synthetic peptide

<400> 370

Arg Ala Ser
1

<210>  371
<211>  11
<212>  PRT
<213>  Artificial Organism

<220>
<223>  Synthetic peptide

<400> 371

Ala Val Arg Arg Gly Leu Arg Glu Gly Gly Ala
1               5                   10

<210>  372
<211>  34
<212>  PRT
<213>  Artificial Organism

<220>
<223>  Synthetic peptide

<400> 372

Tyr Gly Ser Phe Val Leu Ile Tyr Pro Trp Thr Phe Arg Arg Asn Tyr
1               5                   10                  15

Ser Cys Asn Thr Ser Glu Gly Phe Pro Leu Asp Glu Pro Ser Pro Phe
            20                  25                  30

Glu Ile

<210>  373
<211>  7
<212>  PRT
<213>  Artificial Organism

<220>
<223>  Synthetic peptide

<400> 373

Ala Val Arg Arg Gly Leu Arg
1               5

<210>  374
<211>  125
<212>  PRT
<213>  Artificial Organism

<220>
<223>  Synthetic peptide

<400> 374

Ala Val Arg Arg Gly Leu Arg Glu Gly Gly Ala Met Ala Ala Ala Arg
1               5                   10                  15

Asp Pro Pro Glu Val Ser Leu Arg Glu Ala Thr Gln Arg Lys Leu Arg

20 25 30

Arg Phe Ser Glu Leu Arg Gly Lys Leu Val Ala Arg Gly Glu Phe Trp
35 40 45

Asp Ile Val Ala Ile Thr Ala Ala Asp Glu Lys Gln Glu Leu Ala Tyr
50 55 60

Asn Gln Gln Leu Ser Glu Lys Leu Lys Arg Lys Glu Leu Pro Leu Gly
65 70 75 80

Val Gln Tyr His Val Phe Val Asp Pro Ala Gly Ala Lys Ile Gly Asn
85 90 95

Gly Gly Ser Thr Leu Cys Ala Leu Gln Cys Leu Glu Lys Leu Tyr Gly
100 105 110

Asp Lys Trp Asn Ser Phe Thr Ile Leu Leu Ile His Ser
115 120 125

<210> 375
<211> 13
<212> PRT
<213> Artificial Organism

<220>
<223> Synthetic peptide

<400> 375

Met Gly Asn Tyr Lys Ser Arg Pro Thr Gln Thr Cys Thr
1 5 10

<210> 376
<211> 34
<212> PRT
<213> Artificial Organism

<220>
<223> Synthetic peptide

<400> 376

Tyr Gly Ser Phe Val Leu Ile Tyr Pro Trp Thr Phe Arg Arg Asn Tyr
1 5 10 15

Ser Cys Asn Thr Ser Glu Gly Phe Pro Leu Asp Glu Pro Ser Pro Phe
20 25 30

Glu Ile

<210> 377
<211> 13
<212> PRT
<213> Artificial Organism

<220>
<223> Synthetic peptide

<400> 377

```
Met Gly Asn Tyr Lys Ser Arg Pro Thr Gln Thr Cys Thr
1               5                   10
```

<210> 378
<211> 3
<212> PRT
<213> Artificial Organism

<220>
<223> Synthetic peptide

<400> 378

```
Asn Leu Lys
1
```

<210> 379
<211> 114
<212> PRT
<213> Artificial Organism

<220>
<223> Synthetic peptide

<400> 379

```
Met Ala Ala Ala Arg Asp Pro Pro Glu Val Ser Leu Arg Glu Ala Thr
1               5                   10                  15

Gln Arg Lys Leu Arg Arg Phe Ser Glu Leu Arg Gly Lys Leu Val Ala
            20                  25                  30

Arg Gly Glu Phe Trp Asp Ile Val Ala Ile Thr Ala Ala Asp Glu Lys
        35                  40                  45

Gln Glu Leu Ala Tyr Asn Gln Gln Leu Ser Glu Lys Leu Lys Arg Lys
    50                  55                  60

Glu Leu Pro Leu Gly Val Gln Tyr His Val Phe Val Asp Pro Ala Gly
65                  70                  75                  80
```

```
Ala Lys Ile Gly Asn Gly Gly Ser Thr Leu Cys Ala Leu Gln Cys Leu
                85                  90                  95

Glu Lys Leu Tyr Gly Asp Lys Trp Asn Ser Phe Thr Ile Leu Leu Ile
            100                 105                 110

His Ser
```

<210>  380
<211>  266
<212>  PRT
<213>  Artificial Organism

<220>
<223>  Synthetic peptide

<400> 380

```
Ser Ala Ile Thr Ser Arg Ile Trp Ile Thr His Ser Ile Cys Ile Trp
1               5                   10                  15

Arg Gly Ala His Tyr Phe Asn Arg Glu Glu Cys Asn Phe Arg Cys Met
            20                  25                  30

Leu Thr Ser Ala Ile Leu Lys Glu Ser Arg Phe Leu Gln Ser Leu Asp
        35                  40                  45

Glu Asp Asn Met Thr Lys Gln Pro Gly Asn Leu Arg Trp Met Ala Pro
    50                  55                  60

Glu Val Phe Thr Gln Cys Thr Arg Tyr Thr Ile Lys Ala Asp Val Phe
65                  70                  75                  80

Ser Tyr Ala Leu Cys Leu Trp Glu Ile Leu Thr Gly Glu Ile Pro Phe
                85                  90                  95

Ala His Leu Lys Pro Ala Ala Ala Ala Ala Asp Met Ala Tyr His His
            100                 105                 110

Ile Arg Pro Pro Ile Gly Tyr Ser Ile Pro Lys Pro Ile Ser Ser Leu
        115                 120                 125

Leu Ile Arg Gly Trp Asn Ala Cys Pro Glu Gly Arg Pro Glu Phe Ser
    130                 135                 140

Glu Val Val Met Lys Leu Glu Glu Cys Leu Cys Asn Ile Glu Leu Met
145                 150                 155                 160
```

```
Ser Pro Ala Ser Ser Asn Ser Ser Gly Ser Leu Ser Pro Ser Ser Ser
                165                 170                 175

Ser Asp Cys Leu Val Asn Arg Gly Gly Pro Gly Arg Ser His Val Ala
            180                 185                 190

Ala Leu Arg Ser Arg Phe Glu Leu Glu Tyr Ala Leu Asn Ala Arg Ser
        195                 200                 205

Tyr Ala Ala Leu Ser Gln Ser Ala Gly Gln Tyr Ser Ser Gln Gly Leu
    210                 215                 220

Ser Leu Glu Glu Met Lys Arg Ser Leu Gln Tyr Thr Pro Ile Asp Lys
225                 230                 235                 240

Tyr Gly Tyr Val Ser Asp Pro Met Ser Ser Met His Phe His Ser Cys
                245                 250                 255

Arg Asn Ser Ser Ser Phe Glu Asp Ser Ser
            260                 265


<210>  381
<211>  35
<212>  PRT
<213>  Artificial Organism

<220>
<223>  Synthetic peptide

<400> 381

Ala Lys Ser Arg Pro Ser His Tyr Pro Val Ser Ser Val Tyr Thr Glu
1               5                   10                  15

Thr Leu Lys Lys Lys Asn Glu Asp Arg Phe Gly Met Trp Ile Glu Tyr
            20                  25                  30

Leu Arg Arg
        35


<210>  382
<211>  152
<212>  PRT
<213>  Artificial Organism

<220>
<223>  Synthetic peptide

<400> 382

Ser His Asn Ile Leu Leu Tyr Glu Asp Gly His Ala Val Val Ala Asp
```

```
1               5                   10                  15

Phe Gly Glu Ser Arg Phe Leu Gln Ser Leu Asp Glu Asp Asn Met Thr
            20                  25                  30

Lys Gln Pro Gly Asn Leu Arg Trp Met Ala Pro Glu Val Phe Thr Gln
        35                  40                  45

Cys Thr Arg Tyr Thr Ile Lys Ala Asp Val Phe Ser Tyr Ala Leu Cys
    50                  55                  60

Leu Trp Glu Ile Leu Thr Gly Glu Ile Pro Phe Ala His Leu Lys Pro
65                  70                  75                  80

Ala Ala Ala Ala Ala Asp Met Ala Tyr His His Ile Arg Pro Pro Ile
                85                  90                  95

Gly Tyr Ser Ile Pro Lys Pro Ile Ser Ser Leu Leu Ile Arg Gly Trp
            100                 105                 110

Asn Ala Cys Pro Glu Ala Lys Ser Arg Pro Ser His Tyr Pro Val Ser
        115                 120                 125

Ser Val Tyr Thr Glu Thr Leu Lys Lys Lys Asn Glu Asp Arg Phe Gly
    130                 135                 140

Met Trp Ile Glu Tyr Leu Arg Arg
145                 150


<210>  383
<211>  114
<212>  PRT
<213>  Artificial Organism

<220>
<223>  Synthetic peptide

<400> 383

Met Ala Ala Ala Arg Asp Pro Pro Glu Val Ser Leu Arg Glu Ala Thr
1               5                   10                  15

Gln Arg Lys Leu Arg Arg Phe Ser Glu Leu Arg Gly Lys Leu Val Ala
            20                  25                  30

Arg Gly Glu Phe Trp Asp Ile Val Ala Ile Thr Ala Ala Asp Glu Lys
        35                  40                  45

Gln Glu Leu Ala Tyr Asn Gln Gln Leu Ser Glu Lys Leu Lys Arg Lys
```

```
    50                  55                  60

Glu Leu Pro Leu Gly Val Gln Tyr His Val Phe Val Asp Pro Ala Gly
65                  70                  75                  80

Ala Lys Ile Gly Asn Gly Gly Ser Thr Leu Cys Ala Leu Gln Cys Leu
                85                  90                  95

Glu Lys Leu Tyr Gly Asp Lys Trp Asn Ser Phe Thr Ile Leu Leu Ile
            100                 105                 110

His Ser



<210>  384
<211>  6
<212>  PRT
<213>  Artificial Organism

<220>
<223>  Synthetic peptide

<400> 384

Arg Tyr Phe Phe Pro Lys
1               5



<210>  385
<211>  114
<212>  PRT
<213>  Artificial Organism

<220>
<223>  Synthetic peptide

<400> 385

Met Ala Ala Ala Arg Asp Pro Pro Glu Val Ser Leu Arg Glu Ala Thr
1               5                   10                  15

Gln Arg Lys Leu Arg Arg Phe Ser Glu Leu Arg Gly Lys Leu Val Ala
            20                  25                  30

Arg Gly Glu Phe Trp Asp Ile Val Ala Ile Thr Ala Ala Asp Glu Lys
        35                  40                  45

Gln Glu Leu Ala Tyr Asn Gln Gln Leu Ser Glu Lys Leu Lys Arg Lys
    50                  55                  60

Glu Leu Pro Leu Gly Val Gln Tyr His Val Phe Val Asp Pro Ala Gly
65                  70                  75                  80
```

Ala Lys Ile Gly Asn Gly Gly Ser Thr Leu Cys Ala Leu Gln Cys Leu
85 90 95

Glu Lys Leu Tyr Gly Asp Lys Trp Asn Ser Phe Thr Ile Leu Leu Ile
100 105 110

His Ser

<210> 386
<211> 34
<212> PRT
<213> Artificial Organism

<220>
<223> Synthetic peptide

<400> 386

Tyr Gly Ser Phe Val Leu Ile Tyr Pro Trp Thr Phe Arg Arg Asn Tyr
1 5 10 15

Ser Cys Asn Thr Ser Glu Gly Phe Pro Leu Asp Glu Pro Ser Pro Phe
20 25 30

Glu Ile

<210> 387
<211> 114
<212> PRT
<213> Artificial Organism

<220>
<223> Synthetic peptide

<400> 387

Met Ala Ala Ala Arg Asp Pro Pro Glu Val Ser Leu Arg Glu Ala Thr
1 5 10 15

Gln Arg Lys Leu Arg Arg Phe Ser Glu Leu Arg Gly Lys Leu Val Ala
20 25 30

Arg Gly Glu Phe Trp Asp Ile Val Ala Ile Thr Ala Ala Asp Glu Lys
35 40 45

Gln Glu Leu Ala Tyr Asn Gln Gln Leu Ser Glu Lys Leu Lys Arg Lys
50 55 60

```
Glu Leu Pro Leu Gly Val Gln Tyr His Val Phe Val Asp Pro Ala Gly
65                  70                  75                  80

Ala Lys Ile Gly Asn Gly Gly Ser Thr Leu Cys Ala Leu Gln Cys Leu
                85                  90                  95

Glu Lys Leu Tyr Gly Asp Lys Trp Asn Ser Phe Thr Ile Leu Leu Ile
            100                 105                 110

His Ser
```

<210> 388
<211> 42
<212> PRT
<213> Artificial Organism

<220>
<223> Synthetic peptide

<400> 388

```
Met Pro Arg Lys Asp Arg Asn Ser Ser Arg Ala Glu Ser Ala Gln Cys
1               5                   10                  15

Gln Val Leu Ser Cys Val Ile His Gly Ile Leu Leu Met Ala Arg Glu
            20                  25                  30

Ile Ala Val Val Val Leu Pro Leu Ser Gln
        35                  40
```

<210> 389
<211> 50
<212> PRT
<213> Artificial Organism

<220>
<223> Synthetic peptide

<400> 389

```
Val Gly Glu Ser Gly Pro Thr Thr Asn Leu Arg Lys Gly Leu Leu Gly
1               5                   10                  15

Pro Asp Pro Gln Gly Met Asp Pro Ser Leu Pro Pro Gly Ser Thr Pro
            20                  25                  30

Tyr Pro Cys Ile Asn Met Ile Gly Tyr Phe Pro Leu Ser Gly Pro His
        35                  40                  45
```

Phe Thr
    50

<210>  390
<211>  33
<212>  PRT
<213>  Artificial Organism

<220>
<223>  Synthetic peptide

<400> 390

Val Arg Leu Pro Ser Pro Phe Asn Thr His Pro Gln His Leu Leu Trp
1               5                   10                  15

Ala Phe Thr His Asp Pro Glu Pro Ser Thr Ser Glu Ala Val Ala Gly
            20                  25                  30

Arg

<210>  391
<211>  38
<212>  PRT
<213>  Artificial Organism

<220>
<223>  Synthetic peptide

<400> 391

Asp Gln Pro Phe Pro Ala Pro Trp Glu Pro Pro Tyr Pro Pro Ser Leu
1               5                   10                  15

Cys Ser His Ser Pro Ala Val Ser Cys Ser Asp Pro Pro His Pro Pro
            20                  25                  30

Gly Ser Ser Ser Phe Ser
        35

<210>  392
<211>  23
<212>  PRT
<213>  Artificial Organism

<220>
<223>  Synthetic peptide

<400> 392

Val Ser Pro Pro Pro Pro Thr Phe Pro Arg Ala Gly Gly Cys Ser His
1               5                   10                  15

Leu Lys Ala Pro Ile Pro Gln
            20

<210>  393
<211>  6
<212>  PRT
<213>  Artificial Organism

<220>
<223>  Synthetic peptide

<400> 393

Trp Ser Arg Cys Ser Pro
1               5

<210>  394
<211>  2
<212>  PRT
<213>  Artificial Organism

<220>
<223>  Synthetic peptide

<400> 394

Ala Ser
1

<210>  395
<211>  764
<212>  PRT
<213>  Homo sapiens

<400> 395

Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

```
Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190

Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335
```

```
Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
                405                 410                 415

Asn Pro Arg Glu Asp Tyr Leu Asp Val Tyr Val Phe Gly Val Gly Pro
            420                 425                 430

Leu Val Asn Gln Val Asn Ile Asn Ala Leu Ala Ser Lys Lys Asp Asn
        435                 440                 445

Glu Gln His Val Phe Lys Val Lys Asp Met Glu Asn Leu Glu Asp Val
    450                 455                 460

Phe Tyr Gln Met Ile Asp Glu Ser Gln Ser Leu Ser Leu Cys Gly Met
465                 470                 475                 480

Val Trp Glu His Arg Lys Gly Thr Asp Tyr His Lys Gln Pro Trp Gln
                485                 490                 495

Ala Lys Ile Ser Val Ile Arg Pro Ser Lys Gly His Glu Ser Cys Met
            500                 505                 510

Gly Ala Val Val Ser Glu Tyr Phe Val Leu Thr Ala Ala His Cys Phe
        515                 520                 525

Thr Val Asp Asp Lys Glu His Ser Ile Lys Val Ser Val Gly Gly Glu
    530                 535                 540

Lys Arg Asp Leu Glu Ile Glu Val Val Leu Phe His Pro Asn Tyr Asn
545                 550                 555                 560

Ile Asn Gly Lys Lys Glu Ala Gly Ile Pro Glu Phe Tyr Asp Tyr Asp
                565                 570                 575

Val Ala Leu Ile Lys Leu Lys Asn Lys Leu Lys Tyr Gly Gln Thr Ile
```

580 585 590

Arg Pro Ile Cys Leu Pro Cys Thr Glu Gly Thr Thr Arg Ala Leu Arg
595 600 605

Leu Pro Pro Thr Thr Thr Cys Gln Gln Gln Lys Glu Glu Leu Leu Pro
610 615 620

Ala Gln Asp Ile Lys Ala Leu Phe Val Ser Glu Glu Glu Lys Lys Leu
625 630 635 640

Thr Arg Lys Glu Val Tyr Ile Lys Asn Gly Asp Lys Lys Gly Ser Cys
645 650 655

Glu Arg Asp Ala Gln Tyr Ala Pro Gly Tyr Asp Lys Val Lys Asp Ile
660 665 670

Ser Glu Val Val Thr Pro Arg Phe Leu Cys Thr Gly Gly Val Ser Pro
675 680 685

Tyr Ala Asp Pro Asn Thr Cys Arg Gly Asp Ser Gly Gly Pro Leu Ile
690 695 700

Val His Lys Arg Ser Arg Phe Ile Gln Val Gly Val Ile Ser Trp Gly
705 710 715 720

Val Val Asp Val Cys Lys Asn Gln Lys Arg Gln Lys Gln Val Pro Ala
725 730 735

His Ala Arg Asp Phe His Ile Asn Leu Phe Gln Val Leu Pro Trp Leu
740 745 750

Lys Glu Lys Leu Gln Asp Glu Asp Leu Gly Phe Leu
755 760

<210> 396
<211> 936
<212> PRT
<213> Homo sapiens

<400> 396

Met Ala Ala Ala Arg Asp Pro Pro Glu Val Ser Leu Arg Glu Ala Thr
1 5 10 15

Gln Arg Lys Leu Arg Arg Phe Ser Glu Leu Arg Gly Lys Leu Val Ala
20 25 30

```
Arg Gly Glu Phe Trp Asp Ile Val Ala Ile Thr Ala Ala Asp Glu Lys
        35                  40                  45

Gln Glu Leu Ala Tyr Asn Gln Gln Leu Ser Glu Lys Leu Lys Arg Lys
    50                  55                  60

Glu Leu Pro Leu Gly Val Gln Tyr His Val Phe Val Asp Pro Ala Gly
65                  70                  75                  80

Ala Lys Ile Gly Asn Gly Gly Ser Thr Leu Cys Ala Leu Gln Cys Leu
                85                  90                  95

Glu Lys Leu Tyr Gly Asp Lys Trp Asn Ser Phe Thr Ile Leu Leu Ile
            100                 105                 110

His Ser Asp Glu Trp Lys Lys Lys Val Ser Glu Ser Tyr Val Ile Thr
        115                 120                 125

Ile Glu Arg Leu Glu Asp Asp Leu Gln Ile Lys Glu Lys Glu Leu Thr
    130                 135                 140

Glu Leu Arg Asn Ile Phe Gly Ser Asp Glu Ala Phe Ser Lys Val Asn
145                 150                 155                 160

Leu Asn Tyr Arg Thr Glu Asn Gly Leu Ser Leu Leu His Leu Cys Cys
                165                 170                 175

Ile Cys Gly Gly Lys Lys Ser His Ile Arg Thr Leu Met Leu Lys Gly
            180                 185                 190

Leu Arg Pro Ser Arg Leu Thr Arg Asn Gly Phe Thr Ala Leu His Leu
        195                 200                 205

Ala Val Tyr Lys Asp Asn Ala Glu Leu Ile Thr Ser Leu Leu His Ser
    210                 215                 220

Gly Ala Asp Ile Gln Gln Val Gly Tyr Gly Gly Leu Thr Ala Leu His
225                 230                 235                 240

Ile Ala Thr Ile Ala Gly His Leu Glu Ala Ala Asp Val Leu Leu Gln
                245                 250                 255

His Gly Ala Asn Val Asn Ile Gln Asp Ala Val Phe Phe Thr Pro Leu
            260                 265                 270

His Ile Ala Ala Tyr Tyr Gly His Glu Gln Val Thr Arg Leu Leu Leu
        275                 280                 285
```

```
Lys Phe Gly Ala Asp Val Asn Val Ser Gly Glu Val Gly Asp Arg Pro
    290                 295                 300

Leu His Leu Ala Ser Ala Lys Gly Phe Leu Asn Ile Ala Lys Leu Leu
305                 310                 315                 320

Met Glu Glu Gly Ser Lys Ala Asp Val Asn Ala Gln Asp Asn Glu Asp
                325                 330                 335

His Val Pro Leu His Phe Cys Ser Arg Phe Gly His His Asp Ile Val
            340                 345                 350

Lys Tyr Leu Leu Gln Ser Asp Leu Glu Val Gln Pro His Val Val Asn
        355                 360                 365

Ile Tyr Gly Asp Thr Pro Leu His Leu Ala Cys Tyr Asn Gly Lys Phe
    370                 375                 380

Glu Val Ala Lys Glu Ile Ile Gln Ile Ser Gly Thr Glu Ser Leu Thr
385                 390                 395                 400

Lys Glu Asn Ile Phe Ser Glu Thr Ala Phe His Ser Ala Cys Thr Tyr
                405                 410                 415

Gly Lys Ser Ile Asp Leu Val Lys Phe Leu Leu Asp Gln Asn Val Ile
            420                 425                 430

Asn Ile Asn His Gln Gly Arg Asp Gly His Thr Gly Leu His Ser Ala
        435                 440                 445

Cys Tyr His Gly His Ile Arg Leu Val Gln Phe Leu Leu Asp Asn Gly
    450                 455                 460

Ala Asp Met Asn Leu Val Ala Cys Asp Pro Ser Arg Ser Ser Gly Glu
465                 470                 475                 480

Lys Asp Glu Gln Thr Cys Leu Met Trp Ala Tyr Glu Lys Gly His Asp
                485                 490                 495

Ala Ile Val Thr Leu Leu Lys His Tyr Lys Arg Pro Gln Asp Glu Leu
            500                 505                 510

Pro Cys Asn Glu Tyr Ser Gln Pro Gly Gly Asp Gly Ser Tyr Val Ser
        515                 520                 525

Val Pro Ser Pro Leu Gly Lys Ile Lys Ser Met Thr Lys Glu Lys Ala
    530                 535                 540
```

Asp Ile Leu Leu Leu Arg Ala Gly Leu Pro Ser His Phe His Leu Gln
545 550 555 560

Leu Ser Glu Ile Glu Phe His Glu Ile Ile Gly Ser Gly Ser Phe Gly
565 570 575

Lys Val Tyr Lys Gly Arg Cys Arg Asn Lys Ile Val Ala Ile Lys Arg
580 585 590

Tyr Arg Ala Asn Thr Tyr Cys Ser Lys Ser Asp Val Asp Met Phe Cys
595 600 605

Arg Glu Val Ser Ile Leu Cys Gln Leu Asn His Pro Cys Val Ile Gln
610 615 620

Phe Val Gly Ala Cys Leu Asn Asp Pro Ser Gln Phe Ala Ile Val Thr
625 630 635 640

Gln Tyr Ile Ser Gly Gly Ser Leu Phe Ser Leu Leu His Glu Gln Lys
645 650 655

Arg Ile Leu Asp Leu Gln Ser Lys Leu Ile Ile Ala Val Asp Val Ala
660 665 670

Lys Gly Met Glu Tyr Leu His Asn Leu Thr Gln Pro Ile Ile His Arg
675 680 685

Asp Leu Asn Ser His Asn Ile Leu Leu Tyr Glu Asp Gly His Ala Val
690 695 700

Val Ala Asp Phe Gly Glu Ser Arg Phe Leu Gln Ser Leu Asp Glu Asp
705 710 715 720

Asn Met Thr Lys Gln Pro Gly Asn Leu Arg Trp Met Ala Pro Glu Val
725 730 735

Phe Thr Gln Cys Thr Arg Tyr Thr Ile Lys Ala Asp Val Phe Ser Tyr
740 745 750

Ala Leu Cys Leu Trp Glu Ile Leu Thr Gly Glu Ile Pro Phe Ala His
755 760 765

Leu Lys Pro Ala Ala Ala Ala Ala Asp Met Ala Tyr His His Ile Arg
770 775 780

Pro Pro Ile Gly Tyr Ser Ile Pro Lys Pro Ile Ser Ser Leu Leu Ile

785 790 795 800

Arg Gly Trp Asn Ala Cys Pro Glu Gly Arg Pro Glu Phe Ser Glu Val
805 810 815

Val Met Lys Leu Glu Glu Cys Leu Cys Asn Ile Glu Leu Met Ser Pro
820 825 830

Ala Ser Ser Asn Ser Ser Gly Ser Leu Ser Pro Ser Ser Ser Ser Asp
835 840 845

Cys Leu Val Asn Arg Gly Gly Pro Gly Arg Ser His Val Ala Ala Leu
850 855 860

Arg Ser Arg Phe Glu Leu Glu Tyr Ala Leu Asn Ala Arg Ser Tyr Ala
865 870 875 880

Ala Leu Ser Gln Ser Ala Gly Gln Tyr Ser Ser Gln Gly Leu Ser Leu
885 890 895

Glu Glu Met Lys Arg Ser Leu Gln Tyr Thr Pro Ile Asp Lys Tyr Gly
900 905 910

Tyr Val Ser Asp Pro Met Ser Ser Met His Phe His Ser Cys Arg Asn
915 920 925

Ser Ser Ser Phe Glu Asp Ser Ser
930 935

<210> 397
<211> 931
<212> PRT
<213> Homo sapiens

<400> 397

Met Ala Glu Val Glu Ala Val Gln Leu Lys Glu Glu Gly Asn Arg His
1 5 10 15

Phe Gln Leu Gln Asp Tyr Lys Ala Ala Thr Asn Ser Tyr Ser Gln Ala
20 25 30

Leu Lys Leu Thr Lys Asp Lys Ala Leu Leu Ala Thr Leu Tyr Arg Asn
35 40 45

Arg Ala Ala Cys Gly Leu Lys Thr Glu Ser Tyr Val Gln Ala Ala Ser
50 55 60

Asp Ala Ser Arg Ala Ile Asp Ile Asn Ser Ser Asp Ile Lys Ala Leu
65 70 75 80

Tyr Arg Arg Cys Gln Ala Leu Glu His Leu Gly Lys Leu Asp Gln Ala
85 90 95

Phe Lys Asp Val Gln Arg Cys Ala Thr Leu Glu Pro Arg Asn Gln Asn
100 105 110

Phe Gln Glu Met Leu Arg Arg Leu Asn Thr Ser Ile Gln Glu Lys Leu
115 120 125

Arg Val Gln Phe Ser Thr Asp Ser Arg Val Gln Lys Met Phe Glu Ile
130 135 140

Leu Leu Asp Glu Asn Ser Glu Ala Asp Lys Arg Glu Lys Ala Ala Asn
145 150 155 160

Asn Leu Ile Val Leu Gly Arg Glu Glu Ala Gly Ala Glu Lys Ile Phe
165 170 175

Gln Asn Asn Gly Val Ala Leu Leu Leu Gln Leu Leu Asp Thr Lys Lys
180 185 190

Pro Glu Leu Val Leu Ala Ala Val Arg Thr Leu Ser Gly Met Cys Ser
195 200 205

Gly His Gln Ala Arg Ala Thr Val Ile Leu His Ala Val Arg Ile Asp
210 215 220

Arg Ile Cys Ser Leu Met Ala Val Glu Asn Glu Glu Met Ser Leu Ala
225 230 235 240

Val Cys Asn Leu Leu Gln Ala Ile Ile Asp Ser Leu Ser Gly Glu Asp
245 250 255

Lys Arg Glu His Arg Gly Lys Glu Glu Ala Leu Val Leu Asp Thr Lys
260 265 270

Lys Asp Leu Lys Gln Ile Thr Ser His Leu Leu Asp Met Leu Val Ser
275 280 285

Lys Lys Val Ser Gly Gln Gly Arg Asp Gln Ala Leu Asn Leu Leu Asn
290 295 300

Lys Asn Val Pro Arg Lys Asp Leu Ala Ile His Asp Asn Ser Arg Thr
305 310 315 320

```
Ile Tyr Val Val Asp Asn Gly Leu Arg Lys Ile Leu Lys Val Val Gly
                325                 330                 335

Gln Val Pro Asp Leu Pro Ser Cys Leu Pro Leu Thr Asp Asn Thr Arg
            340                 345                 350

Met Leu Ala Ser Ile Leu Ile Asn Lys Leu Tyr Asp Asp Leu Arg Cys
        355                 360                 365

Asp Pro Glu Arg Asp His Phe Arg Lys Ile Cys Glu Glu Tyr Ile Thr
    370                 375                 380

Gly Lys Phe Asp Pro Gln Asp Met Asp Lys Asn Leu Asn Ala Ile Gln
385                 390                 395                 400

Thr Val Ser Gly Ile Leu Gln Gly Pro Phe Asp Leu Gly Asn Gln Leu
                405                 410                 415

Leu Gly Leu Lys Gly Val Met Glu Met Met Val Ala Leu Cys Gly Ser
            420                 425                 430

Glu Arg Glu Thr Asp Gln Leu Val Ala Val Glu Ala Leu Ile His Ala
        435                 440                 445

Ser Thr Lys Leu Ser Arg Ala Thr Phe Ile Ile Thr Asn Gly Val Ser
    450                 455                 460

Leu Leu Lys Gln Ile Tyr Lys Thr Thr Lys Asn Glu Lys Ile Lys Ile
465                 470                 475                 480

Arg Thr Leu Val Gly Leu Cys Lys Leu Gly Ser Ala Gly Gly Thr Asp
                485                 490                 495

Tyr Gly Leu Arg Gln Phe Ala Glu Gly Ser Thr Glu Lys Leu Ala Lys
            500                 505                 510

Gln Cys Arg Lys Trp Leu Cys Asn Met Ser Ile Asp Thr Arg Thr Arg
        515                 520                 525

Arg Trp Ala Val Glu Gly Leu Ala Tyr Leu Thr Leu Asp Ala Asp Val
    530                 535                 540

Lys Asp Asp Phe Val Gln Asp Val Pro Ala Leu Gln Ala Met Phe Glu
545                 550                 555                 560

Leu Ala Lys Ala Gly Thr Ser Asp Lys Thr Ile Leu Tyr Ser Val Ala
                565                 570                 575
```

```
Thr Thr Leu Val Asn Cys Thr Asn Ser Tyr Asp Val Lys Glu Val Ile
            580                 585                 590

Pro Glu Leu Val Gln Leu Ala Lys Phe Ser Lys Gln His Val Pro Glu
        595                 600                 605

Glu His Pro Lys Asp Lys Lys Asp Phe Ile Asp Met Arg Val Lys Arg
    610                 615                 620

Leu Leu Lys Ala Gly Val Ile Ser Ala Leu Ala Cys Met Val Lys Ala
625                 630                 635                 640

Asp Ser Ala Ile Leu Thr Asp Gln Thr Lys Glu Leu Leu Ala Arg Val
                645                 650                 655

Phe Leu Ala Leu Cys Asp Asn Pro Lys Asp Arg Gly Thr Ile Val Ala
            660                 665                 670

Gln Gly Gly Gly Lys Ala Leu Ile Pro Leu Ala Leu Glu Gly Thr Asp
        675                 680                 685

Val Gly Lys Val Lys Ala Ala His Ala Leu Ala Lys Ile Ala Ala Val
    690                 695                 700

Ser Asn Pro Asp Ile Ala Phe Pro Gly Glu Arg Val Tyr Glu Val Val
705                 710                 715                 720

Arg Pro Leu Val Arg Leu Leu Asp Thr Gln Arg Asp Gly Leu Gln Asn
                725                 730                 735

Tyr Glu Ala Leu Leu Gly Leu Thr Asn Leu Ser Gly Arg Ser Asp Lys
            740                 745                 750

Leu Arg Gln Lys Ile Phe Lys Glu Arg Ala Leu Pro Asp Ile Glu Asn
        755                 760                 765

Tyr Met Phe Glu Asn His Asp Gln Leu Arg Gln Ala Ala Thr Glu Cys
    770                 775                 780

Met Cys Asn Met Val Leu His Lys Glu Val Gln Glu Arg Phe Leu Ala
785                 790                 795                 800

Asp Gly Asn Asp Arg Leu Lys Leu Val Val Leu Leu Cys Gly Glu Asp
                805                 810                 815

Asp Asp Lys Val Gln Asn Ala Ala Ala Gly Ala Leu Ala Met Leu Thr
```

820 825 830

Ala Ala His Lys Lys Leu Cys Leu Lys Met Thr Gln Val Thr Thr Gln
835 840 845

Trp Leu Glu Ile Leu Gln Arg Leu Cys Leu His Asp Gln Leu Ser Val
850 855 860

Gln His Arg Gly Leu Val Ile Ala Tyr Asn Leu Leu Ala Ala Asp Ala
865 870 875 880

Glu Leu Ala Lys Lys Leu Val Glu Ser Glu Leu Leu Glu Ile Leu Thr
885 890 895

Val Val Gly Lys Gln Glu Pro Asp Glu Lys Lys Ala Glu Val Val Gln
900 905 910

Thr Ala Arg Glu Cys Leu Ile Lys Cys Met Asp Tyr Gly Phe Ile Lys
915 920 925

Pro Val Ser
930

<210> 398
<211> 175
<212> PRT
<213> Homo sapiens

<400> 398

Met Ala Ser Arg Lys Ala Gly Thr Arg Gly Lys Val Ala Ala Thr Lys
1 5 10 15

Gln Ala Gln Arg Gly Ser Ser Asn Val Phe Ser Met Phe Glu Gln Ala
20 25 30

Gln Ile Gln Glu Phe Lys Glu Ala Phe Ser Cys Ile Asp Gln Asn Arg
35 40 45

Asp Gly Ile Ile Cys Lys Ala Asp Leu Arg Glu Thr Tyr Ser Gln Leu
50 55 60

Gly Lys Val Ser Val Pro Glu Glu Glu Leu Asp Ala Met Leu Gln Glu
65 70 75 80

Gly Lys Gly Pro Ile Asn Phe Thr Val Phe Leu Thr Leu Phe Gly Glu
85 90 95

```
Lys Leu Asn Gly Thr Asp Pro Glu Glu Ala Ile Leu Ser Ala Phe Arg
            100                 105                 110

Met Phe Asp Pro Ser Gly Lys Gly Val Val Asn Lys Asp Glu Phe Lys
        115                 120                 125

Gln Leu Leu Leu Thr Gln Ala Asp Lys Phe Ser Pro Ala Glu Val Glu
    130                 135                 140

Gln Met Phe Ala Leu Thr Pro Met Asp Leu Ala Gly Asn Ile Asp Tyr
145                 150                 155                 160

Lys Ser Leu Cys Tyr Ile Ile Thr His Gly Asp Glu Lys Glu Glu
                165                 170                 175
```